(19) Europäisches Patentamt
European Patent Office
Office européen des brevets

(11) EP 3 740 493 B1

(12) EUROPEAN PATENT SPECIFICATION

(45) Date of publication and mention of the grant of the patent:
**01.12.2021 Bulletin 2021/48**

(21) Application number: **19700695.0**

(22) Date of filing: **14.01.2019**

(51) Int Cl.:
***C07D 513/04*** *(2006.01)* ***A61K 31/431*** *(2006.01)*
***A61P 35/00*** *(2006.01)*

(86) International application number:
**PCT/EP2019/050803**

(87) International publication number:
**WO 2019/138107 (18.07.2019 Gazette 2019/29)**

# (54) INHIBTEURS DE L'INDOLEAMINE 2,3-DIOXYGÉNASE ET/OU DU TRYPTOPHANE DIOXYGÉNASE

INHIBITOREN VON INDOLAMIN-2,3-DIOXYGENASE UND/ODER TRYPTOPHAN-2,3-DIOXYGENASE

INHIBTEURS DE L'INDOLÉAMINE 2,3-DIOXYGÉNASE ET/OU DU TRYPTOPHANE DIOXYGÉNASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.01.2018 PCT/EP2018/050884**
**27.09.2018 PCT/EP2018/076272**

(43) Date of publication of application:
**25.11.2020 Bulletin 2020/48**

(73) Proprietor: **Idorsia Pharmaceuticals Ltd**
**4123 Allschwil (CH)**

(72) Inventors:
- **BOSS, Christoph**
  **4123 Allschwil (CH)**
- **CREN, Sylvaine**
  **4123 Allschwil (CH)**
- **KIMMERLIN, Thierry**
  **4123 Allschwil (CH)**
- **LOTZ-JENNE, Carina**
  **4123 Allschwil (CH)**
- **POTHIER, Julien**
  **4123 Allschwil (CH)**
- **TIDTEN-LUKSCH, Naomi**
  **4123 Allschwil (CH)**

(74) Representative: **Velker, Jörg**
**c/o Idorsia Pharmaceuticals Ltd**
**Hegenheimermattweg 91**
**4123 Allschwil (CH)**

(56) References cited:
**WO-A1-2011/037780**
**WO-A1-2016/161960**
**WO-A1-2018/036414**
**WO-A1-2018/054365**
**WO-A1-2019/034725**
**CA-A1- 3 029 305**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 740 493 B1

## Description

The present invention relates to compounds represented by Formula (I), or pharmaceutically acceptable salts thereof, and their use as active ingredients in medicine. The invention further concerns a process for the preparation of said compounds, pharmaceutical compositions containing one or more of said compounds, and their use, either alone or in combination with other active compounds or therapies as modulators of the activity of indoleamine 2,3-dioxygenase (IDO; also known as IDO1) and/or tryptophan 2,3-dioxygenase (TDO) enzymes.

The enzymes IDO and TDO catalyze the first and rate limiting step in the kynurenine pathway which is responsible for more than 95% of the degradation of the essential amino acid tryptophan (TRP). The catabolism of TRP is a central pathway maintaining the immunosuppressive microenvironment in many types of cancers. The kynurenine pathway is also involved in physiological functions such as behavior, sleep, thermoregulation and pregnancy.

The classic concept proposes that tumor cells or myeloid cells in the tumor microenvironment or draining lymph nodes express high levels of IDO resulting in the depletion of TRP and accumulation of TRP metabolites in the local microenvironment and subsequent inhibition of T cell responses. This IDO-centered concept is supported by numerous preclinical studies in models of tumor immunity, autoimmunity, infection, and allergy. More recent preclinical studies propose an alternative route of TRP degradation in tumors via the enzyme TDO. It has been suggested that targeting TDO may complement IDO inhibition. Thus, inhibition of IDO and/or TDO enzymes may be utilized in preventing and/or treating cancers. Moreover, a wide spectrum of further diseases and/or disorders notably neurological conditions, infectious and other diseases may be prevented and/or treated by targeting IDO and/or TDO.

Several IDO and/or TDO inhibitors are described in WO2010005958, WO2011037780, WO2012142237, WO2015173764, WO2016073770 and some have been clinically tested as anticancer agents either alone or in combination with other compounds/therapies. WO2016161960, WO2017134555, WO2018036414, WO2017007700, WO2017189386, WO2017133258, CN107556244, WO2018057973, WO2018136887, WO2018171602 and WO2018054365 disclose certain heterocyclic derivatives which may be used for inhibiting IDO and/or TDO enzymes. PCT/EP2018/072187 relates to certain imidazothiazole inhibitors of IDO and/or TDO.

Studying human tumor samples for expression of TDO2 gene revealed significant expression in 41% of bladder carcinomas, 50% of melanomas and 100% of hepatocarcinomas (Pilotte et al.; Proc Natl Acad Sci. 2012,109(7):2497-502). Moreover, TDO is expressed constitutively in human glioblastomas. Besides the suppression of anti-tumor immune responses, TDO-derived kynurenine (KYN) has been shown to have a tumor cell autonomous effect in glioblastoma, promoting tumor-cell survival and motility through the aryl hydrocarbon receptor (AHR) in an autocrine fashion. The TDO-AHR pathway in human brain tumors was found to be associated with malignant progression and poor survival. Elevated expression of TDO has also been observed in clinical specimens of Triple Negative Breast Cancer (TNBC) and was associated with increased disease grade, estrogen receptor negative status and shorter overall survival. KYN production mediated by TDO in TNBC cells was sufficiently to activate the AhR promoting anoikis resistance, migration, and invasion (D'Amato et al.; Cancer Res. 2015,75(21):4651-64).

TDO expression has been detected in other cancer indications, such as for example renal cell carcinoma, mesothelioma, neuroblastoma, leukemia, lung carcinoma (NSCLC), head&neck carcinoma, colorectal carcinoma, sarcoma, astrocytoma, myeloma, and pancreatic carcinoma (Pilotte et al.; Proc Natl Acad Sci. 2012,109(7):2497-502).

IDO expression levels in patient tumor samples varied slightly with the use of different antibodies reflecting the potential for alternative splice variants and/or post-translational modifications. Overall, IDO expression was found in a large fraction (>50%) of human tumors comprising tumor cells, endothelial cells, and stromal cells in proportions that varied depending on the tumor type (Uyttenhove et al.; Nat Med. 2003,9(10):1269-74). Tumors showing the highest proportions of IDO-immunolabeled samples were carcinomas of the endometrium and cervix, followed by kidney, lung, and colon. This hierarchy of IDO expression was confirmed by gene expression data mined from The Cancer Genome Atlas database (Theate et al.; Cancer Immunol Res. 2015,3(2):161-72). In most studies, high expression of IDO in the tumor or draining lymph nodes has been an adverse prognostic factor. Tumor in this category include melanoma, colon cancer, brain tumors, ovarian cancer, acute myelogenous leukemia, endometrial cancer, high-grade osteosarcoma and a number of others (Munn and Mellor; Trends in Immunol. 2016, 37(3): 193-207). In a smaller number of tumor types, IDO expression appears to be induced or 'reactive' - that is associated with increased T cell infiltration and inflammation. In this situation, upregulation of IDO may be a proxy for a stronger spontaneous anti-tumor immune response, and thus associated with more favorable prognosis. However, even in these immuneresponsive patients, the IDO itself is not beneficial, and the patient might do even better if IDO were blocked.

Because of the differences observed for IDO expression levels in patient samples using different antibodies, measuring IDO activity by determining concentrations of KYN and TRP in the serum might be more meaningful. Indeed, increased KYN/TRP ratios have been detected in sera from cancer patients compared to normal volunteers (Liu et al.; Blood. 2010,115(17):3520-30). The KYN/TRP ratio was recently validated as a prognostic tool in cervical cancer patients whereby low TRP levels indicated a tumor size greater than 4 cm and metastatic spread to the lymph node (Ferns et al.; Oncoimmunology. 2015,4(2):e981457). Accordingly, high KYN/TRP ratios in patient sera were associated with lymph

node metastasis, FIGO stage, tumor size, parametrial invasion and poor disease-specific survival, further suggesting the relevance of IDO targeting based on a TRP catabolic signature. Moreover, serum KYN/TRP ratio was a significantly independent detrimental prognostic factor in patients with adult T-cell leukemia/lymphoma (Zhai et al.; Clin Cancer Res. 2015,21(24):5427-33).

In preclinical models transfection of immunogenic tumor cells with recombinant IDO prevented their rejection in mice (Uyttenhove et al.; Nat Med. 2003,9(10):1269-74). While ablation of IDO expression led to a decrease in the incidence and growth of 7,12-dimethylbenz[a]anthracene-induced premalignant skin papillomas (Muller et al.; Proc Natl Acad Sci USA. 2008,105(44):17073-8).

In preclinical models of B16 melanoma overexpressing IDO and 4T1 breast cancer, IDO expression by tumor cells promoted tumor growth through the recruitment and activation of myeloid-derived suppressor cells (MDSC) and resistance to checkpoint blockade using anti-CTLA-4 and anti-PD-1. In the same study, it was also noted that IDO expression in human melanoma tumors is strongly associated with MDSC infiltration (Holmgaard et al.; Cell Rep. 2015,13(2):412-24).

Imatinib, a small-molecule receptor tyrosine kinase inhibitor targeting KIT (CD117), used for treatment of gastrointestinal stromal tumor (GIST), has been shown to modulate the KYN pathway. In a mouse model of GIST, imatinib therapy produced a number of immunological responses by reducing tumor cell expression of IDO. To test the hypothesis that the immune effects of imatinib are partially mediated by its reduction of IDO expression, GIST mice were treated with a cocktail of KYN pathway metabolites-KYN, 3-hydroxyanthranilic acid (3-HAA), and 3-hydroxykynurenine (3-HK), designed to simulate a system with competent IDO activity. The antitumor effects of imatinib were diminished by coadministration of the TRP metabolite cocktail. However, the antitumor effects of imatinib were not increased by co-administration of the IDO inhibitor 1-methyl-tryptophan (1-MT), consistent with the hypothesis that both agents are impacting the same pathway (Balachandran et al.; Nat Med. 2011, 17(9): 1094-100).

It has been shown that TDO expression by tumors prevented their rejection by immunized mice and systemic treatment with a TDO inhibitor restored the ability of mice to reject the TDO-expressing tumors (Pilotte et al.; Proc Natl Acad Sci. 2012,109(7):2497-502). In a transplantable model of glioma, TDO expression in tumor cells promoted tumor growth while TDO knockdown decreased tumor incidence (Opitz et al.; Nature 2011, 478(7368):197-203).

IDO inhibitors have been found to suppress TRP metabolism in vivo in tumors and blood which was accompanied by a slowdown of tumor outgrowth in experimental models of colorectal cancer (Lin et al.; J Med Chem. 2016,59(1):419-30; Koblish et al.; Mol Cancer Ther. 2010,9(2):489-98; Kraus et al.; AACR Annual Meeting (April 16-20, New Orleans, Louisiana) 2016: abstract 4863 ; Wise et al.; AACR Annual Meeting (April 16-20, New Orleans, Louisiana) 2016: abstract 5115; Liu et al.; AACR Annual Meeting (April 16-20, New Orleans, Louisiana) 2016: abstract 4877), pancreatic cancer (Koblish et al.; Mol Cancer Ther. 2010,9(2):489-98), melanoma (Yue et al.; J Med Chem. 2009,52(23):7364-7), lung (Yang et al.; J Med Chem. 2013, 56(21):8321-31), breast cancer (Holmgaard et al.; Cell Rep. 2015,13(2):412-24), glioma (Hanihara et al.; J Neurosurg. 2016,124(6):1594-601).

1-Methyl-Tryptophan (1-MT) augmented the effect of chemotherapy in mouse models of transplantable melanoma (B16) and transplantable and autochthonous breast cancer (4T1) (Hou et al.; Cancer Res. 2007,67(2):792-801). Furthermore, 1-MT enhanced chemo-radiation therapy to prolong survival in mice bearing intracranial glioblastoma tumors (GL-261). In this context inhibition of IDO allowed chemo-radiation to trigger widespread complement deposition at sites of tumor growth. IDO-blockade led to upregulation of VCAM-1 on vascular endothelium within the tumor microenvironment. Mice genetically deficient in complement component C3 lost all of the synergistic effects of IDO-blockade on chemo-radiation-induced survival (Li et al.; Journal Immunother Cancer. 2014,2:21). IDO expression is induced in the tumor epithelium of a significant number of patients with pancreatic cancer after GVAX (irradiated, GM-CSF-secreting, allogeneic PDAC) vaccination. GVAX vaccination combined with IDO inhibition increases survival in a preclinical model of pancreatic cancer and with the combination of cyclophosphamide, GVAX vaccine, IDO inhibition and PD-L1 blockade all mice survived (Zheng, John Hopkins School of Medicine; ITOC3 conference (March 21-23, Munich, Germany) 2016). In this context, vaccination combined with increasing doses of anti-OX40 has also been shown to induce IDO in the TC1 tumor model and inhibition of IDO by 1-MT showed synergistic effects with anti-OX40 and vaccination in the same model (Khleif, Georgia Cancer Center; ITOC3 conference (March 21-23, Munich, Germany) 2016). Moreover, IDO inhibitor epacadostat has been shown to enhance the effect of anti-OX40 and anti-GITR in preclinical models (Koblish et al.; AACR Annual Meeting (April 1-5, Washington DC) 2017: abstract #2618).

The IDO/TDO dual inhibitor NLG919 enhanced the antitumor responses of naive, resting adoptively transferred pmel-1 cells to vaccination with cognate human gp100 peptide in the B16F10 tumor model. The effect was additive with chemotherapy and even more pronounced once chemotherapy was combined with indoximod/anti-PD-1 (Mautino et al.; AACR Annual Meeting (April 5-9, San Diego, California) 2014: abstract 5023). Along these lines, improved depth and duration of tumor growth inhibition was detected when NLG-919 was combined with anti-PD-L1 in the EMT-6 mouse model (Spahn et al.; Journal for ImmunoTherapy of Cancer 2015, 3 (Suppl 2) : P303).

IDO-selective inhibitors have been shown to enhance chemotherapy in the tumor mouse models: An IDO-selective inhibitor from IOMet Pharma enhances chemotherapy (gemcitabine and abraxane) in the PAN02 model (Wise

et al.; AACR Annual Meeting (April 16-20, New Orleans, Louisiana) 2016: abstract 5115).

In plasma and tumor tissue, anti-PD-L1 and anti-CTLA4 checkpoint blockade induce IDO activity, while the combination of an IDO-selective inhibitor (PF-06840003) and anti-PD-L1 treatment resulted in significant tumor growth inhibition in the CT-26 syngeneic mouse colon tumor model (Kraus et al.; AACR Annual Meeting (April 16-20, New Orleans, Louisiana) 2016: abstract 4863). In another study, doublet therapies using either anti-CTLA-4, anti-PD-L1 and/or an IDO inhibitor showed synergistic retardation of tumor outgrowth in the B16(SIY) melanoma mouse model (Spranger et al.; J Immunother Cancer. 2014,2:3). The major biologic correlate to this improved efficacy was restored IL-2 production and proliferation of tumor-infiltrating CD8 T cells. Functional restoration did not require new T cell migration to the tumor. In yet another study, inhibition of IDO by 1-MT in combination with therapies targeting immune checkpoints such as CTL-4, PD-1/PD-L1, and GITR synergize to control tumor outgrowth and enhance overall survival in the B16-F10 and 4T1 tumor mouse models (Holmgaard et al.; J Exp Med. 2013,210(7):1389-402). In an orthotopic glioma model triple treatment with anti-CTLA-4, anti-PD-L1 and 1-MT as well as the combination of Epacadostat and anti-PD-1 resulted in a highly effective durable survival advantage (Wainwright et al.; Clin Cancer Res. 2014,20(20):5290-301; Reardon et al.; AACR Annual Meeting (April 1-5, Washington DC) 2017: abstract 572). The concept of targeting IDO in combination with checkpoint blockade has been investigated in several clinical trials (NCT02752074, NCT02658890, NCT02327078, NCT02318277, NCT02178722, NCT02471846, NCT02298153).

Intra-tumoral treatment with a TLR9 agonist was shown to induce IDO expression in treated and distant tumors and the combination of an IDO inhibitor with the same TLR9 agonist showed additive anti tumor effects in the CT-26 syngeneic mouse colon tumor model (Wang et al.; AACR Annual Meeting (April 16-20, New Orleans, Louisiana) 2016: abstract 3847).

High IDO expression induces recruitment of immunosuppressive MDSC to tumors in several mouse models. CSF-1R was found to be expressed on MDSCs and CSF-1R blockade to inhibit intratumoral MDSCs. Accordingly, inhibiting IDO with D-1-MT was shown to synergize with CSF-1R blockade in the B16 model overexpressing IDO (Holmgaard et al.; EBioMedicine 2016,6:50-8).

There is experimental evidence that IDO inhibition also improves the therapeutic response to chimeric antigen receptor (CAR) T cell therapy in B cell lymphoma. In a mouse model of B cell lymphoma IDO expression in tumor cells suppress CD19 CAR T cell therapy through the action of TRP metabolites. The treatment with the IDO inhibitor 1-MT restored tumor control by CAR T cells in this model (Ninomiya et al.; Blood, 2015,125(25):3905-16).

DNA nanoparticles can induce IDO via a pathway dependent on the stimulator of interferon genes (STING) sensor of cytosolic DNA. Accordingly, STING agonists can induce IDO and promote tolerogenic responses. This scenario has been studied in preclinical models using tumors with low and high antigenicity. In tumors exhibiting low antigenicity IDO activation by STING is predominant and overcomes STING/IFN immunogenic responses while in tumors with high antigenicity the STING/IFN signaling rather potentiates immunogenic responses and fails to induce IDO. Overall these data suggest that IDO inhibition can enhance the anti-tumor response to STING agonists particularly in tumors with low antigenicity (Lemos et al.; Cancer Res. 2016,76(8):2076-81).

Given the role of the JAK-STAT (signal transducer and activator of transcription) signalling system in mediating interferon-$\gamma$-induced IDO expression, it is obvious to combine IDO inhibitors with JAK/STAT inhibitors. A clinical trial on this treatment concept has been reported (NCT02559492).

In the central nervous system both fates of TRP which act as a precursor to KYN and serotonin are pathways of interest and importance. Metabolites produced by the KYN pathway have been implicated to play a role in the pathomechanism of neuroinflammatory and neurodegenerative disorder such as Huntington's disease. The first stable intermediate from the KYN pathway is KYN. Subsequently, several neuroactive intermediates are generated. They include Kynurenic acid (KYNA), 3-Hydroxykynurenine (3-HK), and Quinolinic acid (QUIN). 3-HK and QUIN are neurotoxic by distinct mechanisms; 3-HK is a potent free-radical generator (Thevandavakkam et al.; CNS Neurol Disord. Drug Targets. 2010, 9(6):791—800; Ishii et al.; Arch Biochem Biophys. 1992, 294(2):616—622; Hiraku et al.; Carcinogenesis. 1995,16(2):349-56), whereas QUIN is an excitotoxic N-methyl-D-aspartate (NMDA) receptor agonist (Stone and Perkins; Eur J Pharmacol. 1981, 72(4):411-2; Schwarcz et al; Science. 1983, 219(4582):316-8). KYNA, on the other hand, is neuroprotective through its antioxidant properties and antagonism of both the $\alpha$7 nicotinic acetylcholine receptor and the glycine coagonist site of the NMDA receptor (Vecsei and Beal; Brain Res Bull. 1990,25(4):623-7; Foster et al.; Neurosci Lett. 1984, 48(3):273—8; Carpenedo et al.; Eur J Neurosci. 2001,13(11):2141—7; Goda et al.; Adv. Exp. Med. Biol. 1999,467:397-402). Changes in the concentration levels of TRP catabolites can shift the balance to pathological conditions. The ability to influence the metabolism towards the neuroprotective branch of the KYN pathway, i.e. towards KYNA synthesis, may be used in preventing neurodegenerative diseases.

In the CNS, the KYN pathway is present to varying extents in most cell types, infiltrating macrophages, activated microglia and neurons have the complete repertoire of KYN pathway enzymes. On the other hand, neuroprotective astrocytes and oligodendrocytes lack the enzyme, KYN 3-monooxygenase (KMO) and IDO-1 respectively, and are incapable of synthesizing the excitotoxin QUIN (Guillemin et al.; Redox Rep 2000, 5(2-3): 108-11 ; Lim et al.; International Congress Series. 2007,1304: 213-7). TDO is expressed in low quantities in the brain, and is induced by TRP or corticosteroids (Salter and Pogson; Biochem J. 1985,229(2): 499-504; Miller et al.; Neurobiol Dis. 2004,15(3): 618-29). Given the role of TDO and IDO in the pathogenesis of several CNS disorders such as schizophrenia as well as the role of TDO in controlling systemic TRP levels, IDO and/or TDO inhibitors could be used to improve the outcomes of patients with a wide variety of CNS diseases and neurodegeneration.

IDO and/or TDO inhibitors may in addition be useful for the treatment of Amyotrophic lateral sclerosis (ALS) (or Lou Gehrig's disease). ALS results in the selective attacking and destruction of motor neurons in the motor cortex, brainstem and spinal cord. Although multiple mechanisms are likely to contribute to ALS, the KYN pathway activated during neuroinflammation is emerging as a contributing factor. Initial inflammation may inflict a nonlethal injury to motor neurons of individuals with a susceptible genetic constitution, in turn triggering a progressive inflammatory process which activates microglia to produce neurotoxic KYN metabolites that further destroy motor neurons. In the brain and spinal cord of ALS patients large numbers of activated microglia, reactive astrocytes, T cells and infiltrating macrophages have been observed (Graves et al.; Amyotroph Lateral Scler Other Motor Neuron Disord. 2004, 5(4):213-9; Henkel et al.; Ann Neurol. 2004, 55(2):221-35). These cells release inflammatory and neurotoxic mediators, among others IFN-$\gamma$, the most potent inducer of IDO (McGeer and McGeer; Muscle Nerve. 2002;26(4):459-70). The neuronal and microglial expression of IDO is increased in ALS motor cortex and spinal cord (Chen et al.; Neurotox Res. 2010,18(2):132—42). It has been proposed that the release of immune activating agents activates the rate-limiting enzyme of the KYN pathway, IDO, which generates metabolites such as the neurotoxin QUIN. Therefore, inhibition of IDO may reduce the synthesis of neurotoxic QUIN, which has been clearly implicated in the pathogenesis of ALS.

IDO and/or TDO inhibitors may in addition be useful for the treatment of Huntington's disease (HD). HD is a genetic autosomal dominant neurodegenerative disorder caused by expansion of the CAG repeats in the huntingtin (htt) gene. Patients affected by HD display progressive motor dysfunctions characterized by abnormality of voluntary and involuntary movements (choreoathetosis) and psychiatric and cognitive disturbances. In-life monitoring of metabolites within the KYN pathway provide one of the few biomarkers that correlates with the number of CAG repeats and hence the severity of the disorder (Forrest et al.; J Neurochem 2010, 112(1):112-22). Indeed, in patients with HD and HD model mice, 3-HK and QUIN levels are increased in the neostriatum and cortex. Moreover, KYNA levels are reduced in the striatum of patients with HD. Intrastriatal injection of QUIN in rodents reproduces behavioural and pathological features of HD (Sapko et al.; Exp Neurol. 2006 197(1):31-40). Importantly, TDO ablation in a Drosophila model of HD ameliorated neurodegeneration (Campesan et al.; Curr Biol. 2011;21(11):961-6).

IDO and/or TDO inhibitors may in addition be useful for the treatment of Alzheimer's disease (AD). AD is an age-related neurodegenerative disorder characterised by neuronal loss and dementia. The histopathology of the disease is manifested by the accumulation of intracellular $\beta$-amyioid (A$\beta$) and subsequent formation of neuritic plaques as well as the presence of neurofibrillary tangles in specific brain regions associated with learning and memory. The pathological mechanisms underlying this disease are still controversial, however, there is growing evidence implicating KYN pathway metabolites in the development and progression of AD. It has been shown that A$\beta$ (1-42) can activate primary cultured microglia and induce IDO expression (Guillemin et al.; Redox Rep. 2002,7(4):199-206; Walker et al.; J Leukoc Biol. 2006, 79:596-610). Furthermore, IDO overexpression and increased production of QUIN have been observed in microglia associated with the amyloid plaques in the brain of AD patients (Guillemin et al.; Neuropathol Appl Neurobiol. 2005, 31(4):395-404). QUIN has been shown to lead to tau hyperphosphorylation in human cortical neurons (Rahman et al.; PLOS One. 2009, 4(7):e6344). Thus, overexpression of IDO and over-activation of the KYN pathway in microglia are implicated in the pathogenesis of AD. There is also evidence for TDO involvement in Alzheimer's disease. TDO is upregulated in the brain of patients and AD mice models. Furthermore, TDO co-localizes with quinolinic acid, neurofibrillary tangles-tau and amyloid deposits in the hippocampus of AD patients (Wu et al.; PLOS One. 2013, 8(4):e59749). Preclinical evidence supports the use of KMO, TDO, IDO, and 3HAO inhibitors to offset the effects of neuroinflammation in AD. Moreover, other observations have demonstrated that the ratio of KYN/TRP is increased in the serum of AD patients (Widner et al.; J Neural Transm (Vienna). 2000, 107(3):343-53). In fly models of AD both genetic and pharmacological inhibition of TDO provides robust neuroprotection (Breda et al.; Proc Natl Acad Sci. 2016,113(19):5435-40). Therefore, the KYN pathway is over-activated in AD by both TDO and IDO and may be involved in neurofibrillary tangle formation and associated with senile plaque formation.

IDO and/or TDO inhibitors may in addition be useful for the treatment of Parkinson's disease (PD). PD is a common neurodegenerative disorder characterised by loss of dopaminergic neurons and localized neuroinflammation. Parkinson's disease is associated with chronic activation of microglia (Gao and Hong; Trends Immunol. 2008, 29(8):357—65). Microglia activation release neurotoxic substances including reactive oxygen species (ROS) and proinflammatory cytokines such as INF-$\gamma$ (Block et al.; Nat Rev Neurosci. 2007; 8(1):57—69), a potent activator of KYN pathway via induction of IDO expression. KYN pathway in activated microglia leads to upregulation of 3HK and QUIN. 3HK is toxic primarily as a result of conversion to ROS (Okuda et al.; J Neurochem. 1998;70(1):299—307). The combined effects of ROS and NMDA receptormediated excitotoxicity by QUIN contribute to the dysfunction of neurons and their death (Stone and Perkins; Eur J Pharmacol. 1981, 72(4): 411-2; Braidy et al.; Neurotox Res. 2009, 16(1):77-86). However, picolinic acid (PIC) produced through KYN pathway activation in neurons, has the ability to protect neurons against

QUIN-induced neurotoxicity, being a NMDA agonist (Jhamandas et al.; Brain Res. 1990, 529(1-2):185-91). Microglia can become overactivated, by proinflammatory mediators and stimuli from dying neurons and cause perpetuating cycle of further microglia activation microgliosis. Excessive microgliosis will cause neurotoxicity to neighbouring neurons and resulting in neuronal death, contributing to progression of Parkinson's disease. Therefore, PD is associated with an imbalance between the two main branches of the KYN pathway within the brain. KYNA synthesis by astrocytes is decreased and concomitantly, QUIN production by microglia is increased. Importantly, both genetic and pharmacological inhibition of TDO provided robust neuroprotection in a fly model of PD (Breda et al.; Proc Natl Acad Sci. 2016,113(19):5435-40).

IDO and/or TDO inhibitors may in addition be useful for the treatment of Multiple sclerosis (MS). MS is an autoimmune disease characterized by inflammatory lesions in the white matter of the nervous system, consisting of a specific immune response to the myelin sheet resulting in inflammation and axonal loss (Trapp et al.; Curr Opin Neurol. 1999, 12: 295-302; Owens; Curr Opin Neurol. 2003, 16:259—265). Accumulation of neurotoxic KYN metabolites caused by the activation of the immune system is implicated in the pathogenesis of MS. QUIN was found to be selectively elevated in the spinal cords of rats with EAE, an autoimmune animal model of MS (Flanagan et al.; J Neurochem. 1995, 64: 1192-6). The origin of the increased QUIN in EAE was suggested to be the macrophages. QUIN is an initiator of lipid peroxidation and high local levels of QUIN near myelin may contribute to the demyelination in EAE and possibly MS. Interferon-β Ib (IFN-pib) induces KYN pathway metabolism in macrophages at concentrations comparable to those found in the sera of IFN-β treated patients, which may be a limiting factor in its efficacy in the treatment of MS (Guillemin et al.; J Interferon Cytokine Res. 2001, 21:1097-1101). After IFN-β administration, increased KYN levels and KYN/TRP ratio were found in the plasma of MS patients receiving IFN- β injection compared to healthy subjects indicating an induction of IDO by IFN-β (Amirkhani et al.; Eur. J. Neurol. 2005,12, 625-31). IFN-pib, leads to production of QUIN at concentrations sufficient to disturb the ability of neuronal dendrites to integrate incoming signals and kill oligodendrocytes (Cammer et al.; Brain Res. 2001, 896: 157-160). In IFN-pib-treated patients concomitant blockade of the KYN pathway with an IDO/TDO inhibitor may improve its efficacy of IFN-pib.

A homolog of IDO (IDO2) has been identified that shares 44% amino acid homology with IDO, but its function is largely distinct from that of IDO (Ball et al., Gene 2007, 396(1):203-13; Yuasa et al., J Mol Evol 2007, 65(6):705-14. An IDO inhibitor may modulate IDO1 and/or IDO2. Current evidence reveals IDO2 to be an immunomodulatory enzyme that acts in B cells to modulate autoimmune disease. Although its enzymatic function is poorly characterized, the mechanism of immune modulation by IDO2 is distinct from its better-studied homolog, IDO1. IDO2 acts as a pro-inflammatory mediator in multiple models of autoimmune inflammatory disorders, including rheumatoid arthritis, Contact hypersensitivity, and Systemic lupus erythematosus (Merlo and Mandik-Nayak, Clinical Medicine Insights: Pathology 2016, 9(S1): 21-28). Because IDO2 is acting to promote inflammation, it may be a candidate for therapeutic targeting for treatment of these diseases, particularly in a co-therapeutic setting.

Most TRP is processed through the KYN pathway. A small proportion of TRP is processed to 5-HT and hence to melatonin, both of which are also substrates for IDO. It has long been known that amongst other effects acute TRP depletion can trigger a depressive episode and produces a profound change in mood even in healthy individuals. These observations link well with the clinical benefits of serotonergic drugs both to enhance mood and stimulate neurogenesis.

In recent years, the general view of the pathophysiology of schizophrenia (i.e., disturbances in dopamine [DA] transmission) has been expanded to also involve a glutamatergic dysfunction of the brain. Thus, clinical observations show that systemic administration of N-methyl-D-aspartate (NMDA) receptor antagonists (e.g., phencyclidine [PCP] and ketamine) evokes schizophrenia-like symptoms in healthy individuals and provokes symptoms in patients with schizophrenia (Holtze et al.; J Psychiatry Neurosci. 2012,37(1):53-7). Furthermore, the glutamate deficiency theory has gained some support from genetic findings. A hypoglutamatergic state of the brain can also be achieved by elevation of the endogenous NMDA receptor antagonist KYNA. Indeed, altered brain level of KYNA and of KYNA-producing enzymes are found in the post-mortem brains of schizophrenic patients (Barry et al.; J Psychopharmacol. 2009,23(3):287-94). In particular, elevated KYN and KYNA levels are found in the frontal cortex and an upregulation of the first step of the KYN pathway is observed in the anterior cingulate cortex of individuals with schizophrenia (Miller et al.; Brain Res. 2006,1073-1074:25-37). However, other researchers have found that KYNA is decreased and 3-HAA is increased in schizophrenia (Miller et al.; Neurochem Int. 2008,52(6):1297-303). The mechanism of elevation of KYN metabolites in schizophrenia has not been fully elucidated. Mechanisms include KMO polymorphisms and TDO upregulation (Miller et al.; Neurobiol Dis. 2004, 15(3):618-29). Therefore, IDO and/or TDO inhibitors may be useful for the treatment of schizophrenia.

IDO and/or TDO inhibitors may in addition be useful for the treatment of pain and depression. Pain and depression are frequently comorbid disorders. It has been shown that IDO plays a key role in this comorbidity. Recent studies have shown that IDO activity is linked to (a) decreased serotonin content and depression (Dantzer et al.; Nat Rev Neurosci. 2008,9(1):46-56; Sullivan et al; Pain. 1992,50(1):5-13) and (b) increased KYN content and neuroplastic changes through the effect of its derivatives such as quinolinic acid on glutamate receptors (Heyes et al.; Brain. 1992,115(Pt5):1249-73).

In rats chronic pain induced depressive behaviour and IDO upregulation in the bilateral hippocampus. Upregulation of IDO resulted in the increased KYN/TRP ratio and decreased serotonin/TRP ratio in the bilateral hippocampus. Furthermore, IDO gene knockout or pharmacological inhibition of hippocampal IDO activity attenuated both nociceptive and depressive behaviour (Kim et al.; J Clin Invest.2012, 122(8):2940-54).

Since proinflammatory cytokines have been implicated in the pathophysiology of both pain and depression, the regulation of brain IDO by proinflammatory cytokines serves as a critical mechanistic link in the comorbid relationship between pain and depression through the regulation of TRP metabolism.

Moreover, the KYN pathway has been associated with traumatic brain injury (TBI). TBI has been shown to induce a striking activation of the KYN pathway with sustained increase of QUIN (Yan et al.; Journal of Neuroinflammation 2015, 12 (110): 1-17). The exceeding production of QUIN together with increased IDO1 activation and mRNA expression in brain-injured areas suggests that TBI selectively induces a robust stimulation of the neurotoxic branch of the KYN pathway. QUIN's detrimental roles are supported by its association to adverse outcome potentially becoming an early prognostic factor post-TBI. Hence, IDO and/or TDO inhibitors may in addition be useful for the prevention/treatment of TBI.

Infection by bacteria, parasites, or viruses induces a strong IFN-$\gamma$-dependent inflammatory response. IDO can dampen protective host immunity, thus indirectly leading to increased pathogen burdens. For example, in mice infected with murine leukaemia virus (MuLV), IDO was found to be highly expressed, and ablation of IDO enhanced control of viral replication and increased survival (Hoshi et al.; J Immunol. 2010, 185(6):3305-3312). In a model of influenza infection, the immunosuppressive effects of IDO could predispose lungs to secondary bacterial infection (van der Sluijs et al.; J Infect Dis. 2006, 193(2): 214-22). Hence, IDO activity was increased in community-acquired pneumonia (CAP), and this activity was associated with the severity and outcome of this disease. These results suggest that IDO activity can predict prognosis of CAP (Suzuki et al.; J Infect. 2011 Sep;63(3):215-22).

In Chagas Disease, which is caused by the Trypanosoma cruzi parasite, KYN is increased in patients and correlates with disease severity (Maranon et al.; Parasite Immunol.2013,35 (5-6):180-7). Infection with chlamydia trachomatis induces the production of a large amount of IFN-$\gamma$ which in turn causes IDO induction. A study has shown that IDO mediated depletion of the TRP pool causes Chlamydia to convert into a persistent form which is highly adapted to survive in hostile environments (Barth and Raghuraman; Crit Rev Microbiol. 2014,40(4):360-8). In patients with chronic cutaneous leishmaniasis, high levels of IDO mRNA expression has been detected in infectious lesions and was associated with the accumulation of intralesional Treg cells. Leishmania major infection in mice induces IDO expression in local cutaneous lesions and draining lymph nodes. Genetic and pharmacological ablation of IDO resulted in improved control of L. major. Cerebral malaria can be a fatal manifestation of Plasmodium falciparum infection in humans. IDO activity is increased in the mouse brain during cerebral malaria and inhibition of IDO in a mouse model of malaria enhanced the function of anti-malarial T cells and slightly reduce the parasite load (Barth and Raghuraman; Crit Rev Microbiol. 2014,40(4):360-8).

Measuring serum concentrations of KYN and TRP and assessed IDO activity in patients with pulmonary tuberculosis showed significant increases in Kyn concentrations and IDO activity and significant decreases in Trp concentrations compared to control subjects. Interestingly, among the pulmonary tuberculosis patients, nonsurvivors had significantly higher Kyn concentrations and significantly lower Trp concentrations, resulting in a significant increase in IDO activity over that in survivors. Most importantly, multivariate analysis showed that the IDO activity was a significant independent predictor of death in pulmonary tuberculosis (Suzuki et al.; Clin Vaccine Immunol. 2012, 19(3): 436-442).

Therefore, IDO inhibitors could be used to improve the outcomes of patients with a wide variety of infectious diseases and inflammatory conditions. Given the role of TDO in controlling systemic TRP levels, TDO inhibitors could also be used to improve the outcomes of patients with a wide variety of infectious diseases and inflammatory conditions.

Patients infected with HIV have chronically reduced levels of plasma TRP and increased levels of KYN, and increased IDO expression (Murray; Lancet Infect Dis. 2003, 3(10):644-52). In HIV patients the upregulation of IDO acts to suppress immune responses to HIV antigens contributing to the immune evasion of the virus. A characteristic feature during advanced HIV infection is the preferential depletion of Th17 cells from both the gastrointestinal tract and blood. Interestingly, the loss of Th17 cells in HIV infection is accompanied by a concomitant rise in the frequency of induced Treg cells and directly correlated with IDO activity. Treg cells may dampen efficient HIV specific cellular immune responses while the progressive depletion of Th17 cells may increase susceptibility to mucosal infections. Thus, sustained IDO activation may establish a favourable environment for HIV persistence and contribute to the immunodeficiency seen in HIV-infected individuals with progressive disease (Barth and Raghuraman; Crit Rev Microbiol. 2014,40(4):360-8). HIV patients, particularly those with HIV-linked dementia (Kandanearatchi & Brew; FEBS J. 2012, 279(8):1366-74), often have significantly elevated KYN levels in CSF. These levels are directly related to the development of neurocognitive decline (HIV-associated neurocognitive disorder (HAND)) and often the presence of severe psychotic symptoms (Stone & Darlington; Trends Pharmacol Sci. 2013, 34(2):136-43). Therefore, IDO and/or TDO inhibitors may in addition be useful for the treatment of HIV (AIDS including its manifestations such as cachexia, dementia and diarrhea).

As with HIV infection, patients chronically infected with HCV present increased KYN to TRP ratios in blood compared to patients with resolved HCV infections and healthy individuals (Larrea et al.; J Virol. 2007,81(7):3662-6).

Furthermore, it has been suggested that expression of IDO correlated with the pathogenesis of the disease and the high expression of IDO in progressively cirrhotic livers of HCV-infected patients might contribute to the development of hepatocellular carcinoma (Asghar et al.; Exp Ther Med. 2015, 9(3):901-4). Hence, IDO and/or TDO inhibitors may be useful for the treatment of patients chronically infected with HCV.

IDO plays a role in regulating mucosal immunity to the intestinal microbiota. IDO has been shown to regulate commensal induced antibody production in the gut; IDO-deficient mice had elevated baseline levels of immunoglobulin A (IgA) and immunoglobulin G (IgG) in the serum and increased IgA in intestinal secretions. Due to elevated antibody production, IDO deficient mice were more resistant to intestinal colonization by the gram-negative enteric bacterial pathogen Citrobacter rodentium than WT mice. IDO-deficient mice also displayed enhanced resistance to the colitis caused by infection with C. rodentium (Harrington et al.; Infect Immunol. 2008, 76(7):3045-53).

Therefore, pharmacological targeting of IDO/TDO activity may represent a new approach to manipulating intestinal immunity and controlling the pathology caused by enteric pathogens including colitis (Harrington et al.; Infect Immunol. 2008, 76(7):3045-53).

Recent literature highlights a role for IDO in metabolic disorders (Laurans et al.; Nature Medicine https://doi.org/10.1038/s41591-018-0060-4 (2018); Natividad et al.; Cell Metabolism 2018, 28: 1-13). It was found that Ido1 knockout mice that were fed a high-fat diet gained less weight, had a lower fat mass, better glucose and insulin tolerance and less macrophage infiltration into fat tissue than wild-type mice did. Treatment with an IDO inhibitor, L-1-MT, concurrent with a high-fat diet had a similar effect on insulin and glucose tolerance to that in the knockout. The fact that antibiotic treatment prevented Ido1 knockout mice from gaining weight on a high-fat diet and co-housing of Ido1 knockout and wt mice had metabolic measurements similar to those of Ido1 knockout mice suggested that the microbiota from Ido1 knock-out mice is protective. Consistent with these hypotheses, Ido-1 knock-out mice had different intestinal microbiota composition.TRP can be metabolized either by IDO to produce KYN or by the gut microbiota to produce indole derivatives such as indole-3-acetic acid, a ligand for the AhR. Depletion of IDO increased the lecels of indole-3-acetic acid in the faeces. Indole-3-acetic acid induced activation of the AhR in intestinal immune cells increases the production of IL-17 and IL-22. Reduced levels of IL-22 were accompanied with dysfunction of the gut barrier. These data support the importance of IDO in controlling KYN and indole-3-acetic acid-activating AhR balance. Consistent with the observations in mice, people with obesity or type 2 diabetes had higher levels of KYN in their plasma and faeces and lower levels of indole-3-acetic acid in their faeces (Laurans et al.; Nature Medicine https://doi.org/10.1038/s41591-018-0060-4 (2018). Increased KYN levels were also found in fecal samples of individuals with metabolic syndrome compared to healthy subjects in another study (Natividad et al.; Cell Metabolism 2018, 28: 1-13). Thus far it is unknown whether the alterations of AhR agonist production by the gut microbiota is the primary event in metabolic syndrome pathogenesis. However, the therapeutic effects of the correction of this defect by applying an AhR agonist shows its involvement in the pathogenesis ((Natividad et al.; Cell Metabolism 2018, 28: 1-13). Hence IDO inhibitors through altering the balance of TRP derived AhR agonist balance could be useful in regulating metabolic disorders such as obesity, type 2 diabetes and/or fatty acid liver disease.

A cataract is a clouding of the lens inside the eye that leads to a decrease in vision. Recent studies suggest that KYNs might chemically alter protein structure in the human lens leading to cataract formation. In the human lens IDO activity is present mainly in the anterior epithelium (Takikawa et al.; Adv Exp Med Biol. 1999, 467: 241-5). Several KYNs, such as KYN, 3-HK, and 3-hydroxykynurenine glucoside (3-HK-G) have been detected in the lens; where they were thought to protect the retina by absorbing UV light and therefore are commonly referred to as UV filters. However, several recent studies show that KYNs are prone to deamination and oxidation to form $\alpha,\beta$-unsaturated ketones that chemically react and modify lens proteins (Taylor et al.; Exp Eye Res. 2002; 75(2): 165-75). KYN mediated modification could contribute to the lens protein modifications during aging and cataractogenesis. They may also reduce the chaperone function of a-crystallin, which is necessary for maintaining lens transparency.

Transgenic mouse lines that overexpress human IDO in the lens developed bilateral cataracts within 3 months of birth. It was demonstrated that IDO-mediated production of KYNs results in defects in fibre cell differentiation and their apoptosis (Mailankot et al.; Lab Invest. 2009; 89(5):498-512). Therefore, inhibition of IDO/TDO may slow the progression of cataract formation.

Endometriosis, the presence of endometrium outside the uterine cavity, is a common gynaecological disorder, causing abdominal pain, dyspareunia and infertility. IDO expression was found to be higher in eutopic endometrium from women with endometriosis by microarray analysis (Burney et al.; Endocrinology. 2007;148(8): 3814-26; Aghajanova et al.; Reprod Sci. 2011, 18(3):229-251). Furthermore, IDO was shown to enhance the survival and invasiveness of endometrial stromal cells (Mei et al.; Int J Clin Exp Pathol. 2013; 6(3): 431-44). Therefore, an IDO/TDO inhibitor may be used as a treatment for endometriosis.

The process of implantation of an embryo requires mechanisms that prevent allograft rejection; and tolerance to the fetal allograft represents an important mechanism for maintaining a pregnancy. Cells expressing IDO in the foeto-maternal interface protect the allogeneic foetus from lethal rejection by maternal immune responses. Inhibition of IDO by exposure of pregnant mice to 1-methyl-tryptophan induced a T cell-mediated rejection of allogeneic concepti, whereas

syngeneic concepti were not affected; this suggests that IDO expression at the foetal-maternal interface is necessary to prevent rejection of the foetal allograft (Munn et al.; Science 1998, 281(5380): 1191-3:). Accumulating evidence indicates that IDO production and normal function at the foetal-maternal interface may play a prominent role in pregnancy tolerance (Dürr and Kindler; J Leukoc Biol. 2013, 93(5): 681-700). Therefore, an IDO/TDO inhibitor could be used as a contraceptive or abortive agent.

In experimental chronic renal failure, activation of IDO leads to increased blood levels of KYNs (Tankiewicz et al.; Adv Exp Med Biol. 2003,527:409-14), and in uremic patients KYN-modified proteins are present in urine (Sala et al.; J Biol Chem. 2004,279(49):51033-41). Further, renal IDO expression may be deleterious during inflammation, because it enhances tubular cell injury.

In coronary heart disease, inflammation and immune activation are associated with increased blood levels of KYN (Wirleitner et al.; Eur J Clin Invest. 2003,33(7):550-4) possibly via interferon-y-mediated activation of IDO. Cardiac surgery involving extra-corporeal circulation can lead to cognitive dysfunction. As such surgery is associated with signs of inflammation and pro-inflammatory mediators activate tryptophan oxidation to neuroactive kynurenines which modulate NMDA receptor function and oxidative stress. Post anaesthesia cognitive dysfunction has often been correlated with these sequelae. Recently these deficits have been shown to be correlated with changes in KYN pathway markers, but not cytokines, following cardiac surgery and in recovering stroke patients (Forrest et al.; J. Neurochem. 201,119(1):136-52).

In general, TRP catabolism has been reported to be altered in stroke. The activation of the KYN pathway in the acute phase of stroke may participate in the ischemic damage by direct mechanisms which include excitotoxicity and oxidative stress among others, since inhibition of the KYN pathway decreases brain injury in animal models of stroke. Probably, an interplay between the immune system and the KYN pathway could exist after stroke, but also different inflammatory-independent mechanisms could mediate a role in the regulation of this pathway, modulating the rate-limiting enzymes of TRP catabolism. Interestingly, the KYN pathway after cerebral ischemia could also play a role during the chronic phase of this pathology in which stroke survivors present a high incidence of disabilities such as dementia and depression or even being a risk factor for stroke outcome and mortality. All together the KYN and TRP catabolism could have a significant role in after cerebral ischemia and IDO/TDO inhibitors may provide new pharmacological tools in both acute and chronic phases of stroke (Cuartero et al.; Curr Pharm Des. 2016; 22(8): 1060-1073).

The present invention provides novel compounds of Formula (I) which inhibit the activity of IDO and/or TDO enzymes.

1) A first embodiment of the present invention relates to compounds of Formula (I)

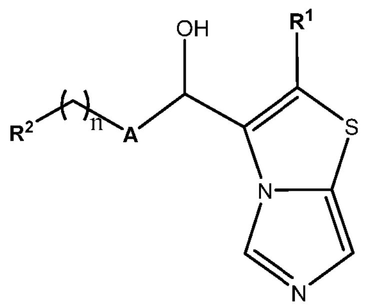

Formula (I)

wherein

A represents phenylene or 5- to 6-membered heteroarylene (especially 5-membered heteroarylene), wherein said phenylene or 5- to 6-membered heteroarylene independently are unsubstituted, mono- or di-substituted (especially unsubstituted or mono- substituted), wherein the substituents are independently selected from $C_{1-4}$-alkyl (especially methyl, ethyl, iso-propyl or tert-butyl), halogen (especially fluorine or iodine), or $C_{3-5}$-cycloalkyl (especially cyclopropyl);

**n** represents 1 or 0 (especially **n** represents 0) (i.e. when n=0, $\mathbf{R^2}$ is directly attached to **A;** or when n=1, $\mathbf{R^2}$ is directly attached to -$CH_2$-**A**);

$\mathbf{R^1}$ represents:

- $C_{1-4}$-alkyl (especially methyl or ethyl);

- -C($R^A$)=C($R^B$)($R^C$), wherein $R^A$, $R^B$ and $R^C$ are independently selected from hydrogen, $C_{1-4}$-alkyl (especially methyl or propyl) and $C_{3-5}$-cycloalkyl (especially cyclopropyl); or wherein $R^A$ and $R^B$ together with the carbon atoms to which they are attached form a $C_{4-6}$-cycloalkenyl (especially cyclopent-1-en-1-yl) and $R^C$ represents hydrogen;
- $C_{1-3}$-fluoroalkyl (especially trifluoromethyl or 1,1-difluoroethyl);
- phenyl, which is unsubstituted or mono, di- or tri-substituted (especially unsubstituted, mono-, or di-substituted), wherein the substituents are independently selected from $C_{1-3}$-alkyl (especially methyl) and halogen (especially fluorine);
- 5-membered heteroaryl (especially containing from one to three ring nitrogen atoms), which is unsubstituted or mono-substituted with $C_{1-4}$-alkyl (especially methyl); or
- $C_{3-6}$-cycloalkyl (especially cyclopropyl or cyclopentyl), which is unsubstituted or mono, di- or tri-substituted (especially unsubstituted or mono-substituted), wherein the substituents are independently selected from $C_{1-3}$-alkyl (especially methyl) and halogen (especially fluorine);

**$R^2$** represents:

- phenyl or 6-membered heteroaryl (especially pyridinyl, pyrimidinyl or pyridazinyl), which are independently unsubstituted, or mono-, di- or tri-substituted, wherein the substituents are independently selected from:

  - ➢ $C_{1-4}$-alkyl (especially methyl or ethyl), cyano, nitro, halogen (especially chlorine, fluorine or bromine), hydroxy, hydroxy-$C_{1-4}$-alkyl (especially hydroxymethyl), $C_{1-3}$-alkoxy-$C_{1-4}$-alkyl (especially methoxymethyl), $C_{1-3}$-fluoroalkyl (especially trifluoromethyl), $C_{1-3}$-fluoroalkoxy (especially trifluoromethoxy or 2,2,2-trifluoroethoxy), $C_{1-4}$-alkoxy (especially methoxy, ethoxy, iso-propoxy), $C_{3-5}$-cycloalkoxy (especially cyclobutoxy), hydroxymethyl-cyclopropyl (especially 1-(hydroxymethyl)-cycloprop-1-yl), $C_{1-3}$-alkyl-carbonyl (especially acetyl), $C_{1-4}$-alkoxy-carbonyl (especially methoxy-carbonyl), amino, morpholin-4-yl or morpholin-4-yl-methyl;

  - ➢ **-N$R^{N1}R^{N2}$**, wherein $R^{N1}$ represents hydrogen and $R^{N2}$ represents -(C=O)-$R^{CO}$, wherein $R^{CO}$ represents $C_{1-4}$-alkyl (especially methyl, iso-propyl or tert-butyl), $C_{1-4}$-alkoxy (especially methoxy or tert-butoxy), amino, $C_{1-4}$-alkylamino (especially ethylamino or n-butylamino), phenyl or morpholin-4-yl;

  - ➢ -(C=O)-N$R^{N3}R^{N4}$, wherein $R^{N3}$ represents hydrogen or $C_{1-3}$-alkyl (especially methyl), and $R^{N4}$ represents hydrogen, $C_{1-3}$-alkyl (especially methyl or ethyl), $C_{3-5}$-cycloalkyl (especially cyclopropyl), hydroxy-$C_{2-4}$-alkyl (especially 2-hydroxyethyl or 3-hydroxypropyl), $C_{1-3}$-alkoxy-$C_{2-4}$-alkyl (especially 2-methoxyethyl or 3-methoxypropyl), cyano-$C_{1-3}$-alkyl (especially 2-cyanoethyl), $C_{3-4}$-alkenyl (especially prop-1-en-3-yl), furanyl-$C_{1-3}$-alkyl (especially furan-2-yl-methyl), 2-di-$C_{1-3}$-alkylamino-$C_{2-3}$-alkyl (especially dimethylaminoethyl), or allyl; or wherein $R^{N3}$ and $R^{N4}$ together with the nitrogen atom to which they are attached form a morpholine ring;

  - ➢ -(NH)p-$SO_2$-N$R^{S1}R^{S2}$, wherein $R^{S1}$ and $R^{S2}$ independently represent hydrogen or $C_{1-4}$-alkyl (especially methyl or ethyl); or wherein $R^{S1}$ and $R^{S2}$ together with the nitrogen atom to which they are attached form a 4- to 6-membered heterocycloalkyl selected from azetidinyl, pyrrolidinyl and piperidinyl (especially pyrrolidinyl); and **p** represents 1 or 0 (i.e. when p=0, - $(NH)_p$-$SO_2$-N$R^{S1}R^{S2}$ represents -$SO_2$-N$R^{S1}R^{S2}$); and

  - ➢ **-O$R^6$**, wherein $R^6$ represents $C_{1-3}$-alkoxy-$C_{2-4}$-alkyl (especially 2-methoxyethyl), hydroxy-$C_{2-5}$-alkyl (especially 3-hydroxypropyl, 2-hydroxy-2-methylpropyl, 2-hydroxyethyl, 3-hydroxy-2,2-dimethylpropyl or 3-hydroxy-3-methylbutyl), di-hydroxy-$C_{2-5}$-alkyl (especially 2,3-dihydroxypropyl), sulfamoyl-$C_{2-4}$-alkyl (especially 3-(sulfamoyl)-propyl), pyridinyl-$C_{1-3}$-alkyl (especially pyridine-2-yl-methyl), $C_{1-3}$-alkylsulfonyl-$C_{2-4}$-alkyl (especially 2-(methylsulfonyl)-ethyl), $C_{3-5}$-cycloalkyl-$C_{1-3}$-alkyl (especially cyclopropyl-methyl), oxetan-3-yl-$C_{1-3}$-alkyl (especially oxetan-3-yl-methyl), ($C_{1-3}$-alkyl-oxetan-3-yl)-$C_{1-3}$-alkyl (especially (3-methyloxetan-3-yl)-methyl), (fluoro-oxetan-3-yl)-$C_{1-3}$-alkyl (especially (3-fluorooxetan-3-yl)-methyl), tetrahydrofuranyl-$C_{1-3}$-alkyl (especially tetrahydrofuran-2-yl-methyl), hydroxy-$C_{3-5}$-cycloalkyl-$C_{1-3}$-alkyl (especially (1-hydroxycyclopropyl)-ethyl), tetrahydropyranyl-$C_{1-3}$-alkyl (especially tetrahydropyran-2-yl-methyl or tetrahydropyran-4-yl-methyl), benzyl, (hydroxy-$C_{2-4}$-alkoxy)-$C_{2-4}$-alkyl (especially 2-(2-hydroxyethoxy)-ethyl), piperidin-1-yl-$C_{1-3}$-alkyl (especially 2-(piperidin-1-yl)-ethyl), (1-methyl-piperidin-3-yl)-$C_{1-3}$-alkyl (especially (1-methyl-piperidin-3-yl)-methyl)), (1,1-dioxidotetrahydro-thiopyran-4-yl)-$C_{1-3}$-alkyl (especially (1,1-dioxidotetrahydro-thiopyran-4-yl)-methyl), $C_{1-3}$-alkoxy-carbonyl-$C_{1-3}$-alkyl (especially methoxy-carbonyl-methyl or ethoxy-carbonyl-methyl), ((hydroxy-$C_{2-3}$-alkoxy)-$C_{2-3}$-alkoxy)-$C_{2-3}$-alkyl (especially 2-(2-(2-hydroxyethoxy)ethoxy)-ethyl), $C_{1-3}$-fluoroalkoxy-$C_{2-3}$-alkyl (especially 2-(2,2,2-trifluoroethoxy)-ethyl) or $C_{1-3}$-alkyl-carbonyl (especially acetyl);

- 5-membered heteroaryl (especially pyrazolyl or triazolyl) which independently is unsubstituted, or mono-, di- or tri-substituted (especially unsubstituted, mono-, or di-substituted), wherein the substituents are independently selected from:

  ➢ $C_{1-4}$-alkyl (especially methyl), cyano, halogen (especially chlorine or fluorine), $C_{1-3}$-fluoroalkyl (especially trifluoromethyl), $C_{1-3}$-fluoroalkoxy (especially trifluoromethoxy or 2,2,2-trifuloroethoxy), $C_{1-4}$-alkoxy (especially methoxy, ethoxy or iso-propoxy), $C_{3-5}$-cycloalkoxy (especially cyclobutoxy), hydroxymethyl-cyclopropyl (especially 1-(hydroxymethyl)-cycloprop-1-yl), morpholin-4-yl, morpholin-4-yl-methyl, $C_{1-3}$-alkyl-carbonyl (especially acetyl), $C_{1-4}$-alkoxy-carbonyl (especially methoxy-carbonyl);

  ➢ **-NR$^{N1}$R$^{N2}$**, wherein **R$^{N1}$** represents hydrogen and **R$^{N2}$** represents -(C=O)-**R**$^{CO}$, wherein **R**$^{CO}$ represents $C_{1-4}$-alkoxy (especially methoxy); or

  ➢ **-OR$^6$**, wherein **R$^6$** represents $C_{3-5}$-cycloalkyl-$C_{1-3}$-alkyl (especially cyclopropyl-methyl), benzyl or $C_{1-3}$-alkoxy-carbonyl-$C_{1-3}$-alkyl (especially methoxy-carbonyl-methyl);

- 9- to 10-membered bicyclic heteroaryl (especially quinolinyl, isoquinolinyl, indazolyl or indolyl), wherein said 9- to 10-membered bicyclic heteroaryl independently is unsubstituted, or mono-, di- or tri-substituted (especially unsubstituted), wherein the substituents are independently selected from:

  ➢ $C_{1-4}$-alkyl, cyano, halogen, $C_{1-3}$-fluoroalkyl, $C_{1-3}$-fluoroalkoxy, $C_{1-4}$-alkoxy, hydroxymethyl-cyclopropyl (especially 1-(hydroxymethyl)-cycloprop-1-yl), morpholin-4-yl, morpholin-4-yl-methyl, $C_{1-3}$-alkyl-carbonyl (especially acetyl), $C_{1-4}$-alkoxy-carbonyl (especially methoxy-carbonyl);

  ➢ N**R$^{N1}$R$^{N2}$**, wherein **R$^{N1}$** represents hydrogen and **R$^{N2}$** represents -(C=O)-**R**$^{CO}$, wherein **R$^{CO}$** represents $C_{1-4}$-alkoxy (especially methoxy); or

  ➢ **-OR$^6$**, wherein **R$^6$** represents $C_{3-5}$-cycloalkyl-$C_{1-3}$-alkyl (especially cyclopropyl-methyl); or

- 2,3-dihydro-benzo[1,4]dioxin-6-yl, 1,3-dihydro-2H-benzoimidazol-2-one-5-yl, 3H-benzooxazol-2-one-6-yl, 3-methyl-1,1-dioxo-2,3-dihydro-1*H*-1l6-benzo[*d*]isothiazol-5-yl, 1-methyl-1H-pyrrolo[2,3-b]pyridin-5-yl or 3,4-dihydro-2H-pyrano[2,3-b]pyridine-6-yl.

[In a sub-embodiment of embodiment 1), when **R$^2$** represents phenyl or 6-membered heteroaryl (especially phenyl), wherein said phenyl or 6-membered heteroaryl are independently mono-, di- or tri-substituted, one substituent (especially hydroxy, **-NR$^{N1}$R$^{N2}$**, -(C=O)-N**R$^{N3}$R$^{N4}$** or **-OR$^6$**) is attached in para-position with regard to the point of attachment to the rest of the molecule]

Definitions provided hereinbelow are intended to apply uniformly to the compounds of Formula (I)/(II), as defined in any one of embodiments 1) to 28), and, *mutatis mutandis,* throughout the description and the claims unless an otherwise expressly set out definition provides a broader or narrower definition. It is well understood that a definition or preferred definition of a term defines and may replace the respective term independently of (and in combination with) any definition or preferred definition of any or all other terms as defined herein. If not explicitly defined otherwise in the respective embodiment or claim, groups defined herein are unsubstituted.

The term "alkyl", used alone or in combination, refers to a saturated straight or branched hydrocarbon chain containing one to six carbon atoms. Examples are methyl, ethyl, n-propyl, iso-propyl, n-butyl, tert-butyl, sec-butyl, iso-butyl, n-pentyl, 1,1-dimethylpropyl, 2,2-dimethylpropyl, 3-methylbutyl, 3-pentyl, 2-pentyl, 1,2-dimethylpropyl and 2-methylbutyl. The term "$C_{x-y}$-alkyl" (x and y each being an integer), used alone or in combination, refers to a saturated straight or branched hydrocarbon chain with x to y carbon atoms. Thus, the term $C_{1-4}$-alkyl, alone or in combination with other groups, means saturated, branched or straight chain groups with one to four carbon atoms. Examples of $C_{1-4}$-alkyl groups are methyl, ethyl, n-propyl, iso-propyl, n-butyl, tert-butyl, sec-butyl and iso-butyl. For the substituents attached to the group **A** examples are methyl, ethyl, iso-propyl and tert-butyl (especially methyl). For avoidance of any doubt the term "substituents attached to the group **A"** used herein does not refer to $\mathbf{R^2}$. For the substituents $\mathbf{R^1}$ and $\mathbf{R^2}$ independently preferred examples of $C_{1-4}$-alkyl groups are methyl and ethyl.

The term "fluoroalkyl", used alone or in combination, refers to an alkyl group as defined before containing one to three carbon atoms in which one or more (and possibly all) hydrogen atoms have been replaced with fluorine. The term "$C_{x-y}$-fluoroalkyl" (x and y each being an integer) refers to a fluoroalkyl group as defined before containing x to y carbon atoms. For example, a $C_{1-3}$-fluoroalkyl group contains from one to three carbon atoms in which one to seven hydrogen atoms have been replaced with fluorine. Representative examples of fluoroalkyl groups include trifluoromethyl, 2-fluoroethyl, 2,2-difluoroethyl and 2,2,2-trifluoroethyl. Preferred are $C_1$-fluoroalkyl groups such as trifluoromethyl, difluoromethyl, fluoromethyl (notably trifluoromethyl). For the substituent $\mathbf{R^1}$ preferred examples of $C_{1-3}$-fluoroalkyl are trifluoromethyl and 1,1-difluoroethyl.

The term "cycloalkyl", used alone or in combination, refers to a saturated monocyclic hydrocarbon ring containing three to six carbon atoms. The term "$C_{x-y}$-cycloalkyl" (x and y each being an integer), refers to a saturated monocyclic hydrocarbon ring containing x to y carbon atoms. For example, a $C_{3-6}$-cycloalkyl group contains from three to six carbon atoms. Examples of $C_{3-6}$-cycloalkyl group are cyclopropyl, cyclobutyl, cyclopentyl and cyclohexyl; especially cyclopropyl, cyclobutyl and cyclopentyl. Examples of $C_{3-5}$-cycloalkyl group are cyclopropyl, cyclobutyl, and cyclopentyl; especially cyclopropyl and cyclobutyl. Examples of $C_{3-5}$-cycloalkyl groups, as used for the substituent $\mathbf{R^1}$**,** which are unsubstituted or mono, di- or tri-substituted, wherein the substituents are independently selected from $C_{1-3}$-alkyl and halogen, are $C_{3-5}$-cycloalkyl groups which are unsubstituted or mono-substituted, wherein the substituents are independently selected from methyl or fluorine. Particular examples are cyclopropyl, cyclobutyl, 1-methyl-cycloprop-1-yl, 1-fluoro-cycloprop-1-yl or 2-fluoro-cycloprop-1-yl; notably cyclopropyl. For the substituents $\mathbf{R^A}$, $\mathbf{R^B}$, $\mathbf{R^C}$, $\mathbf{R^{N4}}$ and $\mathbf{R^6}$ a preferred example of $C_{3-5}$-cycloalkyl is cyclopropyl. For the substituents of **A,** a preferred example of $C_{3-5}$-cycloalkyl is cyclopropyl. All of the above groups are unsubstituted or substituted as explicitly defined.

The term "4- to 6-membered heterocycloalkyl", used alone or in combination, refers to a saturated monocyclic ring containing three to five ring carbon atoms and one ring heteroatom (especially nitrogen). Representative examples of 4- to 6-memebered heterocycloalkyl comprising one nitrogen atom are azetidinyl, pyrrolidinyl and piperidinyl; especially pyrrolidinyl.

The term "cycloalkenyl", used alone or in combination, refers to an unsaturated monocyclic hydrocarbon ring containing three to six carbon atoms, further containing one double carbon-carbon bond. The term "$C_{x-y}$-cycloalkenyl" (x and y each being an integer), refers to an unsaturated monocyclic hydrocarbon ring containing x to y carbon atoms, further containing one double carbon-carbon bond. For example, a $C_{4-6}$-cycloalkenyl contains from four to six carbon atoms and one carbon-carbon double bond. Examples of a $C_{4-6}$-cycloalkenyl as used for the group "-C($\mathbf{R^A}$)=C($\mathbf{R^B}$)($\mathbf{R^C}$)" are cyclobut-1-en-1-yl, cyclopent-1-en-1-yl and cyclohex-1-en-1-yl; especially cyclopent-1-en-1-yl.

The term "alkoxy", used alone or in combination, refers to an alkyl-O- group wherein the alkyl group is as defined before. The term "$C_{x-y}$-alkoxy" (x and y each being an integer) refers to an alkoxy group as defined before containing x to y carbon atoms. For example, the term "$C_{x-y}$-alkoxy" (x and y each being an integer), used alone or in combination, refers to an alkyl-O- group wherein the alkyl group refers to a straight or branched hydrocarbon chain with x to y carbon atoms. For example, a $C_{1-4}$-alkoxy refers to methoxy, ethoxy, n-propoxy, iso-propoxy, n-butoxy, iso-butoxy, sec-butoxy and tert-butoxy; especially methoxy. For substituents of the group $\mathbf{R^2}$, preferred are methoxy, ethoxy and iso-propoxy.

The term "fluoroalkoxy", used alone or in combination, refers to an alkoxy group as defined before containing one to three carbon atoms in which one or more (and possibly all) hydrogen atoms have been replaced with fluorine. The term "$C_{x-y}$-fluoroalkoxy" (x and y each being an integer) refers to a fluoroalkoxy group as defined before containing x to y carbon atoms. For example, a $C_{1-3}$-fluoroalkoxy group contains from one to three carbon atoms in which one to seven hydrogen atoms have been replaced with fluorine. Representative examples of fluoroalkoxy groups include trifluoromethoxy, difluoromethoxy, 2-fluoroethoxy, 2,2-difluoroethoxy and 2,2,2-trifluoroethoxy. Preferred are $C_1$-fluoroalkoxy groups such as trifluoromethoxy and difluoromethoxy, as well as the $C_2$-fluoroalkoxy group 2,2,2-trifluoroethoxy. For substituents of the group $\mathbf{R^2}$, preferred is trifluoromethoxy and 2,2,2-trifluoroethoxy.

The term "$C_{1-3}$-fluoroalkoxy-$C_{2-3}$alkyl" refers to an alkyl group as defined before, wherein one hydrogen atom has been replaced by a $C_{1-3}$-fluoroalkoxy group as defined before. Representative examples of $C_{1-3}$-fluoroalkoxy-$C_{2-3}$-alkyl are 2-(2,2,2-trifluoroethoxy)-ethyl, 3-(2,2,2-trifluoroethoxy)-propyl and 2-(trifluoromethoxy)-ethyl; especially 2-(2,2,2-trifluoroethoxy)-ethyl.

The term "$C_{3-5}$-cycloalkoxy", used alone or in combination, refers to a $C_{3-5}$-cycloalkyl-O- group wherein the $C_{3-5}$-cylcoalkyl group is as defined before. Examples of $C_{3-5}$-cycloalkoxy groups are cyclopropoxy, cyclobutoxy and cyclopentoxy; especially cyclobutoxy.

The term "$C_{1-3}$-alkyl-carbonyl" refers to a $C_{1-3}$-alkyl-(C=O)- group. Examples of $C_{1-3}$-alkyl-carbonyl groups are the groups methyl-carbonyl, ethyl-carbonyl, propyl-carbonyl and isopropyl-carbonyl; especially methyl-carbonyl (i.e. acetyl).

The term "$C_{1-4}$-alkoxy-carbonyl" refers to a $C_{1-4}$-alkoxy-(C=O)- group. Examples of $C_{1-4}$-alkoxy-carbonyl groups are methoxy-carbonyl, ethoxy-carbonyl, propoxy-carbonyl, iso-propoxy-carbonyl, n-butoxy-carbonyl, iso-butoxy-carbonyl, sec-butoxy-carbonyl and tert-butoxy-carbonyl; especially methoxy-carbonyl. The term "$C_{1-3}$-alkoxycarbonyl" refers to a $C_{1-3}$-alkoxy group as defined above, wherein one hydrogen atom is substituted by a carbonyl group. Examples of $C_{1-3}$-alkoxy-carbonyl groups are methoxy-carbonyl, ethoxy-carbonyl, propoxy-carbonyl and iso-propoxy-carbonyl.

The term "$C_{1-3}$-alkoxy-carbonyl-$C_{1-3}$-alkyl" refers to a $C_{1-3}$-alkoxy-(C=O)-$C_{1-3}$-alkyl- group. Representative examples include methoxy-carbonyl-methyl, ethoxy-carbonyl-methyl, propoxy-carbonyl-methyl, isopropoxycarbonyl-methyl methoxy-carbonyl-ethyl, ethoxy-carbonyl-ethyl, propoxy-carbonyl-ethyl, isopropoxy-carbonyl-ethyl, methoxy-carbonyl-propyl, ethoxy-carbonyl-propyl, propoxy-carbonyl-propyl and isopropoxy-carbonyl-propyl; especially methoxy-carbonyl-methyl.

The term "halogen" means fluorine, chlorine, bromine or iodine; especially fluorine, chlorine or bromine. For halogen substituents of a cycloalkyl group fluorine is preferred. For halogen substituents of the groups phenyl, 5- to 6-membered heteroaryl, and 9- to 10-membered bicyclic heteroaryl independently preferred are fluorine and chlorine. Halogen substituents of **A** are fluorine, chlorine, bromine and iodine; preferred are fluorine and chlorine. When $\mathbf{R^2}$ represents phenyl, halogen substituents of $\mathbf{R^2}$ are chlorine, fluorine, bromine and iodine; preferably chlorine.

The term "cyano" refers to the group -CN. The term "nitro" refers to the group $-NO_2$. The term "allyl" refers to the group $-CH_2-CH=CH_2$. The term "vinyl" refers to the group $-CH=CH_2$.

The term "cyano-$C_{1-3}$-alkyl" refers to a $C_{1-3}$-alkyl group as defined before, wherein one of the hydrogen atoms has been replaced by the group -CN. Representative examples of cyano-$C_{1-3}$-alkyl groups include cyanomethyl, 1-cyanoethyl, 2-cyanoethyl, 1-cyanoethyl 1-cyanopropyl, 2-cyanopropyl, 3-cyanopropyl, 2-cyano-1-methylethyl and 1-cyano-1,1-dimethylmethyl; preferred is 2-cyanoethyl.

The term "$C_{x-y}$-alkenyl" (x and y each being an integer), used alone or in combination, refers to a monovalent unsaturated straight or branched hydrocarbon chain having x to y carbon atoms and comprising one carbon-carbon double bond. Thus, the term $C_{3-4}$-alkenyl, alone or in combination with other groups, means an unsaturated, branched or straight, monovalent group comprising one carbon-carbon double bond, having three to four carbon atoms. Representative examples of such groups are $CH_2=CH-CH_2-$, $CH_3-CH=CH-CH_2-$, $CH_2=$ $CH-CH_2-CH_2-$, $CH_2=CH-CH(CH_3)-$, $CH_2=C(CH_3)-CH_2-$; especially $CH_2=CH-CH_2-$.

The term "hydroxyalkyl", used alone or in combination, refers to an alkyl group as defined before, wherein one hydrogen atom has been replaced by a hydroxy group. Representative examples of hydroxyalkyl groups include 2-hydroxyethyl, 2-hydroxypropyl, 3-hydroxypropyl, 4-hydroxybutyl, 2-hydroxybutyl, 3-hydroxybutyl, 3-hydroxy-1-methylpropyl, 3-hydroxy-2-methylpropyl, 2-hydroxy-1-methylpropyl 2-hydroxy-2-methylpropyl, 3-hydroxy-1,1-dimethylpropyl, 3-hydroxy-2,2-dimethylpropyl, 3-hydroxy-1,2-dimethylpropyl, 3-hydroxy-1-ethylpropyl, 1-hydroxymethyl-butyl, 2-hydroxypentyl, 3-hydroxypentyl, 4-hydroxypentyl, 5-hydroxypentyl, 2-hydroxy-3-methylbutyl and 3-hydroxy-3-methylbutyl. The term "hydroxy-$C_{x-y}$-alkyl" (x and y each being an integer), used alone or in combination, refers to a hydroxyalkyl group as defined before wherein the alkyl group contains x to y carbon atoms. A hydroxy-$C_{2-5}$-alkyl group is a hydroxyalkyl group as defined before which contains from two to five carbon atoms, especially 3-hydroxypropyl, 2-hydroxy-2-methylpropyl, 2-hydroxyethyl, 3-hydroxy-2,2-dimethylpropyl or 3-hydroxy-3-methylbutyl. A hydroxy-$C_{1-4}$-alkyl group is a hydroxyalkyl group as defined before containing from one to four carbon atoms. Representative examples are hydroxymethyl, 1-hydroxyethyl, 2-hydroxyethyl, 3-hydroxypropyl, 2-hydroxy-2-methylpropyl, 3-hydroxy-2,2-dimethylpropyl or 3-hydroxy-3-methylbutyl; preferred is hydroxymethyl. A hydroxy-$C_{2-4}$-alkyl group is especially 3-hydroxypropyl or 2-hydroxyethyl.

The term "di-hydroxyalkyl", used alone or in combination, refers to an alkyl group as defined before, wherein two hydrogen atoms have been replaced each by one hydroxy group. The term "di-hydroxy-$C_{x-y}$-alkyl", used alone or in combination, refers to a di-hydroxyalkyl group as defined before, wherein the alkyl group contains from x to y carbon atoms. Representative examples of di-hydroxy-$C_{2-5}$-alkyl are 2,3-dihydroxypropyl, 2,4-dihydroxybutyl and 3,5-dihydroxypently; preferred is 2,3-dihydroxypropyl.

The term "alkylamino", used alone or in combination, refers to an amino group, wherein one hydrogen atom is replaced by an alkyl group as defined before. Examples of $C_{1-4}$-alkylamino are methylamino, ethylamino, n-propylamino, iso-propylamino, n-butylamino, tert-butylamino, sec-butylamino and iso-butylamino; especially ethylamino and n-butylamino. Examples of $C_{1-3}$-alkylamino groups are methylamino, ethylamino, n-proplyamino and iso-propylamino, especially ethylamino.

The term "di-alkylamino", used alone or in combination, refers to an amino group, wherein two hydrogen atoms

have been independently replaced by an alkyl group as defined before. Examples of di-$C_{1-3}$-alkylamino groups are dimethylamino, diethylamino, diproplyamino, diisopropylamino, methylethylamino, methylpropylamino, methylisopropylamino, ethylpropylamino and ethylisopropylamino.

The term "di-$C_{1-3}$-alkylamino-$C_{2-3}$-alkyl" refers to an alkyl group as defined before, wherein one hydrogen atoms has been replaced by a di-alkylamino group as defined above. Representative examples include dimethylaminomethyl, diethylaminomethyl, methylethylaminomethyl, 2-dimethylaminoethyl, 2-diethylaminoethyl; especially 2-dimethylaminoethyl.

The term "$C_{1-3}$-alkoxy-$C_{1-4}$-alkyl" refers to an alkyl group as defined before, wherein one of the hydrogen atoms has been replaced by a $C_{1-3}$-alkoxy group as defined before. Representative examples of $C_{1-3}$-alkoxy-$C_{1-4}$-alkyl include methoxymethyl, ethoxymethyl, propoxyethyl, ethoxyethyl, ethoxypropyl and propoxypropyl. A preferred example of $C_{1-3}$-alkoxy-$C_{1-4}$-alkyl is methoxymethyl. Representative examples of $C_{1-3}$-alkoxy-$C_{2-3}$-alkyl groups include 2-methoxyethyl, 2-ethoxyethyl, 3-methoxypropyl and 2-propoxyethyl. A preferred example for the substituent $\mathbf{R^6}$ is 2-methoxyethyl. Preferred examples for the substituent $\mathbf{R^{CO}}$ are 2-methoxyethyl and 3-methoxypropyl.

The term "(hydroxy-$C_{2-4}$-alkoxy)-$C_{2-4}$-alkyl refers to an alkyl group as defined before, wherein one hydrogen atoms is replaced by an alkoxy group as defined above, wherein said alkoxy group bears a hydroxyl group. Representative examples of (hydroxy-$C_{2-4}$-alkoxy)-$C_{2-4}$-alkyl groups include 2-(2-hydroxyethoxy)-ethyl, 3-(2-hydroxyethoxy)-propyl, 2-(2-hydroxyethoxy)-butyl or 2-(1-hydroxypropoxy)-propyl; especially 2-(2-hydroxyethoxy)-ethyl.

The term " ((hydroxy-$C_{2-3}$-alkoxy)-$C_{2-3}$-alkoxy)-$C_{2-3}$-alkyl" refers to an alkyl group as defined before, wherein one hydrogen atom of said alkyl group is replaced by a first alkoxy group as defined above, wherein one hydrogen atom of the first alkoxy group is replaced by a second alkoxy group as defined above, wherein one hydrogen atom of the second alkoxy group is replaced by a hydroxy group. Representative examples of a ((hydroxy-$C_{2-3}$-alkoxy)-$C_{2-3}$-alkoxy)-$C_{2-3}$-alkyl are 2-(2-(2-hydroxyethoxy)ethoxy)-ethyl, 2-(3-(2-hydroxyethoxy)propoxy)-ethyl and 3-(3-(2-hydroxypropoxy)propoxy)-propyl; especially 2-(2-(2-hydroxyethoxy)ethoxy)-ethyl.

The term "$C_{3-5}$-cycloalkyl-$C_{1-3}$-alkyl" refers to an $C_{1-3}$-alkyl group as defined before, wherein one of the hydrogen atoms has been replaced by a $C_{3-5}$-cycloalkyl as defined before. For example, a cyclopropyl-$C_{1-3}$-alkyl group is a $C_{1-3}$-alkyl group, wherein one hydrogen atom is replaced by a cyclopropane ring. A representative example is cyclopropylmethyl.

The term "hydroxy-$C_{3-5}$-cycloalkyl-$C_{1-3}$-alkyl" refers to a $C_{3-5}$-cycloalkyl-$C_{1-3}$-alkyl group as defined before, wherein one of the hydrogen atoms of the $C_{3-5}$-cycloalkyl ring has been replaced by a hydroxy group. Representative examples include hydroxy-cyclopropyl-$C_{1-3}$-alkyl, hydroxy-cyclobutyl-$C_{1-3}$-alkyl and hydroxycyclopentyl-$C_{1-3}$-alkyl, wherein any of the hydrogen atoms of the $C_{3-5}$-cycloalkyl ring may be substituted with a hydroxyl group. A representative example of hydroxy-cyclopropyl-$C_{1-3}$-alkyl is 1-(1-hydroxycyclopropyl)-ethyl.

The term "heteroaryl", used alone or in combination, means a 5- to 10-membered monocyclic or bicyclic aromatic ring containing one to a maximum of four heteroatoms (notably containing one to a maximum of three heteroatoms), each independently selected from oxygen, nitrogen and sulfur. Examples of such heteroaryl groups are 5-membered heteroaryl groups such as furanyl, oxazolyl, isoxazolyl, oxadiazolyl, thiophenyl, thiazolyl, isothiazolyl, thiadiazolyl, pyrrolyl, imidazolyl, pyrazolyl, triazolyl, tetrazolyl; 6-membered heteroaryl groups such as pyridinyl, pyrimidinyl, pyridazinyl, pyrazinyl; and 8- to 10-membered bicyclic heteroaryl groups such as indolyl, isoindolyl, benzofuranyl, isobenzofuranyl, benzothiophenyl, indazolyl, benzimidazolyl, benzoxazolyl, benzisoxazolyl, benzothiazolyl, benzoisothiazolyl, benzotriazolyl, benzoxadiazolyl, benzothiadiazolyl, thienopyridinyl, quinolinyl, isoquinolinyl, naphthyridinyl, cinnolinyl, quinazolinyl, quinoxalinyl, phthalazinyl, pyrrolopyridinyl, pyrazolopyridinyl, pyrazolopyrimidinyl, pyrrolopyrazinyl, imidazopyridinyl, imidazopyridazinyl, and imidazothiazolyl. Likewise, a heteroarylene group is a heteroaryl group as defined before having two points of attachment to the respective rests of the molecule. The above-mentioned heteroaryl / heteroarylene groups are unsubstituted or substituted as explicitly defined.

The term "5-membered heteroaryl", used alone or in combination, means a heteroaryl as defined before having five ring atoms. For the group $\mathbf{R^1}$, the term 5-membered heteroaryl preferably refers to pyrrolyl, imidazolyl, pyrazolyl and triazolyl; especially to pyrazolyl. For the group $\mathbf{R^2}$, the term "5-membered heteroaryl" notably refers to pyrazolyl and triazolyl; in particular pyrazol-4-yl and [1,2,3]triazol-4-yl. All of the above groups are unsubstituted or substituted as explicitly defined.

The term "6-membered heteroaryl", used alone or in combination, means a heteroaryl as defined before having six ring atoms (wherein said 6-membered heteroaryl notably contains one or two ring nitrogen atoms). Examples of 6-membered heteroaryl groups include pyridinyl, pyrimidinyl, pyridazinyl, pyrazinyl. For the group $\mathbf{R^2}$, the term "6-membered heteroaryl" notably refers to pyridinyl, pyridazinyl and pyrimidinyl; in particular pyridin-2-yl, pyridin-3-yl, pyridin-4-yl, pyridazine-4-yl and pyrimidin-5-yl. All of the above groups are unsubstituted or substituted as explicitly defined.

The term "5- to 6-membered heteroarylene" as used for the group **A** refers to a 5- or 6-membered heteroaryl group as defined above, said heteroaryl group having one single covalent bond to $\mathbf{R^2}$-$(CH_2)_n$ and one single covalent bond to the carbon atom bearing the hydroxy group, as depicted in Formula (I). The term "5- to 6-membered heteroarylene" particularly refers 6-membered heteroarylene such as pyridinylene, pyrimidinylene, pyridazinylene, pyrazinylene; and

5-membered heteroarylene such as thiophenylene, triazolylene, imidazolylene, isoxazolylene, thiazolylene, pyrazolylene, oxadiazolylene and thiadiazolylene (respectively also known as pyridindiyl, pyrimidindiyl, pyridazindiyl, pyrazindiyl, thiophendiyl, triazoldiyl, imidazoldiyl, isoxazoldiyl, thiazoldiyl, pyrazoldiyl, oxadiazoldiyl and thiadiazoldiyl). The term "5-membered heteroarylene" especially refers to thiophen-2,5-diyl, [1,2,3]triazol-1,4-diyl, [1,2,3]triazol-2,4-diyl, imidazole-1,4-diyl, isoxazol-3,5-diyl, thiazol-2,5-diyl, pyrazol-1,4-diyl, [1,2,4]oxadiazol-3,5-diyl, [1,3,4]oxadiazol-2,5-diyl and [1,3,4]thiadiazol-2,5-diyl; notably to the following groups:

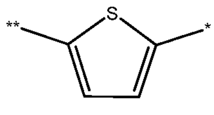
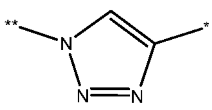
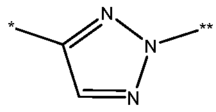
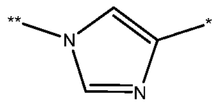
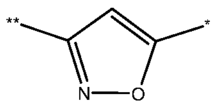
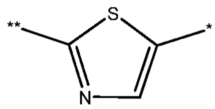
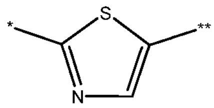
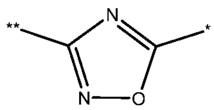
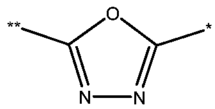
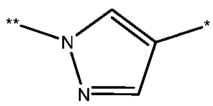
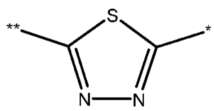

wherein one asterisk "*" denotes the point of attachment to the carbon bearing the OH group; and two asterisks "**" denote the point of attachment to **R**$^{2}$-$(CH_2)_n$ as depicted in Formula (I). The most preferred examples of "5-membered heteroarylene" are [1,2,3]triazol-1,4-diyl and pyrazol-1,4-diyl (notably the following groups:

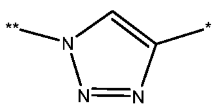
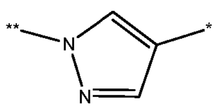
).

Particularly preferred is [1,2,3]triazol-1,4-diyl; notably the following group:

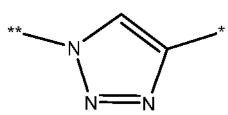

wherein one asterisk "*" denotes the point of attachment to the carbon bearing the OH group; and two asterisks "**" denote the point of attachment to -$(CH_2)_n$**R**$^{2}$ as depicted in Formula (I). All of the above groups are unsubstituted or substituted as explicitly defined.

The term "phenylene" as used for the group **A** refers to a di-substituted benzene ring, wherein the first substituent is **R**$^{2}$-$(CH_2)_n$ and the second substituent is the carbon atom bearing the hydroxy group, as depicted in Formula (I). Examples of phenylene groups are benzene-1,2-diyl, benzene-1,3-diyl and benzene-1,4-diyl. Preferred examples are benzene-1,3-diyl and benzene-1,4-diyl; especially benzene-1,4-diyl. For avoidance of any doubt, said di-substituted benzene may or may not be further substituted as explicitly defined.

The term "9- to 10-membered bicyclic heteroaryl", used alone or in combination, means a heteroaryl as defined before having nine or ten ring atoms (wherein said 9- to 10-membered heteroaryl notably contains one to a maximum of three heteroatoms). For the group **R**$^{2}$, the term "9-membered bicyclic heteroaryl" especially refers to indolyl and isoindolyl; in particular indol-4-yl, indol-5-yl, indol-6-yl, indol-7-yl. For the group **R**$^{2}$, the term "10-membered bicyclic heteroaryl" especially refers to quinolinyl and isoquinolinyl; in particular quinolin-4-yl, quinolin-5-yl, isoquinolin-4-yl, isoquinolin-5-yl and isoquinolin-8-yl. All of the above groups are unsubstituted, or substituted as explicitly defined (especially unsubstituted).

The term "furanyl-$C_{1-3}$-alkyl" refers to an alkyl group as defined before, wherein one of the hydrogen atoms has been replaced by a furane ring. It is understood that said furane ring is attached to said alkyl group in ring position 2 or 3. Representative examples of furanyl-$C_{1-3}$-alkyl groups are furan-2-yl-methyl, 2-(furan-2-yl)-ethyl, furan-3-yl-methyl, 2-(furan-3-yl)-ethyl; especially furan-2-yl-methyl.

The term "tetrahydrofuranyl-$C_{1-3}$-alkyl" refers to an alkyl group as defined before, wherein one of the hydrogen atoms has been replaced by a tetrahydrofurane ring. It is understood that said tetrahydrofurane ring is attached to said alkyl group in ring position 2 or 3. Representative examples of tetrahydrofuranyl-$C_{1-3}$-alkyl groups include tetrahydrofuran-2-yl-methyl, 1-(tetrahydrofuran-2-yl)-ethyl and 2-(tetrahydrofuran-3-yl)-ethyl; especially tetrahydrofuran-2-yl-methyl.

The term "tetrahydropyranyl-$C_{1-3}$-alkyl" refers to an alkyl group as defined before, wherein one of the hydrogen atoms has been replaced by a tetrahydropyrane ring. It is understood that said tetrahydropyrane ring is attached to said alkyl group in ring position 2, 3 or 4. Representative examples of tetrahydropyranyl-$C_{1-4}$-alkyl groups include tetrahydropyran-2-yl-methyl, tetrahydropyran-3-yl-methyl, tetrahydropyran-4-yl-methyl, 1-(tetrahydropyran-2-yl)-ethyl and 2-(tetrahydropyran-3-yl)-ethyl; especially tetrahydropyran-4-yl-methyl and tetrahydropyran-2-yl-methyl.

The term "pyridinyl-$C_{1-3}$-alkyl" refers to an alkyl group as defined before, wherein one of the hydrogen atoms has been replaced by a pyridine ring, wherein said pyridine ring is attached to said alkyl group in ring position 2, 3 or 4. Representative examples of pyridinyl-$C_{1-3}$-alkyl are pyridin-2-yl-methyl, pyridin-3-yl-methyl, pyridin-4-yl-methyl, pyridin-2-yl-ethyl and pyridin-2-yl-propyl; especially pyridin-2-yl-methyl.

The term "piperidin-1-yl-$C_{1-3}$-alkyl" refers to an alkyl group as defined before, wherein one of the hydrogen atoms has been replaced by a piperidine ring, wherein said piperidine ring is attached to said alkyl in ring position 1. Representative examples of piperidin-1-yl-$C_{1-3}$-alkyl groups include piperidin-1-yl-methyl, 1-(piperidin-1-yl)-ethyl and 2-(piperidin-1-yl)-ethyl; especially 2-(piperidin-1-yl)-ethyl.

The term "(1-methyl-piperidin-3-yl)-$C_{1-3}$-alkyl" refers to an alkyl group as defined before, wherein one of the hydrogen atoms has been replaced by a 1-methyl-piperidin-3-yl group. Representative examples of (1-methyl-piperidin-3-yl)-$C_{1-3}$-alkyl groups include (1-methyl-piperidin-3-yl)-methyl and 1-(1-methyl-piperidin-3-yl)-ethyl; especially (1-methyl-piperidin-3-yl)-methyl.

The term "(1,1-dioxidotetrahydro-thiopyran-4-yl)-$C_{1-3}$-alkyl" refers to an alkyl group as defined before, wherein one of the hydrogen atoms has been replaced by a 1,1-dioxidotetrahydro-thiopyran-4-yl group. Representative examples of (1,1-dioxidotetrahydro-thiopyran-4-yl)-$C_{1-3}$-alkyl groups include (1,1-dioxidotetrahydro-thiopyran-4-yl)-methyl, (1,1-dioxidotetrahydro-thiopyran-4-yl)-ethyl and (1,1-dioxidotetrahydro-thiopyran-4-yl)-propyl; especially (1,1-dioxidotetrahydro-thiopyran-4-yl)-methyl.

The term "oxetan-3-yl-$C_{1-3}$-alkyl" refers to an alkyl group as defined before, wherein one of its hydrogen atoms has been replaced by an oxetane ring, wherein said oxetane ring is attached to said alkyl group in ring position 3. Representative examples include oxetan-3-yl-methyl, 1-(oxetan-3-yl)-ethyl, 2-(oxetan-3-yl)-ethyl and 1-(oxetan-3-yl)-propyl; especially oxetan-3-yl-methyl.

The term "($C_{1-3}$-alkyl-oxetan-3-yl)-$C_{1-3}$-alkyl" refers to an oxetan-3-yl-$C_{1-3}$-alkyl group as defined before, wherein one hydrogen atom of the oxetane ring has been replaced by a $C_{1-3}$-alkyl group. Representative examples include (3-methyl-oxetan-3-yl)-methyl, 2-(2-methyl-oxetan-3-yl)-ethyl and 3-(3-methyl-oxetan-3-yl)-propyl especially (3-methyl-oxetan-3-yl)-methyl.

The term "(fluoro-oxetan-3-yl)-$C_{1-3}$-alkyl" refers to oxetan-3-yl-$C_{1-3}$-alkyl group as defined before, wherein one of the hydrogen atoms of the oxetane ring has been replaced by fluorine. Representative examples include (3-fluoro-oxetan-3-yl)-methyl, 2-(2-fluoro-oxetan-3-yl)-ethyl and 3-(3-fluoro-oxetan-3-yl)-propyl; especially (3-fluorooxetan-3-yl)-methyl.

The term "sulfamoyl-$C_{2-4}$-alkyl" refers to an alkyl group as defined before, wherein one hydrogen atom has been replaced by a sulfamoyl group (i.e. $H_2N$-$SO_2$-). Representative examples include 4-(sulfamoyl)-butyl, 3-(sulfamoyl)-propyl and 2-(sulfamoyl)-ethyl; especially 3-(sulfamoyl)-propyl.

The term "alkylsulfonyl", when used alone or in combination, refers to an alkyl group as defined before, wherein one hydrogen atom has been replaced by the group -$SO_2$-. $C_{x-y}$-alkylsulfonyl refers to an alkylsulfonyl group comprising from x to y carbon atoms. Representative examples of $C_{1-3}$-alkylsulfonyl are $CH_3$-$SO_2$-, $C_2H_5$-$SO_2$- and $C_3H_7$-$SO_2$-; especially $CH_3$-$SO_2$-. The term "$C_{1-3}$-alkylsulfonyl-$C_{2-4}$-alkyl" refers to an alkyl group as defined before, wherein one hydrogen atom has been replaced by a alkylsulfonyl group as defined before. Representative examples of $C_{1-3}$-alkylsulfonyl-$C_{2-4}$-alkyl are 2-(methylsulfonyl)-ethyl, 3-(methylsulfonyl)-propyl; especially 2-(methylsulfonyl)-ethyl.

2) A further embodiment of the present invention relates to compounds according to embodiment 1), wherein A represents

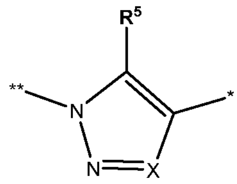

,

wherein one asterisk "*" denotes the point of attachment to the carbon bearing the OH group; two asterisks "**" denote the point of attachment to **$R^2$**-$(CH_2)_n$; **X** represents N or CH (especially **X** represents N); and **$R^5$** represents hydrogen or $C_{1-4}$-alkyl (especially methyl, ethyl, iso-propyl or tert-butyl), halogen (especially fluorine or iodine), or $C_{3-5}$-cycloalkyl (especially cyclopropyl). In a sub-embodiment of embodiment 2) **$R^5$** represents hydrogen or $C_{1-4}$-alkyl (especially methyl). In a further sub-embodiment of embodiment 2) **$R^5$** represents hydrogen.

3) A further embodiment relates to compounds according to embodiment 1) or 2), wherein **$R^2$** represents

- phenyl, which is unsubstituted, or mono-, di- or tri-substituted (especially unsubstituted, or mono-substituted), wherein the substituents independently represent cyano, halogen, $C_{1-4}$-alkyl, $C_{1-3}$-fluoroalkyl, $C_{1-4}$-alkoxy, $C_{1-3}$-fluoroalkoxy, 1-(hydroxymethyl)-cycloprop-1-yl, morpholin-4-yl, morpholin-4-yl-methyl, cyclopropyl-methoxy, acetyl, or **-$NR^{N1}R^{N2}$**, wherein **$R^{N1}$** represents hydrogen and **$R^{N2}$** represents -(C=O)-**$R^{CO}$**, wherein **$R^{CO}$** represents $C_{1-4}$-alkoxy; or

- 5- to 6-membered heteroaryl (especially pyrazolyl, pyridinyl or pyrimidinyl), wherein said 5- to 6-membered heteroaryl independently is unsubstituted, or mono-, di- or tri-substituted (especially unsubstituted, or mono-substituted), wherein the substituents independently represent cyano, halogen, $C_{1-4}$-alkyl, $C_{1-3}$-fluoroalkyl, $C_{1-4}$-alkoxy, $C_{1-3}$-fluoroalkoxy, cyclopropyl-methoxy, acetyl, or - N**$R^{N1}R^{N2}$**, wherein **$R^{N1}$** represents hydrogen and **$R^{N2}$** represents -(C=O)-**$R^{CO}$**, wherein **$R^{CO}$** represents $C_{1-4}$-alkoxy (notably the substituents independently represent cyano, halogen, $C_{1-4}$-alkyl, $C_{1-4}$-alkoxy, or cyclopropyl-methoxy; especially $C_{1-4}$-alkyl, or $C_{1-4}$-alkoxy); or

- 9- to 10-membered bicyclic heteroaryl (especially indolyl, isoindolyl, quinolinyl, or isoquinolinyl), wherein said 9- to 10-membered bicyclic heteroaryl independently is unsubstituted, or mono-, or di-substituted (especially unsubstituted), wherein the substituents independently represent cyano, halogen $C_{1-4}$-alkyl, $C_{1-3}$-fluoroalkyl, $C_{1-4}$-alkoxy, $C_{1-3}$-fluoroalkoxy, cyclopropyl-methoxy, acetyl, or - N**$R^{N1}R^{N2}$**, wherein **$R^{N1}$** represents hydrogen and **$R^{N2}$** represents -(C=O)-**$R^{CO}$**, wherein **$R^{CO}$** represents $C_{1-4}$-alkoxy (notably the substituents independently represent cyano, halogen, $C_{1-4}$-alkyl, $C_{1-4}$-alkoxy, or cyclopropyl-methoxy; especially $C_{1-4}$-alkyl, or $C_{1-4}$-alkoxy); or

- 2,3-dihydro-benzo[1,4]dioxin-6-yl.

4) A further embodiment relates to compounds according to embodiment 1) or 2), wherein **$R^2$** represents

- phenyl, which is unsubstituted, or mono-substituted, (wherein especially the substituent is in *meta* or para position with respect to the point of attachment to **A)** wherein said substituent represents cyano, halogen, $C_{1-4}$-alkyl, $C_{1-3}$-fluoroalkyl, $C_{1-4}$-alkoxy, $C_{1-3}$-fluoroalkoxy, 1-(hydroxymethyl)-cycloprop-1-yl, morpholin-4-yl, morpholin-4-yl-methyl, cyclopropyl-methoxy, acetyl, or **-$NR^{N1}R^{N2}$**, wherein **$R^{N1}$** represents hydrogen and **$R^{N2}$** represents -(C=O)-**$R^{CO}$**, wherein **$R^{CO}$** represents $C_{1-4}$-alkoxy; or

- 5- to 6-membered heteroaryl containing 1, 2 or 3 heteroatoms independently selected from nitrogen and oxygen (notably containing 1, 2 or 3 nitrogen atoms; especially pyrazolyl, pyridinyl, or pyrimidinyl), wherein said 5- to 6-membered heteroaryl is unsubstituted, or mono-, or di-substituted (especially unsubstituted or mono-substituted), wherein the substituents independently represent cyano, halogen, $C_{1-4}$-alkyl, $C_{1-3}$-fluoroalkyl, $C_{1-4}$-alkoxy, $C_{1-3}$-fluoroalkoxy, cyclopropyl-methoxy, acetyl, or **-$NR^{N1}R^{N2}$**, wherein **$R^{N1}$** represents hydrogen and **$R^{N2}$** represents -(C=O)-**$R^{CO}$**, wherein **$R^{CO}$** represents $C_{1-4}$-alkoxy (notably the substituents independently represent cyano, halogen, $C_{1-4}$-alkyl, $C_{1-4}$-alkoxy, or cyclopropyl-methoxy; especially $C_{1-4}$-alkyl, or $C_{1-4}$-alkoxy); or

- 9- to 10-membered bicyclic heteroaryl containing 1, 2 or 3 heteroatoms independently selected from nitrogen

and oxygen (notably containing 1, 2 or 3 nitrogen atoms; especially indolyl, isoindolyl, quinolinyl, or isoquinolinyl), wherein said 9- to 10-membered bicyclic heteroaryl independently is unsubstituted, or mono-, or di-substituted (especially unsubstituted), wherein the substituents independently represent cyano, halogen $C_{1-4}$-alkyl, $C_{1-3}$-fluoroalkyl, $C_{1-4}$-alkoxy, $C_{1-3}$-fluoroalkoxy, cyclopropyl-methoxy, acetyl, or **-NR^N1^R^N2^**, wherein $\mathbf{R^{N1}}$ represents hydrogen and $\mathbf{R^{N2}}$ represents - (C=O)-$\mathbf{R^{CO}}$, wherein $\mathbf{R^{CO}}$ represents $C_{1-4}$-alkoxy (notably the substituents independently represent cyano, halogen, $C_{1-4}$-alkyl, $C_{1-4}$-alkoxy, or cyclopropyl-methoxy; especially $C_{1-4}$-alkyl, or $C_{1-4}$-alkoxy); or

- 2,3-dihydro-benzo[1,4]dioxin-6-yl.

5) A further embodiment relates to compounds according to embodiment 1) or 2), wherein $\mathbf{R^2}$ represents

- phenyl or 6-membered heteroaryl (wherein especially said 6-membered heteroaryl contains 1 or 2 ring nitrogen atoms; notably said 6-membered heteroaryl is pyridinyl, pyrimidinyl or pyridazinyl), wherein said phenyl or 6-membered heteroaryl is independently unsubstituted, or mono-, di- or tri-substituted (especially unsubstituted, mono-, or di-substituted), and wherein the substituents are independently selected from:

  ➢ $C_{1-4}$-alkyl (especially methyl), cyano, halogen (especially chlorine or fluorine), hydroxy, $C_{1-3}$-fluoroalkyl (especially trifluoromethyl), $C_{1-3}$-fluoroalkoxy (especially trifluoromethoxy or 2,2,2-trifuloroethoxy), $C_{1-4}$-alkoxy (especially methoxy, ethoxy, iso-propoxy), $C_{3-5}$-cycloalkoxy (especially cyclobutoxy), hydroxymethyl-cyclopropyl (especially 1-(hydroxymethyl)-cycloprop-1-yl), $C_{1-3}$-alkyl-carbonyl (especially acetyl), $C_{1-4}$-alkoxy-carbonyl (especially methoxy-carbonyl) or morpholin-4-yl;

  ➢ $\mathbf{-NR^{N1}R^{N2}}$, wherein $\mathbf{R^{N1}}$ represents hydrogen and $\mathbf{R^{N2}}$ represents -(C=O)-$\mathbf{R}^{CO}$, wherein $\mathbf{R^{CO}}$ represents $C_{1-4}$-alkyl (especially methyl, iso-propyl or tert-butyl), $C_{1-4}$-alkoxy (especially methoxy or tert-butoxy), amino or morpholin-4-yl;

  ➢ -(C=O)-N$\mathbf{R^{N3}R^{N4}}$, wherein $\mathbf{R^{N3}}$ represents hydrogen or $C_{1-3}$-alkyl (especially methyl), and $\mathbf{R^{N4}}$ represents hydrogen, $C_{1-3}$-alkyl (especially methyl or ethyl), $C_{3-5}$-cycloalkyl (especially cyclopropyl), hydroxy-$C_{2-4}$-alkyl (especially 2-hydroxyethyl or 3-hydroxypropyl), $C_{1-3}$-alkoxy-$C_{2-4}$-alkyl (especially 2-methoxyethyl or 3-methoxypropyl), cyano-$C_{1-3}$-alkyl (especially 2-cyanoethyl), $C_{3-4}$-alkenyl (especially prop-1-en-3-yl) or furanyl-$C_{1-3}$-alkyl (especially furan-2-yl-methyl); or wherein $\mathbf{R^{N3}}$ and $\mathbf{R^{N4}}$ together with the nitrogen atom to which they are attached form a morpholine ring; and

  ➢ $\mathbf{-OR^6}$, wherein $\mathbf{R^6}$ represents $C_{1-3}$-alkoxy-$C_{2-4}$-alkyl (especially 2-methoxyethyl), hydroxy-$C_{2-5}$-alkyl (especially 3-hydroxypropyl, 2-hydroxy-2-methylpropyl, 2-hydroxyethyl, 3-hydroxy-2,2-dimethylpropyl or 3-hydroxy-3-methylbutyl), $C_{3-5}$-cycloalkyl-$C_{1-3}$-alkyl (especially cyclopropyl-methyl), oxetan-3-yl-$C_{1-3}$-alkyl (especially oxetan-3-yl-methyl), ($C_{1-3}$-alkyl-oxetan-3-yl)-$C_{1-3}$-alkyl (especially (3-methyloxetan-3-yl)-methyl), (fluoro-oxetan-3-yl)-$C_{1-3}$-alkyl (especially (3-fluorooxetan-3-yl)-methyl), tetrahydrofuranyl-$C_{1-3}$-alkyl (especially tetrahydrofuran-2-yl-methyl), hydroxy-$C_{3-5}$-cycloalkyl-$C_{1-3}$-alkyl (especially (1-hydroxycyclopropyl)-ethyl), tetrahydropyranyl-$C_{1-3}$-alkyl (especially tetrahydropyran-2-yl-methyl or tetrahydropyran-4-yl-methyl), benzyl, (hydroxy-$C_{2-4}$-alkoxy)-$C_{2-4}$-alkyl (especially 2-(2-hydroxyethoxy)-ethyl), piperidin-1-yl-$C_{1-3}$-alkyl (especially 2-(piperidin-1-yl)-ethyl), (1-methyl-piperidin-3-yl)-$C_{1-3}$-alkyl (especially (1-methyl-piperidin-3-yl)-methyl)), (1,1-dioxidotetrahydro-thiopyran-4-yl)-$C_{1-3}$-alkyl (especially (1,1-dioxidotetrahydro-thiopyran-4-yl)-methyl), $C_{1-3}$-alkoxy-carbonyl-$C_{1-3}$-alkyl (especially methoxy-carbonyl-methyl) or $C_{1-3}$-alkyl-carbonyl (especially acetyl);

- 9- to 10-membered bicyclic heteroaryl (especially quinolinyl, isoquinolinyl or indolyl), wherein said 9- to 10-membered bicyclic heteroaryl is unsubstituted; or

- 2,3-dihydro-benzo[1,4]dioxin-6-yl, 1,3-dihydro-2H-benzoimidazol-2-one-5-yl or 3,4-dihydro-2H-pyrano[2,3-b]pyridine-6-yl.

6) A further embodiment relates to compounds according to embodiment 1) or 2), wherein $\mathbf{R^2}$ represents

- phenyl or 6-membered heteroaryl (wherein especially said 6-membered heteroaryl contains 1 or 2 ring nitrogen atoms; notably said 6-membered heteroaryl is pyridinyl, pyrimidinyl or pyridazinyl), wherein said phenyl or 6-membered heteroaryl is independently unsubstituted, or mono-, di- or tri-substituted (especially unsubstituted,

mono-, or di-substituted), and wherein the substituents are independently selected from:

➢ $C_{1-4}$-alkyl (especially methyl or ethyl), cyano, nitro, halogen (especially chlorine, fluorine or bromine), hydroxy, hydroxy-$C_{1-4}$-alkyl (especially hydroxymethyl), $C_{1-3}$-alkoxy-$C_{1-4}$-alkyl (especially methoxymethyl), $C_{1-3}$-fluoroalkyl (especially trifluoromethyl), $C_{1-3}$-fluoroalkoxy (especially trifluoromethoxy or 2,2,2-trifuloroethoxy), $C_{1-4}$-alkoxy (especially methoxy, ethoxy, iso-propoxy), $C_{3-5}$-cycloalkoxy (especially cyclobutoxy), hydroxymethyl-cyclopropyl (especially 1-(hydroxymethyl)-cycloprop-1-yl), $C_{1-3}$-alkyl-carbonyl (especially acetyl), $C_{1-4}$-alkoxy-carbonyl (especially methoxy-carbonyl), amino, morpholin-4-yl or morpholin-4-yl-methyl;

➢ **-NR$^{N1}$R$^{N2}$**, wherein **R$^{N1}$** represents hydrogen and **R$^{N2}$** represents -(C=O)-**R$^{CO}$**, wherein **R$^{CO}$** represents $C_{1-4}$-alkyl (especially methyl, iso-propyl or tert-butyl), $C_{1-4}$-alkoxy (especially methoxy or tert-butoxy), amino, phenyl or morpholin-4-yl;

➢ -(C=O)-N**R$^{N3}$R$^{N4}$**, wherein **R$^{N3}$** represents hydrogen or $C_{1-3}$-alkyl (especially methyl), and **R$^{N4}$** represents hydrogen, $C_{1-3}$-alkyl (especially methyl or ethyl), $C_{3-5}$-cycloalkyl (especially cyclopropyl), hydroxy-$C_{2-4}$-alkyl (especially 2-hydroxyethyl or 3-hydroxypropyl), $C_{1-3}$-alkoxy-$C_{2-4}$-alkyl (especially 2-methoxyethyl or 3-methoxypropyl), cyano-$C_{1-3}$-alkyl (especially 2-cyanoethyl), $C_{3-4}$-alkenyl (especially prop-1-en-3-yl) or furanyl-$C_{1-3}$-alkyl (especially furan-2-yl-methyl) or allyl; or wherein **R$^{N3}$** and **R$^{N4}$** together with the nitrogen atom to which they are attached form a morpholine ring; and

➢ **-OR$^6$**, wherein **R$^6$** represents $C_{1-3}$-alkoxy-$C_{2-4}$-alkyl (especially 2-methoxyethyl), hydroxy-$C_{2-5}$-alkyl (especially 3-hydroxypropyl, 2-hydroxy-2-methylpropyl, 2-hydroxyethyl, 3-hydroxy-2,2-dimethylpropyl or 3-hydroxy-3-methylbutyl), di-hydroxy-$C_{2-5}$-alkyl (especially 2,3-dihydroxypropyl), sulfamoyl-$C_{2-4}$-alkyl (especially 3-(sulfamoyl)-propyl), pyridinyl-$C_{1-3}$-alkyl (especially pyridine-2-yl-methyl), $C_{1-3}$-alkylsulfonyl-$C_{2-4}$-alkyl (especially 2-(methylsulfonyl)-ethyl), $C_{3-5}$-cycloalkyl-$C_{1-3}$-alkyl (especially cyclopropyl-methyl), oxetan-3-yl-$C_{1-3}$-alkyl (especially oxetan-3-yl-methyl), ($C_{1-3}$-alkyl-oxetan-3-yl)-$C_{1-3}$-alkyl (especially (3-methyloxetan-3-yl)-methyl), (fluoro-oxetan-3-yl)-$C_{1-3}$-alkyl (especially (3-fluorooxetan-3-yl)-methyl), tetrahydrofuranyl-$C_{1-3}$-alkyl (especially tetrahydrofuran-2-yl-methyl), hydroxy-$C_{3-5}$-cycloalkyl-$C_{1-3}$-alkyl (especially (1-hydroxycyclopropyl)-ethyl), tetrahydropyranyl-$C_{1-3}$-alkyl (especially tetrahydropyran-2-yl-methyl or tetrahydropyran-4-yl-methyl), benzyl, (hydroxy-$C_{2-4}$-alkoxy)-$C_{2-4}$-alkyl (especially 2-(2-hydroxyethoxy)-ethyl), piperidin-1-yl-$C_{1-3}$-alkyl (especially 2-(piperidin-1-yl)-ethyl), (1-methyl-piperidin-3-yl)-$C_{1-3}$-alkyl (especially (1-methyl-piperidin-3-yl)-methyl)), (1,1-dioxidotetrahydro-thiopyran-4-yl)-$C_{1-3}$-alkyl (especially (1,1-dioxidotetrahydro-thiopyran-4-yl)-methyl), $C_{1-3}$-alkoxy-carbonyl-$C_{1-3}$-alkyl (especially methoxy-carbonyl-methyl or ethoxy-carbonyl-methyl), ((hydroxy-$C_{2-3}$-alkoxy)-$C_{2-3}$-alkoxy)-$C_{2-3}$-alkyl (especially 2-(2-(2-hydroxyethoxy)ethoxy)-ethyl), $C_{1-3}$-fluoroalkoxy-$C_{2-3}$-alkyl (especially 2-(2,2,2-trifluoroethoxy)-ethyl) or $C_{1-3}$-alkyl-carbonyl (especially acetyl);

- 9- to 10-membered bicyclic heteroaryl (especially quinolinyl, isoquinolinyl, indazolyl or indolyl), wherein said 9- to 10-membered bicyclic heteroaryl is unsubstituted; or

- 2,3-dihydro-benzo[1,4]dioxin-6-yl, 1,3-dihydro-2H-benzoimidazol-2-one-5-yl, 3H-benzooxazol-2-one-6-yl, 3-methyl-1,1-dioxo-2,3-dihydro-1*H*-1l6-benzo[*d*]isothiazol-5-yl, 1-methyl-1H-pyrrolo[2,3-b]pyridin-5-yl or 3,4-dihydro-2H-pyrano[2,3-b]pyridine-6-yl.

7) A further embodiment relates to compounds according to embodiment 1) or 2), wherein **R$^2$** represents phenyl, which is unsubstituted, or mono-, di- or tri-substituted (especially unsubstituted, or mono-substituted), wherein the substituents independently represent cyano, halogen, $C_{1-4}$-alkyl, $C_{1-3}$-fluoroalkyl, $C_{1-4}$-alkoxy, $C_{1-3}$-fluoroalkoxy, 1-(hydroxymethyl)-cycloprop-1-yl, morpholin-4-yl, morpholin-4-yl-methyl, cyclopropyl-methoxy, acetyl, or **-NR$^{N1}$R$^{N2}$**, wherein **R$^{N1}$** represents hydrogen and **R$^{N2}$** represents -(C=O)-**R**$^{CO}$, wherein **R$^{CO}$**represents $C_{1-4}$-alkoxy. In a sub-embodiment, **R$^2$** especially represents phenyl, 4-chloro-phenyl, 2-methyl-phenyl, 3-methyl-phenyl, 4-methyl-phenyl, 2-methoxy-phenyl, 3-methoxy-phenyl, 4-methoxy-phenyl, 4-ethoxy-phenyl, 3-cyano-phenyl, 4-cyano-phenyl, 2-trifluoromethyl-phenyl, 3-trifluoromethyl-phenyl, 4-trifluoromethyl-phenyl, 4-isopropoxy-phenyl, 4-(cyclopropyl-methoxy)-phenyl, 4-(1-hydroxymethyl-cyclopropyl)-phenyl, 4-trifluoromethoxy-phenyl, 4-(morpholin-4-yl)-phenyl, 4-(morpholin-4-yl-methyl)-phenyl, 4-(methoxy-carbamoyl)-phenyl, or 4-acetyl-phenyl; preferably **R$^2$** represents phenyl, 4-chloro-phenyl, 2-methyl-phenyl, 3-methyl-phenyl, 4-methyl-phenyl, 2-methoxy-phenyl, 3-methoxy-phenyl, 4-methoxy-phenyl, 4-ethoxy-phenyl, 3-cyano-phenyl, 4-cyano-phenyl, 4-isopropoxy-phenyl, 4-(cyclopropyl-methoxy)-phenyl, 4-(methoxy-carbamoyl)-phenyl, or 4-acetyl-phenyl.

8) A further embodiment of the present invention relates to compounds according to embodiment 1) or 2), when **$R^2$** represents phenyl or 6-membered heteroaryl (especially phenyl), wherein said phenyl or 6-membered heteroaryl is independently mono-, di- or tri-substituted (especially mono- or di-substituted); wherein a first substituent independently represents hydroxy, **$-NR^{N1}R^{N2}$**, -(C=O)-N**$R^{N3}R^{N4}$** or **$-OR^6$**; wherein said first substituent is attached in para-position with regard to the point of attachment to the rest of the molecule; and wherein a further substituent(s), if present, is/are independently selected from $C_{1-4}$-alkyl (especially methyl), cyano, halogen (especially chlorine or fluorine), hydroxy, $C_{1-3}$-fluoroalkyl (especially trifluoromethyl), $C_{1-3}$-fluoroalkoxy (especially trifluoromethoxy or 2,2,2-trifuloroethoxy), $C_{1-4}$-alkoxy (especially methoxy, ethoxy or iso-propoxy), $C_{1-3}$-alkyl-carbonyl (especially acetyl) or $C_{1-4}$-alkoxy-carbonyl (especially methoxy-carbonyl); and wherein said further substituent(s), if present, is/are attached in *meta*-position with regard to the point of attachment to the rest of the molecule).

9) A further embodiment relates to compounds according to embodiments 1) or 2), wherein the fragment **$R^2$**-$(CH_2)_n$-is selected from group I, II, III, IV, V, VI, VII, VIII, IX, X, XI or XII:

I. phenyl, 4-hydroxy-phenyl, 4-chloro-phenyl, 2-methyl-phenyl, 3-methyl-phenyl, 4-methyl-phenyl, 2-methoxy-phenyl, 3-methoxy-phenyl, 4-methoxy-phenyl, 4-ethoxy-phenyl, 4-(2-methoxy-ethoxy)-phenyl, 4-benzyloxy-phenyl, 3-cyano-phenyl, 4-cyano-phenyl, 2-trifluoromethyl-phenyl, 3-trifluoromethyl-phenyl, 4-trifluoromethyl-phenyl, 4-isopropoxy-phenyl, 4-(cyclopropyl-methoxy)-phenyl, 4-(1-hydroxymethyl-cyclopropyl)-phenyl, 4-trifluoromethoxy-phenyl, 4-(morpholin-4-yl)-phenyl, 4-(morpholin-4-yl-methyl)-phenyl or 4-(methoxy-carbamoyl)-phenyl;

II. 4-(2-hydroxy-2-methyl-propoxy)-phenyl, 4-(3-hydroxy-propoxy)-phenyl, 3-cyano-4-methoxy-phenyl, 4-(2-hydroxy-ethoxy)-phenyl, 3-chloro-4-hydroxy-phenyl, 3-fluoro-4-hydroxy-phenyl, 3,5-difluoro-4-hydroxy-phenyl, 4-((methoxy-carbonyl)amino)-phenyl, 4-(morpholine-4-carbonyl)-phenyl, 4-((2-cyanoethyl)-carbamoyl)-phenyl, 4-(morpholine-4-yl-carboxamido)-phenyl, 4-(*tert*-butyl-carboxamido)-phenyl, 2-fluoro-4-(*tert*-butoxyl-carboxamido)-phenyl, 4-(3-ethylureido)-3-methoxy-phenyl, 4-(methoxy-carboxamido)-phenyl, 4-(3-ethyl-ureido)-3-methoxy-phenyl, 4-(3-(3-methoxy-propyl)-ureido)-phenyl, 4-(3-butyl-ureido)-phenyl, 4-isobutyramido-phenyl, 4-acetyl-phenyl, 4-(tetrahydropyran-4-yl-methoxy)-phenyl, 4-((3-fluoro-oxetan-3-yl)-methoxy)-phenyl, 4-((3-methyl-oxetan-3-yl)-methoxy)-phenyl, 4-(oxetan-3-yl-methoxy)-phenyl, 4-(2-(1-hydroxy-cyclopropyl)-ethoxy)-phenyl, 4-(3-hydroxy-2,2-dimethylpropyl)-phenyl, 4-(3-hydroxy-3-methyl-butyl)-phenyl, 4-(2-(2-hydroxy-ethoxy)-ethoxy)-phenyl, 4-(2-(piperidin-1-yl)-ethoxy)-phenyl, 4-(tetrahydrofuran-2-yl-methoxy)-phenyl, 4-(tetrahydropyran-4-yl-methoxy)-phenyl, 4-(tetrahydropyran-2-yl-methoxy)-phenyl, 4-((2-methoxy-ethyl)-carbamoyl)-phenyl, 4-(cyclopropyl-carbamoyl)-phenyl, 4-(ethyl-carbamoyl)-phenyl, 4-(3-methoxypropyl-carbamoyl)-phenyl, 4-ureido-phenyl, 4-acetamido-phenyl, 4-(methyl-carbamoyl)-phenyl, 4-((1-methyl-piperidin-3-yl)-methoxy)-phenyl 4-((2-hydroxy-ethyl)-carbamoyl)-phenyl, 4-(ethyl(methyl)carbamoyl)-phenyl, 4-((3-hydroxy-propyl)-carbamoyl)-phenyl, 4-carbamoyl-phenyl, 4-((furan-2-yl-methyl)-carbamoyl)-phenyl, 4-(allyl-carbamoyl)-phenyl, 4-(dimethyl-carbamoyl)-phenyl, 4-((2-(dimethylamino)-ethyl)-carbamoyl)-phenyl, 4-((1,1-dioxo-hexahydrothiopyran-4-yl)-methyl)-phenyl;

III. 3-chloro-4-(3-hydroxy-propoxy)-phenyl, 3-chloro-4-(2-methyl-2-hydroxy-propoxy)-phenyl, 3-chloro-4-(3-methyl-3-hydroxy-butoxy)-phenyl, 4-(2-methyl-2-hydroxy-propoxy)-phenyl, 4-(2,3-dihydroxy-propoxy)-phenyl, 3-cyano-5-methoxy-phenyl, 3-hydroxy-4-cyano-phenyl, 3-nitro-4-hydroxy-phenyl, 2-chloro-4-hydroxy-phenyl, 2-fluoro-4-hydroxy-phenyl, 3-hydorxy-4-chloro-phenyl, 2-hydorxy-4-chloro-phenyl, 3-hydroxy-phenyl, 2,5-difluoro-4-hydoxy-phenyl, 2,5-difluoro-4-hydoxy-phenyl, 3-trifluoromethoxy-4-hydroxy-phenyl, 3-cyano-4-trifluoromethoxy-phenyl, 2-trifluoromethoxy-4-hydroxy-phenyl, 3-methyl-5-hydroxy-phenyl, 2-methyl-4-hydroxy-phenyl, 3-hydroxy-4-methyl-phenyl, 3-methyl-4-hydroxy-phenyl, 3-ethyl-4-hydroxy-phenyl, 4-(hydroxy-methyl)-phenyl, (4-hydroxy-phenyl)-methyl, (2-hydroxy-phenyl)-methyl, 3-chloro-4-amino-phenyl, 4-(amino-carbonyl)-phenyl, 4-(methyl-aminocarbonyl)-phenyl, (4-(methoxy-carbonyl)-phenyl)-methyl, (3-(methoxy-carbonyl)-phenyl)-methyl, 4-(methyl-carboxamido)-phenyl, 4-(phenyl-carboxamido)-phenyl, 4-(iso-propyl-carboxamido)-phenyl, 3-chloro-4-(iso-propyl-carboxamido)-phenyl, 4-(tert-butoxy-carboxamido)-phenyl, 3-hydroxy-4-methoxy-phenyl, 3-chloro-4-methoxy-phenyl, 3-chloro-4-ethoxy-phenyl, 3-fluoro-4-methoxy-phenyl, 3-bromo-4-methoxy-phenyl, 3-iodo-4-methoxy-phenyl,3-chloro-4-methoxy-5-fluoro-phenyl, 3-chloro-4-methoxy-6-fluoro-phenyl, 3,6-difluoro-4-methoxy-phenyl, 2-fluoro-3-chloro-4-methoxy-phenyl, 2,6-dimethyl-4-methoxy-phenyl, 3-chloro-4-(cyclopropyl-methoxy)-pheny, 3-ethyl-4-hydroxy-phenyl, 4-(methyl-methoxy)-phenyl, 3-hydroxy-5-trifluoromethyl-phenyl, 2-trifluoromethyl-4-hydroxy-phenyl, 3,4-dimethoxy-5-cyano-phenyl, 4-((2-dimethylamino-ethoxy))-phenyl, 4-(2-(2-(2-hydroxyethoxy)-ethoxy)-ethoxy)-phenyl, 3-chloro-4-((3-fluoro-oxetan-3-yl)-methoxy)-phenyl, 4-(pyrrolidine-1-sulfonyl)-phenyl, 4-(pyridine-2-yl-methoxy)-phenyl, 4-(ethoxy-carbonyl-methoxy)-phenyl, 4-((2-hydroxy-ethyl)-carbamoyl)-phenyl, 4-((2-methoxy-ethyl)-carbamoyl)-phenyl, 4-((3-hydroxy-propyl)-carbamoyl)-phenyl, 4-(allyl-carbamoyl)-phenyl, 4-(allyl-carbamoyl)-phenyl, 4-(dimethyl-carbamoyl)-phenyl, 4-(N-methyl-N-ethyl-carbamoyl)-phenyl, 3-fluoro-4-benzyloxy-phenyl, 4-sulfamoyl-phenyl, 4-(methyl-sulfamoyl)-phenyl, 4-(ethyl-sulfamoyl)-phenyl, 4-(dimethyl-sulfamoyl)-phenyl, 4-(diethyl-sulfamoyl)-phenyl, 4-((methyl-sulfamoyl)-amino)-phenyl, 4-(2-methanesulfonyl-ethoxy)-phenyl, 4-(sulfamoyl-propoxy)-phenyl, 4-(2-(2,2,2-trifluoro-ethoxy)-ethoxy)-phenyl;

IV. 6-methoxy-pyridin-3-yl, 5-fluoro-6-methoxy-pyridin-3-yl, 6-methoxy-5-methyl-pyridin-3-yl, 2,6-dimethoxy-pyridin-3-yl, 5,6-dimethoxy-pyridin-3-yl, 5,6-dimethoxy-pyridin-2-yl, 5-chloro-6-ethoxy-pyridin-3-yl, 6-isopropoxy-pyridin-3-yl, 2-methoxy-3-(methoxy-carbonyl)-pyridin-5-yl, 5-chloro-6-isopropoxy-pyridin-3-yl, 6-ethoxy-5-trifluoromethyl-pyridin-3-yl, 6-(methoxy-carbonyl-methoxy)-pyridin-3-yl, 6-methoxy-5-(methoxycarbonyl)-pyridin-3-yl, 2-methoxy-pyrimidin-5-yl, 2-ethoxy-pyrimidin-5-yl, 2,4-dimethoxy-pyrimidin-5-yl, 2-benzyloxy-pyrimidin-5-yl, 2-cyclobutoxy-pyrimidin-5-yl, 3,6-dimethoxy-pyridazin-4-yl or 6-(2,2,2-trifluoro-ethoxy)-pyridin-3-yl;

V. pyridin-3-yl, 4-methoxy-pyridin-3-yl or 3-chloro-4-bromo-pyridin-2-yl;

VI. pyrazol-4-yl, 1-methyl-pyrazol-4-yl, pyrimidin-5-yl, pyridin-2-yl, pyridin-3-yl, pyridin-4-yl or 6-ethoxy-pyridin-3-yl;

VII. indol-4-yl, indol-5-yl, indol-6-yl, indol-7-yl, quinolin-4-yl, quinolin-5-yl, isoquinolin-4-yl, isoquinolin-5-yl or isoquinolin-8-yl;

VIII. 1H-indazol-5-yl, 3-chloro-1H-indazol-6-yl or 1-methyl-1H-indazol-7-yl;

IX. benzyl;

X. 2,3-dihydro-[1,4]benzodioxan-6-yl;

XI. 1,3-dihydro-2H-benzoimidazol-2-one-5-yl or 3,4-dihydro-2H-pyrano[2,3-b]pyridine-6-y; and

XII. 3H-benzooxazol-2-one-6-yl, 3-methyl-1,1-dioxo-2,3-dihydro-1*H*-1l6-benzo[*d*]isothiazol-5-yl, 1-methyl-1H-pyrrolo[2,3-b]pyridin-5-yl, 2,3-dihydro-benzo[1 ,4]dioxin-6-yl;

10) A further embodiment relates to compounds according to embodiment 9), wherein the fragment $\mathbf{R^2}$-$(CH_2)_n$-is selected from group I, VI, VII, IX or X.

11) A further embodiment relates to compounds according to embodiment 9), wherein the fragment $\mathbf{R^2}$-$(CH_2)_n$-is selected from group II, IV or XI.

12) A further embodiment relates to compounds according to embodiment 9), wherein the fragment $\mathbf{R^2}$-$(CH_2)_n$-is selected from group III, V, VIII or XII.

13) A further embodiment relates to compounds according to any one of embodiments 1) to 8), wherein **n** represents 1.

14) A further embodiment relates to compounds according to any one of embodiments 1) to 8), wherein **n** represents 0.

15) A further embodiment relates to compounds according to any one of embodiments 1) to 14), wherein $\mathbf{R^1}$ represents:

- $C_{1-4}$-alkyl (especially methyl or ethyl);
- vinyl, 1-methyl-vinyl or 1-propenyl;
- $C_{1-3}$-fluoroalkyl (especially trifluoromethyl or 1,1-difluoroethyl); or
- $C_{3-6}$-cycloalkyl (especially cyclopropyl), which is unsubstituted, mono- or di-substituted (especially unsubstituted or mono-substituted), wherein the substituents are independently selected from $C_{1-3}$-alkyl (especially methyl) or fluorine; or

- phenyl, which is unsubstituted or mono, di- or tri-substituted (especially unsubstituted, mono-, or di-substituted), wherein the substituents are independently selected from $C_{1-3}$-alkyl (especially methyl) and halogen (especially fluorine).

16) A further embodiment relates to compounds according to any one of embodiments 1) to 14), wherein **$R^1$** represents phenyl, $C_{1-4}$-alkyl (especially methyl or ethyl), or $C_{3-6}$-cycloalkyl (especially cyclopropyl), wherein said $C_{3-6}$-cycloalkyl is unsubstituted or mono-substituted with methyl or fluorine. In a sub-embodiment, **$R^1$** represents cyclopropyl, wherein the cyclopropyl is unsubstituted or mono-substituted with methyl (especially 1-methyl-cyclopropyl) or fluorine or (especially 2-fluoro-cyclopropyl).

17) A further embodiment of the present invention relates to compounds according to any one of embodiments 1) to 14), wherein **$R^1$** represents cyclopropyl.

18) A further embodiment of the present invention relates to compounds according to any one of embodiments 1) to 14), wherein **$R^1$** represents phenyl.

19) A further embodiment of the present invention relates to compounds according to any one of embodiments 1) to 14), wherein **$R^1$** represents $C_{1-4}$-alkyl (especially ethyl or methyl).

20) A further embodiment relates to compounds according to any one of embodiments 1) to 14), wherein

**A** represents 5-membered heteroarylene containing 1, 2 or 3 ring heteroatoms independently selected from oxygen, nitrogen and sulphur (especially triazolylene or pyrazolylene), wherein said 5-membered heteroarylene is unsubstituted or mono-substituted, wherein the substituents are independently selected from $C_{1-4}$-alkyl (especially methyl).
**n** represents 0 (i.e. **$R^2$** is directly attached to **A);**
**$R^1$** represents:

- $C_{1-4}$-alkyl (especially methyl or ethyl);
- $C_{1-3}$-fluoroalkyl (especially trifluoromethyl);
- phenyl, which is unsubstituted or mono-substituted (especially unsubstituted, mono-, or di-substituted), wherein the substituents are independently selected from $C_{1-3}$-alkyl (especially methyl) and halogen (especially fluorine); or
- $C_{3-6}$-cycloalkyl (especially cyclopropyl or cyclopentyl), which is unsubstituted or mono-substituted, wherein the substituents are independently selected from $C_{1-3}$-alkyl (especially methyl) and halogen (especially fluorine);

**$R^2$** represents:

- phenyl or 6-membered heteroaryl containing 1, 2 or 3 ring heteroatoms independently selected from oxygen, nitrogen and sulphur (especially 1, 2 or 3 ring nitrogen atoms; notably pyridinyl, pyrimidinyl, pyridazinyl), wherein said phenyl or 6-membered heteroaryl are independently unsubstituted, or mono-, di- or tri-substituted (especially unsubstituted, mono-, or di-substituted), and wherein the substituents are independently selected from:

    ➢ $C_{1-4}$-alkyl (especially methyl), cyano, halogen (especially chlorine or fluorine), hydroxy, $C_{1-3}$-fluoroalkyl (especially trifluoromethyl), $C_{1-3}$-fluoroalkoxy (especially trifluoromethoxy or 2,2,2-trifuloroethoxy), $C_{1-4}$-alkoxy (especially methoxy, ethoxy, iso-propoxy), $C_{3-5}$-cycloalkoxy (especially cyclobutoxy), hydroxymethyl-cyclopropyl (especially 1-(hydroxymethyl)-cycloprop-1-yl), $C_{1-3}$-alkyl-carbonyl (especially acetyl), $C_{1-4}$-alkoxy-carbonyl (especially methoxy-carbonyl), morpholin-4-yl, morpholin-4-yl-methyl;

    ➢ **$-NR^{N1}R^{N2}$**, wherein **$R^{N1}$** represents hydrogen and **$R^{N2}$** represents -(C=O)-**$R^{CO}$**, wherein **$R^{CO}$** represents $C_{1-4}$-alkyl (especially methyl, iso-propyl or tert-butyl), $C_{1-4}$-alkoxy (especially methoxy or tert-butoxy), amino, $C_{1-4}$-alkylamino (especially ethylamino or n-butylamino) or morpholin-4-yl;

➢ -(C=O)-N**$R^{N3}$$R^{N4}$**, wherein **$R^{N3}$** represents hydrogen or $C_{1-3}$-alkyl (especially methyl), and **$R^{N4}$** represents hydrogen, $C_{1-3}$-alkyl (especially methyl or ethyl), $C_{3-5}$-cycloalkyl (especially cyclopropyl), hydroxy-$C_{2-4}$-alkyl (especially 2-hydroxyethyl or 3-hydroxypropyl), $C_{1-3}$-alkoxy-$C_{2-4}$-alkyl (especially 2-methoxyethyl or 3-methoxypropyl), cyano-$C_{1-3}$-alkyl (especially 2-cyanoethyl), $C_{3-4}$-alkenyl (especially prop-1-en-3-yl), furanyl-$C_{1-3}$-alkyl (especially furan-2-yl-methyl), 2-di-$C_{1-3}$-alkylamino-$C_{2-3}$-alkyl (especially dimethylaminoethyl); or wherein **$R^{N3}$** and **$R^{N4}$** together with the nitrogen atom to which they are attached form a morpholine ring; and

➢ **-O$R^6$**, wherein **$R^6$** represents $C_{1-3}$-alkoxy-$C_{2-4}$-alkyl (especially 2-methoxyethyl), hydroxy-$C_{2-5}$-alkyl (especially 3-hydroxypropyl, 2-hydroxy-2-methylpropyl, 2-hydroxyethyl, 3-hydroxy-2,2-dimethylpropyl or 3-hydroxy-3-methylbutyl), $C_{3-5}$-cycloalkyl-$C_{1-3}$-alkyl (especially cyclopropyl-methyl), oxetan-3-yl-$C_{1-3}$-alkyl (especially oxetan-3-yl-methyl), ($C_{1-3}$-alkyl-oxetan-3-yl)-$C_{1-3}$-alkyl (especially (3-methyloxetan-3-yl)-methyl), (fluoro-oxetan-3-yl)-$C_{1-3}$-alkyl (especially (3-fluorooxetan-3-yl)-methyl), tetrahydrofuranyl-$C_{1-3}$-alkyl (especially tetrahydrofuran-2-yl-methyl), hydroxy-$C_{3-5}$-cycloalkyl-$C_{1-3}$-alkyl (especially (1-hydroxycyclopropyl)-ethyl), tetrahydropyranyl-$C_{1-3}$-alkyl (especially tetrahydropyran-2-yl-methyl or tetrahydropyran-4-yl-methyl), benzyl, (hydroxy-$C_{2-4}$-alkoxy)-$C_{2-4}$-alkyl (especially 2-(2-hydroxyethoxy)-ethyl), piperidin-1-yl-$C_{1-3}$-alkyl (especially 2-(piperidin-1-yl)-ethyl), (1-methyl-piperidin-3-yl)-$C_{1-3}$-alkyl (especially (1-methyl-piperidin-3-yl)-methyl)), (1,1-dioxidotetrahydro-thiopyran-4-yl)-$C_{1-3}$-alkyl (especially (1,1-dioxidotetrahydro-thiopyran-4-yl)-methyl), $C_{1-3}$-alkoxy-carbonyl-$C_{1-3}$-alkyl (especially methoxy-carbonyl-methyl) or $C_{1-3}$-alkyl-carbonyl (especially acetyl);

[In a sub-embodiment, when **$R^2$** represents phenyl or 6-membered heteroaryl, wherein said phenyl or 6-membered heteroaryl are independently mono-, di- or tri-substituted, one substituent (especially hydroxy, **-N$R^{N1}$$R^{N2}$**, -(C=O)-N**$R^{N3}$$R^{N4}$** and **-O$R^6$**) is attached in para-position with regard to the point of attachment to the rest of the molecule]

- 9- to 10-membered bicyclic heteroaryl containing 1, 2, 3 or 4 ring heteroatoms independently selected from oxygen, nitrogen and sulphur (especially 1, 2, 3 or 4 ring nitrogen atoms; notably quinolin-4-yl, quinolin-5-yl, isoquinolin-4-yl, isoquinolin-5-yl, isoquinolin-8-yl, indol-4-yl, indol-5-yl, indol-6-yl or indol-7-yl), wherein said 9- to 10-membered bicyclic heteroaryl is unsubstituted; or

- 2,3-dihydro-benzo[1,4]dioxin-6-yl, 1,3-dihydro-2H-benzoimidazol-2-one-5-yl or 3,4-dihydro-2H-pyrano[2,3-b]pyridine-6-yl.

21) A further embodiment of the present invention relates to compounds according to embodiment 1), wherein

**A** represents phenylene or 5- to 6-membered heteroarylene (especially 5-membered heteroarylene), wherein said phenylene or 5- to 6-membered heteroarylene independently are unsubstituted, mono- or di-substituted (especially unsubstituted or mono- substituted), wherein the substituents are independently selected from $C_{1-4}$-alkyl (especially methyl);

**n** represents 1 or 0 (especially n represents 0) (i.e. when n=0, **R**$^2$ is directly attached to **A**; or when n=1, **R**$^2$ is directly attached to -$CH_2$-**A**);

**R**$^1$ represents:

- $C_{1-4}$-alkyl (especially methyl or ethyl);

- $C_{1-3}$-fluoroalkyl (especially trifluoromethyl);

- phenyl, which is unsubstituted or mono, di- or tri-substituted (especially unsubstituted, mono-, or di-substituted), wherein the substituents are independently selected from $C_{1-3}$-alkyl (especially methyl) and halogen (especially fluorine);

- $C_{3-6}$-cycloalkyl (especially cyclopropyl or cyclopentyl), which is unsubstituted or mono, di- or tri-substituted (especially unsubstituted or mono-substituted), wherein the substituents are independently selected from $C_{1-3}$-alkyl (especially methyl) and halogen (especially fluorine);

$R^2$ represents:

- phenyl or 6-membered heteroaryl (especially pyridinyl, pyrimidinyl or pyridazinyl), which are independently unsubstituted, or mono-, di- or tri-substituted, wherein the substituents are independently selected from:

  ➢ $C_{1-4}$-alkyl (especially methyl), cyano, halogen (especially chlorine, fluorine, ), hydroxy, $C_{1-3}$-fluoroalkyl (especially trifluoromethyl), $C_{1-3}$-fluoroalkoxy (especially trifluoromethoxy or 2,2,2-trifuloroethoxy), $C_{1-4}$-alkoxy (especially methoxy, ethoxy, iso-propoxy), $C_{3-5}$-cycloalkoxy (especially cyclobutoxy), hydroxymethyl-cyclopropyl (especially 1-(hydroxymethyl)-cycloprop-1-yl), $C_{1-3}$-alkyl-carbonyl (especially acetyl), $C_{1-4}$-alkoxy-carbonyl (especially methoxy-carbonyl), morpholin-4-yl, morpholin-4-yl-methyl;

  ➢ **-NR$^{N1}$R$^{N2}$**, wherein **R$^{N1}$** represents hydrogen and **R$^{N2}$** represents -(C=O)**-R$^{CO}$**, wherein **R$^{CO}$** represents $C_{1-4}$-alkyl (especially methyl, iso-propyl or tert-butyl), $C_{1-4}$-alkoxy (especially methoxy or tert-butoxy), amino, $C_{1-4}$-alkylamino (especially ethylamino or n-butylamino) or morpholin-4-yl;

  ➢ -(C=O)-N**R$^{N3}$R$^{N4}$**, wherein **R$^{N3}$** represents hydrogen or $C_{1-3}$-alkyl (especially methyl), and **R$^{N4}$** represents hydrogen, $C_{1-3}$-alkyl (especially methyl or ethyl), $C_{3-5}$-cycloalkyl (especially cyclopropyl), hydroxy-$C_{2-4}$-alkyl (especially 2-hydroxyethyl or 3-hydroxypropyl), $C_{1-3}$-alkoxy-$C_{2-4}$-alkyl (especially 2-methoxyethyl or 3-methoxypropyl), cyano-$C_{1-3}$-alkyl (especially 2-cyanoethyl), $C_{3-4}$-alkenyl (especially prop-1-en-3-yl), furanyl-$C_{1-3}$-alkyl (especially furan-2-yl-methyl), 2-di-$C_{1-3}$-alkylamino-$C_{2-3}$-alkyl (especially dimethylaminoethyl); or wherein **R$^{N3}$** and **R$^{N4}$** together with the nitrogen atom to which they are attached form a morpholine ring; and

  ➢ **-OR$^6$**, wherein **R$^6$** represents $C_{1-3}$-alkoxy-$C_{2-4}$-alkyl (especially 2-methoxyethyl), hydroxy-$C_{2-5}$-alkyl (especially 3-hydroxypropyl, 2-hydroxy-2-methylpropyl, 2-hydroxyethyl, 3-hydroxy-2,2-dimethylpropyl or 3-hydroxy-3-methylbutyl), $C_{3-5}$-cycloalkyl-$C_{1-3}$-alkyl (especially cyclopropyl-methyl), oxetan-3-yl-$C_{1-3}$-alkyl (especially oxetan-3-yl-methyl), ($C_{1-3}$-alkyl-oxetan-3-yl)-$C_{1-3}$-alkyl (especially (3-methyloxetan-3-yl)-methyl), (fluoro-oxetan-3-yl)-$C_{1-3}$-alkyl (especially (3-fluorooxetan-3-yl)-methyl), tetrahydrofuranyl-$C_{1-3}$-alkyl (especially tetrahydrofuran-2-yl-methyl), hydroxy-$C_{3-5}$-cycloalkyl-$C_{1-3}$-alkyl (especially (1-hydroxycyclopropyl)-ethyl), tetrahydropyranyl-$C_{1-3}$-alkyl (especially tetrahydropyran-2-yl-methyl or tetrahydropyran-4-yl-methyl), benzyl, (hydroxy-$C_{2-4}$-alkoxy)-$C_{2-4}$-alkyl (especially 2-(2-hydroxyethoxy)-ethyl), piperidin-1-yl-$C_{1-3}$-alkyl (especially 2-(piperidin-1-yl)-ethyl), (1-methyl-piperidin-3-yl)-$C_{1-3}$-alkyl (especially (1-methyl-piperidin-3-yl)-methyl)), (1,1-dioxidotetrahydro-thiopyran-4-yl)-$C_{1-3}$-alkyl (especially (1,1-dioxidotetrahydro-thiopyran-4-yl)-methyl), $C_{1-3}$-alkoxy-carbonyl-$C_{1-3}$-alkyl (especially methoxy-carbonyl-methyl) or $C_{1-3}$-alkyl-carbonyl (especially acetyl);

- 5-membered heteroaryl (especially pyrazolyl or triazolyl) which independently is unsubstituted, or mono-, di- or tri-substituted (especially unsubstituted, mono-, or di-substituted), wherein the substituents are independently selected from:

  ➢ $C_{1-4}$-alkyl (especially methyl), cyano, halogen (especially chlorine or fluorine), $C_{1-3}$-fluoroalkyl (especially trifluoromethyl), $C_{1-3}$-fluoroalkoxy (especially trifluoromethoxy or 2,2,2-trifuloroethoxy), $C_{1-4}$-alkoxy (especially methoxy, ethoxy or iso-propoxy), $C_{3-5}$-cycloalkoxy (especially cyclobutoxy), hydroxymethyl-cyclopropyl (especially 1-(hydroxymethyl)-cycloprop-1-yl), morpholin-4-yl, morpholin-4-yl-methyl, $C_{1-3}$-alkyl-carbonyl (especially acetyl), $C_{1-4}$-alkoxy-carbonyl (especially methoxy-carbonyl);

  ➢ **-NR$^{N1}$R$^{N2}$**, wherein **R$^{N1}$** represents hydrogen and **R$^{N2}$** represents -(C=O)-**R**$^{CO}$, wherein **R$^{CO}$** represents $C_{1-4}$-alkoxy (especially methoxy); or

  ➢ **-OR$^6$**, wherein **R$^6$** represents $C_{3-5}$-cycloalkyl-$C_{1-3}$-alkyl (especially cyclopropyl-methyl), benzyl or $C_{1-3}$-alkoxy-carbonyl-$C_{1-3}$-alkyl (especially methoxy-carbonyl-methyl);

- 9- to 10-membered bicyclic heteroaryl (especially quinolinyl, isoquinolinyl or indolyl), wherein said 9- to 10-membered bicyclic heteroaryl independently is unsubstituted, or mono-, di- or tri-substituted (especially unsubstituted), wherein the substituents are independently selected from:

  ➢ $C_{1-4}$-alkyl, cyano, halogen, $C_{1-3}$-fluoroalkyl, $C_{1-3}$-fluoroalkoxy, $C_{1-4}$-alkoxy, hydroxymethyl-cyclopropyl (especially 1-(hydroxymethyl)-cycloprop-1-yl), morpholin-4-yl, morpholin-4-yl-methyl, $C_{1-3}$-alkyl-carbonyl

(especially acetyl), $C_{1-4}$-alkoxy-carbonyl (especially methoxy-carbonyl);

➢ N$R^{N1}R^{N2}$, wherein $R^{N1}$ represents hydrogen and $R^{N2}$ represents -(C=O)-$R^{CO}$, wherein $R^{CO}$ represents $C_{1-4}$-alkoxy (especially methoxy); or

➢ **-OR⁶**, wherein $R^6$ represents $C_{3-5}$-cycloalkyl-$C_{1-3}$-alkyl (especially cyclopropyl-methyl); or

• 2,3-dihydro-benzo[1,4]dioxin-6-yl, 1,3-dihydro-2H-benzoimidazol-2-one-5-yl or 3,4-dihydro-2H-pyrano[2,3-b]pyridine-6-yl.

[In a sub-embodiment of embodiment 21), when $R^2$ represents phenyl or 6-membered heteroaryl, wherein said phenyl or 6-membered heteroaryl are independently mono-, di- or tri-substituted, one substituent (especially hydroxy, **-N**$R^{N1}R^{N2}$, -(C=O)-N$R^{N3}R^{N4}$ or **-O**$R^6$) is attached in para-position with regard to the point of attachment to the rest of the molecule]

22) A further embodiment of the present invention relates to the compounds of embodiment 1), wherein A represents

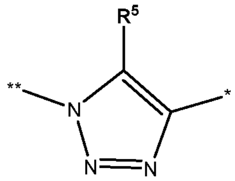
,

wherein one asterisk "*" denotes the point of attachment to the carbon bearing the OH group; two asterisks "**" denote the point of attachment to $R^2$-$(CH_2)_n$; **n** represents 1 or 0 (i.e. when n=0, $R^2$ is directly attached to the nitrogen atom of the triazole ring); $R^5$ represents hydrogen; $R^1$ represents $C_{1-4}$-alkyl, $C_{1-3}$-fluoroalkyl or $C_{3-6}$-cycloalkyl, wherein said $C_{3-6}$-cycloalkyl is unsubstituted or mono, di- or tri-substituted, wherein the substituents independently represent methyl or fluorine; and $R^2$ represents phenyl, 5- to 6-membered heteroaryl, or 9- to 10-membered bicyclic heteroaryl, wherein said phenyl, 5- to 6-membered heteroaryl and 9- to 10-membered bicyclic heteroaryl independently are unsubstituted, or mono-, di- or tri-substituted, wherein the substituents independently represent cyano, halogen $C_{1-4}$-alkyl, $C_{1-3}$-fluoroalkyl, $C_{1-4}$-alkoxy, $C_{1-3}$-fluoroalkoxy, 1-(hydroxymethyl)-cycloprop-1-yl, morpholin-4-yl, morpholin-4-yl-methyl, cyclopropyl-methoxy, acetyl, or - N$R^{N1}R^{N2}$ , wherein $R^{N1}$ represents hydrogen and $R^{N2}$ represents -(C=O)-$R^{CO}$, wherein $R^{CO}$ represents $C_{1-4}$-alkoxy; or $R^2$ represents 2,3-dihydro-benzo[1,4]dioxin-6-yl.

23) A further embodiment relates to compounds according to embodiment 1), wherein
**A** represents

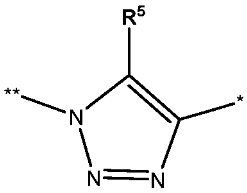
,

wherein one asterisk "*" denotes the point of attachment to the carbon bearing the OH group; two asterisks "**" denote the point of attachment to $R^2$-$(CH_2)_n$; $R^5$ represents hydrogen or $C_{1-3}$-alkyl (especially methyl);

**n** represents 0;

$R^1$ represents

- $C_{1-3}$-alkyl (especially ethyl);
- -C($R^A$)=C($R^B$)($R^C$), wherein $R^A$, $R^B$ and $R^C$ are independently selected from hydrogen and methyl (especially 1-methylvinyl);

- $C_{1-3}$-fluoroalkyl (especially 1,1-difluoroethyl);
- phenyl, which is unsubstituted or mono-substituted (especially unsubstituted) with halogene (especially fluorine); or
- cyclopropyl, which is unsubstituted or mono-substituted (especially unsubstituted) with methyl or halogene (especially fluorine); and

**$R^2$** represents

- phenyl which is unsubstituted, mono-, di-, or tri-substituted, wherein one substituent independently represents

  ➢ **-OR$^6$**, wherein **$R^6$** represents hydrogen, $C_{1-3}$-alkyl (especially methyl or ethyl), $C_{1-3}$-alkoxy-$C_{2-4}$-alkyl (especially 2-methoxyethyl), hydroxy-$C_{2-5}$-alkyl (especially 3-hydroxypropyl, 2-hydroxy-2-methylpropyl, 2-hydroxyethyl, 3-hydroxy-2,2-dimethylpropyl or 3-hydroxy-3-methylbutyl), $C_{3-5}$-cycloalkyl-$C_{1-3}$-alkyl (especially cyclopropyl-methyl), oxetan-3-yl-$C_{1-3}$-alkyl (especially oxetan-3-yl-methyl), (fluoro-oxetan-3-yl)-$C_{1-3}$-alkyl (especially (3-fluorooxetan-3-yl)-methyl), hydroxy-$C_{3-5}$-cycloalkyl-$C_{1-3}$-alkyl (especially (1-hydroxycyclopropyl)-ethyl), tetrahydropyranyl-$C_{1-3}$-alkyl (especially tetrahydropyran-2-yl-methyl or tetrahydropyran-4-yl-methyl), $C_{1-3}$-fluoroalkoxy-$C_{2-3}$-alkyl (especially 2-(2,2,2-trifluoroethoxy)-ethyl) or benzyl; or

  ➢ **-NR$^{N1}$R$^{N2}$**, wherein **$R^{N1}$** represents hydrogen; **$R^{N2}$** represents hydrogen or -(C=O)**-$R^{CO}$**, wherein **$R^{CO}$** represents $C_{1-4}$-alkyl (especially methyl, iso-propyl or tert-butyl), $C_{1-4}$-alkoxy (especially methoxy or tert-butoxy) or phenyl;

  said one substituent being attached to said phenyl in para-position with regard to the point of attachment to the rest of the molecule and wherein any further substituent(s) of said phenyl ring, if present, independently represent halogen (especially chlorine or fluorine), cyano or nitro.

24) Another embodiment relates to compounds according to any one of embodiments 1) to 23), wherein the asymmetric carbon atom to which **$R^2$**-$(CH_2)_n$**-A-** is attached has the absolute configuration depicted in Formula (II)

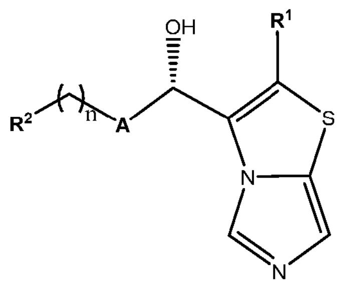

Formula (II)

It is understood that when **A** represents substituted or unsubstituted [1,2,3]triazol-1,4-diyl as defined hereinabove (especially the following group:

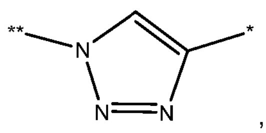

,

wherein one asterisk "*" denotes the point of attachment to the asymmetric carbon atom bearing the OH group; and two asterisks "**" denote the point of attachment to **$R^2$**-$(CH_2)$n- as depicted in Formula (II)), said asymmetric carbon atom is in absolute R-configuration.
It is further understood that when A represents substituted or unsubstituted pyrazol-1,4-diyl as defined hereinabove

(especially the group:

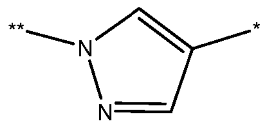
,

wherein one asterisk "*" denotes the point of attachment to the asymmetric carbon atom bearing the OH group; and two asterisks "**" denote the point of attachment to **R²**-$(CH_2)_n$- as depicted in Formula (II)), said asymmetric carbon atom is in absolute S-configuration.

25) Another embodiment relates to a compound of embodiment 1) selected from a group consisting of:

(2-methyl-imidazo[5,1-b]thiazol-3-yl)-(1-phenyl-1H-[1,2,3]triazol-4-yl)-methanol;
(2-ethyl-imidazo[5,1-b]thiazol-3-yl)-(1-phenyl-1H-[1,2,3]triazol-4-yl)-methanol;
(2-cyclopropyl-imidazo[5,1-b]thiazol-3-yl)-(1-phenyl-1H-[1,2,3]triazol-4-yl)-methanol;
(R)-(2-cyclopropyl-imidazo[5,1-b]thiazol-3-yl)-(1-phenyl-1H-[1,2,3]triazol-4-yl)-methanol;
(2-cyclopentyl-imidazo[5,1-b]thiazol-3-yl)-(1-phenyl-1H-[1,2,3]triazol-4-yl)-methanol;
(2-cyclopropyl-imidazo[5,1-b]thiazol-3-yl)-(1-o-tolyl-1H-[1,2,3]triazol-4-yl)-methanol;
(2-cyclopropyl-imidazo[5,1-b]thiazol-3-yl)-(1-m-tolyl-1H-[1,2,3]triazol-4-yl)-methanol;
(2-cyclopropyl-imidazo[5,1-b]thiazol-3-yl)-(1-p-tolyl-1H-[1,2,3]triazol-4-yl)-methanol;
(2-cyclopropyl-imidazo[5,1-b]thiazol-3-yl)-[1-(3-methoxy-phenyl)-1H-[1,2,3]triazol-4-yl]-methanol;
(R)-(2-cyclopropyl-imidazo[5,1-b]thiazol-3-yl)-[1-(4-methoxy-phenyl)-1H-[1,2,3]triazol-4-yl]-methanol;
(2-cyclopropyl-imidazo[5,1-b]thiazol-3-yl)-[1-(4-methoxy-phenyl)-1H-[1,2,3]triazol-4-yl]-methanol;
(2-cyclopropyl-imidazo[5,1-b]thiazol-3-yl)-[1-(2-methoxy-phenyl)-1H-[1,2,3]triazol-4-yl]-methanol;
(1-benzyl-1H-[1,2,3]triazol-4-yl)-(2-cyclopropyl-imidazo[5,1-b]thiazol-3-yl)-methanol;
3-{4-[(2-cyclopropyl-imidazo[5,1-b]thiazol-3-yl)-hydroxy-methyl]-[1,2,3]triazol-1-yl}-benzonitrile;
4-{4-[(2-cyclopropyl-imidazo[5,1-b]thiazol-3-yl)-hydroxy-methyl]-[1,2,3]triazol-1-yl}-benzonitrile;
(2-cyclopropyl-imidazo[5,1-b]thiazol-3-yl)-[1-(2-trifluoromethyl-phenyl)-1H-[1,2,3]triazol-4-yl]-methanol;
(2-cyclopropyl-imidazo[5,1-b]thiazol-3-yl)-[1-(3-trifluoromethyl-phenyl)-1H-[1,2,3]triazol-4-yl]-methanol;
(2-cyclopropyl-imidazo[5,1-b]thiazol-3-yl)-[1-(4-trifluoromethyl-phenyl)-1H-[1,2,3]triazol-4-yl]-methanol;
1-(4-{4-[(2-cyclopropyl-imidazo[5,1-b]thiazol-3-yl)-hydroxy-methyl]-[1,2,3]triazol-1-yl}-phenyl)-ethanone;
(2-cyclopropyl-imidazo[5,1-b]thiazol-3-yl)-[1-(4-cyclopropylmethoxy-phenyl)-1H-[1,2,3]triazol-4-yl]-methanol;
(2-cyclopropyl-imidazo[5,1-b]thiazol-3-yl)-[1-(4-ethoxy-phenyl)-1H-[1,2,3]triazol-4-yl]-methanol;
(4-{4-[(2-cyclopropyl-imidazo[5,1-b]thiazol-3-yl)-hydroxy-methyl]-[1,2,3]triazol-1-yl}-phenyl)-carbamic acid methyl ester;
(2-cyclopropyl-imidazo[5,1-b]thiazol-3-yl)-[1-(4-isopropoxy-phenyl)-1H-[1,2,3]triazol-4-yl]-methanol;
3-{4-[(R)-(2-cyclopropyl-imidazo[5,1-b]thiazol-3-yl)-hydroxy-methyl]-[1,2,3]triazol-1-yl}-benzonitrile;
[1-(4-methoxy-phenyl)-1H-[1,2,3]triazol-4-yl]-[2-(1-methyl-cyclopropyl)-imidazo[5,1-b]thiazol-3-yl]-methanol;
[1-(4-chloro-phenyl)-1H-[1,2,3]triazol4-yl]-(2-cyclopropyl-imidazo[5,1-b]thiazol-3-yl)-methanol;
(2-cyclopropyl-imidazo[5,1-b]thiazol-3-yl)-{1-[4-(1-hydroxymethyl-cyclopropyl)-phenyl]-1H-[1,2,3]triazol-4-yl}-methanol;
(2-cyclopropyl-imidazo[5,1-b]thiazol-3-yl)-[1-(4-morpholin-4-yl-phenyl)-1H-[1,2,3]triazol-4-yl]-methanol;
(2-cyclopropyl-imidazo[5,1-b]thiazol-3-yl)-[1-(4-morpholin-4-ylmethyl-phenyl)-1H-[1,2,3]triazol-4-yl]-methanol;
(2-cyclopropyl-imidazo[5,1-b]thiazol-3-yl)-[1-(4-trifluoromethoxy-phenyl)-1H-[1,2,3]triazol-4-yl]-methanol;
(2-cyclopropyl-imidazo[5,1-b]thiazol-3-yl)-(1-pyrimidin-5-yl-1H-[1,2,3]triazol-4-yl)-methanol;
(2-cyclopropyl-imidazo[5,1-b]thiazol-3-yl)-(1-pyridin-2-yl-1H-[1,2,3]triazol-4-yl)-methanol;
(2-cyclopropyl-imidazo[5,1-b]thiazol-3-yl)-(1-pyridin-3-yl-1H-[1,2,3]triazol-4-yl)-methanol;
(2-cyclopropyl-imidazo[5,1-b]thiazol-3-yl)-(1-pyridin-4-yl-1H-[1,2,3]triazol-4-yl)-methanol;
(2-cyclopropyl-imidazo[5,1-b]thiazol-3-yl)-[1-(6-ethoxy-pyridin-3-yl)-1H-[1,2,3]triazol-4-yl]-methanol;
(2-cyclopropyl-imidazo[5,1-b]thiazol-3-yl)-[1-(1-methyl-1H-pyrazol-4-yl)-1H-[1,2,3]triazol-4-yl]-methanol;
(2-cyclopropyl-imidazo[5,1-b]thiazol-3-yl)-[1-(1H-pyrazol-4-yl)-1H-[1,2,3]triazol-4-yl]-methanol;
(2-cyclopropyl-imidazo[5,1-b]thiazol-3-yl)-[1-(1H-indol-6-yl)-1H-[1,2,3]triazol-4-yl]-methanol;
(2-cyclopropyl-imidazo[5,1-b]thiazol-3-yl)-[1-(1H-indol-4-yl)-1H-[1,2,3]triazol-4-yl]-methanol;
(R)-(2-cyclopropyl-imidazo[5,1-b]thiazol-3-yl)-[1-(1H-indol-5-yl)-1H-[1,2,3]triazol-4-yl]-methanol;
(2-cyclopropyl-imidazo[5,1-b]thiazol-3-yl)-[1-(1H-indol-5-yl)-1H-[1,2,3]triazol-4-yl]-methanol;
(2-cyclopropyl-imidazo[5,1-b]thiazol-3-yl)-[1-(1H-indol-7-yl)-1H-[1,2,3]triazol-4-yl]-methanol;

(2-cyclopropyl-imidazo[5,1-b]thiazol-3-yl)-(1-isoquinolin-5-yl-1H-[1,2,3]triazol-4-yl)-methanol;
(2-cyclopropyl-imidazo[5,1-b]thiazol-3-yl)-(1-quinolin-5-yl-1H-[1,2,3]triazol-4-yl)-methanol;
(2-cyclopropyl-imidazo[5,1-b]thiazol-3-yl)-(1-isoquinolin-8-yl-1H-[1,2,3]triazol-4-yl)-methanol;
(2-cyclopropyl-imidazo[5,1-b]thiazol-3-yl)-(1-quinolin-4-yl-1H-[1,2,3]triazol-4-yl)-methanol;
(2-cyclopropyl-imidazo[5,1-b]thiazol-3-yl)-(1-isoquinolin-4-yl-1H-[1,2,3]triazol-4-yl)-methanol; and
(2-cyclopropyl-imidazo[5,1-b]thiazol-3-yl)-[1-(2,3-dihydro-benzo[1,4]dioxin-6-yl)-1H-[1,2,3]triazol4-yl]-methanol.

26) Another embodiment relates to a compound of embodiment 1) selected from a group consisting of:

[1-(4-benzyloxy-phenyl)-1*H*-[1,2,3]triazol-4-yl]-(2-cyclopropyl-imidazo[5,1-*b*]thiazol-3-yl)-methanol;
[1-(6-ethoxy-pyridin-3-yl)-1*H*-[1,2,3]triazol4-yl]-(2-ethyl-imidazo[5,1-*b*)]thiazol-3-yl)-methanol;
(2-ethyl-imidazo[5,1-*b*]thiazol-3-yl)-[1-(4-morpholin4-yl-phenyl)-1*H*-[1,2,3]triazol-4-yl]-methanol;
(2-ethyl-imidazo[5,1-*b*]thiazol-3-yl)-{1-[4-(1-hydroxymethyl-cyclopropyl)-phenyl]-1*H*-[1,2,3]triazol-4-yl}-methanol;
(4-{4-[(2-ethyl-imidazo[5,1-*b*]thiazol-3-yl)-hydroxy-methyl]-[1,2,3]triazol-1-yl}-phenyl)-carbamic acid methyl ester;
(2-ethyl-imidazo[5,1-*b*]thiazol-3-yl)-[1-(1*H*-indol-5-yl)-1*H*-[1,2,3]triazol-4-yl]-methanol;
3-{4-[(2-ethyl-imidazo[5,1-*b*]thiazol-3-yl)-hydroxy-methyl]-[1,2,3]triazol-1-yl}-benzonitrile;
(2-ethyl-imidazo[5,1-*b*]thiazol-3-yl)-(1-isoquinolin-4-yl-1*H*-[1,2,3]triazol-4-yl)-methanol;
(1-benzyl-1*H*-[1,2,3]triazol-4-yl)-(2-ethyl-imidazo[5,1-*b*]thiazol-3-yl)-methanol;
[1-(4-cyclopropylmethoxy-phenyl)-1*H*-[1,2,3]triazol-4-yl]-(2-ethyl-imidazo[5,1-*b*]thiazol-3-yl)-methanol;
[1-(6-ethoxy-pyridin-3-yl)-1*H*-[1,2,3]triazol-4-yl]-(2-methyl-imidazo[5,1-*b*]thiazol-3-yl)-methanol;
(2-methyl-imidazo[5,1-*b*]thiazol-3-yl)-[1-(4-morpholin-4-yl-phenyl)-1*H*-[1,2,3]triazol-4-yl]-methanol;
{1-[4-(1-hydroxymethyl-cyclopropyl)-phenyl]-1*H*-[1,2,3]triazol-4-yl}-(2-methyl-imidazo[5,1-*b*]thiazol-3-yl)-methanol;
(4-{4-[hydroxy-(2-methyl-imidazo[5,1-b]thiazol-3-yl)-methyl]-[1,2,3]triazol-1-yl}-phenyl)-carbamic acid methyl ester;
[1-(1*H*-Indol-5-yl)-1*H*-[1,2,3]triazol-4-yl]-(2-methyl-imidazo[5,1-*b*]thiazol-3-yl)-methanol;
3-{4-[hydroxy-(2-methyl-imidazo[5,1-*b*]thiazol-3-yl)-methyl]-[1,2,3]triazol-1-yl}-benzonitrile;
(1-isoquinolin-4-yl-1*H*-[1,2,3]thazol-4-yl)-(2-methyl-imidazo[5,1-*b*]thiazol-3-yl)-methanol;
(1-benzyl-1*H*-[1,2,3]triazol-4-yl)-(2-methyl-imidazo[5,1-*b*]thiazol-3-yl)-methanol;
[1-(4-cyclopropylmethoxy-phenyl)-1*H*-[1,2,3]triazol-4-yl]-(2-methyl-imidazo[5,1-*b*]thiazol-3-yl)-methanol;
[2-(3-fluoro-phenyl)-imidazo[5,1-*b*]thiazol-3-yl]-[1-(4-methoxy-phenyl)-1*H*-[1,2,3]triazol-4-yl]-methanol;
(2-cyclopropyl-imidazo[5,1-*b*]thiazol-3-yl)-[1-(2-ethoxy-pyrimidin-5-yl)-1*H*-[1,2,3]triazol-4-yl]-methanol;
(2-cyclopropyl-imidazo[5,1-*b*]thiazol-3-yl)-[1-(5,6-dimethoxy-pyridin-2-yl)-1*H*-[1,2,3]triazol-4-yl]-methanol;
(2-cyclopropyl-imidazo[5,1-*b*]thiazol-3-yl)-[1-(2-methoxy-pyrimidin-5-yl)-1*H*-[1,2,3]triazol-4-yl]-methanol;
4-{4-[(2-cyclopropyl-imidazo[5,1-*b*]thiazol-3-yl)-hydroxy-methyl]-[1,2,3]triazol-1-yl}-phenol;
(2-cyclopropyl-imidazo[5,1-*b*]thiazol-3-yl)-[1-(6-methoxy-pyridin-3-yl)-1*H*-[1,2,3]triazol-4-yl]-methanol;
5-{4-[(2-cyclopropyl-imidazo[5,1-b]thiazol-3-yl)-hydroxy-methyl]-[1,2,3]triazol-1-yl}-2-methoxy-nicotinic acid methyl ester;
(2-cyclopropyl-imidazo[5,1-*b*]thiazol-3-yl)-[1-(6-methoxy-5-methyl-pyridin-3-yl)-1*H*-[1,2,3]triazol-4-yl]-methanol;
(2-cyclopropyl-imidazo[5,1-*b*]thiazol-3-yl)-[1-(3,4-dihydro-2*H*-pyrano[2,3-b]pyridin-6-yl)-1*H*-[1,2,3]triazol-4-yl]-methanol;
[1-(5-chloro-6-isopropoxy-pyridin-3-yl)-1*H*-[1,2,3]triazol4-yl]-(2-cyclopropyl-imidazo[5,1-*b*)]thiazol-3-yl)-methanol;
[1-(5-chloro-6-ethoxy-pyridin-3-yl)-1*H*-[1,2,3]triazol-4-yl]-(2-cyclopropyl-imidazo[5,1-*b*]thiazol-3-yl)-methanol;
(5-{4-[(2-cyclopropyl-imidazo[5,1-b]thiazol-3-yl)-hydroxy-methyl]-[1,2,3]triazol-1-yl}-pyridin-2-yloxy)-acetic acid methyl ester;
(2-cyclopropyl-imidazo[5,1-*b*]thiazol-3-yl)-[1-(6-ethoxy-5-trifluoromethyl-pyridin-3-yl)-1*H*-[1,2,3]triazol-4-yl]-methanol;
[1-(6-benzyloxy-pyridin-3-yl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-{1-[6-(2,2,2-trifluoro-ethoxy)-pyridin-3-yl]-1*H*-[1,2,3]triazol-4-yl}-methanol;
(2-cyclopropyl-imidazo[5,1-*b*]thiazol-3-yl)-[1-(5-fluoro-6-methoxy-pyridin-3-yl)-1*H*-[1,2,3]triazol4-yl]-methanol;
(2-cyclopropyl-imidazo[5,1-*b*]thiazol-3-yl)-[1-(5,6-dimethoxy-pyridin-3-yl)-1*H*-[1,2,3]triazol-4-yl]-methanol;
(2-cyclopropyl-imidazo[5,1-*b*]thiazol-3-yl)-[1-(2,6-dimethoxy-pyridin-3-yl)-1*H*-[1,2,3]triazol-4-yl]-methanol;

(2-cyclopropyl-imidazo[5,1-*b*]thiazol-3-yl)-[1-(6-isopropoxy-pyridin-3-yl)-1*H*-[1,2,3]triazol-4-yl]-methanol;
[1-(2-cyclobutoxy-pyrimidin-5-yl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[1-(2,4-dimethoxy-pyrimidin-5-yl)-1*H*-[1,2,3]triazol-4-yl]-methanol;
[1-(2-benzyloxy-pyrimidin-5-yl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[1-(3,6-dimethoxy-pyridazin-4-yl)-1*H*-[1,2,3]triazol-4-yl]-methanol;
(2-cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol;
(2-cyclopropyl-imidazo[5,1-*b*]thiazol-3-yl)-{1-[4-(oxetan-3-ylmethoxy)-phenyl]-1*H*-[1,2,3]triazol4-yl}-methanol;
(2-cyclopropyl-imidazo[5,1-*b*]thiazol-3-yl)-{1-[4-(2-piperidin-1-yl-ethoxy)-phenyl]-1*H*-[1,2,3]triazol-4-yl}-methanol;
(2-cyclopropyl-imidazo[5,1-*b*]thiazol-3-yl)-{1-[4-(2-methoxy-ethoxy)-phenyl]-1*H*-[1,2,3]triazol-4-yl}-methanol;
[2-(1-fluoro-cyclopropyl)-imidazo[5,1-*b*]thiazol-3-yl]-[1-(4-methoxy-phenyl)-1*H*-[1,2,3]triazol-4-yl]-methanol;
*N*-(4-{4-[(2-cyclopropyl-imidazo[5,1-*b*]thiazol-3-yl)-hydroxy-methyl]-[1,2,3]triazol-1-yl}-phenyl)-acetamide;
(2-methyl-imidazo[5,1-*b*]thiazol-3-yl)-(1-phenyl-1*H*-pyrazol-4-yl)-methanol;
[1-(4-methoxy-phenyl)-1*H*-pyrazol-4-yl]-(2-methyl-imidazo[5,1-b]thiazol-3-yl)-methanol;
(2-methyl-imidazo[5,1-*b*]thiazol-3-yl)-(5-methyl-1-phenyl-1*H*-pyrazol-4-yl)-methanol;
(2-Cyclopropyl-imidazo[5,1-*b*]thiazol-3-yl)-[1-(4-methoxy-phenyl)-1*H*-pyrazol-4-yl]-methanol;
(2-cyclopropyl-imidazo[5,1-*b*]thiazol-3-yl)-(1-phenyl-1*H*-pyrazol-4-yl)-methanol;
(*S*)-(2-cyclopropyl-imidazo[5,1-*b*]thiazol-3-yl)-(1-phenyl-1*H*-pyrazol-4-yl)-methanol;
(2-Cyclopropyl-imidazo[5,1-*b*]thiazol-3-yl)-(5-methyl-1-phenyl-1*H*-[1,2,3]triazol4-yl)-methanol;
4-{4-[(2-cyclopropyl-imidazo[5,1-*b*]thiazol-3-yl)-hydroxy-methyl]-[1,2,3]triazol-1-yl}-*N*-(2-methoxy-ethyl)-benzamide;
(4-{4-[(2-cyclopropyl-imidazo[5,1-b]thiazol-3-yl)-hydroxy-methyl]-[1,2,3]triazol-1-yl}-phenyl)-morpholin-4-yl-methanone;
morpholine-4-carboxylic acid (4-{4-[(2-cyclopropyl-imidazo[5,1-*b*]thiazol-3-yl)-hydroxy-methyl]-[1,2,3]triazol-1 -yl}-phenyl)-amide;
*N*-cyclopropyl-4-{4-[(2-cyclopropyl-imidazo[5,1-*b*]thiazol-3-yl)-hydroxy-methyl]-[1,2,3]triazol-1-yl}-benzamide;
4-{4-[(2-cyclopropyl-imidazo[5,1-*b*]thiazol-3-yl)-hydroxy-methyl]-[1,2,3]triazol-1-yl}-N-(3-methoxy-propyl)-benzamide;
4-{4-[(2-cyclopropyl-imidazo[5,1-*b*]thiazol-3-yl)-hydroxy-methyl]-[1,2,3]triazol-1-yl}-*N*-ethyl-benzamide;
*N*-(2-cyano-ethyl)-4-{4-[(2-cyclopropyl-imidazo[5,1-*b*]thiazol-3-yl)-hydroxy-methyl]-[1,2,3]triazol-1-yl}-benzamide;
(4-{4-[(2-cyclopropyl-imidazo[5,1-*b*]thiazol-3-yl)-hydroxy-methyl]-[1,2,3]triazol-1-yl}-phenyl)-urea;
*N*-(4-{4-[(2-ethyl-imidazo[5,1-*b*]thiazol-3-yl)-hydroxy-methyl]-[1,2,3]triazol-1-yl}-phenyl)-acetamide;
4-{4-[(2-ethyl-imidazo[5,1-*b*]thiazol-3-yl)-hydroxy-methyl]-[1,2,3]triazol-1-yl}-*N*-methyl-benzamide;
5-{4-[(2-ethyl-imidazo[5,1-*b*]thiazol-3-yl)-hydroxy-methyl]-[1,2,3]triazol-1-yl}-1,3-dihydro-benzoimidazol-2-one;
1-ethyl-3-(4-{4-[(2-ethyl-imidazo[5,1-*b*]thiazol-3-yl)-hydroxy-methyl]-[1,2,3]triazol-1-yl}-2-methoxy-phenyl)-urea;
4-{4-[(2-ethyl-imidazo[5,1-*b*]thiazol-3-yl)-hydroxy-methyl]-[1,2,3]triazol-1-yl}-*N*,*N*-dimethyl-benzamide;
4-{4-[(2-ethyl-imidazo[5,1-*b*]thiazol-3-yl)-hydroxy-methyl]-[1,2,3]triazol-1-yl}-*N*-(3-hydroxy-propyl)-benzamide;
4-{4-[(2-ethyl-imidazo[5,1-*b*]thiazol-3-yl)-hydroxy-methyl]-[1,2,3]triazol-1-yl}-benzamide;
(4-{4-[(2-ethyl-imidazo[5,1-*b*]thiazol-3-yl)-hydroxy-methyl]-[1,2,3]triazol-1-yl}-phenyl)-morpholin-4-yl-methanone;
4-{4-[(2-ethyl-imidazo[5,1-*b*]thiazol-3-yl)-hydroxy-methyl]-[1,2,3]triazol-1-yl}-*N*-(2-hydroxy-ethyl)-benzamide;
*N*-(2-dimethylamino-ethyl)-4-{4-[(2-ethyl-imidazo[5,1-*b*]thiazol-3-yl)-hydroxy-methyl]-[1,2,3]triazol-1-yl}-benzamide;
*N*-(4-{4-[(2-ethyl-imidazo[5,1-*b*]thiazol-3-yl)-hydroxy-methyl]-[1,2,3]triazol-1-yl}-phenyl)-2,2-dimethyl-propionamide;
morpholine-4-carboxylic acid (4-{4-[(2-ethyl-imidazo[5,1-*b*]thiazol-3-yl)-hydroxy-methyl]-[1,2,3]triazol-1-yl}-phenyl)-amide;
1-butyl-3-(4-{4-[(2-ethyl-imidazo[5,1-*b*]thiazol-3-yl)-hydroxy-methyl]-[1,2,3]triazol-1-yl}-phenyl)-urea;
4-{4-[(2-ethyl-imidazo[5,1-*b*]thiazol-3-yl)-hydroxy-methyl]-[1,2,3]triazol-1-yl}-*N*-furan-2-ylmethyl-benzamide;
*N*-ethyl-4-{4-[(2-ethyl-imidazo[5,1-*b*]thiazol-3-yl)-hydroxy-methyl]-[1,2,3]triazol-1-yl}-*N*-methyl-benzamide;
*N*-cyclopropyl-4-{4-[(2-ethyl-imidazo[5,1-*b*]thiazol-3-yl)-hydroxy-methyl]-[1,2,3]triazol-1-yl}-benzamide;
4-{4-[(2-ethyl-imidazo[5,1-*b*]thiazol-3-yl)-hydroxy-methyl]-[1,2,3]triazol-1-yl}-*N*-(3-methoxy-propyl)-benzamide;
N-(4-{4-[(2-ethyl-imidazo[5,1-*b*]thiazol-3-yl)-hydroxy-methyl]-[1,2,3]triazol-1-yl}-phenyl)-isobutyramide;
*N*-ethyl-4-{4-[(2-ethyl-imidazo[5,1-*b*]thiazol-3-yl)-hydroxy-methyl]-[1,2,3]triazol-1-yl}-benzamide;
*N*-allyl-4-{4-[(2-ethyl-imidazo[5,1-*b*]thiazol-3-yl)-hydroxy-methyl]-[1,2,3]triazol-1-yl}-benzamide;

*N*-(2-cyano-ethyl)-4-{4-[(2-ethyl-imidazo[5,1-*b*]thiazol-3-yl)-hydroxy-methyl]-[1,2,3]triazol-1-yl}-benzamide;
(4-{4-[(2-ethyl-imidazo[5,1-*b*]thiazol-3-yl)-hydroxy-methyl]-[1,2,3]triazol-1-yl}-phenyl)-urea;
(4-{4-[(2-cyclopropyl-imidazo[5,1-b]thiazol-3-yl)-hydroxy-methyl]-pyrazol-1-yl}-phenyl)-carbamic acid methyl ester;
(*R*)-(2-cyclopropyl-imidazo[5,1-*b*]thiazol-3-yl)-[1-(4-cyclopropylmethoxy-phenyl)-1*H*-[1,2,3]triazol-4-yl]-methanol;
(2-cyclopropyl-imidazo[5,1-*b*]thiazol-3-yl)-{1-[4-(tetrahydro-furan-2-ylmethoxy)-phenyl]-1*H*-[1,2,3]triazol-4-yl}-methanol;
(1-phenyl-1*H*-[1,2,3]triazol-4-yl)-(2-trifluoromethyl-imidazo[5,1-*b*]thiazol-3-yl)-methanol;
[1-(4-methoxy-phenyl)-1*H*-[1,2,3]triazol-4-yl]-(2-trifluoromethyl-imidazo[5,1-*b*]thiazol-3-yl)-methanol;
5-{4-[(2-cyclopropyl-imidazo[5,1-*b*]thiazol-3-yl)-hydroxy-methyl]-[1,2,3]triazol-1-yl}-2-methoxy-benzonitrile;
2-(4-{4-[(2-cyclopropyl-imidazo[5,1-b]thiazol-3-yl)-hydroxy-methyl]-[1,2,3]triazol-1-yl}-phenoxy)-ethanol;
3-(4-{4-[(2-cyclopropyl-imidazo[5,1-*b*]thiazol-3-yl)-hydroxy-methyl]-[1,2,3]triazol-1-yl}-phenoxy)-propan-1-ol;
3-(4-{4-[(*R*)-(2-cyclopropyl-imidazo[5,1-*b*]thiazol-3-yl)-hydroxy-methyl]-[1,2,3]triazol-1-yl}-phenoxy)-propan-1-ol;
2-[2-(4-{4-[(2-cyclopropyl-imidazo[5,1-b]thiazol-3-yl)-hydroxy-methyl]-[1,2,3]triazol-1-yl}-phenoxy)-ethoxy]-ethanol;
1-(4-{4-[(2-cyclopropyl-imidazo[5,1-b]thiazol-3-yl)-hydroxy-methyl]-[1,2,3]triazol-1-yl}-phenoxy)-2-methyl-propan-2-ol;
4-(4-{4-[(2-cyclopropyl-imidazo[5,1-*b*]thiazol-3-yl)-hydroxy-methyl]-[1,2,3]triazol-1-yl}-phenoxy)-2-methyl-butan-2-ol;
3-(4-{4-[(2-cyclopropyl-imidazo[5,1-*b*]thiazol-3-yl)-hydroxy-methyl]-[1,2,3]triazol-1-yl}-phenoxy)-2,2-dimethyl-propan-1-ol;
1-[2-(4-{4-[(2-cyclopropyl-imidazo[5,1-*b*]thiazol-3-yl)-hydroxy-methyl]-[1,2,3]triazol-1-yl}-phenoxy)-ethyl]-cyclopropanol;
1-[2-(4-{4-[(R)-(2-cyclopropyl-imidazo[5,1-b]thiazol-3-yl)-hydroxy-methyl]-[1,2,3]triazol-1-yl}-phenoxy)-ethyl]-cyclopropanol;
(2-cyclopropyl-imidazo[5,1-*b*]thiazol-3-yl)-{1-[4-(tetrahydro-pyran-2-ylmethoxy)-phenyl]-1*H*-[1,2,3]triazol-4-yl}-methanol;
(2-cyclopropyl-imidazo[5,1-*b*]thiazol-3-yl)-{1-[4-(3-methyl-oxetan-3-ylmethoxy)-phenyl]-1*H*-[1,2,3]triazol-4-yl}-methanol;
(2-cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol;
(*R*)-(2-cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol;
(2-cyclopropyl-imidazo[5,1-*b*]thiazol-3-yl)-{1-[4-(1-methyl-piperidin-3-ylmethoxy)-phenyl]-1*H*-[1,2,3]triazol-4-yl}-methanol;
(2-cyclopropyl-imidazo[5,1-*b*]thiazol-3-yl)-{1-[4-(1,1-dioxo-hexahydro-1l6-thiopyran-4-ylmethoxy)-phenyl]-1*H*-[1,2,3]triazol-4-yl}-methanol;
2-chloro-4-{4-[(2-cyclopropyl-imidazo[5,1-*b*]thiazol-3-yl)-hydroxy-methyl]-[1,2,3]triazol-1-yl}-phenol;
2-chloro-4-{4-[(*R*)-(2-cyclopropyl-imidazo[5,1-*b*]thiazol-3-yl)-hydroxy-methyl]-[1,2,3]triazol-1-yl}-phenol;
4-{4-[(2-cyclopropyl-imidazo[5,1-*b*]thiazol-3-yl)-hydroxy-methyl]-[1,2,3]triazol-1-yl}-2,6-difluoro-phenol;
4-{4-[(2-cyclopropyl-imidazo[5,1-*b*]thiazol-3-yl)-hydroxy-methyl]-[1,2,3]triazol-1-yl}-2-fluoro-phenol;
(4-{4-[(2-cyclopropyl-imidazo[5,1-b]thiazol-3-yl)-hydroxy-methyl]-[1,2,3]triazol-1-yl}-3-fluoro-phenyl)-carbamic acid tert-butyl ester;
biphenyl-4-yl-(2-cyclopropyl-imidazo[5,1-*b*)]thiazol-3-yl)-methanol;
biphenyl-3-yl-(2-cyclopropyl-imidazo[5,1-*b*]thiazol-3-yl)-methanol;
(2-methyl-imidazo[5,1-*b*]thiazol-3-yl)-(5-phenyl-thiophen-2-yl)-methanol;
(2-methyl-imidazo[5,1-*b*]thiazol-3-yl)-(2-phenyl-thiazol-5-yl)-methanol;
(2-methyl-imidazo[5,1-*b*]thiazol-3-yl)-(3-phenyl-isoxazol-5-yl)-methanol;
(2-cyclopropyl-imidazo[5,1-*b*]thiazol-3-yl)-(5-phenyl-[1,3,4]oxadiazol-2-yl)-methanol;
(2-cyclopropyl-imidazo[5,1-*b*]thiazol-3-yl)-(2-phenyl-2*H*-[1,2,3]triazol-4-yl)-methanol;
3-{5-[(2-cyclopropyl-imidazo[5,1-*b*]thiazol-3-yl)-hydroxy-methyl]-thiophen-2-yl}-benzonitrile;
(2-cyclopropyl-imidazo[5,1-*b*]thiazol-3-yl)-(5-pyridin-3-yl-thiophen-2-yl)-methanol;
(2-cyclopropyl-imidazo[5,1-*b*]thiazol-3-yl)-[5-(4-methoxy-phenyl)-thiophen-2-yl]-methanol;
(2-cyclopropyl-imidazo[5,1-*b*]thiazol-3-yl)-[5-(4-trifluoromethyl-phenyl)-thiophen-2-yl]-methanol;
(2-cyclopropyl-imidazo[5,1-*b*]thiazol-3-yl)-(3-phenyl-[1,2,4]oxadiazol-5-yl)-methanol;
(2-cyclopropyl-imidazo[5,1-*b*]thiazol-3-yl)-(5-phenyl-thiophen-2-yl)-methanol;

(2-cyclopropyl-imidazo[5,1-*b*]thiazol-3-yl)-[5-(3-trifluoromethyl-phenyl)-thiophen-2-yl]-methanol;
(2-cyclopropyl-imidazo[5,1-b]thiazol-3-yl)-[5-(2-methoxy-phenyl)-thiophen-2-yl]-methanol;
(2-cyclopropyl-imidazo[5,1-*b*]thiazol-3-yl)-[5-(3-methoxy-phenyl)-thiophen-2-yl]-methanol;
(2-cyclopropyl-imidazo[5,1-*b*]thiazol-3-yl)-(5-pyridin4-yl-thiophen-2-yl)-methanol;
(2-cyclopropyl-imidazo[5,1-*b*]thiazol-3-yl)-[5-(2-methyl-2*H*-pyrazol-3-yl)-thiophen-2-yl]-methanol;
(2-cyclopropyl-imidazo[5,1-*b*]thiazol-3-yl)-[5-(2-trifluoromethyl-phenyl)-thiophen-2-yl]-methanol;
(2-cyclopropyl-imidazo[5,1-*b*]thiazol-3-yl)-(5-phenyl-thiazol-2-yl)-methanol;
2-{5-[(2-cyclopropyl-imidazo[5,1-*b*]thiazol-3-yl)-hydroxy-methyl]-thiophen-2-yl}-benzonitrile;
4-{5-[(2-cyclopropyl-imidazo[5,1-*b*]thiazol-3-yl)-hydroxy-methyl]-thiophen-2-yl}-benzonitrile;
(2-cyclopropyl-imidazo[5,1-*b*]thiazol-3-yl)-[5-(3-methyl-3*H*-[1,2,3]triazol-4-yl)-thiophen-2-yl]-methanol;
(2-cyclopropyl-imidazo[5,1-*b*]thiazol-3-yl)-(5-phenyl-[1,3,4]thiadiazol-2-yl)-methanol;
(2-cyclopropyl-imidazo[5,1-*b*]thiazol-3-yl)-[5-(1*H*-indol-5-yl)-thiophen-2-yl]-methanol;
(2-cyclopropyl-imidazo[5,1-*b*]thiazol-3-yl)-(5-isoquinolin-4-yl-thiophen-2-yl)-methanol;
(2-cyclopropyl-imidazo[5,1-*b*]thiazol-3-yl)-(1-phenyl-1*H*-imidazol-4-yl)-methanol;
[2-(cis-2-fluoro-cyclopropyl)-imidazo[5,1-*b*]thiazol-3-yl]-[1-(4-methoxy-phenyl)-1*H*-[1,2,3]triazol4-yl]-methanol; and
[2-(trans-2-fluoro-cyclopropyl)-imidazo[5,1-*b*]thiazol-3-yl]-[1-(4-methoxy-phenyl)-1*H*-[1,2,3]triazol-4-yl]-methanol.

27) Another embodiment relates to a compound of embodiment 1) selected from a group consisting of:

(4-{4-[(R)-(2-ethyl-imidazo[5,1-b]thiazol-3-yl)-hydroxy-methyl]-[1,2,3]triazol-1-yl}-phenyl)-carbamic acid methyl ester;
(*R*)-(2-cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol;
1-(4-{4-[(*R*)-(2-cyclopropyl-imidazo[5,1-*b*]thiazol-3-yl)-hydroxy-methyl]-[1,2,3]triazol-1-yl}-phenoxy)-2-methyl-propan-2-ol;
(*R*)-(2-cyclopropyl-imidazo[5,1-*b*]thiazol-3-yl)-(5-methyl-1-phenyl-1*H*-[1,2,3]triazol-4-yl)-methanol;
6-{4-[(2-cyclopropyl-imidazo[5,1-*b*]thiazol-3-yl)-hydroxy-methyl]-[1,2,3]triazol-1-yl}-3*H*-benzooxazol-2-one;
(4-{4-[(2-cyclopropyl-imidazo[5,1-b]thiazol-3-yl)-hydroxy-methyl]-[1,2,3]triazol-1-yl}-phenyl)-carbamic acid tert-butyl ester;
6-14-[(2-ethyl-imidazo[5,1-*b*]thiazol-3-yl)-hydroxy-methyl]-[1,2,3]triazol-1-yl}-3*H*-benzooxazol-2-one;
4-{4-[(2-cyclopropyl-imidazo[5,1-*b*]thiazol-3-yl)-hydroxy-methyl]-[1,2,3]triazol-1-yl}-*N*-methyl-benzamide;
1-(4-{4-[(2-cyclopropyl-imidazo[5,1-*b*]thiazol-3-yl)-hydroxy-methyl]-[1,2,3]triazol-1-yl}-2-methoxy-phenyl)-3-ethyl-urea;
4-{4-[(2-cyclopropyl-imidazo[5,1-*b*]thiazol-3-yl)-hydroxy-methyl]-[1,2,3]triazol-1-yl}-*N*-(3-hydroxy-propyl)-benzamide;
4-{4-[(2-cyclopropyl-imidazo[5,1-*b*]thiazol-3-yl)-hydroxy-methyl]-[1,2,3]triazol-1-yl}-benzamide;
4-{4-[(2-cyclopropyl-imidazo[5,1-*b*]thiazol-3-yl)-hydroxy-methyl]-[1,2,3]triazol-1-yl}-*N*-(2-hydroxy-ethyl)-benzamide;
*N*-(4-{4-[(2-cyclopropyl-imidazo[5,1-*b*]thiazol-3-yl)-hydroxy-methyl]-[1,2,3]triazol-1-yl}-phenyl)-2,2-dimethyl-propionamide;
1-butyl-3-(4-{4-[(2-cyclopropyl-imidazo[5,1-*b*]thiazol-3-yl)-hydroxy-methyl]-[1,2,3]thazol-1-yl}-phenyl)-urea;
*N*-(4-{4-[(2-cyclopropyl-imidazo[5,1-*b*]thiazol-3-yl)-hydroxy-methyl]-[1,2,3]triazol-1-yl}-phenyl)-benzamide;
4-{4-[(2-cyclopropyl-imidazo[5,1-*b*]thiazol-3-yl)-hydroxy-methyl]-[1,2,3]triazol-1 -yl}-*N*-furan-2-ylmethyl-benzamide;
4-{4-[(2-cyclopropyl-imidazo[5,1-*b*]thiazol-3-yl)-hydroxy-methyl]-[1,2,3]triazol-1-yl}-*N*-ethyl-*N*-methyl-benzamide;
*N*-(4-{4-[(2-cyclopropyl-imidazo[5,1-*b*]thiazol-3-yl)-hydroxy-methyl]-[1,2,3]triazol-1-yl}-phenyl)-isobutyramide;
*N*-(4-{4-[(*R*)-(2-cyclopropyl-imidazo[5,1-*b*]thiazol-3-yl)-hydroxy-methyl]-[1,2,3]thazol-1-yl}-phenyl)-isobutyramide;
*N*-allyl-4-{4-[(2-cyclopropyl-imidazo[5,1-*b*]thiazol-3-yl)-hydroxy-methyl]-[1,2,3]triazol-1-yl}-benzamide;
5-{4-[(2-cyclopropyl-imidazo[5,1-*b*]thiazol-3-yl)-hydroxy-methyl]-[1,2,3]triazol-1-yl}-1,3-dihydro-benzoimidazol-2-one;
4-{4-[(2-cyclopropyl-imidazo[5,1-*b*]thiazol-3-yl)-hydroxy-methyl]-[1,2,3]triazol-1-yl}-*N*,*N*-dimethyl-benzamide;
4-{4-[(2-ethyl-imidazo[5,1-*b*]thiazol-3-yl)-hydroxy-methyl]-[1,2,3]triazol-1-yl}-*N*-(2-methoxy-ethyl)-benzamide;
3-{4-[(2-cyclopropyl-imidazo[5,1-*b*]thiazol-3-yl)-hydroxy-methyl]-[1,2,3]triazol-1-yl}-5-methoxy-benzonitrile;
5-{4-[(2-cyclopropyl-imidazo[5,1-*b*]thiazol-3-yl)-hydroxy-methyl]-[1,2,3]triazol-1-yl}-2,3-dimethoxy-benzonitrile;

5-{4-[(2-cyclopropyl-imidazo[5,1-b]thiazol-3-yl)-hydroxy-methyl]-[1,2,3]triazol-1-yl}-2-trifluoromethoxy-benzonitrile;
[1-(4-bromo-3-chloro-pyridin-2-yl)-1*H*-[1,2,3]triazol-4-yl]-(2-cyclopropyl-imidazo[5,1-*b*]thiazol-3-yl)-methanol;
2-{2-[2-(4-{4-[(2-cyclopropyl-imidazo[5,1-b]thiazol-3-yl)-hydroxy-methyl]-[1,2,3]triazol-1-yl}-phenoxy)-ethoxy]-ethoxy}-ethanol;
(*S*)-3-(4-{4-[(*R*)-(2-cyclopropyl-imidazo[5,1-*b*]thiazol-3-yl)-hydroxy-methyl]-[1,2,3]triazol-1-yl}-phenoxy)-propane-1,2-diol;
(*S*)-3-(4-{4-[(*S*)-(2-cyclopropyl-imidazo[5,1-*b*]thiazol-3-yl)-hydroxy-methyl]-[1,2,3]triazol-1-yl}-phenoxy)-propane-1,2-diol;
(2-cyclopropyl-imidazo[5,1-*b*]thiazol-3-yl)-{1-[4-(pyridin-2-ylmethoxy)-phenyl]-1*H*-[1,2,3]triazol-4-yl}-methanol;
(2-cyclopropyl-imidazo[5,1-*b*]thiazol-3-yl)-{1-[4-(2-dimethylamino-ethoxy)-phenyl]-1*H*-[1,2,3]triazol-4-yl}-methanol;
(2-cyclopropyl-imidazo[5,1-*b*]thiazol-3-yl)-{1-[4-(2-methanesulfonyl-ethoxy)-phenyl]-1*H*-[1,2,3]triazol4-yl}-methanol;
3-(4-{4-[(2-cyclopropyl-imidazo[5,1-*b*]thiazol-3-yl)-hydroxy-methyl]-[1,2,3]triazol-1-yl}-phenoxy)-propane-1-sulfonic acid amide;
4-{4-[(2-cyclopropyl-imidazo[5,1-*b*]thiazol-3-yl)-hydroxy-methyl]-[1,2,3]triazol-1-yl}-3-methyl-phenol;
4-{4-[(2-cyclopropyl-imidazo[5,1-*b*]thiazol-3-yl)-hydroxy-methyl]-[1,2,3]triazol-1-yl}-3-fluoro-phenol;
4-{4-[(2-cyclopropyl-imidazo[5,1-b]thiazol-3-yl)-hydroxy-methyl]-[1,2,3]triazol-1-yl}-2-trifluoromethyl-phenol;
4-{4-[(2-cyclopropyl-imidazo[5,1-b]thiazol-3-yl)-hydroxy-methyl]-[1,2,3]triazol-1-yl}-2-trifluoromethoxy-phenol;
4-{4-[(2-cyclopropyl-imidazo[5,1-*b*]thiazol-3-yl)-hydroxy-methyl]-[1,2,3]triazol-1-yl}-2-methyl-phenol;
3-chloro-4-{4-[(2-cyclopropyl-imidazo[5,1-*b*]thiazol-3-yl)-hydroxy-methyl]-[1,2,3]triazol-1-yl}-phenol;
4-{4-[(2-cyclopropyl-imidazo[5,1-*b*]thiazol-3-yl)-hydroxy-methyl]-[1,2,3]triazol-1-yl}-3-trifluoromethoxy-phenol;
4-{4-[(2-cyclopropyl-imidazo[5,1-*b*]thiazol-3-yl)-hydroxy-methyl]-[1,2,3]triazol-1-yl}-3-trifluoromethyl-phenol;
3-{4-[(2-cyclopropyl-imidazo[5,1-*b*]thiazol-3-yl)-hydroxy-methyl]-[1,2,3]triazol-1-yl}-phenol;
5-{4-[(2-cyclopropyl-imidazo[5,1-*b*]thiazol-3-yl)-hydroxy-methyl]-[1,2,3]triazol-1-yl}-2-methoxy-phenol;
2-chloro-5-{4-[(2-cyclopropyl-imidazo[5,1-*b*]thiazol-3-yl)-hydroxy-methyl]-[1,2,3]triazol-1-yl}-phenol;
5-{4-[(2-cyclopropyl-imidazo[5,1-b]thiazol-3-yl)-hydroxy-methyl]-[1,2,3]triazol-1-yl}-2-methyl-phenol;
3-{4-[(2-cyclopropyl-imidazo[5,1-b]thiazol-3-yl)-hydroxy-methyl]-[1,2,3]triazol-1-yl}-5-trifluoromethyl-phenol;
3-{4-[(2-cyclopropyl-imidazo[5,1-b]thiazol-3-yl)-hydroxy-methyl]-[1,2,3]triazol-1-yl}-5-methyl-phenol;
5-chloro-2-{4-[(2-cyclopropyl-imidazo[5,1-*b*]thiazol-3-yl)-hydroxy-methyl]-[1,2,3]triazol-1-yl}-phenol;
(2-cyclopropyl-imidazo[5,1-*b*]thiazol-3-yl)-[1-(2,3-dihydro-benzo[1,4]dioxin-6-yl)-1*H*-pyrazol-4-yl]-methanol;
(2-cyclopropyl-imidazo[5,1-*b*]thiazol-3-yl)-[1-(6-methoxy-pyridin-3-yl)-1*H*-pyrazol-4-yl]-methanol;
*N*-(4-{4-[(2-cyclopropyl-imidazo[5,1-*b*]thiazol-3-yl)-hydroxy-methyl]-pyrazol-1-yl}-phenyl)-acetamide;
4-{4-[(2-ethyl-imidazo[5,1-*b*]thiazol-3-yl)-hydroxy-methyl]-[1,2,3]triazol-1-yl}-*N*-methyl-benzenesulfonamide;
4-{4-[(2-cyclopropyl-imidazo[5,1-*b*]thiazol-3-yl)-hydroxy-methyl]-[1,2,3]triazol-1-yl}-*N*-methyl-benzenesulfonamide;
(2-ethyl-imidazo[5,1-*b*]thiazol-3-yl)-[1-(3-methyl-1,1-dioxo-2,3-dihydro-1*H*-1l6-benzo[d]isothiazol-5-yl)-1*H*-[1,2,3]triazol-4-yl]-methanol;
(2-cyclopropyl-imidazo[5,1-*b*]thiazol-3-yl)-[1-(3-methyl-1,1-dioxo-2,3-dihydro-1*H*-1l6-benzo[d]isothiazol-5-yl)-1*H*-[1,2,3]triazol-4-yl]-methanol;
(2-cyclopropyl-imidazo[5,1-*b*]thiazol-3-yl)-[1-(1-methyl-1*H*-pyrrolo[2,3-*b*]pyhdin-5-yl)-1*H*-pyrazol-4-yl]-methanol;
*N*-(4-{4-[(2-ethyl-imidazo[5,1-*b*]thiazol-3-yl)-hydroxy-methyl]-[1,2,3]triazol-1-yl}-phenyl)-benzamide;
(4-{4-[(2-cyclopropyl-imidazo[5,1-b]thiazol-3-yl)-hydroxy-methyl]-[1,2,3]triazol-1-yl}-phenoxy)-acetic acid ethyl ester;
4-{4-[(2-cyclopropyl-imidazo[5,1-*b*]thiazol-3-yl)-hydroxy-methyl]-[1,2,3]triazol-1-yl}-2-ethyl-phenol;
4-{4-[(2-cyclopropyl-imidazo[5,1-*b*]thiazol-3-yl)-hydroxy-methyl]-[1,2,3]triazol-1-yl}-2-hydroxy-benzonitrile;
(4-{4-[(2-ethyl-imidazo[5,1-*b*]thiazol-3-yl)-hydroxy-methyl]-[1,2,3]trazol-1-yl}-phenyl)-carbamc acid tert-butyl ester;
4-{4-[(2-cyclopropyl-imidazo[5,1-b]thiazol-3-yl)-hydroxy-methyl]-[1,2,3]triazol-1-yl}-benzenesulfonamide;
4-{4-[(2-cyclopropyl-imidazo[5,1-*b*]thiazol-3-yl)-hydroxy-methyl]-[1,2,3]triazol-1 -yl}-*N*,*N*-dimethyl-benzenesulfonamide;
(2-cyclopropyl-imidazo[5,1-*b*]thiazol-3-yl)-{1-[4-(pyrrolidine-1-sulfonyl)-phenyl]-1*H*-[1,2,3]triazol-4-yl}-methanol;
4-{4-[(2-cyclopropyl-imidazo[5,1-*b*]thiazol-3-yl)-hydroxy-methyl]-[1,2,3]triazol-1-yl}-*N*,*N*-diethyl-benzenesulfonamide;
4-{4-[(2-cyclopropyl-imidazo[5,1-*b*]thiazol-3-yl)-hydroxy-methyl]-[1,2,3]triazol-1-yl}-*N*-ethyl-benzenesulfonamide;
4-{4-[(2-ethyl-imidazo[5,1-*b*]thiazol-3-yl)-hydroxy-methyl]-[1,2,3]triazol-1-yl}-benzenesulfonamide;
4-{4-[(2-ethyl-imidazo[5,1-*b*]thiazol-3-yl)-hydroxy-methyl]-[1,2,3]triazol-1-yl}-*N*,*N*-dimethyl-benzenesulfonamide;
(2-ethyl-imidazo[5,1-*b*]thiazol-3-yl)-{1-[4-(pyrrolidine-1-sulfonyl)-phenyl]-1*H*-[1,2,3]triazol-4-yl}-methanol;
*N*,*N*-diethyl-4-{4-[(2-ethyl-imidazo[5,1-*b*]thiazol-3-yl)-hydroxy-methyl]-[1,2,3]triazol-1-yl}-benzenesulfonamide;
*N*-ethyl-4-{4-[(2-ethyl-imidazo[5,1-*b*]thiazol-3-yl)-hydroxy-methyl]-[1,2,3]triazol-1-yl}-benzenesulfonamide;
(4-{4-[(2-ethyl-imidazo[5,1-*b*]thiazol-3-yl)-hydroxy-methyl]-[1,2,3]thazol-1-yl}-3-fluoro-phenyl)-carbamic acid tert-butyl ester;
*N*-(4-{4-[(2-cyclopropyl-imidazo[5,1-*b*]thiazol-3-yl)-hydroxy-methyl]-pyrazol-1-yl}-phenyl)-isobutyramide;
5-{4-[(2-cyclopropyl-imidazo[5,1-*b*]thiazol-3-yl)-hydroxy-methyl]-pyrazol-1-yl}-2-methoxy-benzonitrile;
4-{4-[(2-cyclopropyl-imidazo[5,1-*b*]thiazol-3-yl)-hydroxy-methyl]-[1,2,3]triazol-1-yl}-2-nitro-phenol;
[1-(4-methoxy-phenyl)-1*H*-[1,2,3]triazol-4-yl]-(2-phenyl-imidazo[5,1-*b*]thiazol-3-yl)-methanol;
4-{4-[hydroxy-(2-phenyl-imidazo[5,1-*b*]thiazol-3-yl)-methyl]-[1,2,3]triazol-1-yl}-2-nitro-phenol;
(4-{4-[hydroxy-(2-phenyl-imidazo[5,1-b]thiazol-3-yl)-methyl]-[1,2,3]triazol-1-yl}-phenyl)-carbamic acid *tert*-butyl ester;
(2-cyclopropyl-imidazo[5,1-*b*]thiazol-3-yl)-[1-(4-methoxy-phenyl)-5-methyl-1*H*-[1,2,3]triazol-4-yl]-methanol;
(2-cyclopropyl-imidazo[5,1-*b*]thiazol-3-yl)-[1-(1*H*-indazol-5-yl)-1*H*-[1,2,3]triazol-4-yl]-methanol;
(2-cyclopropyl-imidazo[5,1-b]thiazol-3-yl)-[1-(1-methyl-1H-indazol-7-yl)-1H-[1,2,3]triazol-4-yl]-methanol;
[1-(4-methoxy-phenyl)-5-methyl-1*H*-[1,2,3]triazol-4-yl]-(2-methyl-imidazo[5,1-*b*]thiazol-3-yl)-methanol;
4-{4-[(2-cyclopropyl-imidazo[5,1-*b*]thiazol-3-yl)-hydroxy-methyl]-[1,2,3]triazol-1-yl}-((*N*-methylsulfamoyl)amino)-benzene;
[2-(1,1-difluoro-ethyl)-imidazo[5,1-*b*]thiazol-3-yl]-[1-(4-methoxy-phenyl)-1*H*-[1,2,3]triazol-4-yl]-methanol;
[1-(3-chloro-1*H*-indazol-6-yl)-1*H*-[1,2,3]triazol-4-yl]-(2-cyclopropyl-imidazo[5,1-b]thiazol-3-yl)-methanol;
(2-(cyclopent-1-enyl)-imidazo[5,1-*b*]thiazol-3-yl)-[1-(4-methoxy-phenyl)-1*H*-[1,2,3]triazol-4-yl]-methanol;
(2-cyclopropyl-imidazo[5,1-*b*]thiazol-3-yl)-(1-pyridin-3-yl-1*H*-pyrazol-4-yl)-methanol;
[1-(4-methoxy-phenyl)-1*H*-[1,2,3]triazol-4-yl]-(2-methyl-imidazo[5,1-*b*]thiazol-3-yl)-methanol;
[1-(4-methoxy-phenyl)-1*H*-[1,2,3]triazol-4-yl]-[2-(2-methyl-propenyl)-imidazo[5,1-*b*]thiazol-3-yl]-methanol;
[1-(4-methoxy-phenyl)-1*H*-[1,2,3]triazol-4-yl]-[((*E*)-2-(prop-1-enyl))-imidazo[5,1-*b*]thiazol-3-yl]-methanol;
2-chloro-4-{4-[hydroxy-(2-phenyl-imidazo[5,1-*b*]thiazol-3-yl)-methyl]-[1,2,3]triazol-1-yl}-phenol;
[1-(3-chloro-4-cyclopropylmethoxy-phenyl)-1*H*-[1,2,3]triazol-4-yl]-(2-phenyl-imidazo[5,1-*b*]thiazol-3-yl)-methanol;
{1-[3-chloro-4-(3-fluoro-oxetan-3-ylmethoxy)-phenyl]-1*H*-[1,2,3]triazol-4-yl}-(2-phenyl-imidazo[5,1-*b*]thiazol-3-yl)-methanol;
3-(2-chloro-4-{4-[hydroxy-(2-phenyl-imidazo[5,1-*b*]thiazol-3-yl)-methyl]-[1,2,3]triazol-1-yl}-phenoxy)-propan-1-ol;
4-(2-chloro-4-{4-[hydroxy-(2-phenyl-imidazo[5,1-*b*]thiazol-3-yl)-methyl]-[1,2,3]triazol-1-yl}-phenoxy)-2-methyl-butan-2-ol;
4-(2-chloro-4-{4-[(*R*)-hydroxy-(2-phenyl-imidazo[5,1-*b*]thiazol-3-yl)-methyl]-[1,2,3]triazol-1-yl}-phenoxy)-2-methyl-butan-2-ol;
(2-isopropenyl-imidazo[5,1-*b*]thiazol-3-yl)-[1-(4-methoxy-phenyl)-1*H*-[1,2,3]triazol-4-yl]-methanol;
[2-((*E*)-2-cyclopropyl-vinyl)-imidazo[5,1-*b*]thiazol-3-yl]-[1-(4-methoxy-phenyl)-1*H*-[1,2,3]triazol-4-yl]-methanol;
[1-(4-methoxy-phenyl)-1*H*-[1,2,3]triazol-4-yl]-[((*E*)-2-(pent-1-enyl))-imidazo[5,1-*b*]thiazol-3-yl]-methanol;
[1-(4-amino-3-chloro-phenyl)-1*H*-[1,2,3]triazol-4-yl]-(2-ethyl-imidazo[5,1-*b*]thiazol-3-yl)-methanol;
[1-(4-methoxy-phenyl)-1*H*-[1,2,3]triazol-4-yl]-[2-(1-methyl-1H-pyrazol-4-yl)-imidazo[5,1-*b*]thiazol-3-yl]-methanol;
[5-*tert*-butyl-1-(4-methoxy-phenyl)-1*H*-[1,2,3]triazol-4-yl]-(2-cyclopropyl-imidazo[5,1-*b*]thiazol-3-yl)-methanol;
3-(2-chloro-4-{4-[(*R*)-(2-cyclopropyl-imidazo[5,1-*b*]thiazol-3-yl)-hydroxy-methyl]-[1,2,3]triazol-1-yl}-phenoxy)-propan-1-ol;
(*R*)-{1-[3-chloro-4-(tetrahydro-pyran-4-ylmethoxy)-phenyl]-1*H*-[1,2,3]triazol-4-yl}-(2-cyclopropyl-imidazo[5,1-*b*]thiazol-3-yl)-methanol;
4-(2-chloro-4-{4-[(*R*)-(2-cyclopropyl-imidazo[5,1-*b*]thiazol-3-yl)-hydroxy-methyl]-[1,2,3]triazol-1-yl}-phenoxy)-2-methyl-butan-2-ol;
[1-(4-amino-3-chloro-phenyl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[5-ethyl-1-(4-methoxy-phenyl)-1*H*-[1,2,3]triazol-4-yl]-methanol;
(2-cyclopropyl-imidazo[5,1-*b*]thiazol-3-yl)-[5-isopropyl-1-(4-methoxy-phenyl)-1*H*-[1,2,3]triazol-4-yl]-methanol;
(2-cyclopropyl-imidazo[5,1-*b*]thiazol-3-yl)-[5-cyclopropyl-1-(4-methoxy-phenyl)-1*H*-[1,2,3]triazol-4-yl]-methanol;

(*R*)-{1-[3-chloro-4-(3-fluoro-oxetan-3-ylmethoxy)-phenyl]-1*H*-[1,2,3]triazol-4-yl}-(2-cyclopropyl-imidazo[5,1-*b*]thiazol-3-yl)-methanol;
1-(2-chloro-4-{4-[(*R*)-(2-cyclopropyl-imidazo[5,1-*b*]thiazol-3-yl)-hydroxy-methyl]-[1,2,3]triazol-1-yl}-phenoxy)-2-methyl-propan-2-ol;
*N*-(2-chloro-4-{4-[(2-cyclopropyl-imidazo[5,1-*b*]thiazol-3-yl)-hydroxy-methyl]-[1,2,3]triazol-1-yl}-phenyl)-isobutyramide;
(2-ethyl-imidazo[5,1-*b*]thiazol-3-yl)-[1-(4-methoxy-phenyl)-1*H*-[1,2,3]triazol-4-yl]-methanol;
[2-(4-fluoro-phenyl)-imidazo[5,1-*b*]thiazol-3-yl]-[1-(4-methoxy-phenyl)-1*H*-[1,2,3]triazol-4-yl]-methanol;
[2-(2-fluoro-phenyl)-imidazo[5,1-*b*]thiazol-3-yl]-[1-(4-methoxy-phenyl)-1*H*-[1,2,3]triazol-4-yl]-methanol;
[1-(3-chloro-5-fluoro-4-methoxy-phenyl)-1*H*-[1,2,3]triazol-4-yl]-(2-cyclopropyl-imidazo[5,1-*b*]thiazol-3-yl)-methanol;
[1-(3-chloro-4-methoxy-phenyl)-1*H*-[1,2,3]triazol-4-yl]-(2-cyclopropyl-imidazo[5,1-*b*]thiazol-3-yl)-methanol;
[1-(3-bromo-4-methoxy-phenyl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[1-(3-iodo-4-methoxy-phenyl)-1*H*-[1,2,3]triazol-4-yl]-methanol;
[1-(3-chloro-2-fluoro-4-methoxy-phenyl)-1*H*-[1,2,3]triazol-4-yl]-(2-cyclopropyl-imidazo[5,1-*b*]thiazol-3-yl)-methanol;
4-(2-chloro-4-{4-[hydroxy-(2-isopropenyl-imidazo[5,1-*b*]thiazol-3-yl)-methyl]-[1,2,3]triazol-1-yl}-phenoxy)-2-methyl-butan-2-ol;
3-{4-[(2-cyclopropyl-imidazo[5,1-*b*]thiazol-3-yl)-hydroxy-methyl]-[1,2,3]triazol-1-ylmethyl}-benzoic acid methyl ester;
[1-(3-chloro-4-methoxy-phenyl)-5-methyl-1*H*-[1,2,3]triazol-4-yl]-(2-cyclopropyl-imidazo[5,1-b]thiazol-3-yl)-methanol;
[1-(3-chloro-4-ethoxy-phenyl)-5-methyl-1*H*-[1,2,3]triazol-4-yl]-(2-cyclopropyl-imidazo[5,1-b]thiazol-3-yl)-methanol;
4-{4-[(2-cyclopropyl-imidazo[5,1-b]thiazol-3-yl)-hydroxy-methyl]-[1,2,3]triazol-1-ylmethyl}-benzoic acid methyl ester;
3-{4-[(2-cyclopropyl-imidazo[5,1-*b*]thiazol-3-yl)-hydroxy-methyl]-[1,2,3]triazol-1-ylmethyl}-phenol;
{1-[3-chloro4-(3-fluoro-oxetan-3-ylmethoxy)-phenyl]-1*H*-[1,2,3]triazol-4-yl}-(2-isopropenyl-imidazo[5,1-*b*)]thiazol-3-yl)-methanol;
[1-(5-chloro-2-fluoro-4-methoxy-phenyl)-1*H*-[1,2,3]triazol-4-yl]-(2-cyclopropyl-imidazo[5,1-*b*]thiazol-3-yl)-methanol;
3-(2-chloro-4-{4-[hydroxy-(2-isopropenyl-imidazo[5,1-*b*]thiazol-3-yl)-methyl]-[1,2,3]triazol-1-yl}-phenoxy)-propan-1-ol;
4-(2-chloro-4-{4-[(2-ethyl-imidazo[5,1-*b*]thiazol-3-yl)-hydroxy-methyl]-[1,2,3]triazol-1-yl}-phenoxy)-2-methyl-butan-2-ol;
4-[2-chloro4-(4-{hydroxy-[2-(1-methyl-cyclopropyl)-imidazo[5,1-*b*]thiazol-3-yl]-methyl}-[1,2,3]triazol-1-yl)-phenoxy]-2-methyl-butan-2-ol;
4-{4-[(2-cyclopropyl-imidazo[5,1-*b*]thiazol-3-yl)-hydroxy-methyl]-[1,2,3]triazol-1-ylmethyl}-phenol;
2-{4-[(2-cyclopropyl-imidazo[5,1-*b*]thiazol-3-yl)-hydroxy-methyl]-[1,2,3]triazol-1-ylmethyl}-phenol;
(2-cyclopropyl-imidazo[5,1-*b*]thiazol-3-yl)-[1-(4-methoxy-2-methyl-phenyl)-1*H*-[1,2,3]triazol-4-yl]-methanol;
4-[2-chloro4-(4-{[2-(1,1-difluoro-ethyl)-imidazo[5,1-*b*]thiazol-3-yl]-hydroxy-methyl}-[1,2,3]triazol-1-yl)-phenoxy]-2-methyl-butan-2-ol;
4-[2-chloro-4-(4-{[2-(*trans*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol;
4-[2-chloro-4-(4-{[2-(*cis*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol;
(2-cyclopropyl-imidazo[5,1-*b*]thiazol-3-yl)-[1-(4-hydroxymethyl-phenyl)-1*H*-[1,2,3]triazol-4-yl]-methanol;
(2-cyclopropyl-imidazo[5,1-*b*]thiazol-3-yl)-[1-(4-methoxy-2,6-dimethyl-phenyl)-1*H*-[1,2,3]triazol-4-yl]-methanol;
(2-cyclopropyl-imidazo[5,1-*b*]thiazol-3-yl)-(1-{4-[2-(2,2,2-trifluoro-ethoxy)-ethoxy]-phenyl}-1*H*-[1,2,3]triazol-4-yl)-methanol;
3-[2-chloro4-(4-{hydroxy-[2-(1-methyl-cyclopropyl)-imidazo[5,1-b]thiazol-3-yl]-methyl}-[1,2,3]triazol-1-yl)-phenoxy]-propan-1-ol;
3-[2-chloro4-(4-{[2-(1,1-difluoro-ethyl)-imidazo[5,1-*b*]thiazol-3-yl]-hydroxy-methyl}-[1,2,3]triazol-1-yl)-phenoxy]-propan-1-ol;
{1-[3-chloro-4-(3-fluoro-oxetan-3-ylmethoxy)-phenyl]-1*H*-[1,2,3]triazol-4-yl}-[2-(1-methyl-cyclopropyl)-imidazo[5,1-*b*]thiazol-3-yl]-methanol;
{1-[3-chloro-4-(3-fluoro-oxetan-3-ylmethoxy)-phenyl]-1*H*-[1,2,3]triazol-4-yl}-[2-(1,1-difluoro-ethyl)-imidazo[5,1-*b*]thiazol-3-yl]-methanol;

{1-[3-chloro-4-(3-fluoro-oxetan-3-ylmethoxy)-phenyl]-1*H*-[1,2,3]triazol-4-yl}-(2-ethyl-imidazo[5,1-*b*]thiazol-3-yl)-methanol;
(2-cyclopropyl-imidazo[5,1-*b*]thiazol-3-yl)-[1-(2,5-difluoro-4-methoxy-phenyl)-1*H*-[1,2,3]triazol-4-yl]-methanol;
[1-(5-chloro-2-fluoro-4-methoxy-phenyl)-1*H*-[1,2,3]triazol-4-yl]-(2-ethyl-imidazo[5,1-b]thiazol-3-yl)-methanol;
3-(2-chloro-4-{4-[(2-ethyl-imidazo[5,1-b]thiazol-3-yl)-hydroxy-methyl]-[1,2,3]triazol-1-yl}-phenoxy)-propan-1-ol;
[1-(2,5-difluoro-4-methoxy-phenyl)-1*H*-[1,2,3]triazol-4-yl]-(2-ethyl-imidazo[5,1-*b*]thiazol-3-yl)-methanol;
[1-(4-benzyloxy-3-fluoro-phenyl)-1*H*-[1,2,3]triazol-4-yl]-[2-(*trans*-2-fluoro-cyclopropyl)-imidazo[5,1-*b*]thiazol-3-yl]-methanol;
2-chloro-4-{4-[(2-ethyl-imidazo[5,1-*b*]thiazol-3-yl)-hydroxy-methyl]-[1,2,3]triazol-1-yl}-phenol;
2-chloro-4-(4-{hydroxy-[2-(1-methyl-cyclopropyl)-imidazo[5,1-b]thiazol-3-yl]-methyl}-[1,2,3]triazol-1-yl)-phenol;
2-chloro-4-(4-{[2-(*cis*-2-fluoro-cyclopropyl)-imidazo[5,1-*b*]thiazol-3-yl]-hydroxy-methyl}-[1,2,3]triazol-1-yl)-phenol;
(2-cyclopropyl-imidazo[5,1-*b*]thiazol-3-yl)-[1-(4-methoxy-2-methyl-phenyl)-5-methyl-1*H*-[1,2,3]triazol-4-yl]-methanol;
(2-cyclopropyl-imidazo[5,1-*b*]thiazol-3-yl)-[1-(3-fluoro-4-methoxy-phenyl)-1*H*-[1,2,3]triazol-4-yl]-methanol;
2-chloro-4-(4-{[2-(*trans*-2-fluoro-cyclopropyl)-imidazo[5,1-*b*]thiazol-3-yl]-hydroxy-methyl}-[1,2,3]triazol-1-yl)-phenol;
2-chloro-4-(4-{[2-(1,1-difluoro-ethyl)-imidazo[5,1-*b*]thiazol-3-yl]-hydroxy-methyl}-[1,2,3]triazol-1-yl)-phenol;
(2-cyclopropyl-imidazo[5,1-*b*]thiazol-3-yl)-[1-(4-methoxymethyl-phenyl)-1*H*-[1,2,3]triazol-4-yl]-methanol;
4-{4-[(2-cyclopropyl-imidazo[5,1-*b*]thiazol-3-yl)-hydroxy-methyl]-[1,2,3]triazol-1-yl}-2,5-difluoro-phenol;
4-{4-[(2-ethyl-imidazo[5,1-*b*]thiazol-3-yl)-hydroxy-methyl]-[1,2,3]triazol-1-yl}-2,5-difluoro-phenol;
[1-(5-chloro-2-fluoro-4-methoxy-phenyl)-5-methyl-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[5-iodo-1-(4-methoxy-phenyl)-1*H*-[1,2,3]triazol-4-yl]-methanol;
[2-(*trans*-2-fluoro-cyclopropyl)-imidazo[5,1-*b*]thiazol-3-yl]-[5-fluoro-1-(4-methoxy-phenyl)-1*H*-[1,2,3]triazol-4-yl]-methanol; and
(2-cyclopropyl-imidazo[5,1-*b*]thiazol-3-yl)-[5-fluoro-1-(4-methoxy-phenyl)-1*H*-[1,2,3]triazol-4-yl]-methanol.

28) Another embodiment relates to a compound of embodiment 1) selected from a group consisting of:

(R)-(2-cyclopropyl-imidazo[5,1-b]thiazol-3-yl)-(1-phenyl-1H-[1,2,3]triazol-4-yl)-methanol;
(R)-(2-cyclopropyl-imidazo[5,1-b]thiazol-3-yl)-[1-(4-methoxy-phenyl)-1H-[1,2,3]triazol-4-yl]-methanol;
(R)-(2-cyclopropyl-imidazo[5,1-b]thiazol-3-yl)-[1-(1H-indol-5-yl)-1H-[1,2,3]triazol-4-yl]-methanol;
3-{4-[(R)-(2-cyclopropyl-imidazo[5,1-b]thiazol-3-yl)-hydroxy-methyl]-[1,2,3]triazol-1-yl}-benzonitrile;
(*S*)-(2-cyclopropyl-imidazo[5,1-*b*]thiazol-3-yl)-(1-phenyl-1*H*-pyrazol-4-yl)-methanol;
(*R*)-(2-cyclopropyl-imidazo[5,1-*b*]thiazol-3-yl)-[1-(4-cyclopropylmethoxy-phenyl)-1*H*-[1,2,3]triazol-4-yl]-methanol;
3-(4-{4-[(*R*)-(2-cyclopropyl-imidazo[5,1-*b*]thiazol-3-yl)-hydroxy-methyl]-[1,2,3]triazol-1-yl}-phenoxy)-propan-1-ol;
4-(4-{4-[(*R*)-(2-cyclopropyl-imidazo[5,1-*b*]thiazol-3-yl)-hydroxy-methyl]-[1,2,3]triazol-1-yl}-phenoxy)-2-methyl-butan-2-ol;
1-[2-(4-{4-[(R)-(2-cyclopropyl-imidazo[5,1-b]thiazol-3-yl)-hydroxy-methyl]-[1,2,3]triazol-1-yl}-phenoxy)-ethyl]-cyclopropanol;
(*R*)-(2-cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol;
2-chloro-4-{4-[(*R*)-(2-cyclopropyl-imidazo[5,1-*b*]thiazol-3-yl)-hydroxy-methyl]-[1,2,3]triazol-1-yl}-phenol;
(4-{4-[(R)-(2-ethyl-imidazo[5,1-b]thiazol-3-yl)-hydroxy-methyl]-[1,2,3]triazol-1-yl}-phenyl)-carbamic acid methyl ester;
(*R*)-(2-cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol;
1-(4-{4-[(*R*)-(2-cyclopropyl-imidazo[5,1-b]thiazol-3-yl)-hydroxy-methyl]-[1,2,3]triazol-1-yl}-phenoxy)-2-methyl-propan-2-ol;
(*R*)-(2-cyclopropyl-imidazo[5,1-*b*]thiazol-3-yl)-(5-methyl-1-phenyl-1*H*-[1,2,3]triazol-4-yl)-methanol;
*N*-(4-{4-[(*R*)-(2-cyclopropyl-imidazo[5,1-*b*]thiazol-3-yl)-hydroxy-methyl]-[1,2,3]triazol-1-yl}-phenyl)-isobutyramide;
(*S*)-3-(4-{4-[(*R*)-(2-cyclopropyl-imidazo[5,1-*b*]thiazol-3-yl)-hydroxy-methyl]-[1,2,3]triazol-1-yl}-phenoxy)-propane-1,2-diol;

4-(2-chloro-4-{4-[(*R*)-hydroxy-(2-phenyl-imidazo[5,1-*b*]thiazol-3-yl)-methyl]-[1,2,3]triazol-1-yl}-phenoxy)-2-methyl-butan-2-ol;
3-(2-chloro-4-{4-[(*R*)-(2-cyclopropyl-imidazo[5,1-*b*]thiazol-3-yl)-hydroxy-methyl]-[1,2,3]triazol-1-yl}-phenoxy)-propan-1-ol;
(*R*)-{1-[3-chloro-4-(tetrahydro-pyran-4-ylmethoxy)-phenyl]-1*H*-[1,2,3]triazol-4-yl}-(2-cyclopropyl-imidazo[5,1-*b*]thiazol-3-yl)-methanol;
4-(2-chloro-4-{4-[(*R*)-(2-cyclopropyl-imidazo[5,1-*b*]thiazol-3-yl)-hydroxy-methyl]-[1,2,3]triazol-1-yl}-phenoxy)-2-methyl-butan-2-ol;
(*R*)-{1-[3-chloro-4-(3-fluoro-oxetan-3-ylmethoxy)-phenyl]-1*H*-[1,2,3]triazol-4-yl}-(2-cyclopropyl-imidazo[5,1-*b*]thiazol-3-yl)-methanol; and
1-(2-chloro-4-{4-[(R)-(2-cyclopropyl-imidazo[5,1-b]thiazol-3-yl)-hydroxy-methyl]-[1,2,3]triazol-1-yl}-phenoxy)-2-methyl-propan-2-ol.

Based on the dependencies of the different embodiments 1) to 24) as disclosed hereinabove, the following embodiments are thus possible and intended, and herewith specifically disclosed in individualized form:
2+1, 3+1, 3+2+1, 4+1, 4+2+1, 5+1, 5+2+1, 6+1, 6+2+1, 7+1, 7+2+1, 8+1, 8+2+1, 9+1, 9+2+1, 10+9+1, 10+9+2+1, 11+9+1, 11+9+2+1, 12+9+1, 12+9+2+1, 13+1, 13+2+1, 13+3+1, 13+3+2+1, 13+4+1, 13+4+2+1, 13+5+1, 13+5+2+1, 13+6+1, 13+6+2+1, 13+7+1, 13+7+2+1, 13+8+1, 13+8+2+1, 14+1, 14+2+1, 14+3+1, 14+3+2+1, 14+4+1, 14+4+2+1, 14+5+1, 14+5+2+1, 14+6+1, 14+6+2+1, 14+7+1, 14+7+2+1, 14+8+1, 14+8+2+1, 15+1, 15+2+1, 15+3+1, 15+3+2+1, 15+4+1, 15+4+2+1, 15+5+1, 15+5+2+1, 15+6+1, 15+6+2+1, 15+7+1, 15+7+2+1, 15+8+1, 15+8+2+1, 15+9+1, 15+9+2+1, 15+10+9+1, 15+10+9+2+1, 15+11+9+1, 15+11+9+2+1, 15+12+9+1, 15+12+9+2+1, 15+13+1, 15+13+2+1, 15+13+3+1, 15+13+3+2+1, 15+13+4+1, 15+13+4+2+1, 15+13+5+1, 15+13+5+2+1, 15+13+6+1, 15+13+6+2+1, 15+13+7+1, 15+13+7+2+1, 15+13+8+1, 15+13+8+2+1, 15+14+1, 15+14+2+1, 15+14+3+1, 15+14+3+2+1, 15+14+4+1, 15+14+4+2+1, 15+14+5+1, 15+14+5+2+1, 15+14+6+1, 15+14+6+2+1, 15+14+7+1, 15+14+7+2+1, 15+14+8+1, 15+14+8+2+1, 16+1, 16+2+1, 16+3+1, 16+3+2+1, 16+4+1, 16+4+2+1, 16+5+1, 16+5+2+1, 16+6+1, 16+6+2+1, 16+7+1, 16+7+2+1, 16+8+1, 16+8+2+1, 16+9+1, 16+9+2+1, 16+10+9+1, 16+10+9+2+1, 16+11+9+1, 16+11+9+2+1, 16+12+9+1, 16+12+9+2+1, 16+13+1, 16+13+2+1, 16+13+3+1, 16+13+3+2+1, 16+13+4+1, 16+13+4+2+1, 16+13+5+1, 16+13+5+2+1, 16+13+6+1, 16+13+6+2+1, 16+13+7+1, 16+13+7+2+1, 16+13+8+1, 16+13+8+2+1, 16+14+1, 16+14+2+1, 16+14+3+1, 16+14+3+2+1, 16+14+4+1, 16+14+4+2+1, 16+14+5+1, 16+14+5+2+1, 16+14+6+1, 16+14+6+2+1, 16+14+7+1, 16+14+7+2+1, 16+14+8+1, 16+14+8+2+1, 17+1, 17+2+1, 17+3+1, 17+3+2+1, 17+4+1, 17+4+2+1, 17+5+1, 17+5+2+1, 17+6+1, 17+6+2+1, 17+7+1, 17+7+2+1, 17+8+1, 17+8+2+1, 17+9+1, 17+9+2+1, 17+10+9+1, 17+10+9+2+1, 17+11+9+1, 17+11+9+2+1, 17+12+9+1, 17+12+9+2+1, 17+13+1, 17+13+2+1, 17+13+3+1, 17+13+3+2+1, 17+13+4+1, 17+13+4+2+1, 17+13+5+1, 17+13+5+2+1, 17+13+6+1, 17+13+6+2+1, 17+13+7+1, 17+13+7+2+1, 17+13+8+1, 17+13+8+2+1, 17+14+1, 17+14+2+1, 17+14+3+1, 17+14+3+2+1, 17+14+4+1, 17+14+4+2+1, 17+14+5+1, 17+14+5+2+1, 17+14+6+1, 17+14+6+2+1, 17+14+7+1, 17+14+7+2+1, 17+14+8+1, 17+14+8+2+1, 18+1, 18+2+1, 18+3+1, 18+3+2+1, 18+4+1, 18+4+2+1, 18+5+1, 18+5+2+1, 18+6+1, 18+6+2+1, 18+7+1, 18+7+2+1, 18+8+1, 18+8+2+1, 18+9+1, 18+9+2+1, 18+10+9+1, 18+10+9+2+1, 18+11+9+1, 18+11+9+2+1, 18+12+9+1, 18+12+9+2+1, 18+13+1, 18+13+2+1, 18+13+3+1, 18+13+3+2+1, 18+13+4+1, 18+13+4+2+1, 18+13+5+1, 18+13+5+2+1, 18+13+6+1, 18+13+6+2+1, 18+13+7+1, 18+13+7+2+1, 18+13+8+1, 18+13+8+2+1, 18+14+1, 18+14+2+1, 18+14+3+1, 18+14+3+2+1, 18+14+4+1, 18+14+4+2+1, 18+14+5+1, 18+14+5+2+1, 18+14+6+1, 18+14+6+2+1, 18+14+7+1, 18+14+7+2+1, 18+14+8+1, 18+14+8+2+1, 19+1, 19+2+1, 19+3+1, 19+3+2+1, 19+4+1, 19+4+2+1, 19+5+1, 19+5+2+1, 19+6+1, 19+6+2+1, 19+7+1, 19+7+2+1, 19+8+1, 19+8+2+1, 19+9+1, 19+9+2+1, 19+10+9+1, 19+10+9+2+1, 19+11+9+1, 19+11+9+2+1, 19+12+9+1, 19+12+9+2+1, 19+13+1, 19+13+2+1, 19+13+3+1, 19+13+3+2+1, 19+13+4+1, 19+13+4+2+1, 19+13+5+1, 19+13+5+2+1, 19+13+6+1, 19+13+6+2+1, 19+13+7+1, 19+13+7+2+1, 19+13+8+1, 19+13+8+2+1, 19+14+1, 19+14+2+1, 19+14+3+1, 19+14+3+2+1, 19+14+4+1, 19+14+4+2+1, 19+14+5+1, 19+14+5+2+1, 19+14+6+1, 19+14+6+2+1, 19+14+7+1, 19+14+7+2+1, 19+14+8+1, 19+14+8+2+1, 20+1, 20+2+1, 20+3+1, 20+3+2+1, 20+4+1, 20+4+2+1, 20+5+1, 20+5+2+1, 20+6+1, 20+6+2+1, 20+7+1, 20+7+2+1, 20+8+1, 20+8+2+1, 20+9+1, 20+9+2+1, 20+10+9+1, 20+10+9+2+1, 20+11+9+1, 20+11+9+2+1, 20+12+9+1, 20+12+9+2+1, 20+13+1, 20+13+2+1, 20+13+3+1, 20+13+3+2+1, 20+13+4+1, 20+13+4+2+1, 20+13+5+1, 20+13+5+2+1, 20+13+6+1, 20+13+6+2+1, 20+13+7+1, 20+13+7+2+1, 20+13+8+1, 20+13+8+2+1, 20+14+1, 20+14+2+1, 20+14+3+1, 20+14+3+2+1, 20+14+4+1, 20+14+4+2+1, 20+14+5+1, 20+14+5+2+1, 20+14+6+1, 20+14+6+2+1, 20+14+7+1, 20+14+7+2+1, 20+14+8+1, 20+14+8+2+1, 21+1, 22+1, 23+1, 24+1, 24+2+1, 24+3+1, 24+3+2+1, 24+4+1, 24+4+2+1, 24+5+1, 24+5+2+1, 24+6+1, 24+6+2+1, 24+7+1, 24+7+2+1, 24+8+1, 24+8+2+1, 24+9+1, 24+9+2+1, 24+10+9+1, 24+10+9+2+1, 24+11+9+1, 24+11+9+2+1, 24+12+9+1, 24+12+9+2+1, 24+13+1, 24+13+2+1, 24+13+3+1, 24+13+3+2+1, 24+13+4+1, 24+13+4+2+1, 24+13+5+1, 24+13+5+2+1, 24+13+6+1, 24+13+6+2+1, 24+13+7+1, 24+13+7+2+1, 24+13+8+1, 24+13+8+2+1, 24+14+1, 24+14+2+1, 24+14+3+1, 24+14+3+2+1, 24+14+4+1, 24+14+4+2+1, 24+14+5+1, 24+14+5+2+1, 24+14+6+1, 24+14+6+2+1, 24+14+7+1, 24+14+7+2+1, 24+14+8+1, 24+14+8+2+1, 24+15+1, 24+15+2+1, 24+15+3+1, 24+15+3+2+1, 24+15+4+1,

24+15+4+2+1, 24+15+5+1, 24+15+5+2+1, 24+15+6+1, 24+15+6+2+1, 24+15+7+1, 24+15+7+2+1, 24+15+8+1, 24+15+8+2+1, 24+15+9+1, 24+15+9+2+1, 24+15+10+9+1, 24+15+10+9+2+1, 24+15+11+9+1, 24+15+11+9+2+1, 24+15+12+9+1, 24+15+12+9+2+1, 24+15+13+1, 24+15+13+2+1, 24+15+13+3+1, 24+15+13+3+2+1, 24+15+13+4+1, 24+15+13+4+2+1, 24+15+13+5+1, 24+15+13+5+2+1, 24+15+13+6+1, 24+15+13+6+2+1, 24+15+13+7+1, 24+15+13+7+2+1, 24+15+13+8+1, 24+15+13+8+2+1, 24+15+14+1, 24+15+14+2+1, 24+15+14+3+1, 24+15+14+3+2+1, 24+15+14+4+1, 24+15+14+4+2+1, 24+15+14+5+1, 24+15+14+5+2+1, 24+15+14+6+1, 24+15+14+6+2+1, 24+15+14+7+1, 24+15+14+7+2+1, 24+15+14+8+1, 24+15+14+8+2+1, 24+16+1, 24+16+2+1, 24+16+3+1, 24+16+3+2+1, 24+16+4+1, 24+16+4+2+1, 24+16+5+1, 24+16+5+2+1, 24+16+6+1, 24+16+6+2+1, 24+16+7+1, 24+16+7+2+1, 24+16+8+1, 24+16+8+2+1, 24+16+9+1, 24+16+9+2+1, 24+16+10+9+1, 24+16+10+9+2+1, 24+16+11+9+1, 24+16+11+9+2+1, 24+16+12+9+1, 24+16+12+9+2+1, 24+16+13+1, 24+16+13+2+1, 24+16+13+3+1, 24+16+13+3+2+1, 24+16+13+4+1, 24+16+13+4+2+1, 24+16+13+5+1, 24+16+13+5+2+1, 24+16+13+6+1, 24+16+13+6+2+1, 24+16+13+7+1, 24+16+13+7+2+1, 24+16+13+8+1, 24+16+13+8+2+1, 24+16+14+1, 24+16+14+2+1, 24+16+14+3+1, 24+16+14+3+2+1, 24+16+14+4+1, 24+16+14+4+2+1, 24+16+14+5+1, 24+16+14+5+2+1, 24+16+14+6+1, 24+16+14+6+2+1, 24+16+14+7+1, 24+16+14+7+2+1, 24+16+14+8+1, 24+16+14+8+2+1, 24+17+1, 24+17+2+1, 24+17+3+1, 24+17+3+2+1, 24+17+4+1, 24+17+4+2+1, 24+17+5+1, 24+17+5+2+1, 24+17+6+1, 24+17+6+2+1, 24+17+7+1, 24+17+7+2+1, 24+17+8+1, 24+17+8+2+1, 24+17+9+1, 24+17+9+2+1, 24+17+10+9+1, 24+17+10+9+2+1, 24+17+11+9+1, 24+17+11+9+2+1, 24+17+12+9+1, 24+17+12+9+2+1, 24+17+13+1, 24+17+13+2+1, 24+17+13+3+1, 24+17+13+3+2+1, 24+17+13+4+1, 24+17+13+4+2+1, 24+17+13+5+1, 24+17+13+5+2+1, 24+17+13+6+1, 24+17+13+6+2+1, 24+17+13+7+1, 24+17+13+7+2+1, 24+17+13+8+1, 24+17+13+8+2+1, 24+17+14+1, 24+17+14+2+1, 24+17+14+3+1, 24+17+14+3+2+1, 24+17+14+4+1, 24+17+14+4+2+1, 24+17+14+5+1, 24+17+14+5+2+1, 24+17+14+6+1, 24+17+14+6+2+1, 24+17+14+7+1, 24+17+14+7+2+1, 24+17+14+8+1, 24+17+14+8+2+1, 24+18+1, 24+18+2+1, 24+18+3+1, 24+18+3+2+1, 24+18+4+1, 24+18+4+2+1, 24+18+5+1, 24+18+5+2+1, 24+18+6+1, 24+18+6+2+1, 24+18+7+1, 24+18+7+2+1, 24+18+8+1, 24+18+8+2+1, 24+18+9+1, 24+18+9+2+1, 24+18+10+9+1, 24+18+10+9+2+1, 24+18+11+9+1, 24+18+11+9+2+1, 24+18+12+9+1, 24+18+12+9+2+1, 24+18+13+1, 24+18+13+2+1, 24+18+13+3+1, 24+18+13+3+2+1, 24+18+13+4+1, 24+18+13+4+2+1, 24+18+13+5+1, 24+18+13+5+2+1, 24+18+13+6+1, 24+18+13+6+2+1, 24+18+13+7+1, 24+18+13+7+2+1, 24+18+13+8+1, 24+18+13+8+2+1, 24+18+14+1, 24+18+14+2+1, 24+18+14+3+1, 24+18+14+3+2+1, 24+18+14+4+1, 24+18+14+4+2+1, 24+18+14+5+1, 24+18+14+5+2+1, 24+18+14+6+1, 24+18+14+6+2+1, 24+18+14+7+1, 24+18+14+7+2+1, 24+18+14+8+1, 24+18+14+8+2+1, 24+19+1, 24+19+2+1, 24+19+3+1, 24+19+3+2+1, 24+19+4+1, 24+19+4+2+1, 24+19+5+1, 24+19+5+2+1, 24+19+6+1, 24+19+6+2+1, 24+19+7+1, 24+19+7+2+1, 24+19+8+1, 24+19+8+2+1, 24+19+9+1, 24+19+9+2+1, 24+19+10+9+1, 24+19+10+9+2+1, 24+19+11+9+1, 24+19+11+9+2+1, 24+19+12+9+1, 24+19+12+9+2+1, 24+19+13+1, 24+19+13+2+1, 24+19+13+3+1, 24+19+13+3+2+1, 24+19+13+4+1, 24+19+13+4+2+1, 24+19+13+5+1, 24+19+13+5+2+1, 24+19+13+6+1, 24+19+13+6+2+1, 24+19+13+7+1, 24+19+13+7+2+1, 24+19+13+8+1, 24+19+13+8+2+1, 24+19+14+1, 24+19+14+2+1, 24+19+14+3+1, 24+19+14+3+2+1, 24+19+14+4+1, 24+19+14+4+2+1, 24+19+14+5+1, 24+19+14+5+2+1, 24+19+14+6+1, 24+19+14+6+2+1, 24+19+14+7+1, 24+19+14+7+2+1, 24+19+14+8+1, 24+19+14+8+2+1, 24+20+1, 24+20+2+1, 24+20+3+1, 24+20+3+2+1, 24+20+4+1, 24+20+4+2+1, 24+20+5+1, 24+20+5+2+1, 24+20+6+1, 24+20+6+2+1, 24+20+7+1, 24+20+7+2+1, 24+20+8+1, 24+20+8+2+1, 24+20+9+1, 24+20+9+2+1, 24+20+10+9+1, 24+20+10+9+2+1, 24+20+11+9+1, 24+20+11+9+2+1, 24+20+12+9+1, 24+20+12+9+2+1, 24+20+13+1, 24+20+13+2+1, 24+20+13+3+1, 24+20+13+3+2+1, 24+20+13+4+1, 24+20+13+4+2+1, 24+20+13+5+1, 24+20+13+5+2+1, 24+20+13+6+1, 24+20+13+6+2+1, 24+20+13+7+1, 24+20+13+7+2+1, 24+20+13+8+1, 24+20+13+8+2+1, 24+20+14+1, 24+20+14+2+1, 24+20+14+3+1, 24+20+14+3+2+1, 24+20+14+4+1, 24+20+14+4+2+1, 24+20+14+5+1, 24+20+14+5+2+1, 24+20+14+6+1, 24+20+14+6+2+1, 24+20+14+7+1, 24+20+14+7+2+1, 24+20+14+8+1, 24+20+14+8+2+1, 24+21+1, 24+22+1 or 24+23+1.

In the list above the numbers refer to the embodiments according to their numbering provided hereinabove whereas "+" indicates the dependency from another embodiment. The different individualized embodiments are separated by commas. In other words, "3+2+1" for example refers to embodiment 3) depending on embodiment 2), depending on embodiment 1), i.e. embodiment "3+2+1" corresponds to embodiment 1) further characterized by the features of the embodiments 2) and 3).

The compounds of Formula (I) encompass compounds with at least one (i.e. the asymmetric carbon atom to which the fragment **$R^2$**-$(CH_2)_n$**-A-** is attached) and possibly more asymmetric centers, such as one or more asymmetric carbon atoms, which are allowed to be present in (R)- as well as (S)-configuration. The compounds of Formula (I) may further encompass compounds with one or more double bonds which are allowed to be present in Z- as well as E-configuration and/or compounds with substituents at a ring system which are allowed to be present, relative to each other, in cis- as well as trans-configuration. The compounds of Formula (I) may thus be present as mixtures of stereoisomers or preferably in stereoisomerically enriched form, especially as essentially pure stereoisomers. In Formula (II), in addition to the asymmetric carbon atom to which the fragment **$R^2$**-$(CH_2)_n$**-A-** is attached and which has the defined

absolute configuration shown in Formula (II), the compounds of said formula may contain further asymmetric carbon atoms which are allowed to be present in (R)- as well as (S)-configuration. The compounds of Formula (II) may thus be present as mixtures of stereoisomers or preferably as pure stereoisomers. Mixtures of stereoisomers may be separated in a manner known to a person skilled in the art.

In case a particular compound (or generic structure) is designated as (R)- or (S)-enantiomer, such designation is to be understood as referring to the respective compound (or generic structure) in enriched, especially essentially pure, enantiomeric form. Likewise, in case a specific asymmetric center in a compound is designated as being in (R)- or (S)-configuration or as being in a certain relative configuration, such designation is to be understood as referring to the compound that is in enriched, especially essentially pure, form with regard to the respective configuration of said asymmetric center. In analogy, cis- or trans-designations are to be understood as referring to the respective stereoisomer in enriched, especially essentially pure, form. Likewise, in case a particular compound (or generic structure) is designated as Z- or E-stereoisomer (or in case a specific double bond in a compound is designated as being in Z- or E-configuration), such designation is to be understood as referring to the respective compound (or generic structure) in enriched, especially essentially pure, stereoisomeric form (or to the compound that is in enriched, especially essentially pure, form with regard to the respective configuration of the double bond).

The term "enriched", when used in the context of stereoisomers, is to be understood in the context of the present invention to mean that the respective stereoisomer is present in a ratio of at least 70:30, especially of at least 90:10 (i.e., in a purity of at least 70% by weight, especially of at least 90% by weight), with regard to the respective other stereoisomer / the entirety of the respective other stereoisomers.

The term "essentially pure", when used in the context of stereoisomers, is to be understood in the context of the present invention to mean that the respective stereoisomer is present in a purity of at least 95% by weight, especially of at least 99% by weight, with regard to the respective other stereoisomer / the entirety of the respective other stereoisomers.

The present invention also includes isotopically labeled, especially $^{2}H$ (deuterium) labeled compounds of Formula (I), which compounds are identical to the compounds of Formula (I) except that one or more atoms have each been replaced by an atom having the same atomic number but an atomic mass different from the atomic mass usually found in nature. Isotopically labeled, especially $^{2}H$ (deuterium) labeled compounds of Formula (I) and salts thereof are within the scope of the present invention. Substitution of hydrogen with the heavier isotope $^{2}H$ (deuterium) may lead to greater metabolic stability, resulting e.g. in increased in-vivo half-life or reduced dosage requirements, or may lead to a modified metabolism, resulting e.g. in an improved safety profile. In one embodiment of the invention, the compounds of Formula (I) are not isotopically labeled, or they are labeled only with one or more deuterium atoms. In a sub-embodiment, the compounds of Formula (I) are not isotopically labeled at all. Isotopically labeled compounds of Formula (I) may be prepared in analogy to the methods described hereinafter, but using the appropriate isotopic variation of suitable reagents or starting materials.

Where the plural form is used for compounds, salts, pharmaceutical compositions, diseases, this is intended to mean also a single compound, salt, composition and disease.

The term "modulate", "modulation" or "modulator" used throughout the current text relate to an increase or to a decrease of the activity of an enzyme or a receptor. The term IDO and/or TDO inhibitor refers to an agent capable of inhibiting the activity of IDO and/or TDO enzymes.

Any reference hereinbefore or hereinafter to a compound of Formula (I) is to be understood as referring also to salts, especially pharmaceutically acceptable salts, of a compound of Formula (I), as appropriate and expedient.

The term "pharmaceutically acceptable salts" refers to salts that retain the desired biological activity of the subject compound and exhibit minimal undesired toxicological effects. Such salts include inorganic or organic acid and/or base addition salts depending on the presence of basic and/or acidic groups in the subject compound. For reference see for example 'Handbook of Pharmaceutical Salts. Properties, Selection and Use.', P. Heinrich Stahl, Camille G. Wermuth (Eds.), Wiley-VCH, 2008, and 'Pharmaceutical Salts and Co-crystals', Johan Wouters and Luc Quéré (Eds.), RSC Publishing, 2012.

The compounds of Formula (I) and their pharmaceutically acceptable salts can be used as medicaments, e.g. in the form of pharmaceutical compositions for enteral (such as especially oral) or parenteral (including topical application or inhalation) administration.

The compounds of Formula (I) are suitable for inhibiting IDO and/or TDO enzymes, and for the prevention and/or treatment of diseases or disorders related to the IDO and/or TDO enzymes (such as especially cancers) in mammals, such as especially humans.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of Formula (I) or their pharmaceutically acceptable salts, optionally in combination with other therapeutically valuable substances, into a galenical administration form together with suitable, non-toxic, inert, pharmaceutically acceptable

solid or liquid carrier materials and, if desired, usual pharmaceutical adjuvants.

In a preferred embodiment of the invention, the administered amount is comprised between 1 mg and 1000 mg per day, particularly between 5 mg and 500 mg per day, more particularly between 25 mg and 400 mg per day, especially between 50 mg and 200 mg per day.

Whenever the word "between" is used to describe a numerical range, it is to be understood that the end points of the indicated range are explicitly included in the range. For example: if a temperature range is described to be between 40 °C and 80 °C, this means that the end points 40 °C and 80 °C are included in the range; or if a variable is defined as being an integer between 1 and 4, this means that the variable is the integer 1, 2, 3, or 4.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10 °C to Y plus 10 °C, and preferably to an interval extending from Y minus 5 °C to Y plus 5 °C.

For avoidance of any doubt, if compounds are described as useful for the prevention or treatment of certain diseases, such compounds are likewise suitable for use in the preparation of a medicament for the prevention or treatment of said diseases.

The present invention also relates to a method for the prevention or treatment of a disease or disorder mentioned hereinabove comprising administering to a subject a pharmaceutically active amount of a compound of Formula (I) either alone or in combination with other pharmacologically active compounds and/or therapies.

The meaning of the term "prevention" may also be understood as "prophylaxis".

One or more compounds of Formula (I) may be used in the prevention and/or treatment of diseases or disorders related to the IDO and/or TDO enzymes; such as especially cancers.

Cancers may be defined as including skin cancer including melanoma; metastatic melanoma; lung cancer including non-small cell lung cancer; bladder cancer including urinary bladder cancer; urothelial cell carcinoma; renal carcinomas including renal cell carcinoma; metastatic renal cell carcinoma; metastatic renal clear cell carcinoma; gastro-intestinal cancers including colorectal cancer; metastatic colorectal cancer; familial adenomatous polyposis (FAP); esophageal cancer; gastric cancer; gallbladder cancer; cholangiocarcinoma; hepatocellular carcinoma; and pancreatic cancer such as pancreatic adenocarcinoma or pancreatic ductal carcinoma; endometrial cancer; ovarian cancer; cervical cancer; neuroblastoma; prostate cancer including castrate-resistant prostate cancer; brain tumors including brain metastases, malignant gliomas, glioblastoma multiforme, medulloblastoma, meningiomas, neuroblastoma, astrocytoma; breast cancer including triple negative breast carcinoma; oral tumors; nasopharyngeal tumors; thoracic cancer; head and neck cancer; mesothelioma; leukemias including acute myeloid leukemia, adult T-cell leukemia; carcinomas; adenocarcinomas; thyroid carcinoma including papillary thyroid carcinoma; choriocarcinoma; sarcomas including Ewing's sarcoma; osteosarcoma; rhabdomyosarcoma; Kaposi's sarcoma; lymphoma including Burkitt's lymphoma, Hodgkin's lymphoma, MALT lymphoma; multiple myelomas; and virally induced tumors.

Further, cancers may be defined as including include brain cancers, skin cancers, bladder cancers, ovarian cancers, breast cancers, gastric cancers, pancreatic cancers, prostate cancers, colon cancers, blood cancers, lung cancers and bone cancers. Examples of such cancer types include neuroblastoma, intestine carcinoma such as rectum carcinoma, colon carcinoma, familiar adenomatous polyposis carcinoma and hereditary non-polyposis colorectal cancer, esophageal carcinoma, labial carcinoma, larynx carcinoma, hypopharynx carcinoma, tongue carcinoma, salivary gland carcinoma, gastric carcinoma, adenocarcinoma, medullary thyroid carcinoma, papillary thyroid carcinoma, renal carcinoma, kidney parenchymal carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, diffuse large B-cell lymphoma (DLBCL), hepatocellular carcinoma, gall bladder carcinoma, bronchial carcinoma, small cell lung carcinoma, non-small cell lung carcinoma, multiple myeloma, basalioma, teratoma, retinoblastoma, choroid melanoma, seminoma, rhabdomyosarcoma, craniopharyngioma, osteosarcoma, chondrosarcoma, myosarcoma, liposarcoma, fibrosarcoma, Ewing sarcoma and plasmacytoma.

Cancers may notably be defined as including skin cancer in particular advanced melanoma and Merkel cell carcinoma; lung cancer including non-small cell lung cancer; bladder cancer; head and neck cancer; renal cell cancer; Hodgkin's lymphoma; cervical cancer; endometrial cancer; breast cancer; colon cancer; gastrointestinal stromal tumors; pancreatic cancer; prostatic cancer; leukemia including acute myeloid leukemia; lymphoma; gastric cancer; ovarian cancer; esophageal carcinomas; hepatocarcinoma; and brain tumors in particular glioblastoma, mesothelioma, neuroblastoma, sarcoma in particular high-grade osteosarcoma, astrocytoma, myeloma.

Cancers may especially be defined as including solid tumors that have specific genetic features, called mismatch repair deficiency and high microsatellite instability; skin cancer, in particular advanced melanoma, Merkel cell carcinoma,

and cutaneous squamous cell carcinoma; lung cancer (especially non-small cell lung cancer (NSCLC)); bladder cancer; advanced cervical cancer; advanced gastric cancer; head and neck cancer; renal cell carcinoma; metastatic colorectal cancer with mismatch repair deficiency (dMMR) or high microsatellite instability (MSI-H); primary mediastinal large B-cell lymphoma; advanced liver cancer; and Hodgkin's lymphoma.

One or more compounds of Formula (I) may be used in the prevention and/or treatment of any cancer, notably the cancers mentioned hereinabove, either alone, or in combination with further pharmacologically active compounds and/or therapies.

In addition to cancers, especially cancers as listed above, further diseases or disorders related to the IDO and/or TDO enzymes may be defined as including neurodegenerative disorders such as Parkinson's disease, Alzheimer's disease, Huntington's disease and Amyotrophic lateral sclerosis; Central nervous system (CNS) disorders such as Psychiatric disorders (schizophrenia, depression); pain; stroke; epilepsy; chronic infectious diseases such as HIV (AIDS including its manifestations such as cachexia, dementia and diarrhea) and HCV; infection and inflammation caused by various bacteria (such as Chlamydia strains and enteropathogenic strains), parasites (such as Trypanosoma, Leishmania, plasmodium) or viruses (such as influenza, human papilloma virus, cytomegalovirus, herpes simplex virus, Epstein-Barr virus, poliovirus, varicella zoster virus and coxsackie virus) as well as other infections (e.g. skin infections, GI infection, urinary tract infections, genito-urinary infections, systemic infections),autoimmune diseases including asthma, rheumatoid arthritis, multiple sclerosis, allergic inflammation, inflammatory bowel disease, psoriasis and systemic lupus erythematosus, organ transplantation (e.g. organ transplant rejection), metabolic disorders such as obesity, type 2 diabetes and/or fatty acid liver disease; cataracts; endometriosis; contraception and abortion.

Further autoimmune diseases include collagen diseases such as rheumatoid arthritis, systemic lupus erythematosus, Sharp's syndrome, CREST syndrome (calcinosis, Raynaud's syndrome, esophageal dysmotility, telangiectasia), dermatomyositis, vasculitis (Morbus Wegener's) and Sjogren's syndrome, renal diseases such as Goodpasture's syndrome, rapidly- progressing glomerulonephritis and membranoproliferative glomerulonephritis type II, endocrine diseases such as type-I diabetes, autoimmune polyendocrinopathy-candidiasis- ectodermal dystrophy (APECED), autoimmune parathyroidism, pernicious anemia, gonad insufficiency, idiopathic Morbus Addison's, hyperthyreosis, Hashimoto's thyroiditis and primary myxedema, skin diseases such as pemphigus vulgaris, bullous pemphigoid, herpes gestationis, epidermolysis bullosa and erythema multiforme major, liver diseases such as primary biliary cirrhosis, autoimmune cholangitis, autoimmune hepatitis type-1, autoimmune hepatitis type-2, primary sclerosing cholangitis, neuronal diseases such as multiple sclerosis, myasthenia gravis, myasthenic Lambert-Eaton syndrome, acquired neuromyotomy, Guillain-Barre syndrome (Muller-Fischer syndrome), stiff-man syndrome, cerebellar degeneration, ataxia, opsoclonus, sensoric neuropathy and achalasia, blood diseases such as autoimmune hemolytic anemia, idiopathic thrombocytopenic purpura (Morbus Werlhof), infectious diseases with associated autoimmune reactions such as AIDS, malaria and Chagas disease.

The terms "radiotherapy" or "radiation therapy" or "radiation oncology", refer to the medical use of ionizing radiation in the prevention (adjuvant therapy) and / or treatment of cancer; including external and internal radiotherapy.

The term "targeted therapy" refers to the prevention / prophylaxis (adjuvant therapy) and / or treatment of cancer with one or more anti-neoplastic agents such as small molecules or antibodies which act on specific types of cancer cells or stromal cells. Some targeted therapies block the action of certain enzymes, proteins, or other molecules involved in the growth and spread of cancer cells. Other types of targeted therapies help the immune system kill cancer cells (immunotherapies); or deliver toxic substances directly to cancer cells and kill them. An example of a targeted therapy which is in particular suitable to be combined with the compounds of the present invention is immunotherapy, especially immunotherapy targeting the programmed death 1 (PD-1) receptor or its ligand PD-L1 (Feig C et al, PNAS 2013).

Immunotherapy further refers to (i) an agonist of a stimulatory (including a co-stimulatory) receptor or (ii) an antagonist of an inhibitory (including a co- inhibitory) signal on T cells, both of which result in amplifying antigen-specific T cell responses (often referred to as immune checkpoint regulators). Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-HI (PD-LI), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-IBBL, CD137 (4-IBB), TRAIL/Apo2-L, TRAILR1/DR4, TRAILR2/DR5, TRAILR3, TRAILR4, OPG, RANK, RANKL, TWEAKR/Fnl4, TWEAK, BAFFR, EDAR, XEDAR, TACI, APRIL, BCMA, LTpR, LIGHT, DcR3, HVEM, VEGI/TLIA, TRAMP/DR3, EDAR, EDAI, XEDAR, EDA2, TNFRI, Lymphotoxin a/TNFp, TNFR2, TNFa, LTPR, Lymphotoxin a 1p2, FAS, FASL, RELT, DR6, TROY, NGFR.

When used in combination with the compounds of Formula (I), the term "targeted therapy" especially refers to agents such as: a) Epidermal growth factor receptor (EGFR) inhibitors or blocking antibodies (for example Gefitinib, Erlotinib, Afatinib, Icotinib, Lapatinib, Panitumumab, Zalutumumab, Nimotuzumab, Matuzumab and Cetuximab) as well as trastuzumab (HERCEPTIN); b) RAS/RAF/MEK pathway inhibitors (for example Vemurafenib, Sorafenib, Dabrafenib, GDC-0879, PLX-4720, LGX818, RG7304, Trametinib (GSK1120212), Cobimetinib (GDC-0973/XL518), Binimetinib (MEK162, ARRY-162), Selumetinib (AZD6244)); c) Janus kinase (JAK) inhibitors ( for example Ruxolitinib, Itacitinib,

Momelotinib); d) Aromatase inhibitors (for example Exemestane, Letrozole, Anastrozole, Vorozole, Formestane, Fadrozole); e); signal transduction inhibitors (STI). A "signal transduction inhibitor" is an agent that selectively inhibits one or more vital steps in signaling pathways, in the normal function of cancer cells, thereby leading to apoptosis. Suitable STIs include but are not limited to: (i) bcr/abl kinase inhibitors such as, for example, STI 571 (GLEEVEC®), Dasatinib; (ii) epidermal growth factor (EGF) receptor inhibitors such as, for example, kinase inhibitors (IRESSA®, SSI-774) and antibodies (Imclone: C225 [Goldstein et al., Clin. Cancer Res., 1:1311-1318 (1995)], and Abgenix: ABX-EGF); (iii) her-2/neu receptor inhibitors such as famesyl transferase inhibitors (FTI) such as, for example, L-744,832 (Kohl et al., Nat. Med., 1(8):792-797 (1995)); (iv) inhibitors of Akt family kinases or the Akt pathway, such as, for example, rapamycin (see, for example, Sekulic et al., Cancer Res., 60:3504-3513 (2000)); (v) cell cycle kinase inhibitors such as, for example, flavopiridol and UCN-O1 (see, for example, Sausville, Curr. Med. Chem. Anti-Cane. Agents, 3:47-56 (2003)); and (vi) phosphatidyl inositol kinase inhibitors such as, for example, LY294002 (see, for example, Vlahos et al., J Biol. Chem., 269:5241-5248 (1994)). f) Angiogenesis inhibitors, especially VEGF signalling inhibitors such as Bevacuzimab (Avastin), Ramucirumab , Sorafenib or Axitinib; g) Immune Checkpoint inhibitors (for example: anti-PD1 antibodies such as Pembrolizumab (Lambrolizumab, MK-3475), Nivolumab, Pidilizumab (CT-011), AMP-514/MEDI0680, PDR001, SHR-1210; REGN2810, BGBA317, PF-06801591, MGA-012, TSR042, JS-001, BCD100, IBI-308, BI-754091; fusion proteins targeting PD-1 such as AMP-224; small molecule anti-PD1 agents such as for example compounds disclosed in WO2015/033299, WO2015/044900 and WO2015/034820; anti-PD1L antibodies, such as BMS-936559, atezolizumab (MPDL3280A, RG7446), avelumab (MSB0010718C), durvalumab (MEDI4736); anti-PDL2 antibodies, such as AMP224; anti-CTLA-4 antibodies, such as ipilimumab, tremelimumab; anti-Lymphocyte-activation gene 3 (LAG-3) antibodies, such as Relatlimab (BMS-986016), IMP701, IMP731, MK-4280, ImmuFact IMP321; anti T cell immunoglobulin mucin-3 (TIM-3) antibodies, such as MBG453, TSR-022; anti T cell immunoreceptor with Ig and ITIM domains (TIGIT) antibodies, such as RG6058 (anti-TIGIT, MTIG7192A); anti- Killer-cell immunoglobulin-like receptors (KIR) for example Lirilumab (IPH2102/BMS-986015), antagonists of Galectins (such as Galectin-1, Galectin-9), BTLA; h) Vaccination approaches (for example dendritic cell vaccination, DNA, peptide or protein vaccination (for example with gp100 peptide or MAGE-A3 peptide) as well as recombinant viruses; i)Re-introduction of patient derived or allogenic (non-self) cancer cells genetically modified to secrete immunomodulatory factors such as granulocyte monocyte colony stimulating factor (GMCSF) gene-transfected tumor cell vaccine (GVAX) or Fms-related tyrosine kinase 3 (Flt-3) ligand gene-transfected tumor cell vaccine (FVAX),or Toll like receptor enhanced GM-CSF tumor based vaccine (TEGVAX); j) T-cell based adoptive immunotherapies, including chimeric antigen receptor (CAR) engineered T-cells (for example CTL019); k) Cytokine or immunocytokine based therapy (for example Interferon alpha, interferon beta, interferon gamma, interleukin 2, interleukin 6, interleukin 10, interleukin 15, TGF-$\beta$); l) Toll-like receptor (TLR) agonists (for example resiquimod, imiquimod, motolimod, glucopyranosyl lipid A, CpG oligodesoxynucleotides); m) Thalidomide analogues (for example Lenalidomide, Pomalidomide); n) Activators of T-cell co-stimulatory receptors (for example anti-CD137/4-1BB antibodies, such as BMS-663513 (urelumab), Utomilumab (PF-05082566); anti-OX40/CD134 (Tumor necrosis factor receptor superfamily, member 4) (such as RG7888 (MOXR0916), 9B12; MEDI6469, GSK3174998, MEDI6383, MEDI0562), anti OX40-Ligand/CD252; anti-glucocorticoid-induced TNFR family related gene (GITR) (such as TRX518, MEDI1873, MK-4166, BMS-986156, BMS-986153) , anti-CD40 (TNF receptor superfamily member 5) antibodies (such as Dacetuzumab (SGN-40), HCD122, CP-870,893, RG7876, ADC-1013, APX005M, SEA-CD40); anti-CD40-Ligand antibodies (such as BG9588); anti-CD27 antibodies such as Varlilumab; anti-CD28 antibodies; anti-ICOS antibodies; o) Molecules binding a tumor specific antigen as well as a T-cell surface marker such as bispecific antibodies or antibody fragments, antibody mimetic proteins such as designed ankyrin repeat proteins (DARPINS), bispecific T-cell engager (BITE, for example AMG103, AMG330); p) Antibodies or small molecular weight inhibitors targeting colony-stimulating factor-1 receptor (CSF-1R) (for example Emactuzumab (RG7155), Cabiralizumab (FPA-008), PLX3397). q) Agents targeting immune cell check points on natural killer cells such as antibodies against Killer-cell immunoglobulin-like receptors (KIR) for example Lirilumab (IPH2102/BMS-986015); r) Agents targeting the Adenosine receptors or the ectonucleases CD39 and CD73 that convert adenosin triphosphate (ATP) to Adenosine, such as MEDI9447 (anti-CD73 antibody), PBF-509; CPI-444 (Adenosine A2a receptor antagonist); s) antagonists to chemokine receptors including CCR2 or CCR4; t) modulators of the complement system v) agents that deplete or inhibit T regulatory cells (e.g., using an anti-CD25 monoclonal antibody (e.g., daclizumab) or by ex *vivo* anti-CD25 bead depletion) or reverse/prevent T cell anergy or exhaustion.

When used in combination with the compounds of Formula (I), immune checkpoint inhibitors such as those listed under f), and especially those targeting the programed cell death receptor 1 (PD-1 receptor) or its ligand PD-L1, are preferred.

The term "chemotherapy" refers to the treatment of cancer with one or more cytotoxic anti-neoplastic agents ("cytotoxic chemotherapy agents"). Chemotherapy is often used in conjunction with other cancer treatments, such as radiation therapy or surgery. The term especially refers to conventional chemotherapeutic agents which act by killing cells that divide rapidly, one of the main properties of most cancer cells. Chemotherapy may use one drug at a time (single-agent chemotherapy) or several drugs at once (combination chemotherapy or polychemotherapy). Chemotherapy using drugs that convert to cytotoxic activity only upon light exposure is called photochemotherapy or photodynamic

therapy.

The term "cytotoxic chemotherapy agent" or "chemotherapy agent" as used herein refers to an active anti-neoplastic agent inducing apoptosis or necrotic cell death. When used in combination with the compounds of Formula (I), the term especially refers to conventional cytotoxic chemotherapy agents such as: 1) alkylating agents (including, without limitation, nitrogen mustards, ethylenimine derivatives, alkyl sulfonates, nitrosoureas and triazenes) such as uracil mustard, mechlorethamine, chlorambucil, cyclophosphamide, ifosfamide, streptozocin, carmustine, lomustine, melphalan, busulfan, procarbazine, dacarbazine, temozolomide, pipobroman, triethylene-melamine, triethylenethiophosphoramine, thiotepa or altretamine; in particular temozolomide); 2) platinum drugs (for example cisplatin, carboplatin or oxaliplatin); 3) antimetabolite drugs (for example 5-fluorouracil, floxuridine, pentostatine, capecitabine, 6-mercaptopurine, methotrexate, gemcitabine, cytarabine, fludarabine or pemetrexed); 4) anti-tumor antibiotics (for example daunorubicin, doxorubicin, epirubicin, idarubicin, actinomycin-D, bleomycin, mitomycin-C or mitoxantrone); 5) mitotic inhibitors (for example paclitaxel, docetaxel, ixabepilone, vinblastine, vincristine, vinorelbine, vindesine or estramustine); or 6) topoisomerase inhibitors (for example etoposide, teniposide, topotecan, irinotecan, diflomotecan or elomotecan). Also suitable are cytotoxic agents such as biological response modifiers; growth inhibitors; antihormonal therapeutic agents; leucovorin; tegafur; and haematopoietic growth factors.

When used in combination with the compounds of Formula (I), preferred cytotoxic chemotherapy agents are the above-mentioned alkylating agents (notably fotemustine, cyclophosphamide, ifosfamide, carmustine, dacarbazine and prodrugs thereof such as especially temozolomide or pharmaceutically acceptable salts of these compounds; in particular temozolomide); mitotic inhibitors (notably paclitaxel, docetaxel, ixabepilone,; or pharmaceutically acceptable salts of these compounds; in particular paclitaxel); platinum drugs (notably cisplatin, oxaliplatin and carboplatin); as well etoposide and gemcitabine. 1) Chemotherapy may be given with a curative intent or it may aim to prolong life or to palliate symptoms. 2) Combined modality chemotherapy is the use of drugs with other cancer treatments, such as radiation therapy or surgery. 3) Induction chemotherapy is the first line treatment of cancer with a chemotherapeutic drug. This type of chemotherapy is used for curative intent. 4) Consolidation chemotherapy is the given after remission in order to prolong the overall disease-free time and improve overall survival. The drug that is administered is the same as the drug that achieved remission. 5) Intensification chemotherapy is identical to consolidation chemotherapy but a different drug than the induction chemotherapy is used. 6) Combination chemotherapy involves treating a patient with a number of different drugs simultaneously. The drugs differ in their mechanism and side effects. The biggest advantage is minimising the chances of resistance developing to any one agent. Also, the drugs can often be used at lower doses, reducing toxicity. 7) Neoadjuvant chemotherapy is given prior to a local treatment such as surgery, and is designed to shrink the primary tumor. It is also given to cancers with a high risk of micrometastatic disease. 8) Adjuvant chemotherapy is given after a local treatment (radiotherapy or surgery). It can be used when there is little evidence of cancer present, but there is risk of recurrence. It is also useful in killing any cancerous cells that have spread to other parts of the body. These micrometastases can be treated with adjuvant chemotherapy and can reduce relapse rates caused by these disseminated cells. 9) Maintenance chemotherapy is a repeated low-dose treatment to prolong remission. 10) Salvage chemotherapy or palliative chemotherapy is given without curative intent, but simply to decrease tumor load and increase life expectancy. For these regimens, a better toxicity profile is generally expected.

**Preparation of compounds of Formula (I):**

The compounds of Formula (I) can be manufactured by the methods given below, by the methods given in the Examples or by analogous methods. Optimum reaction conditions may vary with the particular reactants or solvents used, but such conditions can be determined by a person skilled in the art by routine optimization procedures.

In the schemes below, the generic groups A, $R^1$, $R^2$, $R^5$, $R^6$, X and n are as defined for the compounds of Formula (I). For avoidance of doubt, X refers to halogen or, when comprised in a heterocycle, it refers to nitrogen or CH. In some instances, said generic groups may be incompatible with the assembly illustrated in the schemes, or will require the use of protecting groups (PG). The use of protecting groups is well known in the art (see for example "Protective Groups in Organic Synthesis", T.W. Greene, P.G.M. Wuts, Wiley-Interscience, 1999). For the purposes of this discussion, it will be assumed that such protecting groups as necessary are in place. In some cases, the final product may be further modified, for example, by manipulation of substituents to give a new final product. These manipulations may include, but are not limited to, reduction, oxidation, alkylation, acylation, and hydrolysis reactions which are commonly known to those skilled in the art. The compounds obtained may also be converted into salts, especially pharmaceutically acceptable salts in a manner known in the art.

Compounds of the Formula (I) of the present invention can be prepared according to the general synthetic scheme (Scheme 1) as outlined below.

The synthesis starts by deprotonating ethyl N-(diphenylmethylene) glycinate **(1)** with a strong base such as lithium bis(trimethylsilyl) amide in a solvent like THF at low temperature as for example -78°C. This reactive intermediate is then added to a solution of a carbonyl chloride **2** in a solvent such as THF at low temperature as for example -78°C,

to give, after standard aq. work up and chromatographic purification the amino-ester derivative **3.** Other methods to prepare compound **3** can be used, such as opening of the corresponding oxazole (formed by standard methods) in acidic medium (Scheme 2). Compound **3** is then reacted with the Boc-protected glycine **4.** In this step the acid functionality is activated at low temperature such as -20°C in a solvent such as THF, in the presence of a base such as NMM by forming the mixed anhydride with *i*-butyl chloroformate. To the activated intermediate of **4,** derivative **3** is added at low temperature (e.g. -20°C). After termination of the reaction, a standard aq. work up and chromatographic purification results in the isolation of compound **5.** An alternative way of making compound **5** involves formation of the corresponding diazo compound and reaction with *tert*-butyl (2-amino-2-oxoethyl)carbamate in the presence of a ruthenium catalyst in a solvent such as dichloromethane at a temperature around 40°C (Scheme 2b). Precursor **5** is transformed into the tri-substituted thiazole derivative **6** by reacting it with Lawesson's reagent in a polar aprotic solvent such as THF at reflux for several hours. The thiazole derivative **6** is obtained after standard aq. work up and chromatographic purification. The Boc-protecting group is cleaved off by dissolving **6** in an inert chlorinated solvent such as for example dichloromethane and trifluoroacetic acid is carefully added to the mixture. The reaction is usually fast and the unprotected amine is obtained by evaporating the solvents after about 1 hour of reaction time. Formylation to **7** can be achieved by dissolving the unprotected amine in a solvent such as for example dichloromethane and adjusting the pH with aq. carbonate base solutions to 8. To this mixture is added a mixture of formic acid and acetic anhydride (1/1 molar ratio; 3 equivalents as compared to **6)** at elevated temperature such as for example 50°C. After 60 minutes compound **7** is obtained by a standard aq. work up and used in the next step without further purification. The dehydrating cyclization (condensation) of **7** to **8** is done by dissolving **7** in an inert chlorinated solvent such as dichloromethane and by addition of a dehydrating agent such as for example phosphorous(V) oxychloride followed by stirring the reaction mixture at elevated temperatures (e.g. reflux of dichloromethane) for several hours. The reaction mixture can then be quenched by carefully adding an aq. carbonate base solution and the product **8** is obtained by standard aq. work up followed by chromatographic purification. Aldehyde **9** can be obtained from ester **8** either *via* the corresponding Weinreb amide or *via* the corresponding alcohol. The Weinreb amide is obtained from the ester **8** in a two-step procedure starting with the hydrolysis of the ethylester by dissolving **8** in a THF / water = 2/1 mixture and adding lithium hydroxide monohydrate. The reaction takes place over several hours at RT. The product is isolated by evaporating the mixture to dryness. The following amide coupling is performed by dissolving the obtained residue in a solvent mixture such as for example DMF / dichloromethane (ratio = 1/1 volumes). A base such as for example DIPEA and a coupling reagent such as for example HATU and N,O-dimethylhydroxylamine hydrochloride are added to the acid. The reaction takes place over several hours (e.g. overnight) at RT. The Weinreb amide can be isolated by a standard aq. work up followed by chromatographic purification. Reduction of the Weinreb amide gives aldehyde **9.** Reducing agents such as DIBALH or $LiAlH_4$ can be used in a solvent such as THF, at a temperature between 0°C and RT. Alternatively, aldehyde **9** can be obtained from ester **8** by reduction to the corresponding alcohol using a reducing agent such as $NaBH_4$ in a solvent such as ethanol and at a temperature around RT, followed by oxidation using an oxidizing agent such as Dess-Martin periodinane or $MnO_2$ in a solvent such as $CH_2Cl_2$ or $CH_3CN$ at a temperature of RT or higher.

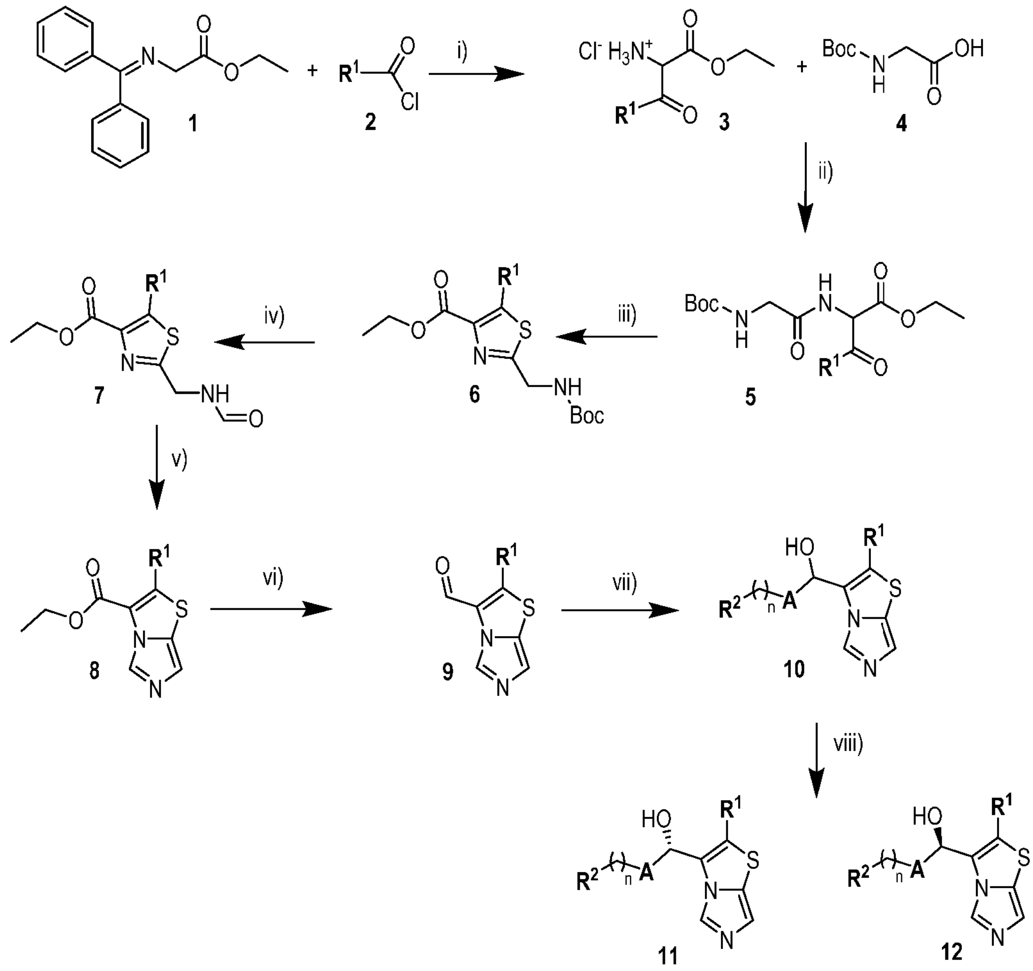


**Scheme 1**

General approach for the preparation of compounds of Formula (I); i) LiHMDS, THF, -78°C; ii) NMM, *i*-butyl chloroformate, -20°C, THF; iii) Lawesson's reagent, THF, rflx; iv) a) DCM, TFA, then b) DCM, pH = 8 (aq $NaHCO_3$), formic acid/formic anhydride = 1/1; v) DCM, POCl3, 40°C; vi) a) THF/water = 2/1, LiOH then b) DMF/DCM = 1/1, DIPEA, HATU, N,O-dimethylhydroxylamine x HCl; c) THF, 0°C, DIBALH in toluene; vii) THF, 0°C, $R^2$-$(CH_2)_n$-A-MgBr or $R^2$-$(CH_2)_n$-A-MgCl; viii) separation of the enantiomers on chiral stationary phases by HPLC.

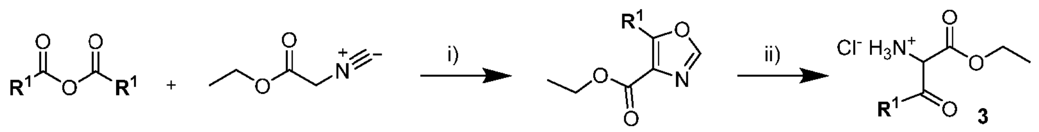


**Scheme 2**

Alternative synthesis of compound 3: i) DBU, DMF, 80°C; ii) HCl 6N, MeOH, 50°C.

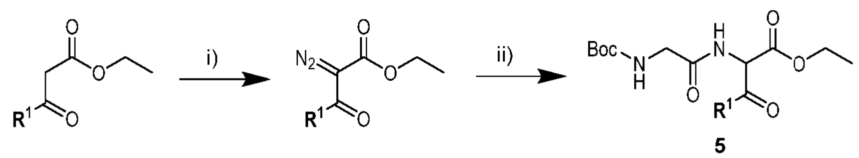


**Scheme 2b**

Alternative synthesis of compound **5:** i) 4-acetamidobenzenesulfonyl azide, TEA, $CH_3CN$, RT; ii) *tert*-butyl (2-amino-2-oxoethyl)-carbamate, dirhodium tetraacetate, $CH_2Cl_2$, 40°C.

Aldehyde **9** is then used in a Grignard reaction in solvents such as THF or ether at low temperatures such as 0°C and by adding $R^2$-$(CH_2)_n$-A-MgBr or $R^2$-$(CH_2)_n$-A-MgCl, either commercially available or synthesized according to known procedures, to obtain racemic alcohol **10.** The racemic compounds can then be separated using chiral preparative HPLC to give alcohols **11** and **12.**

Alternatively, $R^2$-$(CH_2)_n$-A-MgBr or $R^2$-$(CH_2)_n$-A-MgCl can be added onto Weinreb amide **13** to give ketone **14,** which can then be reduced into the corresponding racemic alcohol **10** using a reducing agent such as $NaBH_4$ in a solvent such as ethanol at a temperature ranging from 0°C to RT (Scheme 3). The racemic compounds can then be separated using chiral preparative HPLC to give alcohols **11** and **12.**

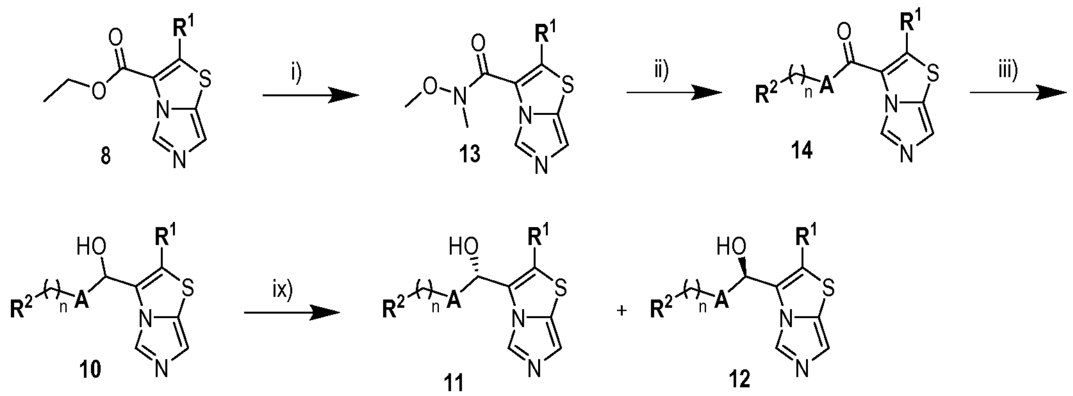


**Scheme 3**

Alternative approach for the preparation of compounds of Formula (I); i) a) THF/water = 2/1, LiOH then b) DMF/DCM = 1/1, DIPEA, HATU, N,O-dimethylhydroxylamine x HCl; ii) $R^2$-$(CH_2)_n$-A-MgBr or $R^2$-$(CH_2)_n$-A-MgCl, THF, 0°C-RT; iii) $NaBH_4$, EtOH; ix) separation of the enantiomers on chiral stationary phases by HPLC.

Aldehyde **9** can be obtained *via* the alternative pathway depicted below (Scheme 4). Starting from methyl 5-bromo-2-methylthiazole-4-carboxylate **15,** bromination using for example *N*-bromosuccinimide and a radical initiator such as AIBN in a solvent such as trifluorotoluene and at a temperature ranging from 85°C to 100°C, gives dibromo compound **16.** The benzylic bromide can be converted into the corresponding formamide for example by reacting it with sodium diformylamide in a solvent such as DMF and at a temperature around 20°C. Formamide **17** can then be cyclized using a dehydrating agent such as $POCl_3$ either neat or in a solvent such as $CH_2Cl_2$ or toluene at a temperature ranging from RT to 100°C to give imidazothiazole **18.** The ester function can then be transformed into the corresponding alcohol using a reducing such as $NaBH_4$ in a solvent such as EtOH at a temperature ranging from 0°C to RT. Protection of the primary alcohol can be carried out using standard protecting group chemistry, for example with a silyl-based protecting group using *tert*butyldimethylsilyl chloride in the presence of a base such as imidazole in a solvent such as DMF or DCM at a temperature such as RT. Metal-catalyzed coupling reactions allow the introduction of $R^1$ substituent using for example boronic acids or esters in the presence of a Pd-based catalyst such as $Pd(PPh_3)_4$ and of a base such as $Na_2CO_3$, in a solvent such as a mixture of dioxane and water at a temperature ranging from RT to 100°C. Removing the silylated protecting group for example using a fluorine source such as TBAF in a solvent such as THF at a temperature around RT gives the corresponding alcohol which can then be oxidized to the aldehyde using an oxidizing agent such as Dess-Martin periodinane in a solvent such as DCM at a temperature ranging from 0°C to RT. Alternatively, metal-catalyzed coupling reactions allowing the introduction of $R^1$ substituent can be performed on the appropriate Weinreb amide **19b** using for example boronic acids or esters in the presence of a Pd-based catalyst such as $Pd(PPh_3)_4$ and of a base such as $Na_2CO_3$, in a solvent such as a mixture of dioxane and water at a temperature ranging from RT to 100°C to give compound **20b.** Aldehyde **9** is then obtained by reduction of Weinreb amide **20b** using a reducing agent such as DIBALH or $LiAlH_4$ in a solvent such as THF, at a temperature between -78°C and RT.

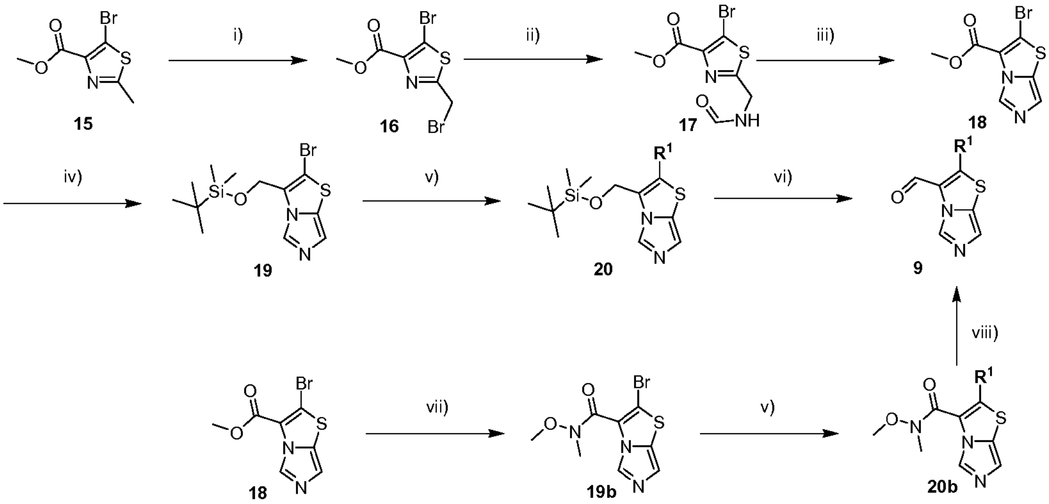


Scheme 4

Introduction of $R^1$ substituents *via* metal-catalyzed coupling reactions; i) NBS, AIBN, trifluorotoluene, 85°C; ii) sodium diformylamide, DMF; iii) $POCl_3$, RT-90°C; iv) a) $NaBH_4$, EtOH, then b) *t*-$BuMe_2SiCl$, imidazole, DMF; v) $R^1$-$B(OH)_2$, $Pd(PPh_3)_4$, $Na_2CO_3$, dioxane; vi) a) TBAF, THF; b) Dess-Martin periodinane, DCM; vii) a) THF/water = 2/1, LiOH then b) DMF/DCM = 1/1, DIPEA, HATU, N,O-dimethylhydroxylamine x HCl; viii) THF, 0°C, DIBALH in toluene.

Alternatively, for compounds of Formula (I) where A is 1H-[1,2,3]triazol-4-yl substituted with $R^2$-$(CH_2)_n$- in position 1, aldehyde **9** can be transformed into propargylic alcohol **21** *via* a Grignard reaction using ethynylmagnesium bromide in a solvent such as THF at a temperature ranging from 0°C to RT (Scheme 5). Alkyne **21** can be then reacted with either commercially available or previously prepared azides in the presence of copper in order to obtain 1,2,3-triazoles **22.** Azides can be prepared using standard methods (for example, from halides or boronic acids). The racemic compounds can then be separated using chiral preparative HPLC to give alcohols **23** and **24.**

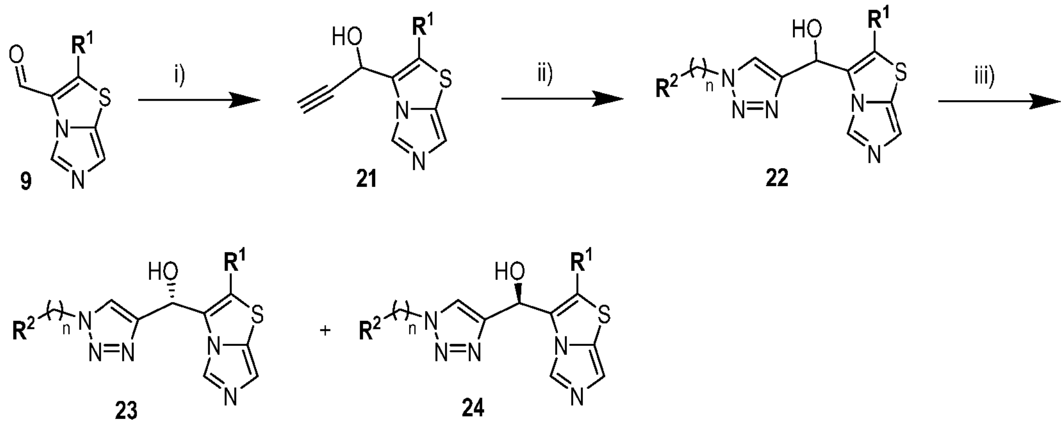


Scheme 5

General approach for the preparation of compounds of Formula (I), where A is 1H-[1,2,3]triazol-4-yl substituted with $R^2$-$(CH_2)_n$- in position 1; i) THF, 0°C, ethynyl-MgBr; ii) $R^2$-$N_3$ for compounds of Formula (I) wherein n is 0; or, in analogy: $R^2$-$CH_2$-$N_3$ for compounds of Formula (I) wherein n is 1: $CuSO_4$, ascorbic acid sodium salt, DMF/$H_2O$; iii) separation of the enantiomers on chiral stationary phases by HPLC.

Alternatively, compounds of Formula (I) can be prepared by reacting bromide **28** with n-BuLi and subsequent addition of an aldehyde (Scheme 6). Bromide **28** can be prepared *via* 3 steps starting from dibromo thiazole **25** (either commercially available or prepared by bromination of the corresponding 2-bromo-thiazole). Lithium halogen exchange can be performed using for example n-BuLi in a solvent such as THF at a temperature around -78°C and subsequent reaction of the lithiated species with an electrophile such as DMF at a temperature ranging from -78°C to RT. Carbaldehyde **26** can then be converted into formamide **27** by standard functional group conversion methods. One way to convert the formyl functional group into the corresponding amine is to convert the aldehyde to the corresponding oxime followed by

reduction of the oxime to the amine using for example zinc as a reducing agent under acidic conditions and formylation using similar conditions as described above. An alternative way is to convert the aldehyde to the formamide *via* the corresponding chloride. First, reduction of the aldehyde to the alcohol can be carried out using $NaBH_4$ as a reducing agent in a solvent such as EtOH at a temperature ranging from 0°C to RT, then conversion of the resulting alcohol functional group into the corresponding chloride using for example thionyl chloride in a solvent such as DCM and at a temperature around 20°C, and finally substitution of the chloride by sodium diformylamide (followed by elimination of one of the 2 formyl groups). Cyclization of formylated amine **27** using a dehydrating agent such as $POCl_3$ either neat or in a solvent such as DCM at a temperature ranging from 0°C to 90°C. Bromide **28** can then be reacted first with n-BuLi in a solvent such as THF at a temperature around -78°C and then with $R^2$-$(CH_2)_n$-A-CHO to give alcohol **10.** The racemic compounds can then be separated using chiral preparative HPLC to give alcohols **11** and **12**

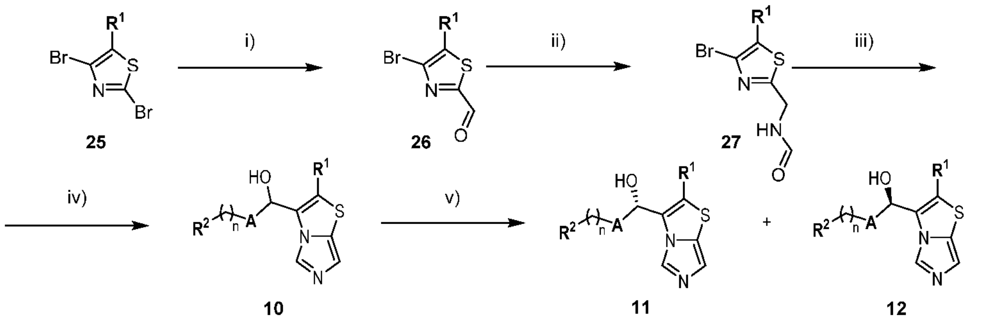


**Scheme 6**

General approach for the preparation of compounds of Formula (I); i) a) n-BuLi, THF, -78°C; then b) DMF; ii) a) $NaBH_4$, EtOH; b) $SOCl_2$, DCM; c) sodium diformylamide, DMF; iii) POCl3, RT-90°C; iv) a) n-BuLi, THF, -78°C; then b) $R^2$-$(CH_2)_n$-A-CHO; v) separation of the enantiomers on chiral stationary phases by HPLC.

Alternatively, a protecting/directing group strategy can be used to prepare compounds of Formula (I) (Scheme 7). Primary amine **29** (either commercially available or synthesized using standard procedures) is cyclized using thiophosgene in the presence of a base such as $K_2CO_3$ and alkylated at the thiol group using for example ethyl iodide (EtI) in the presence of a base such as $K_2CO_3$ to give imidazothiazole **30.** Deprotonation using a base such as n-BuLi in a solvent such as THF at a temperature around -78°C, and subsequent addition of $R^2$-$(CH_2)_n$-A-CHO give alcohol **31.** Removal of the thioether function is performed using a catalyst such as Raney nickel in a solvent such as a mixture of ethanol and water, at a temperature ranging from RT to 90°C to give compound **10.** The racemic compounds can then be separated using chiral preparative HPLC to give alcohols **11** and **12.**

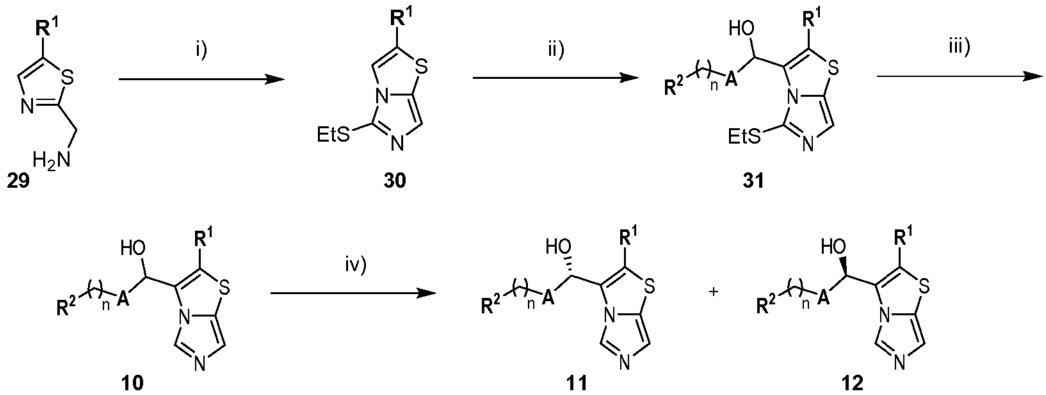


**Scheme 7**

Directing group approach for the preparation of compounds of Formula (I); i) a) thiophosgene, $K_2CO_3$, DCM/$H_2O$, RT; then b) EtI, $K_2CO_3$, acetone; ii) a) n-BuLi, THF, -78°C; then b) $R^2$-$(CH_2)_n$-A-CHO; iii) Raney nickel, EtOH/$H_2O$, RT-90°C; iv) separation of the enantiomers on chiral stationary phases by HPLC.

The aldehyde ($R^2$-$(CH_2)_n$-A-CHO) used in the lithiation/addition reaction, if not commercially available, can be prepared by standard methods, two of them are described in Scheme 8 and Scheme 9. Aldehyde **34** can be prepared

by copper-catalyzed coupling of an appropriately substituted heterocycle and a suitable boronic acid to give compound **33** (Scheme 8). Ester **33** can be reduced to the alcohol, which can be subsequently oxidized to said aldehyde. Alternatively, ester **33** can be transformed into the corresponding Weinreb amide, which can in turn be reduced to aldehyde **34.**

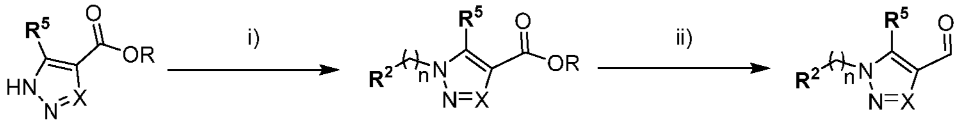


**32** R = Me, Et

**33**

**34** X = N or CH

**Scheme 8**

Preparation of $R^2$-$(CH_2)_n$-A-CHO **34** by copper-catalyzed coupling; i) $R^2$-$B(OH)_2$ for compounds of Formula (I) wherein n is 0; or, in analogy: $R^2$-$CH_2$-$B(OH)_2$ for compounds of Formula (I) wherein n is 1, $Cu(OAc)_2$, pyridine, DMF, RT; ii) a) reduction; b) oxidation.

Alternatively, aldehyde **34** can be made using a cycloaddition approach as described in Scheme 9.

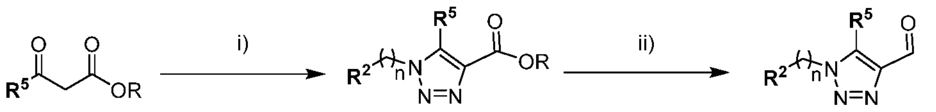


**35** R = Me, Et

**33**

**34**

**Scheme 9**

Preparation of aldehyde **34** by cycloaddition reaction; i) $R^2$-$N_3$ for compounds of Formula (I) wherein n is 0; or, in analogy: $R^2$-$CH_2$-$N_3$ for compounds of Formula (I) wherein n is 1, $Et_3N$, DMF, RT; ii) a) reduction; b) oxidation.

Alternatively, compounds of Formula (I) can be prepared by alkylation of **36** (Scheme 10) using standard alkylation conditions such as a halide ($R^6$-X) in the presence of NaI and a base such as $K_2CO_3$ in a solvent such as DMF at a temperature ranging from RT to 100°C. The racemic compounds **37** can then be separated using chiral preparative HPLC to give alcohols **38** and **39.**

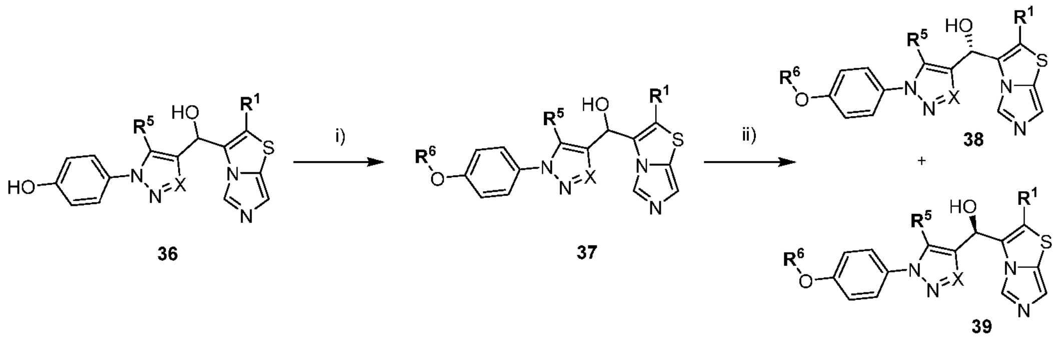


**36**

**37**

**38**

\+

**39**

**Scheme 10**

Preparation of compounds of Formula (I) *via* alkylation of hydroxy aryl compounds; i) $R^6$-X, wherein X is halogen; $K_2CO_3$; NaI; DMF; RT-100°C; ii) separation of the enantiomers on chiral stationary phases by HPLC.

Alternatively, compounds of Formula (I) where $R^5$ = I can be prepared by cycloaddition in the presence of copper of the appropriate alkynyl iodide **40** and a suitable azide $R^2$-$N_3$ for compounds of Formula (I) wherein n is 0; or, in analogy: $R^2$-$CH_2$-$N_3$ for compounds of Formula (I) wherein n is 1 (Scheme 11). Compounds of Formula (I) where $R^5$ = F can then be prepared by fluorination of iodide **41** using for example silver (I) fluoride as a source of fluoride in the presence of a base such as *N,N,N,N*-tetramethylethylenediamine in a solvent such as toluene and at a temperature ranging from RT to 110°C.

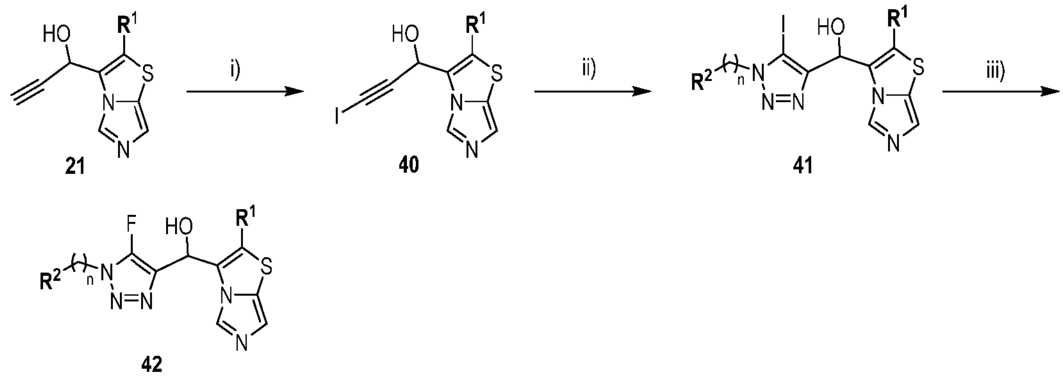


21 40 41 42

**Scheme 11**

General approach for the preparation of compounds of Formula (I), where A is 1H-[1,2,3]triazol-4-yl substituted with $R^5$ = halide; i) *N*-iodomorpholine, CuI, THF, RT; ii) $R^2$-$N_3$ for compounds of Formula (I) wherein n is 0; or, in analogy: $R^2$-$CH_2$-$N_3$ for compounds of Formula (I) wherein n is 1: $CuSO_4$, ascorbic acid sodium salt, DMF/$H_2O$; iii) AgF, *N,N,N,N*-tetramethylethylenediamine, toluene, RT-110°C.

Whenever the compounds of Formula (I) are obtained in the form of mixtures of enantiomers, the enantiomers can be separated using methods known to one skilled in the art: e.g. by formation and separation of diastereomeric salts or by HPLC over a chiral stationary phase such as a Regis Whelk-O1(R,R) (10 μm) column, a Daicel ChiralCel OD-H (5-10 μm) column, or a Daicel ChiralPak IA (10 μm), IA, IB, IC, IE, or IF (5 μm) or AD-H (5 μm) column. Typical conditions of chiral HPLC are an isocratic mixture of eluent A (EtOH, in presence or absence of an amine such as triethylamine or diethylamine) and eluent B (heptane), at a flow rate of 0.8 to 150 mL/min.

The following examples are provided to illustrate the invention. These examples are illustrative only and should not be construed as limiting the invention in any way.

## Experimental Part

### Chemistry

All temperatures are stated in °C.

### Preparative HPLC conditions:

The conditions for preparative HPLC purifications were chosen among the possibilities given below depending on the properties of the compounds to be purified. More than one option per problem can lead to a successful result. **Equipment:** HPLC pumps: Gilson 333/334 or equivalent Autosampler: Gilson LH215 (with Gilson 845z injector) or equivalent Degasser: Dionex SRD-3200 or equivalent Make-up pump: Dionex ISO-3100A or equivalent DAD detector: Dionex DAD-3000 or equivalent MS detector: Single quadrupole mass analyzer Thermo Finnigan MSQ Plus or equivalent MRA splitter: MRA100-000 flow splitter or equivalent ELS detector: Polymer Laboratories PL-ELS1000 or equivalent. **Method:** Column: variable Waters Atlantis T3 30x75 mm 10 μm (acidic conditions only); Waters XBridge C18, 30 x 75 mm 10 μm (acidic/basic conditions); Waters XBridge C18, 50 x 150 mm 10 μm (acidic/basic conditions); Flow rate: variable 75 mL/min (for columns with dimension 30x75 mm), 150 ml/min (for columns with dimension 50x150 mm). Mobile phase: gradient mode A: Water + 0.5% formic acid (acidic conditions) A: Water + 0.5% ammonium hydroxide solution (25%) (basic conditions) B: Acetonitrile Gradient: variable, e.g. for 75 mL/min: "extremely polar": t[min] %A %B Flow mL/min: 0.000 100 0 75; 1.000 100 0 75; 3.500 80 20 75; 4.000 5 95 75; 6.000 5 95 75; 6.200 100 0 75; 6.600 100 0 75. "very polar": t[min] %A %B Flow mL/min: 0.000 95 5 75; 0.100 95 5 75; 3.000 50 50 75; 4.000 5 95 75; 6.000 5 95 75; 6.200 95 5 75; 6.600 95 5 75; "polar": t[min] %A %B Flow mL/min: 0.000 90 10 75; 0.010 90 10 75; 4.000 5 95 75; 6.000 5 95 75; 6.200 90 10 75; 6.600 90 10 75; "normal": t[min] %A %B Flow mL/min: 0.000 80 20 75; 0.010 80 20 75; 4.000 5 95 75; 6.000 5 95 75; 6.200 80 20 75; 6.600 80 20 75; "lipophilic": t[min] %A %B Flow mL/min: 0.000 70 30 75; 0.010 70 30 75; 3.500 5 95 75; 6.000 5 95 75; 6.200 70 30 75; 6.600 70 30 75; "very lipophilic": t[min] %A %B Flow mL/min: 0.000 50 50 75; 0.010 50 50 75; 3.000 5 95 75; 6.000 5 95 75; 6.200 50 50 75; 6.600 50 50 75. Injection volume: 100-2500 μL. Collection: UV / MS / ELSD if available, and all possible combinations; Make-up flow rate: 0.50mL/min. Make-up eluent MS: acetonitrile/water/TFA 70:30:0.025 (V/V/V); MS ionization mode: ESI+.

**LC-MS-conditions:**

*Basic conditions:* Column: Waters BEH C18, 3.0x50 mm, 2.5μm/01593635616710; Temperature: 40°C; Injection volume: 0.30 μl Eluent A: water/$NH_3$ with c($NH_3$) = 13 mmol/l; Eluent B: Acetonitrile; Ionisation: ESI+; Gradient: at 0.0 min = 5% B, at 0.01 min = 5% B, at 1.20 min = 95% B, at 1.90 min = 95% B, at 2.00 min = 5% B; Flow = 1.6 ml/min.

*Acidic conditions*: Column: Zorbax RRHD SB-Aq, 2.1x50 mm, 1.8μm/USEAF01579; Temperature: 40°C; Injection volume: 0.15 μl; Eluent A: water 0.04% TFA; Eluent B: Acetonitrile; Ionisation: ESI+; Gradient: at 0.0 min = 5% B, at 0.01 min = 5% B, at 1.20 min = 95% B, at 1.90 min = 95% B, at 2.10 min = 5% B; Flow = 0.8 ml/min.

QC *conditions*: Column: Acquity UPLC CSH C18 1.7 μm 2.1x50 mm; Temperature: 60°C; Injection volume: 0.25 μl partial loop 2 μl Eluent A1: H2O + 0.05% v/v Formic Acid; Eluent B1 : Acetonitrile + 0.045% v/v Formic Acid; Ionisation: ESI+; Gradient: at 0.0 min = 2% B1, at 1.4 min = 5% A1, at 1.90 min = 2% A1, at 2.00 min = 2% B1; Flow = 1.0 ml/min.

**Abbreviations** (as used hereinbefore or hereinafter):

| | |
|---|---|
| Ac | acetate |
| aq. | aqueous |
| AIBN | azobisisobutyronitrile |
| BRP | back pressure regulator |
| BSA | bovine serum albumin |
| Boc | *tert*-butyloxycarbonyl |
| Boc-Gly-OH | Boc-glycine |
| DBU | 1,8-diazabicyclo[5.4.0]undec-7-ene |
| DCM | dichloromethane |
| DEA | diethylamine |
| DIBALH | diisobutylaluminium hydride |
| DIPEA | diisopropyl ethyl amine (Hünig's base) |
| DMEM | Dulbecco's modified eagle medium |
| DMF | dimethyl formamide |
| DMSO | dimethylsulfoxide |
| Et | ethyl |
| EtOAc | ethyl acetate |
| $Et_2O$ | diethyl ether |
| EtOH | ethanol |
| FC | flash chromatography |
| FCS | fetal calf serum |
| h | hour(s) |
| HATU | (1-[Bis(dimethylamino)methylene]-1*H*-1,2,3-triazolo[4,5-*b*]pyridinium 3-oxid hexafluorophosphate) |
| HV | high vacuum |
| LiHMDS | lithium bis(trimethylsilyl)amide |
| MeOH | methanol |
| min. | minute(s) |
| ml or mL | milliliters |
| NBS | *N*-bromo succinimide |
| n-Bu | n-butyl |
| NMM | *N*-methyl-morpholine |
| org. | organic |
| prepHPLC | preparative HPLC |
| RT | room temperature |
| rflx | reflux |
| sat. | saturated |
| SFC | supercritical fluid chromatography |
| TBAF | tetrabutylammonium fluoride |
| TFA | trifluoroacetic acid |
| THF | tetrahydrofuran |
| $t_R$ | HPLC retention time in minutes |

**INTERMEDIATES SYNTHESIS**

**Intermediate 1: *rac*-1-(2-Cyclopropylimidazo[5,1-b]thiazol-3-yl)prop-2-yn-1-ol**

Step 1: Preparation of ethyl 2-amino-3-cyclopropyl-3-oxopropanoate hydrochloride

In a first reaction vessel, ethyl *N*-(diphenylmethylene)glycinate (1069 mg; 4 mmol) is dissolved in THF (4 ml) and cooled to -78°C followed by the dropwise addition of a 1M THF solution of lithium bis(trimethylsilyl) amide (4 ml; 4 mmol). Stirring is continued at -78°C for 1 h. In a second reaction vessel, cyclopropanecarbonyl chloride (0.389 ml; 4.2 mmol) is dissolved in THF (4 ml) and cooled to -78°C. Then the reaction mixture obtained in the first vessel is slowly added to the content of the second vessel. The resulting reaction mixture is warmed to RT over 3 h followed by quenching the reaction by careful addition of 2M aq. HCl (4 ml). The THF is evaporated under reduced pressure and the remaining aq. phase is extracted twice with EtOAc. The aq. phase is concentrated under reduced pressure. The residue is treated with EtOH and filtered and the filtrate is again concentrated under reduced pressure and dried at HV overnight. 890 mg of ethyl 2-amino-3-cyclopropyl-3-oxopropanoate hydrochloride is obtained as a pale yellow solid. LC-MS (basic): $t_R$ = 0.57; $[M+H]^+$ = 172.01.

Step 2: Preparation of ethyl 2-(2-((*tert*-butoxycarbonyl)amino)acetamido)-3-cyclopropyl-3-oxopropanoate

In a first vessel, Boc-Gly-OH (779 mg; 4.4 mmol) is dissolved in THF (4 ml) and cooled to -20°C followed by the addition of NMM (0.494 ml; 4.4 mmol) and isobutyl chloroformate (0.582 ml; 4.4 mmol) and stirring is continued for 30 minutes at -20°C. In a second vessel, the product from step 1, ethyl 2-amino-3-cyclopropyl-3-oxopropanoate hydrochloride (831 mg; 4 mmol) is dissolved in THF (2 ml). This solution is carefully added to the previously prepared solution of the mixed anhydride in vessel one followed by the dropwise addition of NMM (0.494 ml; 4.4 mmol). The reaction mixture is warmed to RT and stirring is continued for 60 minutes. The reaction is quenched by the addition of water. The product is extracted with EtOAc (2x 25 ml). The combined organic layers are dried over $MgSO_4$, filtered and the solvents are evaporated under reduced pressure. The residue is purified by FC (Silicagel; DCM / MeOH = 95 / 5) to give 531.2 mg of ethyl 2-(2-((*tert*-butoxycarbonyl)amino)acetamido)-3-cyclopropyl-3-oxopropanoate as a slightly yellow, thick oil. LC-MS (basic): $t_R$ = 0.84; $[M+H]^+$ = 329.17.

Step 3: Preparation of ethyl 2-(((*tert*-butoxycarbonyl)amino)methyl)-5-cyclopropylthiazole-4-carboxylate (see also *J. Med. Chem.,* **1996,** 39, 957-967 for general procedure)

The product from step 2, ethyl 2-(2-((*tert*-butoxycarbonyl)amino)acetamido)-3-cyclopropyl-3-oxopropanoate (531 mg; 1.62 mmol) and Lawesson's reagent (1011 mg; 2.43 mmol) are suspended in THF (10 ml) and heated to reflux for 4 hours. The THF is evaporated under reduced pressure and the residue is taken up into EtOAc, washed with saturated aq. $NaHCO_3$ solution and brine, dried over $Na_2SO_4$, filtered and the solvent is evaporated under reduced pressure. The residue is purified by FC (Silicagel; EtOAc / heptane = 1 / 1) to give 403 mg of ethyl 2-(((tert-butoxycarbonyl)amino)methyl)-5-cyclopropylthiazole-4-carboxylate. LC-MS (basic): $t_R$ = 1.01; $[M+H]^+$ = 327.12.

Step 4: Preparation of ethyl 5-cyclopropyl-2-(formamidomethyl)thiazole-4-carboxylate

Step 4.1: Boc-cleavage: The product from step 3, ethyl 2-(((*tert*-butoxycarbonyl)amino)methyl)-5-cyclopropylthiazole-4-carboxylate (403 mg; 1.23 mmol) is dissolved in DCM (3 ml) followed by careful addition of TFA (3 ml) and stirring is continued for 30 minutes. Then the reaction mixture is evaporated to dryness under reduced pressure.

Step 4.2: The residue from step 4.1 is dissolved in DCM (7 ml) and saturated aq. $NaHCO_3$ solution is added until the pH is 8. Under vigorous stirring at 50°C, a mixture of formic acid (0.319 ml; 8.2 mmol) and acetic anhydride (0.319 ml; 3.34 mmol) is added and stirring is continued for 1 hour. The organic layer is separated and the aq. layer is extracted twice with DCM (2 $\times$ 7 ml). The combined organic layers are dried over $MgSO_4$, filtered and the solvent is evaporated under reduced pressure to give 390 mg of ethyl 5-cyclopropyl-2-(formamidomethyl)thiazole-4-carboxylate which is used without further purification in Step 5. LC-MS (basic): $t_R$ = 0.69; $[M+H]^+$ = 255.13.

Step 5: Preparation of ethyl 2-cyclopropylimidazo[5,1-*b*]thiazole-3-carboxylate

The product from step 4.2, ethyl 5-cyclopropyl-2-(formamidomethyl)thiazole-4-carboxylate (313 mg; 1.23 mmol) is dissolved in DCM (5 ml) and cooled to -20°C followed by the addition of phosphorus oxychloride (0.232 ml; 2.46 mmol). The reaction mixture is slowly heated to 65°C and kept at this temperature for 5 hours. Then the mixture is evaporated to dryness under reduced pressure, the residue is taken up in DCM followed by careful addition of saturated aq. $NaHCO_3$

solution (pH = 8). The organic layer is separated, the aq. layer is washed with DCM (2 × 10 ml). The combined organic layers are dried over $MgSO_4$, filtered and the solvent is evaporated under reduced pressure. The residue is purified by FC (Silicagel; EtOAc / MeOH = 9 / 1) to give 216 mg of ethyl 2-cyclopropylimidazo[5,1-b]thiazole-3-carboxylate. LC-MS (basic): $t_R$ = 0.91; $[M+H]^+$ = 237.11.

Step 6: Preparation of 2-cyclopropyl-*N*-methoxy-*N*-methylimidazo[5,1-*b*]thiazole-3-carboxamide

Step 6.1: Ester hydrolysis: The product form step 5, ethyl 2-cyclopropylimidazo[5,1-b]thiazole-3-carboxylate (216 mg; 0.914 mmol) is dissolved in THF (2 ml) and water (1 ml) followed by the addition of LiOH monohydrate (46 mg; 1.1. mmol) and stirring at RT is continued for 1 hour. A second portion of lithium hydroxide monohydrate (46 mg; 1.1 mmol) is added and the reaction mixture is stirred for another hour. The mixture is evaporated to dryness under reduced pressure.

Step 6.2: The residue from step 6.1 is dissolved in a 1 / 1 mixture of DMF / DCM (6 ml in total) followed by the subsequent addition of DIPEA (0.469 ml; 2.74 mmol), HATU (417 mg; 1.1 mmol) and N,O-dimethylhydroxylamine hydrochloride (109 mg; 1.1 mmol). Stirring is continued at RT overnight. The reaction mixture is concentrated under reduced pressure and the residue is purified by FC (Silicagel; EtOAc / MeOH = 9 / 1) to give 294 mg of 2-cyclopropyl-N-methoxy-N-methylimidazo[5,1-b]thiazole-3-carboxamide. LC-MS (basic): $t_R$ = 0.71; $[M+H]^+$ = 252.14.

Step 7: Preparation of 2-cyclopropylimidazo[5,1-b]thiazole-3-carbaldehyde

To an ice-cold solution of the product from Step 6.2, 2-cyclopropyl-N-methoxy-N-methylimidazo[5,1-b]thiazole-3-carboxamide (2.875 g; 10.9 mmol) in THF (40 ml) is added a solution of DIBALH (1 M in toluene, 10.9 ml, 10.9 mmol). The reaction mixture is stirred at 0°C for 30 min, treated with more DIBALH (1 M in toluene, 10.9 ml, 10.9 mmol), and further stirred at 0°C for 30 min. A sat. aq. $NH_4Cl$ solution is added followed by a 1.2 M aq. solution of Rochelle's salts and the product is extracted with EtOAc (3x). The combined org. extracts are dried over $MgSO_4$, filtered and concentrated under reduced pressure to give 2.657 g of 2-cyclopropylimidazo[5,1-b]thiazole-3-carbaldehyde. The crude product can be used without further purification. FC (Silicagel; Hept / EtOAc) gives the pure product. LC-MS (acidic): $t_R$ = 0.44; $[M+H]^+$ = 193.03. $^1$H NMR (500 MHz, $d_6$-DMSO) $\delta$: 10.14 (s, 1 H), 8.52 (d, J = 0.5 Hz, 1 H), 7.15 (d, J = 0.6 Hz, 1 H), 2.92 (m, 1 H), 1.30-1.34 (m, 2 H), 0.98 (m, 2 H).

Step 8: Preparation of *rac*-1-(2-cyclopropylimidazo[5,1-*b*]thiazol-3-yl)prop-2-yn-1-ol (intermediate 1)

To an ice-cold solution of the product from Step 7, 2-cyclopropylimidazo[5,1-b]thiazole-3-carbaldehyde (1000 mg; 5.20 mmol) in THF (20 ml) is added a solution of ethynylmagnesium bromide (0.5 M in THF, 31.2 ml, 15.6 mmol) in a dropwise manner. The reaction mixture is stirred at 0°C for 1 h. The reaction is quenched by careful addition of aq. ammonium chloride solution. The product is extracted with EtOAc (3 × 20 ml) and the combined organic extracts are dried over $MgSO_4$, filtered and concentrated under reduced pressure. The residue is purified by preparative HPLC (basic conditions) to give 689 mg of rac-1-(2-cyclopropylimidazo[5,1-*b*]thiazol-3-yl)prop-2-yn-1-ol. LC-MS (basic): $t_R$ = 0.66; $[M+H]^+$ = 219.01. $^1$H NMR (500 MHz, $d_6$-DMSO) $\delta$: 8.14 (d, J = 0.6 Hz, 1 H), 7.02 (d, J = 0.6 Hz, 1 H), 6.54 (d, J = 4.5 Hz, 1 H), 5.91 (dd, $J_1$ = 2.3 Hz, $J_2$ = 4.5 Hz, 1 H), 3.64 (d, J = 2.3 Hz, 1 H), 2.25 (m, 1 H), 1.05 (m, 2 H), 0.65-0.74 (m, 2 H).

**Intermediate 2: *rac*-1-(2-Methylimidazo[5,1-*b*]thiazol-3-yl)prop-2-yn-1-ol**

Step 1: Preparation of ethyl 2-amino-3-oxobutanoate hydrochloride

According to the procedure described for the preparation of Intermediate 1/Step 1 but using acetyl chloride as starting material, 4.84 g of ethyl 2-amino-3-oxobutanoate hydrochloride are obtained. LC-MS (basic): $t_R$ = 0.41; $[M+H]^+$ = 146.15.

Step 2: Preparation of ethyl 2-(2-((*tert*-butoxycarbonyl)amino)acetamido)-3-oxobutanoate

According to the procedure described for the preparation of Intermediate 1/Step 2 but using ethyl 2-amino-3-oxobutanoate hydrochloride as starting material, 2.77 g of ethyl 2-(2-((*tert*-butoxycarbonyl)amino)acetamido)-3-oxobutanoate are obtained. LC-MS (basic): $t_R$ = 0.61; $[M+H]^+$ = 303.2.

Step 3: Preparation of ethyl 2-(((*tert*-butoxycarbonyl)amino)methyl)-5-methylthiazole-4-carboxylate

According to the procedure described for the preparation of Intermediate 1/Step 3 but using ethyl 2-(2-((*tert*-

butoxycarbonyl)amino)acetamido)-3-oxobutanoate as starting material, 2.28 g of ethyl 2-(((*tert*-butoxycarbonyl)amino)methyl)-5-methylthiazole-4-carboxylate are obtained. LC-MS (basic): $t_R$ = 0.94; $[M+H]^+$ = 301.15.

Step 4: Preparation of ethyl 2-(formamidomethyl)-5-methylthiazole-4-carboxylate

According to the procedure described for the preparation of Intermediate 1/Step 4 but using ethyl 2-(((*tert*-butoxycarbonyl)amino)methyl)-5-methylthiazole-4-carboxylate as starting material, 1.727 g of ethyl 2-(formamidomethyl)-5-methylthiazole-4-carboxylate are obtained. LC-MS (basic): $t_R$ = 0.60; $[M+H]^+$ = 229.13.

Step 5: Preparation of ethyl 2-methylimidazo[5,1-*b*]thiazole-3-carboxylate

According to the procedure described for the preparation of Intermediate 1/Step 5 but using ethyl 2-(formamidomethyl)-5-methylthiazole-4-carboxylate as starting material, 1.044 g of ethyl 2-methylimidazo[5,1-*b*]thiazole-3-carboxylate are obtained. LC-MS (basic): $t_R$ = 0.81; $[M+H]^+$ = 211.15.

Step 6: Preparation of (2-methylimidazo[5,1-b]thiazol-3-yl)methanol

To an ice-cold solution of ethyl 2-methylimidazo[5,1-b]thiazole-3-carboxylate (1.675 g; 7.97 mmol) in THF (15 ml) is added $LiAlH_4$ (2 M in THF, 3.98 ml; 7.96 mmol). The reaction mixture is stirred at RT for 10 min, poured into water and extracted with EtOAc (2x). The combined organic extracts are dried over $MgSO_4$, filtered and the solvent is evaporated under reduced pressure to give 913 mg of (2-methylimidazo[5,1-b]thiazol-3-yl)methanol as a brown solid which is used in the next step without further purification. LC-MS (acidic): $t_R$ = 0.34; $[M+H]^+$ = 168.98.

Step 7: Preparation of 2-methylimidazo[5,1-b]thiazole-3-carbaldehyde

To a solution of the product from Step 6, (2-methylimidazo[5,1-*b*]thiazol-3-yl)methanol (1.549 g, 9.21 mmol) in $CH_2Cl_2$ (10 ml) is added Dess-Martin periodinane (4.101 g, 9.67 mmol). The resulting suspension is stirred at RT or 1 h. Sat. aq. $NaHCO_3$ solution (20 ml) and sat. aq. $Na_2S_2O_3$ (20 ml) are added and the mixture is extracted with $CH_2Cl_2$ (2x). The combined organic extracts are dried over $MgSO_4$, filtered and concentrated under reduced pressure to give 1.376 g of 2-methylimidazo[5,1-b]thiazole-3-carbaldehyde. LC-MS (acidic): $t_R$ = 0.34; $[M+H]^+$ = 167.01.

Step 8: Preparation of *rac*-1-(2-methylimidazo[5,1-*b*]thiazol-3-yl)prop-2-yn-1-ol (intermediate 2)

To an ice-cold solution of the product from Step 7, 2-methylimidazo[5,1-*b*]thiazole-3-carbaldehyde (249 mg; 1.50 mmol) in THF (6 ml) is added a solution of ethynylmagnesium bromide (0.5 M in THF, 9.0 ml, 4.50 mmol) in a dropwise manner. The reaction mixture is stirred at 0°C for 1.5 h. The reaction is quenched by careful addition of aq. ammonium chloride solution. The product is extracted with EtOAc (3 $\times$ 20 ml) and the combined organic extracts are dried over $MgSO_4$, filtered and concentrated under reduced pressure. The residue is triturated with $CH_2Cl_2$ / $Et_2O$ then filtered to give 233 mg of rac-1-(2-methylimidazo[5,1-b]thiazol-3-yl)prop-2-yn-1-ol. LC-MS (acidic): $t_R$ = 0.41; $[M+H]^+$ = 193.16. $^1H$ NMR (500 MHz, $d_6$-DMSO) $\delta$: 8.16 (d, J = 0.2 Hz, 1 H), 7.02 (d, J = 0.2 Hz, 1 H), 6.46 (d, J = 4.5 Hz, 1 H), 5.81 (dd, $J_1$ = 2.3 Hz, $J_2$ = 4.5 Hz, 1 H), 3.63 (d, J = 2.3 Hz, 1 H), 2.37 (s, 3 H).

**Intermediate 3: *rac*-1-(2-Ethylimidazo[5,1-*b*]thiazol-3-yl)prop-2-yn-1-ol**

Step 1: Preparation of ethyl 2-amino-3-oxopentanoate hydrochloride

To a solution of ethyl isocyanoacetate (2.8 ml, 25.1 mmol) in DMF (25 ml) is added DBU (5.75 ml, 37.7 mmol) followed by propionic anhydride (4.3 ml, 32.5 mmol). The resulting dark brown solution is stirred at 80°C for 4 h. The mixture is cooled down to RT, poured into water (150 ml) and extracted with EtOAc (3x). The combined organic extracts are washed with water, dried over $MgSO_4$, filtered and concentrated under reduced pressure. The crude residue is purified by FC (siliga gel, Hept / EtOAc) to give 3.767 g of ethyl 5-ethyloxazole-4-carboxylate as a pale yellow oil. LC-MS (acidic): $t_R$ = 0.72, $[M+H]^+$ = 170.08. $^1H$ NMR (500 MHz, $COCl_3$) $\delta$: 7.77 (s, 1 H), 4.39 (q, *J* = 7.1 Hz, 2 H), 3.09 (q, *J* = 7.6 Hz, 2 H), 1.41 (t, *J* = 7.1 Hz, 3 H), 1.29 (t, *J* = 7.6 Hz, 3 H). To a solution of ethyl 5-ethyloxazole-4-carboxylate (1.960 g, 11.6 mmol) in MeOH (33.6 ml) is added 6 N HCl (5.8 ml) and the reaction mixture is stirred at 50°C until completion of the reaction. MeOH is removed under reduced pressure. To the residue is added water (11.6 ml) and the acidic solution is washed with $Et_2O$ (11.6 ml). The aq layer is treated with activated charcoal and then concentrated under reduced pressure. The residue is redissolved in MeOH, the mixture concentrated under reduced pressure, and the process is repeated 5 times in order to give 2.500 g of ethyl 2-amino-3-oxopentanoate hydrochloride as a pale yellow

solid. LC-MS (acidic): $t_R$ = 0.32, $[M+H]^+$ = 160.12.

Step 2: Preparation of ethyl 2-(2-((*tert*-butoxycarbonyl)amino)acetamido)-3-oxopentanoate

According to the procedure described for the preparation of Intermediate 1/Step 2 but using ethyl 2-amino-3-oxopentanoate hydrochloride as starting material, 2.71 g of ethyl 2-(2-((*tert*-butoxycarbonyl)amino)acetamido)-3-oxopentanoate are obtained. LC-MS (acidic): $t_R$ = 0.77; $[M+H]^+$ = 317.13.

Step 3: Preparation of ethyl 2-(((*tert*-butoxycarbonyl)amino)methyl)-5-ethylthiazole-4-carboxylate

According to the procedure described for the preparation of Intermediate 1/Step 3 but using ethyl 2-(2-((*tert*-butoxycarbonyl)amino)acetamido)-3-oxopentanoate as starting material, 2.60 g of ethyl 2-(((*tert*-butoxycarbonyl)amino)methyl)-5-ethylthiazole-4-carboxylate. LC-MS (acidic): $t_R$ = 0.90; $[M+H]^+$ = 315.11.

Step 4: Preparation of ethyl 5-ethyl-2-(formamidomethyl)thiazole-4-carboxylate

According to the procedure described for the preparation of Intermediate 1/Step 4 but using ethyl 2-(((*tert*-butoxycarbonyl)amino)methyl)-5-ethylthiazole-4-carboxylate as starting material, 2.060 g of ethyl 5-ethyl-2-(formamidomethyl)thiazole-4-carboxylate are obtained. LC-MS (acidic): $t_R$ = 0.64; $[M+H]^+$ = 243.04.

Step 5: Preparation of ethyl 2-ethylimidazo[5,1-b]thiazole-3-carboxylate

According to the procedure described for the preparation of Intermediate 1/Step 5 but using ethyl 5-ethyl-2-(formamidomethyl)thiazole-4-carboxylate as starting material, 1.140 g of ethyl 2-ethylimidazo[5,1-b]thiazole-3-carboxylate are obtained. LC-MS (acidic): $t_R$ = 0.57; $[M+H]^+$ = 225.01.

Step 6: Preparation of 2-ethyl-*N*-methoxy-*N*-methylimidazo[5,1-*b*]thiazole-3-carboxamide

Step 6.1: Ester hydrolysis: The product form step 5, ethyl 2-ethylimidazo[5,1-b]thiazole-3-carboxylate (1.114 g; 5.08 mmol) is dissolved in THF (11.1 ml) and water (5.5 ml). LiOH monohydrate (259 mg; 6.10 mmol) is added and the mixture stirred at RT for 1 hour. The mixture is evaporated to dryness under reduced pressure.

Step 6.2: The residue from step 6.1 is dissolved in DMF (16.5 ml). DIPEA (2.61 ml; 15.20 mmol), HATU (2.319 g; 6.10 mmol) and N,O-dimethylhydroxylamine hydrochloride (607 mg; 6.10 mmol) are added and the mixture is stirred at RT for 3 h. The reaction mixture is concentrated under reduced pressure and the residue is purified by preparative HPLC (basic conditions) to give 969 mg of 2-ethyl-*N*-methoxy-*N*-methylimidazo[5,1-*b*]thiazole-3-carboxamide. LC-MS (acidic): $t_R$ = 0.48; $[M+H]^+$ = 240.08.

Step 7: Preparation of 2-ethylimidazo[5,1-b]thiazole-3-carbaldehyde

To an ice-cold solution of the product from Step 6.2, 2-ethyl-*N*-methoxy-*N*-methylimidazo[5,1-*b*]thiazole-3-carboxamide (407 mg; 1.70 mmol) in THF (10 ml) is added a solution of DIBALH (1 M in toluene, 1.7 ml, 1.70 mmol). The reaction mixture is stirred at 0°C for 1 h, treated with more DIBALH (1 M in toluene, 0.85 ml, 0.85 mmol), and further stirred at 0°C for 1 h. A sat. aq. $NH_4Cl$ solution is added and the product is extracted with EtOAc (3x). The combined org. extracts are dried over $MgSO_4$, filtered and concentrated under reduced pressure to give 300 mg of 2-ethylimidazo[5,1-b]thiazole-3-carbaldehyde as a yellow solid. LC-MS (acidic): $t_R$ = 0.41; $[M+H]^+$ = 181.19.

Step 8: Preparation of *rac*-1-(2-ethyiimidazo[5,1-*b*]thiazol-3-yl)prop-2-yn-1-ol (intermediate 3)

To an ice-cold solution of the product from Step 7, 2-ethylimidazo[5,1-b]thiazole-3-carbaldehyde (324 mg; 1.80 mmol) in THF (7.2 ml) is added a solution of ethynylmagnesium bromide (0.5 M in THF, 10.8 ml, 5.40 mmol) in a dropwise manner. The reaction mixture is stirred at 0°C for 1 h. The reaction is quenched by careful addition of aq. ammonium chloride solution. The product is extracted with EtOAc (3 × 20 ml) and the combined organic extracts are dried over $MgSO_4$, filtered and concentrated under reduced pressure to give 375 mg of rac-1-(2-ethylimidazo[5,1-*b*]thiazol-3-yl)prop-2-yn-1-ol. LC-MS (acidic): $t_R$ = 0.47; $[M+H]^+$ = 207.26. $^1H$ NMR (500 MHz, $d_6$-DMSO) $\delta$: 8.17 (s, 1 H), 7.04 (s, 1 H), 6.47 (d, J = 4.5 Hz, 1 H), 5.82 (dd, $J_1$ = 2.3 Hz, $J_2$ = 4.5 Hz, 1 H), 3.63 (d, J = 2.3 Hz, 1 H), 2.79 (m, 2 H), 1.19 (t, J = 7.5 Hz, 4 H).

**Intermediate 4: *rac*-1-(2-Cyclopentylimidazo[5,1-*b*]thiazol-3-yl)prop-2-yn-1-ol**

Step 1: Preparation of ethyl 2-amino-3-cyclopentyl-3-oxopropanoate hydrochloride

According to the procedure described for the preparation of Intermediate 1/Step 1 but using cyclopentanecarbonyl chloride chloride as starting material, 870 mg of ethyl 2-amino-3-cyclopentyl-3-oxopropanoate hydrochloride are obtained as a pale yellow solid. LC-MS (basic): $t_R$ = 0.78; $[M+H]^+$ = 200.24.

Step 2: Preparation of ethyl 2-(2-((*tert*-butoxycarbonyl)amino)acetamido)-3-cyclopentyl-3-oxopropanoate

According to the procedure described for the preparation of Intermediate 1/Step 2 but using ethyl 2-amino-3-cyclopentyl-3-oxopropanoate hydrochloride as starting material, 620 mg of ethyl 2-(2-((*tert*-butoxycarbonyl)amino)acetamido)-3-cyclopentyl-3-oxopropanoate are obtained. LC-MS (basic): $t_R$ = 0.99; $[M+H]^+$ = 357.21.

Step 3: Preparation of ethyl 2-(((*tert*-butoxycarbonyl)amino)methyl)-5-cyclopentylthiazole-4-carboxylate

According to the procedure described for the preparation of Intermediate 1/Step 3 but using ethyl 2-(2-((*tert*-butoxycarbonyl)amino)acetamido)-3-cyclopentyl-3-oxopropanoate as starting material, 580 mg of ethyl 2-(((*tert*-butoxycarbonyl)amino)methyl)-5-cyclopentylthiazole-4-carboxylate are obtained. LC-MS (basic): $t_R$ = 1.15; $[M+H]^+$ = 355.18.

Step 4: Preparation of ethyl 5-cyclopentyl-2-(formamidomethyl)thiazole-4-carboxylate

According to the procedure described for the preparation of Intermediate 1/Step 4 but using ethyl 2-(((*tert*-butoxycarbonyl)amino)methyl)-5-cyclopentylthiazole-4-carboxylate as starting material, 487 mg of ethyl 5-cyclopentyl-2-(formamidomethyl)thiazole-4-carboxylate are obtained. LC-MS (basic): $t_R$ = 0.85; $[M+H]^+$ = 283.18.

Step 5: Preparation of ethyl 2-cyclopentylimidazo[5,1-b]thiazole-3-carboxylate

According to the procedure described for the preparation of Intermediate 1/Step 5 but using ethyl 5-cyclopentyl-2-(formamidomethyl)thiazole-4-carboxylate as starting material, 440 mg of ethyl 2-cyclopentylimidazo[5,1-*b*]thiazole-3-carboxylate are obtained. LC-MS (basic): $t_R$ = 1.10; $[M+H]^+$ = 265.18.

Step 6: Preparation of 2-cyclopentyl-*N*-methoxy-*N*-methylimidazo[5,1-*b*]thiazole-3-carboxamide

Step 6.1: Ester hydrolysis: The product form step 5, ethyl 2-cyclopentylimidazo[5,1-b]thiazole-3-carboxylate (440 mg; 1.64 mmol) is dissolved in THF (5 ml) and water (2.5 ml) followed by the addition of LiOH monohydrate (80 mg; 1.92 mmol) and stirring at RT is continued for 1 hour. A second portion of lithium hydroxide monohydrate (80 mg; 1.92 mmol) is added and the reaction mixture is stirred for another hour. The mixture is evaporated to dryness under reduced pressure.

Step 6.2: The residue from step 6.1 is dissolved in a 1 / 1 mixture of DMF / DCM (15 ml in total) followed by the subsequent addition of DIPEA (0.842 ml; 4.92 mmol), HATU (749 mg; 1.97 mmol) and *N*,*O*-dimethylhydroxylamine hydrochloride (196 mg; 1.97 mmol). Stirring is continued at RT overnight. The reaction mixture is concentrated under reduced pressure and the residue is purified by FC (Silicagel; EtOAc / MeOH = 9 / 1) to give 560 mg of 2-cyclopentyl-*N*-methoxy-*N*-methylimidazo[5,1-*b*]thiazole-3-carboxamide. LC-MS (basic): $t_R$ = 0.89; $[M+H]^+$ = 280.15.

Step 7: Preparation of 2-cyclopentylimidazo[5,1-b]thiazole-3-carbaldehyde

To an ice-cold solution of the product from Step 6.2, 2-cyclopentyl-*N*-methoxy-*N*-methylimidazo[5,1-*b*]thiazole-3-carboxamide (155 mg; 0.50 mmol) in THF (2 ml) is added a solution of DIBALH (1 M in toluene, 0.50 ml, 0.50 mmol). The reaction mixture is stirred at 0°C for 30 min, treated with more DIBALH (1 M in toluene, 0.50 ml, 0.50 mmol), and further stirred at 0°C for 30 min. A sat. aq. $NH_4Cl$ solution is added dropwise, the mixture concentrated under reduced pressure and the crude residue purified by prepHPLC (basic conditions) to give 44 mg of 2-cyclopentylimidazo[5,1-b]thiazole-3-carbaldehyde. LC-MS (basic): $t_R$ = 0.88; $[M+H]^+$ = 221.15.

Step 8: Preparation of *rac*-1-(2-cyclopentylimidazo[5,1-*b*]thiazol-3-yl)prop-2-yn-1-ol (intermediate 4)

To an ice-cold solution of the product from Step 7, 2-cyclopentylimidazo[5,1-b]thiazole-3-carbaldehyde (66 mg; 0.30 mmol) in THF (1.2 ml) is added a solution of ethynylmagnesium bromide (0.5 M in THF, 1.8 ml, 0.90 mmol) in a

dropwise manner. The reaction mixture is stirred at 0°C for 30 min. The reaction is quenched by careful addition of aq. ammonium chloride solution. The product is extracted with EtOAc (3 × 20 ml) and the combined organic extracts are dried over $MgSO_4$, filtered and concentrated under reduced pressure to give 73 mg of *rac*-1-(2-cyclopentylimidazo[5,1-*b*]thiazol-3-yl)prop-2-yn-1-ol. LC-MS (acidic): $t_R$ = 0.62; $[M+H]^+$ = 247.10.

**Intermediate 5: *rac*-1-(2-(1-Methylcyclopropyl)imidazo[5,1-*b*]thiazol-3-yl)prop-2-yn-1-ol**

Step 1: Preparation of ethyl 2-diazo-3-(1-methylcyclopropyl)-3-oxopropanoate

In a reaction vessel, a solution of 1-methylcyclopropane-1-carboxylic acid (1890 mg; 17.9 mmol) and 1,1'-carbonyldiimidazole (3938 mg; 24.3 mmol) in anhydrous THF (30 ml) is stirred at RT for 4 hours (solution A). In a second reaction vessel, a solution of ethyl potassium malonate (8093 mg; 46.6 mmol), anhydrous magnesium chloride (5241 mg; 53.9 mmol) and 4-dimethylaminopyridine (219 mg; 1.79 mmol) in a mixture of THF (60 ml) and acetonitrile (30 ml) is stirred at RT for 6 hours (solution B). The two solutions are cooled to 0°C and solution A is added together with $Et_3N$ (10 ml; 71.7 mmol) to solution B. The reaction mixture is then stirred at RT for 18 hours. After completion of the reaction, the solvent is removed under reduced pressure and the residue is suspended in water and extracted with DCM (3x). The combined org. extracts are dried over sodium sulfate, filtered and concentrated under reduced pressure to give ethyl 3-(1-methylcyclopropyl)-3-oxopropanoate as a yellow oil which is used without purification. LC-MS (acidic): $t_R$ = 0.71; $[M+H]^+$ = 171.05. Ethyl 3-(1-methylcyclopropyl)-3-oxopropanoate is redissolved in acetonitrile (100 ml) and successively treated with 4-acetamidobenzenesulfonyl azide (5544 mg; 2.4 mmol) and $Et_3N$ (3.12 ml; 22.4 mmol). The reaction mixture is stirred at RT for 18 hours. A mixture of heptane / DCM (95:5) is added to precipitate the salts, and the mixture is filtered. The filter cake is washed with heptane and the filtrate concentrated under reduced pressure. A mixture of heptane / DCM (95:5) is added again, and the mixture filtered. The filter cake is washed with heptane and the filtrate concentrated under reduced pressure. The crude oil is purified by FC (Silicagel; Heptane / EtOAc) to give ethyl 2-diazo-3-(1-methylcyclopropyl)-3-oxopropanoate as a yellow oil. LC-MS (acidic): $t_R$ = 0.80; $[M+H]^+$ 197.07.

Step 2: Preparation of *rac*-ethyl-2-(2-((*terl*-butoxycarbonyl)amino)acetamido)-3-(1-methylcyclopropyl)-3-oxopropanoate

Ethyl 2-diazo-3-(1-methylcyclopropyl)-3-oxopropanoate (2560 mg; 13.0 mmol) is dissolved in DCM (50 ml) and *tert*-butyl (2-amino-2-oxoethyl)carbamate (2486 mg; 13.7 mmol) is added followed by dirhodium tetraacetate (62 mg, 0.13 mmol). The reaction mixture is stirred at 45°C for 3 days. Water is added, the layers separated and the aq. layer extracted with DCM (2x). The combined org. extracts are dried over magnesium sulfate, filtered and concentrated under reduced pressure. Purification by FC (Silicagel; Heptane / EtOAc) gives 3654 mg of rac-ethyl-2-(2-((*tert*-butoxycarbonyl)amino)acetamido)-3-(1-methylcyclopropyl)-3-oxopropanoate as a pink solid. LC-MS (acidic): $t_R$ = 0.84; $[M+H]^+$ = 342.99.

Step 3: Preparation of ethyl 2-(((*tert*-butoxycarbonyl)amino)methyl)-5-(1-methylcyclopropyl)thiazole-4-carboxylate

The product from step 2, *rac*-ethyl-2-(2-((*tert*-butoxycarbonyl)amino)acetamido)-3-(1-methylcyclopropyl)-3-oxopropanoate (3654 mg; 10.7 mmol) and Lawesson's reagent (5000 mg; 12.0 mmol) are suspended in THF (50 ml) and heated to reflux for 24 hours. THF is evaporated under reduced pressure and the residue is purified by FC (Silicagel; EtOAc / heptane) to give 4601 mg of ethyl 2-(((*tert*-butoxycarbonyl)amino)methyl)-5-(1-methylcyclopropyl)thiazole-4-carboxylate. LC-MS (acidic): $t_R$ = 0.96; $[M+H]^+$ = 340.98.

Step 4: Preparation of ethyl 2-(formamidomethyl)-5-(1-methylcyclopropyl)thiazole-4-carboxylate

Step 4.1: Boc-cleavage: The product from step 3, ethyl 2-(((*tert*-butoxycarbonyl)amino)methyl)-5-(1-methylcyclopropyl)thiazole-4-carboxylate (4601 mg; 13.5 mmol) is dissolved in dioxane (50 ml) followed by careful addition of 4N HCl in dioxane (10 ml; 40.0 mmol). The mixture is stirred at RT for 15 hours. The reaction mixture is evaporated to dryness under reduced pressure.

Step 4.2: The residue from step 4.1 is dissolved in ethyl formate (30 ml) and $Et_3N$ (4.48 ml; 32.2 mmol) is added. The mixture is stirred at reflux for 4 hours. A sat. aq. $NaHCO_3$ solution is added and the mixture extracted with DCM (2x). The combined org. extracts are dried over $MgSO_4$, filtered and concentrated under reduced pressure to give 3691 mg of ethyl 2-(formamidomethyl)-5-(1-methylcyclopropyl)thiazole-4-carboxylate which is used without further purification in Step 5. LC-MS (acidic): $t_R$ = 0.73; $[M+H]^+$ = 269.00.

Step 5: Preparation of ethyl 2-(1-methylcyclopropyl)imidazo[5,1-b]thiazole-3-carboxylate

The product from step 4.2, ethyl 2-(formamidomethyl)-5-(1-methylcyclopropyl)thiazole-4-carboxylate (3691 mg; 13.8 mmol) is dissolved in DCM (100 ml) and cooled to -20°C followed by the addition of phosphorus oxychloride (2.6 ml; 27.6 mmol). The reaction mixture is stirred at RT for 48 h. More phosphorus oxychloride (1 ml; 10.6 mmol) is added and the mixture stirred at reflux for 6 hours. Water was added followed by sat. aq. $NaHCO_3$. The layers were separated and the aq. layer extracted with DCM (2x). The combined organic layers are dried over $MgSO_4$, filtered and concentrated under reduced pressure to give 3066 mg of ethyl 2-(1-methylcyclopropyl)imidazo[5,1-*b*]thiazole-3-carboxylate as a beige foam. LC-MS (acidic): $t_R$ = 0.67; $[M+H]^+$ = 251.01.

Step 6: Preparation of (2-(1-methylcyclopropyl)imidazo[5,1-b]thiazol-3-yl)methanol

The product from step 5, ethyl 2-(1-methylcyclopropyl)imidazo[5,1-b]thiazole-3-carboxylate (3062 mg; 12.2 mmol) is dissolved in EtOH (150 ml) and sodium borohydride (1000 mg; 26.4 mmol) is added. The reaction mixture is stirred at RT for 4 hours. The mixture is concentrated under reduced pressure. The residue is redissolved in DCM and water is carefully added followed by sat. aq. $NH_4Cl$. The layers are separated and the aq. layer extracted with DCM (2x). The combined org. extracts are dried over $MgSO_4$, filtered and concentrated under reduced pressure to give 2059 mg of (2-(1-methylcyclopropyl)imidazo[5,1-b]thiazol-3-yl)methanol. LC-MS (basic): $t_R$ = 0.69; $[M+H]^+$ = 209.15.

Step 7: Preparation of 2-(1-methylcyclopropyl)imidazo[5,1-b]thiazole-3-carbaldehyde

To an ice-cold solution of the product from Step 6, (2-(1-methylcyclopropyl)imidazo[5,1-b]thiazol-3-yl)methanol (2059 mg; 9.89 mmol) in DCM (150 ml) is added Dess-Martin periodinane (4612 mg; 10.9 mmol). The reaction mixture is stirred at RT for 18 hours. A sat. aq. $NaHCO_3$ solution is added and the mixture stirred for 30 min. The white precipitate is then filtered and the filtrate extracted with DCM (3x). The combined org. extracts are washed with brine, dried over $MgSO_4$, filtered and concentrated under reduced pressure. Purification by FC (Silicagel; Hept / EtOAc) gives 2-(1-methylcyclopropyl)imidazo[5,1-b]thiazole-3-carbaldehyde. LC-MS (acidic): $t_R$ = 0.50; $[M+H]^+$ = 207.35. $^1H$ NMR (500 MHz, $d_6$-DMSO) $\delta$: 10.16 (s, 1 H), 8.54 (s, 1 H), 7.17 (s, 1 H), 1.55 (s, 3 H), 1.16-1.18 (m, 2 H), 1.04 (m, 2 H).

Step 8: Preparation of *rac*-1-(2-(1-methylcyclopropyl)imidazo[5,1-*b*]thiazol-3-yl)prop-2-yn-1-ol (intermediate 5)

To an ice-cold solution of the product from Step 7, 2-(1-methylcyclopropyl)imidazo[5,1-*b*]thiazole-3-carbaldehyde (270 mg; 1.31 mmol) in THF (10 ml) is added a solution of ethynylmagnesium bromide (0.5 M in THF, 3 ml, 1.50 mmol) in a dropwise manner. The reaction mixture is stirred at 0°C for 1 h. The reaction is quenched by careful addition of aq. ammonium chloride solution. The product is extracted with EtOAc (3 $\times$ 20 ml) and the combined organic extracts are dried over $MgSO_4$, filtered and concentrated under reduced pressure to give 274 mg of *rac*-1-(2-(1-methylcyclopropyl)imidazo[5,1-*b*]thiazol-3-yl)prop-2-yn-1-ol. LC-MS (acidic): $t_R$ = 0.57; $[M+H]^+$ = 233.01. $^1H$ NMR (500 MHz, $d_6$-DMSO) $\delta$: 8.14 (s, 1 H), 7.04 (s, 1 H), 6.58 (d, J = 4.4 Hz, 1 H), 5.88 (dd, $J_1$ = 2.3 Hz, $J_2$ = 4.3 Hz, 1 H), 3.65 (d, J = 2.2 Hz, 1 H), 1.36 (s, 3 H), 0.84-0.99 (m, 4 H).

**Intermediate 6: *rac*-1-(2-(3-Fluorophenyl)imidazo[5,1-*b*]thiazol-3-yl)prop-2-yn-1-ol:**

Step 1: Preparation of methyl 5-bromo-2-(bromomethyl)thiazole-4-carboxylate

To a solution of methyl 5-bromo-2-methyl-1,3-thiazole-4-carboxylate (19.97 g; 84.6 mmol) in trifluorotoluene (400 ml) is added at rt under argon NBS (7.529 g; 42.3 mmol) and AIBN (4.168 g; 25.4 mmol). The reaction mixture is stirred at 85°C for 3 h. More NBS and AIBN are added until the conversion is complete. The mixture is cooled to RT, filtered, washed with toluene and concentrated under reduced pressure. Purification by FC (Silicagel; Hept / EtOAc) gives 6.78 g of methyl 5-bromo-2-(bromomethyl)thiazole-4-carboxylate. LC-MS (acidic): $t_R$ = 0.91; $[M+H]^+$ = 313.87.

Step 2: Preparation of methyl 5-bromo-2-(formamidomethyl)thiazole-4-carboxylate

To a solution of the product from step 1, 5-bromo-2-(bromomethyl)thiazole-4-carboxylate (6.78 g; 21.5 mmol) in DMF (50 ml) is added sodium diformylamide (2.25 g; 23.7 mmol). The reaction mixture is stirred at RT for 2 h. A sat. aq. $NaHCO_3$ solution is then added to the reaction mixture and is stirred overnight at rt. The mixture is extracted with EtOAc (3x) and the combined organic extracts are washed with brine, dried over $MgSO_4$, filtered and concentrated under reduced pressure to give 5.74 g of methyl 5-bromo-2-(formamidomethyl)thiazole-4-carboxylate LC-MS (acidic): $t_R$ = 0.57; $[M+H]^+$ = 278.84.

Step 3: Preparation of methyl 2-bromoimidazo[5,1-*b*]thiazole-3-carboxylate

To a solution of the product from step 2, methyl 5-bromo-2-(formamidomethyl)thiazole-4-carboxylate (5.74 g; 20.6 mmol) is added $POCl_3$ (1.94 ml, 20.6 mmol). The reaction mixture is stirred at 60°C for 1 h. A sat. aq. $NaHCO_3$ solution is added to the reaction mixture until pH 8 is obtained. The mixture is then extracted with DCM (3x) and the combined organic extracts are washed with brine, dried over $MgSO_4$, filtered and concentrated under reduced pressure. Purification by FC (Silicagel; Hept / EtOAc) gives 1.779 g of methyl 2-bromoimidazo[5,1-b]thiazole-3-carboxylate. LC-MS (acidic): $t_R$ = 0.51; $[M+H]^+$ = 260.77. $^1$H NMR (400 MHz, $d_6$-DMSO) $\delta$: 8.48 (d, *J* = 0.6 Hz, 1 H), 7.14 (d, *J* = 0.6 Hz, 1 H), 3.96 (s, 3 H).

Step 4: Preparation of (2-bromoimidazo[5,1-b]thiazol-3-yl)methanol

To a solution of the product from step 3, methyl 2-bromoimidazo[5,1-*b*]thiazole-3-carboxylate (1.779 g; 6.81 mmol) in EtOH (80 ml) is added $NaBH_4$ (1 g; 26.4 mmol) at rt under argon. The reaction mixture is stirred 24 h at RT. The reaction mixture is concentrated under reduced pressure, diluted with DCM and quenched carefully with $H_2O$ and sat. aq. $NH_4Cl$. When the gas evolution ceases the mixture is extracted with DCM (3x) and the combined organic extracts are dried over $MgSO_4$, filtered and concentrated under reduced pressure to give 896 mg of (2-bromoimidazo[5,1-*b*]thiazol-3-yl)methanol. LC-MS (acidic): $t_R$ = 0.37; $[M+H]^+$ = 232.85.

Step 5: Preparation of 2-bromo-3-(((*tert*-butyldimethylsilyl)oxy)methyl)imidazo[5,1-*b*]thiazole

To a cooled solution of the product from step 4, (2-bromoimidazo[5,1-b]thiazol-3-yl)methanol (896 mg; 3.84 mmol) in dry DCM (20 ml) is added *tert*-butyl(chloro)dimethylsilane (0.798 mL; 4.61 mmol) and imidazole (314 mg; 4.61 mmol). The reaction mixture is stirred at RT for 2 h. The reaction mixture is diluted with water and extracted with DCM (3x) and the combined organic extracts are washed with brine, dried over $MgSO_4$, filtered and concentrated under reduced pressure. Purification by FC (Silicagel; Hept / EtOAc) gives 380 mg of 2-bromo-3-(((*tert*-butyldimethylsilyl)oxy)methyl)imidazo[5,1-*b*]thiazole. LC-MS (acidic): $t_R$ = 0.89; $[M+H]^+$ = 346.87. $^1$H NMR (400 MHz, $d_6$-DMSO) $\delta$: 8.13 (s, 1 H), 7.08 (s, 1 H), 4.88 (s, 2 H), 0.85 (s, 9 H), 0.08 (s, 6 H).

Step 6: Preparation of 3-(((*tert*-butyldimethylsilyl)oxy)methyl)-2-(3-fluorophenyl)imidazo[5,1-b]thiazole

To a solution of the product from step 5, 2-bromo-3-(((*tert*-butyldimethylsilyl)oxy)methyl)imidazo[5,1-*b*]thiazole (150 mg; 0.432 mmol) in dioxane (5 ml) are added aq. 1.6 M $Na_2CO_3$ (3 ml), 3-fluorobenzeneboronic acid (125 mg; 0.864 mmol) and $Pd(PPh_3)_4$ (18 mg; 3.5 mol%). The reaction mixture is stirred at 90°C for 2 h. The mixture is poured into water and extracted with DCM (2x). The combined organic extracts are dried (MgSO4), filtered and concentrated under reduced pressure. Purification by FC (Silicagel; Hept / EtOAc) gives 146 mg of 3-(((*tert*-butyldimethylsilyl)oxy)methyl)-2-(3-fluorophenyl)imidazo[5,1-b]thiazole. LC-MS (acidic): $t_R$ = 0.95; $[M+H]^+$ = 362.97. $^1$H NMR (400 MHz, $d_6$-DMSO) $\delta$: 8.21 (s, 1 H), 7.60 (m, 1 H), 7.35-7.43 (m, 3 H), 7.15 (s, 1 H), 4.90 (s, 2 H), 0.81 (s, 9 H), -0.02 (s, 6 H).

Step 7: Preparation of (2-(3-fluorophenyl)imidazo[5,1-b]thiazol-3-yl)methanol

To a solution of the product from step 5, 3-(((*tert*-butyldimethylsilyl)oxy)methyl)-2-(3-fluorophenyl)imidazo[5,1-b]thiazole (146 mg; 0.403 mmol) in THF (2 ml) is added TBAF (1 M in THF, 0.604 ml, 0.604 mmol). The reaction mixture is stirred at RT for 20 min. The mixture is poured into saturated aq. $NaHCO_3$ and extracted with DCM (3x). The combined organic extracts are washed with brine, dried (MgSO4), filtered and concentrated under reduced pressure to give 91 mg of (2-(3-fluorophenyl)imidazo[5,1-b]thiazol-3-yl)methanol. LC-MS (acidic): $t_R$ = 0.57; $[M+H]^+$ = 248.97.

Step 8: Preparation of 2-(3-fluorophenyl)imidazo[5,1-b]thiazole-3-carbaldehyde

To an ice-cold solution of the product from Step 7, (2-(3-fluorophenyl)imidazo[5,1-b]thiazol-3-yl)methanol (45 mg; 0.181 mmol) in DCM (3 ml) is added Dess-Martin periodinane (119 mg; 0.281 mmol). The reaction mixture is stirred at RT for 2 hours. A sat. aq. $NaHCO_3$ solution (5 ml) is added followed by a sat. aq. $Na_2S_2O_3$ (5 ml) and the mixture stirred for 20 min. The mixture is then extracted with DCM (2x). The combined org. extracts are washed with brine, dried over $MgSO_4$, filtered and concentrated under reduced pressure to give 2-(3-fluorophenyl)imidazo[5,1-*b*]thiazole-3-carbaldehyde. LC-MS (acidic): $t_R$ = 0.60; $[M+H]^+$ = 246.96. $^1$H NMR (400 MHz, $d_6$-DMSO) $\delta$: 9.73 (s, 1 H), 8.70 (s, 1 H), 7.76-7.79 (m, 1 H), 7.64-7.69 (m, 2 H), 7.48-7.53 (m, 1 H), 7.28 (s, 1 H).

Step 9: Preparation of *rac*-1-(2-(3-fluorophenyl)imidazo[5,1-*b*]thiazol-3-yl)prop-2-yn-1-ol (intermediate 6)

To an ice-cold solution of the product from Step 8, 2-(3-fluorophenyl)imidazo[5,1-b]thiazole-3-carbaldehyde (39 mg; 0.159 mmol) in THF (2 ml) is added a solution of ethynylmagnesium bromide (0.5 M in THF, 0.382 ml, 0.191 mmol) in a dropwise manner. The reaction mixture is stirred at 0°C for 5 h. The reaction is quenched by careful addition of aq. ammonium chloride solution. The product is extracted with EtOAc (3 × 20 ml) and the combined organic extracts are dried over $MgSO_4$, filtered and concentrated under reduced pressure. Purification by preparative HPLC (basic conditions) gives 20 mg of *rac*-1-(2-(3-fluorophenyl)imidazo[5,1-*b*]thiazol-3-yl)prop-2-yn-1-ol. LC-MS (acidic): $t_R$ = 0.62; $[M+H]^+$ = 272.98. $^1H$ NMR (400 MHz, $d_6$-DMSO) $\delta$: 8.33 (s, 1 H), 7.60-7.65 (m, 1 H), 7.36-7.42 (m, 3 H), 7.17 (s, 1 H), 6.76 (d, J = 4.6 Hz, 1 H), 5.62 (dd, $J_1$ = 2.3 Hz, $J_2$ = 4.6 Hz, 1 H), 3.74 (d, J = 2.3 Hz, 1 H).

**Intermediate 7: *rac*-1-(2-(1-Fluorocyclopropyl)imidazo[5,1-*b*]thiazol-3-yl)prop-2-yn-1-ol**

Step 1: Preparation of ethyl 2-diazo-3-(1-fluorocyclopropyl)-3-oxopropanoate

According to the procedure described for the preparation of Intermediate 5/Step 1 and using 1-fluorocyclopropane-1-carboxylic acid (2014 mg; 18.8 mmol) as starting material, 3092 mg of ethyl 3-(1-fluorocyclopropyl)-3-oxopropanoate are obtained and used without further purification. LC-MS (acidic): $t_R$ = 0.71; $[M+H]^+$ = not observed. Ethyl 2-diazo-3-(1-fluorocyclopropyl)-3-oxopropanoate is then obtained as a brown oil and used without any further purification. LC-MS (acidic): $t_R$ = 0.75; $[M+H]^+$ = 201.03.

Step 2: Preparation of rac-ethyl 2-(2-((*tert*-butoxycarbonyl)amino)acetamido)-3-(1-fluorocyclopropyl)-3-oxopropanoate

According to the procedure described for the preparation of Intermediate 5/Step 2 and using ethyl 2-diazo-3-(1-fluorocyclopropyl)-3-oxopropanoate (2124 mg; 10.6 mmol) as starting material. In this case, the reaction mixture is stirred at 45°C for 5 days and 3223 mg of rac-ethyl 2-(2-((*tert*-butoxycarbonyl)amino)acetamido)-3-(1-fluorocyclopropyl)-3-oxopropanoate are obtained as a brown solid and used without further purification. LC-MS (acidic): $t_R$ = 0.81; $[M+H]^+$ = 347.01.

Step 3: Preparation of rac-ethyl 2-(((*tert*-butoxycarbonyl)amino)methyl)-5-((1*R*,2*R*)-2-fluorocyclopropyl)thiazole-4-carboxylate

According to the procedure described for the preparation of Intermediate 5/Step 3 and using rac-ethyl-2-(2-((*teit*-butoxycarbonyl)amino)acetamido)-3-(1-fluorocyclopropyl)-3-oxopropanoate (3223 mg, 9.31 mmol) as starting material, 2727 mg of ethyl 2-(((*tert*-butoxycarbonyl)amino)methyl)-5-(1-fluorocyclopropyl)thiazole-4-carboxylate are obtained as a light yellow sticky oil. LC-MS (acidic): $t_R$ = 0.92; $[M+H]^+$ = 344.99.

Step 4: Preparation of ethyl 2-(formamidomethyl)-5-(1-fluorocyclopropyl)thiazole-4-carboxylate

According to the procedure described for the preparation of Intermediate 5/Step 4 and using ethyl 2-(((*tert*-butoxycarbonyl)amino)methyl)-5-(1-fluorocyclopropyl)thiazole-4-carboxylate (2727 mg) as starting material, 2129 mg of ethyl 2-(formamidomethyl)-5-(1-fluorocyclopropyl)thiazole-4-carboxylate are obtained as a reddish oil which is used without further purification. LC-MS (acidic): $t_R$ = 0.66; $[M+H]^+$ = 272.99.

Step 5: Preparation of ethyl 2-(1-fluorocyclopropyl)imidazo[5,1-b]thiazole-3-carboxylate

According to the procedure described for the preparation of Intermediate 5/Step 5 and using 2-(formamidomethyl)-5-(1-fluorocyclopropyl)thiazole-4-carboxylate (2129 mg) as starting material, 1790 mg of ethyl 2-(1-fluorocyclopropyl)imidazo[5,1-*b*)]thiazole-3-carboxylate are obtained as a yellow powder. LC-MS (acidic): $t_R$ = 0.63; $[M+H]^+$ = 255.00.

Step 6: Preparation of (2-(1-fluorocyclopropyl)imidazo[5,1-b]thiazol-3-yl)methanol

According to the procedure described for the preparation of Intermediate 5/Step 6 and using ethyl 2-(1-fluorocyclopropyl)imidazo[5,1-b]thiazole-3-carboxylate (1582 mg, 6.22 mmol) as starting material, 782 mg of (2-(1-fluorocyclopropyl)imidazo[5,1-*b*]thiazol-3-yl)methanol are obtained as a yellow powder and used in the next step without purification. LC-MS (basic): $t_R$ = 0.45; $[M+H]^+$ = 213.04.

Step 7: Preparation of 2-(1-fluorocyclopropyl)imidazo[5,1-b]thiazole-3-carbaldehyde

According to the procedure described for the preparation of Intermediate 5/Step 7 and using (2-(1-fluorocyclopropyl)imidazo[5,1-b]thiazol-3-yl)methanol (782 mg) as starting material, 687 mg of 2-(1-fluorocyclopropyl)imidazo[5,1-b]thiazole-3-carbaldehyde are obtained as a yellow powder. LC-MS (acidic): $t_R$ = 0.47; $[M+H]^+$ = 211.00. $^1$H NMR(500 MHz, $d_6$-DMSO) $\delta$: 10.16 (s, 1 H), 8.64 (s, 1 H), 7.25 (s, 1 H), 1.75 (m, 2 H), 1.46 (m, 2 H).

Step 8: Preparation of *rac*-1-(2-(1-fluorocyclopropyl)imidazo[5,1-*b*]thiazol-3-yl)prop-2-yn-1-ol (intermediate 7)

According to the procedure described for the preparation of Intermediate 5/Step 8 and using 2-(1-fluorocyclopropyl)imidazo[5,1-b]thiazole-3-carbaldehyde (130 mg) as starting material, 135 mg of rac-1-(2-(1-fluorocyclopropyl)imidazo[5,1-*b*]thiazol-3-yl)prop-2-yn-1-ol are obtained as a white powder. LC-MS (acidic): $t_R$ = 0.52; $[M+H]^+$ = 236.64.

**Intermediate 8: *cis*-1-[(2-Fluoro-cyclopropyl)-imidazo[5,1-*b*]thiazol-3-yl]prop-2-yn-1-ol**

Step 1: Preparation of rac-ethyl 2-diazo-3-((1R,2R)-2-fluoro-cyclopropyl)-3-oxo-propanoate

According to the procedure described for the preparation of Intermediate 5/Step 1 and using rac-ethyl 3-((1R,2R)-2-fluorocyclopropyl)-3-oxopropanoate (2000 mg; 10.9 mmol) as starting material, 2400 mg of rac-ethyl-2-diazo-3-((1R,2R)-2-fluoro-cyclopropyl)-3-oxo-propanoate are obtained as a yellow oil and used without any further purification. LC-MS (acidic): $t_R$ = 0.75; $[M+H]^+$ = 201.01.

Step 2: Preparation of *cis*-ethyl 2-(2-((*tert*-butoxycarbonyl)amino)acetamido)-3-(2-fluorocyclopropyl)-3-oxopropanoate

According to the procedure described for the preparation of Intermediate 5/Step 2 and using rac-ethyl 2-diazo-3-((1*R*,2*R*)-2-fluoro-cyclopropyl)-3-oxo-propionic acid (2400 mg; 12 mmol) as starting material. In this case, the reaction mixture is stirred at 45°C for 2 days and 4075 mg of *cis*-ethyl 2-(2-((*tert*-butoxycarbonyl)amino)acetamido)-3-(2-fluorocyclopropyl)-3-oxopropanoate are obtained as a brown oil and used without further purification. LC-MS (acidic): $t_R$ = 0.77; $[M+H]^+$ = 347.04.

Step 3: Preparation of rac-ethyl 2-(((*tert*-butoxycarbonyl)amino)methyl)-5-((1R,2S)-2-fluorocyclopropyl)thiazole-4-carboxylate

According to the procedure described for the preparation of Intermediate 5/Step 3 and using *cis*-ethyl 2-(2-((*tert*-butoxycarbonyl)amino)acetamido)-3-(2-fluorocyclopropyl)-3-oxopropanoate (4075 mg, 11.8 mmol) as starting material, 5326 mg of rac-ethyl 2-(((*tert*-butoxycarbonyl)amino)methyl)-5-((1*R*,2*S*)-2-fluorocyclopropyl)thiazole-4-carboxylate are obtained as a white solid after FC (silicagel, Hept / EtOAc). LC-MS (acidic): $t_R$ = 0.89; $[M+H]^+$ = 344.99.

Step 4: Preparation of rac-ethyl 5-((1*R*,2*S*)-2-fluorocyclopropyl)-2-(formamidomethyl)thiazole-4-carboxylate

According to the procedure described for the preparation of Intermediate 5/Step 4 and using rac-ethyl 2-(((*tert*-butoxycarbonyl)amino)methyl)-5-((1*R*,2*S*)-2-fluorocyclopropyl)thiazole-4-carboxylate (5326 mg) as starting material, 1965 mg of rac-ethyl 5-((1*R*,2*S*)-2-fluorocyclopropyl)-2-(formamidomethyl)thiazole4-carboxylate are obtained as a white solid after FC (silicagel, Hept / EtOAc). LC-MS (acidic): $t_R$ = 0.64; $[M+H]^+$ = 272.99.

Step 5: Preparation of rac-ethyl 2-((1*R*,2*S*)-2-fluorocyclopropyl)imidazo[5,1-*b*]thiazole-3-carboxylate

According to the procedure described for the preparation of Intermediate 5/Step 5 and using rac-ethyl 5-((1R,2S)-2-fluorocyclopropyl)-2-(formamidomethyl)thiazole-4-carboxylate (1965 mg) as starting material, 1810 mg of rac-ethyl 2-((1R,2S)-2-fluorocyclopropyl)imidazo[5,1-b]thiazole-3-carboxylate are obtained as an orange powder. LC-MS (acidic): $t_R$ = 0.58; $[M+H]^+$ = 255.28.

Step 6: Preparation of *rac*-(2-((1*R*,2*S*)-2-fluorocyclopropyl)imidazo[5,1-*b*]thiazol-3-yl)methanol

According to the procedure described for the preparation of Intermediate 5/Step 6 and using rac-ethyl 2-((1R,2S)-2-fluorocyclopropyl)imidazo[5,1-b]thiazole-3-carboxylate (1810 mg, 7.12 mmol) as starting material, 940 mg of rac-(2-((1R,2S)-2-fluorocyclopropyl)imidazo[5,1-b]thiazol-3-yl)methanol are obtained as a white powder and used in the next step without purification. LC-MS (basic): $t_R$ = 0.42; $[M+H]^+$ = 213.00.

Step 7: Preparation of *rac*-2-((1*R*,2*S*)-2-fluorocyclopropyl)imidazo[5,1-b]thiazole-3-carbaldehyde

According to the procedure described for the preparation of Intermediate 5/Step 7 and using *rac*-(2-((1*R*,2*S*)-2-fluorocyclopropyl)imidazo[5,1-b]thiazol-3-yl)methanol (940 mg) as starting material, 1655 mg of rac-2-((1R,2S)-2-fluorocyclopropyl)imidazo[5,1-*b*]thiazole-3-carbaldehyde are obtained as a yellow gum. LC-MS (acidic): $t_R$ = 0.44; $[M+H]^+$ = 211.01. $^1$H NMR (400 MHz, $d_6$-DMSO) $\delta$: 10.08 (s, 1 H), 8.57 (s, 1 H), 7.17 (s, 1 H), 5.25 (m, 1 H), 2.95-3.02 (m, 1 H), 1.61-1.71 (m, 1 H), 1.48 (dtd, $J_1$ = 3.2 Hz, $J_2$ = 7.6 Hz, $J_3$ = 23.9 Hz, 1 H).

Step 8: Preparation of *cis*-1-[2-(2-fluoro-cyclopropyl)-imidazo[5,1-*b*]thiazol-3-yl]-prop-2-yn-1-ol (intermediate 8)

According to the procedure described for the preparation of Intermediate 5/Step 8 and using rac-2-((1R,2S)-2-fluorocyclopropyl)imidazo[5,1-b]thiazole-3-carbaldehyde (1410 mg) as the starting material, 1079 mg of *cis*-1-[2-(2-fluoro-cyclopropyl)-imidazo[5,1-*b*)]thiazol-3-yl]-prop-2-yn-1-ol are obtained as a brown gum. LC-MS (acidic): $t_R$ = 0.48; $[M+H]^+$ = 237.08.

**Intermediate 9: *trans*-1-[(2-Fluoro-cyclopropyl)-imidazo[5,1-*b*]thiazol-3-yl]prop-2-yn-1-ol**

Step 1: Preparation of rac-ethyl 2-diazo-3-((1*S*,2*R*)-2-fluoro-cyclopropyl)-3-oxo-propanoate

According to the procedure described for the preparation of Intermediate 5/Step 1 and using rac-ethyl-3-((1*S*,2*R*)-2-fluorocyclopropyl)-3-oxopropanoate (2000 mg; 10.9 mmol) as starting material, 2190 mg of rac-ethyl-2-diazo-3-((1S,2R)-2-fluoro-cyclopropyl)-3-oxo-propanoate are obtained as a yellow oil and used without any further purification. LC-MS (acidic): $t_R$ = 0.81; $[M+H]^+$ = not observed.

Step 2: Preparation of *trans*-ethyl 2-(2-((*tert*-butoxycarbonyl)amino)acetamido)-3-(2-fluorocyclopropyl)-3-oxopropanoate

According to the procedure described for the preparation of Intermediate 5/Step 2 and using rac-ethyl 2-diazo-3-((1*S*,2*R*)-2-fluoro-cyclopropyl)-3-oxo-propionic acid (2195 mg; 11 mmol) as starting material. In this case, the reaction mixture is stirred at 45°C for 2 days and 3816 mg of *trans*-ethyl 2-(2-((*tert*-butoxycarbonyl)amino)acetamido)-3-(2-fluorocyclopropyl)-3-oxopropanoate are obtained as a brown oil and used without further purification. LC-MS (acidic): $t_R$ = 0.81; $[M+H]^+$ = 347.05.

Step 3: Preparation of rac-ethyl 2-(((*tert*-butoxycarbonyl)amino)methyl)-5-((1R,2R)-2-fluorocyclopropyl)thiazole-4-carboxylate

According to the procedure described for the preparation of Intermediate 5/Step 3 and using *trans*-ethyl 2-(2-((*tert*-butoxycarbonyl)amino)acetamido)-3-(2-fluorocyclopropyl)-3-oxopropanoate (3816 mg, 11 mmol) as starting material, 3956 mg of rac-ethyl 2-(((*tert*-butoxycarbonyl)amino)methyl)-5-((1*R*,2*R*)-2-fluorocyclopropyl)thiazole-4-carboxylate are obtained as a brown sticky oil and used without purification in the next step. LC-MS (acidic): $t_R$ = 0.91; $[M+H]^+$ = 345.07.

Step 4: Preparation of rac-ethyl 5-((1R,2R)-2-fluorocyclopropyl)-2-(formamidomethyl)thiazole-4-carboxylate

According to the procedure described for the preparation of Intermediate 5/Step 4 and using rac-ethyl 2-(((*tert*-butoxycarbonyl)amino)methyl)-5-((1R,2R)-2-fluorocyclopropyl)thiazole-4-carboxylate (3956 mg) as starting material, 1210 mg of rac-ethyl 5-((1*R*,2*R*)-2-fluorocyclopropyl)-2-(formamidomethyl)thiazole-4-carboxylate are obtained as a brown sticky gum after flash chromatography (silicagel, Hept / EtOAc). LC-MS (acidic): $t_R$ = 0.67; $[M+H]^+$ = 273.04.

Step 5: Preparation of rac-ethyl 2-((1*R*,2*R*)-2-fluorocyclopropyl)imidazo[5,1-*b*]thiazole-3-carboxylate

According to the procedure described for the preparation of Intermediate 5/Step 5 and using rac-ethyl 5-((1R,2R)-2-fluorocyclopropyl)-2-(formamidomethyl)thiazole-4-carboxylate (1210 mg) as starting material, 950 mg of rac-ethyl 2-((1*R*,2*R*)-2-fluorocyclopropyl)imidazo[5,1-*b*]thiazole-3-carboxylate are obtained as a yellow powder. LC-MS (acidic): $t_R$ = 0.60; $[M+H]^+$ = 254.98.

Step 6: Preparation of *rac*-(2-((1*R*,2*R*)-2-fluorocyclopropyl)imidazo[5,1-b]thiazol-3-yl)methanol

According to the procedure described for the preparation of Intermediate 5/Step 6 and using rac-ethyl 2-((1*R*,2*R*)-2-fluorocyclopropyl)imidazo[5,1-*b*]thiazole-3-carboxylate (950 mg, 3.74 mmol) as starting material, 810 mg of *rac*-(2-((1*R*,2*R*)-2-fluorocyclopropyl)imidazo[5,1-*b*]thiazol-3-yl)methanol are obtained as a yellow powder and used in the next step without purification. LC-MS (basic): $t_R$ = 0.42; $[M+H]^+$ = 213.02.

Step 7: Preparation of *rac*-2-((1*R*,2*R*)-2-fluorocyclopropyl)imidazo[5,1-*b*]thiazole-3-carbaldehyde

According to the procedure described for the preparation of Intermediate 5/Step 7 and using *rac*-(2-((1*R*,2*R*)-2-fluorocyclopropyl)imidazo[5,1-*b*]thiazol-3-yl)methanol (810 mg) as starting material, 1210 mg of *rac*-2-((1*R*,2*R*)-2-fluorocyclopropyl)imidazo[5,1-b]thiazole-3-carbaldehyde are obtained as a yellow powder. LC-MS (acidic): $t_R$ = 0.42; $[M+H]^+$ = 211.02.$^1$H NMR (400 MHz, $d_6$-DMSO) $\delta$: 10.13 (s, 1 H), 8.55 (s, 1 H), 7.17 (s, 1 H), 5.11-5.29 (m), 3.43-3.44 (m, 1H), 1.92 (m, 1 H), 1.40 (dd, J1 = 6.8 Hz, J2 = 11.6 Hz, 1 H).

Step 8: Preparation of *trans*-1-[2-(2-fluoro-cyclopropyl)-imidazo[5,1-*b*]thiazol-3-yl]-prop-2-yn-1-ol (intermediate 9)

According to the procedure described for the preparation of Intermediate 5/Step 8 and using *rac*-2-((1R,2R)-2-fluorocyclopropyl)imidazo[5,1-b]thiazole-3-carbaldehyde (890 mg) as the starting material, 701 mg of *trans*-1-[2-(2-fluoro-cyclopropyl)-imidazo[5,1-*b*]thiazol-3-yl]-prop-2-yn-1-ol are obtained as a brown foam. LC-MS (acidic): $t_R$ = 0.48; $[M+H]^+$ = 237.3.

**Intermediate 10: 3-Bromo-2-cyclopropylimidazo[5,1-b]thiazole**

Step 1: Preparation of 2,4-dibromo-5-cyclopropylthiazole

To a solution of 4-bromo-5-cyclopropyl-thiazole (6.10 g; 28.1 mmol) in $CH_3CN$ (200 ml) is added HBr (10.3 ml; 91.3 mmol) and bromine (4.69 ml; 91.3 mmol). The resulting orange solution is stired at 95°C for 7 h. The mixture is cooled down to RT, treated with aq. $Na_2S_2O_3$ and extracted with EtOAc (2x). The combined organic extracts are washed with brine, dried ($MgSO_4$), filtered and concentrated dunder reduced pressure. Purification by FC (silicagel, Hept / EtOAc) gives 2.91 mg of 2,4-dibromo-5-cyclopropylthiazole as a light yellow oil. LC-MS (acidic): $t_R$ = 0.97; $[M+H]^+$ = 283.75. $^1$H NMR (400 MHz, $d_6$-DMSO) $\delta$: 2.03 (m, 1 H), 1.14 (m, 2 H), 0.72 (m, 2 H).

Step 2: Preparation of 4-bromo-5-cyclopropylthiazole-2-carbaldehyde

To a solution of the product of step 1, 2,4-dibromo-5-cyclopropylthiazole (2.91 g; 10.2 mmol) in THF (60 ml) is added n-BuLi (2.5 M in hexanes; 4.28 ml, 10.7 mmol) at -78°C. The mixture is stirred at this temperature for 10 min then DMF (2.05 ml; 26.5 mmol) is added and the stirring continued for 1 h. The mixture is allowed to warm up to RT, treated with 1M HCl (50 ml) and extracted with EtOAc (2x). The combined organic extracts are washed with brine, dried (MgSO4), filtered and concentrated under reduced pressure to give 2.48 g of 4-bromo-5-cyclopropylthiazole-2-carbaldehyde as a brown oil, which is used without further purification in the next step. LC-MS (acidic): $t_R$ = 0.88; $[M+H]^+$ = 231.91.

Step 3: Preparation of (4-bromo-5-cyclopropylthiazol-2-yl)methanol

To a solution of 4-bromo-5-cyclopropylthiazole-2-carbaldehyde (3500 mg, 12.4 mmol) in EtOH (100 ml) is added $NaBH_4$ (1000 mg, 26.4 mmol) and the resulting mixture is stirred at RT for 45 min. Water is added and the resulting aqueous phase is extracted twice with DCM. The combined organic layers are washed with saturated aqueous $NaHCO_3$ solution and brine then dried over $MgSO_4$, filtered and concentrated under reduced pressure to give (4-bromo-5-cyclopropylthiazol-2-yl)methanol (3060 mg) as an amber oil wihch is used in the next step without purification. LC-MS (acidic): $t_R$ = 0.72; $[M+H]^+$ = 233.91.

Step 4: Preparation of 4-bromo-2-(chloromethyl)-5-cyclopropylthiazole

To a solution of (4-bromo-5-cyclopropylthiazol-2-yl)methanol (3060 mg, 11.5 mmol) in DCM (60 ml) is added thionyl chloride (2.46 g) and the resulting mixture is stirred at RT for 45 min. The mixture is poured onto saturated aqueous $NaHCO_3$ solution and the aqueous phase is extracted thrice with DCM. The combined organic layers are washed with brine then dried over $MgSO_4$, filtered and concentrated under reduced pressure to give 4-bromo-2-(chloromethyl)-5-cyclopropylthiazole (2929 mg) as a brown oil wihch is used in the next step without purification. LC-MS

(acidic): $t_R$ = 0.94; $[M+H]^+$ = 253.84.

Step 5: Preparation of *N*-((4-bromo-5-cyclopropylthiazol-2-yl)methyl)formamide

To a solution of 4-bromo-2-(chloromethyl)-5-cyclopropylthiazole (2930 mg, 10.8 mmol) in DMF (50 ml) is added sodium difomylamide (1.54 g, 16.2 mmol). The resulting brown solution is stirred at RT for 4 h. The mixture is poured onto saturated aqueous $NaHCO_3$ solution and stirred for 1 h at RT before being extracted twice with ethyl acetate. The combined organic layers are washed with brine then dried over $MgSO_4$, filtered and concentrated under reduced pressure to give *N*-((4-bromo-5-cyclopropylthiazol-2-yl)methyl)formamide (3010 mg) as a brown oil which is used in the next step without purification. LC-MS (acidic): $t_R$ = 0.70; $[M+H]^+$ = 262.89. $^1H$ NMR (400 MHz, $d_6$-DMSO) $\delta$: 8.84 (m, 1 H), 8.16 (s, 1 H), 4.49 (d, J = 6.2 Hz, 2 H), 2.01 (m, 1 H), 1.10-1.14 (m, 2 H), 0.63-0.68 (m, 2 H).

Step 6: Preparation of 3-bromo-2-cyclopropylimidazo[5,1-b]thiazole (Intermediate 10)

To a solution of *N*-((4-bromo-5-cyclopropylthiazol-2-yl)methyl)formamide (3010 mg, 10.3 mmol) in toluene (60 ml) is added $POCl_3$ (1740 mg, 11.3 mmol) and the resulting mixture is stirred at 70°C for 1 h. After cooling, the mixture is poured slowly onto saturated aqueous $NaHCO_3$ solution and extracted twice with ethyle acetate. The combined organic extracts are washed with brine, dried over $MgSO_4$, filtered and concentrated under reduced pressure. Purification by FC (silicagel, Hept / EtOAc) gives 1980 mg of 3-bromo-2-cyclopropylimidazo[5,1-b]thiazole (Intermediate 10) as a brown oil. LC-MS (acidic): $t_R$ = 0.54; $[M+H]^+$ = 242.77. $^1H$ NMR (400 MHz, $d_6$-DMSO) $\delta$: 8.13 (s, 1 H), 7.14 (s, 1 H), 2.06 (m, 1 H), 1.07-1.12 (m, 2 H), 0.75 (m, 2 H).

**Intermediate 11: 3-Bromo-2-methylimidazo[5,1-b]thiazole**

Step 1: Preparation of (4-bromo-5-methylthiazol-2-yl)methanol

According to the procedure described for the preparation of Intermediate 10/Step 3 and using 4-bromo-5-methylthiazole-2-carbaldehyde (7740 mg, 36.1 mmol) as starting material, 7060 mg of (4-bromo-5-methylthiazol-2-yl)methanol are obtained as a yellow oil and used without any further purification. LC-MS (acidic): $t_R$ = 0.60; $[M+H]^+$ = 207.92.

Step 2: Preparation of 4-bromo-2-(chloromethyl)-5-methylthiazole

According to the procedure described for the preparation of Intermediate 10/Step 4 and using (4-bromo-5-methylthiazol-2-yl)methanol (7060 mg, 26.8 mmol) as starting material, 6850 mg of 4-bromo-2-(chloromethyl)-5-methylthiazole are obtained as a brown oil and used without further purification. LC-MS (acidic): $t_R$ = 0.85; $[M+H]^+$ = 227.87.

Step 3: Preparation of *N*-((4-bromo-5-methylthiazol-2-yl)methyl)formamide

According to the procedure described for the preparation of Intermediate 10/Step 5 and using 4-bromo-2-(chloromethyl)-5-methylthiazole (6850 mg, 25.1 mmol) as starting material, 6050 mg of *N*-((4-bromo-5-methylthiazol-2-yl)methyl)formamide are obtained as a brown solid and used without purification in the next step. LC-MS (acidic): $t_R$ = 0.59; $[M+H]^+$ = 236.90. $^1H$ NMR (400 MHz, $d_6$-DMSO) $\delta$8.87 (s, 1 H), 8.17 (s, 1 H), 4.51 (d, J = 6.2 Hz, 2 H), 2.51 (s, 3 H).

Step 4: Preparation of 3-bromo-2-methylimidazo[5,1-b]thiazole (Intermediate 11)

According to the procedure described for the preparation of Intermediate 10/Step 6 and using *N*-((4-bromo-5-methylthiazol-2-yl)methyl)formamide (6050 g, 22.1 mmol) as starting material, 3680 mg of 3-bromo-2-methylimidazo[5,1-*b*]thiazole are obtained as a light brown solid. LC-MS (acidic): $t_R$ = 0.44; $[M+H]^+$ = 218.91. $^1H$ NMR (400 MHz, $d_6$-DMSO) $\delta$ 9.54 (s, 1 H), 7.76 (d, J = 0.9 Hz, 1 H), 2.41 (s, 3 H).

**Intermediate 12: *rac*-1-(2-(Trifluoromethyl)imidazo[5,1-*b*]thiazol-3-yl)prop-2-yn-1-ol**

Step 1: Preparation of ethyl 4,4,4-trifluoro-2-(hydroxyimino)-3-oxobutanoate

To an aqueous solution of sodium nitrite (40 ml; 312 mmol) is added a solution of ethyl 4,4,4-trifluoro-3-oxobutanoate (50.0 g; 272 mmol) in glacial acetic acid (90 ml) while maintaining the temperature at 0-5°C. The reaction mixture is stirred in an ice-water bath for 30 min, and at RT for another 5 h. The solution is concentrated under reduced

pressure to give 54 g of ethyl 4,4,4-trifluoro-2-(hydroxyimino)-3-oxobutanoate. $^1$H NMR (400 MHz, $CDCl_3$) $\delta$: 8.40-9.55 (br s, 1 H), 4.42 (q, *J* = 7.0 Hz, 2 H), 1.45 (t, J = 7.0 Hz, 3 H).

Step 2: Preparation of ethyl 2-amino-4,4,4-trifluoro-3-oxobutanoate hydrochloride

A suspension of ethyl 4,4,4-trifluoro-2-(hydroxyimino)-3-oxobutanoate (54.0 g; 253 mmol) and palladium on activated carbon in a mixture of ethanol (150 ml) and 4N HCI (100 ml) is stirred under 0.3-0.5 MPa of hydrogen at RT for 2.5 h. The reaction mixture is then filtered, the filtrate concentrated under reduced pressure to give 42.0 g of ethyl 2-amino-4,4,4-trifluoro-3-oxobutanoate hydrochloride as a yellow solid. $^1$H NMR (400 MHz, $COCl_3$) $\delta$: 8.40-9.55 (br s, 1 H), 4.31 (q, J = 7.0 Hz, 2 H), 4.10 (s, 1 H), 1.22 (t, J = 7.0 Hz, 3 H).

Step 3: Preparation of ethyl 2-(2-((*tert*-butoxycarbonyl)amino)acetamido)-4,4,4-trifluoro-3-oxobutanoate

Boc-Gly-OH (24.98 g; 143 mmol) is dissolved in THF (250 ml) and cooled to -20°C. $Et_3N$ (19.8 ml; 143 mmol) is added, followed by isobutyl chloroformate (19.48 g; 143 mmol). The reaction mixture is stirred for 30 minutes at -20°C followed by slow addition of a solution of ethyl 2-amino-4,4,4-trifluoro-3-oxobutanoate hydrochloride (42.0 g; 178.7 mmol) in THF (250 ml). Then a second portion of $Et_3N$ (19.8 ml; 143 mmol) is slowly added to the reaction mixture. The reaction mixture is warmed to RT and stirring is continued for 2 h. Water and EtOAc are added, the layers separated and the organic layer washed with saturated aqueous $NaHCO_3$ solution, dried over $MgSO_4$, filtered and concentrated under reduced pressure. The residue is purified by FC (Silicagel; Hexanes / EtOAc) to give 46.5 g of ethyl 2-(2-((*tert*-butoxycarbonyl)amino)acetamido)-4,4,4-trifluoro-3-oxobutanoate as a thick yellow oil (mixture of keto- and enol tautomers). $^1$H NMR (400 MHz, $COCl_3$) $\delta$: 7.05-7.47 (m, 1.5 H), 4.92-5.00 (m, 1 H), 4.33-4.45 (m, 2 H), 4.10 (s, 0.5 H), 3.77-3.89 (m, 2 H), 1.45 (s, 9 H), 1.25-1.33 (m, 3 H).

Step 4: Preparation of ethyl 2-(((*tert*-butoxycarbonyl)amino)methyl)-5-(trifluoromethyl)thiazole-4-carboxylate

Ethyl 2-(2-((*tert*-butoxycarbonyl)amino)acetamido)-4,4,4-trifluoro-3-oxobutanoate (46.5 g; 131 mmol) is dissolved in THF (800 ml) followed by the addition of Lawesson's reagent (79.0 g; 195 mmol). The mixture is stirred at reflux for 8 hours. THF is then removed under reduced pressure, the residue is dissolved in EtOAc and washed twice with saturated aq. $NaHCO_3$ solution and with brine, dried over $MgSO_4$, filtered and the solvent is evaporated under reduced pressure to give 24.8 g of ethyl 2-(((*tert*-butoxycarbonyl)amino)methyl)-5-(trifluoromethyl)thiazole-4-carboxylate.

Step 5: Preparation of ethyl 2-(formamidomethyl)-5-(trifluoromethyl)thiazole-4-carboxylate

Step 5.1: Boc-cleavage: The product from step 4, ethyl 2-(((*tert*-butoxycarbonyl)amino)methyl)-5-(trifluoromethyl)thiazole-4-carboxylate (24.8 g; 70 mmol) is dissolved in TFA (96 g; 840 mmol). The mixture is stirred at RT for 1 h. The reaction mixture is evaporated to dryness under reduced pressure.

Step 5.2: The residue from step 5.1 is dissolved in ethyl formate (150 ml) and the mixture stirred at reflux for 5 h. The solution is then concentrated under reduced pressure to give 14.1 g of ethyl 2-(formamidomethyl)-5-(trifluoromethyl)thiazole-4-carboxylate. $^1$H NMR (400 MHz, $CDCl_3$) $\delta$: 8.22-8.33 (m, 1 H), 4.20-4.48 (m, 2 H), 3.95-4.05 (m, 2 H), 1.15-1.44 (m, 3 H).

Step 6: Preparation of ethyl 2-(trifluoromethyl)imidazo[5,1-b]thiazole-3-carboxylate

Phosphorous(V) oxychloride ($POCl_3$) (7 ml; 74.9 mmol) is added at RT to a solution of ethyl 2-(formamidomethyl)-5-(trifluoromethyl)thiazole-4-carboxylate (14.1 g; 50.0 mmol) in toluene (150 ml). The reaction mixture is stirrd at reflux for 2 h. Toluene and $POCl_3$ are removed under reduced pressure, water is added to the residue and the pH adjusted to pH 8 by adding solid $NaHCO_3$. The aq layer is extracted with $CH_2Cl_2$ (2x), dried ($MgSO_4$), filtered and concentrated under reduced pressure to give 10.5 g of ethyl 2-(trifluoromethyl)imidazo[5,1-b]thiazole-3-carboxylate as a yellow solid.

Step 7: Preparation of (2-(trifluoromethyl)imidazo[5,1-b]thiazol-3-yl)methanol

$NaBH_4$ (4.76 g; 119 mmol) is added to a solution of ethyl 2-(trifluoromethyl)imidazo[5,1-b]thiazole-3-carboxylate (10.5 g; 39.7 mmol) in methanol (50 ml) and stirring is continued at 50°C for 5 h. The solvent is removed under reduced pressure, water is added and the mixture extracted with EtOAc (5x). The combined organic layers are dried over $MgSO_4$, filtered and concentrated under reduced pressure to give 7.0 g of (2-(trifluoromethyl)imidazo[5,1-b]thiazol-3-yl)methanol as a yellow solid. $^1$H NMR (400 MHz, $CDCl_3$) $\delta$: 8.22 (s, 1 H), 7.04 (s, 1 H), 4.98 (s, 2 H).

Step 8: Preparation of 2-(trifluoromethyl)imidazo[5,1-b]thiazole-3-carbaldehyde

Dess-Martin periodinane (14.7 g; 34.7 mmol) is added to a solution of (2-(trifluoromethyl)imidazo[5,1-b]thiazol-3-yl)methanol (7.0 g; 0.541 mmol) in dichloromethane (250 ml) and stirring is continued for 2 hours at RT. The reaction mixture is then concentrated under reduced pressure, diluted with 1N NaOH, and extracted with EtOAc (3x). The combined organic extracts are dried over $MgSO_4$, filtered and concentrated under reduced pressure to give 3.0 g of 2-(trifluoromethyl)imidazo[5,1-*b*]thiazole-3-carbaldehyde. $^1H$ NMR (400 MHz, $CDCl_3$) $\delta$: 10.06 (s, 1 H), 8.86 (s, 1 H), 7.12 (s, 1 H).

Step 9: Preparation of *rac*-1-(2-(trifluoromethyl)imidazo[5,1-*b*]thiazol-3-yl)prop-2-yn-1-ol (Intermediate 12)

According to the procedure described for the preparation of Intermediate 5/Step 8 and using 2-(trifluoromethyl)imidazo[5,1-b]thiazole-3-carbaldehyde (70 mg) as the starting material, 76 mg of 1-(2-(trifluoromethyl)imidazo[5,1-*b*]thiazol-3-yl)prop-2-yn-1-ol are obtained as a brown sticky oil. LC-MS (acidic): $t_R$ = 0.58; $[M+H]^+$ = 247.07.

**EXAMPLES SYNTHESIS**

**Example 1: rac-(1-Benzyl-1H-[1,2,3]triazol-4-yl)-(2-cyclopropyl-imidazo[5,1-b]thiazol-3-yl)-methanol**

In a vial under argon, Intermediate 1, *rac*-1-(2-cyclopropylimidazo[5,1-*b*]thiazol-3-yl)prop-2-yn-1-ol (22 mg, 0.10 mmol), benzylazide (20 mg, 0.15 mmol), copper (II) sulfate (1.25 mg, 0.005 mmol) and L-(+)-ascorbic acid sodium salt (2 mg, 0.01 mmol) are dissolved in a mixture of degassed DMF (0.6 mL) and water (0.1 mL). The reaction mixture is stirred at RT for 16 h. The mixture is filtered through a Whatman 0.45 μM glass microfiber filter, concentrated under reduced pressure and purified by prepHPLC (basic conditions) to give 26 mg of *rac*-(1-benzyl-1H-[1,2,3]triazol-4-yl)-(2-cyclopropyl-imidazo[5,1-b]thiazol-3-yl)-methanol. LC-MS (QC): $t_R$ = 0.57; $[M+H]^+$ = 352.40.

**Example 2: rac-(2-Cyclopropyl-imidazo[5,1-b]thiazol-3-yl)-(1-phenyl-1H-[1,2,3]triazol-4-yl)-methanol**

Prepared following the procedure described for Example 1, and using azidobenzene. Purification by prepHPLC (basic conditions) gives 28 mg of *rac*-(2-cyclopropyl-imidazo[5,1-*b*]thiazol-3-yl)-(1-phenyl-1*H*-[1,2,3]triazol-4-yl)-methanol. LC-MS (QC): $t_R$ = 0.58; $[M+H]^+$ = 338.00.

**Example 2a: (*R*)-(2-Cyclopropyl-imidazo[5,1-*b*]thiazol-3-yl)-(1-phenyl-1*H*-[1,2,3]triazol-4-yl)-methanol**

Separation of the enantiomers on chiral stationary phase:
Method: Column: ChiralPak IA 30x250mm, 5μM; Detector Settings: UV-Vis-1; 210 nm; Eluent: 65% $CO_2$ and 35% (EtOH, 0.1% DEA); Flow: 160.00 ml/min; BPR: 100 bar; Temperature: 40°C. Injection volume: 1500μl.
10 mg of the racemate are separated by the method described above to give:
3.2 mg of the R-enantiomer Example 2a and 4.0 mg of the S-enantiomer.

**Example 3: rac-(2-Cyclopropyl-imidazo[5,1-b]thiazol-3-yl)-(1-o-tolyl-1H-[1,2,3]triazol-4-yl)-methanol**

Prepared following the procedure described for Example 1, and using 2-azidotoluene. Purification by prepHPLC (basic conditions) gives 2.5 mg of *rac*-(2-cyclopropyl-imidazo[5,1-*b*]thiazol-3-yl)-(1-*o*-tolyl-1*H*-[1,2,3]triazol-4-yl)-methanol. LC-MS (QC): $t_R$ = 0.61; $[M+H]^+$ = 352.01.

**Example 4: rac-(2-Cyclopropyl-imidazo[5,1-b]thiazol-3-yl)-(1-p-tolyl-1H-[1,2,3]triazol-4-yl)-methanol**

Prepared following the procedure described for Example 1, and using 4-azidotoluene. Purification by prepHPLC (basic conditions) gives 12 mg of *rac*-(2-cyclopropyl-imidazo[5,1-*b*]thiazol-3-yl)-(1-*p*-tolyl-1*H*-[1,2,3]triazol-4-yl)-methanol. LC-MS (QC): $t_R$ = 0.65; $[M+H]^+$ = 352.05.

**Example 5: rac-(2-Cyclopropyl-imidazo[5,1-b]thiazol-3-yl)-[1-(3-trifluoromethyl-phenyl)-1H-[1,2,3]triazol-4-yl]-methanol**

Prepared following the procedure described for Example 1, and using 1-azido-3-(trifluoromethyl)benzene. Purification by prepHPLC (basic conditions) gives 17 mg of *rac*-(2-cyclopropyl-imidazo[5,1-*b*]thiazol-3-yl)-[1-(3-trifluoromethyl-phenyl)-1*H*-[1,2,3]triazol-4-yl]-methanol. LC-MS (QC): $t_R$ = 0.73; $[M+H]^+$ = 406.09.

**Example 6: rac-(2-Cyclopropyl-imidazo[5,1-b]thiazol-3-yl)-[1-(4-trifluoromethyl-phenyl)-1H-[1,2,3]triazol-4-yl]-methanol**

Prepared following the procedure described for Example 1, and using 1-azido-4-(trifluoromethyl)benzene. Purification by prepHPLC (basic conditions) gives 9 mg of *rac*-(2-cyclopropyl-imidazo[5,1-*b*]thiazol-3-yl)-[1-(4-trifluoromethyl-phenyl)-1*H*-[1,2,3]triazol-4-yl]-methanol. LC-MS (QC): $t_R$ = 0.74; $[M+H]^+$ = 406.05.

**Example 7: rac-(2-Cyclopropyl-imidazo[5,1-b]thiazol-3-yl)-[1-(2-methoxy-phenyl)-1H-[1,2,3]triazol-4-yl]-methanol**

Prepared following the procedure described for Example 1, and using 2-azido-anisole. Purification by prepHPLC (basic conditions) gives 27 mg of *rac*-(2-cyclopropyl-imidazo[5,1-*b*)]thiazol-3-yl)-[1-(2-methoxy-phenyl)-1H-[1,2,3]triazol-4-yl]-methanol. LC-MS (QC): $t_R$ = 0.60; $[M+H]^+$ = 368.10.

**Example 8: rac-(2-Cyclopropyl-imidazo[5,1-b]thiazol-3-yl)-[1-(3-methoxy-phenyl)-1H-[1,2,3]triazol-4-yl]-methanol**

Prepared following the procedure described for Example 1, and using 3-azido-anisole. Purification by prepHPLC (basic conditions) gives 21 mg of rac-(2-cyclopropyl-imidazo[5,1-b]thiazol-3-yl)-[1-(3-methoxyphenyl)-1*H*-[1,2,3]triazol-4-yl]-methanol. LC-MS (QC): $t_R$ = 0.62; $[M+H]^+$ = 368.01.

**Example 9: rac-(2-Cyclopropyl-imidazo[5,1-b]thiazol-3-yl)-[1-(4-methoxy-phenyl)-1H-[1,2,3]triazol-4-yl]-methanol**

Prepared following the procedure described for Example 1, and using 4-azido-anisole. Purification by prepHPLC (basic conditions) gives 14 mg of rac-(2-cyclopropyl-imidazo[5,1-b]thiazol-3-yl)-[1-(4-methoxyphenyl)-1*H*-[1,2,3]triazol-4-yl]-methanol. LC-MS (QC): $t_R$ = 0.61; $[M+H]^+$ = 368.23

**Example 9a: (*R*)-(2-Cyclopropyl-imidazo[5,1-*b*]thiazol-3-yl)-[1-(4-methoxy-phenyl)-1*H*-[1,2,3]triazol-4-yl]-methanol**

Separation of the enantiomers on chiral stationary phase:
Method: Column: ChiralPak IA 30x250mm, 5μM; Detector Settings: UV-Vis-1; 266 nM; Eluent: 60% $CO_2$ and 40% ($CH_2Cl_2$/EtOH 1:1, 0.1% DEA); Flow: 160.00 ml/min; BPR: 100 bar; Temperature: 40°C. Injection volume: 2000μl.
30 mg of the racemate are separated by the method described above to give:
15 mg of the R-enantiomer Example 9a and 15 mg of the S-enantiomer.

**Example 10: *rac*-1-(4-{4-[(2-Cyclopropyl-imidazo[5,1-*b*]thiazol-3-yl)-hydroxy-methyl]-[1,2,3]triazol-1-yl}-phenyl)-ethanone**

Prepared following the procedure described for Example 1, and using p-azidoacetophenone. Purification by prepHPLC (basic conditions) gives 6 mg of *rac*-1-(4-{4-[(2-cyclopropyl-imidazo[5,1-*b*]thiazol-3-yl)-hydroxy-methyl]-[1,2,3]triazol-1-yl}-phenyl)-ethanone. LC-MS (QC): $t_R$ = 0.58; $[M+H]^+$ = 380.27.

**Example 11: *rac*-4-{4-[(2-Cyclopropyl-imidazo[5,1-*b*]thiazol-3-yl)-hydroxy-methyl]-[1,2,3]triazol-1-yl}-benzonitrile**

Step 1: Preparation of 4-azidobenzonitrile

In a vial, 4-cyanophenylboronic acid (147 mg, 1.0 mmol), sodium azide (98 mg, 1.5 mmol) and copper (II) acetate (18 mg, 0.1 mmol) are suspended in MeOH (5 mL). The reaction mixture is stirred at 60°C for 3 h under an air atmosphere. The solvent is removed under reduced pressure and the residue purified by FC (silica gel, heptane / EtOAc) to give 107 mg of 4-aziodobenzonitrile as a beige solid. LC-MS (basic): $t_R$ = 0.82; $[M+H]^+$ = not observed.

Step 2: Preparation of *rac*-4-{4-[(2-cyclopropyl-imidazo[5,1-*b*]thiazol-3-yl)-hydroxy-methyl]-[1,2,3]triazol-1-yl}-benzonitrile (Example 11)

In a vial under argon, *rac*-1-(2-cyclopropylimidazo[5,1-*b*]thiazol-3-yl)prop-2-yn-1-ol (22 mg, 0.10 mmol), the

product from step1, 4-azidobenzonitrile (14 mg, 0.10 mmol), copper (II) sulfate (1.25 mg, 0.005 mmol) and L-(+)-ascorbic acid sodium salt (2 mg, 0.01 mmol) are dissolved in a mixture of degassed DMF (0.6 mL) and water (0.1 mL). The reaction mixture is stirred at RT for 2 h. The mixture is filtered through a Whatman 0.45 μM glass microfiber filter, concentrated under reduced pressure and purified by prepHPLC (basic conditions) to give 15 mg of *rac*-4-{4-[(2-cyclopropyl-imidazo[5,1-*b*]thiazol-3-yl)-hydroxy-methyl]-[1,2,3]triazol-1-yl}-benzonitrile. LC-MS (QC): $t_R$ = 0.58; $[M+H]^+$ = 363.37.

**Example 12: rac-(2-Ethyl-imidazo[5,1-b]thiazol-3-yl)-(1-phenyl-1H-[1,2,3]triazol-4-yl)-methanol**

Prepared following the procedure described for Example 1, and using Intermediate 3, *rac-1-(2*-ethylimidazo[5,1-*b*]thiazol-3-yl)prop-2-yn-1-ol and azidobenzene. Purification by prepHPLC (basic conditions) gives 16 mg of rac-(2-ethyl-imidazo[5,1-b]thiazol-3-yl)-(1-phenyl-1H-[1,2,3]triazol-4-yl)-methanol. LC-MS (QC): $t_R$ = 0.56; $[M+H]^+$ = 326.34.

**Example 13: rac-(2-Cyclopentyl-imidazo[5,1-b]thiazol-3-yl)-(1-phenyl-1H-[1,2,3]triazol-4-yl)-methanol**

Prepared following the procedure described for Example 1, and using Intermediate 4, *rac-1-(2*-cyclopentylimidazo[5,1-*b*]thiazol-3-yl)prop-2-yn-1-ol and azidobenzene. Purification by prepHPLC (basic conditions) gives 14 mg of *rac*-(2-cyclopentyl-imidazo[5,1-b]thiazol-3-yl)-(1-phenyl-1H-[1,2,3]triazol-4-yl)-methanol: LC-MS (QC): $t_R$ = 0.72; $[M+H]^+$ = 366.28.

**Example 14: rac-(2-Cyclopropyl-imidazo[5,1-b]thiazol-3-yl)-(1-pyridin-2-yl-1H-[1,2,3]triazol-4-yl)-methanol**

Step 1: Preparation of 2-azidopyridine

Prepared according to the procedure described for Example 11, step 1 using pyridin-2-ylboronic acid.

Step 2: Preparation of *rac*-(2-cyciopropyl-imidazo[5,1-*b*]thiazol-3-yl)-(1-pyridin-2-yl-1*H*-[1,2,3]triazol-4-yl)-methanol (Example 14)

A solution of 2-azidopyridine (12 mg, 0.10 mmol), Intermediate 1, rac-1-(2-cyclopropylimidazo[5,1-b]thiazol-3-yl)prop-2-yn-1-ol (22 mg, 0.10 mmol), and L-(+)-ascorbic acid sodium salt (2 mg, 0.01 mmol) is injected in a flow reactor (consisting of an HPLC pump and a copper coil with internal volume of approximately 10 ml and a back pressure regulator (250 psi total pressure)) using the following conditions: T = 140°C, P = 14 bar, F = 0.250 ml/min, RT = 40 min. The solution is concentrated under reduced pressure and purified by prepHPLC (acidic conditions) to give 20 mg of *rac*-(2-cyclopropyl-imidazo[5,1-*b*]thiazol-3-yl)-(1-pyridin-2-yl-1*H*-[1,2,3]triazol-4-yl)-methanol. LC-MS (QC): $t_R$ = 0.46; $[M+H]^+$ = 339.24.

**Example 15: rac-(2-Cyclopropyl-imidazo[5,1-b]thiazol-3-yl)-(1-pyridin-3-yl-1H-[1,2,3]triazol-4-yl)-methanol**

Prepared according to the procedure described for Example 14.
Purification by prepHPLC (acidic conditions) to give *rac*-(2-cyclopropyl-imidazo[5,1-*b*]thiazol-3-yl)-(1-pyridin-3-yl-1*H*-[1,2,3]triazol-4-yl)-methanol. LC-MS (QC): $t_R$ = 0.78; $[M+H]^+$ = 339.17.

**Example 16: rac-(2-Cyclopropyl-imidazo[5,1-b]thiazol-3-yl)-[1-(1H-indol-5-yl)-1H-[1,2,3]triazol-4-yl]-methanol**

Prepared according to the procedure described for Example 14.
Purification by prepHPLC (basic conditions) to give *rac*-(2-cyclopropyl-imidazo[5,1-*b*]thiazol-3-yl)-[1-(1*H*-indol-5-yl)-1H-[1,2,3]triazol-4-yl]-methanol. LC-MS (QC): $t_R$ = 0.58; $[M+H]^+$ = 377.25.

**Example 16a: (*R*)-(2-Cyclopropyl-imidazo[5,1-*b*]thiazol-3-yl)-[1-(1*H*-indol-5-yl)-1*H*-[1,2,3]triazol-4-yl]-methanol**

Separation of the enantiomers on chiral stationary phase:
Method: Column: ChiralCel OJ-H 30x250mm, 5μM; Detector Settings: UV-Vis-1; 244 nM; Eluent: 65% $CO_2$ and 35% (MeOH, 0.1% DEA); Flow: 160.00 ml/min; BPR: 100 bar; Temperature: 40°C. Injection volume: 4000μl.
25 mg of the racemate are separated by the method described above to give:
11 mg of the R-enantiomer Example 16a and 10 mg of the S-enantiomer.

**Example 17: rac-(2-Methyl-imidazo[5,1-b]thiazol-3-yl)-(1-phenyl-1H-[1,2,3]triazol-4-yl)-methanol**

Prepared following the procedure described for Example 1, and using Intermediate 2, *rac*-1-(2-methylimidazo[5,1-*b*]thiazol-3-yl)prop-2-yn-1-ol and azidobenzene. Purification by prepHPLC (basic conditions) gives 20 mg of *rac*-(2-methyl-imidazo[5,1-*b*]thiazol-3-yl)-(1-phenyl-1*H*-[1,2,3]triazol-4-yl)-methanol. LC-MS (QC): $t_R$ = 0.50; $[M+H]^+$ = 312.15.

**Example 18: *rac*-3-{4-[(2-Cyclopropyl-imidazo[5,1-*b*]thiazol-3-yl)-hydroxy-methyl]-[1,2,3]triazol-1-yl}-benzonitrile**

Prepared according to the procedure described for Example 14.
Purification by prepHPLC (basic conditions) to give *rac*-3-{4-[(2-cyclopropyl-imidazo[5,1-*b*]thiazol-3-yl)-hydroxymethyl]-[1,2,3]triazol-1-yl}-benzonitrile. LC-MS (QC): $t_R$ = 0.58; $[M+H]^+$ = 363.23.

**Example 18a: 3-{4-[(*R*)-(2-Cyclopropyl-imidazo[5,1-*b*]thiazol-3-yl)-hydroxy-methyl]-[1,2,3]triazol-1-yl}-benzonitrile**

Separation of the enantiomers on chiral stationary phase:
Method: Column: ChiralCel OD-H 30x250mm, 5μM; Detector Settings: UV-Vis-1; 210 nM; Eluent: 70% $CO_2$ and 30% MeOH; Flow: 160.00 ml/min; BPR: 100 bar; Temperature: 40°C; Injection volume: 3000 μl.
12 mg of the racemate are separated by the method described above to give:
5 mg of the R-enantiomer Example 18a and 4 mg of the S-enantiomer.

**Example 19: rac-(2-Cyclopropyl-imidazo[5,1-b]thiazol-3-yl)-(1-pyrimidin-5-yl-1H-[1,2,3]triazol-4-yl)-methanol**

Prepared according to the procedure described for Example 14.
Purification by prepHPLC (acidic conditions) to give *rac*-(2-cyclopropyl-imidazo[5,1-*b*]thiazol-3-yl)-(1-pyrimidin-5-yl-1*H*-[1,2,3]triazol-4-yl)-methanol. LC-MS (QC): $t_R$ = 0.73; $[M+H]^+$ = 340.02.

**Example 20: rac-(2-Cyclopropyl-imidazo[5,1-b]thiazol-3-yl)-[1-(1H-indol-4-yl)-1H-[1,2,3]triazol-4-yl]-methanol**

Prepared according to the procedure described for Example 14.
Purification by prepHPLC (acidic conditions) to give *rac*-(2-cyclopropyl-imidazo[5,1-*b*]thiazol-3-yl)-[1-(1*H*-indol-4-yl)-1*H*-[1,2,3]triazol-4-yl]-methanol. LC-MS (QC): $t_R$ = 0.57; $[M+H]^+$ = 377.27.

**Example 21: rac-(2-Cyclopropyl-imidazo[5,1-b]thiazol-3-yl)-[1-(1H-indol-6-yl)-1H-[1,2,3]triazol-4-yl]-methanol**

Prepared according to the procedure described for Example 14.
Purification by prepHPLC (basic conditions) to give *rac*-(2-cyclopropyl-imidazo[5,1-*b*]thiazol-3-yl)-[1-(1*H*-indol-6-yl)-1*H*-[1,2,3]triazol-4-yl]-methanol. LC-MS (QC): $t_R$ = 0.62; $[M+H]^+$ = 377.07.

**Example 22: rac-(2-Cyclopropyl-imidazo[5,1-b]thiazol-3-yl)-(1-isoquinolin-8-yl-1H-[1,2,3]triazol-4-yl)-methanol**

Prepared according to the procedure described for Example 14.
Purification by prepHPLC (basic conditions) to give *rac*-(2-cyclopropyl-imidazo[5,1-*b*]thiazol-3-yl)-(1-isoquinolin-8-yl-1*H*-[1,2,3]triazol-4-yl)-methanol. LC-MS (QC): $t_R$ = 0.50; $[M+H]^+$ = 389.15.

**Example 23: *rac*-(2-Cyclopropyl-imidazo[5,1-*b*]thiazol-3-yl)-(1-isoquinolin-5-yl-1*H*-[1,2,3]triazol-4-yl)-methanol**

Prepared according to the procedure described for Example 14.
Purification by prepHPLC (acidic conditions) to give *rac*-(2-cyclopropyl-imidazo[5,1-*b*]thiazol-3-yl)-(1-isoquinolin-5-yl-1*H*-[1,2,3]triazol-4-yl)-methanol. LC-MS (QC): $t_R$ = 0.49; $[M+H]^+$ = 389.37.

**Example 24: rac-(2-Cyclopropyl-imidazo[5,1-b]thiazol-3-yl)-(1-quinolin-5-yl-1H-[1,2,3]triazol-4-yl)-methanol**

Prepared according to the procedure described for Example 14.
Purification by prepHPLC (acidic conditions) to give *rac*-(2-cyclopropyl-imidazo[5,1-*b*]thiazol-3-yl)-(1-quinolin-5-yl-1*H*-[1,2,3]triazol-4-yl)-methanol. LC-MS (QC): $t_R$ = 0.52; $[M+H]^+$ = 389.10.

**Example 25: rac-(2-Cyclopropyl-imidazo[5,1-b]thiazol-3-yl)-(1-quinolin-4-yl-1H-[1,2,3]triazol-4-yl)-methanol**

Prepared according to the procedure described for Example 14.

Purification by prepHPLC (basic conditions) to give *rac*-(2-cyclopropyl-imidazo[5,1-*b*]thiazol-3-yl)-(1-quinolin-4-yl-1*H*-[1,2,3]triazol-4-yl)-methanol. LC-MS (QC): $t_R$ = 0.57; $[M+H]^+$ = 389.21.

**Example 26: *rac*-(2-Cyclopropyl-imidazo[5,1-*b*]thiazol-3-yl)-(1-isoquinolin-4-yl-1*H*-[1,2,3]triazol-4-yl)-methanol**

Prepared according to the procedure described for Example 14.

Purification by prepHPLC (basic conditions) to give rac-(2-cyclopropyl-imidazo[5,1-b]thiazol-3-yl)-(1-isoquinolin-4-yl-1*H*-[1,2,3]triazol-4-yl)-methanol. LC-MS (QC): $t_R$ = 0.57; $[M+H]^+$ = 389.15.

**Example 27: rac-(2-Cyclopropyl-imidazo[5,1-b]thiazol-3-yl)-[1-(1H-indol-7-yl)-1H-[1,2,3]triazol-4-yl]-methanol**

Prepared according to the procedure described for Example 14.

Purification by prepHPLC (basic conditions) to give *rac*-(2-cyclopropyl-imidazo[5,1-*b*]thiazol-3-yl)-[1-(1*H*-indol-7-yl)-1*H*-[1,2,3]triazol-4-yl]-methanol. LC-MS (QC): $t_R$ = 0.67; $[M+H]^+$ = 377.09.

**Example 28: rac-(2-Cyclopropyl-imidazo[5,1-b]thiazol-3-yl)-(1-m-tolyl-1H-[1,2,3]triazol-4-yl)-methanol**

Prepared following the procedure described for Example 1, and using Intermediate 1 and 3-azidotoluene. Purification by prepHPLC (basic conditions) gives 15 mg of *rac*-(2-cyclopropyl-imidazo[5,1-*b*]thiazol-3-yl)-(1-*m*-tolyl-1*H*-[1,2,3]triazol-4-yl)-methanol. LC-MS (QC): $t_R$ = 0.66; $[M+H]^+$ = 352.22.

**Example 29: rac-(2-Cyclopropyl-imidazo[5,1-b]thiazol-3-yl)-[1-(2-trifluoromethyl-phenyl)-1H-[1,2,3]triazol-4-yl]-methanol**

Prepared following the procedure described for Example 1, and using Intermediate 1 and 1-azido-2-(trifluoromethyl)benzene. Purification by prepHPLC (basic conditions) gives 30 mg of *rac*-(2-cyclopropyl-imidazo[5,1-*b*]thiazol-3-yl)-[1-(2-trifluoromethyl-phenyl)-1*H*-[1,2,3]triazol-4-yl]-methanol. LC-MS (QC): $t_R$ = 0.64; $[M+H]^+$ = 406.21.

**Example 30: rac-(2-Cyclopropyl-imidazo[5,1-b]thiazol-3-yl)-(1-pyridin-4-yl-1H-[1,2,3]triazol-4-yl)-methanol**

Prepared according to the procedure described for Example 14.

Purification by prepHPLC (basic conditions) to give *rac*-(2-cyclopropyl-imidazo[5,1-*b*]thiazol-3-yl)-(1-pyridin-4-yl-1*H*-[1,2,3]triazol-4-yl)-methanol. LC-MS (QC): $t_R$ = 0.75; $[M+H]^+$ = 338.92.

**Example 31: *rac*-(2-Cyclopropyl-imidazo[5,1-*b*]thiazol-3-yl)-[1-(4-trifluoromethoxy-phenyl)-1*H*-[1,2,3]triazol-4-yl]-methanol**

Prepared according to the procedure described for Example 14.

Purification by prepHPLC (basic conditions) to give *rac*-(2-cyclopropyl-imidazo[5,1-*b*]thiazol-3-yl)-[1-(4-trifluoromethoxy-phenyl)-1*H*-[1,2,3]triazol-4-yl]-methanol. LC-MS (QC): $t_R$ = 0.75; $[M+H]^+$ = 422.35.

**Example 32: rac-(2-Cyclopropyl-imidazo[5,1-b]thiazol-3-yl)-[1-(4-ethoxy-phenyl)-1H-[1,2,3]triazol-4-yl]-methanol**

Prepared according to the procedure described for Example 14.

Purification by prepHPLC (basic conditions) to give rac-(2-cyclopropyl-imidazo[5,1-b]thiazol-3-yl)-[1-(4-ethoxyphenyl)-1H-[1,2,3]triazol-4-yl]-methanol. LC-MS (QC): $t_R$ = 0.67; $[M+H]^+$ = 382.40.

**Example 33: *rac*-(2-Cyclopropyl-imidazo[5,1-*b*]thiazol-3-yl)-[1-(4-isopropoxy-phenyl)-1*H*-[1,2,3]triazol-4-yl]-methanol**

Prepared according to the procedure described for Example 14.

Purification by prepHPLC (basic conditions) to give *rac*-(2-cyclopropyl-imidazo[5,1-*b*]thiazol-3-yl)-[1-(4-isopropoxy-phenyl)-1*H*-[1,2,3]triazol-4-yl]-methanol. LC-MS (QC): $t_R$ = 0.72; $[M+H]^+$ = 396.11.

**Example 34: rac-[1-(4-Chloro-phenyl)-1H-[1,2,3]triazol-4-yl]-(2-cyclopropyl-imidazo[5,1-b]thiazol-3-yl)-methanol**

Prepared according to the procedure described for Example 14.

Purification by prepHPLC (basic conditions) to give *rac*-[1-(4-chloro-phenyl)-1*H*-[1,2,3]triazol-4-yl]-(2-cyclopropyl-imidazo[5,1-*b*]thiazol-3-yl)-methanol. LC-MS (QC): $t_R$ = 0.68; $[M+H]^+$ = 372.19.

**Example 35: *rac*-(2-Cyclopropyl-imidazo[5,1-*b*]thiazol-3-yl)-[1-(4-cyclopropylmethoxy-phenyl)-1*H*-[1,2,3]triazol-4-yl]-methanol**

Prepared according to the procedure described for Example 14.

Purification by prepHPLC (basic conditions) to give *rac*-(2-cyclopropyl-imidazo[5,1-*b*]thiazol-3-yl)-[1-(4-cyclopropylmethoxy-phenyl)-1*H*-[1,2,3]triazol-4-yl]-methanol. LC-MS (QC): $t_R$ = 0.74; $[M+H]^+$ = 407.96.

**Example 35a: (*R*)-(2-Cyclopropyl-imidazo[5,1-*b*]thiazol-3-yl)-[1-(4-cyclopropylmethoxy-phenyl)-1*H*-[1,2,3]triazol-4-yl]-methanol**

Separation of the enantiomers on chiral stationary phase: Method: Column: ChiralPak IA 30x250mm, 5μM; Detector Settings: UV-Vis-1; 266 nM; Eluent: 60% $CO_2$ and 40% (DCM/MeOH/DEA 50:50:0.1); Flow: 160.00 ml/min; BPR: 100 bar; Temperature: 40°C. Injection volume: 1000μl. 328.8 mg of the racemate of Example 35 are separated by the method described above to give both enantiomers contaminated with diethylamine. Purification by prep HPLC (basic conditions) to give 83.2 mg of the R-enantiomer Example 35a and 85.6 mg of the S-enantiomer.

**Example 36: *rac*-(4-{4-[(2-Cyclopropyl-imidazo[5,1-*b*]thiazol-3-yl)-hydroxy-methyl]-[1,2,3]triazol-1-yl}-phenyl)-carbamic acid methyl ester**

Prepared according to the procedure described for Example 14.

Purification by prepHPLC (basic conditions) to give *rac*-(4-{4-[(2-cyclopropyl-imidazo[5,1-*b*]thiazol-3-yl)-hydroxy-methyl]-[1,2,3]triazol-1-yl}-phenyl)-carbamic acid methyl ester. LC-MS (QC): $t_R$ = 0.56; $[M+H]^+$ = 411.07.

**Example 37: rac-(2-Cyclopropyl-imidazo[5,1-b]thiazol-3-yl)-[1-(4-morpholin-4-ylmethyl-phenyl)-1H-[1,2,3]triazol-4-yl]-methanol**

Prepared according to the procedure described for Example 14.

Purification by prepHPLC (basic conditions) to give *rac*-(2-cyclopropyl-imidazo[5,1-*b*]thiazol-3-yl)-[1-(4-morpholin-4-ylmethyl-phenyl)-1*H*-[1,2,3]triazol-4-yl]-methanol. LC-MS (QC): $t_R$ = 0.34; $[M+H]^+$ = 437.00.

**Example 38: *rac*-(2-Cyclopropyl-imidazo[5,1-*b*]thiazol-3-yl)-[1-(4-morpholin-4-yl-phenyl)-1*H*-[1,2,3]triazol-4-yl]-methanol**

Prepared according to the procedure described for Example 14.

Purification by prepHPLC (acidic conditions) to give *rac*-(2-cyclopropyl-imidazo[5,1-*b*]thiazol-3-yl)-[1-(4-morpholin-4-yl-phenyl)-1*H*-[1,2,3]triazol-4-yl]-methanol. LC-MS (QC): $t_R$ = 0.60; $[M+H]^+$ = 423.26.

**Example 39: rac-(2-Cyclopropyl-imidazo[5,1-b]thiazol-3-yl)-[1-(2,3-dihydro-benzo[1,4]dioxin-6-yl)-1H-[1,2,3]triazol-4-yl]-methanol**

Prepared according to the procedure described for Example 14.

Purification by prepHPLC (acidic conditions) to give *rac*-(2-cyclopropyl-imidazo[5,1-*b*]thiazol-3-yl)-[1-(2,3-dihydro-benzo[1,4]dioxin-6-yl)-1*H*-[1,2,3]triazol-4-yl]-methanol. LC-MS (QC): $t_R$ = 0.60; $[M+H]^+$ = 396.37.

**Example 40: *rac*-(2-Cyclopropyl-imidazo[5,1-*b*]thiazol-3-yl)-{1-[4-(1-hydroxymethyl-cyclopropyl)-phenyl]-1*H*-[1,2,3]triazol-4-yl}-methanol**

Prepared according to the procedure described for Example 14.

Purification by prepHPLC (basic conditions) to give *rac*-(2-cyclopropyl-imidazo[5,1-*b*]thiazol-3-yl)-{1-[4-(1-hydroxymethyl-cyclopropyl)-phenyl]-1*H*-[1,2,3]triazol-4-yl}-methanol. LC-MS (QC): $t_R$ = 0.59; $[M+H]^+$ = 408.09.

**Example 41: rac-(2-Cyclopropyl-imidazo[5,1-b]thiazol-3-yl)-[1-(1H-pyrazol-4-yl)-1H-[1,2,3]triazol-4-yl]-methanol**

Prepared according to the procedure described for Example 14.

Purification by prepHPLC (acidic conditions) to give *rac*-(2-cyclopropyl-imidazo[5,1-*b*]thiazol-3-yl)-[1-(1*H*-pyrazol-4-yl)-1*H*-[1,2,3]triazol-4-yl]-methanol. LC-MS (QC): $t_R$ = 0.40; $[M+H]^+$ = 328.08.

**Example 42: rac-(2-Cyclopropyl-imidazo[5,1-b]thiazol-3-yl)-[1-(6-ethoxy-pyridin-3-yl)-1H-[1,2,3]triazol-4-yl]-methanol**

Prepared according to the procedure described for Example 14.

Purification by prepHPLC (basic conditions) to give *rac*-(2-cyclopropyl-imidazo[5,1-*b*]thiazol-3-yl)-[1-(6-ethoxy-pyridin-3-yl)-1*H*-[1,2,3]triazol-4-yl]-methanol. LC-MS (QC): $t_R$ = 0.63; $[M+H]^+$ = 382.91.

**Example 43: rac-(2-Cyclopropyl-imidazo[5,1-b]thiazol-3-yl)-[1-(1-methyl-1H-pyrazol-4-yl)-1H-[1,2,3]triazol-4-yl]-methanol**

Prepared according to the procedure described for Example 14.

Purification by prepHPLC (acidic conditions) to give *rac*-(2-cyclopropyl-imidazo[5,1-*b*]thiazol-3-yl)-[1-(1-methyl-1*H*-pyrazol-4-yl)-1*H*-[1,2,3]triazol-4-yl]-methanol. LC-MS (QC): $t_R$ = 0.44; $[M+H]^+$ = 342.36.

**Example 44: *rac*-[1-(4-Methoxy-phenyl)-1*H*-[1,2,3]triazol-4-yl]-[2-(1-methyl-cyclopropyl)-imidazo[5,1-*b*]thiazol-3-yl]-methanol**

Prepared following the procedure described for Example 1, and using Intermediate 5, *rac*-1-(2-(1-methylcyclopropyl)imidazo[5,1-*b*]thiazol-3-yl)prop-2-yn-1-ol and 4-azidoanisole. Purification by prepHPLC (basic conditions) to give *rac*-[1-(4-methoxy-phenyl)-1*H*-[1,2,3]triazol-4-yl]-[2-(1-methyl-cyclopropyl)-imidazo[5,1-*b*]thiazol-3-yl]-methanol. LC-MS (QC): $t_R$ = 0.68; $[M+H]^+$ = 382.25.

**Example 45: *rac*-[1-(4-Benzyloxy-phenyl)-1*H*-[1,2,3]triazol-4-yl]-(2-cyclopropyl-imidazo[5,1-*b*]thiazol-3-yl)-methanol**

Step 1: Preparation of 1-azido-4-(benzyloxy)benzene

In a round-bottomed flask, 4-benzyloxyphenylboronic acid (1000 mg, 4.3 mmol), sodium azide (423 mg, 6.45 mmol) and copper (II) acetate (78 mg, 0.43 mmol) are suspended in MeOH (25 mL). The reaction mixture is stirred at 60°C for 3 h under an air atmosphere. The mixture is filtered, the solvent removed under reduced pressure and the residue purified by FC (silica gel, heptane / EtOAc) to give 583 mg of 1-azido-4-(benzyloxy)benzene. $^1H$ NMR (400 MHz, $CDCl_3$) $\delta$: 7.29-7.49 (m, 6 H), 7.01 (m, 3 H), 5.10 (d, J= 10.0 Hz, 2 H).

Step 2: Preparation of *rac*-[1-(4-(benzyloxy)phenyl)-1*H*-[1,2,3]triazol-4-yl]-(2-cyclopropylimidazo[5,1-*b*]thiazol-3-yl)methanol (Example 45)

In a flask under argon, *rac*-1-(2-cyclopropylimidazo[5,1-*b*]thiazol-3-yl)prop-2-yn-1-ol (350 mg, 1.6 mmol), the product from step1, 1-azido-4-(benzyloxy)benzene (542 mg, 2.41 mmol), copper (II) sulfate (20 mg, 0.08 mmol) and L-(+)-ascorbic acid sodium salt (32 mg, 0.16 mmol) are dissolved in a mixture of degassed DMF (6 mL) and water (1 mL). The reaction mixture is stirred at RT for 16 h. The mixture is filtered through a Whatman 0.45 $\mu$M glass microfiber filter, concentrated under reduced pressure and purified by FC (Hept/EtOAc) to give rac-[1-(4-benzyloxy-phenyl)-1*H*-[1,2,3]triazol-4-yl]-(2-cyclopropyl-imidazo[5,1-*b*]thiazol-3-yl)-methanol. LC-MS (QC): $t_R$ = 0.824; [M+H]+ = 444.4.

**Example 46: rac-[1-(6-Ethoxy-pyridin-3-yl)-1H-[1,2,3]triazol-4-yl]-(2-ethyl-imidazo[5,1-b]thiazol-3-yl)-methanol**

Step 1: Preparation of 5-azido-2-ethoxypyridine

Prepared according to the procedure described for Example 45, step 1 using 2-ethoxypyridine-5-boronic acid.

Step 2: Prepared following the procedure described for Example 45, and using Intermediate 3, rac-1-(2-ethylimidazo[5,1-*b*]thiazol-3-yl)prop-2-yn-1-ol and 5-azido-2-ethoxypyridine. Purification by prepHPLC (basic conditions) to give *rac*-[1-(6-ethoxy-pyridin-3-yl)-1*H*-[1,2,3]triazol-4-yl]-(2-ethyl-imidazo[5,1-*b*]thiazol-3-yl)-methanol. LC-MS (QC): $t_R$ = 0.616; $[M+H]^+$ = 371.4.

**Example 47: rac-(2-Ethyl-imidazo[5,1-b]thiazol-3-yl)-[1-(4-morpholin-4-yl-phenyl)-1H-[1,2,3]triazol-4-yl]-methanol**

Step 1: Preparation of 4-(4-azidophenyl)morpholine

Prepared according to the procedure described for Example 45, step 1 using 4-(morpholino)phenylboronic acid.
Step 2: Prepared following the procedure described for Example 45, and using Intermediate 3, rac-1-(2-ethyl-imidazo[5,1-*b*]thiazol-3-yl)prop-2-yn-1-ol and 4-(4-azidophenyl)morpholine. Purification by prepHPLC (basic conditions) to give *rac*-(2-ethyl-imidazo[5,1-*b*]thiazol-3-yl)-[1-(4-morpholin4-yl-phenyl)-1*H*-[1,2,3]triazol-4-yl]-methanol. LC-MS (QC): $t_R$ = 0.592; $[M+H]^+$ = 411.4.

**Example 48: *rac*-(2-Ethyl-imidazo[5,1-*b*]thiazol-3-yl)-{1-[4-(1-hydroxymethyl-cyclopropyl)-phenyl]-1*H*-[1,2,3]triazol-4-yl}-methanol**

Step 1: Preparation of (1-(4-azidophenyl)cyclopropyl)methanol

Prepared according to the procedure described for Example 45, step 1 using 4-(1 (hydroxymethyl)cyclopropyl)phenylboronic acid.
Step 2: Prepared following the procedure described for Example 45, and using Intermediate 3, *rac-1-(2*-ethyl-imidazo[5,1-*b*]thiazol-3-yl)prop-2-yn-1-ol and (1-(4-azidophenyl)cyclopropyl)methanol. Purification by prepHPLC (basic conditions) to give *rac*-(2-ethyl-imidazo[5,1-*b*]thiazol-3-yl)-{1-[4-(1-hydroxymethyl-cyclopropyl)-phenyl]-1*H*-[1,2,3]triazol-4-yl}-methanol. LC-MS (QC): $t_R$ = 0.584; $[M+H]^+$ = 396.4.

**Example 49: *rac*-(4-{4-[(2-Ethyl-imidazo[5,1-*b*]thiazol-3-yl)-hydroxy-methyl]-[1,2,3]triazol-1-yl}-phenyl)-carbamic acid methyl ester**

Step 1: Preparation of methyl (4-azidophenyl)carbamate

Prepared according to the procedure described for Example 45, step 1 using 4-methoxycarbonylaminophenylboronic acid.
Step 2: Prepared following the procedure described for Example 45, and using Intermediate 3, *rac-1-(2*-ethyl-imidazo[5,1-*b*]thiazol-3-yl)prop-2-yn-1-ol and methyl (4-azidophenyl)carbamate. Purification by prepHPLC (basic conditions) to give *rac*-(4-{4-[(2-ethyl-imidazo[5,1-*b*]thiazol-3-yl)-hydroxy-methyl]-[1,2,3]triazol-1-yl}-phenyl)-carbamic acid methyl ester. LC-MS (QC): $t_R$ = 0.556; $[M+H]^+$ = 399.4.

**Example 50: *rac*-(2-Ethyl-imidazo[5,1-*b*]thiazol-3-yl)-[1-(1*H*-indol-5-yl)-1*H*-[1,2,3]triazol-4-yl]-methanol**

Step 1: Preparation of 5-azido-1*H*-indole

Prepared according to the procedure described for Example 45, step 1 using 1*H*-indole-5-boronic acid.
Step 2: Prepared following the procedure described for Example 45, and using Intermediate 3, *rac-1-(2*-ethyl-imidazo[5,1-*b*]thiazol-3-yl)prop-2-yn-1-ol and 5-azido-1H-indole. Purification by prepHPLC (acidic conditions) to give *rac*-(2-ethyl-imidazo[5,1-*b*]thiazol-3-yl)-[1-(1*H*-indol-5-yl)-1*H*-[1,2,3]triazol-4-yl]-methanol. LC-MS (QC): $t_R$ = 0.555; $[M+H]^+$ = 365.2.

**Example 51: *rac*-3-{4-[(2-Ethyl-imidazo[5,1-*b*]thiazol-3-yl)-hydroxy-methyl]-[1,2,3]triazol-1-yl}-benzonitrile**

Step 1: Preparation of 3-azidobenzonitrile

Prepared according to the procedure described for Example 45, step 1 using 3-cyanophenylboronic acid.
Step 2: Prepared following the procedure described for Example 45, and using Intermediate 3, *rac-1-(2*-ethyl-imidazo[5,1-*b*]thiazol-3-yl)prop-2-yn-1-ol and 3-azidobenzonitrile. Purification by prepHPLC (basic conditions) to give rac-3-{4-[(2-ethyl-imidazo[5,1-b]thiazol-3-yl)-hydroxy-methyl]-[1,2,3]triazol-1-yl}-benzonitrile. LC-MS (QC): $t_R$ = 0.556; $[M+H]^+$ = 351.4.

**Example 52: rac-(2-Ethyl-imidazo[5,1-b]thiazol-3-yl)-(1-isoquinolin-4-yl-1H-[1,2,3]triazol-4-yl)-methanol**

Step 1: Preparation of 4-azidoisoquinoline

Prepared according to the procedure described for Example 45, step 1 using isoquinoline-4-boronic acid.

Step 2: Prepared following the procedure described for Example 45, and using Intermediate 3, *rac-1-(2*-ethyl-imidazo[5,1-*b*]thiazol-3-yl)prop-2-yn-1-ol and 4-azidoisoquinoline. Purification by prepHPLC (basic conditions) to give *rac*-(2-ethyl-imidazo[5,1-*b*]thiazol-3-yl)-(1-isoquinolin-4-yl-1*H*-[1,2,3]triazol-4-yl)-methanol. LC-MS (QC): $t_R$ = 0.556; $[M+H]^+$ = 377.3.

**Example 53: rac-(1-Benzyl-1H-[1,2,3]triazol-4-yl)-(2-ethyl-imidazo[5,1-b]thiazol-3-yl)-methanol**

Prepared following the procedure described for Example 45, step 2 and using Intermediate 3, *rac-1-(2*-ethyl-imidazo[5,1-*b*]thiazol-3-yl)prop-2-yn-1-ol and (azidomethyl)benzene. Purification by prepHPLC (basic conditions) to give *rac*-(1-benzyl-1*H*-[1,2,3]triazol-4-yl)-(2-ethyl-imidazo[5,1-*b*]thiazol-3-yl)-methanol. LC-MS (QC): $t_R$ = 0.556; $[M+H]^+$ = 340.3.

**Example 54: rac-[1-(4-Cyclopropylmethoxy-phenyl)-1H-[1,2,3]triazol-4-yl]-(2-ethyl-imidazo[5,1-b]thiazol-3-yl)-methanol**

Step 1: Preparation of 1-azido-4-(cyclopropylmethoxy)benzene

Prepared according to the procedure described for Example 45, step 1 using 4-(cyclopropylmethoxy)phenylboronic acid.

Step 2: Prepared following the procedure described for Example 45, and using Intermediate 3, *rac-1-(2*-ethyl-imidazo[5,1-*b*]thiazol-3-yl)prop-2-yn-1-ol and 1-azido-4-(cyclopropylmethoxy)benzen. Purification by prepHPLC (basic conditions) to give *rac*-[1-(4-cyclopropylmethoxy-phenyl)-1*H*-[1,2,3]triazol-4-yl]-(2-ethyl-imidazo[5,1-*b*]thiazol-3-yl)-methanol. LC-MS (QC): $t_R$ = 0.728; $[M+H]^+$ = 396.4.

**Example 55: rac-[1-(6-Ethoxy-pyridin-3-yl)-1H-[1,2,3]triazol-4-yl]-(2-methyl-imidazo[5,1-b]thiazol-3-yl)-methanol**

Step 1: Preparation of 5-azido-2-ethoxypyridine

Prepared according to the procedure described for Example 45, step 1 using 2-ethoxypyridine-5-boronic acid.

Step 2: Prepared following the procedure described for Example 45, and using Intermediate 2, *rac-1-(2*-methylimidazo[5,1-*b*]thiazol-3-yl)prop-2-yn-1-ol and 5-azido-2-ethoxypyridine. Purification by prepHPLC (basic conditions) to give *rac*-[1-(6-ethoxy-pyridin-3-yl)-1*H*-[1,2,3]triazol-4-yl]-(2-methyl-imidazo[5,1-*b*]thiazol-3-yl)-methanol. LC-MS (QC): $t_R$ = 0.887; $[M+H]^+$ = 357.

**Example 56: rac-(2-Methyl-imidazo[5,1-b]thiazol-3-yl)-[1-(4-morpholin-4-yl-phenyl)-1H-[1,2,3]triazol-4-yl]-methanol**

Step 1: Preparation of 4-(4-azidophenyl)morpholine

Prepared according to the procedure described for Example 45, step 1 using 4-(morpholino)phenylboronic acid.

Step 2: Prepared following the procedure described for Example 45, and using Intermediate 2, *rac-1-(2*-methylimidazo[5,1-*b*]thiazol-3-yl)prop-2-yn-1-ol and 4-(4-azidophenyl)morpholine. Purification by prepHPLC (basic conditions) to give *rac*-(2-methyl-imidazo[5,1-*b*]thiazol-3-yl)-[1-(4-morpholin-4-yl-phenyl)-1*H*-[1,2,3]triazol-4-yl]-methanol. LC-MS (QC): $t_R$ = 0.872; $[M+H]^+$ = 397.4.

**Example 57: *rac*-{1-[4-(1-Hydroxymethyl-cyclopropyl)-phenyl]-1*H*-[1,2,3]triazol-4-yl}-(2-methyl-imidazo[5,1-*b*]thiazol-3-yl)-methanol**

Step 1: Preparation of (1-(4-azidophenyl)cyclopropyl)methanol

Prepared according to the procedure described for Example 45, step 1 using 4-(1-(hydroxymethyl)cyclopropyl)phenylboronic acid.

Step 2: Prepared following the procedure described for Example 45, and using Intermediate 2, *rac-1-(2*-methylimidazo[5,1-*b*]thiazol-3-yl)prop-2-yn-1-ol and (1-(4-azidophenyl)cyclopropyl)methanol. Purification by prepHPLC (basic conditions) to give *rac*-{1-[4-(1-hydroxymethyl-cyclopropyl)-phenyl]-1*H*-[1,2,3]triazol-4-yl}-(2-methyl-imidazo[5,1-*b*]thiazol-3-yl)-methanol. LC-MS (QC): $t_R$ = 0.872; $[M+H]^+$ = 382.4.

**Example 58: *rac*-(4-{4-[Hydroxy-(2-methyl-imidazo[5,1-*b*]thiazol-3-yl)-methyl]-[1,2,3]triazol-1-yl}-phenyl)-carbamic acid methyl ester**

Step 1: Preparation of methyl (4-azidophenyl)carbamate

Prepared according to the procedure described for Example 45, step 1 using 4-methoxycarbonylaminophenylboronic acid.

Step 2: Prepared following the procedure described for Example 45, and using Intermediate 2, *rac-1-(2*-methylimidazo[5,1-*b*]thiazol-3-yl)prop-2-yn-1-ol and methyl (4-azidophenyl)carbamate. Purification by prepHPLC (basic conditions) to give *rac*-(4-{4-[hydroxy-(2-methyl-imidazo[5,1-*b*]thiazol-3-yl)-methyl]-[1,2,3]triazol-1-yl}-phenyl)-carbamic acid methyl ester. LC-MS (QC): $t_R$ = 0.818; $[M+H]^+$ = 385.3.

**Example 59: *rac*-[1-(1*H*-Indol-5-yl)-1*H*-[1,2,3]triazol-4-yl]-(2-methyl-imidazo[5,1-*b*)]thiazol-3-yl)-methanol**

Step 1: Preparation of 5-azido-1*H*-indole

Prepared according to the procedure described for Example 45, step 1 using 1*H*-indole-5-boronic acid.

Step 2: Prepared following the procedure described for Example 45, and using Intermediate 2, *rac-1-(2*-methylimidazo[5,1-*b*]thiazol-3-yl)prop-2-yn-1-ol and 5-azido-1H-indole. Purification by prepHPLC (basic conditions) to give *rac*-[1-(1*H*-indol-5-yl)-1*H*-[1,2,3]triazol-4-yl]-(2-methyl-imidazo[5,1-*b*]thiazol-3-yl)-methanol. LC-MS (QC): $t_R$ = 0.821; $[M+H]^+$ = 351.3.

**Example 60: *rac*-3-{4-[Hydroxy-(2-methyl-imidazo[5,1-*b*]thiazol-3-yl)-methyl]-[1,2,3]triazol-1-yl}-benzonitrile**

Step 1: Preparation of 3-azidobenzonitrile

Prepared according to the procedure described for Example 45, step 1 using 3-cyanophenylboronic acid.

Step 2: Prepared following the procedure described for Example 45, and using Intermediate 2, *rac*-1-(2-methylimidazo[5,1-*b*]thiazol-3-yl)prop-2-yn-1-ol and 3-azidobenzonitrile. Purification by prepHPLC (basic conditions) to give *rac*-3-{4-[hydroxy-(2-methyl-imidazo[5,1-*b*]thiazol-3-yl)-methyl]-[1,2,3]triazol-1-yl}-benzonitrile. LC-MS (QC): $t_R$ = 0.807; $[M+H]^+$ = 337.3.

**Example 61: *rac*-(1-Isoquinolin-4-yl-1*H*-[1,2,3]triazol-4-yl)-(2-methyl-imidazo[5,1-*b*]thiazol-3-yl)-methanol**

Step 1: Preparation of 4-azidoisoquinoline

Prepared according to the procedure described for Example 45, step 1 using isoquinoline-4-boronic acid.Step 2: Prepared following the procedure described for Example 45, and using Intermediate 2, *rac*-1-(2-methylimidazo[5,1-*b*]thiazol-3-yl)prop-2-yn-1-ol and 4-azidoisoquinoline. Purification by prepHPLC (acidic conditions) to give *rac*-(1-isoquinolin-4-yl-1*H*-[1,2,3]triazol-4-yl)-(2-methyl-imidazo[5,1-*b*]thiazol-3-yl)-methanol. LC-MS (QC): $t_R$ = 0.492; $[M+H]^+$ = 363.4.

**Example 62: rac-(1-Benzyl-1H-[1,2,3]triazol-4-yl)-(2-methyl-imidazo[5,1-b]thiazol-3-yl)-methanol**

Prepared following the procedure described for Example 45, step 2 and using Intermediate 2, *rac-1-(2*-methylimidazo[5,1-*b*]thiazol-3-yl)prop-2-yn-1-ol and (azidomethyl)benzene. Purification by prepHPLC (basic conditions) to give *rac*-(1-benzyl-1*H*-[1,2,3]triazol-4-yl)-(2-methyl-imidazo[5,1-*b*]thiazol-3-yl)-methanol. LC-MS (QC): $t_R$ = 0.488; $[M+H]^+$ = 326.2.

**Example 63: *rac*-[1-(4-Cyclopropylmethoxy-phenyl)-1*H*-[1,2,3]triazol-4-yl]-(2-methyl-imidazo[5,1-*b*]thiazol-3-yl)-methanol**

Step 1: Preparation of 1-azido-4-(cyclopropylmethoxy)benzene

Prepared according to the procedure described for Example 45, step 1 using 4-(cyclopropylmethoxy)phenylboronic acid.

Step 2: Prepared following the procedure described for Example 45, and using Intermediate 2, *rac-1-(2*-methylimidazo[5,1-*b*]thiazol-3-yl)prop-2-yn-1-ol and 1-azido-4-(cyclopropylmethoxy)benzene. Purification by prepHPLC (basic conditions) to give *rac*-[1-(4-cyclopropylmethoxy-phenyl)-1*H*-[1,2,3]triazol-4-yl]-(2-methyl-imidazo[5,1-*b*]thiazol-3-yl)-methanol. LC-MS (QC): $t_R$ = 0.668; $[M+H]^+$ = 382.4.

**Example 64: *rac*-[2-(3-Fluoro-phenyl)-imidazo[5,1-*b*]thiazol-3-yl]-[1-(4-methoxy-phenyl)-1*H*-[1,2,3]triazol-4-yl]-methanol**

Prepared following the procedure described for Example 45, step 2 and using Intermediate 6, *rac*-1-(2-(3-fluorophenyl)imidazo[5,1-*b*]thiazol-3-yl)prop-2-yn-1-ol and 4-azidoanisole. Purification by prepHPLC (basic conditions) to give *rac*-[2-(3-fluoro-phenyl)-imidazo[5,1-*b*]thiazol-3-yl]-[1-(4-methoxy-phenyl)-1*H*-[1,2,3]triazol-4-yl]-methanol. LC-MS (QC): $t_R$ = 0.804; $[M+H]^+$ = 422.3.

**Example 65: *rac*-(2-Cyclopropyl-imidazo[5,1-*b*]thiazol-3-yl)-[1-(2-ethoxy-pyrimidin-5-yl)-1*H*-[1,2,3]triazol-4-yl]-methanol**

Step 1: Preparation of 5-azido-2-ethoxypyrimidine

Prepared according to the procedure described for Example 45, step 1 using 2-ethoxypyrimidine-5-boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-2-ethoxypyrimidine. Purification by FC (Silicagel; Hept / EtOAc) to give *rac*-(2-cyclopropyl-imidazo[5,1-*b*]thiazol-3-yl)-[1-(2-ethoxy-pyrimidin-5-yl)-1*H*-[1,2,3]triazol-4-yl]-methanol. LC-MS (QC): $t_R$ = 0.914; $[M+H]^+$ = 384.4.

**Example 66: *rac*-(2-Cyclopropyl-imidazo[5,1-*b*]thiazol-3-yl)-[1-(5,6-dimethoxy-pyridin-2-yl)-1*H*-[1,2,3]triazol-4-yl]-methanol**

Step 1: Preparation of 6-azido-2,3-dimethoxypyridine

Prepared according to the procedure described for Example 45, step 1 using 2,3-dimethoxy-6-(4,4,5,5-tetramethyl-1,3,2-dioxaborolan-2-yl)pyridine.

Step 2: Prepared according to the procedure described for Example 45 and using intermediate 1, *rac-1-(2*-cyclopropylimidazo[5,1-*b*)]thiazol-3-yl)prop-2-yn-1-ol and 6-azido-2,3-dimethoxypyridine. Purification by FC (Silicagel; Hept / EtOAc) to give *rac*-(2-cyclopropyl-imidazo[5,1-*b*]thiazol-3-yl)-[1-(5,6-dimethoxy-pyridin-2-yl)-1*H*-[1,2,3]triazol-4-yl]-methanol. LC-MS (QC): $t_R$ = 0.62; $[M+H]^+$ = 399.3.

**Example 67: *rac*-(2-Cyclopropyl-imidazo[5,1-*b*]thiazol-3-yl)-[1-(2-methoxy-pyrimidin-5-yl)-1*H*-[1,2,3]triazol-4-yl]-methanol**

Step 1: Preparation of 5-azido-2-methoxypyrimidine

Prepared according to the procedure described for Example 45, step 1 using 2-methoxypyrimidine-5-boronic acid.

Step 2: Prepared according to the procedure described for Example 45 and using intermediate 1, *rac-1-(2*-cyclopropylimidazo[5,1-*b*)]thiazol-3-yl)prop-2-yn-1-ol and 5-azido-2-methoxypyrimidine. Purification by FC (Silicagel; Hept / EtOAc) to give *rac*-(2-cyclopropyl-imidazo[5,1-*b*]thiazol-3-yl)-[1-(2-methoxy-pyrimidin-5-yl)-1*H*-[1,2,3]triazol-4-yl]-methanol. LC-MS (QC): $t_R$ = 0.831; $[M+H]^+$ = 370.4.

**Example 68: *rac*-4-{4-[(2-Cyclopropyl-imidazo[5,1-*b*]thiazol-3-yl)-hydroxy-methyl]-[1,2,3]triazol-1-yl)-phenol**

To an ice-cold solution of the product from Example 45, *rac*-[1-(4-(benzyloxy)phenyl)-1*H*-[1,2,3]triazol-4-yl]-(2-cyclopropylimidazo[5,1-*b*]thiazol-3-yl)methanol (400 mg, 0.902 mmol) in DCM (15 ml) is added a boron trichloride solution 1 M in DCM (9.02 ml, 9.02 mmol). The mixture is stirred for 15 min at 0°C, then at RT for 2 h. The reaction mixture is filtered through a Whatman 0.45 μm glass microfiber filter and purified by prepHPLC (basic conditions) to give rac-4-{4-[(2-cyclopropyl-imidazo[5,1-*b*]thiazol-3-yl)-hydroxy-methyl]-[1,2,3]triazol-1-yl}-phenol. LC-MS (QC): $t_R$ = 0.495; $[M+H]^+$ = 354.3.

**Example 69: rac-(2-Cyclopropyl-imidazo[5,1-b]thiazol-3-yl)-[1-(6-methoxy-pyridin-3-yl)-1H-[1,2,3]triazol-4-yl]-methanol**

Step 1: Preparation of 5-azido-2-methoxypyridine

Prepared according to the procedure described for Example 45, step 1 using 2-methoxypyridine-5-boronic acid. Step 2: Prepared according to the procedure described for Example 45 and using intermediate 1, rac-1-(2-cyclopropylimidazo[5,1-*b*)]thiazol-3-yl)prop-2-yn-1-ol and 5-azido-2-methoxypyridine. Purification by prepHPLC (basic conditions) to give *rac*-(2-cyclopropyl-imidazo[5,1-*b*]thiazol-3-yl)-[1-(6-methoxy-pyridin-3-yl)-1*H*-[1,2,3]triazol-4-yl]-methanol. LC-MS (QC): $t_R$ = 0.915; $[M+H]^+$ = 369.4.

**Example 70: *rac*-5-{4-[(2-Cyclopropyl-imidazo[5,1-*b*]thiazol-3-yl)-hydroxy-methyl]-[1,2,3]triazol-1-yl)-2-methoxy-nicotinic acid methyl ester**

Step 1: Preparation of methyl 5-azido-2-methoxynicotinate

Prepared according to the procedure described for Example 45, step 1 using methyl 2-methoxy-5-(4,4,5,5-tetramethyl-1,3,2-dioxaborolan-2-yl)nicotinate.

Step 2: Prepared according to the procedure described for Example 45 and using intermediate 1, *rac*-1-(2-cyclopropylimidazo[5,1-*b*)]thiazol-3-yl)prop-2-yn-1-ol and methyl 5-azido-2-methoxynicotinate. Purification by prepHPLC (basic conditions) to give *rac*-5-{4-[(2-cyclopropyl-imidazo[5,1-*b*]thiazol-3-yl)-hydroxy-methyl]-[1,2,3]triazol-1-yl}-2-methoxy-nicotinic acid methyl ester. LC-MS (QC): $t_R$ = 0.968; $[M+H]^+$ = 427.3.

**Example 71: *rac*-(2-Cyclopropyl-imidazo[5,1-*b*]thiazol-3-yl)-[1-(6-methoxy-5-methyl-pyridin-3-yl)-1*H*-[1,2,3]triazol-4-yl]-methanol**

Step 1: Preparation of 5-azido-2-methoxy-3-methylpyridine

Prepared according to the procedure described for Example 45, step 1 using 2-methoxy-3-methylpyridine-5-boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-2-methoxy-3-methylpyridine. Purification by prepHPLC (basic conditions) to give *rac*-(2-cyclopropyl-imidazo[5,1-*b*]thiazol-3-yl)-[1-(6-methoxy-5-methyl-pyridin-3-yl)-1*H*-[1,2,3]triazol-4-yl]-methanol. LC-MS (QC): $t_R$ = 0.652; $[M+H]^+$ = 383.4

**Example 72: *rac*-(2-Cyclopropyl-imidazo[5,1-*b*]thiazol-3-yl)-[1-(3,4-dihydro-2*H*-pyrano[2,3-b]pyridin-6-yl)-1*H*-[1,2,3]triazol-4-yl]-methanol**

Step 1: Preparation of 6-azido-3,4-dihydro-2*H*-pyrano[2,3-*b*]pyridine

Prepared according to the procedure described for Example 45, step 1 using 6-(4,4,5,5-tetramethyl-1,3,2-dioxaborolan-2-yl)-3,4-dihydro-2*H*-pyrano[2,3-*b*]pyridine.

Step 2: Prepared according to the procedure described for Example 45 and using intermediate 1, *rac-1-(2-*cyclopropylimidazo[5,1-*b*)]thiazol-3-yl)prop-2-yn-1-ol and 6-azido-3,4-dihydro-2H-pyrano[2,3-b]pyridine. Purification by prepHPLC (basic conditions) to give *rac*-(2-cyclopropyl-imidazo[5,1-*b*]thiazol-3-yl)-[1-(3,4-dihydro-2*H*-pyrano[2,3-b]pyridin-6-yl)-1*H*-[1,2,3]triazol-4-yl]-methanol. LC-MS (QC): $t_R$ = 0.544; $[M+H]^+$ = 395.4

**Example 73: *rac*-[1-(5-Chloro-6-isopropoxy-pyridin-3-yl)-1*H*-[1,2,3]triazol-4-yl]-(2-cyclopropyl-imidazo[5,1-*b*]thiazol-3-yl)-methanol**

Step 1: Preparation of 5-azido-3-chloro-2-isopropoxypyridine

Prepared according to the procedure described for Example 45, step 1 using 5-chloro-6-isopropoxypyridine-3-boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-3-chloro-2-isopropoxypyridine. Purification by prepHPLC (basic conditions) to give *rac*-[1-(5-chloro-6-isopropoxy-pyridin-3-yl)-1*H*-[1,2,3]triazol-4-yl]-(2-cyclopropyl-imidazo[5,1-*b*]thiazol-3-yl)-methanol. LC-MS (QC): $t_R$ = 0.82; $[M+H]^+$ = 431.3.

**Example 74: *rac*-[1-(5-Chloro-6-ethoxy-pyridin-3-yl)-1*H*-[1,2,3]triazol-4-yl]-(2-cyclopropyl-imidazo[5,1-*b*]thiazol-3-yl)-methanol**

Step 1: Preparation of 5-azido-3-chloro-2-ethoxypyridine

Prepared according to the procedure described for Example 45, step 1 using 5-chloro-6-ethoxypyridine-3-boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-3-chloro-2-ethoxypyridine. Purification by prepHPLC (basic conditions) to give *rac*-[1-(5-chloro-6-ethoxy-pyridin-3-yl)-1*H*-[1,2,3]triazol-4-yl]-(2-cyclopropyl-imidazo[5,1-*b*]thiazol-3-yl)-methanol. LC-MS (QC): $t_R$ = 0.752; $[M+H]^+$ = 417.3.

**Example 75: *rac*-(5-{4-[(2-Cyclopropyl-imidazo[5,1-*b*]thiazol-3-yl)-hydroxy-methyl]-[1,2,3]triazol-1-yl}-pyridin-2-yloxy)-acetic acid methyl ester**

Step 1: Preparation of methyl 2-((5-azidopyridin-2-yl)oxy)acetate

Prepared according to the procedure described for Example 45, step 1 using methyl 2-((5-(4,4,5,5-tetramethyl-1,3,2-dioxaborolan-2-yl)pyridin-2-yl)oxy)acetate.

Step 2: Prepared according to the procedure described for Example 45 and using intermediate 1, *rac*-1-(2-cyclopropylimidazo[5,1-*b*)]thiazol-3-yl)prop-2-yn-1-ol and 2-((5-azidopyridin-2-yl)oxy)acetate. Purification by prepHPLC (basic conditions) to give *ra*c-(5-{4-[(2-cyclopropyl-imidazo[5,1-*b*]thiazol-3-yl)-hydroxy-methyl]-[1,2,3]triazol-1-yl}-pyridin-2-yloxy)-acetic acid methyl ester. LC-MS (QC): $t_R$ = 0.952; $[M+H]^+$ = 427.3.

**Example 76: *rac*-(2-Cyclopropyl-imidazo[5,1-*b*]thiazol-3-yl)-[1-(6-ethoxy-5-trifluoromethyl-pyridin-3-yl)-1*H*-[1,2,3]triazol-4-yl]-methanol**

Step 1: Preparation of 5-azido-2-ethoxy-3-(trifluoromethyl)pyridine

Prepared according to the procedure described for Example 45, step 1 using 2-ethoxy-5-(4,4,5,5-tetramethyl-1,3,2-dioxaborolan-2-yl)-3-(trifluoromethyl)pyridine.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-2-ethoxy-3-(trifluoromethyl)pyridin. Purification by prepHPLC (basic conditions) to give *rac*-(2-cyclopropyl-imidazo[5,1-*b*]thiazol-3-yl)-[1-(6-ethoxy-5-trifluoromethyl-pyridin-3-yl)-1*H*-[1,2,3]triazol-4-yl]-methanol. LC-MS (QC): $t_R$ = 0.812; $[M+H]^+$ = 451.3.

**Example 77: rac-[1-(6-Benzyloxy-pyridin-3-yl)-1H-[1,2,3]triazol-4-yl]-(2-cyclopropyl-imidazo[5,1-b]thiazol-3-yl)-methanol**

Step 1: Preparation of 5-azido-2-(benzyloxy)pyridine

Prepared according to the procedure described for Example 45, step 1 using 2-(benzyloxy)-5-(4,4,5,5-tetramethyl-1,3,2-dioxaborolan-2-yl)pyridine.

Step 2: Prepared according to the procedure described for Example 45 and using intermediate 1, rac-1-(2-cyclopropylimidazo[5,1-*b*)]thiazol-3-yl)prop-2-yn-1-ol and 5-azido-2-(benzyloxy)pyridine. Purification by prepHPLC (basic conditions) to give rac-[1-(6-benzyloxy-pyridin-3-yl)-1*H*-[1,2,3]triazol-4-yl]-(2-cyclopropyl-imidazo[5,1-*b*]thiazol-3-yl)-methanol. LC-MS (QC): $t_R$ = 0.796; $[M+H]^+$ = 445.4.

**Example 78: *rac*-(2-Cyclopropyl-imidazo[5,1-*b*]thiazol-3-yl)-{1-[6-(2,2,2-trifluoro-ethoxy)-pyridin-3-yl]-1*H*-[1,2,3]triazol-4-yl}-methanol**

Step 1: Preparation of 5-azido-2-(2,2,2-trifluoroethoxy)pyridine

Prepared according to the procedure described for Example 45, step 1 using (6-(2,2,2-trifluoroethoxy)pyridin-3-yl)boronic acid.

Step 2: Prepared according to the procedure described for Example 45 and using intermediate 1, rac-1-(2-cyclopropylimidazo[5,1-*b*)]thiazol-3-yl)prop-2-yn-1-ol and 5-azido-2-(2,2,2-trifluoroethoxy)pyridine. Purification by prepHPLC (basic conditions) to give *rac*-(2-cyclopropyl-imidazo[5,1-*b*]thiazol-3-yl)-{1-[6-(2,2,2-trifluoro-ethoxy)-pyridin-3-yl]-1*H*-[1,2,3]triazol-4-yl}-methanol. LC-MS (QC): $t_R$ = 0.732; $[M+H]^+$ = 437.2.

**Example 79: *rac*-(2-Cyclopropyl-imidazo[5,1-*b*]thiazol-3-yl)-[1-(5-fluoro-6-methoxy-pyridin-3-yl)-1*H*-[1,2,3]triazol-4-yl]-methanol**

Step 1: Preparation of 5-azido-3-fluoro-2-methoxypyridine

Prepared according to the procedure described for Example 45, step 1 using (5-fluoro-6-methoxypyridin-3-yl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-3-fluoro-2-methoxypyridine. Purification by prepHPLC (basic conditions) to give *rac*-(2-cyclopropyl-imidazo[5,1-*b*)]thiazol-3-yl)-[1-(5-fluoro-6-methoxy-pyridin-3-yl)-1*H*-[1,2,3]triazol-4-yl]-methanol. LC-MS (QC): $t_R$ = 0.628; $[M+H]^+$ = 387.3.

**Example 80: *rac*-(2-Cyclopropyl-imidazo[5,1-*b*]thiazol-3-yl)-[1-(5,6-dimethoxy-pyridin-3-yl)-1*H*-[1,2,3]triazol-4-yl]-methanol**

Step 1: Preparation of 5-azido-2,3-dimethoxypyridine

Prepared according to the procedure described for Example 45, step 1 using 2,3-dimethoxy-5-(4,4,5,5-tetramethyl-1,3,2-dioxaborolan-2-yl)pyridine.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-2,3-dimethoxypyridine. Purification by prepHPLC (basic conditions) to give *rac*-(2-cyclopropyl-imidazo[5,1-*b*)]thiazol-3-yl)-[1-(5,6-dimethoxy-pyridin-3-yl)-1*H*-[1,2,3]triazol-4-yl]-methanol. LC-MS (QC): $t_R$ = 0.944; $[M+H]^+$ = 399.3.

**Example 81: *rac*-(2-Cyclopropyl-imidazo[5,1-*b*]thiazol-3-yl)-[1-(2,6-dimethoxy-pyridin-3-yl)-1*H*-[1,2,3]triazol-4-yl]-methanol**

Step 1: Preparation of 3-azido-2,6-dimethoxypyridine

Prepared according to the procedure described for Example 45, step 1 using potassium 2,6-dimethoxypyridine-3-trifluoroborate.

Step 2: Prepared according to the procedure described for Example 45 and using intermediate 1, *rac*-1-(2-cyclopropylimidazo[5,1-*b*)]thiazol-3-yl)prop-2-yn-1-ol and 3-azido-2,6-dimethoxypyridine. Purification by prepHPLC (basic conditions) to give *rac*-(2-cyclopropyl-imidazo[5,1-*b*]thiazol-3-yl)-[1-(2,6-dimethoxy-pyridin-3-yl)-1*H*-[1,2,3]triazol-4-yl]-methanol. LC-MS (QC): $t_R$ = 0.66; $[M+H]^+$ = 399.4.

**Example 82: *rac*-(2-Cyclopropyl-imidazo[5,1-*b*]thiazol-3-yl)-[1-(6-isopropoxy-pyridin-3-yl)-1*H*-[1,2,3]triazol-4-yl]-methanol**

Step 1: Preparation of 5-azido-2-isopropoxypyridine

Prepared according to the procedure described for Example 45, step 1 using (6-isopropoxypyridin-3-yl)boronic acid.

Step 2: Prepared according to the procedure described for Example 45 and using intermediate 1, *rac*-1-(2-

cyclopropylimidazo[5,1-*b*)]thiazol-3-yl)prop-2-yn-1-ol and 5-azido-2-isopropoxypyridine. Purification by prepHPLC (basic conditions) to give *rac*-(2-cyclopropyl-imidazo[5,1-*b*]thiazol-3-yl)-[1-(6-isopropoxy-pyridin-3-yl)-1*H*-[1,2,3]triazol-4-yl]-methanol. LC-MS (QC): $t_R$ = 0.704; $[M+H]^+$ = 397.4.

**Example 83: rac-[1-(2-Cyclobutoxy-pyrimidin-5-yl)-1H-[1,2,3]triazol-4-yl]-(2-cyclopropyl-imidazo[5,1-*b*]thiazol-3-yl)-methanol**

Step 1: Preparation of 5-azido-2-cyclobutoxypyrimidine

Prepared according to the procedure described for Example 45, step 1 using (2-cyclobutoxypyrimidin-5-yl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-2-cyclobutoxypyrimidine. Purification by prepHPLC (basic conditions) to give *rac*-[1-(2-cyclobutoxy-pyrimidin-5-yl)-1*H*-[1,2,3]triazol-4-yl]-(2-cyclopropyl-imidazo[5,1-*b*]thiazol-3-yl)-methanol. LC-MS (QC): $t_R$ = 0.668; $[M+H]^+$ = 410.4.

**Example 84: *rac*-(2-Cyclopropyl-imidazo[5,1-*b*]thiazol-3-yl)-[1-(2,4-dimethoxy-pyrimidin-5-yl)-1*H*-[1,2,3]triazol-4-yl]-methanol**

Step 1: Preparation of 5-azido-2,4-dimethoxypyrimidine

Prepared according to the procedure described for Example 45, step 1 using (2,4-dimethoxypyrimidin-5-yl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-2,4-dimethoxypyrimidine. Purification by prepHPLC (basic conditions) to give *rac*-(2-cyclopropyl-imidazo[5,1-*b*]thiazol-3-yl)-[1-(2,4-dimethoxy-pyrimidin-5-yl)-1*H*-[1,2,3]triazol-4-yl]-methanol. LC-MS (QC): $t_R$ = 0.908; $[M+H]^+$ = 400.4.

**Example 85: *rac*-[1-(2-Benzyloxy-pyrimidin-5-yl)-1*H*-[1,2,3]triazol-4-yl]-(2-cyclopropyl-imidazo[5,1-*b*]thiazol-3-yl)-methanol**

Step 1: Preparation of 5-azido-2-(benzyloxy)pyrimidine

Prepared according to the procedure described for Example 45, step 1 using (2-(benzyloxy)pyrimidin-5-yl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 5-azido-2-(benzyloxy)pyrimidine. Purification by prepHPLC (basic conditions) to give *rac*-[1-(2-benzyloxy-pyrimidin-5-yl)-1*H*-[1,2,3]triazol-4-yl]-(2-cyclopropyl-imidazo[5,1-*b*]thiazol-3-yl)-methanol. LC-MS (QC): $t_R$ = 0.728; $[M+H]^+$ = 446.3.

**Example 86: *rac*-(2-Cyclopropyl-imidazo[5,1-*b*]thiazol-3-yl)-[1-(3,6-dimethoxy-pyridazin-4-yl)-1*H*-[1,2,3]triazol-4-yl]-methanol**

Step 1: Preparation of 4-azido-3,6-dimethoxypyridazine

Prepared according to the procedure described for Example 45, step 1 using (3,6-dimethoxypyridazin-4-yl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 4-azido-3,6-dimethoxypyridazine. Purification by prepHPLC (basic conditions) to give *rac*-(2-cyclopropyl-imidazo[5,1-*b*]thiazol-3-yl)-[1-(3,6-dimethoxy-pyridazin-4-yl)-1*H*-[1,2,3]triazol-4-yl]-methanol. LC-MS (QC): $t_R$ = 0.948; $[M+H]^+$ = 400.4.

**Example 87: *rac*-(2-Cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol**

A mixture of the product from Example 68, *rac*-4-{4-[(2-cyclopropyl-imidazo[5,1-*b*)]thiazol-3-yl)-hydroxy-methyl]-[1,2,3]triazol-1-yl}-phenol (14.1 mg, 0.04 mmol), 4-(bromomethyl)tetrahydro-2*H*-pyran (29.8 mg, 0.16 mmol), sodium iodide (0.3 mg, 0.05 eq.) and $K_2CO_3$ (22.1 mg, 0.16 mmol) in DMF (2 ml) is stirred at 50°C for 16 h. The mixture is then

filtered and purified by preparative HPLC (basic conditions) to give rac-(2-cyclopropyl-imidazo[5,1-b]thiazol-3-yl)-{1-[4-(tetrahydro-pyran-4-ylmethoxy)-phenyl]-1H-[1,2,3]triazol-4-yl}-methanol. LC-MS (QC): $t_R$ = 0.704; $[M+H]^+$ = 452.3.

**Example 88: *rac*-(2-Cyclopropyl-imidazo[5,1-*b*]thiazol-3-yl)-{1-[4-(oxetan-3-ylmethoxy)-phenyl]-1*H*-[1,2,3]triazol-4-yl}-methanol**

Prepared following the procedure described for Example 87, and using 3-(bromomethyl)oxetane. Purification by preparative HPLC (basic conditions) gives *rac*-(2-cyclopropyl-imidazo[5,1-*b*]thiazol-3-yl)-{1-[4-(oxetan-3-ylmethoxy)-phenyl]-1*H*-[1,2,3]triazol-4-yl}-methanol. LC-MS (QC): $t_R$ = 0.604; $[M+H]^+$ = 424.4.

**Example 89: *rac*-(2-Cyclopropyl-imidazo[5,1-*b*]thiazol-3-yl)-{1-[4-(2-piperidin-1-yl-elhoxy)-phenyl]-1*H*-[1,2,3]triazol-4-yl}-methanol**

Prepared following the procedure described for Example 87, and using 1-(2-chloroethyl)piperidine hydrochloride. Purification by preparative HPLC (basic conditions) gives *rac*-(2-cyclopropyl-imidazo[5,1-*b*]thiazol-3-yl)-{1-[4-(2-piperidin-1-yl-ethoxy)-phenyl]-1*H*-[1,2,3]triazol-4-yl}-methanol. LC-MS (QC): $t_R$ = 0.412; $[M+H]^+$ = 465.4.

**Example 90: *rac*-(2-Cyclopropyl-imidazo[5,1-*b*]thiazol-3-yl)-{1-[4-(tetrahydro-furan-2-ylmethoxy)-phenyl]-1*H*-[1,2,3]triazol-4-yl}-methanol**

Prepared following the procedure described for Example 87, and using tetrahydrofurfuryl bromide. Purification by preparative HPLC (basic conditions) gives *rac*-(2-cyclopropyl-imidazo[5,1-*b*]thiazol-3-yl)-{1-[4-(tetrahydro-furan-2-ylmethoxy)-phenyl]-1*H*-[1,2,3]triazol-4-yl}-methanol. LC-MS (QC): $t_R$ = 0.672; $[M+H]^+$ = 438.4.

**Example 91: *rac*-(2-Cyclopropyl-imidazo[5,1-*b*]thiazol-3-yl)-{1-[4-(2-methoxy-ethoxy)-phenyl]-1*H*-[1,2,3]triazol-4-yl}-methanol**

Prepared following the procedure described for Example 87, and using 1-bromo-2-methoxyethane. Purification by preparative HPLC (basic conditions) gives *rac*-(2-cyclopropyl-imidazo[5,1-*b*]thiazol-3-yl)-{1-[4-(2-methoxy-ethoxy)-phenyl]-1*H*-[1,2,3]triazol-4-yl}-methanol. LC-MS (QC): $t_R$ = 0.62; $[M+H]^+$ = 412.4.

**Example 92: rac-[2-(1-Fluoro-cyclopropyl)-imidazo[5,1-b]thiazol-3-yl]-[1-(4-methoxy-phenyl)-1H-[1,2,3]triazol-4-yl]-methanol**

Prepared following the procedure described for Example 45, step 2 and using Intermediate 7, *rac*-1-(2-(1-fluorocyclopropyl)imidazo[5,1-*b*]thiazol-3-yl)prop-2-yn-1-ol and 4-azidoanisole. Purification by preparative HPLC (basic conditions) to give *rac*-[2-(1-fluoro-cyclopropyl)-imidazo[5,1-*b*]thiazol-3-yl]-[1-(4-methoxy-phenyl)-1*H*-[1,2,3]triazol-4-yl]-methanol. LC-MS (QC): $t_R$ = 0.708; $[M+H]^+$ = 386.3.

**Example 93: [2-(cis-2-Fluoro-cyclopropyl)-imidazo[5,1-*b*]thiazol-3-yl]-[1-(4-methoxy-phenyl)-1*H*-[1,2,3]triazol-4-yl]-methanol**

Prepared following the procedure described for Example 45, step 2 and using Intermediate 8, *cis*-1-[(2-fluoro-cyclopropyl)-imidazo[5,1-*b*]thiazol-3-yl]prop-2-yn-1-ol and 4-azidoanisole. Purification by preparative HPLC (basic conditions) to give [2-(cis-2-fluoro-cyclopropyl)-imidazo[5,1-*b*]thiazol-3-yl]-[1-(4-methoxy-phenyl)-1*H*-[1,2,3]triazol-4-yl]-methanol. LC-MS (QC): $t_R$ = 0.592; $[M+H]^+$ = 386.4.

**Example 94: [2-(trans-2-Fluoro-cyclopropyl)-imidazo[5,1-b]thiazol-3-yl]-[1-(4-methoxy-phenyl)-1H-[1,2,3]triazol-4-yl]-methanol**

Prepared following the procedure described for Example 45, step 2 and using Intermediate 9, *trans*-1-[(2-fluoro-cyclopropyl)-imidazo[5,1-*b*)]thiazol-3-yl]prop-2-yn-1-ol and 4-azidoanisole. Purification by preparative HPLC (basic conditions) to give [2-(trans-2-fluoro-cyclopropyl)-imidazo[5,1-*b*]thiazol-3-yl]-[1-(4-methoxy-phenyl)-1*H*-[1,2,3]triazol-4-yl]-methanol. LC-MS (QC): $t_R$ = 0.628; $[M+H]^+$ = 386.4.

**Example 95: *rac-N*-(4-{4-[(2-Cyclopropyl-imidazo[5,1-*b*]thiazol-3-yl)-hydroxy-methyl]-[1,2,3]triazol-1-yl)-phenyl)-acetamide**

Step 1: Preparation of *N*-(4-azidophenyl)acetamide

Prepared according to the procedure described for Example 45, step 1 using (4-acetamidophenyl)boronic acid. Step 2: Prepared according to the procedure described for Example 45 and using intermediate 1, *rac-1-(2-*cyclopropylimidazo[5,1-*b*)]thiazol-3-yl)prop-2-yn-1-ol and *N*-(4-azidophenyl)acetamide. Purification by preparative HPLC (basic conditions) to give *rac-N*-(4-{4-[(2-cyclopropyl-imidazo[5,1-*b*]thiazol-3-yl)-hydroxy-methyl]-[1,2,3]triazol-1-yl}-phenyl)-acetamide. LC-MS (QC): $t_R$ = 0.504; $[M+H]^+$ = 395.4.

**Example 96: rac-(2-Methyl-imidazo[5,1-b]thiazol-3-yl)-(1-phenyl-1H-pyrazol-4-yl)-methanol**

To a solution of intermediate 11, 3-bromo-2-methylimidazo[5,1-b]thiazole (50 mg, 0.23 mmol) in THF (3 ml) is added n-BuLi (1.6 M in hexanes, 0.719 ml, 1.15 mmol) at -78°C. After stirring at this temperature for 30 min, a solution of 1-phenyl-1*H*-pyrazole-4-carbaldehyde (198 mg, 1.15 mmol) in THF (1 ml) is added dropwise. The mixture is stirred for 2 h at -78°C. Water is added, the layers separated and the aqueous layer extracted with DCM (2x). The combined org extracts are washed with brine, dried ($MgSO_4$), filtered and concentrated under reduced pressure. Purification by preparative HPLC (acidic conditions) followed by neutralization and extraction with DCM to give *rac*-(2-methyl-imidazo[5,1-*b*]thiazol-3-yl)-(1-phenyl-1*H*-pyrazol-4-yl)-methanol. LC-MS (QC): $t_R$ = 0.552; [M+H]+ = 311.2.

**Example 97: *rac*-[1-(4-Methoxy-phenyl)-1*H*-pyrazol-4-yl]-(2-methyl-imidazo[5,1-*b*]thiazol-3-yl)-methanol**

Prepared following the procedure described for Example 96 using intermediate 11, 3-bromo-2-cyclopropylimidazo[5,1-*b*]thiazole and 1-(4-methoxyphenyl)-1*H*-pyrazole-4-carbaldehyde. Purification by preparative HPLC (acidic conditions) followed by neutralization and extraction with DCM to give *rac*-[1-(4-methoxy-phenyl)-1*H*-pyrazol-4-yl]-(2-methyl-imidazo[5,1-*b*]thiazol-3-yl)-methanol. LC-MS (QC): $t_R$ = 0.567; $[M+H]^+$ = 341.4.

**Example 98: *rac*-(2-Methyl-imidazo[5,1-*b*]thiazol-3-yl)-(5-methyl-1-phenyl-1*H*-pyrazol-4-yl)-methanol**

Prepared following the procedure described for Example 96 using intermediate 11, 3-bromo-2-cyclopropylimidazo[5,1-*b*]thiazole and 5-methyl-1-phenyl-1*H*-pyrazole-4-carbaldehyde. Purification by preparative HPLC (acidic conditions) followed by neutralization and extraction with DCM to give *rac*-(2-methyl-imidazo[5,1-*b*]thiazol-3-yl)-(5-methyl-1-phenyl-1*H*-pyrazol-4-yl)-methanol. LC-MS (QC): $t_R$ = 0.547; $[M+H]^+$ = 325.2.

**Example 99: rac-(2-Cyclopropyl-imidazo[5,1-b]thiazol-3-yl)-[1-(4-methoxy-phenyl)-1H-pyrazol-4-yl]-methanol**

Prepared following the procedure described for Example 96 using intermediate 10, 3-bromo-2-cyclopropylimidazo[5,1-*b*]thiazole and 1-(4-methoxyphenyl)-1H-pyrazole-4-carbaldehyde. Purification by preparative HPLC (acidic conditions) followed by neutralization and extraction with DCM to give *rac*-(2-cyclopropyl-imidazo[5,1-*b*]thiazol-3-yl)-[1-(4-methoxy-phenyl)-1*H*-pyrazol-4-yl]-methanol. LC-MS (QC): $t_R$ = 0.66; $[M+H]^+$ = 367.2.

**Example 100: rac-(2-Cyclopropyl-imidazo[5,1-b]thiazol-3-yl)-(1-phenyl-1H-pyrazol-4-yl)-methanol**

Prepared following the procedure described for Example 96 using intermediate 10, 3-bromo-2-cyclopropylimidazo[5,1-*b*]thiazole and 1-phenyl-1*H*-pyrazole-4-carbaldehyde. Purification by preparative HPLC (acidic conditions) followed by neutralization and extraction with DCM to give *rac*-(2-cyclopropyl-imidazo[5,1-*b*]thiazol-3-yl)-(1-phenyl-1*H*-pyrazol-4-yl)-methanol. LC-MS (QC): $t_R$ = 0.65; $[M+H]^+$ = 337.2.

**Example 100a: (*S*)-(2-Cyclopropyl-imidazo[5,1-*b*]thiazol-3-yl)-(1-phenyl-1*H*-pyrazol-4-yl)-methanol**

Separation of the enantiomers on chiral stationary phase:
Method: Column: ChiralPak AD-H 30x250mm, 5μM; Detector Settings: UV-Vis-1; 210 nM; Eluent: 60% $CO_2$ and 40% EtOH; Flow: 160.00 ml/min; BPR: 100 bar; Temperature: 40°C. Injection volume: 1000μl.

14 mg of the racemate are separated by the method described above to give 6 mg of the S-enantiomer Example 100a and 5 mg of the R-enantiomer.

**Example 101: rac-(2-Cyclopropyl-imidazo[5,1-b]thiazol-3-yl)-(5-methyl-1-phenyl-1H-[1,2,3]triazol-4-yl)-methanol**

Prepared following the procedure described for Example 96 using intermediate 10, 3-bromo-2-cyclopropylimidazo[5,1-*b*]thiazole and 5-methyl-1-phenyl-1*H*-1,2,3-triazole-4-carbaldehyde. Purification by preparative HPLC (acidic conditions) followed by neutralization and extraction with DCM to give *rac*-(2-cyclopropyl-imidazo[5,1-*b*]thiazol-3-yl)-(5-methyl-1-phenyl-1*H*-[1,2,3]triazol-4-yl)-methanol. LC-MS (QC): $t_R$ = 0.606; $[M+H]^+$ = 352.4.

**Example 102: *rac*-4-{4-[(2-Cyclopropyl-imidazo[5,1-*b*]thiazol-3-yl)-hydroxy-methyl]-[1,2,3]triazol-1-yl}-*N*-(2-methoxy-ethyl)-benzamide**

Step 1: Preparation of 4-azido-*N*-(2-methoxyethyl)benzamide

Prepared according to the procedure described for Example 45, step 1 using (4-((2-methoxyethyl)carbamoyl)phenyl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 4-azido-*N*-(2-methoxyethyl)benzamide. Purification by preparative HPLC (basic conditions) to give *rac*-4-{4-[(2-cyclopropyl-imidazo[5,1-*b*]thiazol-3-yl)-hydroxymethyl]-[1,2,3]triazol-1-yl}-*N*-(2-methoxy-ethyl)-benzamide. LC-MS (QC): $t_R$ = 0.528; $[M+H]^+$ = 439.3.

**Example 103: *rac*-(4-{4-[(2-Cyclopropyl-imidazo[5,1-*b*]thiazol-3-yl)-hydroxy-methyl]-[1,2,3]triazol-1-yl}-phenyl)-morpholin-4-yl-methanone**

Step 1: Preparation of (4-azidophenyl)(morpholino)methanone

Prepared according to the procedure described for Example 45, step 1 using (4-(morpholine-4-carbonyl)phenyl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and (4-azidophenyl)(morpholino)methanone. Purification by preparative HPLC (basic conditions) to give *rac*-(4-{4-[(2-cyclopropyl-imidazo[5,1-*b*]thiazol-3-yl)-hydroxy-methyl]-[1,2,3]triazol-1-yl}-phenyl)-morpholin-4-yl-methanone. LC-MS (QC): $t_R$ = 0.528; $[M+H]^+$ = 451.

**Example 104: *rac*-Morpholine-4-carboxylic acid (4-{4-[(2-cyclopropyl-imidazo[5,1-*b*]thiazol-3-yl)-hydroxy-methyl]-[1,2,3]triazol-1-yl}-phenyl)-amide**

Step 1: Preparation of *N*-(4-azidophenyl)morpholine-4-carboxamide

Prepared according to the procedure described for Example 45, step 1 using *N*-(4-(4,4,5,5-tetramethyl-1,3,2-dioxaborolan-2-yl)phenyl)morpholine-4-carboxamide.

Step 2: Prepared according to the procedure described for Example 45 and using intermediate 1, *rac*-1-(2-cyclopropylimidazo[5,1-*b*)]thiazol-3-yl)prop-2-yn-1-ol and *N*-(4-azidophenyl)morpholine-4-carboxamide. Purification by preparative HPLC (basic conditions) to give rac-morpholine-4-carboxylic acid (4-{4-[(2-cyclopropyl-imidazo[5,1-b]thiazol-3-yl)-hydroxy-methyl]-[1,2,3]triazol-1-yl}-phenyl)-amide. LC-MS (QC): $t_R$ = 0.543; $[M+H]^+$ = 466.3.

**Example 105: *rac*-*N*-Cyclopropyl-4-{4-[(2-cyclopropyl-imidazo[5,1-*b*]thiazol-3-yl)-hydroxy-methyl]-[1,2,3]triazol-1-yl}-benzamide**

Step 1: Preparation of 4-azido-*N*-cyclopropylbenzamide

Prepared according to the procedure described for Example 45, step 1 using (4-(cyclopropylcarbamoyl)phenyl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 4-azido-*N*-cyclopropylbenzamide. Purification by preparative HPLC (basic conditions) to give *rac*-*N*-cyclopropyl-4-{4-[(2-cyclopropyl-imidazo[5,1-*b*]thiazol-3-yl)-hydroxy-methyl]-[1,2,3]triazol-1-yl}-benzamide. LC-MS (QC): $t_R$ = 0.553; $[M+H]^+$ = 421.3.

**Example 106: *rac*-4-{4-[(2-Cyclopropyl-imidazo[5,1-*b*]thiazol-3-yl)-hydroxy-methyl]-[1,2,3]triazol-1-yl}-N-(3-methoxy-propyl)-benzamide**

Step 1: Preparation of 4-azido-*N*-(3-methoxypropyl)benzamide

Prepared according to the procedure described for Example 45, step 1 using (4-((3-methoxypropyl)carbamoyl)phenyl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 4-azido-*N*-(3-methoxypropyl)benzamide. Purification by preparative HPLC (basic conditions) to give *rac*-4-{4-[(2-cyclopropyl-imidazo[5,1-*b*]thiazol-3-yl)-hydroxymethyl]-[1,2,3]triazol-1-yl}-N-(3-methoxy-propyl)-benzamide. LC-MS (QC): $t_R$ = 0.563; $[M+H]^+$ = 453.3.

**Example 107: *rac*-4-{4-[(2-Cyclopropyl-imidazo[5,1-*b*]thiazol-3-yl)-hydroxy-methyl]-[1,2,3]triazol-1-yl}-*N*-ethyl-benzamide**

Step 1: Preparation of 4-azido-*N*-ethylbenzamide

Prepared according to the procedure described for Example 45, step 1 using (4-(ethylcarbamoyl)phenyl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 4-azido-*N*-ethylbenzamide. Purification by preparative HPLC (basic conditions) to give rac-4-{4-[(2-cyclopropyl-imidazo[5,1-b]thiazol-3-yl)-hydroxy-methyl]-[1,2,3]triazol-1-yl}-*N*-ethyl-benzamide. LC-MS (QC): $t_R$ = 0.543; $[M+H]^+$ = 409.2.

**Example 108: *rac*-*N*-(2-Cyano-ethyl)-4-{4-[(2-cyclopropyl-imidazo[5,1-*b*]thiazol-3-yl)-hydroxy-methyl]-[1,2,3]triazol-1-yl}-benzamide**

Step 1: Preparation of 4-azido-*N*-(2-cyanoethyl)benzamide

Prepared according to the procedure described for Example 45, step 1 using (4-((2-cyanoethyl)carbamoyl)phenyl)boronic acid.

Step 2: Prepared according to the procedure described for Example 45 and using intermediate 1, *rac*-1-(2-cyclopropylimidazo[5,1-*b*)]thiazol-3-yl)prop-2-yn-1-ol and 4-azido-*N*-(2-cyanoethyl)benzamide. Purification by preparative HPLC (basic conditions) to give *rac*-*N*-(2-cyano-ethyl)-4-{4-[(2-cyclopropyl-imidazo[5,1-*b*]thiazol-3-yl)-hydroxy-methyl]-[1,2,3]triazol-1-yl}-benzamide. LC-MS (QC): $t_R$ = 0.513; $[M+H]^+$ = 434.0.

**Example 109: *rac*-(4-{4-[(2-Cyclopropyl-imidazo[5,1-*b*]thiazol-3-yl)-hydroxy-methyl]-[1,2,3]triazol-1-yl}-phenyl)-urea**

Step 1: Preparation of 1-(4-azidophenyl)urea

Prepared according to the procedure described for Example 45, step 1 using 1-(4-(4,4,5,5-tetramethyl-1,3,2-dioxaborolan-2-yl)phenyl)urea.

Step 2: Prepared according to the procedure described for Example 45 and using intermediate 1, *rac*-1-(2-cyclopropylimidazo[5,1-*b*)]thiazol-3-yl)prop-2-yn-1-ol and 1-(4-azidophenyl)urea. Purification by preparative HPLC (basic conditions) to give rac-(4-{4-[(2-cyclopropyl-imidazo[5,1-b]thiazol-3-yl)-hydroxy-methyl]-[1,2,3]triazol-1-yl}-phenyl)-urea. LC-MS (QC): $t_R$ = 0.434; $[M+H]^+$ = 396.3.

**Example 110: *rac*-*N*-(4-{4-[(2-Ethyl-imidazo[5,1-*b*]thiazol-3-yl)-hydroxy-methyl]-[1,2,3]triazol-1-yl}-phenyl)-acetamide**

Step 1: Preparation of *N*-(4-azidophenyl)acetamide

Prepared according to the procedure described for Example 45, step 1 using (4-acetamidophenyl)boronic acid. Step 2: Prepared according to the procedure described for Example 45 and using intermediate 3, *rac-1-(2*-ethylimidazo[5,1-*b*]thiazol-3-yl)prop-2-yn-1-ol and *N*-(4-azidophenyl)acetamide. Purification by preparative HPLC (basic conditions) to give *rac*-*N*-(4-{4-[(2-ethyl-imidazo[5,1-*b*]thiazol-3-yl)-hydroxy-melhyl]-[1,2,3]triazol-1-yl}-phenyl)-acetamide. LC-MS (QC): $t_R$ = 0.487; $[M+H]^+$ = 383.3.

**Example 111: *rac*-4-{4-[(2-Ethyl-imidazo[5,1-*b*]thiazol-3-yl)-hydroxy-methyl]-[1,2,3]triazol-1-yl}-*N*-methyl-benzamide**

Step 1: Preparation of 4-azido-*N*-methylbenzamide

Prepared according to the procedure described for Example 45, step 1 using (4-(methylcarbamoyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and 4-azido-*N*-methylbenzamide. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-*N*-methyl-benzamide. LC-MS (QC): $t_R$ = 0.449; $[M+H]^+$ = 383.2.

**Example 112: *rac*-5-{4-[(2-Ethyl-imidazo[5,1-*b*]thiazol-3-yl)-hydroxy-methyl]-[1,2,3]triazol-1-yl}-1,3-dihydro-benzoimidazol-2-one**

Step 1: Preparation of 5-azido-1,3-dihydro-2*H*-benzo[*d*]imidazol-2-one

Prepared according to the procedure described for Example 45, step 1 using 5-(4,4,5,5-tetramethyl-1,3,2-dioxaborolan-2-yl)-1,3-dihydro-2*H*-benzo[*d*]imidazol-2-one.

Step 2: Prepared according to the procedure described for Example 45 and using intermediate 3, *rac-1-(2-*ethylimidazo[5,1-*b*]thiazol-3-yl)prop-2-yn-1-ol and 5-azido-1,3-dihydro-2*H*-benzo[*d*]imidazol-2-one. Purification by preparative HPLC (basic conditions) to give rac-5-{4-[(2-ethyl-imidazo[5,1-b]thiazol-3-yl)-hydroxy-methyl]-[1,2,3]triazol-1-yl}-1,3-dihydro-benzoimidazol-2-one. LC-MS (QC): $t_R$ = 0.409; $[M+H]^+$ = 382.2.

**Example 113: *rac*-1-Ethyl-3-(4-{4-[(2-ethyl-imidazo[5,1-*b*]thiazol-3-yl)-hydroxy-methyl]-[1,2,3]triazol-1-yl}-2-methoxy-phenyl)-urea**

Step 1: Preparation of 1-(4-azido-2-methoxyphenyl)-3-ethylurea

Prepared according to the procedure described for Example 45, step 1 using 1-ethyl-3-(2-methoxy-4-(4,4,5,5-tetramethyl-1,3,2-dioxaborolan-2-yl)phenyl)urea.

Step 2: Prepared according to the procedure described for Example 45 and using intermediate 3, *rac-1-(2-*ethylimidazo[5,1-*b*]thiazol-3-yl)prop-2-yn-1-ol and 1-(4-azido-2-methoxyphenyl)-3-ethylurea. Purification by preparative HPLC (basic conditions) to give rac-1-ethyl-3-(4-{4-[(2-ethyl-imidazo[5,1-b]thiazol-3-yl)-hydroxymethyl]-[1,2,3]triazol-1-yl}-2-methoxy-phenyl)-urea. LC-MS (QC): $t_R$ = 0.579; $[M+H]^+$ = 442.4.

**Example 114: *rac*-4-{4-[(2-Ethyl-imidazo[5,1-*b*]thiazol-3-yl)-hydroxy-methyl]-[1,2,3]triazol-1-yl}-*N,N*-dimethyl-benzamide**

Step 1: Preparation of 4-azido-*N*,*N*-dimethylbenzamide

Prepared according to the procedure described for Example 45, step 1 using (4-(dimethylcarbamoyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and 4-azido-*N*,*N*-dimethylbenzamide. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-*N*,*N*-dimethyl-benzamide. LC-MS (QC): $t_R$ = 0.508; $[M+H]^+$ = 397.0.

**Example 115: *rac*-4-{4-[(2-Ethyl-imidazo[5,1-*b*]thiazol-3-yl)-hydroxy-methyl]-[1,2,3]triazol-1-yl}-*N*-(3-hydroxy-propyl)-benzamide**

Step 1: Preparation of 4-azido-*N*-(3-hydroxypropyl)benzamide

Prepared according to the procedure described for Example 45, step 1 using (4-((3-hydroxypropyl)carbamoyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and 4-azido-*N*-(3-hydroxypropyl)benzamide. Purification by preparative HPLC (basic conditions) to give rac-4-{4-[(2-ethyl-imidazo[5,1-b]thiazol-3-yl)-hydroxy-methyl]-[1,2,3]triazol-1-yl}-*N*-(3-hydroxy-propyl)-benzamide. LC-MS (QC): $t_R$ = 0.443; $[M+H]^+$ = 427.3.

**Example 116: *rac*-4-{4-[(2-Ethyl-imidazo[5,1-*b*]thiazol-3-yl)-hydroxy-methyl]-[1,2,3]triazol-1-yl}-benzamide**Step 1: Preparation of 4-azidobenzamide

Prepared according to the procedure described for Example 45, step 1 using 4-(4,4,5,5-tetramethyl-1,3,2-dioxaborolan-2-yl)benzamide.

Step 2: Prepared according to the procedure described for Example 45 and using intermediate 3, *rac-1-(2-*ethylimidazo[5,1-*b*]thiazol-3-yl)prop-2-yn-1-ol and 4-azidobenzamide. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-benzamide. LC-MS (QC): $t_R$ = 0.411; $[M+H]^+$ = 369.2.

**Example 117: *rac*-(4-{4-[(2-Ethyl-imidazo[5,1-*b*]thiazol-3-yl)-hydroxy-methyl]-[1,2,3]triazol-1-yl}-phenyl)-morpholin-4-yl-methanone**

Step 1: Preparation of (4-azidophenyl)(morpholino)methanone

Prepared according to the procedure described for Example 45, step 1 using (4-(morpholine-4-carbonyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and (4-azidophenyl)(morpholino)methanone. Purification by preparative HPLC (basic conditions) to give rac-(4-{4-[(2-ethyl-imidazo[5,1-b]thiazol-3-yl)-hydroxy-methyl]-[1,2,3]triazol-1-yl}-phenyl)-morpholin-4-yl-methanone. LC-MS (QC): $t_R$ = 0.508; $[M+H]^+$ = 439.1.

**Example 118: *rac*-4-{4-[(2-Ethyl-imidazo[5,1-*b*]thiazol-3-yl)-hydroxy-methyl]-[1,2,3]triazol-1-yl}-*N*-(2-hydroxy-ethyl)-benzamide**

Step 1: Preparation of 4-azido-*N*-(2-hydroxyethyl)benzamide

Prepared according to the procedure described for Example 45, step 1 using (4-((2-hydroxyethyl)carbamoyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and 4-azido-*N*-(2-hydroxyethyl)benzamide. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-*N*-(2-hydroxy-ethyl)-benzamide. LC-MS (QC): $t_R$ = 0.419; $[M+H]^+$ = 413.3.

**Example 119: *rac*-*N*-(2-Dimethylamino-ethyl)-4-{4-[(2-ethyl-imidazo[5,1-*b*]thiazol-3-yl)-hydroxy-methyl]-[1,2,3]triazol-1-yl}-benzamide**

Step 1: Preparation of 4-azido-*N*-(2-(dimethylamino)ethyl)benzamide

Prepared according to the procedure described for Example 45, step 1 using (4-((2-(dimethylamino)ethyl)carbamoyl)phenyl)boronic acid hydrochloride.

Step 2: Prepared according to the procedure described for Example 45 and using intermediate 3, *rac*-1-(2-ethylimidazo[5,1-*b*]thiazol-3-yl)prop-2-yn-1-ol and 4-azido-*N*-(2-(dimethylamino)ethyl)benzamide. Purification by preparative HPLC (basic conditions) to give *rac*-*N*-(2-dimethylamino-ethyl)-4-{4-[(2-ethyl-imidazo[5,1-*b*]thiazol-3-yl)-hydroxy-methyl]-[1,2,3]triazol-1-yl}-benzamide. LC-MS (QC): $t_R$ = 0.315; $[M+H]^+$ = 440.1.

**Example 120: *rac*-*N*-(4-{4-[(2-Ethyl-imidazo[5,1-*b*]thiazol-3-yl)-hydroxy-methyl]-[1,2,3]triazol-1-yl}-phenyl)-2,2-dimethyl-propionamide**

Step 1: Preparation of *N*-(4-azidophenyl)pivalamide

Prepared according to the procedure described for Example 45, step 1 using (4-pivalamidophenyl)boronic acid. Step 2: Prepared according to the procedure described for Example 45 and using intermediate 3, *rac*-1-(2-ethylimidazo[5,1-*b*]thiazol-3-yl)prop-2-yn-1-ol and *N*-(4-azidophenyl)pivalamide. Purification by preparative HPLC (basic conditions) to give *rac*-*N*-(4-{4-[(2-ethyl-imidazo[5,1-*b*]thiazol-3-yl)-hydroxy-methyl]-[1,2,3]triazol-1-yl}-phenyl)-2,2-dimethyl-propionamide. LC-MS (QC): $t_R$ = 0.655; $[M+H]^+$ = 425.4.

**Example 121: *rac*-Morpholine-4-carboxylic acid (4-{4-[(2-ethyl-imidazo[5,1-*b*]thiazol-3-yl)-hydroxy-methyl]-[1,2,3]triazol-1-yl}-phenyl)-amide**

Step 1: Preparation of *N*-(4-azidophenyl)morpholine-4-carboxamide

Prepared according to the procedure described for Example 45, step 1 using *N*-(4-(4,4,5,5-tetramethyl-1,3,2-dioxaborolan-2-yl)phenyl)morpholine-4-carboxamide.

Step 2: Prepared according to the procedure described for Example 45 and using intermediate 3, *rac*-1-(2-ethylimidazo[5,1-*b*]thiazol-3-yl)prop-2-yn-1-ol and *N*-(4-azidophenyl)morpholine-4-carboxamide. Purification by preparative HPLC (basic conditions) to give rac-morpholine-4-carboxylic acid (4-{4-[(2-ethyl-imidazo[5,1-b]thiazol-3-yl)-hydroxy-methyl]-[1,2,3]triazol-1-yl}-phenyl)-amide. LC-MS (QC): $t_R$ = 0.523; $[M+H]^+$ = 454.2.

**Example 122: *rac*-1-Butyl-3-(4-{4-[(2-ethyl-imidazo[5,1-*b*]thiazol-3-yl)-hydroxy-methyl]-[1,2,3]triazol-1-yl}-phenyl)-urea**

Step 1: Preparation of 1-(4-azidophenyl)-3-butylurea

Prepared according to the procedure described for Example 45, step 1 using 1-butyl-3-(4-(4,4,5,5-tetramethyl-1,3,2-dioxaborolan-2-yl)phenyl)urea.

Step 2: Prepared according to the procedure described for Example 45 and using intermediate 3, *rac-1-(2-*ethylimidazo[5,1-*b*]thiazol-3-yl)prop-2-yn-1-ol and 1-(4-azidophenyl)-3-butylurea. Purification by preparative HPLC (basic conditions) to give *rac*-1-butyl-3-(4-{4-[(2-ethyl-imidazo[5,1-*b*]thiazol-3-yl)-hydroxy-methyl]-**[1,2,3]triazol-1**-yl}-phenyl)-urea. LC-MS (QC): $t_R$ = 0.644; $[M+H]^+$ = 440.2.

**Example 123: *rac*-4-{4-[(2-Ethyl-imidazo[5,1-*b*]thiazol-3-yl)-hydroxy-methyl]-[1,2,3]triazol-1-yl}-*N*-furan-2-ylmethyl-benzamide**

Step 1: Preparation of 4-azido-*N*-(furan-2-ylmethyl)benzamide

Prepared according to the procedure described for Example 45, step 1 using (4-((furan-2-ylmethyl)carbamoyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and 4-azido-*N*-(furan-2-ylmethyl)benzamide. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-*N*-furan-2-ylmethyl-benzamide. LC-MS (QC): $t_R$ = 0.599; $[M+H]^+$ = 449.1.

**Example 124: *rac-N*-Ethyl-4-{4-[(2-ethyl-imidazo[5,1-*b*]thiazol-3-yl)-hydroxy-methyl]-[1,2,3]triazol-1-yl}-*N*-methyl-benzamide**

Step 1: Preparation of 4-azido-*N*-ethyl-*N*-methylbenzamide

Prepared according to the procedure described for Example 45, step 1 using (4-(ethyl(methyl)carbamoyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and 4-azido-*N*-ethyl-*N*-methylbenzamide. Purification by preparative HPLC (basic conditions) to give *rac-N*-ethyl-4-{4-[(2-ethyl-imidazo[5,1-*b*]thiazol-3-yl)-hydroxy-methyl]-[1,2,3]triazol-1-yl}-*N*-methyl-benzamide. LC-MS (QC): $t_R$ = 0.559; $[M+H]^+$ = 411.3.

**Example 125: *rac-N*-Cyclopropyl-4-{4-[(2-ethyl-imidazo[5,1-*b*]thiazol-3-yl)-hydroxy-methyl]-[1,2,3]triazol-1-yl}-benzamide**

Step 1: Preparation of 4-azido-*N*-cyclopropylbenzamide

Prepared according to the procedure described for Example 45, step 1 using (4-(cyclopropylcarbamoyl)phenyl)boronic acid.

Step 2: Prepared according to the procedure described for Example 45 and using intermediate 3, *rac*-1-(2-ethylimidazo[5,1-*b*]thiazol-3-yl)prop-2-yn-1-ol and 4-azido-*N*-cyclopropylbenzamide. Purification by preparative HPLC (basic conditions) to give *rac-N*-cyclopropyl-4-{4-[(2-ethyl-imidazo[5,1-*b*]thiazol-3-yl)-hydroxy-methyl]-[1,2,3]triazol-1-yl}-benzamide. LC-MS (QC): $t_R$ = 0.528; $[M+H]^+$ = 409.1.

**Example 126: *rac*-4-{4-[(2-Ethyl-imidazo[5,1-*b*]thiazol-3-yl)-hydroxy-methyl]-[1,2,3]triazol-1-yl}-*N*-(3-methoxy-propyl)-benzamide**

Step 1: Preparation of 4-azido-*N*-(3-methoxypropyl)benzamide

Prepared according to the procedure described for Example 45, step 1 using (4-((3-methoxypropyl)carbamoyl)phenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and 4-azido-*N*-(3-methoxypropyl)benzamide. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-*N*-(3-methoxy-propyl)-benzamide. LC-MS (QC): $t_R$ = 0.543; $[M+H]^+$ = 441.2.

**Example 127: *rac-N*-(4-{4-[(2-Ethyl-imidazo[5,1-*b*]thiazol-3-yl)-hydroxy-methyl]-[1,2,3]triazol-1-yl}-phenyl)-isobutyramide**

Step 1: Preparation of *N*-(4-azidophenyl)isobutyramide

Prepared according to the procedure described for Example 45, step 1 using (4-isobutyramidophenyl)boronic acid.

Step 2: Prepared according to the procedure described for Example 45 and using intermediate 3, *rac-1-(2-*ethylimidazo[5,1-*b*]thiazol-3-yl)prop-2-yn-1-ol and *N*-(4-azidophenyl)isobutyramide. Purification by preparative HPLC (basic conditions) to give *rac-N*-(4-{4-[(2-ethyl-imidazo[5,1-*b*]thiazol-3-yl)-hydroxy-methyl]-[1,2,3]triazol-1-yl}-phenyl)-isobutyramide. LC-MS (QC): $t_R$ = 0.594; $[M+H]^+$ = 411.4.

**Example 128: *rac-N*-Ethyl-4-{4-[(2-ethyl-imidazo[5,1-*b*]thiazol-3-yl)-hydroxy-methyl]-[1,2,3]triazol-1-yl}-benzamide**

Step 1: Preparation of 4-azido-*N*-ethylbenzamide

Prepared according to the procedure described for Example 45, step 1 using (4-(ethylcarbamoyl)phenyl)boronic acid.

Step 2: Prepared according to the procedure described for Example 45 and using intermediate 3, *rac*-1-(2-ethylimidazo[5,1-*b*]thiazol-3-yl)prop-2-yn-1-ol and 4-azido-*N*-ethylbenzamide. Purification by preparative HPLC (basic conditions) to give *rac*-*N*-ethyl-4-{4-[(2-ethyl-imidazo[5,1-*b*]thiazol-3-yl)-hydroxy-methyl]-[1,2,3]triazol-1-yl}-benzamide. LC-MS (QC): $t_R$ = 0.518; $[M+H]^+$ = 397.2.

**Example 129: *rac-N*-Allyl-4-{4-[(2-ethyl-imidazo[5,1-*b*]thiazol-3-yl)-hydroxy-methyl]-[1,2,3]triazol-1-yl}-benzamide**

Step 1: Preparation of *N*-allyl-4-azidobenzamide

Prepared according to the procedure described for Example 45, step 1 using (4-(allylcarbamoyl)phenyl)boronic acid.

Step 2: Prepared according to the procedure described for Example 45 and using intermediate 3, *rac*-1-(2-ethylimidazo[5,1-*b*]thiazol-3-yl)prop-2-yn-1-ol and *N*-allyl-4-azidobenzamide. Purification by preparative HPLC (basic conditions) to give *rac*-*N*-allyl-4-{4-[(2-ethyl-imidazo[5,1-*b*]thiazol-3-yl)-hydroxy-methyl]-[1,2,3]triazol-1-yl}-benzamide. LC-MS (QC): $t_R$ = 0.548; $[M+H]^+$ = 409.3.

**Example 130: *rac-N*-(2-Cyano-ethyl)-4-{4-[(2-ethyl-imidazo[5,1-*b*]thiazol-3-yl)-hydroxy-methyl]-[1,2,3]triazol-1-yl}-benzamide**

Step 1: Preparation of 4-azido-*N*-(2-cyanoethyl)benzamide

Prepared according to the procedure described for Example 45, step 1 using (4-((2-cyanoethyl)carbamoyl)phenyl)boronic acid.

Step 2: Prepared according to the procedure described for Example 45 and using intermediate 3, *rac-1-(2-*

ethylimidazo[5,1-*b*]thiazol-3-yl)prop-2-yn-1-ol and 4-azido-*N*-(2-cyanoethyl)benzamide. Purification by preparative HPLC (basic conditions) to give *rac-N*-(2-cyano-ethyl)-4-{4-[(2-ethyl-imidazo[5,1-*b*]thiazol-3-yl)-hydroxy-methyl]-[1,2,3]triazol-1-yl}-benzamide. LC-MS (QC): $t_R$ = 0.469; $[M+H]^+$ = 422.1.

**Example 131: *rac*-(4-{4-[(2-Ethyl-imidazo[5,1-*b*]thiazol-3-yl)-hydroxy-methyl]-[1,2,3]triazol-1-yl}-phenyl)-urea**

Step 1: Preparation of 1-(4-azidophenyl)urea

Prepared according to the procedure described for Example 45, step 1 using 1-(4-(4,4,5,5-tetramethyl-1,3,2-dioxaborolan-2-yl)phenyl)urea.

Step 2: Prepared according to the procedure described for Example 45 and using intermediate 3, *rac-1-(2-*ethylimidazo[5,1-*b*]thiazol-3-yl)prop-2-yn-1-ol and 1-(4-azidophenyl)urea. Purification by preparative HPLC (basic conditions) to give *rac*-(4-{4-[(2-ethyl-imidazo[5,1-*b*]thiazol-3-yl)-hydroxy-methyl]-[1,2,3]triazol-1-yl}-phenyl)-urea. LC-MS (QC): $t_R$ = 0.410; $[M+H]^+$ = 384.4.

**Example 132: *rac*-(4-{4-[(2-Cyclopropyl-imidazo[5,1-*b*]thiazol-3-yl)-hydroxy-methyl]-pyrazol-1-yl}-phenyl)-carbamic acid methyl ester**

Step 1: Preparation of ethyl 1-(4-((methoxycarbonyl)amino)phenyl)-1*H*-pyrazole-4-carboxylate To a solution of ethyl pyrazole-4-carboxylate (300 mg; 2.14 mmol) and 4-(methoxycarbonylamino)phenylboronic acid (417 mg; 2.14 mmol) in DMF (10 ml) are added $Cu(OAc)_2$ (292 mg; 1.61 mmol) and pyridine (0.345 ml; 4.28 mmol). The suspension is stirred at RT for 12 days. Sat. aq. $NaHCO_3$ solution is added and the mixture extracted with DCM (2x). The combined organic extracts are washed with brine, dried ($MgSO_4$), filtered and concentrated under reduced pressure. The crude residue is purified by FC (silicagel, Hept / EtOAc) to give 305 mg of ethyl 1-(4-((methoxycarbonyl)amino)phenyl)-1*H*-pyrazole-4-carboxylate as a white solid. LC-MS (acidic): $t_R$ = 0.86; $[M+H]^+$ = 289.82.

Step 2: Preparation of methyl (4-(4-(hydroxymethyl)-1*H*-pyrazol-1-yl)phenyl)carbamate

To a solution of the product of step 1, ethyl 1-(4-((methoxycarbonyl)amino)phenyl)-1*H*-pyrazole-4-carboxylate (305 mg; 1.00 mmol) in THF (10 ml) is added $LiAlH_4$ (1M in THF; 1.5 ml; 1.50 mmol) at RT. The reaction mixture is stirred at RT for 30 min. Water is carefully added followed by aq. 1N NaOH. The aq. layer is extracted with DCM (2x). The combined organic extracts are washed with brine, dried ($MgSO_4$), filtered and concentrated under reduced pressure to give 204 mg of methyl (4-(4-(hydroxymethyl)-1*H*-pyrazol-1-yl)phenyl)carbamate as a beige solid. LC-MS (acidic): $t_R$ = 0.61; $[M+H]^+$ = 248.06.

Step 3: Preparation of methyl (4-(4-formyl-1*H*-pyrazol-1-yl)phenyl)carbamate

To a solution of the product of step 2, methyl (4-(4-(hydroxymethyl)-1*H*-pyrazol-1-yl)phenyl)carbamate (204 mg; 0.83 mmol) in DCM (10 ml) is added Dess-Martin periodinane (525 mg; 1.24 mmol) at RT. The reaction mixture is stirred at RT for 1 h. Sat. aq. $NaHCO_3$ solution is added, followed by sat. aq. $Na_2S_2O_3$ solution. The mixture is stirred at RT for 10 min. The layers are separated and the aq. layer is extracted with DCM (2x). The combined organic extracts are washed with brine, dried ($MgSO_4$), filtered and concentrated under reduced pressure to give 177 mg of methyl (4-(4-formyl-1*H*-pyrazol-1-yl)phenyl)carbamate as a brown solid. LC-MS (acidic): $t_R$ = 0.72; $[M+H]^+$ = 246.06.

Step 4: Preparation of *rac*-(4-{4-[(2-cyclopropyl-imidazo[5,1-*b*]thiazol-3-yl)-hydroxy-methyl]-pyrazol-1-yl}-phenyl)-carbamic acid methyl ester (Example 132)

Prepared following the procedure described for Example 96 using intermediate 10, 3-bromo-2-cyclopropylimidazo[5,1-*b*]thiazole and methyl (4-(4-formyl-1*H*-pyrazol-1-yl)phenyl)carbamate. Purification by preparative HPLC (acidic conditions) followed by neutralization and extraction with DCM to give rac-(4-{4-[(2-cyclopropyl-imidazo[5,1-b]thiazol-3-yl)-hydroxy-methyl]-pyrazol-1-yl}-phenyl)-carbamic acid methyl ester. LC-MS (QC): $t_R$ = 0.0.599; $[M+H]^+$ = 410.3.

**Example 133: rac-(1-Phenyl-1H-[1,2,3]triazol-4-yl)-(2-trifluoromethyl-imidazo[5,1-b]thiazol-3-yl)-methanol**

Prepared according to the procedure described for Example 1 and using intermediate 12, *rac-1-(2-*(trifluoromethyl)imidazo[5,1-*b*]thiazol-3-yl)prop-2-yn-1-ol and azidobenzene. Purification by preparative HPLC (basic conditions) to give *rac*-(1-Phenyl-1*H*-[1,2,3]triazol-4-yl)-(2-trifluoromethyl-imidazo[5,1-*b*]thiazol-3-yl)-methanol. LC-MS (QC): $t_R$ = 0.870; $[M+H]^+$ = 366.2.

**Example 134: rac-[1-(4-Methoxy-phenyl)-1H-[1,2,3]triazol-4-yl]-(2-trifluoromethyl-imidazo[5,1-b]thiazol-3-yl)-methanol**

Prepared according to the procedure described for Example 1 and using intermediate 12, *rac-1-(2-*(trifluoromethyl)imidazo[5,1-*b*]thiazol-3-yl)prop-2-yn-1-ol and 1-azido-4-methoxybenzene. Purification by preparative HPLC (basic conditions) to give *rac*-[1-(4-methoxy-phenyl)-1*H*-[1,2,3]triazol-4-yl]-(2-trifluoromethyl-imidazo[5,1-b]thiazol-3-yl)-methanol. LC-MS (QC): $t_R$ = 0.885; $[M+H]^+$ = 396.2.

**Example 135: *rac*-5-{4-[(2-Cyclopropyl-imidazo[5,1-*b*]thiazol-3-yl)-hydroxy-methyl]-[1,2,3]triazol-1-yl}-2-methoxy-benzonitrile**

Step 1: Preparation of 5-azido-2-methoxybenzonitrile

Prepared according to the procedure described for Example 45, step 1 using (3-cyano-4-methoxyphenyl)boronic acid.

Step 2: Prepared following the procedure described for Example 45, and using Intermediate 1, *rac*-1-(2-cyclopropylimidazo[5,1-*b*)]thiazol-3-yl)prop-2-yn-1-ol and 5-azido-2-methoxybenzonitrile. Purification by prepHPLC (basic conditions) to give rac-5-{4-[(2-cyclopropyl-imidazo[5,1-b]thiazol-3-yl)-hydroxy-methyl]-[1,2,3]triazol-1-yl}-2-methoxy-benzonitrile. LC-MS (QC): $t_R$ = 0.590; $[M+H]^+$ = 393.1.

**Example 136: *rac*-2-(4-{4-[(2-Cyclopropyl-imidazo[5,1-*b*]thiazol-3-yl)-hydroxy-methyl]-[1,2,3]triazol-1-yl}-phenoxy)-ethanol**

Prepared following the procedure described for Example 87, and using 2-bromoethanol. Purification by preparative HPLC (basic conditions) gives *rac*-2-(4-{4-[(2-cyclopropyl-imidazo[5,1-*b*]thiazol-3-yl)-hydroxy-methyl]-[1,2,3]triazol-1-yl}-phenoxy)-ethanol. LC-MS (QC): $t_R$ = 0.492; $[M+H]^+$ = 398.0.

**Example 137: *rac*-3-(4-{4-[(2-Cyclopropyl-imidazo[5,1-*b*]thiazol-3-yl)-hydroxy-methyl]-[1,2,3]triazol-1-yl}-phenoxy)-propan-1-ol**

Prepared following the procedure described for Example 87, and using 3-bromo-1-propanol. Purification by preparative HPLC (basic conditions) gives *rac*-3-(4-{4-[(2-cyclopropyl-imidazo[5,1-*b*]thiazol-3-yl)-hydroxy-methyl]-[1,2,3]triazol-1-yl}-phenoxy)-propan-1-ol. LC-MS (QC): $t_R$ = 0.541; $[M+H]^+$ = 412.1.

**Example 137a: 3-(4-{4-[(*R*)-(2-Cyclopropyl-imidazo[5,1-*b*]thiazol-3-yl)-hydroxy-methyl]-[1,2,3]triazol-1-yl}-phenoxy)-propan-1-ol**

Separation of the enantiomers on chiral stationary phase:
Method: Column: ChiralPak AD-H 30x250mm, 5μM; Detector Settings: UV-Vis-1; 210 nM; Eluent: 50% $CO_2$ and 50% EtOH; Flow: 160.00 ml/min; BPR: 100 bar; Temperature: 40°C. Injection volume: 1000μl.

18 mg of the racemate are separated by the method described above to give 6 mg of the R-enantiomer Example 137a and 6 mg of the S-enantiomer. LC-MS (QC): $t_R$ = 0.543; $[M+H]^+$ = 412.2.

**Example 138: *rac*-2-[2-(4-{4-[(2-Cyclopropyl-imidazo[5,1-*b*]thiazol-3-yl)-hydroxy-methyl]-[1,2,3]triazol-1-yl}-phenoxy)-ethoxy]-ethanol**

Prepared following the procedure described for Example 87, and using diethylene glycol monochlorohydrin. Purification by preparative HPLC (basic conditions) gives *rac*-2-[2-(4-{4-[(2-cyclopropyl-imidazo[5,1-*b*]thiazol-3-yl)-hydroxy-methyl]-[1,2,3]triazol-1-yl}-phenoxy)-ethoxy]-ethanol. LC-MS (QC): $t_R$ = 0.524; $[M+H]^+$ = 442.1.

**Example 139: *rac*-1-(4-{4-[(2-Cyclopropyl-imidazo[5,1-*b*]thiazol-3-yl)-hydroxy-methyl]-[1,2,3]triazol-1-yl}-phenoxy)-2-methyl-propan-2-ol**

Prepared following the procedure described for Example 87, and using 1-chloro-2-methyl-2-propanol. Purification by preparative HPLC (basic conditions) gives *rac*-1-(4-{4-[(2-cyclopropyl-imidazo[5,1-*b*]thiazol-3-yl)-hydroxy-methyl]-[1,2,3]triazol-1-yl}-phenoxy)-2-methyl-propan-2-ol. LC-MS (QC): $t_R$ = 0.590; $[M+H]^+$ = 426.0.

**Example 140: *rac*-4-(4-{4-[(2-Cyclopropyl-imidazo[5,1-*b*]thiazol-3-yl)-hydroxy-methyl]-[1,2,3]triazol-1-yl}-phenoxy)-2-methyl-butan-2-ol**

Prepared following the procedure described for Example 87, and using 4-bromo-2-methylbutan-2-ol. Purification by preparative HPLC (basic conditions) gives *rac*-4-(4-{4-[(2-cyclopropyl-imidazo[5,1-*b*]thiazol-3-yl)-hydroxy-methyl]-[1,2,3]triazol-1-yl}-phenoxy)-2-methyl-butan-2-ol. LC-MS (QC): $t_R$ = 0.626; $[M+H]^+$ = 440.1.

**Example 140a: 4-(4-{4-[(*R*)-(2-Cyclopropyl-imidazo[5,1-*b*]thiazol-3-yl)-hydroxy-methyl]-[1,2,3]triazol-1-yl}-phenoxy)-2-methyl-butan-2-ol**

Separation of the enantiomers on chiral stationary phase:
Method: Column: ChiralPak AD-H 30x250mm, 5μM; Detector Settings: UV-Vis-1; 210 nM; Eluent: 55% $CO_2$ and 45% EtOH; Flow: 160.00 ml/min; BPR: 100 bar; Temperature: 40°C. Injection volume: 1000μl.

26.9 mg of the racemate are separated by the method described above to give 10 mg of the R-enantiomer Example 140a and 9 mg of the S-enantiomer. LC-MS (QC): $t_R$ = 0.629; $[M+H]^+$ = 440.0.

**Example 141: *rac*-3-(4-{4-[(2-Cyclopropyl-imidazo[5,1-*b*]thiazol-3-yl)-hydroxy-methyl]-[1,2,3]triazol-1-yl}-phenoxy)-2,2-dimethyl-propan-1-ol**

Prepared following the procedure described for Example 87, and using 3-bromo-2,2-dimethyl-1-propanol. Purification by preparative HPLC (basic conditions) gives *rac*-3-(4-{4-[(2-cyclopropyl-imidazo[5,1-*b*]thiazol-3-yl)-hydroxy-methyl]-[1,2,3]triazol-1-yl}-phenoxy)-2,2-dimethyl-propan-1-ol. LC-MS (QC): $t_R$ = 0.667; $[M+H]^+$ = 440.3.

**Example 142: *rac*-1-[2-(4-{4-[(2-Cyclopropyl-imidazo[5,1-*b*]thiazol-3-yl)-hydroxy-methyl]-[1,2,3]triazol-1-yl}-phenoxy)-ethyl]-cyclopropanol**

Prepared following the procedure described for Example 87, and using 1-(2-bromoethyl)cyclopropan-1-ol. Purification by preparative HPLC (basic conditions) gives *rac*-1-[2-(4-{4-[(2-cyclopropyl-imidazo[5,1-*b*]thiazol-3-yl)-hydroxy-methyl]-[1,2,3]triazol-1-yl}-phenoxy)-ethyl]-cyclopropanol. LC-MS (QC): $t_R$ = 0.619; $[M+H]^+$ = 438.1.

**Example 142a: 1-[2-(4-{4-[(*R*)-(2-Cyclopropyl-imidazo[5,1-*b*]thiazol-3-yl)-hydroxy-methyl]-[1,2,3]triazol-1-yl}-phenoxy)-ethyl]-cyclopropanol**

Separation of the enantiomers on chiral stationary phase:
Method: Column: ChiralPak AD-H 30x250mm, 5μM; Detector Settings: UV-Vis-1; 210 nM; Eluent: 50% $CO_2$ and 50% EtOH; Flow: 160.00 ml/min; BPR: 100 bar; Temperature: 40°C. Injection volume: 1000μl.

28.4 mg of the racemate are separated by the method described above to give 10 mg of the R-enantiomer Example 142a and 11 mg of the S-enantiomer. LC-MS (QC): $t_R$ = 0.621; $[M+H]^+$ = 438.0.

**Example 143: (2-Cyclopropyl-imidazo[5,1-*b*]thiazol-3-yl)-{1-[4-(tetrahydro-pyran-2-ylmethoxy)-phenyl]-1*H*-[1,2,3]triazol-4-yl}-methanol**

Prepared following the procedure described for Example 87, and using 2-(bromomethyl)tetrahydro-2*H*-pyran. Purification by preparative HPLC (basic conditions) gives (2-cyclopropyl-imidazo[5,1-b]thiazol-3-yl)-{1-[4-(tetrahydro-pyran-2-ylmethoxy)-phenyl]-1*H*-[1,2,3]triazol-4-yl}-methanol. LC-MS (QC): $t_R$ = 0.717; $[M+H]^+$ = 452.2.

**Example 144: *rac*-(2-Cyclopropyl-imidazo[5,1-*b*]thiazol-3-yl)-{1-[4-(3-methyl-oxetan-3-ylmethoxy)-phenyl]-1*H*-[1,2,3]triazol-4-yl}-methanol**

Prepared following the procedure described for Example 87, and using 3-(iodomethyl)-3-methyloxetane. Purification by preparative HPLC (basic conditions) gives *rac*-(2-cyclopropyl-imidazo[5,1-*b*]thiazol-3-yl)-{1-[4-(3-methyl-oxetan-3-ylmethoxy)-phenyl]-1*H*-[1,2,3]triazol-4-yl}-methanol. LC-MS (QC): $t_R$ = 0.637; $[M+H]^+$ = 438.4.

**Example 145: *rac*-(2-Cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol**

Prepared following the procedure described for Example 87, and using 3-fluoro-3-iodomethyloxetane. Purification by preparative HPLC (basic conditions) gives *rac*-(2-cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol. LC-MS (QC): $t_R$ = 0.606; $[M+H]^+$ = 442.3.

**Example 145a: (*R*)-(2-Cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol**

Separation of the enantiomers on chiral stationary phase:
Method: Column: ChiralPak AD-H 30x250mm, 5µM; Detector Settings: UV-Vis-1; 210 nM; Eluent: 50% $CO_2$ and 50% EtOH; Flow: 160.00 ml/min; BPR: 100 bar; Temperature: 40°C. Injection volume: 2000µl.
25.6 mg of the racemate are separated by the method described above to give 13 mg of the R-enantiomer Example 145a and 9 mg of the S-enantiomer. LC-MS (QC): $t_R$ = 0.608; $[M+H]^+$ = 442.2.

**Example 146: (2-Cyclopropyl-imidazo[5,1-*b*]thiazol-3-yl)-{1-[4-(1-methyl-piperidin-3-ylmethoxy)-phenyl]-1*H*-[1,2,3]triazol-4-yl}-methanol**

Prepared following the procedure described for Example 87, and using 3-chloromethyl-1-methylpiperidine hydrochloride. Purification by preparative HPLC (basic conditions) gives (2-cyclopropyl-imidazo[5,1-*b*]thiazol-3-yl)-{1-[4-(1-methyl-piperidin-3-ylmethoxy)-phenyl]-1*H*-[1,2,3]triazol-4-yl}-methanol. LC-MS (QC): $t_R$ = 0.404; $[M+H]^+$ = 465.3.

**Example 147: *rac*-(2-Cyclopropyl-imidazo[5,1-*b*]thiazol-3-yl)-{1-[4-(1,1-dioxo-hexahydro-1l6-thiopyran-4-ylmethoxy)-phenyl]-1*H*-[1,2,3]triazol-4-yl}-methanol**

Prepared following the procedure described for Example 87, and using 4-(bromomethyl)-tetrahydro-2*H*-thiopyran-1,1-dioxide. Purification by preparative HPLC (basic conditions) gives *rac*-(2-cyclopropyl-imidazo[5,1-*b*]thiazol-3-yl)-{1-[4-(1,1-dioxo-hexahydro-1l6-thiopyran-4-ylmethoxy)-phenyl]-1*H*-[1,2,3]triazol-4-yl}-methanol. LC-MS (QC): $t_R$ = 0.585; $[M+H]^+$ = 500.0.

**Example 148: *rac*-2-Chloro-4-{4-[(2-cyclopropyl-imidazo[5,1-*b*]thiazol-3-yl)-hydroxy-methyl]-[1,2,3]triazol-1-yl}-phenol**

Step 1: Preparation of 4-azido-2-chlorophenol

Prepared according to the procedure described for Example 45, step 1 using (3-chloro-4-hydroxyphenyl)boronic acid.
Step 2: Prepared following the procedure described for Example 45, and using Intermediate 1, *rac*-1-(2-cyclopropylimidazo[5,1-*b*)]thiazol-3-yl)prop-2-yn-1-ol and 4-azido-2-chlorophenol. Purification by prepHPLC (basic conditions) to give rac-2-chloro-4-{4-[(2-cyclopropyl-imidazo[5,1-b]thiazol-3-yl)-hydroxy-methyl]-[1,2,3]triazol-1-yl}-phenol. LC-MS (QC): $t_R$ = 0.560; $[M+H]^+$ = 388.2.

**Example 148a: 2-Chloro-4-{4-[(*R*)-(2-cyclopropyl-imidazo[5,1-*b*]thiazol-3-yl)-hydroxy-methyl]-[1,2,3]triazol-1-yl}-phenol**

Separation of the enantiomers on chiral stationary phase:
Method: Column: ChiralPak AD-H 30x250mm, 5µM; Detector Settings: UV-Vis-1; 252 nM; Eluent: 70% $CO_2$ and 30% EtOH; Flow: 160.00 ml/min; BPR: 100 bar; Temperature: 40°C. Injection volume: 4000µl.
35.3 mg of the racemate are separated by the method described above to give 13 mg of the R-enantiomer Example 148a and 14 mg of the S-enantiomer. LC-MS (QC): $t_R$ = 0.561; $[M+H]^+$ = 388.2.

**Example 149: *rac*-4-{4-[(2-Cyclopropyl-imidazo[5,1-*b*]thiazol-3-yl)-hydroxy-methyl]-[1,2,3]triazol-1-yl}-2,6-difluoro-phenol**

Step 1: Preparation of 4-azido-2,6-difluorophenol

Prepared according to the procedure described for Example 45, step 1 using (3,5-difluoro-4-hydroxyphenyl)boronic acid.
Step 2: Prepared following the procedure described for Example 45, and using Intermediate 1, *rac*-1-(2-cyclopropylimidazo[5,1-*b*)]thiazol-3-yl)prop-2-yn-1-ol and 4-azido-2,6-difluorophenol. Purification by prepHPLC (basic conditions) to give *rac*-4-{4-[(2-cyclopropyl-imidazo[5,1-*b*]thiazol-3-yl)-hydroxy-methyl]-[1,2,3]triazol-1-yl}-2,6-difluoro-phenol. LC-MS (QC): $t_R$ = 0.545; $[M+H]^+$ = 390.2.

**Example 150: *rac*-4-{4-[(2-Cyclopropyl-imidazo[5,1-*b*]thiazol-3-yl)-hydroxy-methyl]-[1,2,3]triazol-1-yl)-2-fluoro-phenol**

Step 1: Preparation of 4-azido-2-fluorophenol

Prepared according to the procedure described for Example 45, step 1 using (3,5-difluoro-4-hydroxyphenyl)boronic acid.

Step 2: Prepared following the procedure described for Example 45, and using Intermediate 1, *rac*-1-(2-cyclopropylimidazo[5,1-*b*)]thiazol-3-yl)prop-2-yn-1-ol and 4-azido-2-fluorophenol. Purification by prepHPLC (basic conditions) to *rac*-4-{4-[(2-cyclopropyl-imidazo[5,1-*b*]thiazol-3-yl)-hydroxy-methyl]-[1,2,3]triazol-1-yl}-2-fluoro-phenol. LC-MS (QC): $t_R$ = 0.512; $[M+H]^+$ = 372.2.

**Example 151: *rac*-(4-{4-[(2-Cyclopropyl-imidazo[5,1-*b*]thiazol-3-yl)-hydroxy-methyl]-[1,2,3]triazol-1-yl)-3-fluoro-phenyl)-carbamic acid tert-butyl ester**

Step 1: Preparation of *tert*-butyl (4-azido-3-fluorophenyl)carbamate

Prepared according to the procedure described for Example 45, step 1 using (4-((*tert*-butoxycarbonyl)amino)-2-fluorophenyl)boronic acid.

Step 2: Prepared following the procedure described for Example 45, and using Intermediate 1, *rac*-1-(2-cyclopropylimidazo[5,1-*b*)]thiazol-3-yl)prop-2-yn-1-ol and *tert*-butyl-(4-azido-3-fluorophenyl)carbamate. Purification by prepHPLC (basic conditions) to rac-(4-{4-[(2-cyclopropyl-imidazo[5,1-b]thiazol-3-yl)-hydroxymethyl]-[1,2,3]triazol-1-yl}-3-fluoro-phenyl)-carbamic acid tert-butyl ester. LC-MS (QC): $t_R$ = 0.767; $[M+H]^+$ = 471.3.

**Example 152: *rac*-Biphenyl-4-yl-(2-cyclopropyl-imidazo[5,1-*b*]thiazol-3-yl)-methanol**

The product of Intermediate 1/Step 7, 2-cyclopropylimidazo[5,1-*b*]thiazole-3-carbaldehyde (96 mg; 0.5 mmol) is dissolved in THF (3.5 ml), cooled to 0°C and a solution of 4-biphenylmagnesium bromide in THF (0.5 M; 3 ml; 1.5 mmol) is slowly added and stirring is continued at 0°C for 30 minutes. The reaction is quenched by the addition of saturated aq. ammonium chloride solution and the product is extracted from the mixture with EtOAc (3x). The combined organic layers are dried over $MgSO_4$, filtered and the solvent is evaporated under reduced pressure. The residue is purified by reversed phase preparative HPLC to give *rac*-biphenyl-4-yl-(2-cyclopropyl-imidazo[5,1-*b*]thiazol-3-yl)-methanol. LC-MS (QC): $t_R$ = 0.808, $[M+H]^+$ = 347.2.

**Example 153: *rac*-Biphenyl-3-yl-(2-cyclopropyl-imidazo[5,1-*b*]thiazol-3-yl)-methanol**

Example 153 is prepared in analogy to the description of the preparation of example 152. LC-MS (QC): $t_R$ = 0.798, $[M+H]^+$ = 347.2.

**Example 154: *rac*-(2-Methyl-imidazo[5,1-*b*]thiazol-3-yl)-(5-phenyl-thiophen-2-yl)-methanol**

Example 154 is prepared in analogy to the description of the preparation of example 96 using Intermediate 11, 3-bromo-2-methylimidazo[5,1-*b*]thiazole, and 5-phenyl-2-thiophenecarboxaldehyde. LC-MS (QC): $t_R$ = 0.710; $[M+H]^+$ = 327.2.

**Example 155: *rac*-(2-Methyl-imidazo[5,1-*b*]thiazol-3-yl)-(2-phenyl-thiazol-5-yl)-methanol**

Example 154 is prepared in analogy to the description of the preparation of example 96 using Intermediate 11, 3-bromo-2-methylimidazo[5,1-*b*]thiazole, and 2-phenyl-1,3-thiazole-5-carbaldehyde. LC-MS (QC): $t_R$ = 0.596; $[M+H]^+$ = 328.1.

**Example 156: *rac*-(2-Methyl-imidazo[5,1-*b*]thiazol-3-yl)-(3-phenyl-isoxazol-5-yl)-methanol**

Example 156 is prepared in analogy to the description of the preparation of example 96 using Intermediate 11, 3-bromo-2-methylimidazo[5,1-*b*]thiazole, and 3-phenyl-5-isoxazolecarbaldehyde.. LC-MS (QC): $t_R$ = 0.583; $[M+H]^+$ = 312.2.

**Example 157: *rac*-(2-Cyclopropyl-imidazo[5,1-*b*]thiazol-3-yl)-(5-phenyl-[1,3,4]oxadiazol-2-yl)-methanol**

Example 157 is prepared in analogy to the description of the preparation of example 152. LC-MS (QC): $t_R$ = 0.587, $[M+H]^+$ = 339.2.

**Example 158: *rac*-(2-Cyclopropyl-imidazo[5,1-*b*]thiazol-3-yl)-(2-phenyl-2*H*-[1,2,3]triazol-4-yl)-methanol**

Example 158 is prepared in analogy to the description of the preparation of example 96 using Intermediate 10, 3-bromo-2-cyclopropylimidazo[5,1-*b*]thiazole, and 5-phenyl-2-thiophenecarboxaldehyde. LC-MS (QC): $t_R$ = 0.676, $[M+H]^+$ = 338.2.

**Example 159: *rac*-3-{5-[(2-Cyclopropyl-imidazo[5,1-*b*]thiazol-3-yl)-hydroxy-methyl]-thiophen-2-yl}-benzonitrile**

Example 159 is prepared in analogy to the description of the preparation of example 152. LC-MS (QC): $t_R$ = ,0.761 $[M+H]^+$ = 378.2.

**Example 160: *rac*-(2-Cyclopropyl-imidazo[5,1-*b*]thiazol-3-yl)-(5-pyridin-3-yl-thiophen-2-yl)-methanol**

Example 160 is prepared in analogy to the description of the preparation of example 152. LC-MS (QC): $t_R$ = 0.514, $[M+H]^+$ = 354.1.

**Example 161: *rac*-(2-Cyclopropyl-imidazo[5,1-*b*]thiazol-3-yl)-[5-(4-methoxy-phenyl)-thiophen-2-yl]-methanol**

Example 161 is prepared in analogy to the description of the preparation of example 152. LC-MS (QC): $t_R$ = 0.805, $[M+H]^+$ = 383.2.

**Example 162: *rac*-(2-Cyclopropyl-imidazo[5,1-*b*]thiazol-3-yl)-[5-(4-trifluoromethyl-phenyl)-thiophen-2-yl]-methanol**

Example 162 is prepared in analogy to the description of the preparation of example 152. LC-MS (QC): $t_R$ = 0.939, $[M+H]^+$ = 421.2.

**Example 163: *rac*-(2-Cyclopropyl-imidazo[5,1-*b*]thiazol-3-yl)-(3-phenyl-[1,2,4]oxadiazol-5-yl)-methanol**

Example 163 is prepared in analogy to the description of the preparation of example 152. LC-MS (QC): $t_R$ = 0.693, $[M+H]^+$ = 339.2.

**Example 164: *rac*-(2-Cyclopropyl-imidazo[5,1-*b*]thiazol-3-yl)-(5-phenyl-thiophen-2-yl)-methanol**

Example 164 is prepared in analogy to the description of the preparation of example 152. LC-MS (QC): $t_R$ = 0.806, $[M+H]^+$ = 353.2.

**Example 165: *rac*-(2-Cyclopropyl-imidazo[5,1-*b*]thiazol-3-yl)-[5-(3-trifluoromethyl-phenyl)-thiophen-2-yl]-methanol**

Example 165 is prepared in analogy to the description of the preparation of example 152. LC-MS (QC): $t_R$ = 0.927, $[M+H]^+$ = 420.9.

**Example 166: *rac*-(2-Cyclopropyl-imidazo[5,1-*b*]thiazol-3-yl)-[5-(2-methoxy-phenyl)-thiophen-2-yl]-methanol**

Example 166 is prepared in analogy to the description of the preparation of example 152. LC-MS (QC): $t_R$ = 0.805, $[M+H]^+$ = 383.2.

**Example 167: *rac*-(2-Cyclopropyl-imidazo[5,1-*b*]thiazol-3-yl)-[5-(3-methoxy-phenyl)-thiophen-2-yl]-methanol**

Example 167 is prepared in analogy to the description of the preparation of example 152. LC-MS (QC): $t_R$ = 0.811, $[M+H]^+$ = 383.0.

**Example 168: *rac*-(2-Cyclopropyl-imidazo[5,1-*b*]thiazol-3-yl)-(5-pyridin-4-yl-thiophen-2-yl)-methanol**

Example 168 is prepared in analogy to the description of the preparation of example 152. LC-MS (QC): $t_R$ = 0.390, $[M+H]^+$ = 354.0.

**Example 169: *rac*-(2-Cyclopropyl-imidazo[5,1-*b*]thiazol-3-yl)-[5-(2-methyl-2*H*-pyrazol-3-yl)-thiophen-2-yl]-methanol**

Example 169 is prepared in analogy to the description of the preparation of example 152. LC-MS (QC): $t_R$ = 0.592, $[M+H]^+$ = 357.2.

**Example 170: *rac*-(2-Cyclopropyl-imidazo[5,1-*b*]thiazol-3-yl)-[5-(2-trifluoromethyl-phenyl)-thiophen-2-yl]-methanol**

Example 170 is prepared in analogy to the description of the preparation of example 152. LC-MS (QC): $t_R$ = 0.889, $[M+H]^+$ = 421.0.

**Example 171: *rac*-(2-Cyclopropyl-imidazo[5,1-*b*]thiazol-3-yl)-(5-phenyl-thiazol-2-yl)-methanol**

Example 171 is prepared in analogy to the description of the preparation of example 96 using Intermediate 10, 3-bromo-2-cyclopropylimidazo[5,1-*b*]thiazole, and 5-phenyl-1,3-thiazole-2-carbaldehyde. LC-MS (acidic): $t_R$ = 0.74, $[M+H]^+$ = 353.72.

**Example 172: *rac*-2-{5-[(2-Cyclopropyl-imidazo[5,1-*b*]thiazol-3-yl)-hydroxy-methyl]-thiophen-2-yl}-benzonitrile**

Example 172 is prepared in analogy to the description of the preparation of example 152. LC-MS (QC): $t_R$ = 0.745, $[M+H]^+$ = 378.2.

**Example 173: *rac*-4-{5-[(2-Cyclopropyl-imidazo[5,1-*b*]thiazol-3-yl)-hydroxy-methyl]-thiophen-2-yl}-benzonitrile**

Example 173 is prepared in analogy to the description of the preparation of example 152. LC-MS (QC): $t_R$ = 0.759, $[M+H]^+$ = 378.2.

**Example 174: *rac*-(2-Cyclopropyl-imidazo[5,1-*b*]thiazol-3-yl)-[5-(3-methyl-3*H*-[1,2,3]triazol-4-yl)-thiophen-2-yl]-methanol**

Example 174 is prepared in analogy to the description of the preparation of example 152. LC-MS (QC): $t_R$ = 0.519, $[M+H]^+$ = 358.0.

**Example 175: *rac*-(2-Cyclopropyl-imidazo[5,1-*b*]thiazol-3-yl)-(5-phenyl-[1,3,4]thiadiazol-2-yl)-methanol**

Example 175 is prepared in analogy to the description of the preparation of example 152. LC-MS (QC): $t_R$ = 0.637, $[M+H]^+$ = 355.1.

**Example 176: *rac*-(2-Cyclopropyl-imidazo[5,1-*b*]thiazol-3-yl)-[5-(1*H*-indol-5-yl)-thiophen-2-yl]-methanol**

Example 176 is prepared in analogy to the description of the preparation of example 152. LC-MS (QC): $t_R$ = 0.754, $[M+H]^+$ = 392.3.

**Example 177: *rac*-(2-Cyclopropyl-imidazo[5,1-*b*]thiazol-3-yl)-(5-isoquinolin-4-yl-thiophen-2-yl)-methanol**

Example 177 is prepared in analogy to the description of the preparation of example 152. LC-MS (QC): $t_R$ = 0.650, $[M+H]^+$ = 404.1.

**Example 178: *rac*-(2-Cyclopropyl-imidazo[5,1-**b**]thiazol-3-yl)-(1-phenyl-1*H*-imidazol-4-yl)-methanol**

Example 178 is prepared in analogy to the description of the preparation of example 96 using Intermediate 10, 3-bromo-2-cyclopropylimidazo[5,1-*b*]thiazole, and 1-phenyl-1*H*-imidazole-4-carbaldehyde. LC-MS (QC): $t_R$ = 0.524,

$[M+H]^+$ = 337.0.

**Example 49a: (4-{4-[(*R*)-(2-Ethyl-imidazo[5,1-*b*]thiazol-3-yl)-hydroxy-methyl]-[1,2,3]triazol-1-yl)-phenyl)-carbamic acid methyl ester**

Separation of the enantiomers on chiral stationary phase:
Method: Column: ChiralCel OD-H 30x250mm, 5μM; Detector Settings: UV-Vis-1; 210 nM; Eluent: 65% $CO_2$ and 35% EtOH/$CH_3$CN 1:1; Flow: 160.00 ml/min; BPR: 100 bar; Temperature: 40°C. Injection volume: 1000μl. 15 mg of the racemate are separated by the method described above to give 6.5 mg of the *R*-enantiomer Example 49a and 5.9 mg of the S-enantiomer. LC-MS (QC): $t_R$ = 0.551; $[M+H]^+$ = 399.3.

**Example 87a: (*R*)-(2-Cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol**

Separation of the enantiomers on chiral stationary phase:
Method: Column: ChiralCel OD-H 30x250mm, 5μM; Detector Settings: UV-Vis-1; 265 nM; Eluent: 65% $CO_2$ and 35% EtOH/$CH_3$CN 1:1; Flow: 160.00 ml/min; BPR: 100 bar; Temperature: 40°C. Injection volume: 2500μl.

9.8 mg of the racemate are separated by the method described above to give 2.7 mg of the *R*-enantiomer Example 87a and 3.0 mg of the *S*-enantiomer. LC-MS (QC): $t_R$ = 0.705; $[M+H]^+$ = 452.2.

**Example 101a: (*R*)-(2-Cyclopropyl-imidazo[5,1-*b*]thiazol-3-yl)-(5-methyl-1-phenyl-1*H*-[1,2,3]triazol-4-yl)-methanol**

Separation of the enantiomers on chiral stationary phase:
Method: Column: ChiralPak AS-H 30x250mm, 5μM; Detector Settings: UV-Vis-1; 280 nM; Eluent: 75% $CO_2$ and 25% EtOH 0.1% DEA; Flow: 160.00 ml/min; BPR: 120 bar; Temperature: 40°C. Injection volume: 2000μl.

15.9 mg of the racemate are separated by the method described above to give 4.7 mg of the *R*-enantiomer Example 101a and 3.3 mg of the *S*-enantiomer. LC-MS (QC): $t_R$ = 0.595; $[M+H]^+$ = 352.3.

**Example 139a: 1-(4-{4-[(*R*)-(2-Cyclopropyl-imidazo[5,1-*b*]thiazol-3-yl)-hydroxy-methyl]-[1,2,3]triazol-1-yl}-phenoxy)-2-methyl-propan-2-ol**

Separation of the enantiomers on chiral stationary phase:
Method: Column: ChiralPak IB 30x250mm, 5μM; Detector Settings: UV-Vis-1; 264 nM; Eluent: 65% $CO_2$ and 35% EtOH/$CH_3$CN 1:1; Flow: 160.00 ml/min; BPR: 100 bar; Temperature: 40°C. Injection volume: 1000μl.

30 mg of the racemate are separated by the method described above to give 11.4 mg of the *R*-enantiomer Example 139a and 11.0 mg of the *S*-enantiomer. LC-MS (QC): $t_R$ = 0.613; $[M+H]^+$ = 426.2.

**Example 179: *rac*-6-{4-[(2-Cyclopropyl-imidazo[5,1-*b*]thiazol-3-yl)-hydroxy-methyl]-[1,2,3]triazol-1-yl}-3*H*-benzooxazol-2-one**

Step 1: Preparation of 6-azidobenzo[*d*]oxazol-2(3*H*)-one

Prepared according to the procedure described for Example 45, step 1 using (2-oxo-2,3-dihydrobenzo[*d*]oxazol-6-yl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 6-azidobenzo[*d*]oxazol-2(3*H*)-one. Purification by preparative HPLC (basic conditions) to give *rac*-6-{4-[(2-cyclopropyl-imidazo[5,1-*b*]thiazol-3-yl)-hydroxy-methyl]-[1,2,3]triazol-1-yl}-3*H*-benzooxazol-2-one. LC-MS (QC): $t_R$ = 0.503; $[M+H]^+$ = 395.3.

**Example 180: *rac*-(4-{4-[(2-Cyclopropyl-imidazo[5,1-*b*]thiazol-3-yl)-hydroxy-methyl]-[1,2,3]triazol-1-yl}-phenyl)-carbamic acid tert-butyl ester**

Step 1: Preparation of *tert*-butyl (4-azidophenyl)carbamate

Prepared according to the procedure described for Example 45, step 1 using (4-((*tert*-butoxycarbonyl)amino)phenyl)boronic acid.

Step 2: Prepared according to the procedure described for Example 45, step 2 and using intermediate 1, *rac*-

1-(2-cyclopropylimidazo[5,1-*b*)]thiazol-3-yl)prop-2-yn-1-ol and *tert*-butyl (4-azidophenyl)carbamate. Purification by preparative HPLC (basic conditions) to give *rac*-(4-{4-[(2-cyclopropyl-imidazo[5,1-*b*]thiazol-3-yl)-hydroxymethyl]-[1,2,3]triazol-1-yl}-phenyl)-carbamic acid tert-butyl ester. LC-MS (QC): $t_R$ = 0.756; $[M+H]^+$ = 453.2.

**Example 181: *rac*-6-{4-[(2-Ethyl-imidazo[5,1-*b*]thiazol-3-yl)-hydroxy-methyl]-[1,2,3]triazol-1-yl}-3*H*-benzooxazol-2-one**

Prepared according to the procedure described for Example 179 and using intermediate 3, *rac-1-(2*-ethylimidazo[5,1-*b*]thiazol-3-yl)prop-2-yn-1-ol. Purification by preparative HPLC (basic conditions) gives *rac*-6-{4-[(2-ethyl-imidazo[5,1-*b*]thiazol-3-yl)-hydroxy-methyl]-[1,2,3]triazol-1-yl}-3*H*-benzooxazol-2-one. LC-MS (QC): $t_R$ = 0.479; $[M+H]^+$ = 383.2.

**Example 182: *rac*-4-{4-[(2-Cyclopropyl-imidazo[5,1-*b*]thiazol-3-yl)-hydroxy-methyl]-[1,2,3]triazol-1-yl}-*N*-methylbenzamide**

Step 1: Preparation of 4-azido-*N*-methylbenzamide

Prepared according to the procedure described for Example 45, step 1 using (4-(methylcarbamoyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-*N*-methylbenzamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*-methyl-benzamide. LC-MS (QC): $t_R$ = 0.488; $[M+H]^+$ = 395.3.

**Example 183: *rac*-1-(4-{4-[(2-Cyclopropyl-imidazo[5,1-*b*]thiazol-3-yl)-hydroxy-methyl]-[1,2,3]triazol-1-yl}-2-methoxy-phenyl)-3-ethyl-urea**

Step 1: Preparation of 1-(4-azido-2-methoxyphenyl)-3-ethylurea

Prepared according to the procedure described for Example 45, step 1 using (4-(3-ethylureido)-3-methoxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 1-(4-azido-2-methoxyphenyl)-3-ethylurea. Purification by preparative HPLC (basic conditions) to give *rac*-1-(4-{4-[(2-cyclopropyl-imidazo[5,1-*b*]thiazol-3-yl)-hydroxy-methyl]-[1,2,3]triazol-1-yl}-2-methoxy-phenyl)-3-ethyl-urea. LC-MS (QC): $t_R$ = 0.593; $[M+H]^+$ = 454.3.

**Example 184: *rac*-4-{4-[(2-Cyclopropyl-imidazo[5,1-*b*]thiazol-3-yl)-hydroxy-methyl]-[1,2,3]triazol-1-yl)-*N*-(3-hydroxy-propyl)-benzamide**

Step 1: Preparation of 4-azido-*N*-(3-hydroxypropyl)benzamide

Prepared according to the procedure described for Example 45, step 1 using (4-((3-hydroxypropyl)carbamoyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-*N*-(3-hydroxypropyl)benzamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*-(3-hydroxy-propyl)-benzamide. LC-MS (QC): $t_R$ = 0.481; $[M+H]^+$ = 439.3.

**Example 185: *rac*-4-{4-[(2-Cyclopropyl-imidazo[5,1-*b*]thiazol-3-yl)-hydroxy-methyl]-[1,2,3]triazol-1-yl}-benzamide**

Step 1: Preparation of 4-azidobenzamide

Prepared according to the procedure described for Example 45, step 1 using (4-carbamoylphenyl)boronic acid. Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azidobenzamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-benzamide. LC-MS (QC): $t_R$ = 0.452; $[M+H]^+$ = 381.1.

**Example 186: *rac*-4-{4-[(2-Cyclopropyl-imidazo[5,1-*b*]thiazol-3-yl)-hydroxy-methyl]-[1,2,3]triazol-1-yl)-*N*-(2-hydroxy-ethyl)-benzamide**

Step 1: Preparation of 4-azido-*N*-(2-hydroxyethyl)benzamide

Prepared according to the procedure described for Example 45, step 1 using (4-((2-hydroxyethyl)carbamoyl)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 4-azido-*N*-(2-hydroxyethyl)benzamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*-(2-hydroxy-ethyl)-benzamide. LC-MS (QC): $t_R$ = 0.459; $[M+H]^+$ = 425.3.

**Example 187: *rac-N*-(4-{4-[(2-Cyclopropyl-imidazo[5,1-*b*]thiazol-3-yl)-hydroxy-methyl]-[1,2,3]triazol-1-yl}-phenyl)-2,2-dimethyl-propionamide**

Step 1: Preparation of *N*-(4-azidophenyl)pivalamide

Prepared according to the procedure described for Example 45, step 1 using (4-pivalamidophenyl)boronic acid. Step 2: Prepared according to the procedure described for Example 45, step 2 and using intermediate 1, *rac*-1-(2-cyclopropylimidazo[5,1-*b*)]thiazol-3-yl)prop-2-yn-1-ol and *N*-(4-azidophenyl)pivalamide. Purification by preparative HPLC (basic conditions) to give *rac*-*N*-(4-{4-[(2-cyclopropyl-imidazo[5,1-*b*]thiazol-3-yl)-hydroxy-methyl]-[1,2,3]triazol-1-yl}-phenyl)-2,2-dimethyl-propionamide. LC-MS (QC): $t_R$ = 0.684; $[M+H]^+$ = 437.2.

**Example 188: *rac*-1-Butyl-3-(4-{4-[(2-cyclopropyl-imidazo[5,1-*b*]thiazol-3-yl)-hydroxy-methyl]-[1,2,3]triazol-1-yl}-phenyl)-urea**

Step 1: Preparation of 1-(4-azidophenyl)-3-butylurea

Prepared according to the procedure described for Example 45, step 1 using (4-(3-butylureido)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 1-(4-azidophenyl)-3-butylurea. Purification by preparative HPLC (basic conditions) to give *rac*-1-butyl-3-(4-{4-[(2-cyclopropyl-imidazo[5,1-*b*]thiazol-3-yl)-hydroxy-methyl]-[1,2,3]triazol-1-yl}-phenyl)-urea. LC-MS (QC): $t_R$ = 0.663; $[M+H]^+$ = 452.2.

**Example 189: *rac-N*-(4-{4-[(2-Cyclopropyl-imidazo[5,1-*b*]thiazol-3-yl)-hydroxy-methyl]-[1,2,3]triazol-1-yl}-phenyl)-benzamide**

Step 1: Preparation of *N*-(4-azidophenyl)benzamide

Prepared according to the procedure described for Example 45, step 1 using (4-benzamidophenyl)boronic acid. Step 2: Prepared according to the procedure described for Example 45, step 2 and using intermediate 1, *rac*-1-(2-cyclopropylimidazo[5,1-*b*)]thiazol-3-yl)prop-2-yn-1-ol and *N*-(4-azidophenyl)benzamide. Purification by preparative HPLC (basic conditions) to give *rac*-*N*-(4-{4-[(2-cyclopropyl-imidazo[5,1-*b*]thiazol-3-yl)-hydroxy-methyl]-[1,2,3]triazol-1-yl}-phenyl)-benzamide. LC-MS (QC): $t_R$ = 0.686; $[M+H]^+$ = 457.3.

**Example 190: *rac*-4-{4-[(2-Cyclopropyl-imidazo[5,1-*b*]thiazol-3-yl)-hydroxy-methyl]-[1,2,3]triazol-1-yl}-*N*-furan-2-ylmethyl-benzamide**

Step 1: Preparation of 4-azido-*N*-(furan-2-ylmethyl)benzamide

Prepared according to the procedure described for Example 45, step 1 using (4-((furan-2-ylmethyl)carbamoyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-*N*-(furan-2-ylmethyl)benzamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*-furan-2-ylmethyl-benzamide. LC-MS (QC): $t_R$ = 0.619; $[M+H]^+$ = 461.2.

**Example 191: *rac*-4-{4-[(2-Cyclopropyl-imidazo[5,1-*b*]thiazol-3-yl)-hydroxy-methyl]-[1,2,3]triazol-1-yl}-*N*-ethyl-*N*-methyl-benzamide**

Step 1: Preparation of 4-azido-*N*-ethyl-*N*-methylbenzamide

Prepared according to the procedure described for Example 45, step 1 using (4-(ethyl(methyl)carbamoyl)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 4-azido-*N*-ethyl-*N*-methylbenzamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*-ethyl-*N*-methyl-benzamide. LC-MS (QC): $t_R$ = 0.576; $[M+H]^+$ = 423.4.

**Example 192: *rac-N*-(4-{4-[(2-Cyclopropyl-imidazo[5,1-*b*]thiazol-3-yl)-hydroxy-methyl]-[1,2,3]triazol-1-yl}-phenyl)-isobutyramide**

Step 1: Preparation of *N*-(4-azidophenyl)isobutyramide

Prepared according to the procedure described for Example 45, step 1 using (4-isobutyramidophenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and *N*-(4-azidophenyl)isobutyramide. Purification by preparative HPLC (basic conditions) to give *rac-N*-(4-{4-[(2-cyclopropyl-imidazo[5,1-*b*]thiazol-3-yl)-hydroxy-methyl]-[1,2,3]triazol-1-yl}-phenyl)-isobutyramide. LC-MS (QC): $t_R$ = 0.614; $[M+H]^+$ = 423.4.

**Example 192a: *N*-(4-{4-[(*R*)-(2-Cyclopropyl-imidazo[5,1-*b*]thiazol-3-yl)-hydroxy-methyl]-[1,2,3]triazol-1-yl}-phenyl)-isobutyramide**

Separation of the enantiomers on chiral stationary phase:
Method: Column: ChiralCel OD-H 30x250mm, 5μM; Detector Settings: UV-Vis-1; 210 nM; Eluent: 65% $CO_2$ and 35% $EtOH/CH_3CN$ 1:1; Flow: 160.00 ml/min; BPR: 100 bar; Temperature: 40°C. Injection volume: 1000μl.

21 mg of the racemate are separated by the method described above to give 10 mg of the *R*-enantiomer Example 192a and 8.3 mg of the *S*-enantiomer. LC-MS (QC): $t_R$ = 0.612; $[M+H]^+$ = 423.3.

**Example 193: *rac-N*-Allyl-4-{4-[(2-cyclopropyl-imidazo[5,1-*b*]thiazol-3-yl)-hydroxy-methyl]-[1,2,3]triazol-1-yl}-benzamide**

Step 1: Preparation of *N*-allyl-4-azidobenzamide

Prepared according to the procedure described for Example 45, step 1 using (4-(allylcarbamoyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and *N*-allyl-4-azidobenzamide. Purification by preparative HPLC (basic conditions) to give *rac-N*-allyl-4-{4-[(2-cyclopropyl-imidazo[5,1-*b*]thiazol-3-yl)-hydroxy-methyl]-[1,2,3]triazol-1-yl}-benzamide. LC-MS (QC): $t_R$ = 0.567; $[M+H]^+$ = 421.2.

**Example 194: *rac*-5-{4-[(2-Cyclopropyl-imidazo[5,1-*b*]thiazol-3-yl)-hydroxy-methyl]-[1,2,3]triazol-1-yl}-1,3-dihydro-benzoimidazol-2-one**

Step 1: Preparation of 5-azido-1,3-dihydro-2*H*-benzo[*d*]imidazol-2-one

Prepared according to the procedure described for Example 45, step 1 using (2-oxo-2,3-dihydro-1*H*-benzo[d]imidazol-5-yl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 5-azido-1,3-dihydro-2*H*-benzo[*d*]imidazol-2-one. Purification by preparative HPLC (basic conditions then acidic conditions) to give *rac*-5-{4-[(2-cyclopropyl-imidazo[5,1-*b*]thiazol-3-yl)-hydroxy-methyl]-[1,2,3]triazol-1-yl}-1,3-dihydro-benzoimidazol-2-one. LC-MS (QC): $t_R$ = 0.450; $[M+H]^+$ = 394.3.

**Example 195: *rac*-4-{4-[(2-Cyclopropyl-imidazo[5,1-*b*]thiazol-3-yl)-hydroxy-methyl]-[1,2,3]triazol-1-yl}-*N*,*N*-dimethyl-benzamide**

Step 1: Preparation of 4-azido-*N,N*-dimethylbenzamide

Prepared according to the procedure described for Example 45, step 1 using (4-(dimethylcarbamoyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-*N*,*N*-dimethylbenzamide. Purification by preparative HPLC (basic conditions then acidic conditions) to give *rac*-4-{4-[(2-cyclopropyl-imidazo[5,1-*b*]thiazol-3-yl)-hydroxy-methyl]-[1,2,3]triazol-1-yl}-*N*,*N*-dimethyl-benzamide. LC-MS (QC): $t_R$ = 0.525; $[M+H]^+$ = 409.4.

**Example 196: *rac*-4-{4-[(2-Ethyl-imidazo[5,1-*b*]thiazol-3-yl)-hydroxy-methyl]-[1,2,3]triazol-1-yl}-*N*-(2-methoxy-ethyl)-benzamide**

Prepared according to the procedure described for Example 102 and using intermediate 3, *rac*-1-(2-ethylimidazo[5,1-*b*)]thiazol-3-yl)prop-2-yn-1-ol. Purification by preparative HPLC (acidic conditions) to give *rac*-4-{4-[(2-ethyl-imidazo[5,1-*b*]thiazol-3-yl)-hydroxy-methyl]-[1,2,3]triazol-1-yl}-*N*-(2-methoxy-ethyl)-benzamide. LC-MS (QC): $t_R$ = 0.505; $[M+H]^+$ = 427.3.

**Example 197: *rac*-3-{4-[(2-Cyclopropyl-imidazo[5,1-*b*]thiazol-3-yl)-hydroxy-methyl]-[1,2,3]triazol-1-yl}-5-methoxy-benzonitrile**

Step 1: Preparation of 3-azido-5-methoxybenzonitrile

Prepared according to the procedure described for Example 45, step 1 using (3-cyano-5-methoxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 3-azido-5-methoxybenzonitrile. Purification by preparative HPLC (basic conditions) to give *rac*-3-{4-[(2-cyclopropyl-imidazo[5,1-*b*]thiazol-3-yl)-hydroxy-methyl]-[1,2,3]triazol-1-yl}-5-methoxy-benzonitrile. LC-MS (QC): $t_R$ = 0.650; $[M+H]^+$ = 393.3.

**Example 198: *rac*-5-{4-[(2-Cyclopropyl-imidazo[5,1-*b*]thiazol-3-yl)-hydroxy-methyl]-[1,2,3]triazol-1-yl}-2,3-dimethoxy-benzonitrile**

Step 1: Preparation of 5-azido-2,3-dimethoxybenzonitrile

Prepared according to the procedure described for Example 45, step 1 using (3-cyano-4,5-dimethoxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 5-azido-2,3-dimethoxybenzonitrile. Purification by preparative HPLC (basic conditions) to give *rac*-5-{4-[(2-cyclopropyl-imidazo[5,1-*b*]thiazol-3-yl)-hydroxy-methyl]-[1,2,3]triazol-1-yl}-2,3-dimethoxy-benzonitrile. LC-MS (QC): $t_R$ = 0.666; $[M+H]^+$ = 423.3.

**Example 199: *rac*-5-{4-[(2-Cyclopropyl-imidazo[5,1-*b*]thiazol-3-yl)-hydroxy-methyl]-[1,2,3]triazol-1-yl}-2-trifluoromethoxy-benzonitrile**

Step 1: Preparation of 5-azido-2-(trifluoromethoxy)benzonitrile

Prepared according to the procedure described for Example 45, step 1 using (3-cyano-4-(trifluoromethoxy)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 5-azido-2-(trifluoromethoxy)benzonitrile. Purification by preparative HPLC (basic conditions) to give *rac*-5-{4-[(2-cyclopropyl-imidazo[5,1-*b*]thiazol-3-yl)-hydroxy-methyl]-[1,2,3]triazol-1-yl}-2-trifluoromethoxy-benzonitrile. LC-MS (QC): $t_R$ = 0.755; $[M+H]^+$ = 447.1.

**Example 200: *rac*-[1-(4-Bromo-3-chloro-pyridin-2-yl)-1*H*-[1,2,3]triazol-4-yl]-(2-cyclopropyl-imidazo[5,1-*b*]thiazol-3-yl)-methanol**

Step 1: Preparation of 2-azido-4-bromo-3-chloropyridine

Prepared according to the procedure described for Example 45, step 1 using (4-bromo-3-chloropyridin-2-yl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 2-azido-4-bromo-3-chloropyridine. Purification by preparative HPLC (basic conditions) to give *rac*-[1-(4-bromo-3-chloro-pyridin-2-yl)-1*H*-[1,2,3]triazol-4-yl]-(2-cyclopropyl-imidazo[5,1-*b*]thiazol-3-yl)-methanol. LC-MS (QC): $t_R$ = 0.648; $[M+H]^+$ = 451.1.

**Example 201: *rac*-2-{2-[2-(4-{4-[(2-Cyclopropyl-imidazo[5,1-*b*]thiazol-3-yl)-hydroxy-methyl]-[1,2,3]triazol-1-yl}-phenoxy)-ethoxy]-ethoxy}-ethanol**

Prepared following the procedure described for Example 87, and using 2-(2-(2-chloroethoxy)ethoxy)ethan-1-ol. Purification by preparative HPLC (basic conditions) gives *rac*-2-{2-[2-(4-{4-[(2-cyclopropyl-imidazo[5,1-*b*]thiazol-3-yl)-hydroxy-methyl]-[1,2,3]triazol-1-yl}-phenoxy)-ethoxy]-ethoxy}-ethanol. LC-MS (QC): $t_R$ = 0.567; $[M+H]^+$ = 486.1.

**Example 202: (*S*)-3-(4-{4-[(*R*)-(2-Cyclopropyl-imidazo[5,1-*b*]thiazol-3-yl)-hydroxy-methyl]-[1,2,3]triazol-1-yl}-phenoxy)-propane-1,2-diol and (*S*)-3-(4-{4-[(*S*)-(2-Cyclopropyl-imidazo[5,1-*b*]thiazol-3-yl)-hydroxy-methyl]-[1,2,3]triazol-1-yl}-phenoxy)-propane-1,2-diol**

Prepared following the procedure described for Example 87, and using (*S*)-3-chloropropane-1,2-diol. Purification by preparative HPLC (basic conditions) gives a mixture of (*S*)-3-(4-{4-[(R)-(2-cyclopropyl-imidazo[5,1-*b*]thiazol-3-yl)-hydroxy-methyl]-[1,2,3]triazol-1-yl}-phenoxy)-propane-1,2-diol and (*S*)-3-(4-{4-[(*S*)-(2-cyclopropyl-imidazo[5,1-*b*]thiazol-3-yl)-hydroxy-methyl]-[1,2,3]triazol-1-yl}-phenoxy)-propane-1,2-diol. LC-MS (QC): $t_R$ = 0.476; $[M+H]^+$ = 428.3.

**Example 203: *rac*-(2-Cyclopropyl-imidazo[5,1-*b*]thiazol-3-yl)-{1-[4-(pyridin-2-ylmethoxy)-phenyl]-1*H*-[1,2,3]triazol-4-yl}-methanol**

Prepared following the procedure described for Example 87, and using 2-(bromomethyl)pyridine. Purification by preparative HPLC (basic conditions) gives *rac*-(2-cyclopropyl-imidazo[5,1-*b*]thiazol-3-yl)-{1-[4-(pyridin-2-ylmethoxy)-phenyl]-1*H*-[1,2,3]triazol4-yl}-methanol. LC-MS (QC): $t_R$ = 0.548; $[M+H]^+$ = 445.3.

**Example 204: *rac*-(2-Cyclopropyl-imidazo[5,1-*b*]thiazol-3-yl)-{1-[4-(2-dimethylamino-ethoxy)-phenyl]-1*H*-[1,2,3]triazol-4-yl}-methanol**

Prepared following the procedure described for Example 87, and using 2-chloro-*N*,*N*-dimethylethan-1-amine. Purification by preparative HPLC (basic conditions) gives *rac*-(2-cyclopropyl-imidazo[5,1-*b*]thiazol-3-yl)-{1-[4-(2-dimethylamino-ethoxy)-phenyl]-1*H*-[1,2,3]triazol-4-yl}-methanol. LC-MS (QC): $t_R$ = 0.352; $[M+H]^+$ = 425.3.

**Example 205: *rac*-(2-Cyclopropyl-imidazo[5,1-*b*]thiazol-3-yl)-{1-[4-(2-methanesulfonyl-ethoxy)-phenyl]-1*H*-[1,2,3]triazol-4-yl}-methanol**

Prepared following the procedure described for Example 87, and using 1-chloro-2-(methylsulfonyl)ethane. Purification by preparative HPLC (basic conditions) gives *rac*-(2-cyclopropyl-imidazo[5,1-*b*]thiazol-3-yl)-{1-[4-(2-methanesulfonyl-ethoxy)-phenyl]-1*H*-[1,2,3]triazol-4-yl}-methanol. LC-MS (QC): $t_R$ = 0.549; $[M+H]^+$ = 460.3.

**Example 206: *rac*-3-(4-{4-[(2-Cyclopropyl-imidazo[5,1-*b*]thiazol-3-yl)-hydroxy-methyl]-[1,2,3]triazol-1-yl}-phenoxy)-propane-1-sulfonic acid amide**

Prepared following the procedure described for Example 87, and using 3-bromopropane-1-sulfonamide. Purification by preparative HPLC (basic conditions) gives *rac*-3-(4-{4-[(2-cyclopropyl-imidazo[5,1-*b*]thiazol-3-yl)-hydroxy-methyl]-[1,2,3]triazol-1-yl}-phenoxy)-propane-1-sulfonic acid amide. LC-MS (QC): $t_R$ = 0.541; $[M+H]^+$ = 475.3.

**Example 207: *rac*-4-{4-[(2-Cyclopropyl-imidazo[5,1-*b*]thiazol-3-yl)-hydroxy-methyl]-[1,2,3]triazol-1-yl}-3-methyl-phenol**

Step 1: Preparation of 4-azido-3-methylphenol

Prepared according to the procedure described for Example 45, step 1 using (4-hydroxy-2-methylphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-3-methylphenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-3-methyl-phenol. LC-MS (QC): $t_R$ = 0.520; $[M+H]^+$ = 368.1.

**Example 208: *rac*-4-{4-[(2-Cyclopropyl-imidazo[5,1-*b*]thiazol-3-yl)-hydroxy-methyl]-[1,2,3]triazol-1-yl}-3-fluoro-phenol**

Step 1: Preparation of 4-azido-3-fluorophenol

Prepared according to the procedure described for Example 45, step 1 using (2-fluoro-4-hydroxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-3-fluorophenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-3-fluoro-phenol. LC-MS (QC): $t_R$ = 0.530; $[M+H]^+$ = 372.3.

**Example 209: *rac*-4-{4-[(2-Cyclopropyl-imidazo[5,1-*b*]thiazol-3-yl)-hydroxy-methyl]-[1,2,3]triazol-1-yl}-2-trifluoromethyl-phenol**

Step 1: Preparation of 4-azido-2-(trifluoromethyl)phenol

Prepared according to the procedure described for Example 45, step 1 using (4-hydroxy-3-(trifluoromethyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-2-(trifluoromethyl)phenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-2-trifluoromethyl-phenol. LC-MS (QC): $t_R$ = 0.656; $[M+H]^+$ = 422.1.

**Example 210: *rac*-4-{4-[(2-Cyclopropyl-imidazo[5,1-*b*]thiazol-3-yl)-hydroxy-methyl]-[1,2,3]triazol-1-yl}-2-trifluoromethoxy-phenol**

Step 1: Preparation of 4-azido-2-(trifluoromethoxy)phenol

Prepared according to the procedure described for Example 45, step 1 using (4-hydroxy-3-(trifluoromethoxy)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 4-azido-2-(trifluoromethoxy)phenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-2-trifluoromethoxy-phenol. LC-MS (QC): $t_R$ = 0.660; $[M+H]^+$ = 438.3.

**Example 211: *rac*-4-{4-[(2-Cyclopropyl-imidazo[5,1-*b*]thiazol-3-yl)-hydroxy-methyl]-[1,2,3]triazol-1-yl}-2-methyl-phenol**

Step 1: Preparation of 4-azido-2-methylphenol

Prepared according to the procedure described for Example 45, step 1 using (4-hydroxy-3-methylphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-2-methylphenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-2-methyl-phenol. LC-MS (QC): $t_R$ = 0.563; $[M+H]^+$ = 368.3.

**Example 212: *rac*-3-Chloro-4-{4-[(2-cyclopropyl-imidazo[5,1-*b*]thiazol-3-yl)-hydroxy-methyl]-[1,2,3]triazol-1-yl}-phenol**

Step 1: Preparation of 4-azido-3-chlorophenol

Prepared according to the procedure described for Example 45, step 1 using (2-chloro-4-hydroxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-3-chlorophenol. Purification by preparative HPLC (basic conditions) to give *rac*-3-chloro-4-{4-[(2-cyclopropyl-imidazo[5,1-*b*]thiazol-3-yl)-hydroxy-methyl]-[1,2,3]triazol-1-yl}-phenol. LC-MS (QC): $t_R$ = 0.556; $[M+H]^+$ = 388.2.

**Example 213: *rac*-4-{4-[(2-Cyclopropyl-imidazo[5,1-*b*]thiazol-3-yl)-hydroxy-methyl]-[1,2,3]triazol-1-yl}-3-trifluoromethoxy-phenol**

Step 1: Preparation of 4-azido-3-(trifluoromethoxy)phenol

Prepared according to the procedure described for Example 45, step 1 using (4-hydroxy-2-(trifluoromethoxy)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-3-(trifluoromethoxy)phenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-3-trifluoromethoxy-phenol. LC-MS (QC): $t_R$ = 0.627; $[M+H]^+$ = 438.3.

**Example 214: *rac*-4-{4-[(2-Cyclopropyl-imidazo[5,1-*b*]thiazol-3-yl)-hydroxy-methyl]-[1,2,3]triazol-1-yl}-3-trifluoromethyl-phenol**

Step 1: Preparation of 4-azido-3-(trifluoromethyl)phenol

Prepared according to the procedure described for Example 45, step 1 using (4-hydroxy-2-(trifluoromethyl)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 4-azido-3-(trifluoromethyl)phenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-3-trifluoromethyl-phenol. LC-MS (QC): $t_R$ = 0.601; $[M+H]^+$ = 422.1.

**Example 215: *rac*-3-{4-[(2-Cyclopropyl-imidazo[5,1-*b*]thiazol-3-yl)-hydroxy-methyl]-[1,2,3]triazol-1-yl}-phenol**

Step 1: Preparation of 3-azidophenol

Prepared according to the procedure described for Example 45, step 1 using (3-hydroxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 3-azidophenol. Purification by preparative HPLC (basic conditions) to give *rac*-3-{4-[(2-cyclopropyl-imidazo[5,1-*b*]thiazol-3-yl)-hydroxy-methyl]-[1,2,3]triazol-1-yl}-phenol. LC-MS (QC): $t_R$ = 0.526; $[M+H]^+$ = 354.2.

**Example 216: *rac*-5-{4-[(2-Cyclopropyl-imidazo[5,1-*b*]thiazol-3-yl)-hydroxy-methyl]-[1,2,3]triazol-1-yl}-2-methoxy-phenol**

Step 1: Preparation of 5-azido-2-methoxyphenol

Prepared according to the procedure described for Example 45, step 1 using (3-hydroxy-4-methoxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 5-azido-2-methoxyphenol. Purification by preparative HPLC (basic conditions) to give *rac*-5-{4-[(2-cyclopropyl-imidazo[5,1-*b*]thiazol-3-yl)-hydroxy-methyl]-[1,2,3]triazol-1-yl}-2-methoxy-phenol. LC-MS (QC): $t_R$ = 0.531; $[M+H]^+$ = 384.3.

**Example 217: *rac*-2-Chloro-5-{4-[(2-cyclopropyl-imidazo[5,1-*b*]thiazol-3-yl)-hydroxy-methyl]-[1,2,3]triazol-1-yl}-phenol**

Step 1: Preparation of 5-azido-2-chlorophenol

Prepared according to the procedure described for Example 45, step 1 using (4-chloro-3-hydroxyphenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 5-azido-2-chlorophenol. Purification by preparative HPLC (basic conditions) to give *rac*-2-chloro-5-{4-[(2-cyclopropyl-imidazo[5,1-*b*]thiazol-3-yl)-hydroxy-methyl]-[1,2,3]triazol-1-yl}-phenol. LC-MS (QC): $t_R$ = 0.615; $[M+H]^+$ = 388.2.

**Example 218: *rac*-5-{4-[(2-Cyclopropyl-imidazo[5,1-*b*]thiazol-3-yl)-hydroxy-methyl]-[1,2,3]triazol-1-yl)-2-methyl-phenol**

Step 1: Preparation of 5-azido-2-methylphenol

Prepared according to the procedure described for Example 45, step 1 using (3-hydroxy-4-methylphenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 5-azido-2-methylphenol. Purification by preparative HPLC (basic conditions) to give *rac*-5-{4-[(2-cyclopropyl-imidazo[5,1-*b*]thiazol-3-yl)-hydroxy-methyl]-[1,2,3]triazol-1-yl}-2-methyl-phenol. LC-MS (QC): $t_R$ = 0.602; $[M+H]^+$ = 368.3.

**Example 219: *rac*-3-{4-[(2-Cyclopropyl-imidazo[5,1-*b*]thiazol-3-yl)-hydroxy-methyl]-[1,2,3]triazol-1-yl}-5-trifluoromethyl-phenol**

Step 1: Preparation of 3-azido-5-(trifluoromethyl)phenol

Prepared according to the procedure described for Example 45, step 1 using (3-hydroxy-5-(trifluoromethyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 3-azido-5-(trifluoromethyl)phenol. Purification by preparative HPLC (basic conditions) to give *rac*-3-{4-[(2-cyclopropyl-imidazo[5,1-*b*]thiazol-3-yl)-hydroxy-methyl]-[1,2,3]triazol-1-yl}-5-trifluoromethyl-phenol. LC-MS (QC): $t_R$ = 0.695; $[M+H]^+$ = 422.2.

**Example 220: *rac*-3-{4-[(2-Cyclopropyl-imidazo[5,1-*b*]thiazol-3-yl)-hydroxy-methyl]-[1,2,3]triazol-1-yl}-5-methyl-phenol**

Step 1: Preparation of 3-azido-5-methylphenol

Prepared according to the procedure described for Example 45, step 1 using (3-hydroxy-5-methylphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 3-azido-5-methylphenol. Purification by preparative HPLC (basic conditions) to give *rac*-3-{4-[(2-cyclopropyl-imidazo[5,1-*b*]thiazol-3-yl)-hydroxy-methyl]-[1,2,3]triazol-1-yl}-5-methyl-phenol. LC-MS (QC): $t_R$ = 0.585; $[M+H]^+$ = 368.1.

**Example 221: *rac*-5-Chloro-2-{4-[(2-cyclopropyl-imidazo[5,1-*b*]thiazol-3-yl)-hydroxy-methyl]-[1,2,3]triazol-1-yl}-phenol**

Step 1: Preparation of 2-azido-5-chlorophenol

Prepared according to the procedure described for Example 45, step 1 using (4-chloro-2-hydroxyphenyl)boronic acid.

Step 2: Prepared according to the procedure described for Example 45, step 2 and using intermediate 1, *rac*-

*1*-(2-cyclopropylimidazo[5,1-*b*)]thiazol-3-yl)prop-2-yn-1-ol and 2-azido-5-chlorophenol. Purification by preparative HPLC (basic conditions) to give *rac*-5-chloro-2-{4-[(2-cyclopropyl-imidazo[5,1-*b*]thiazol-3-yl)-hydroxy-methyl]-[1,2,3]triazol-1-yl}-phenol. LC-MS (QC): $t_R$ = 0.641; $[M+H]^+$ = 388.2.

**Example 222: *rac*-(2-Cyclopropyl-imidazo[5,1-*b*]thiazol-3-yl)-[1-(2,3-dihydro-benzo[1,4]dioxin-6-yl)-1*H*-pyrazol-4-yl]-methanol**

Step 1: Preparation of ethyl 1-(2,3-dihydrobenzo[*b*][1,4]dioxin-6-yl)-1*H*-pyrazole-4-carboxylate

To a solution of ethyl pyrazole-4-carboxylate (300 mg; 2.14 mmol) and 1,4-benzodioxane-6-boronic acid (385 mg; 2.14 mmol) in DMF (10 ml) under argon are added $Cu(OAc)_2$ (292 mg; 1.61 mmol) and pyridine (0.345 ml; 4.28 mmol). The resulting suspension is stirred at RT for 5 days. Sat. aq. $NaHCO_3$ solution is added, and the mixture extracted with $CH_2Cl_2$ (2x). The combined org. extracts are washed with brine, dried ($MgSO_4$), filtered and concentrated under reduced pressure to give 314 mg of ethyl 1-(2,3-dihydrobenzo[*b*][1,4]dioxin-6-yl)-1*H*-pyrazole-4-carboxylate as a white solid. LC-MS (acidic): $t_R$ = 0.92, $[M+H]^+$ = 275.03.

Step 2: Preparation of (1-(2,3-dihydrobenzo[*b*][1,4]dioxin-6-yl)-1*H*-pyrazol-4-yl)methanol

To a solution of ethyl 1-(2,3-dihydrobenzo[*b*][1,4]dioxin-6-yl)-1*H*-pyrazole-4-carboxylate (314 mg; 1.14 mmol) in THF (10 ml) under argon is added $LiAlH_4$ (1M in THF; 1.26 ml; 1.26 mmol) at RT. The reaction mixture is stirred at RT for 1 h. Water is carefully added followed by 1N NaOH. The mixture is extracted with $CH_2Cl_2$ (2x). The combined org. extracts are washed with brine, dried ($MgSO_4$), filtered and concentrated under reduced pressure to give 310 mg of (1-(2,3-dihydrobenzo[*b*][1,4]dioxin-6-yl)-1*H*-pyrazol-4-yl)methanol. LC-MS (acidic): $t_R$ = 0.65, $[M+H]^+$ = 233.09.

Step 3: Preparation of 1-(2,3-dihydrobenzo[*b*][1,4]dioxin-6-yl)-1*H*-pyrazole-4-carbaldehyde

To a solution of (1-(2,3-dihydrobenzo[*b*][1,4]dioxin-6-yl)-1*H*-pyrazol-4-yl)methanol (310 mg; 1.31 mmol) in $CH_2Cl_2$ (10 ml) is added Dess-Martin periodinane (666 mg; 1.57 mmol). The reaction mixture is stirred at RT for 3 h. Sat. aq. $NaHCO_3$ and sat. aq. $Na_2S_2O_3$ are added and the mixture is stirred at RT for 10 min. The layers are separated and the aq. layer extracted with $CH_2Cl_2$. The combined org. extracts are washed with brine, dried ($MgSO_4$), filtered and concentrated under reduced pressure to give 214 mg of 1-(2,3-dihydrobenzo[*b*][1,4]dioxin-6-yl)-1*H*-pyrazole-4-carbaldehyde as a yellow solid. LC-MS (acidic): $t_R$ = 0.78, $[M+H]^+$ = 231.05.

Step 4: Preparation of *rac*-(2-cyclopropyl-imidazo[5,1-*b*]thiazol-3-yl)-[1-(2,3-dihydro-benzo[1,4]dioxin-6-yl)-1*H*-pyrazol-4-yl]-methanol (Example 222)

Prepared following the procedure described for Example 96 using intermediate 10, 3-bromo-2-cyclopropylimidazo[5,1-*b*]thiazole and 1-(2,3-dihydrobenzo[*b*][1,4]dioxin-6-yl)-1*H*-pyrazole-4-carbaldehyde. Purification by preparative HPLC (acidic conditions) followed by neutralization and extraction with DCM to give *rac*-(2-cyclopropyl-imidazo[5,1-*b*]thiazol-3-yl)-[1-(2,3-dihydro-benzo[1,4]dioxin-6-yl)-1*H*-pyrazol-4-yl]-methanol. LC-MS (QC): $t_R$ = 0.643; $[M+H]^+$ = 395.3.

**Example 223: *rac*-(2-Cyclopropyl-imidazo[5,1-b]thiazol-3-yl)-[1-(6-methoxy-pyridin-3-yl)-1*H*-pyrazol-4-yl]-methanol**

Step 1: Preparation of ethyl 1-(6-methoxypyridin-3-yl)-1*H*-pyrazole-4-carboxylate

Prepared following the procedure described for Example 222, step 1 but using (6-methoxypyridin-3-yl)boronic acid. Purification by FC (Silicagel; 0-50% EtOAc in Heptane) gives ethyl 1-(6-methoxypyridin-3-yl)-1*H*-pyrazole-4-carboxylate as a white solid. LC-MS (acidic): $t_R$ = 0.87, $[M+H]^+$ = 248.09.

Step 2: Preparation of (1-(6-methoxypyridin-3-yl)-1*H*-pyrazol-4-yl)methanol

Prepared following the procedure described for Example 222, step 2 but using ethyl 1-(6-methoxypyridin-3-yl)-1*H*-pyrazole-4-carboxylate. (1-(6-Methoxypyridin-3-yl)-1*H*-pyrazol-4-yl)methanol is obtained as a white solid. LC-MS (acidic): $t_R$ = 0.58, $[M+H]^+$ = 206.08.

Step 3: Preparation of 1-(6-methoxypyridin-3-yl)-1*H*-pyrazole-4-carbaldehyde

Prepared following the procedure described for Example 222, step 3 but using (1-(6-methoxypyridin-3-yl)-1*H*-pyrazol-4-yl)methanol. 1-(6-Methoxypyridin-3-yl)-1*H*-pyrazole-4-carbaldehyde is obtained as a white solid. LC-MS (acidic): $t_R$ = 0.69, $[M+H]^+$ = 204.08.

Step 4: Preparation of *rac*-(2-cyclopropyl-imidazo[5,1-*b*]thiazol-3-yl)-[1-(6-methoxy-pyridin-3-yl)-1*H*-pyrazol-4-yl]-methanol (Example 223)

Prepared following the procedure described for Example 96 using intermediate 10, 3-bromo-2-cyclopropylimidazo[5,1-*b*]thiazole and 1-(6-methoxypyridin-3-yl)-1*H*-pyrazole-4-carbaldehyde. Purification by preparative HPLC (acidic conditions) followed by neutralization and extraction with DCM to give *rac*-(2-cyclopropyl-imidazo[5,1-*b*]thiazol-3-yl)-[1-(6-methoxy-pyridin-3-yl)-1*H*-pyrazol-4-yl]-methanol. LC-MS (QC): $t_R$ = 0.596; $[M+H]^+$ = 368.2.

**Example 224: *rac*-*N*-(4-{4-[(2-Cyclopropyl-imidazo[5,1-*b*]thiazol-3-yl)-hydroxy-methyl]-pyrazol-1-yl}-phenyl)-acetamide**

Step 1: Preparation of ethyl 1-(4-acetamidophenyl)-1*H*-pyrazole-4-carboxylate

Prepared following the procedure described for Example 222, step 1 but using (4-acetamidophenyl)boronic acid. Purification by FC (Silicagel; 0-80% EtOAc in Heptane) gives ethyl 1-(4-acetamidophenyl)-1*H*-pyrazole-4-carboxylate as a white solid. LC-MS (acidic): $t_R$ = 0.78, $[M+H]^+$ = 273.86.

Step 2: Preparation of *N*-(4-(4-(hydroxymethyl)-1*H*-pyrazol-1-yl)phenyl)acetamide

Prepared following the procedure described for Example 222, step 2 but using ethyl 1-(4-acetamidophenyl)-1*H*-pyrazole-4-carboxylate. *N*-(4-(4-(Hydroxymethyl)-1*H*-pyrazol-1-yl)phenyl)acetamide is obtained as a yellow solid. LC-MS (acidic): $t_R$ = 0.54, $[M+H]^+$ = 232.11.

Step 3: Preparation of *N*-(4-(4-formyl-1*H*-pyrazol-1-yl)phenyl)acetamide

Prepared following the procedure described for Example 222, step 3 but using *N*-(4-(4-(hydroxymethyl)-1*H*-pyrazol-1-yl)phenyl)acetamide. *N*-(4-(4-Formyl-1*H*-pyrazol-1-yl)phenyl)acetamide is obtained as a brown foam. LC-MS (acidic): $t_R$ = 0.64, $[M+H]^+$ = 230.11.

Step 4: Preparation of *rac*-*N*-(4-{4-[(2-cyclopropyl-imidazo[5,1-*b*]thiazol-3-yl)-hydroxy-methyl]-pyrazol-1-yl}-phenyl)-acetamide (Example 224)

Prepared following the procedure described for Example 96 using intermediate 10, 3-bromo-2-cyclopropylimidazo[5,1-*b*]thiazole and *N*-(4-(4-formyl-1*H*-pyrazol-1-yl)phenyl)acetamide. Purification by preparative HPLC (acidic conditions) followed by neutralization and extraction with DCM to give *rac*-*N*-(4-{4-[(2-cyclopropyl-imidazo[5,1-*b*]thiazol-3-yl)-hydroxy-methyl]-pyrazol-1-yl}-phenyl)-acetamide. LC-MS (QC): $t_R$ = 0.529; $[M+H]^+$ = 394.3.

**Example 225: *rac*-4-{4-[(2-Ethyl-imidazo[5,1-*b*]thiazol-3-yl)-hydroxy-methyl]-[1,2,3]triazol-1-yl}-*N*-methyl-benzenesulfonamide**

Step 1: Preparation of 4-azido-*N*-methylbenzenesulfonamide

Prepared according to the procedure described for Example 45, step 1 using (4-(N-methylsulfamoyl)phenyl)boronic acid.

Step 2: Prepared according to the procedure described for Example 45, step 2 and using intermediate 3, *rac*-1-(2-ethylimidazo[5,1-*b*)]thiazol-3-yl)prop-2-yn-1-ol and 4-azido-*N*-methylbenzenesulfonamide. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-*N*-methyl-benzenesulfonamide. LC-MS (QC): $t_R$ = 0.522; $[M+H]^+$ = 419.3.

**Example 226: *rac*-4-{4-[(2-Cyclopropyl-imidazo[5,1-*b*]thiazol-3-yl)-hydroxy-methyl]-[1,2,3]triazol-1-yl}-*N*-methyl-benzenesulfonamide**

Prepared according to the procedure described for Example 225 and using intermediate 1, *rac-1-(2*-cyclopropylimidazo[5,1-*b*]thiazol-3-yl)prop-2-yn-1-ol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*-methyl-benzenesulfonamide. LC-MS (QC): $t_R$ = 0.555; $[M+H]^+$ = 431.3.

**Example 227: (2-Ethyl-imidazo[5,1-*b*]thiazol-3-yl)-[1-(3-methyl-1,1-dioxo-2,3-dihydro-1*H*-1l6-benzo[*d*)isothiazol-5-yl)-1*H*-[1,2,3]triazol-4-yl]-methanol**

Step 1: Preparation of 4-bromo-2-ethylbenzenesulfonyl azide

A solution of 4-bromo-2-ethylbenzene-1-sulfonylchloride (2089 mg; 7.00 mmol) in water / acetone 1:1 (50 ml) is stirred at 0 °C for 20 min then at RT for 3 h. The mixture is concentrated under reduced pressure (at 25 °C) to remove acetone. The crude product is extracted with EtOAc. The layers are separated, the org. layer is washed with brine, dried over $MgSO_4$, filtered and concentrated under reduced pressure to give 2094 mg of 4-bromo-2-ethylbenzenesulfonyl azide as a pale yellow oil.

Step 2: Preparation of *rac*-5-bromo-3-methyl-2,3-dihydrobenzo[*d*]isothiazole 1,1-dioxide

A degassed mixture of 4-bromo-2-ethylbenzenesulfonyl azide (2031 mg; 7.00 mmol) and Co(II) meso-tetraphenylporphine (470 mg; 0.70 mmol) in chlorobenzene (20.3 ml) is stirred at 85 °C for 36 h. Chlorobenzene is removed under reduced pressure, the residue suspended in EtOAc and filtered. The filtrate is conentrated under reduced pressure and the residue purified by FC (Silicagel; 0-50% EtOAc in Petroleum ether) to give 1025 mg of *rac*-5-bromo-3-methyl-2,3-dihydrobenzo[*d*]isothiazole 1,1-dioxide as a red brown solid.

Step 3: Preparation of *rac*-(3-methyl-1,1-dioxido-2,3-dihydrobenzo[*d*]isothiazol-5-yl)boronic acid

In a 2-ml microwave vial, to a solution of *rac*-5-bromo-3-methyl-2,3-dihydrobenzo[*d*]isothiazole 1,1-dioxide (127 mg; 0.484 mmol) in DMF (2.5 ml) is added bis(pinacolato)diboron (186 mg; 0.727 mmol) followed by potassium acetate (190 mg; 1.94 mmol). The mixture is degassed with argon, then dichloro(1,1'-bis(diphenylphosphino)ferrocene) palladium (II) (39.4 mg; 0.05 mmol) is added. The reaction mixture is heated to 120 °C under microwave irradiations for 30 min. Water and EtOAc are added, the layers separated and the aqueous layer extracted with EtOAc (2x). The combined org. extracts are wased with brine, dried, filtered and concentrated under reduced pressure. The black residue is used without further purification in the next step.

Step 4: Preparation of *rac*-5-azido-3-methyl-2,3-dihydrobenzo[*d*]isothiazole 1,1-dioxide

Prepared according to the procedure described for Example 45, step 1 using *rac*-(3-methyl-1,1-dioxido-2,3-dihydrobenzo[*d*]isothiazol-5-yl)boronic acid.

Step 5: Prepared according to the procedure described for Example 45, step 2 and using intermediate 3, *rac*-1-(2-ethylimidazo[5,1-*b*)]thiazol-3-yl)prop-2-yn-1-ol and *rac*-5-azido-3-methyl-2,3-dihydrobenzo[*d*]isothiazole 1,1-dioxide. Purification by preparative HPLC (basic conditions then acidic conditions) to give (2-ethyl-imidazo[5,1-*b*]thiazol-3-yl)-[1-(3-methyl-1,1-dioxo-2,3-dihydro-1*H*-1l6-benzo[*d*]isothiazol-5-yl)-1*H*-[1,2,3]triazol-4-yl]-methanol. LC-MS (QC): $t_R$ = 0.508; $[M+H]^+$ = 431.2.

**Example 228: (2-Cyclopropyl-imidazo[5,1-*b*]thiazol-3-yl)-[1-(3-methyl-1,1-dioxo-2,3-dihydro-1*H*-1l6-benzo[*d*]isothiazol-5-yl)-1*H*-[1,2,3]triazol-4-yl]-methanol**

Prepared according to the procedure described for Example 227 and using intermediate 1, *rac-1-(2*-cyclopropylimidazo[5,1-*b*]thiazol-3-yl)prop-2-yn-1-ol. Purification by preparative HPLC (basic conditions then acidic conditions) to give (2-cyclopropyl-imidazo[5,1-*b*]thiazol-3-yl)-[1-(3-methyl-1,1-dioxo-2,3-dihydro-1*H*-1l6-benzo[*d*]isothiazol-5-yl)-1*H*-[1,2,3]triazol-4-yl]-methanol. LC-MS (QC): $t_R$ = 0.531; $[M+H]^+$ = 443.3.

**Example 229: *rac*-(2-Cyclopropyl-imidazo[5,1-*b*]thiazol-3-yl)-[1-(1-methyl-1*H*-pyrrolo[2,3-*b*]pyridin-5-yl)-1*H*-pyrazol-4-yl]-methanol**

Step 1: Preparation of ethyl 1-(1-methyl-1*H*-pyrrolo[2,3-*b*]pyridin-5-yl)-1*H*-pyrazole-4-carboxylate

Prepared following the procedure described for Example 222, step 1 but using (1-methyl-1*H*-pyrrolo[2,3-*b*]pyridin-5-yl)boronic acid. Purification by FC (Silicagel; 0-80% EtOAc in Heptane) gives ethyl 1-(1-methyl-1*H*-pyrrolo[2,3-*b*]pyridin-5-yl)-1*H*-pyrazole-4-carboxylate as a pale yellow solid. LC-MS (acidic): $t_R$ = 0.90, $[M+H]^+$ = 271.06.

Step 2: Preparation of (1-(1-methyl-1*H*-pyrrolo[2,3-*b*]pyridin-5-yl)-1*H*-pyrazol-4-yl)methanol

Prepared following the procedure described for Example 222, step 2 but using ethyl 1-(1-methyl-1*H*-pyrrolo[2,3-*b*]pyridin-5-yl)-1*H*-pyrazole-4-carboxylate. (1-(1-Methyl-1*H*-pyrrolo[2,3-*b*]pyridin-5-yl)-1*H*-pyrazol-4-yl)methanol is obtained as a beige solid. LC-MS (acidic): $t_R$ = 0.63, $[M+H]^+$ = 229.09.

Step 3: Preparation of 1-(1-methyl-1*H*-pyrrolo[2,3-*b*]pyridin-5-yl)-1*H*-pyrazole-4-carbaldehyde

Prepared following the procedure described for Example 222, step 3 but using (1-(1-methyl-1*H*-pyrrolo[2,3-*b*]pyridin-5-yl)-1*H*-pyrazol-4-yl)methanol. Purification by FC (Silicagel; EtOAc / Heptane) gives 1-(1-methyl-1*H*-pyrrolo[2,3-*b*]pyridin-5-yl)-1*H*-pyrazole-4-carbaldehyde as a yellow solid. LC-MS (acidic): $t_R$ = 0.75, $[M+H]^+$ = 227.09.

Step 4: Preparation of *rac*-(2-cyclopropyl-imidazo[5,1-*b*]thiazol-3-yl)-[1-(1-methyl-1*H*-pyrrolo[2,3-*b*]pyridin-5-yl)-1*H*-pyrazol-4-yl]-methanol (Example 229)

Prepared following the procedure described for Example 96 using intermediate 10, 3-bromo-2-cyclopropylimidazo[5,1-*b*]thiazole and 1-(1-methyl-1*H*-pyrrolo[2,3-*b*]pyridin-5-yl)-1*H*-pyrazole-4-carbaldehyde. Purification by preparative HPLC (basic conditions) to give *rac*-(2-cyclopropyl-imidazo[5,1-*b*]thiazol-3-yl)-[1-(1-methyl-1*H*-pyrrolo[2,3-*b*]pyridin-5-yl)-1*H*-pyrazol-4-yl]-methanol. LC-MS (QC): $t_R$ = 0.616; $[M+H]^+$ = 391.3.

**Example 230: *rac*-*N*-(4-{4-[(2-Ethyl-imidazo[5,1-*b*]thiazol-3-yl)-hydroxy-methyl]-[1,2,3]triazol-1-yl}-phenyl)-benzamide**

Prepared according to the procedure described for Example 189 and using intermediate 3, *rac*-1-(2-ethylimidazo[5,1-*b*)]thiazol-3-yl)prop-2-yn-1-ol. Purification by preparative HPLC (acidic conditions) to give *rac*-*N*-(4-{4-[(2-ethyl-imidazo[5,1-*b*]thiazol-3-yl)-hydroxy-methyl]-[1,2,3]triazol-1-yl}-phenyl)-benzamide. LC-MS (QC): $t_R$ = 0.667; $[M+H]^+$ = 445.2.

**Example 231: *rac*-(4-{4-[(2-Cyclopropyl-imidazo[5,1-*b*]thiazol-3-yl)-hydroxy-methyl]-[1,2,3]triazol-1-yl}-phenoxy)-acetic acid ethyl ester**

Prepared following the procedure described for Example 87, and using ethyl 2-chloroacetate. Purification by preparative HPLC (basic conditions) gives *rac*-(4-{4-[(2-cyclopropyl-imidazo[5,1-*b*]thiazol-3-yl)-hydroxy-methyl]-[1,2,3]triazol-1-yl}-phenoxy)-acetic acid ethyl ester. LC-MS (QC): $t_R$ = 0.664; $[M+H]^+$ = 440.3.

**Example 232: *rac*-4-{4-[(2-Cyclopropyl-imidazo[5,1-*b*]thiazol-3-yl)-hydroxy-methyl]-[1,2,3]triazol-1-yl}-2-ethylphenol**

Step 1: Preparation of 4-azido-2-ethylphenol

Prepared according to the procedure described for Example 45, step 1 using (3-ethyl-4-hydroxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-2-ethylphenol. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-2-ethylphenol. LC-MS (QC): $t_R$ = 0.625; $[M+H]^+$ = 382.3.

**Example 233: *rac*-4-{4-[(2-Cyclopropyl-imidazo[5,1-*b*]thiazol-3-yl)-hydroxy-methyl]-[1,2,3]triazol-1-yl}-2-hydroxy-benzonitrile**

Step 1: Preparation of 4-azido-2-hydroxybenzonitrile

Prepared according to the procedure described for Example 45, step 1 using (4-cyano-3-hydroxyphenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-2-hydroxybenzonitrile. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-2-hydroxy-benzonitrile. LC-MS (QC): $t_R$ = 0.569; $[M+H]^+$ = 379.3.

**Example 234: *rac*-(4-{4-[(2-Ethyl-imidazo[5,1-*b*]thiazol-3-yl)-hydroxy-methyl]-[1,2,3]triazol-1-yl}-phenyl)-carbamic acid tert-butyl ester**

Prepared according to the procedure described for Example 180 and using intermediate 3, *rac*-1-(2-ethylimidazo[5,1-*b*)]thiazol-3-yl)prop-2-yn-1-ol. Purification by preparative HPLC (acidic conditions) to give *rac*-(4-{4-[(2-ethyl-imidazo[5,1-*b*]thiazol-3-yl)-hydroxy-methyl]-[1,2,3]triazol-1-yl}-phenyl)-carbamic acid tert-butyl ester. LC-MS (QC): $t_R$ = 0.736; $[M+H]^+$ = 441.4.

**Example 235: *rac*-4-{4-[(2-Cyclopropyl-imidazo[5,1-*b*]thiazol-3-yl)-hydroxy-methyl]-[1,2,3]triazol-1-yl}-benzenesulfonamide**

Step 1: Preparation of 4-azidobenzenesulfonamide

Prepared according to the procedure described for Example 45, step 1 using (4-sulfamoylphenyl)boronic acid. Step 2: Prepared according to the procedure described for Example 45, step 2 and using intermediate 1, *rac*-1-(2-cyclopropylimidazo[5,1-*b*)]thiazol-3-yl)prop-2-yn-1-ol and 4-azidobenzenesulfonamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-benzenesulfonamide. LC-MS (QC): $t_R$ = 0.476; $[M+H]^+$ = 417.1.

**Example 236: *rac*-4-{4-[(2-Cyclopropyl-imidazo[5,1-*b*]thiazol-3-yl)-hydroxy-methyl]-[1,2,3]triazol-1-yl}-*N*,*N*-dimethyl-benzenesulfonamide**

Step 1: Preparation of 4-azido-*N*,*N*-dimethylbenzenesulfonamide

Prepared according to the procedure described for Example 45, step 1 using (4-(*N*,*N*-dimethylsulfamoyl)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 4-azido-*N*,*N*-dimethylbenzenesulfonamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*,*N*-dimethyl-benzenesulfonamide. LC-MS (QC): $t_R$ = 0.618; $[M+H]^+$ = 445.3.

**Example 237: *rac*-(2-Cyclopropyl-imidazo[5,1-*b*]thiazol-3-yl)-{1-[4-(pyrrolidine-1-sulfonyl)-phenyl]-1*H*-[1,2,3]triazol-4-yl}-methanol**

Step 1: Preparation of 1-((4-azidophenyl)sulfonyl)pyrrolidine

Prepared according to the procedure described for Example 45, step 1 using (4-(pyrrolidin-1-ylsulfonyl)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 1-((4-azidophenyl)sulfonyl)pyrrolidine. Purification by preparative HPLC (basic conditions) to give *rac*-(2-cyclopropyl-imidazo[5,1-*b*]thiazol-3-yl)-{1-[4-(pyrrolidine-1-sulfonyl)-phenyl]-1*H*-[1,2,3]triazol-4-yl}-methanol. LC-MS (QC): $t_R$ = 0.669; $[M+H]^+$ = 471.2.

**Example 238: *rac*-4-{4-[(2-Cyclopropyl-imidazo[5,1-*b*]thiazol-3-yl)-hydroxy-methyl]-[1,2,3]triazol-1-yl}-*N,N*-diethyl-benzenesulfonamide**

Step 1: Preparation of 4-azido-*N*,*N*-diethylbenzenesulfonamide

Prepared according to the procedure described for Example 45, step 1 using (4-(*N*,*N*-diethylsulfamoyl)phenyl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 4-azido-*N*,*N*-diethylbenzenesulfonamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*,*N*-diethyl-benzenesulfonamide. LC-MS (QC): $t_R$ = 0.721; $[M+H]^+$ = 473.2.

**Example 239: *rac*-4-{4-[(2-Cyclopropyl-imidazo[5,1-*b*]thiazol-3-yl)-hydroxy-methyl]-[1,2,3]triazol-1-yl}-*N*-ethyl-benzenesulfonamide**

Step 1: Preparation of 4-azido-*N*-ethylbenzenesulfonamide

Prepared according to the procedure described for Example 45, step 1 using (4-(*N*-ethylsulfamoyl)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-*N*-ethylbenzenesulfonamide. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-*N*-ethyl-benzenesulfonamide. LC-MS (QC): $t_R$ = 0.594; $[M+H]^+$ = 445.3.

**Example 240: *rac*-4-{4-[(2-Ethyl-imidazo[5,1-*b*]thiazol-3-yl)-hydroxy-methyl]-[1,2,3]triazol-1-yl}-benzenesulfonamide**

Prepared according to the procedure described for Example 235 and using intermediate 3, *rac*-1-(2-ethylimidazo[5,1-*b*)]thiazol-3-yl)prop-2-yn-1-ol. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-benzenesulfonamide. LC-MS (QC): $t_R$ = 0.452; $[M+H]^+$ = 405.2.

**Example 241: *rac*-4-{4-[(2-Ethyl-imidazo[5,1-*b*]thiazol-3-yl)-hydroxy-methyl]-[1,2,3]triazol-1-yl}-*N,N*-dimethyl-benzenesulfonamide**

Prepared according to the procedure described for Example 236 and using intermediate 3, *rac-1-(2*-ethylimidazo[5,1-*b*]thiazol-3-yl)prop-2-yn-1-ol. Purification by preparative HPLC (basic conditions) to give *rac*-4-{4-[(2-ethyl-imidazo[5,1-*b*]thiazol-3-yl)-hydroxy-methyl]-[1,2,3]triazol-1-yl}-*N*,*N*-dimethyl-benzenesulfonamide. LC-MS (QC): $t_R$ = 0.596; $[M+H]^+$ = 433.1.

**Example 242: *rac*-(2-Ethyl-imidazo[5,1-*b*]thiazol-3-yl)-{1-[4-(pyrrolidine-1-sulfonyl)-phenyl]-1*H*-[1,2,3]triazol-4-yl}-methanol**

Prepared according to the procedure described for Example 237 and using intermediate 3, *rac*-1-(2-ethylimidazo[5,1-*b*)]thiazol-3-yl)prop-2-yn-1-ol. Purification by preparative HPLC (basic conditions) to give *rac*-(2-ethyl-imidazo[5,1-*b*]thiazol-3-yl)-{1-[4-(pyrrolidine-1-sulfonyl)-phenyl]-1*H*-[1,2,3]triazol-4-yl}-methanol. LC-MS (QC): $t_R$ = 0.648; $[M+H]^+$ = 459.3.

**Example 243: *rac-N,N*-Diethyl-4-{4-[(2-ethyl-imidazo[5,1-*b*]thiazol-3-yl)-hydroxy-methyl]-[1,2,3]triazol-1-yl}-benzenesulfonamide**

Prepared according to the procedure described for Example 238 and using intermediate 3, *rac*-1-(2-ethylimidazo[5,1-*b*)]thiazol-3-yl)prop-2-yn-1-ol. Purification by preparative HPLC (basic conditions) to give *rac-N*,*N*-diethyl-4-{4-[(2-ethyl-imidazo[5,1-*b*]thiazol-3-yl)-hydroxy-methyl]-[1,2,3]triazol-1-yl}-benzenesulfonamide. LC-MS (QC): $t_R$ = 0.700; $[M+H]^+$ = 461.2.

**Example 244: *rac*-*N*-Ethyl-4-{4-[(2-ethyl-imidazo[5,1-*b*]thiazol-3-yl)-hydroxy-methyl]-[1,2,3]triazol-1-yl}-benzenesulfonamide**

Prepared according to the procedure described for Example 239 and using intermediate 3, *rac*-1-(2-ethylimidazo[5,1-*b*)]thiazol-3-yl)prop-2-yn-1-ol. Purification by preparative HPLC (basic conditions) to give *rac*-*N*-ethyl-4-{4-[(2-ethyl-imidazo[5,1-*b*]thiazol-3-yl)-hydroxy-methyl]-[1,2,3]triazol-1-yl}-benzenesufonamide. LC-MS (QC): $t_R$ = 0.572; $[M+H]^+$ = 433.3.

**Example 245: *rac*-(4-{4-[(2-Ethyl-imidazo[5,1-*b*)]thiazol-3-yl)-hydroxy-methyl]-[1,2,3]triazol-1-yl}-3-fluoro-phenyl)-carbamic acid tert-butyl ester**

Prepared according to the procedure described for Example 151 and using intermediate 3, *rac*-1-(2-ethylimidazo[5,1-*b*)]thiazol-3-yl)prop-2-yn-1-ol. Purification by preparative HPLC (basic conditions) to give *rac*-(4-{4-[(2-ethyl-imidazo[5,1-*b*]thiazol-3-yl)-hydroxy-methyl]-[1,2,3]triazol-1-yl}-3-fluoro-phenyl)-carbamic acid tert-butyl ester. LC-MS (QC): $t_R$ = 0.771; $[M+H]^+$ = 459.2.

**Example 246: *rac*-*N*-(4-{4-[(2-Cyclopropyl-imidazo[5,1-*b*]thiazol-3-yl)-hydroxy-methyl]-pyrazol-1-yl}-phenyl)-isobutyramide**

Step 1: Preparation of *N*-(4-(4-formyl-1*H*-pyrazol-1-yl)phenyl)isobutyramide

Prepared following the procedure described for Example 222, step 1 but using 1*H*-pyrazole-4-carbaldehyde and (4-isobutyramidophenyl)boronic acid. Purification by FC (Silicagel; 0-80% EtOAc in Heptane) gives *N*-(4-(4-formyl-1*H*-pyrazol-1-yl)phenyl)isobutyramide as a white solid. LC-MS (acidic): $t_R$ = 0.74, $[M+H]^+$ = 258.09.

Step 2: Preparation of *rac*-*N*-(4-{4-[(2-cyclopropyl-imidazo[5,1-*b*]thiazol-3-yl)-hydroxy-methyl]-pyrazol-1-yl}-phenyl)-isobutyramide (Example 246)

Prepared following the procedure described for Example 96 using intermediate 10, 3-bromo-2-cyclopropylimidazo[5,1-*b*]thiazole and *N*-(4-(4-formyl-1*H*-pyrazol-1-yl)phenyl)isobutyramide. Purification by preparative HPLC (acidic conditions) followed by neutralization and extraction with DCM to give *rac*-*N*-(4-{4-[(2-cyclopropyl-imidazo[5,1-*b*]thiazol-3-yl)-hydroxy-methyl]-pyrazol-1-yl}-phenyl)-isobutyramide. LC-MS (QC): $t_R$ = 0.634; $[M+H]^+$ = 422.4.

**Example 247: *rac*-5-{4-[(2-Cyclopropyl-imidazo[5,1-*b*]thiazol-3-yl)-hydroxy-methyl]-pyrazol-1-yl)-2-methoxy-benzonitrile**

Step 1: Preparation of 5-(4-formyl-1*H*-pyrazol-1-yl)-2-methoxybenzonitrile

Prepared following the procedure described for Example 222, step 1 but using 1*H*-pyrazole-4-carbaldehyde and (3-cyano-4-methoxyphenyl)boronic acid. Purification by FC (Silicagel; 0-80% EtOAc in Heptane) gives 5-(4-formyl-1*H*-pyrazol-1-yl)-2-methoxybenzonitrile as a white solid. LC-MS (acidic): $t_R$ = 0.77, $[M+H]^+$ = 228.39. Step 2: Preparation of *rac*-5-{4-[(2-cyclopropyl-imidazo[5,1-*b*]thiazol-3-yl)-hydroxy-methyl]-pyrazol-1-yl}-2-methoxy-benzonitrile (Example 247)

Prepared following the procedure described for Example 96 using intermediate 10, 3-bromo-2-cyclopropylimidazo[5,1-*b*]thiazole and 5-(4-formyl-1*H*-pyrazol-1-yl)-2-methoxybenzonitrile. Purification by preparative HPLC (acidic conditions) followed by neutralization and extraction with DCM to give *rac*-5-{4-[(2-cyclopropyl-imidazo[5,1-*b*]thiazol-3-yl)-hydroxy-methyl]-pyrazol-1-yl}-2-methoxy-benzonitrile. LC-MS (QC): $t_R$ = 0.653; $[M+H]^+$ = 392.3.

**Example 248: *rac*-4-{4-[(2-Cyclopropyl-imidazo[5,1-*b*]thiazol-3-yl)-hydroxy-methyl]-[1,2,3]triazol-1-yl}-2-nitro-phenol**

Step 1: Preparation of 4-azido-2-nitrophenol

Prepared according to the procedure described for Example 45, step 1 using 2-nitro-4-(4,4,5,5-tetramethyl-1,3,2-dioxaborolan-2-yl)phenol.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-2-nitrophenol. Purification by preparative HPLC (acidic conditions) to give *rac*-4-{4-[(2-cyclopropyl-imidazo[5,1-*b*]thiazol-3-yl)-hydroxy-methyl]-[1,2,3]triazol-1-yl}-2-nitro-phenol. LC-MS (QC): $t_R$ = 0.586; $[M+H]^+$ = 399.1.

**Example 249: *rac*-[1-(4-Methoxy-phenyl)-1*H*-[1,2,3]triazol-4-yl]-(2-phenyl-imidazo[5,1-*b*]thiazol-3-yl)-methanol**

Prepared according to the procedure described for Example 45, step 2 and using intermediate 13, *rac*-1-(2-phenylimidazo[5,1-*b*]thiazol-3-yl)prop-2-yn-1-ol and 1-azido-4-methoxybenzene. Purification by preparative HPLC (basic conditions) to give *rac*-[1-(4-methoxy-phenyl)-1*H*-[1,2,3]triazol-4-yl]-(2-phenyl-imidazo[5,1-*b*]thiazol-3-yl)-methanol. LC-MS (QC): $t_R$ = 0.752; $[M+H]^+$ = 404.2.

**Intermediate 13: *rac*-1-(2-Phenylimidazo[5,1-*b*]thiazol-3-yl)prop-2-yn-1-ol**

Step 1: Preparation of 2-bromoimidazo[5,1-*b*]thiazole-3-carboxylic acid

To a solution of the product from Intermediate 6, step 3, methyl 2-bromoimidazo[5,1-*b*]thiazole-3-carboxylate (4.09 g; 15.5 mmol) in THF (40 ml), is added LiOH 1 M (18.6 ml, 18.6 mmol). The suspension is stirred at RT for 2 hours. The mixture is acidified with aqueous HCl 1 M until pH 4, evaporated, and dried under high vacuum to give 4.9 g of 2-bromoimidazo[5,1-*b*]thiazole-3-carboxylic acid. LC-MS (acidic): $t_R$ = 0.34; $[M+H]^+$ = 246.80.

Step 2: Preparation of 2-bromo-*N*-methoxy-*N*-methylimidazo[5,1-*b*]thiazole-3-carboxamide

To a solution of the product from step 1, 2-bromoimidazo[5,1-*b*]thiazole-3-carboxylic acid (4.9 g, 16.9 mmol) in $CH_2Cl_2$ (50 ml) and DMF (10 ml) are added at RT HATU (7.724 g, 20.3 mmol), DIPEA (3.48 ml, 20.3 mmol) and N,O-dimethylhydroxylamine hydrochloride (1.734 g, 17.8 mmol). The mixture is stirred at RT for 2 hours. The mixture is poured into a saturated aqueous $NaHCO_3$ solution and extracted with $CH_2Cl_2$ (3x). The combined organic layers are washed with brine, dried over $MgSO_4$, filtered and concentrated under reduced pressure. Purification by FC (Silicagel; Hept / EtOAc) gives 5.25 g of 2-bromo-*N*-methoxy-*N*-methylimidazo[5,1-*b*]thiazole-3-carboxamide. LC-MS (acidic): $t_R$ = 0.48; $[M+H]^+$ = 291.81.

Step 3: Preparation of *N*-methoxy-*N*-methyl-2-phenylimidazo[5,1-*b*]thiazole-3-carboxamide

To a degassed solution of the product from step 2, 2-bromo-*N*-methoxy-*N*-methylimidazo[5,1-*b*]thiazole-3-carboxamide (400 mg, 1.31 mmol) in $Na_2CO_3$ 1.6 M (1 ml) and dioxane (3 ml) are added under argon phenylboronic acid (191 mg, 1.57 mol) and $Pd(Ph_3)_4$ (37.8 mg, 0.03 mmol). The mixture is stirred at 95°C for 16 hours, then cooled to RT, poured into $H_2O$ and extracted with EtOAc (2x). The combined organic layers are washed with brine, dried over $MgSO_4$, filtered and concentrated under reduced pressure. Purification by FC (Silicagel; Hept / EtOAc) gives 222 mg of *N*-methoxy-*N*-methyl-2-phenylimidazo[5,1-*b*]thiazole-3-carboxamide. LC-MS (acidic): $t_R$ = 0.61; $[M+H]^+$ = 288.03.

Step 4: Preparation of 2-phenylimidazo[5,1-*b*]thiazole-3-carbaldehyde

To a solution of the product from step 3, *N*-methoxy-*N*-methyl-2-phenylimidazo[5,1-*b*]thiazole-3-carboxamide (220 mg, 0.766 mmol) in THF (5 ml) is added dropwise at 0°C diisobutylaluminium hydride solution 1 M in toluene (1.57 ml, 1.57 mmol). The mixture is allowed to warm up to RT and stirred for 1 hour. The mixture is quenched with a saturated aqueous $NH_4Cl$ solution and extracted with $CH_2Cl_2$ (3x). The combined organic layers are dried over $MgSO_4$, filtered and concentrated under reduced pressure to give 158 mg of 2-phenylimidazo[5,1-*b*]thiazole-3-carbaldehyde. LC-MS (acidic): $t_R$ = 0.58; $[M+H]^+$ = 229.0.

Step 5: Preparation of *rac*-1-(2-phenylimidazo[5,1-*b*]thiazol-3-yl)prop-2-yn-1-ol (intermediate 13)

To an ice-cold solution of the product from Step 4, 2-phenylimidazo[5,1-*b*]thiazole-3-carbaldehyde (158 mg; 0.549 mmol) in THF (5 ml) is added a solution of ethynylmagnesium bromide (0.5 M in THF, 1.1 ml, 0.549 mmol) in a dropwise manner. The reaction mixture is stirred at RT for 0.5 h. The reaction is quenched by careful addition of aq. ammonium chloride solution. The product is extracted with $CH_2Cl_2$ (3 x 20 ml) and the combined organic extracts are dried over $MgSO_4$, filtered and concentrated under reduced pressure. Purification by FC (Silicagel; Hept / EtOAc) gives 119 mg of *rac*-1-(2-phenylimidazo[5,1-*b*]thiazol-3-yl)prop-2-yn-1-ol. LC-MS (acidic): $t_R$ = 0.62; $[M+H]^+$ = 255.0.

**Example 250: *rac*-4-{4-[Hydroxy-(2-phenyl-imidazo[5,1-*b*]thiazol-3-yl)-methyl]-[1,2,3]triazol-1-yl}-2-nitro-phenol**

Step 1: Preparation of 4-azido-2-nitrophenol

Prepared according to the procedure described for Example 45, step 1 using 2-nitro-4-(4,4,5,5-tetramethyl-1,3,2-dioxaborolan-2-yl)phenol.

Step 2: Prepared according to the procedure described for Example 45, step 2 and using intermediate 13, *rac*-1-(2-phenylimidazo[5,1-*b*)]thiazol-3-yl)prop-2-yn-1-ol and 4-azido-2-nitrophenol. Purification by preparative HPLC (basic conditions) to give *rac*-4-{4-[hydroxy-(2-phenyl-imidazo[5,1-*b*]thiazol-3-yl)-methyl]-[1,2,3]triazol-1-yl}-2-nitro-phenol. LC-MS (QC): $t_R$ = 0.730; $[M+H]^+$ = 435.3.

**Example 251: *rac*-(4-{4-[Hydroxy-(2-phenyl-imidazo[5,1-*b*]thiazol-3-yl)-methyl]-[1,2,3]triazol-1-yl}-phenyl)-carbamic acid *tert*-butyl ester**

Step 1: Preparation of *tert*-butyl (4-azidophenyl)carbamate

Prepared according to the procedure described for Example 45, step 1 using (4-((*tert*-butoxycarbonyl)amino)phenyl)boronic acid.

Step 2: Prepared according to the procedure described for Example 45, step 2 and using intermediate 13, *rac*-1-(2-phenylimidazo[5,1-*b*)]thiazol-3-yl)prop-2-yn-1-ol and *tert*-butyl (4-azidophenyl)carbamate. Purification by preparative HPLC (basic conditions) to give *rac*-(4-{4-[hydroxy-(2-phenyl-imidazo[5,1-*b*]thiazol-3-yl)-methyl]-[1,2,3]triazol-1-yl}-phenyl)-carbamic acid *tert*-butyl ester. LC-MS (QC): $t_R$ = 0.887; $[M+H]^+$ = 489.2.

**Example 252: *rac*-(2-Cyclopropyl-imidazo[5,1-*b*]thiazol-3-yl)-[1-(4-methoxy-phenyl)-5-methyl-1*H*-[1,2,3]triazol-4-yl]-methanol**

Step 1: Preparation of ethyl 1-(4-methoxyphenyl)-5-methyl-1*H*-1,2,3-triazole-4-carboxylate According to the procedure described for the preparation of example 279, step 1 but using ethyl 3-oxobutanoate as starting material. LC-MS (acidic): $t_R$ = 0.85; $[M+H]^+$ = 262.06.

Step 2: Preparation of (1-(4-methoxyphenyl)-5-methyl-1*H*-1,2,3-triazol-4-yl)methanol

According to the procedure described for the preparation of example 279, step 2 but using ethyl 1-(4-methoxyphenyl)-5-methyl-1*H*-1,2,3-triazole-4-carboxylate as starting material. (1-(4-Methoxyphenyl)-5-methyl-1*H*-1,2,3-triazol-4-yl)methanol is obtained as a purple solid. LC-MS (acidic): $t_R$ = 0.61, $[M+H]^+$ = 220.36.

Step 3: Preparation of 1-(4-methoxyphenyl)-5-methyl-1*H*-1,2,3-triazole-4-carbaldehyde

Prepared following the procedure described for Example 222, step 3 but using (1-(4-methoxyphenyl)-5-methyl-1*H*-1,2,3-triazol-4-yl)methanol. 1-(4-Methoxyphenyl)-5-methyl-1*H*-1,2,3-triazole-4-carbaldehyde is obtained as a brown solid. LC-MS (acidic): $t_R$ = 0.77, $[M+H]^+$ = 218.35.

Step 4: Preparation of *rac*-(2-cyclopropyl-imidazo[5,1-*b*]thiazol-3-yl)-[1-(4-methoxy-phenyl)-5-methyl-1*H*-[1,2,3]triazol-4-yl]-methanol (Example 252)

Prepared following the procedure described for Example 96 using intermediate 10, 3-bromo-2-cyclopropylimidazo[5,1-*b*]thiazole and 1-(4-methoxyphenyl)-5-methyl-1*H*-1,2,3-triazole-4-carbaldehyde. Purification by preparative HPLC (basic conditions) to give *rac*-(2-cyclopropyl-imidazo[5,1-*b*]thiazol-3-yl)-[1-(4-methoxy-phenyl)-5-methyl-1*H*-[1,2,3]triazol-4-yl]-methanol. LC-MS (QC): $t_R$ = 0.621; $[M+H]^+$ = 382.2.

**Example 253: *rac*-(2-Cyclopropyl-imidazo[5,1-*b*]thiazol-3-yl)-[1-(1*H*-indazol-5-yl)-1*H*-[1,2,3]triazol-4-yl]-methanol**

Step 1: Preparation of 5-azido-1*H*-indazole

Prepared according to the procedure described for Example 45, step 1 using (1*H*-indazol-5-yl)boronic acid. Step 2: Prepared according to the procedure described for Example 45, step 2 and using intermediate 1, *rac*-1-(2-cyclopropylimidazo[5,1-*b*)]thiazol-3-yl)prop-2-yn-1-ol and 5-azido-1*H*-indazole. Purification by preparative HPLC (basic

conditions) to give *rac*-(2-cyclopropyl-imidazo[5,1-*b*]thiazol-3-yl)-[1-(1*H*-indazol-5-yl)-1*H*-[1,2,3]triazol-4-yl]-methanol. LC-MS (QC): $t_R$ = 0.506; $[M+H]^+$ = 378.3.

**Example 254: *rac*-(2-Cyclopropyl-imidazo[5,1-*b*]thiazol-3-yl)-[1-(1-methyl-1*H*-indazol-7-yl)-1*H*-[1,2,3]triazol-4-yl]-methanol**

Step 1: Preparation of 7-azido-1-methyl-1*H*-indazole

Prepared according to the procedure described for Example 45, step 1 using (1-methyl-1*H*-indazol-7-yl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 7-azido-1-methyl-1*H*-indazole. Purification by preparative HPLC (basic conditions) to give *rac*-(2-cyclopropyl-imidazo[5,1-*b*]thiazol-3-yl)-[1-(1-methyl-1*H*-indazol-7-yl)-1*H*-[1,2,3]triazol-4-yl]-methanol. LC-MS (QC): $t_R$ = 0.585; $[M+H]^+$ = 392.3.

**Example 255: *rac*-[1-(4-Methoxy-phenyl)-5-methyl-1*H*-[1,2,3]triazol-4-yl]-(2-methyl-imidazo[5,1-*b*]thiazol-3-yl)-methanol**

Prepared following the procedure described for Example 252 using intermediate 2, *rac*-1-(2-methylimidazo[5,1-*b*]thiazol-3-yl)prop-2-yn-1-ol and 1-(4-methoxyphenyl)-5-methyi-1*H*-1,2,3-triazole-4-carbaldehyde. Purification by preparative HPLC (basic conditions) to give *rac*-[1-(4-methoxy-phenyl)-5-methyl-1*H*-[1,2,3]triazol-4-yl]-(2-methyl-imidazo[5,1-*b*]thiazol-3-yl)-methanol. LC-MS (QC): $t_R$ = 0.536; $[M+H]^+$ = 356.3.

**Example 256: *rac*-4-{4-[(2-Cyclopropyl-imidazo[5,1-*b*]thiazol-3-yl)-hydroxy-methyl]-[1,2,3]triazol-1-yl}-((*N*-methylsulfamoyl)amino)-benzene**

Step 1: Preparation of 4-azido-((*N*-methylsulfamoyl)amino)-benzene

Prepared according to the procedure described for Example 45, step 1 using (4-((*N*-methylsulfamoyl)amino)phenyl)boronic acid.

Step 2: Prepared according to the procedure described for Example 45, step 2 and using intermediate 1, *rac-1*-(2-cyclopropylimidazo[5,1-*b*)]thiazol-3-yl)prop-2-yn-1-ol and 4-azido-((*N*-methylsulfamoyl)amino)-benzene. Purification by preparative HPLC (basic conditions) to give *rac*-4-{4-[(2-cyclopropyl-imidazo[5,1-*b*]thiazol-3-yl)-hydroxy-methyl]-[1,2,3]triazol-1-yl}-((*N*-methylsulfamoyl)amino)-benzene. LC-MS (QC): $t_R$ = 0.515; $[M+H]^+$ = 446.3.

**Example 257: *rac*-[2-(1,1-Difluoro-ethyl)-imidazo[5,1-*b*]thiazol-3-yl]-[1-(4-methoxy-phenyl)-1*H*-[1,2,3]triazol-4-yl]-methanol**

Prepared according to the procedure described for Example 45, step 2 and using intermediate 14, *rac*-1-(2-(1,1-difluoroethyl)imidazo[5,1-*b*]thiazol-3-yl)prop-2-yn-1-ol and 1-azido-4-methoxybenzene. Purification by preparative HPLC (basic conditions) to give *rac*-[2-(1,1-difluoro-ethyl)-imidazo[5,1-*b*]thiazol-3-yl]-[1-(4-methoxy-phenyl)-1*H*-[1,2,3]triazol-4-yl]-methanol. LC-MS (QC): $t_R$ = 0.752; $[M+H]^+$ = 392.3.

**Intermediate 14: *rac*-1-(2-(1,1-Difluoroethyl)imidazo[5,1-*b*]thiazol-3-yl)prop-2-yn-1-ol**

Step 1: Preparation of ethyl 2-diazo-4,4-difluoro-3-oxopentanoate

A solution of ethyl 4,4-difluoro-3-oxopentanoate (2000 mg; 10.5 mmol) min acetonitrile (50 ml) is successively treated with 4-acetamidobenzenesulfonyl azide (2743 mg; 11.1 mmol) and $Et_3N$ (1.54 ml; 11.1 mmol). The reaction mixture is stirred at RT for 18 hours. The mixture is filtered, the filter cake washed with MeCN and the filtrate concentrated under reduced pressure. The residue is redissolved in CH2Cl2, filtered and the filter cake washed with $CH_2Cl_2$. The filtrate is concentrated under reduced to give ethyl 2-diazo-4,4-difluoro-3-oxopentanoate as a yellow oil. LC-MS (acidic): $t_R$ = 0.79; $[M+H]^+$ 207.03.

Step 2: Preparation of *rac*-ethyl-2-(2-((*tert*-butoxycarbonyl)amino)acetamido)-4,4-difluoro-3-oxopentanoate

Ethyl 2-diazo-4,4-difluoro-3-oxopentanoate (2358 mg; 11.4 mmol) is dissolved in DCM (60 ml) and *tert*-butyl (2-amino-2-oxoethyl)carbamate (2179 mg; 12.0 mmol) is added followed by dirhodium tetraacetate (54 mg, 0.11 mmol).

The reaction mixture is stirred at 45°C for 5 days. Water is added, the layers separated and the aq. layer extracted with DCM (2x). The combined org. extracts are dried over magnesium sulfate, filtered and concentrated under reduced pressure. Crude *rac*-ethyl-2-(2-((*tert*-butoxycarbonyl)amino)acetamido)-4,4-difluoro-3-oxopentanoate is obtained as a black solid, which is used directly in the next step.

Step 3: Preparation of ethyl 2-(((*tert*-butoxycarbonyl)amino)methyl)-5-(1,1-difluoroethyl)thiazole-4-carboxylate

The product from step 2, *rac*-ethyl-2-(2-((*tert*-butoxycarbonyl)amino)acetamido)-4,4-difluoro-3-oxopentanoate (3708 mg; 10.5 mmol) and Lawesson's reagent (4682 mg; 11.6 mmol) are suspended in THF (110 ml) and heated to reflux for 24 hours. THF is evaporated under reduced pressure and the residue is purified by FC (Silicagel; EtOAc / heptane) to give 2333 mg of ethyl 2-(((*tert*-butoxycarbonyl)amino)methyl)-5-(1,1-difluoroethyl)thiazole-4-carboxylate. LC-MS (acidic): $t_R$ = 0.93; $[M+H]^+$ = 351.41.

Step 4: Preparation of ethyl 5-(1,1-difluoroethyl)-2-(formamidomethyl)thiazole-4-carboxylate

Step 4.1: Boc-cleavage: The product from step 3, ethyl 2-(((*tert*-butoxycarbonyl)amino)methyl)-5-(1,1-difluoroethyl)thiazole-4-carboxylate (2333 mg; 6.66 mmol) is dissolved in $CH_2Cl_2$ (60 ml) and treated with 4N HCl in dioxane (20 ml; 80.0 mmol). The mixture is stirred at RT for 5 hours. The reaction mixture is concentrated under reduced pressure.

Step 4.2: The residue from step 4.1 is dissolved in ethyl formate (30 ml) and $Et_3N$ (4.33 ml; 31.1 mmol) is added. The mixture is stirred at reflux for 15 hours. A sat. aq. $NH_4Cl$ solution is added and the mixture extracted with DCM (2x). The combined org. extracts are dried over $MgSO_4$, filtered and concentrated under reduced pressure to give 2065 mg of ethyl 5-(1,1-difluoroethyl)-2-(formamidomethyl)thiazole-4-carboxylate which is used without further purification in Step 5. LC-MS (acidic): $t_R$ = 0.71; $[M+H]^+$ = 279.21.

Step 5: Preparation of ethyl 2-(1,1-difluoroethyl)imidazo[5,1-*b*]thiazole-3-carboxylate

The product from step 4.2, ethyl 5-(1,1-difluoroethyl)-2-(formamidomethyl)thiazole-4-carboxylate (2065 mg; 7.42 mmol) is dissolved in DCM (30 ml) and cooled to -20°C followed by the addition of phosphorus oxychloride (1.4 ml; 14.9 mmol). The reaction mixture is stirred at RT for 30 min and at reflux for 48 h. Water was added followed by sat. aq. $NaHCO_3$. The layers were separated and the aq. layer extracted with DCM (2x). The combined organic layers are dried over $MgSO_4$, filtered and concentrated under reduced pressure to give 1572 mg of ethyl 2-(1,1-difluoroethyl)imidazo[5,1-*b*]thiazole-3-carboxylate as a beige foam. LC-MS (acidic): $t_R$ = 0.68; $[M+H]^+$ = 260.97.

Step 6: Preparation of (2-(1,1-difluoroethyl)imidazo[5,1-*b*]thiazol-3-yl)methanol

The product from step 5, ethyl 2-(1,1-difluoroethyl)imidazo[5,1-*b*]thiazole-3-carboxylate (1572 mg; 6.04 mmol) is dissolved in EtOH (144 ml) and sodium borohydride (1000 mg; 26.4 mmol) is added. The reaction mixture is stirred at RT for 24 hours. The mixture is concentrated under reduced pressure. The residue is redissolved in DCM and water is carefully added followed by sat. aq. $NaHCO_3$l. The layers are separated and the aq. layer extracted with DCM (2x). The combined org. extracts are dried over $MgSO_4$, filtered and concentrated under reduced pressure to give 808 mg of (2-(1,1-difluoroethyl)imidazo[5,1-*b*]thiazol-3-yl)methanol. LC-MS (acidic): $t_R$ = 0.45; $[M+H]^+$ = 219.01.

Step 7: Preparation of 2-(1,1-difluoroethyl)imidazo[5,1-*b*]thiazole-3-carbaldehyde

To an ice-cold solution of the product from Step 6, (2-(1,1-difluoroethyl)imidazo[5,1-*b*]thiazol-3-yl)methanol (808 mg; 3.70 mmol) in DCM (30 ml) is added Dess-Martin periodinane (1727 mg; 4.07 mmol). The reaction mixture is stirred at RT for 18 hours. A sat. aq. $NaHCO_3$ solution is added and the mixture stirred for 30 min. The white precipitate is then filtered and the filtrate extracted with DCM (3x). The combined org. extracts are washed with brine, dried over $MgSO_4$, filtered and concentrated under reduced pressure to give 2-(1,1-difluoroethyl)imidazo[5,1-*b*]thiazole-3-carbaldehyde. LC-MS (acidic): $t_R$ = 0.50; $[M+H]^+$ = 216.98.

Step 8: Preparation of *rac*-1-(2-(1,1-difluoroethyl)imidazo[5,1-*b*]thiazol-3-yl)prop-2-yn-1-ol (intermediate 14)

To an ice-cold solution of the product from Step 7, 2-(1,1-difluoroethyl)imidazo[5,1-*b*]thiazole-3-carbaldehyde (648 mg; 3.00 mmol) in THF (15 ml) is added a solution of ethynylmagnesium bromide (0.5 M in THF, 3.3 ml, 6.60 mmol) in a dropwise manner. The reaction mixture is stirred at RT for 3 h. The reaction is quenched by careful addition of aq. ammonium chloride solution. The product is extracted with DCM (3 x 20 ml) and the combined organic extracts are dried over $MgSO_4$, filtered and concentrated under reduced pressure to give 584 mg of *rac*-1-(2-(1,1-difluoroethyl)imidazo[5,1-

*b*]thiazol-3-yl)prop-2-yn-1-ol. $^1$H NMR (400 MHz, DMSO) $\delta$: 8.33 (s, 1 H), 7.17 (s, 1 H), 6.90 (d, *J* = 4.6 Hz, 1 H), 5.85 (dd, *J*1 = 2.3 Hz, *J*2 = 4.5 Hz, 1 H), 3.73 (d, *J* = 2.2 Hz, 1 H), 2.09 (t, *J* = 19.0 Hz).

**Example 258: *rac*-[1-(3-Chloro-1*H*-indazol-6-yl)-1*H*-[1,2,3]triazol-4-yl]-(2-cyclopropyl-imidazo[5,1-*b*]thiazol-3-yl)-methanol**

Step 1: Preparation of 6-azido-3-chloro-1*H*-indazole

Prepared according to the procedure described for Example 45, step 1 using (3-chloro-1*H*-indazol-6-yl)boronic acid.

Step 2: Prepared according to the procedure described for Example 45, step 2 and using intermediate 1, *rac*-1-(2-cyclopropylimidazo[5,1-*b*)]thiazol-3-yl)prop-2-yn-1-ol and 6-azido-3-chloro-1*H*-indazole. Purification by preparative HPLC (basic conditions) to give *rac*-[1-(3-chloro-1*H*-indazol-6-yl)-1*H*-[1,2,3]triazol-4-yl]-(2-cyclopropyl-imidazo[5,1-*b*]thiazol-3-yl)-methanol. LC-MS (QC): $t_R$ = 0.656; $[M+H]^+$ = 412.2.

**Example 259: *rac*-(2-(Cyclopent-1-enyl)-imidazo[5,1-*b*]thiazol-3-yl)-[1-(4-methoxy-phenyl)-1*H*-[1,2,3]triazol-4-yl]-methanol**

Prepared according to the procedure described for Example 45, step 2 and using intermediate 15, *rac*-1-(2-(cyclopent-1-en-1-yl)imidazo[5,1-*b*]thiazol-3-yl)prop-2-yn-1-ol and 1-azido-4-methoxybenzene. Purification by preparative HPLC (acidic conditions) followed by neutralization and extraction with DCM to give *rac*-(2-(cyclopent-1-enyl)-imidazo[5,1-*b*]thiazol-3-yl)-[1-(4-methoxy-phenyl)-1*H*-[1,2,3]triazol-4-yl]-methanol. LC-MS (acidic): $t_R$ = 0.75; $[M+H]^+$ = 393.94.

**Intermediate 15: *rac*-1-(2-(Cyclopent-1-en-1-yl)imidazo[5,1-*b*]thiazol-3-yl)prop-2-yn-1-ol**

Step 1: Preparation of 2-(cyclopent-1-en-1-yl)-*N*-methoxy-*N*-methylimidazo[5,1-*b*]thiazole-3-carboxamide

According to the procedure described for the preparation of Intermediate 13, step 3 but using cyclopent-1-en-1-ylboronic acid as starting material. LC-MS (acidic): $t_R$ = 0.61; $[M+H]^+$ = 278.24. $^1$H NMR (400 MHz, DMSO) $\delta$: 8.10 (s, 1 H), 7.49 (s, 1 H), 7.06 (s, 1 H), 3.59 (m, 3 H), 3.35-3.41 (m, 3 H), 2.45-2.59 (m, 2 H), 2.31 (t, *J* = 7.4 Hz, 2 H), 1.92-2.01 (m, 2 H).

Step 2: Preparation of 2-(cyclopent-1-en-1-yl)imidazo[5,1-*b*]thiazole-3-carbaldehyde According to the procedure described for the preparation of Intermediate 13, step 4 but using 2-(cyclopent-1-en-1-yl)-*N*-methoxy-*N*-methylimidazo[5,1-*b*]thiazole-3-carboxamide as starting material. LC-MS (acidic): $t_R$ = 0.57; $[M+H]^+$ = 218.99.

Step 3: Preparation of *rac*-1-(2-(cyclopent-1-en-1-yl)imidazo[5,1-*b*]thiazol-3-yl)prop-2-yn-1-ol (intermediate 15)

According to the procedure described for the preparation of Intermediate 13, step 5 but using 2-(cyclopent-1-en-1-yl)imidazo[5,1-b]thiazole-3-carbaldehyde as starting material. LC-MS (acidic): $t_R$ = 0.62; $[M+H]^+$ = 245.02. $^1$H NMR (400 MHz, DMSO) $\delta$: 8.24 (s, 1 H), 7.06 (s, 1 H), 6.64 (d, *J* = 4.5 Hz, 1 H), 6.09-6.11 (m, 1 H), 5.80 (dd, $J_1$ = 2.3 Hz, $J_2$ = 4.4 Hz, 1 H), 3.68 (d, *J* = 2.3 Hz, 1 H), 3.30-3.38 (m, 2 H), 2.68 (m, 2 H), 2.00 (m, 2 H).

**Example 260: *rac*-(2-Cyclopropyl-imidazo[5,1-*b*]thiazol-3-yl)-(1-pyridin-3-yl-1*H*-pyrazol-4-yl)-methanol**

Prepared following the procedure described for Example 96 using intermediate 10, 3-bromo-2-cyclopropylimidazo[5,1-*b*]thiazole and 1-(pyridin-3-yl)-1*H*-pyrazole-4-carbaldehyde. Purification by preparative HPLC (acidic conditions) followed by neutralization and extraction with DCM to give *rac*-(2-cyclopropyl-imidazo[5,1-*b*]thiazol-3-yl)-(1-pyridin-3-yl-1*H*-pyrazol-4-yl)-methanol. LC-MS (QC): $t_R$ = 0.483; $[M+H]^+$ = 338.3.

**Example 261: *rac*-[1-(4-Methoxy-phenyl)-1*H*-[1,2,3]triazol-4-yl]-(2-methyl-imidazo[5,1-*b*]thiazol-3-yl)-methanol**

Prepared according to the procedure described for Example 45, step 2 and using intermediate 2, *rac*-1-(2-methylimidazo[5,1-*b*)]thiazol-3-yl)prop-2-yn-1-ol and 1-azido-4-methoxybenzene. Purification by preparative HPLC (basic conditions) to give *rac*-[1-(4-methoxy-phenyl)-1*H*-[1,2,3]triazol-4-yl]-(2-methyl-imidazo[5,1-*b*]thiazol-3-yl)-methanol. LC-MS (QC): $t_R$ = 0.522; $[M+H]^+$ = 342.3.

**Example 262: *rac*-[1-(4-Methoxy-phenyl)-1*H*-[1,2,3]triazol-4-yl]-[2-(2-methyl-propenyl)-imidazo[5,1-*b*]thiazol-3-yl]-methanol**

Prepared according to the procedure described for Example 45, step 2 and using intermediate 16, *rac*-1-(2-(2-methylprop-1-en-1-yl)imidazo[5,1-*b*]thiazol-3-yl)prop-2-yn-1-ol and 1-azido-4-methoxybenzene. Purification by preparative HPLC (acidic conditions) followed by neutralization and extraction with DCM to give *rac*-[1-(4-methoxy-phenyl)-1*H*-1,2,3]triazol-4-yl]-[2-(2-methyl-propenyl)-imidazo[5,1-*b*]thiazol-3-yl]-methanol. LC-MS (QC): $t_R$ = 0.699; $[M+H]^+$ = 382.1.

**Intermediate 16: *rac*-1-(2-(2-Methylprop-1-en-1-yl)imidazo[5,1-*b*]thiazol-3-yl)prop-2-yn-1-ol**

Step 1: Preparation of *N*-methoxy-*N*-methyl-2-(2-methylprop-1-en-1-yl)imidazo[5,1-*b*]thiazole-3-carboxamide

According to the procedure described for the preparation of Intermediate 13, step 3 but using (2-methylprop-1-en-1-yl)boronic acid as starting material. LC-MS (acidic): $t_R$ = 0.57; $[M+H]^+$ = 266.02. $^1$H NMR (400 MHz, DMSO) $\delta$: 8.08 (s, 1 H), 7.08 (s, 1 H), 6.24 (m, 1 H), 3.60 (s, 3 H), 3.33 (s, 3 H), 1.94 (d, *J* = 0.7 Hz, 3 H), 1.92 (d, *J* = 0.8 Hz, 3 H).

Step 2: Preparation of 2-(2-methylprop-1-en-1-yl)imidazo[5,1-*b*]thiazole-3-carbaldehyde According to the procedure described for the preparation of Intermediate 13, step 4 but using *N*-methoxy-*N*-methyl-2-(2-methylprop-1-en-1-yl)imidazo[5,1-*b*)]thiazole-3-carboxamide as starting material. LC-MS (acidic): $t_R$ = 0.53; $[M+H]^+$ = 207.01.

Step 3: Preparation of *rac*-1-(2-(2-methylprop-1-en-1-yl)imidazo[5,1-*b*]thiazol-3-yl)prop-2-yn-1-ol (intermediate 16)

According to the procedure described for the preparation of Intermediate 13, step 5 but using 2-(2-methylprop-1-en-1-yl)imidazo[5,1-*b*]thiazole-3-carbaldehyde as starting material. LC-MS (acidic): $t_R$ = 0.58; $[M+H]^+$ = 233.02. $^1$H NMR (400 MHz, DMSO) $\delta$: 8.21 (s, 1 H), 7.06 (s, 1 H), 6.53 (d, *J* = 4.5 Hz, 1 H), 6.31 (m, 1 H), 5.69 (m, 1 H), 3.63 (d, *J* = 2.3 Hz, 1 H), 1.93 (d, *J* = 0.8 Hz, 3 H), 1.87 (d, *J* = 0.8 Hz, 3 H).

**Example 263: *rac*-[1-(4-Methoxy-phenyl)-1*H*-[1,2,3]triazol-4-yl]-[((*E*)-2-(prop-1-enyl))-imidazo[5,1-b]thiazol-3-yl]-methanol**

Prepared according to the procedure described for Example 45, step 2 and using intermediate 17, *rac*-(E)-1-(2-(prop-1-en-1-yl)imidazo[5,1-*b*]thiazol-3-yl)prop-2-yn-1-ol and 1-azido-4-methoxybenzene. Purification by preparative HPLC (acidic conditions) followed by neutralization and extraction with DCM to give *rac*-[1-(4-methoxy-phenyl)-1*H*-[1,2,3]triazol-4-yl]-[((*E*)-2-(prop-1-enyl))-imidazo[5,1-*b*]thiazol-3-yl]-methanol. LC-MS (QC): $t_R$ = 0.661; $[M+H]^+$ = 368.3.

**Intermediate 17: *rac*-(*E*)-1-(2-(Prop-1-en-1-yl)imidazo[5,1-*b*]thiazol-3-yl)prop-2-yn-1-ol**

Step 1: Preparation of (*E*)-*N*-methoxy-*N*-methyl-2-(prop-1-en-1-yl)imidazo[5,1-*b*]thiazole-3-carhoxamide

According to the procedure described for the preparation of Intermediate 13, step 3 but using (*E*)-prop-1-en-1-ylboronic acid as starting material. LC-MS (acidic): $t_R$ = 0.54; $[M+H]^+$ = 252.01. $^1$H NMR (400 MHz, DMSO) $\delta$: 8.06 (s, 1 H), 7.06 (s, 1 H), 6.55 (dd, $J_1$ = 0.8 Hz, $J_2$ = 15.5 Hz, 1 H), 6.13 (m, 1 H), 3.60 (s, 3 H), 3.35 (s, 3 H), 1.89 (d, *J* = 6.7 Hz, 3 H).

Step 2: Preparation of (*E*)-2-(prop-1-en-1-yl)imidazo[5,1-*b*]thiazole-3-carbaldehyde According to the procedure described for the preparation of Intermediate 13, step 4 but using (*E*)-*N*-methoxy-*N*-methyl-2-(prop-1-en-1-yl)imidazo[5,1-*b*]thiazole-3-carboxamide as starting material. LC-MS (acidic): $t_R$ = 0.47; $[M+H]^+$ = 193.00.

Step 3: Preparation of *rac*-(*E*)-1-(2-(prop-1-en-1-yl)imidazo[5,1-*b*]thiazol-3-yl)prop-2-yn-1-ol (intermediate 17)

According to the procedure described for the preparation of Intermediate 13, step 5 but using (*E*)-2-(prop-1-en-1-yl)imidazo[5,1-b]thiazole-3-carbaldehyde as starting material. LC-MS (acidic): $t_R$ = 0.53; $[M+H]^+$ = 219.01. $^1$H NMR (400 MHz, DMSO) $\delta$: 8.19 (s, 1 H), 7.04 (s, 1 H), 6.88-6.92 (m, 1 H), 6.56 (d, *J* = 4.3 Hz, 1 H), 5.97 (m, 2 H), 3.65 (d, *J* = 2.2 Hz, 1 H), 1.88 (d, *J* = 6.6 Hz, 3 H).

**Example 264: *rac*-2-Chloro-4-{4-[hydroxy-(2-phenyl-imidazo[5,1-*b*]thiazol-3-yl)-methyl]-[1,2,3]triazol-1-yl}-phenol**

Step 1: Preparation of 4-azido-2-chlorophenol

Prepared according to the procedure described for Example 45, step 1 using (3-chloro-4-hydroxyphenyl)boronic acid.

Step 2: Prepared according to the procedure described for Example 45, step 2 and using intermediate 13, *rac*-1-(2-phenylimidazo[5,1-*b*)]thiazol-3-yl)prop-2-yn-1-ol and 4-azido-2-chlorophenol. Purification by preparative HPLC (acidic conditions) to give *rac*-2-chloro-4-{4-[hydroxy-(2-phenyl-imidazo[5,1-*b*]thiazol-3-yl)-methyl]-[1,2,3]triazol-1-yl}-phenol. LC-MS (QC): $t_R$ = 0.718; $[M+H]^+$ = 424.2.

**Example 265: *rac*-[1-(3-Chloro-4-cyclopropylmethoxy-phenyl)-1*H*-[1,2,3]triazol-4-yl]-(2-phenyl-imidazo[5,1-*b*]thiazol-3-yl)-methanol**

Prepared following the procedure described for Example 87, using *rac*-2-chloro-4-{4-[hydroxy-(2-phenyl-imidazo[5,1-*b*]thiazol-3-yl)-methyl]-[1,2,3]triazol-1-yl}-phenol and (bromomethyl)cyclopropane. Purification by preparative HPLC (acidic conditions) gives *rac*-[1-(3-chloro-4-cyclopropylmethoxy-phenyl)-1*H*-[1,2,3]triazol-4-yl]-(2-phenyl-imidazo[5,1-*b*]thiazol-3-yl)-methanol. LC-MS (QC): $t_R$ = 0.966; $[M+H]^+$ = 478.3.

**Example 266: *rac*-{1-[3-Chloro-4-(3-fluoro-oxetan-3-ylmethoxy)-phenyl]-1*H*-[1,2,3]triazol-4-yl}-(2-phenyl-imidazo[5,1-*b*]thiazol-3-yl)-methanol**

Prepared following the procedure described for Example 87, using *rac*-2-chloro-4-{4-[hydroxy-(2-phenyl-imidazo[5,1-*b*]thiazol-3-yl)-methyl]-[1,2,3]triazol-1-yl}-phenol and 3-(bromomethyl)-3-fluorooxetane. Purification by preparative HPLC (acidic conditions) gives *rac*-{1-[3-chloro-4-(3-fluoro-oxetan-3-ylmethoxy)-phenyl]-1*H*-[1,2,3]triazol-4-yl}-(2-phenyl-imidazo[5,1-*b*]thiazol-3-yl)-methanol. LC-MS (QC): $t_R$ = 0.840; $[M+H]^+$ = 512.2.

**Example 267: *rac*-3-(2-Chloro-4-{4-[hydroxy-(2-phenyl-imidazo[5,1-*b*]thiazol-3-yl)-methyl]-[1,2,3]triazol-1-yl}-phenoxy)-propan-1-ol**

Prepared following the procedure described for Example 87, using *rac*-2-chloro-4-{4-[hydroxy-(2-phenyl-imidazo[5,1-*b*)]thiazol-3-yl)-methyl]-[1,2,3]triazol-1-yl}-phenol and 3-bromopropan-1-ol. Purification by preparative HPLC (acidic conditions) followed by purification by preparative HPLC (basic conditions) gives *rac*-3-(2-chloro-4-{4-[hydroxy-(2-phenyl-imidazo[5,1-*b*]thiazol-3-yl)-methyl]-[1,2,3]triazol-1-yl}-phenoxy)-propan-1-ol. LC-MS (QC): $t_R$ = 0.761; $[M+H]^+$ = 482.2.

**Example 268: *rac*-4-(2-Chloro-4-{4-[hydroxy-(2-phenyl-imidazo[5,1-*b*]thiazol-3-yl)-methyl]-[1,2,3]triazol-1-yl}-phenoxy)-2-methyl-butan-2-ol**

Prepared following the procedure described for Example 87, using *rac*-2-chloro-4-{4-[hydroxy-(2-phenyl-imidazo[5,1-*b*]thiazol-3-yl)-methyl]-[1,2,3]triazol-1-yl}-phenol and 4-bromo-2-methylbutan-2-ol. Purification by preparative HPLC (acidic conditions) gives *rac*-4-(2-chloro-4-{4-[hydroxy-(2-phenyl-imidazo[5,1-*b*]thiazol-3-yl)-methyl]-[1,2,3]triazol-1-yl}-phenoxy)-2-methyl-butan-2-ol. LC-MS (QC): $t_R$ = 0.853; $[M+H]^+$ = 510.3.

**Example 268a: 4-(2-Chloro-4-{4-[(*R*)-hydroxy-(2-phenyl-imidazo[5,1-*b*]thiazol-3-yl)-methyl]-[1,2,3]triazol-1-yl}-phenoxy)-2-methyl-butan-2-ol**

Separation of the enantiomers on chiral stationary phase:
Method: Column: ChiralPak IA 20x250mm, 5$\mu$M; Detector Settings: UV-Vis-1; 210 nM; Eluent: 80% $CH_3CN$ and 20% EtOH; Flow: 16.00 ml/min; Injection volume: 1000$\mu$l.

20 mg of the racemate are separated by the method described above to give 4.2 mg of the R-enantiomer Example 268a and 4.3 mg of the S-enantiomer. LC-MS (QC): $t_R$ = 0.841; $[M+H]^+$ = 510.3.

**Example 269: *rac*-(2-Isopropenyl-imidazo[5,1-*b*]thiazol-3-yl)-[1-(4-methoxy-phenyl)-1*H*-[1,2,3]triazol-4-yl]-methanol**

Prepared according to the procedure described for Example 45, step 2 and using intermediate 18, *rac*-1-(2-(prop-1-en-2-yl)imidazo[5,1-*b*]thiazol-3-yl)prop-2-yn-1-ol and 1-azido-4-methoxybenzene. Purification by preparative HPLC (acidic conditions) to give *rac*-(2-isopropenyl-imidazo[5,1-*b*]thiazol-3-yl)-[1-(4-methoxyphenyl)-1*H*-[1,2,3]triazol-4-yl]-methanol. LC-MS (QC): $t_R$ = 0.666; $[M+H]^+$ = 368.3.

**Intermediate 18: *rac*-1-(2-(Prop-1-en-2-yl)imidazo[5,1-*b*]thiazol-3-yl)prop-2-yn-1-ol**

Step 1: Preparation of *N*-methoxy-*N*-methyl-2-(prop-1-en-2-yl)imidazo[5,1-*b*]thiazole-3-carboxamide According to the procedure described for the preparation of Intermediate 13, step 3 but using prop-1-en-2-ylboronic acid as starting material. LC-MS (acidic): $t_R$ = 0.52; $[M+H]^+$ = 251.98. $^1$H NMR (400 MHz, DMSO) $\delta$: 8.12 (s, 1 H), 7.08 (s, 1 H), 5.32 (s, 2 H), 3.60 (s, 3 H), 3.35 (s, 3 H), 2.05 (s, 3 H).

Step 2: Preparation of (E)-2-(prop-1-en-1-yl)imidazo[5,1-b]thiazole-3-carbaldehyde According to the procedure described for the preparation of Intermediate 13, step 4 but using *N*-methoxy-*N*-methyl-2-(prop-1-en-2-yl)imidazo[5,1-*b*]thiazole-3-carboxamide as starting material. LC-MS (acidic): $t_R$ = 0.46; $[M+H]^+$ = 193.02.

Step 3: Preparation of *rac*-1-(2-(prop-1-en-2-yl)imidazo[5,1-*b*]thiazol-3-yl)prop-2-yn-1-ol (intermediate 18)

According to the procedure described for the preparation of Intermediate 13, step 5 but using 2-(prop-1-en-2-yl)imidazo[5,1-b]thiazole-3-carbaldehyde as starting material. LC-MS (acidic): $t_R$ = 0.52; $[M+H]^+$ = 219.01. $^1$H NMR (400 MHz, DMSO) $\delta$: 8.25 (d, J = 0.3 Hz, 1 H), 7.09 (d, J = 0.5 Hz, 1 H), 6.66 (d, J = 4.5 Hz, 1 H), 5.71 (m, 1 H), 5.46 (t, J = 1.4 Hz, 1 H), 5.24 (s, 1 H), 3.69 (d, *J* = 2.3 Hz, 1 H), 2.05-2.07 (m, 3 H).

**Example 270: *rac*-[2-((*E*)-2-Cyclopropyl-vinyl)-imidazo[5,1-*b*]thiazol-3-yl]-[1-(4-methoxy-phenyl)-1*H*-[1,2,3]triazol-4-yl]-methanol**

Prepared according to the procedure described for Example 45, step 2 and using intermediate 19, *rac*-1-(2-(-(*E*)-2-cyclopropylvinyl)imidazo[5,1-*b*]thiazol-3-yl)prop-2-yn-1-ol and 1-azido-4-methoxybenzene. Purification by preparative HPLC (acidic conditions) to give rac-[2-((E)-2-cyclopropyl-vinyl)-imidazo[5,1-b]thiazol-3-yl]-[1-(4-methoxy-phenyl)-1*H*-[1,2,3]triazol-4-yl]-methanol. LC-MS (QC): $t_R$ = 0.734; $[M+H]^+$ = 394.3.

**Intermediate 19: *rac*-1-(2-(-(*E*)-2-Cyclopropylvinyl)imidazo[5,1-*b*]thiazol-3-yl)prop-2-yn-1-ol**

Step 1: Preparation of (*E*)-2-(2-cyclopropylvinyl)-*N*-methoxy-*N*-methylimidazo[5,1-*b*]thiazole-3-carboxamide

According to the procedure described for the preparation of Intermediate 13, step 3 but using (E)-(2-cyclopropylvinyl)boronic acid as starting material. LC-MS (acidic): $t_R$ = 0.61; $[M+H]^+$ = 277.98. $^1$H NMR (400 MHz, DMSO) $\delta$: 8.04 (s, 1 H), 7.04 (s, 1 H), 6.61 (d, *J* = 15.5 Hz, 1 H), 5.62-5.68 (m, 1 H), 3.61 (s, 3 H), 3.36 (s, 3 H), 1.66-1.70 (m, 1 H), 0.86-0.88 (m, 2 H), 0.60-0.64 (m, 2 H).

Step 2: Preparation of (E)-2-(2-cyclopropylvinyl)imidazo[5,1-b]thiazole-3-carbaldehyde According to the procedure described for the preparation of Intermediate 13, step 4 but using (*E*)-2-(2-cyclopropylvinyl)-*N*-methoxy-*N*-methylimidazo[5,1-*b*]thiazole-3-carboxamide as starting material. LC-MS (acidic): $t_R$ = 0.56; $[M+H]^+$ = 218.99.

Step 3: Preparation of *rac*-1-(2-(-(*E*)-2-cyclopropylvinyl)imidazo[5,1-*b*]thiazol-3-yl)prop-2-yn-1-ol (intermediate 19)

According to the procedure described for the preparation of Intermediate 13, step 5 but using (*E*)-2-(2-cyclopropylvinyl)imidazo[5,1-b]thiazole-3-carbaldehyde as starting material. LC-MS (acidic): $t_R$ = 0.60; $[M+H]^+$ = 245.00.

**Example 271: *rac*-[1-(4-Methoxy-phenyl)-1*H*-[1,2,3]triazol-4-yl]-[((*E*)-2-(pent-1-enyl))-imidazo[5,1-*b*]thiazol-3-yl]-methanol**

Prepared according to the procedure described for Example 45, step 2 and using intermediate 20, rac-1-((E)-2-(pent-1-en-1-yl)imidazo[5,1-*b*]thiazol-3-yl)prop-2-yn-1-ol and 1-azido-4-methoxybenzene. Purification by preparative HPLC (acidic conditions) to give *rac*-[1-(4-methoxy-phenyl)-1*H*-[1,2,3]triazol-4-yl]-[((*E*)-2-(pent-1-enyl))-imidazo[5,1-b]thiazol-3-yl]-methanol. LC-MS (QC): $t_R$ = 0.811; $[M+H]^+$ = 396.2.

**Intermediate 20: *rac*-1-((*E*)-2-(Pent-1-en-1-yl)imidazo[5,1-*b*]thiazol-3-yl)prop-2-yn-1-ol**

Step 1: Preparation of (*E*)-*N*-methoxy-*N*-methyl-2-(pent-1-en-1-yl)imidazo[5,1-*b*]thiazole-3-carboxamide

According to the procedure described for the preparation of Intermediate 13, step 3 but using (E)-pent-1-en-1-ylboronic acid as starting material. LC-MS (acidic): $t_R$ = 0.66; $[M+H]^+$ = 280.02. $^1$H NMR (400 MHz, DMSO) $\delta$: 8.06 (s, 1 H), 7.06 (s, 1 H), 6.51-6.55 (m, 1 H), 6.11 (m, 1 H), 3.60 (s, 3 H), 3.36 (s, 3 H), 2.18-2.24 (m, 2 H), 1.46 (m, 2 H), 0.91 (t, *J* = 7.3 Hz, 3 H).

Step 2: Preparation of (E)-2-(pent-1-en-1-yl)imidazo[5,1-b]thiazole-3-carbaldehyde According to the procedure described for the preparation of Intermediate 13, step 4 but using (*E*)-*N*-methoxy-*N*-methyl-2-(pent-1-en-1-yl)imidazo[5,1-*b*]thiazole-3-carboxamide as starting material. LC-MS (acidic): $t_R$ = 0.62; $[M+H]^+$ = 221.02.

Step 3: Preparation of *rac*-1-((*E*)-2-(pent-1-en-1-yl)imidazo[5,1-*b*]thiazol-3-yl)prop-2-yn-1-ol (intermediate 20) According to the procedure described for the preparation of Intermediate 13, step 5 but using (E)-2-(pent-1-en-1-yl)imidazo[5,1-b]thiazole-3-carbaldehyde as starting material. LC-MS (acidic): $t_R$ = 0.65; $[M+H]^+$ = 247.02.

**Example 272: *rac*-[1-(4-Amino-3-chloro-phenyl)-1*H*-[1,2,3]triazol-4-yl]-(2-ethyl-imidazo[5,1-*b*]thiazol-3-yl)-methanol**

Step 1: Preparation of 4-azido-2-chloroaniline

Prepared according to the procedure described for Example 45, step 1 using 2-chloro-4-(4,4,5,5-tetramethyl-1,3,2-dioxaborolan-2-yl)aniline.

Step 2: Prepared according to the procedure described for Example 45, step 2 and using intermediate 3, *rac*-1-(2-ethylimidazo[5,1-*b*]thiazol-3-yl)prop-2-yn-1-ol and 4-azido-2-chloroaniline. Purification by preparative HPLC (basic conditions) to give *rac*-[1-(4-amino-3-chloro-phenyl)-1*H*-[1,2,3]triazol-4-yl]-(2-ethyl-imidazo[5,1-*b*]thiazol-3-yl)-methanol. LC-MS (QC): $t_R$ = 0.557; $[M+H]^+$ = 375.2.

**Example 273: *rac*-[1-(4-Methoxy-pheny))-1*H*-[1,2,3]triazol-4-yl]-[2-(1-methyl-1H-pyrazol-4-yl)-imidazo[5,1-*b*]thiazol-3-yl]-methanol**

Prepared according to the procedure described for Example 45, step 2 and using intermediate 21, rac-1-(2-(1-methyl-1*H*-pyrazol-4-yl)imidazo[5,1-*b*]thiazol-3-yl)prop-2-yn-1-ol and 1-azido-4-methoxybenzene. Purification by preparative HPLC (basic conditions) to give *rac*-[1-(4-methoxy-phenyl)-1*H*-[1,2,3]triazol-4-yl]-[2-(1-methyl-1H-pyrazol-4-yl)-imidazo[5,1-*b*]thiazol-3-yl]-methanol. LC-MS (QC): $t_R$ = 0.573; $[M+H]^+$ = 408.3.

**Intermediate 21: *rac*-1-(2-(1-Methyl-1*H*-pyrazol-4-yl)imidazo[5,1-*b*]thiazol-3-yl)prop-2-yn-1-ol**

Step 1: Preparation of *N*-methoxy-*N*-methyl-2-(1-methyl-1*H*-pyrazol-4-yl)imidazo[5,1-*b*]thiazole-3-carboxamide

According to the procedure described for the preparation of Intermediate 13, step 3 but using 1-methylpyrazole-4-boronic acid pinacol ester as starting material. LC-MS (acidic): $t_R$ = 0.49; $[M+H]^+$ = 291.94. $^1$H NMR (400 MHz, DMSO) $\delta$: 8.12 (s, 1 H), 8.08 (s, 1 H), 7.65 (s, 1 H), 7.09 (s, 1 H), 3.89 (s, 3 H), 3.54 (s, 3 H), 3.32 (s, 3 H).

Step 2: Preparation of 2-(1-methyl-1*H*-pyrazol-4-yl)imidazo[5,1-*b*]thiazole-3-carbaldehyde

According to the procedure described for the preparation of Intermediate 13, step 4 but using *N*-methoxy-*N*-methyl-2-(1-methyl-1*H*-pyrazol-4-yl)imidazo[5,1-*b*]thiazole-3-carboxamide as starting material. LC-MS (acidic): $t_R$ = 0.46; $[M+H]^+$ = 232.96. $^1$H NMR (400 MHz, DMSO) $\delta$: 9.93 (s, 1 H), 8.63 (s, 1 H), 8.49 (s, 1 H), 8.05 (s, 1 H), 7.21 (d, J = 0.4 Hz, 1 H), 3.94 (s, 3 H).

Step 3: Preparation of *rac*-1-(2-(1-methyl-1*H*-pyrazol-4-yl)imidazo[5,1-*b*]thiazol-3-yl)prop-2-yn-1-ol (intermediate 21)

According to the procedure described for the preparation of Intermediate 13, step 5 but using 2-(1-methyl-1*H*-pyrazol-4-yl)imidazo[5,1-*b*]thiazole-3-carbaldehyde as starting material. LC-MS (acidic): $t_R$ = 0.49; $[M+H]^+$ = 258.87. $^1$H NMR (400 MHz, DMSO) $\delta$: 8.26 (s, 1 H), 8.09 (s, 1 H), 7.68 (s, 1 H), 7.10 (s, 1 H), 6.64 (d, J = 4.6 Hz, 1 H), 5.70 (dd, $J_1$ = 2.3 Hz, $J_2$ = 4.6 Hz, 1 H), 3.91 (s, 3 H), 3.69 (d, J = 2.3 Hz, 1 H).

**Example 274: *rac*-[5-*tert*-Butyl-1-(4-methoxy-phenyl)-1*H*-[1,2,3]triazol-4-yl]-(2-cyclopropyl-imidazo[5,1-*b*]thiazol-3-yl)-methanol**

Step 1: Preparation of methyl 5-(*tert*-butyl)-1-(4-methoxyphenyl)-1*H*-1,2,3-triazole-4-carboxylate According to the procedure described for the preparation of example 279, step 1 but using methyl 4,4-dimethyl-3-oxopentanoate as starting material. LC-MS (acidic): $t_R$ = 0.94; $[M+H]^+$ = 289.85.

Step 2: Preparation of (5-(*tert*-butyl)-1-(4-methoxyphenyl)-1*H*-1,2,3-triazol-4-yl)methanol According to the procedure described for the preparation of example 279, step 2 but using methyl 5-(*tert*-butyl)-1-(4-methoxyphenyl)-1*H*-1,2,3-triazole-4-carboxylate as starting material. (5-(*tert*-butyl)-1-(4-methoxyphenyl)-1*H*-1,2,3-triazol-4-yl)methanol is obtained as a white solid. LC-MS (acidic): $t_R$ = 0.76, $[M+H]^+$ = 262.08.

Step 3: Preparation of 5-(*tert*-butyl)-1-(4-methoxyphenyl)-1*H*-1,2,3-triazole-4-carbaldehyde

Prepared following the procedure described for Example 222, step 3 but using (5-(*tert*-butyl)-1-(4-methoxyphenyl)-1*H*-1,2,3-triazol-4-yl)methanol. 5-(*tert*-Butyl)-1-(4-methoxyphenyl)-1*H*-1,2,3-triazole-4-carbaldehyde is obtained as a white solid. LC-MS (acidic): $t_R$ = 0.92, $[M+H]^+$ = 260.03.

Step 4: Preparation of *rac*-[5-*tert*-butyl-1-(4-methoxy-phenyl)-1*H*-[1,2,3]triazol-4-yl]-(2-cyclopropyl-imidazo[5,1-*b*]thiazol-3-yl)-methanol (Example 274)

Prepared following the procedure described for Example 96 using intermediate 10, 3-bromo-2-cyclopropylimidazo[5,1-*b*]thiazole and 5-(*tert*-butyl)-1-(4-methoxyphenyl)-1*H*-1,2,3-triazole-4-carbaldehyde. Purification by preparative HPLC (basic conditions) to give *rac*-[5-*tert*-butyl-1-(4-methoxy-phenyl)-1*H*-[1,2,3]triazol-4-yl]-(2-cyclopropyl-imidazo[5,1-b]thiazol-3-yl)-methanol. LC-MS (QC): $t_R$ = 0.750; $[M+H]^+$ = 424.2.

**Example 275: 3-(2-Chloro-4-{4-[(*R*)-(2-cyclopropyl-imidazo[5,1-*b*]thiazol-3-yl)-hydroxy-methyl]-[1,2,3]triazol-1-yl}-phenoxy)-propan-1-ol**

Prepared following the procedure described for Example 87, using 2-chloro-4-{4-[(*R*)-(2-cyclopropyl-imidazo[5,1-b]thiazol-3-yl)-hydroxy-methyl]-[1,2,3]triazol-1-yl}-phenol and 3-bromopropan-1-ol. Purification by preparative HPLC (basic conditions) gives 3-(2-chloro-4-{4-[(R)-(2-cyclopropyl-imidazo[5,1-b]thiazol-3-yl)-hydroxy-methyl]-[1,2,3]triazol-1-yl}-phenoxy)-propan-1-ol. LC-MS (QC): $t_R$ = 0.634; $[M+H]^+$ = 446.3.

**Example 276: (*R*)-{1-[3-Chloro-4-(tetrahydro-pyran-4-ylmethoxy)-phenyl]-1*H*-[1,2,3]triazol-4-yl}-(2-cyclopropyl-imidazo[5,1-*b*]thiazol-3-yl)-methanol**

Prepared following the procedure described for Example 87, using 2-chloro-4-{4-[(*R*)-(2-cyclopropyl-imidazo[5,1-b]thiazol-3-yl)-hydroxy-methyl]-[1,2,3]triazol-1-yl}-phenol and 4-(bromomethyl)tetrahydro-2*H*-pyran. Purification by preparative HPLC (basic conditions) gives (*R*)-{1-[3-chloro-4-(tetrahydro-pyran-4-ylmethoxy)-phenyl]-1*H*-[1,2,3]triazol-4-yl}-(2-cyclopropyl-imidazo[5,1-*b*]thiazol-3-yl)-methanol. LC-MS (QC): $t_R$ = 0.777; $[M+H]^+$ = 486.3.

**Example 277: 4-(2-Chloro-4-{4-[(*R*)-(2-cyclopropyl-imidazo[5,1-*b*]thiazol-3-yl)-hydroxy-methyl]-[1,2,3]triazol-1-yl}-phenoxy)-2-methyl-butan-2-ol**

Prepared following the procedure described for Example 87, using 2-chloro-4-{4-[(*R*)-(2-cyclopropyl-imidazo[5,1-*b*]thiazol-3-yl)-hydroxy-methyl]-[1,2,3]triazol-1-yl}-phenol and 4-bromo-2-methylbutan-2-ol. Purification by preparative HPLC (basic conditions) gives 4-(2-chloro-4-{4-[(R)-(2-cyclopropyl-imidazo[5,1-*b*]thiazol-3-yl)-hydroxy-methyl]-[1,2,3]triazol-1-yl}-phenoxy)-2-methyl-butan-2-ol. LC-MS (QC): $t_R$ = 0.724; $[M+H]^+$ = 474.3.

**Example 278: *rac*-[1-(4-Amino-3-chloro-phenyl)-1*H*-[1,2,3]triazol-4-yl]-(2-cyclopropyl-imidazo[5,1-*b*]thiazol-3-yl)-methanol**

Step 1: Preparation of 4-azido-2-chloroaniline

Prepared according to the procedure described for Example 45, step 1 using 2-chloro-4-(4,4,5,5-tetramethyl-1,3,2-dioxaborolan-2-yl)aniline.

Step 2: Prepared according to the procedure described for Example 45, step 2 and using intermediate 1, *rac-1*-(2-cyclopropylimidazo[5,1-*b*]thiazol-3-yl)prop-2-yn-1-ol and 4-azido-2-chloroaniline. Purification by preparative HPLC

(basic conditions) to give *rac*-[1-(4-amino-3-chloro-phenyl)-1*H*-[1,2,3]triazol-4-yl]-(2-cyclopropyl-imidazo[5,1-*b*]thiazol-3-yl)-methanol. LC-MS (QC): $t_R$ = 0.581; $[M+H]^+$ = 387.3.

**Example 279: rac-(2-Cyclopropyl-imidazo[5,1-b]thiazol-3-yl)-[5-ethyl-1-(4-methoxy-phenyl)-1H-[1,2,3]triazol-4-yl]-methanol**

Step 1: Preparation of methyl 5-ethyl-1-(4-methoxyphenyl)-1*H*-1,2,3-triazole-4-carboxylate

A suspension of methyl 3-oxovalerate (0.26 ml; 2.00 mmol), 1-azido-4-methoxybenzene (298 mg; 2.00 mmol) and $K_2CO_3$ (1106 mg; 8.00 mmol) in DMSO (1 ml) is stirred at 50°C for 1 h. The mixture is cooled down to RT, and water (4 ml) is added. The mixture is cooled to 5°C and the resulting solid is filtered and washed with water to give methyl 5-ethyl-1-(4-methoxyphenyl)-1*H*-1,2,3-triazole-4-carboxylate. LC-MS (acidic): $t_R$ = 0.86, $[M+H]^+$ = 262.01.

Step 2: Preparation of (5-ethyl-1-(4-methoxyphenyl)-1*H*-1,2,3-triazol-4-yl)methanol

To a solution of methyl 5-ethyl-1-(4-methoxyphenyl)-1*H*-1,2,3-triazole-4-carboxylate (395 mg; 1.51 mmol) in MeOH (4 ml) is added $NaBH_4$ (114 mg; 3.02 mmol) at RT. The reaction mixture is stirred at RT for 1 h. More $NaBH_4$ is added and the mixture stirred at RT until completion of the reaction. MeOH (3 ml) is added and the mixture concentrated under reduced pressure. The solid is partitioned between DCM (10 ml) and sat. aq. $NaHCO_3$ (10 ml). The layers are seprarated and the aq. layer extracted with DCM (2 x). The combined org. extracts are dried (MgSO4), filtrered and concentrated under reduced pressure to give (5-ethyl-1-(4-methoxyphenyl)-1*H*-1,2,3-triazol-4-yl)methanol as a beige solid. LC-MS (acidic): $t_R$ = 0.67, $[M+H]^+$ = 234.02.

Step 3: Preparation of 5-ethyl-1-(4-methoxyphenyl)-1*H*-1,2,3-triazole-4-carbaldehyde

Prepared following the procedure described for Example 222, step 3 but using (5-ethyl-1-(4-methoxyphenyl)-1*H*-1,2,3-triazol-4-yl)methanol. 5-Ethyl-1-(4-methoxyphenyl)-1H-1,2,3-triazole-4-carbaldehyde is obtained as a pale orange solid. LC-MS (acidic): $t_R$ = 0.84, $[M+H]^+$ = 232.06.

Step 4: Preparation of *rac*-(2-cyclopropyl-imidazo[5,1-*b*]thiazol-3-yl)-[5-ethyl-1-(4-methoxy-phenyl)-1*H*-[1,2,3]triazol-4-yl]-methanol (Example 279)

Prepared following the procedure described for Example 96 using intermediate 10, 3-bromo-2-cyclopropylimidazo[5,1-*b*]thiazole and 5-ethyl-1-(4-methoxyphenyl)-1H-1,2,3-triazole-4-carbaldehyde. Purification by preparative HPLC (basic conditions) followed by purification by preparative HPLC (acidic conditions) to give *rac*-(2-cyclopropyl-imidazo[5,1-*b*]thiazol-3-yl)-[5-ethyl-1-(4-methoxy-phenyl)-1*H*-[1,2,3]triazol-4-yl]-methanol. LC-MS (QC): $t_R$ = 0.672; $[M+H]^+$ = 396.2.

**Example 280: *rac*-(2-Cyclopropyl-imidazo[5,1-*b*]thiazol-3-yl)-[5-isopropyl-1-(4-methoxy-phenyl)-1*H*-[1,2,3]triazol-4-yl]-methanol**

Step 1: Preparation of methyl 5-isopropyl-1-(4-methoxyphenyl)-1*H*-1,2,3-triazole-4-carboxylate According to the procedure described for the preparation of example 279, step 1 but using methyl 4-methyl-3-oxopentanoate as starting material. LC-MS (acidic): $t_R$ = 0.91; $[M+H]^+$ = 276.09.

Step 2: Preparation of (5-isopropyl-1-(4-methoxyphenyl)-1*H*-1,2,3-triazol-4-yl)methanol According to the procedure described for the preparation of example 279, step 2 but using methyl 5-isopropyl-1-(4-methoxyphenyl)-1*H*-1,2,3-triazole-4-carboxylate as starting material. (5-Isopropyl-1-(4-methoxyphenyl)-1H-1,2,3-triazol-4-yl)methanol is obtained as a brown solid. LC-MS (acidic): $t_R$ = 0.73, $[M+H]^+$ = 248.24.

Step 3: Preparation of 5-isopropyl-1-(4-methoxyphenyl)-1H-1,2,3-triazole-4-carbaldehyde

Prepared following the procedure described for Example 222, step 3 but using (5-isopropyl-1-(4-methoxyphenyl)-1*H*-1,2,3-triazol-4-yl)methanol. 5-Isopropyl-1-(4-methoxyphenyl)-1*H*-1,2,3-triazole-4-carbaldehyde is obtained as an orange solid. LC-MS (acidic): $t_R$ = 0.90, $[M+H]^+$ = 246.04.

Step 4: Preparation of rac-(2-cyclopropyl-imidazo[5,1-b]thiazol-3-yl)-[5-isopropyl-1-(4-methoxy-phenyl)-1H-[1,2,3]triazol-4-yl]-methanol (Example 280)

Prepared following the procedure described for Example 96 using intermediate 10, 3-bromo-2-cyclopropylimidazo[5,1-*b*]thiazole and 5-isopropyl-1-(4-methoxyphenyl)-1*H*-1,2,3-triazole-4-carbaldehyde. Purification by preparative HPLC (acidic conditions) to give *rac*-(2-cyclopropyl-imidazo[5,1-*b*]thiazol-3-yl)-[5-isopropyl-1-(4-methoxy-phenyl)-1H-[1,2,3]triazol-4-yl]-methanol. LC-MS (QC): $t_R$ = 0.717; $[M+H]^+$ = 410.2.

**Example 281: *rac*-(2-Cyclopropyl-imidazo[5,1-*b*]thiazol-3-yl)-[5-cyclopropyl-1-(4-methoxy-phenyl)-1*H*-[1,2,3]triazol-4-yl]-methanol**

Step 1: Preparation of methyl 5-cyclopropyl-1-(4-methoxyphenyl)-1H-1,2,3-triazole-4-carboxylate According to the procedure described for the preparation of example 279, step 1 but using methyl 3-cyclopropyl-3-oxopropanoate as starting material. LC-MS (acidic): $t_R$ = 0.86; $[M+H]^+$ = 273.85.

Step 2: Preparation of (5-cyclopropyl-1-(4-methoxyphenyl)-1H-1,2,3-triazol-4-yl)methanol According to the procedure described for the preparation of example 279, step 2 but using methyl 5-cyclopropyl-1-(4-methoxyphenyl)-1*H*-1,2,3-triazole-4-carboxylate as starting material. (5-Cyclopropyl-1-(4-methoxyphenyl)-1*H*-1,2,3-triazol-4-yl)methanol is obtained as a beige solid. LC-MS (acidic): $t_R$ = 0.69, $[M+H]^+$ = 246.03.

Step 3: Preparation of 5-cyclopropyl-1-(4-methoxyphenyl)-1H-1,2,3-triazole-4-carbaldehyde

Prepared following the procedure described for Example 222, step 3 but using (5-cyclopropyl-1-(4-methoxyphenyl)-1*H*-1,2,3-triazol-4-yl)methanol. 5-Cyclopropyl-1-(4-methoxyphenyl)-1*H*-1,2,3-triazole-4-carbaldehyde is obtained as a red oil. LC-MS (acidic): $t_R$ = 0.85, [M+H]+ = 244.03.

Step 4: Preparation of rac-(2-cyclopropyl-imidazo[5,1-b]thiazol-3-yl)-[5-cyclopropyl-1-(4-methoxy-phenyl)-1H-[1,2,3]triazol-4-yl]-methanol (Example 281)

Prepared following the procedure described for Example 96 using intermediate 10, 3-bromo-2-cyclopropylimidazo[5,1-*b*]thiazole and 5-cyclopropyl-1-(4-methoxyphenyl)-1H-1,2,3-triazole-4-carbaldehyde. Purification by preparative HPLC (acidic conditions) to give rac-(2-cyclopropyl-imidazo[5,1-b]thiazol-3-yl)-[5-cyclopropyl-1-(4-methoxy-phenyl)-1*H*-[1,2,3]triazol-4-yl]-methanol. LC-MS (QC): $t_R$ = 0.685; $[M+H]^+$ = 408.2.

**Example 282: (*R*)-{1-[3-Chloro-4-(3-fluoro-oxetan-3-ylmethoxy)-phenyl]-1*H*-[1,2,3]triazol-4-yl}-(2-cyclopropyl-imidazo[5,1-b]thiazol-3-yl)-methanol**

Prepared following the procedure described for Example 87, using 2-chloro-4-{4-[(*R*)-(2-cyclopropyl-imidazo[5,1-b]thiazol-3-yl)-hydroxy-methyl]-[1,2,3]triazol-1-yl}-phenol and 3-(bromomethyl)-3-fluorooxetane. Purification by preparative HPLC (basic conditions) gives (R)-{1-[3-chloro-4-(3-fluoro-oxetan-3-ylmethoxy)-phenyl]-1*H*-[1,2,3]tniazol-4-yl}-(2-cyclopropyl-imidazo[5,1-*b*]thiazol-3-yl)-methanol. LC-MS (QC): $t_R$ = 0.704; $[M+H]^+$ = 476.1.

**Example 283: 1-(2-Chloro-4-{4-[(*R*)-(2-cyclopropyl-imidazo[5,1-*b*]thiazol-3-yl)-hydroxy-methyl]-[1,2,3]triazol-1-yl}-phenoxy)-2-methyl-propan-2-ol**

Prepared following the procedure described for Example 87, using 2-chloro-4-{4-[(*R*)-(2-cyclopropyl-imidazo[5,1-*b*]thiazol-3-yl)-hydroxy-methyl]-[1,2,3]triazol-1-yl}-phenol and 1-bromo-2-methylpropan-2-ol. Purification by preparative HPLC (basic conditions) gives 1-(2-chloro-4-{4-[(R)-(2-cyclopropyl-imidazo[5,1-*b*]thiazol-3-yl)-hydroxy-methyl]-[1,2,3]triazol-1-yl}-phenoxy)-2-methyl-propan-2-ol. LC-MS (QC): $t_R$ = 0.691; $[M+H]^+$ = 460.3.

**Example 284: *rac-N*-(2-Chloro-4-{4-[(2-cyclopropyl-imidazo[5,1-*b*]thiazol-3-yl)-hydroxy-methyl]-[1,2,3]triazol-1-yl}-phenyl)-isobutyramide**

A solution of *rac*-[1-(4-amino-3-chloro-phenyl)-1*H*-[1,2,3]triazol-4-yl]-(2-cyclopropyl-imidazo[5,1-*b*]thiazol-3-yl)-methanol (30 mg; 0.078 mmol), isobutyric acid (8.2 mg; 0.093 mmol), triethylamine (0.014 ml; 0.101 mmol) and HATU (41 mg; 0.109 mmol) in DMF (1 ml) is stirred at RT for 16 h. Purification by preparative HPLC (basic conditions) to give *rac-N*-(2-chloro-4-{4-[(2-cyclopropyl-imidazo[5,1-*b*]thiazol-3-yl)-hydroxy-methyl]-[1,2,3]triazol-1-yl}-phenyl)-isobutyramide. LC-MS (QC): $t_R$ = 0.858; $[M+H]^+$ = 457.3.

**Example 285: rac-(2-Ethyl-imidazo[5,1-b]thiazol-3-yl)-[1-(4-methoxy-phenyl)-1H-[1,2,3]triazol-4-yl]-methanol**

Prepared according to the procedure described for Example 45, step 2 and using intermediate 3, rac-1-(2-ethylimidazo[5,1-*b*]thiazol-3-yl)prop-2-yn-1-ol and 1-azido-4-methoxybenzene. Purification by preparative HPLC (basic conditions) to give *rac*-(2-ethy-imidazo[5,1-*b*]thiazol-3-yl)-[1-(4-methoxy-phenyl)-1*H*-[1,2,3]triazol-4-yl]-methanol. LC-MS (QC): $t_R$ = 0.599; $[M+H]^+$ = 356.2.

**Example 286: rac-[2-(4-Fluoro-phenyl)-imidazo[5,1-b]thiazol-3-yl]-[1-(4-methoxy-phenyl)-1H-[1,2,3]triazol-4-yl]-methanol**

Prepared according to the procedure described for Example 45, step 2 and using intermediate 22, rac-1-(2-(4-fluorophenyl)imidazo[5,1-b]thiazol-3-yl)prop-2-yn-1-ol and 1-azido-4-methoxybenzene. Purification by preparative HPLC (basic conditions) to give rac-[2-(4-fluoro-phenyl)-imidazo[5,1-b]thiazol-3-yl]-[1-(4-methoxy-phenyl)-1H-[1,2,3]triazol-4-yl]-methanol. LC-MS (QC): $t_R$ = 0.785; $[M+H]^+$ = 422.3.

**Intermediate 22: *rac*-1-(2-(4-Fluorophenyl)imidazo[5,1-b]thiazol-3-yl)prop-2-yn-1-ol**

Step 1: Preparation of 2-(4-fluorophenyl)-*N*-methoxy-*N*-methylimidazo[5,1-*b*]thiazole-3-carboxamide According to the procedure described for the preparation of Intermediate 13, step 3 but using 4-fluorophenylboronic acid as starting material. LC-MS (acidic): $t_R$ = 0.61; $[M+H]^+$ = 305.79. $^1H$ NMR (400 MHz, DMSO) $\delta$: 8.19 (s, 1 H), 7.54-7.65 (m, 2 H), 7.35-7.43 (m, 2 H), 7.17 (s, 1 H), 3.49 (s, 3 H), 3.17 (s, 3 H).

Step 2: Preparation of rac-2-(4-fluorophenyl)imidazo[5,1-b]thiazole-3-carbaldehyde According to the procedure described for the preparation of Intermediate 13, step 4 but using 2-(4-fluorophenyl)-*N*-methoxy-*N*-methylimidazo[5,1-*b*]thiazole-3-carboxamide as starting material. LC-MS (acidic): $t_R$ = 0.59; $[M+H]^+$ = 246.94.

Step 3: Preparation of *rac*-1-(2-(4-fluorophenyl)imidazo[5,1-b]thiazol-3-yl)prop-2-yn-1-ol (intermediate 22) According to the procedure described for the preparation of Intermediate 13, step 5 but using rac-2-(4-fluorophenyl)imidazo[5,1-*b*]thiazole-3-carbaldehyde as starting material. LC-MS (acidic): $t_R$ = 0.60; $[M+H]^+$ = 272.95.

**Example 287: rac-[2-(2-Fluoro-phenyl)-imidazo[5,1-b]thiazol-3-yl]-[1-(4-methoxy-phenyl)-1H-[1,2,3]triazol-4-yl]-methanol**

Prepared according to the procedure described for Example 45, step 2 and using intermediate 23, rac-1-(2-(2-fluorophenyl)imidazo[5,1-b]thiazol-3-yl)prop-2-yn-1-ol and 1-azido-4-methoxybenzene. Purification by preparative HPLC (basic conditions) to give *rac*-[2-(2-fluoro-phenyl)-imidazo[5,1-*b*]thiazol-3-yl]-[1-(4-methoxy-phenyl)-1H-[1,2,3]triazol-4-yl]-methanol. LC-MS (QC): $t_R$ = 0.752; $[M+H]^+$ = 422.3.

**Intermediate 23: *rac*-1-(2-(2-fluorophenyl)imidazo[5,1-*b*]thiazol-3-yl)prop-2-yn-1-ol**

Step 1: Preparation of 2-(2-fluorophenyl)-*N*-methoxy-*N*-methylimidazo[5,1-*b*]thiazole-3-carboxamide According to the procedure described for the preparation of Intermediate 13, step 3 but using 2-fluorophenylboronic acid as starting material. LC-MS (acidic): $t_R$ = 0.62; $[M+H]^+$ = 305.76. $^1H$ NMR (400 MHz, DMSO) $\delta$: 8.19 (s, 1 H), 7.55-7.58 (m, 2 H), 7.36-7.40 (m, 2 H), 7.16 (s, 1 H), 3.49 (s, 3 H), 3.21 (m, 3 H).

Step 2: Preparation of rac-2-(2-fluorophenyl)imidazo[5,1-b]thiazole-3-carbaldehyde According to the procedure described for the preparation of Intermediate 13, step 4 but using 2-(2-fluorophenyl)-*N*-methoxy-*N*-methylimidazo[5,1-*b*]thiazole-3-carboxamide as starting material. LC-MS (acidic): $t_R$ = 0.59; $[M+H]^+$ = 246.95.

Step 3: Preparation of *rac*-1-(2-(2-fluorophenyl)imidazo[5,1-b]thiazol-3-yl)prop-2-yn-1-ol (intermediate 23) According to the procedure described for the preparation of Intermediate 13, step 5 but using *rac*-2-(2-fluorophenyl)imidazo[5,1-*b*]thiazole-3-carbaldehyde as starting material. LC-MS (acidic): $t_R$ = 0.62; $[M+H]^+$ = 272.94.

**Example 288: rac-[1-(3-Chloro-5-fluoro-4-methoxy-phenyl)-1H-[1,2,3]triazol-4-yl]-(2-cyclopropyl-imidazo[5,1-*b*]thiazol-3-yl)-methanol**

Step 1: Preparation of 5-azido-1-chloro-3-fluoro-2-methoxybenzene

To a solution of 3-chloro-5-fluoro-4-methoxyaniline (100 mg; 0.57 mmol) in 1M aq. HCl (8 ml) is added at 0°C a solution of sodium nitrite (40 mg; 0.57 mmol) in water (2 ml). The reaction mixture is stirred for 20 minutes, and sodium azide (45 mg; 0.68 mmol) is added. The reaction mixture is stirred at RT for 3 h. The mixture is diluted with EtOAc, the layers separated and the org. layer washed with brine, dried (MgSO4), filtered and concentrated under reduced pressure

to give 5-azido-1-chloro-3-fluoro-2-methoxybenzene as a yellow oil. $^{1}$H NMR (400 MHz, DMSO) $\delta$: 7.19 (dd, $J_1$ = 2.75 Hz, $J_2$ = 22.0 Hz, 1 H), 7.18 (dd, $J_1$ = 2.75 Hz, $J_2$ = 12.3 Hz, 1 H), 3.86 (d, $J$ = 1.0 Hz, 3 H).

Step 2: Prepared according to the procedure described for Example 45, step 2 and using intermediate 1, rac-1-(2-cyclopropylimidazo[5,1-*b*]thiazol-3-yl)prop-2-yn-1-ol and 5-azido-1-chloro-3-fluoro-2-methoxybenzene. Purification by preparative HPLC (basic conditions) to give *rac*-[1-(3-chloro-5-fluoro-4-methoxy-phenyl)-1*H*-[1,2,3]triazol-4-yl]-(2-cyclopropyl-imidazo[5,1-*b*]thiazol-3-yl)-methanol. LC-MS (QC): $t_R$ = 0.741; $[M+H]^+$ = 420.3.

**Example 289: rac-[1-(3-Chloro-4-methoxy-phenyl)-1H-[1,2,3]triazol-4-yl]-(2-cyclopropyl-imidazo[5,1-*b*]thiazol-3-yl)-methanol**

Step 1: Preparation of 4-azido-2-chloro-1-methoxybenzene

Prepared according to the procedure described for Example 288, step 1 using 3-chloro-4-methoxyaniline. Step 2: Prepared according to the procedure described for Example 45, step 2 and using intermediate 1, rac-1-(2-cyclopropylimidazo[5,1-*b*]thiazol-3-yl)prop-2-yn-1-ol and 4-azido-2-chloro-1-methoxybenzene. Purification by preparative HPLC (basic conditions) to give *rac*-[1-(3-chloro-4-methoxy-phenyl)-1*H*-[1,2,3]triazol-4-yl]-(2-cyclopropyl-imidazo[5,1-*b*]thiazol-3-yl)-methanol. LC-MS (QC): $t_R$ = 0.687; $[M+H]^+$ = 402.2.

**Example 290: rac-[1-(3-Bromo-4-methoxy-phenyl)-1H-[1,2,3]triazol-4-yl]-(2-cyclopropyl-imidazo[5,1-*b*]thiazol-3-yl)-methanol**

Step 1: Preparation of 4-azido-2-bromo-1-methoxybenzene

Prepared according to the procedure described for Example 288, step 1 using 3-bromo-4-methoxyaniline.

Step 2: Prepared according to the procedure described for Example 45, step 2 and using intermediate 1, *rac*-1-(2-cyclopropylimidazo[5,1-*b*]thiazol-3-yl)prop-2-yn-1-ol and 4-azido-2-bromo-1-methoxybenzene. Purification by preparative HPLC (basic conditions) to give *rac*-[1-(3-bromo4-methoxy-phenyl)-1*H*-[1,2,3]triazol-4-yl]-(2-cyclopropyl-imidazo[5,1-*b*]thiazol-3-yl)-methanol. LC-MS (QC): $t_R$ = 0.703; $[M+H]^+$ = 446.2.

**Example 291: *rac*-(2-Cyclopropyl-imidazo[5,1-*b*]thiazol-3-yl)-[1-(3-iodo-4-methoxy-phenyl)-1*H*-[1,2,3]triazol-4-yl]-methanol**

Step 1: Preparation of 4-azido-2-iodo-1-methoxybenzene

Prepared according to the procedure described for Example 288, step 1 using 3-iodo-4-methoxyaniline.

Step 2: Prepared according to the procedure described for Example 45, step 2 and using intermediate 1, *rac*-1-(2-cyclopropylimidazo[5,1-*b*]thiazol-3-yl)prop-2-yn-1-ol and 4-azido-2-iodo-1-methoxybenzene. Purification by preparative HPLC (basic conditions) to give rac-(2-cyclopropyl-imidazo[5,1-b]thiazol-3-yl)-[1-(3-iodo-4-methoxy-phenyl)-1H-[1,2,3]triazol-4-yl]-methanol. LC-MS (QC): $t_R$ = 0.740; $[M+H]^+$ = 494.2.

**Example 292: rac-[1-(3-Chloro-2-fluoro-4-methoxy-phenyl)-1H-[1,2,3]triazol-4-yl]-(2-cyclopropyl-imidazo[5,1-*b*]thiazol-3-yl)-methanol**

Step 1: Preparation of 1-azido-3-chloro-2-fluoro-4-methoxybenzene

Prepared according to the procedure described for Example 45, step 1 using (3-chloro-2-fluoro-4-methoxyphenyl)boronic acid.

Step 2: Prepared according to the procedure described for Example 45, step 2 and using intermediate 1, *rac*-1-(2-cyclopropylimidazo[5,1-*b*]thiazol-3-yl)prop-2-yn-1-ol and 1-azido-3-chloro-2-fluoro-4-methoxybenzene. Purification by preparative HPLC (basic conditions) to give *rac*-[1-(3-chloro-2-fluoro-4-methoxy-phenyl)-1*H*-[1,2,3]triazol-4-yl]-(2-cyclopropyl-imidazo[5,1-*b*]thiazol-3-yl)-methanol. LC-MS (QC): $t_R$ = 0.703; $[M+H]^+$ = 420.2.

**Example 293: rac-4-(2-Chloro-4-{4-[hydroxy-(2-isopropenyl-imidazo[5,1-b]thiazol-3-yl)-methyl]-[1,2,3]triazol-1-yl}-phenoxy)-2-methyl-butan-2-ol**

Step 1: preparation of *rac*-2-chloro-4-{4-[hydroxy-(2-isopropenyl-imidazo[5,1-*b*]thiazol-3-yl)-methyl]-[1,2,3]triazol-1-yl}-phenol

According to the procedure described for Example 45, step 2 and using intermediate 18, *rac*-1-(2-(prop-1-en-2-yl)imidazo[5,1-*b*]thiazol-3-yl)prop-2-yn-1-ol and 4-azido-2-chlorophenol. Purification by preparative HPLC (acidic conditions) to give rac-2-chloro-4-{4-[hydroxy-(2-isopropenyl-imidazo[5,1-b]thiazol-3-yl)-methyl]-[1,2,3]triazol-1-yl}-phenol. LC-MS (acidic): $t_R$ = 0.66; $[M+H]^+$ = 387.90.

Step 2: preparation of *rac*-4-(2-chloro-4-{4-[hydroxy-(2-isopropenyl-imidazo[5,1-*b*]thiazol-3-yl)-methyl]-[1,2,3]triazol-1-yl}-phenoxy)-2-methyl-butan-2-ol (example 293)

Prepared following the procedure described for Example 87, using rac-2-chloro-4-{4-[hydroxy-(2-isopropenyl-imidazo[5,1-b]thiazol-3-yl)-methyl]-[1,2,3]triazol-1-yl}-phenol and 4-bromo-2-methylbutan-2-ol. Purification by preparative HPLC (acidic conditions) gives *rac*-4-(2-chloro-4-{4-[hydroxy-(2-isopropenyl-imidazo[5,1-*b*]thiazol-3-yl)-methyl]-[1,2,3]triazol-1-yl}-phenoxy)-2-methyl-butan-2-ol. LC-MS (QC): $t_R$ = 0.778; $[M+H]^+$ = 474.3.

**Example 294: *rac*-3-{4-[(2-Cyclopropyl-imidazo[5,1-*b*]thiazol-3-yl)-hydroxy-methyl]-[1,2,3]triazol-1-ylmethyl}-benzoic acid methyl ester**

Step 1: Preparation of methyl 3-(azidomethyl)benzoate

To a solution of methyl 3-(hydroxymethyl)benzoate (514 mg; 2.94 mmol) and diphenyl phosphoryl azide (0.78 ml; 3.53 mmol) in toluene (5 ml) is added DBU (0.49 ml; 3.23 mmol) at 0°C. The reaction mixture is stirred at 0°C for 1 h and at RT for 18 h. The solution is diluted with water and DCM, and the aq layer made acidic with 1N aq. HCl. The layers are separated, and the aq. layer extracted with EtOAc (2x). The combined org. extracts are successively washed with water and brine, dried (MgSO4), filtered and concentrated under reduced pressure. Purification by FC (Silica gel; Hept / EtOAc) gives 478 mg of as a colorless oil. LC-MS (acidic): $t_R$ = 0.88; [M+H]+ = 192.07.

Step 2: Prepared according to the procedure described for Example 45 and using intermediate 1, rac-1-(2-cyclopropylimidazo[5,1-*b*]thiazol-3-yl)prop-2-yn-1-ol and methyl 3-(azidomethyl)benzoate. Purification by preparative HPLC (basic conditions) to give *rac*-3-{4-[(2-cyclopropyl-imidazo[5,1-*b*]thiazol-3-yl)-hydroxy-methyl]-[1,2,3]triazol-1-ylmethyl}-benzoic acid methyl ester. LC-MS (QC): $t_R$ = 0.600; $[M+H]^+$ = 410.2.

**Example 295: *rac*-[1-(3-Chloro-4-methoxy-phenyl)-5-methyl-1*H*-[1,2,3]triazol-4-yl]-(2-cyclopropyl-imidazo[5,1-*b*]thiazol-3-yl)-methanol**

Step 1: Preparation of ethyl 1-(3-chloro-4-methoxyphenyl)-5-methyl-1*H*-1,2,3-triazole-4-carboxylate According to the procedure described for the preparation of example 279, step 1 but using 4-azido-2-chloro-1-methoxybenzene and ethyl 3-oxobutanoate as starting materials. LC-MS (acidic): $t_R$ = 0.91; $[M+H]^+$ = 295.99.

Step 2: Preparation of (1-(3-chloro-4-methoxyphenyl)-5-methyl-1H-1,2,3-triazol-4-yl)methanol

According to the procedure described for the preparation of example 279, step 2 but using ethyl 1-(3-chloro-4-methoxyphenyl)-5-methyl-1*H*-1,2,3-triazole-4-carboxylate as starting material. (1-(3-Chloro-4-methoxyphenyl)-5-methyl-1H-1,2,3-triazol-4-yl)methanol is obtained as a white solid. LC-MS (acidic): $t_R$ = 0.68, $[M+H]^+$ = 253.96. Step 3: Preparation of 1-(3-chloro-4-methoxyphenyl)-5-methyl-1*H*-1,2,3-triazole-4-carbaldehyde

Prepared following the procedure described for Example 222, step 3 but using (1-(3-chloro-4-methoxyphenyl)-5-methyl-1*H*-1,2,3-triazol-4-yl)methanol. 1-(3-Chloro-4-methoxyphenyl)-5-methyl-1*H*-1,2,3-triazole-4-carbaldehyde is obtained as a white solid. LC-MS (acidic): $t_R$ = 0.84, $[M+H]^+$ = 251.95.

Step 4: Preparation of *rac*-[1-(3-chloro4-methoxy-phenyl)-5-methyl-1*H*-[1,2,3]triazol-4-yl]-(2-cyclopropyl-**imidazo[5,1-*b*]thiazol-3-yl)-methanol** (Example 295)

Prepared following the procedure described for Example 96 using intermediate 10, 3-bromo-2-cyclopropylimidazo[5,1-*b*]thiazole and 1-(3-chloro-4-methoxyphenyl)-5-methyl-1*H*-1,2,3-triazole-4-carbaldehyde. Purification by preparative HPLC (basic conditions) to give rac-[1-(3-chloro-4-methoxy-phenyl)-5-methyl-1*H*-[1,2,3]triazol-4-yl]-(2-cyclopropyl-imidazo[5,1-*b*]thiazol-3-yl)-methanol. LC-MS (QC): $t_R$ = 0.686; $[M+H]^+$ = 416.3.

**Example 296: *rac*-[1-(3-Chbro-4-ethoxy-pheny))-5-methy)-1*H*-[1,2,3]triazol-4-yl]-(2-cyclopropyl-imidazo[5,1-*b*]thiazol-3-yl)-methanol**

Step 1: Preparation of ethyl 1-(3-chloro-4-ethoxyphenyl)-5-methyl-1*H*-1,2,3-triazole-4-carboxylate

According to the procedure described for the preparation of example 279, step 1 but using 4-azido-2-chloro-1-ethoxybenzene (prepared as described for example 45, step 1 using (3-chloro-4-ethoxyphenyl)boronic acid) and ethyl 3-oxobutanoate as starting materials. LC-MS (acidic): $t_R$ = 0.97; $[M+H]^+$ = 309.93.

Step 2: Preparation of (1-(3-chloro-4-ethoxyphenyl)-5-methyl-1*H*-1,2,3-triazol-4-yl)methanol

According to the procedure described for the preparation of example 279, step 2 but using ethyl 1-(3-chloro-4-ethoxyphenyl)-5-methyl-1*H*-1,2,3-triazole-4-carboxylate as starting material. (1-(3-Chloro-4-ethoxyphenyl)-5-methyl-1*H*-1,2,3-triazol-4-yl)methanol is obtained as a white solid. LC-MS (acidic): $t_R$ = 0.75, $[M+H]^+$ = 268.00. Step 3: Preparation of 1-(3-chloro-4-ethoxyphenyl)-5-methyl-1*H*-1,2,3-triazole-4-carbaldehyde

Prepared following the procedure described for Example 222, step 3 but using (1-(3-chloro-4-ethoxyphenyl)-5-methyl-1*H*-1,2,3-triazol-4-yl)methanol. 1-(3-Chloro-4-ethoxyphenyl)-5-methyl-1*H*-1,2,3-triazole-4-carbaldehyde is obtained as a white solid. LC-MS (acidic): $t_R$ = 0.91, $[M+H]^+$ = 265.98.

Step 4: Preparation of *rac*-[1-(3-chloro-4-ethoxy-phenyl)-5-methyl-1*H*-[1,2,3]triazol-4-yl]-(2-cyclopropyl-**imidazo[5,1-*b*]thiazol-3-yl)-methanol** (Example 296)

Prepared following the procedure described for Example 96 using intermediate 10, 3-bromo-2-cyclopropylimidazo[5,1-*b*]thiazole and 1-(3-chloro-4-ethoxyphenyl)-5-methyl-1*H*-1,2,3-triazole-4-carbaldehyde. Purification by preparative HPLC (acidic conditions) to give *rac*-[1-(3-chloro-4-ethoxy-phenyl)-5-methyl-1*H*-[1,2,3]triazol-4-yl]-(2-cyclopropyl-imidazo[5,1-*b*]thiazol-3-yl)-methanol. LC-MS (QC): $t_R$ = 0.760; $[M+H]^+$ = 430.3.

**Example 297: *rac*-4-{4-[(2-Cyclopropyl-imidazo[5,1-*b*]thiazol-3-yl)-hydroxy-methyl]-[1,2,3]triazol-1-ylmethyl}-benzoic acid methyl ester**

Step 1: Preparation of methyl 4-(azidomethyl)benzoate

According to the procedure described for the preparation of example 294, step 1 and using methyl 4-(hydroxymethyl)benzoate as starting material.

Step 2: Prepared according to the procedure described for Example 45, step 2 and using intermediate 1, rac-1-(2-cyclopropylimidazo[5,1-*b*]thiazol-3-yl)prop-2-yn-1-ol and methyl 4-(azidomethyl)benzoate. Purification by preparative HPLC (acidic conditions) to give *rac*-4-{4-[(2-cyclopropyl-imidazo[5,1-*b*]thiazol-3-yl)-hydroxy-methyl]-[1,2,3]triazol-1-ylmethyl}-benzoic acid methyl ester. LC-MS (QC): $t_R$ = 0.596; $[M+H]^+$ = 410.2.

**Example 298: *rac*-3-{4-[(2-Cyclopropyl-imidazo[5,1-*b*]thiazol-3-yl)-hydroxy-methyl]-[1,2,3]triazol-1-ylmethyl}-phenol**

Step 1: Preparation of 3-(azidomethyl)phenol

According to the procedure described for the preparation of example 294, step 1 and using 3-(hydroxymethyl)phenol as starting material.

Step 2: Prepared according to the procedure described for Example 45, step 2 and using intermediate 1, *rac*-1-(2-cyclopropylimidazo[5,1-*b*]thiazol-3-yl)prop-2-yn-1-ol and 3-(azidomethyl)phenol. Purification by preparative HPLC (acidic conditions) to give rac-3-{4-[(2-cyclopropyl-imidazo[5,1-b]thiazol-3-yl)-hydroxy-methyl]-[1,2,3]triazol-1-ylmethyl}-phenol. LC-MS (QC): $t_R$ = 0.495; $[M+H]^+$ = 368.3.

**Example 299: *rac*-{1-[3-Chloro-4-(3-fluoro-oxetan-3-ylmethoxy)-phenyl]-1*H*-[1,2,3]triazol-4-yl}-(2-isopropenyl-imidazo[5,1-*b*]thiazol-3-yl)-methanol**

Prepared following the procedure described for Example 293, using rac-2-chloro-4-{4-[hydroxy-(2-isopropenyl-imidazo[5,1-*b*]thiazol-3-yl)-methyl]-[1,2,3]triazol-1-yl}-phenol and 3-(bromomethyl)-3-fluorooxetane. Purification by preparative HPLC (acidic conditions) gives *rac*-{1-[3-chloro-4-(3-fluoro-oxetan-3-ylmethoxy)-phenyl]-1*H*-[1,2,3]triazol-4-yl}-(2-isopropenyl-imidazo[5,1-*b*]thiazol-3-yl)-methanol. LC-MS (acidic): $t_R$ = 0.76; $[M+H]^+$ = 476.06.

**Example 300: rac-[1-(5-Chloro-2-fluoro-4-methoxy-phenyl)-1H-[1,2,3]triazol-4-yl]-(2-cyclopropyl-imidazo[5,1-*b*]thiazol-3-yl)-methanol**

Step 1: Preparation of 1-azido-5-chloro-2-fluoro-4-methoxybenzene

Prepared according to the procedure described for Example 288, step 1 using 5-chloro-2-fluoro-4-methoxyaniline.

Step 2: Prepared according to the procedure described for Example 45, step 2 and using intermediate 1, *rac*-1-(2-cyclopropylimidazo[5,1-*b*]thiazol-3-yl)prop-2-yn-1-ol and 1-azido-5-chloro-2-fluoro-4-methoxybenzene. Purification by preparative HPLC (acidic conditions) to give *rac*-[1-(5-chloro-2-fluoro-4-methoxy-phenyl)-1*H*-[1,2,3]triazol-4-yl]-(2-cyclopropyl-imidazo[5,1-*b*]thiazol-3-yl)-methanol. LC-MS (QC): $t_R$ = 0.698; $[M+H]^+$ = 420.3.

**Example 301: *rac*-3-(2-Chloro-4-{4-[hydroxy-(2-isopropenyl-imidazo[5,1-*b*]thiazol-3-yl)-methyl]-[1,2,3]triazol-1-yl}-phenoxy)-propan-1-ol**

Prepared following the procedure described for Example 293, using rac-2-chloro-4-{4-[hydroxy-(2-isopropenyl-imidazo[5,1-*b*]thiazol-3-yl)-methyl]-[1,2,3]triazol-1-yl}-phenol and 3-bromopropan-1-ol. Purification by preparative HPLC (acidic conditions) gives *rac*-3-(2-chloro-4-{4-[hydroxy-(2-isopropenyl-imidazo[5,1-*b*]thiazol-3-yl)-methyl]-[1,2,3]triazol-1-yl}-phenoxy)-propan-1-ol. LC-MS (QC): $t_R$ = 0.685; $[M+H]^+$ = 446.3.

**Example 302: *rac*-4-(2-Chloro-4-{4-[(2-ethyl-imidazo[5,1-*b*]thiazol-3-yl)-hydroxy-methyl]-[1,2,3]triazol-1-yl}-phenoxy)-2-methyl-butan-2-ol**

Step 1: Preparation of 4-(4-azido-2-chlorophenoxy)-2-methylbutan-2-ol

Prepared according to the procedure described for Example 288, step 1 using 4-(4-amino-2-chlorophenoxy)-2-methylbutan-2-ol (prepared by alkylation according to the procedure described for example 87, and using 4-amino-2-chlorophenol and 4-bromo-2-methylbutan-2-ol).

Step 2: Prepared according to the procedure described for Example 45, step 2 and using intermediate 3, *rac-1*-(2-ethylimidazo[5,1-b]thiazol-3-yl)prop-2-yn-1-ol and 4-(4-azido-2-chlorophenoxy)-2-methylbutan-2-ol. Purification by preparative HPLC (basic conditions) to give *rac*-4-(2-chloro-4-{4-[(2-ethyl-imidazo[5,1-*b*]thiazol-3-yl)-hydroxy-methyl]-[1,2,3]triazol-1-yl}-phenoxy)-2-methyl-butan-2-ol. LC-MS (QC): $t_R$ = 0.683; $[M+H]^+$ = 462.2.

**Example 303: *rac*-4-[2-Chloro-4-(4-{hydroxy-[2-(1-methyl-cyclopropyl)-imidazo[5,1-*b*]thiazol-3-yl]-methyl}-[1,2,3]triazol-1-yl)-phenoxy]-2-methyl-butan-2-ol**

Step 1: Preparation of 4-(4-azido-2-chlorophenoxy)-2-methylbutan-2-ol

Prepared according to the procedure described for Example 288, step 1 using 4-(4-amino-2-chlorophenoxy)-2-methylbutan-2-ol (prepared by alkylation according to the procedure described for example 87, and using 4-amino-2-chlorophenol and 4-bromo-2-methylbutan-2-ol).

Step 2: Prepared according to the procedure described for Example 45, step 2 and using intermediate 5, *rac*-1-(2-(1-methylcyclopropyl)imidazo[5,1-*b*]thiazol-3-yl)prop-2-yn-1-ol and 4-(4-azido-2-chlorophenoxy)-2-methylbutan-2-ol. Purification by preparative HPLC (basic conditions) to give rac-4-[2-chloro-4-(4-{hydroxy-[2-(1-methyl-cyclopropyl)-imidazo[5,1-*b*]thiazol-3-yl]-methyl}-[1,2,3]triazol-1-yl)-phenoxy]-2-methyl-butan-2-ol. LC-MS (QC): $t_R$ = 0.768; $[M+H]^+$ = 488.2.

**Example 304: *rac*-4-{4-[(2-Cyclopropyl-imidazo[5,1-*b*]thiazol-3-yl)-hydroxy-methyl]-[1,2,3]triazol-1-ylmethyl}-phenol**

Step 1: Preparation of 4-(azidomethyl)phenol

According to the procedure described for the preparation of example 294, step 1 and using 4-(hydroxymethyl)phenol as starting material.

Step 2: Prepared according to the procedure described for Example 45, step 2 and using intermediate 1, *rac*-

1-(2-cyclopropylimidazo[5,1-*b*]thiazol-3-yl)prop-2-yn-1-ol and 4-(azidomethyl)phenol. Purification by preparative HPLC (acidic conditions) to give rac-4-{4-[(2-cyclopropyl-imidazo[5,1-b]thiazol-3-yl)-hydroxy-methyl]-[1,2,3]triazol-1-ylmethyl}-phenol. LC-MS (QC): $t_R$ = 0.458; $[M+H]^+$ = 368.1.

**Example 305: *rac*-2-{4-[(2-Cyclopropyl-imidazo[5,1-*b*]thiazol-3-yl)-hydroxy-methyl]-[1,2,3]triazol-1-ylmethyl}-phenol**

Step 1: Preparation of 2-(azidomethyl)phenol

According to the procedure described for the preparation of example 294, step 1 and using 2-(hydroxymethyl)phenol as starting material.

Step 2: Prepared according to the procedure described for Example 45, step 2 and using intermediate 1, *rac*-1-(2-cyclopropylimidazo[5,1-*b*]thiazol-3-yl)prop-2-yn-1-ol and 2-(azidomethyl)phenol. Purification by preparative HPLC (acidic conditions) to give *rac*-2-{4-[(2-cyclopropyl-imidazo[5,1-*b*]thiazol-3-yl)-hydroxy-methyl]-[1,2,3]triazol-1-ylmethyl}-phenol. LC-MS (QC): $t_R$ = 0.509; $[M+H]^+$ = 368.1.

**Example 306: *rac*-(2-Cyclopropyl-imidazo[5,1-*b*]thiazol-3-yl)-[1-(4-methoxy-2-methyl-phenyl)-1*H*-[1,2,3]triazol-4-yl]-methanol**

Step 1: Preparation of 1-azido-4-methoxy-2-methylbenzene

Prepared according to the procedure described for Example 45, step 1 using (4-methoxy-2-methylphenyl)boronic acid.

Step 2: Prepared according to the procedure described for Example 45, step 2 and using intermediate 1, *rac*-1-(2-cyclopropylimidazo[5,1-*b*]thiazol-3-yl)prop-2-yn-1-ol and 1-azido-4-methoxy-2-methylbenzene. Purification by preparative HPLC (acidic conditions) to give *rac*-(2-cyclopropyl-imidazo[5,1-*b*]thiazol-3-yl)-[1-(4-methoxy-2-methyl-phenyl)-1H-[1,2,3]triazol-4-yl]-methanol. LC-MS (QC): $t_R$ = 0.613; $[M+H]^+$ = 382.1.

**Example 307: *rac*-4-[2-Chloro-4-(4-{[2-(1,1-difluoro-ethyl)-imidazo[5,1-*b*]thiazol-3-yl]-hydroxy-methyl}-[1,2,3]triazol-1-yl)-phenoxy]-2-methyl-butan-2-ol**

Step 1: Preparation of 4-(4-azido-2-chlorophenoxy)-2-methylbutan-2-ol

Prepared according to the procedure described for Example 288, step 1 using 4-(4-amino-2-chlorophenoxy)-2-methylbutan-2-ol (prepared by alkylation according to the procedure described for example 87, and using 4-amino-2-chlorophenol and 4-bromo-2-methylbutan-2-ol).

Step 2: Prepared according to the procedure described for Example 45, step 2 and using intermediate 14, *rac*-1-(2-(1,1-difluoroethyl)imidazo[5,1-*b*]thiazol-3-yl)prop-2-yn-1-ol and 4-(4-azido-2-chlorophenoxy)-2-methylbutan-2-ol. Purification by preparative HPLC (acidic conditions) followed by neutralization and extraction with DCM to give rac-4-[2-chloro-4-(4-{[2-(1,1-difluoro-ethyl)-imidazo[5,1-b]thiazol-3-yl]-hydroxy-methyl}-[1,2,3]triazol-1-yl)-phenoxy]-2-methylbutan-2-ol. LC-MS (QC): $t_R$ = 0.850; $[M+H]^+$ = 498.1.

**Example 308: 4-[2-Chloro-4-(4-{[2-(*trans*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol**

Step 1: Preparation of 4-(4-azido-2-chlorophenoxy)-2-methylbutan-2-ol

Prepared according to the procedure described for Example 288, step 1 using 4-(4-amino-2-chlorophenoxy)-2-methylbutan-2-ol (prepared by alkylation according to the procedure described for example 87, and using 4-amino-2-chlorophenol and 4-bromo-2-methylbutan-2-ol).

Step 2: Prepared according to the procedure described for Example 45, step 2 and using intermediate 9, *trans*-1-[(2-fluoro-cyclopropyl)-imidazo[5,1-*b*]thiazol-3-yl]prop-2-yn-1-ol and 4-(4-azido-2-chlorophenoxy)-2-methylbutan-2-ol. Purification by preparative HPLC (acidic conditions) followed by neutralization and extraction with DCM to give 4-[2-chloro-4-(4-{[2-(*trans*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol. LC-MS (QC): $t_R$ = 0.718; $[M+H]^+$ = 492.1.

**Example 309: 4-[2-Chloro-4-(4-{[2-(*cis*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol**

Step 1: Preparation of 4-(4-azido-2-chlorophenoxy)-2-methylbutan-2-ol

Prepared according to the procedure described for Example 288, step 1 using 4-(4-amino-2-chlorophenoxy)-2-methylbutan-2-ol (prepared by alkylation according to the procedure described for example 87, and using 4-amino-2-chlorophenol and 4-bromo-2-methylbutan-2-ol).

Step 2: Prepared according to the procedure described for Example 45, step 2 and using intermediate 8, *cis*-1-[(2-fluoro-cyclopropyl)-imidazo[5,1-*b*]thiazol-3-yl]prop-2-yn-1-ol and 4-(4-azido-2-chlorophenoxy)-2-methylbutan-2-ol. Purification by preparative HPLC (acidic conditions) followed by neutralization and extraction with DCM to give 4-[2-chloro-4-(4-{[2-(*cis*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol. LC-MS (QC): $t_R$ = 0.685; $[M+H]^+$ = 492.1.

**Example 310: rac-(2-Cyclopropyl-imidazo[5,1-b]thiazol-3-yl)-[1-(4-hydroxymethyl-phenyl)-1H-[1,2,3]triazol-4-yl]-methanol**

Step 1: Preparation of (4-azidophenyl)methanol

Prepared according to the procedure described for Example 288, step 1 using (4-aminophenyl)methanol.

Step 2: Prepared according to the procedure described for Example 45, step 2 and using intermediate 1, *rac*-1-(2-cyclopropylimidazo[5,1-*b*]thiazol-3-yl)prop-2-yn-1-ol and (4-azidophenyl)methanol. Purification by FC (Silica gel; EtOAc / MeOH) to give *rac*-(2-cyclopropyl-imidazo[5,1-*b*]thiazol-3-yl)-[1-(4-hydroxymethyl-phenyl)-1*H*-[1,2,3]triazol-4-yl]-methanol. LC-MS (QC): $t_R$ = 0.474; $[M+H]^+$ = 368.1.

**Example 311: rac-(2-Cyclopropyl-imidazo[5,1-b]thiazol-3-yl)-[1-(4-methoxy-2,6-dimethyl-phenyl)-1*H*-[1,2,3]triazol-4-yl]-methanol**

Step 1: Preparation of 2-azido-5-methoxy-1,3-dimethylbenzene

Prepared according to the procedure described for Example 45, step 1 using (4-methoxy-2,6-dimethylphenyl)boronic acid.

Step 2: Prepared according to the procedure described for Example 45, step 2 and using intermediate 1, rac-1-(2-cyclopropylimidazo[5,1-*b*]thiazol-3-yl)prop-2-yn-1-ol and 2-azido-5-methoxy-1,3-dimethylbenzene. Purification by preparative HPLC (basic conditions) to give rac-(2-cyclopropyl-imidazo[5,1-b]thiazol-3-yl)-[1-(4-methoxy-2,6-dimethyl-phenyl)-1*H*-[1,2,3]triazol-4-yl]-methanol. LC-MS (QC): $t_R$ = 0.646; $[M+H]^+$ = 396.2.

**Example 312: *rac*-(2-Cyclopropyl-imidazo[5,1-*b*]thiazol-3-yl)-(1-{4-[2-(2,2,2-trifluoro-ethoxy)-ethoxy]-phenyl}-1*H*-[1,2,3]triazol-4-yl)-methanol**

Prepared following the procedure described for Example 87, using rac-4-{4-[(2-cyclopropyl-imidazo[5,1-b]thiazol-3-yl)-hydroxy-methyl]-[1,2,3]triazol-1-yl}-phenol and 2-(2-bromoethoxy)-1,1,1-trifluoroethane. Purification by preparative HPLC (basic conditions) gives rac-(2-cyclopropyl-imidazo[5,1-b]thiazol-3-yl)-(1-{4-[2-(2,2,2-trifluoro-ethoxy)-ethoxy]-phenyl}-1*H*-[1,2,3]triazol-4-yl)-methanol. LC-MS (QC): $t_R$ = 0.730; $[M+H]^+$ = 480.2.

**Example 313: *rac*-3-[2-Chloro-4-(4-{hydroxy-[2-(1-methyl-cyclopropyl)-imidazo[5,1-*b*]thiazol-3-yl]-methyl}-[1,2,3]triazol-1-yl)-phenoxy]-propan-1-ol**

Step 1: Preparation of 3-(4-azido-2-chlorophenoxy)propan-1-ol

Prepared according to the procedure described for Example 288, step 1 using 3-(4-amino-2-chlorophenoxy)propan-1-ol (prepared by alkylation according to the procedure described for example 87, and using 4-amino-2-chlorophenol and 3-bromopropan-1-ol).

Step 2: Prepared according to the procedure described for Example 45, step 2 and using intermediate 5, *rac-1*-(2-(1-methylcyclopropyl)imidazo[5,1-*b*]thiazol-3-yl)prop-2-yn-1-ol and 3-(4-azido-2-chlorophenoxy)propan-1-ol. Purification by preparative HPLC (basic conditions) to give *rac*-3-[2-chloro-4-(4-{hydroxy-[2-(1-methyl-cyclopropyl)-imidazo[5,1-*b*]thiazol-3-yl]-methyl}-[1,2,3]triazol-1-yl)-phenoxy]-propan-1-ol. LC-MS (QC): $t_R$ = 0.682; $[M+H]^+$ = 460.1.

**Example 314: *rac*-3-[2-Chloro-4-(4-{[2-(1,1-difluoro-ethyl)-imidazo[5,1-*b*]thiazol-3-yl]-hydroxy-methyl}-[1,2,3]triazol-1-yl)-phenoxy]-propan-1-ol**

Prepared according to the procedure described for Example 313, using intermediate 14, *rac*-1-(2-(1,1-difluoroethyl)imidazo[5,1-*b*]thiazol-3-yl)prop-2-yn-1-ol and 3-(4-azido-2-chlorophenoxy)propan-1-ol. Purification by preparative HPLC (basic conditions) to give *rac*-3-[2-chloro-4-(4-{[2-(1,1-difluoro-ethyl)-imidazo[5,1-*b*]thiazol-3-yl]-hydroxy-methyl}-[1,2,3]triazol-1-yl)-phenoxy]-propan-1-ol. LC-MS (QC): $t_R$ = 0.747; $[M+H]^+$ = 470.1.

**Example 315: *rac*-{1-[3-Chloro-4-(3-fluoro-oxetan-3-ylmethoxy)-phenyl]-1*H*-[1,2,3]triazol-4-yl}-[2-(1-methyl-cyclopropyl)-imidazo[5,1-b]thiazol-3-yl]-methanol**

Step 1: Preparation of 3-((4-azido-2-chlorophenoxy)methyl)-3-fluorooxetane

Prepared according to the procedure described for Example 288, step 1 using 3-chloro-4-((3-fluorooxetan-3-yl)methoxy)aniline (prepared by alkylation according to the procedure described for example 87, and using 4-amino-2-chlorophenol and 3-(bromomethyl)-3-fluorooxetane).

Step 2: Prepared according to the procedure described for Example 45, step 2 and using intermediate 5, *rac*-1-(2-(1-methylcyclopropyl)imidazo[5,1-*b*]thiazol-3-yl)prop-2-yn-1-ol and 3-((4-azido-2-chlorophenoxy)methyl)-3-fluorooxetane. Purification by preparative HPLC (basic conditions) to give rac-{1-[3-chloro-4-(3-fluoro-oxetan-3-ylmethoxy)-phenyl]-1*H*-[1,2,3]triazol-4-yl}-[2-(1-methyl-cyclopropyl)-imidazo[5,1-*b*]thiazol-3-y]-methanol. LC-MS (QC): $t_R$ = 0.753; $[M+H]^+$ = 490.2.

**Example 316: *rac*-{1-[3-Chloro-4-(3-fluoro-oxetan-3-ylmethoxy)-phenyl]-1*H*-[1,2,3]triazol-4-yl}-[2-(1,1-difluoro-ethyl)-imidazo[5,1-*b*]thiazol-3-yl]-methanol**

Prepared according to the procedure described for Example 315, using intermediate 14, *rac*-1-(2-(1,1-difluoroethyl)imidazo[5,1-*b*]thiazol-3-yl)prop-2-yn-1-ol and 3-((4-azido-2-chlorophenoxy)methyl)-3-fluorooxetane. Purification by preparative HPLC (basic conditions) to give rac-{1-[3-chloro-4-(3-fluoro-oxetan-3-ylmethoxy)-phenyl]-1*H*-[1,2,3]triazol-4-yl}-[2-(1,1-difluoro-ethyl)-imidazo[5,1-*b*]thiazol-3-yl]-methanol. LC-MS (QC): $t_R$ = 0.835; $[M+H]^+$ = 500.1.

**Example 317: *rac*-{1-[3-Chloro-4-(3-fluoro-oxetan-3-ylmethoxy)-phenyl]-1*H*-[1,2,3]triazol-4-yl}-(2-ethyl-imidazo[5,1-*b*]thiazol-3-yl)-methanol**

Prepared according to the procedure described for Example 315, using intermediate 3, rac-1-(2-ethylimidazo[5,1-*b*]thiazol-3-yl)prop-2-yn-1-ol and 3-((4-azido-2-chlorophenoxy)methyl)-3-fluorooxetane. Purification by preparative HPLC (basic conditions) to give rac-{1-[3-chloro-4-(3-fluoro-oxetan-3-ylmethoxy)-phenyl]-1*H*-[1,2,3]triazol-4-yl}-(2-ethyl-imidazo[5,1-*b*]thiazol-3-yl)-methanol. LC-MS (QC): $t_R$ = 0.663; $[M+H]^+$ = 464.1.

**Example 318: rac-(2-Cyclopropyl-imidazo[5,1-b]thiazol-3-yl)-[1-(2,5-difluoro-4-methoxy-phenyl)-1H-[1,2,3]triazol-4-yl]-methanol**

Step 1: Preparation of 1-azido-2,5-difluoro-4-methoxybenzene

Prepared according to the procedure described for Example 45, step 1 using (2,5-difluoro-4-methoxyphenyl)boronic acid.

Step 2: Prepared according to the procedure described for Example 45, step 2 and using intermediate 1, *rac-1*-(2-cyclopropylimidazo[5,1-*b*]thiazol-3-yl)prop-2-yn-1-ol and 1-azido-2,5-difluoro-4-methoxybenzene. Purification by preparative HPLC (basic conditions) to give *rac*-(2-cyclopropyl-imidazo[5,1-*b*]thiazol-3-yl)-[1-(2,5-difluoro-4-methoxyphenyl)-1*H*-[1,2,3]triazol-4-yl]-methanol. LC-MS (QC): $t_R$ = 0.627; $[M+H]^+$ = 404.1.

**Example 319: rac-[1-(5-Chloro-2-fluoro-4-methoxy-phenyl)-1H-[1,2,3]triazol-4-yl]-(2-ethyl-imidazo[5,1-*b*]thiazol-3-yl)-methanol**

Step 1: Preparation of 1-azido-5-chloro-2-fluoro-4-methoxybenzene

Prepared according to the procedure described for Example 288, step 1 using 5-chloro-2-fluoro-4-methoxyaniline.

Step 2: Prepared according to the procedure described for Example 45, step 2 and using intermediate 3, *rac*-

*1*-(2-ethylimidazo[5,1-*b*]thiazol-3-yl)prop-2-yn-1-ol and 1-azido-5-chloro-2-fluoro-4-methoxybenzene. Purification by preparative HPLC (basic conditions) to give *rac*-[1-(5-chloro-2-fluoro-4-methoxy-phenyl)-1*H*-[1,2,3]triazol-4-yl]-(2-ethyl-imidazo[5,1-b]thiazol-3-yl)-methanol. LC-MS (QC): $t_R$ = 0.654; $[M+H]^+$ = 408.0.

**Example 320: *rac*-3-(2-Chloro-4-{4-[(2-ethyl-imidazo[5,1-*b*]thiazol-3-yl)-hydroxy-methyl]-[1,2,3]triazol-1-yl}-phenoxy)-propan-1-ol**

Prepared according to the procedure described for Example 313, using intermediate 3, rac-1-(2-ethylimidazo[5,1-*b*]thiazol-3-yl)prop-2-yn-1-ol and 3-(4-azido-2-chlorophenoxy)propan-1-ol. Purification by preparative HPLC (acidic conditions) followed by preparative HPLC (basic conditions) to give rac-3-(2-chloro-4-{4-[(2-ethyl-imidazo[5,1-*b*]thiazol-3-yl)-hydroxy-methyl]-[1,2,3]triazol-1-yl}-phenoxy)-propan-1-ol. LC-MS (acidic): $t_R$ = 0.66; $[M+H]^+$ = 433.77.

**Example 321: *rac*-[1-(2,5-Difluoro-4-methoxy-phenyl)-1*H*-[1,2,3]triazol-4-yl]-(2-ethyl-imidazo[5,1-*b*]thiazol-3-yl)-methanol**

Prepared according to the procedure described for Example 318, using intermediate 3, rac-1-(2-ethylimidazo[5,1-*b*]thiazol-3-yl)prop-2-yn-1-ol and 1-azido-2,5-difluoro-4-methoxybenzene. Purification by preparative HPLC (basic conditions) to give *rac*-[1-(2,5-difluoro-4-methoxy-phenyl)-1*H*-[1,2,3]triazol-4-yl]-(2-ethyl-imidazo[5,1-b]thiazol-3-yl)-methanol. LC-MS (QC): $t_R$ = 0.603; $[M+H]^+$ = 392.1.

**Example 322: [1-(4-Benzyloxy-3-fluoro-phenyl)-1*H*-[1,2,3]triazol-4-yl]-[2-(*trans*-2-fluoro-cyclopropyl)-imidazo[5,1-*b*]thiazol-3-yl]-methanol**

Step 1: Preparation of 4-azido-1-(benzyloxy)-2-fluorobenzene

Prepared according to the procedure described for Example 45, step 1 using (4-(benzyloxy)-3-fluorophenyl)boronic acid.

Step 2: Prepared according to the procedure described for Example 45, step 2 and using intermediate 9, *trans*-1-[(2-fluoro-cyclopropyl)-imidazo[5,1-*b*]thiazol-3-yl]prop-2-yn-1-ol and 4-azido-1-(benzyloxy)-2-fluorobenzene. Purification by preparative HPLC (acidic conditions) to give [1-(4-benzyloxy-3-fluoro-phenyl)-1*H*-[1,2,3]triazol-4-yl]-[2-(*trans*-2-fluoro-cyclopropyl)-imidazo[5,1-*b*]thiazol-3-yl]-methanol. LC-MS (QC): $t_R$ = 0.851; $[M+H]^+$ = 480.2.

**Example 323: *rac*-2-Chloro-4-{4-[(2-ethyl-imidazo[5,1-*b*]thiazol-3-yl)-hydroxy-methyl]-[1,2,3]triazol-1-yl}-phenol**

Prepared according to the procedure described for Example 148, using intermediate 3, rac-1-(2-ethylimidazo[5,1-*b*]thiazol-3-yl)prop-2-yn-1-ol and 4-azido-2-chlorophenol. Purification by preparative HPLC (basic conditions) to give rac-2-chloro-4-{4-[(2-ethyl-imidazo[5,1-b]thiazol-3-yl)-hydroxy-methyl]-[1,2,3]triazol-1-yl}-phenol. LC-MS (QC): $t_R$ = 0.541; $[M+H]^+$ = 376.0.

**Example 324: *rac*-2-Chloro-4-(4-{hydroxy-[2-(1-methyl-cyclopropyl)-imidazo[5,1-*b*]thiazol-3-yl]-methyl}-[1,2,3]triazol-1-yl)-phenol**

Prepared according to the procedure described for Example 148, using intermediate 5, rac-1-(2-(1-methylcyclopropyl)imidazo[5,1-*b*]thiazol-3-yl)prop-2-yn-1-ol and 4-azido-2-chlorophenol. Purification by preparative HPLC (basic conditions) to give *rac*-2-chloro-4-(4-{hydroxy-[2-(1-methyl-cyclopropyl)-imidazo[5,1-b]thiazol-3-yl]-methyl}-[1,2,3]triazol-1-yl)-phenol. LC-MS (QC): $t_R$ = 0.638; $[M+H]^+$ = 402.1.

**Example 325: 2-Chloro-4-(4-{[2-(*cis*-2-fluoro-cyclopropyl)-imidazo[5,1-*b*]thiazol-3-yl]-hydroxy-methyl)-[1,2,3]triazol-1-yl)-phenol**

Prepared according to the procedure described for Example 148, using intermediate 8, *cis*-1-[(2-fluoro-cyclopropyl)-imidazo[5,1-*b*]thiazol-3-yl]prop-2-yn-1-ol and 4-azido-2-chlorophenol. Purification by preparative HPLC (basic conditions) to give 2-chloro-4-(4-{[2-(*cis*-2-fluoro-cyclopropyl)-imidazo[5,1-*b*]thiazol-3-yl]-hydroxy-methyl}-[1,2,3]triazol-1-yl)-phenol. LC-MS (QC): $t_R$ = 0.541; $[M+H]^+$ = 406.0.

**Example 326: *rac*-(2-Cyclopropyl-imidazo[5,1-*b*]thiazol-3-yl)-[1-(4-methoxy-2-methyl-phenyl)-5-methyl-1*H*-[1,2,3]triazol-4-yl]-methanol**

Step 1: Preparation of ethyl 1-(4-methoxy-2-methylphenyl)-5-methyl-1*H*-1,2,3-triazole-4-carboxylate According to the procedure described for the preparation of example 279, step 1 but using 1-azido-4-methoxy-2-methylbenzene (prepared as described for example 45, step 1 using (4-methoxy-2-methylphenyl)boronic acid) and ethyl 3-oxobutanoate as starting materials. LC-MS (acidic): $t_R$ = 0.89; $[M+H]^+$ = 276.23.

Step 2: Preparation of (1-(4-methoxy-2-methylphenyl)-5-methyl-1*H*-1,2,3-triazol-4-yl)methanol

According to the procedure described for the preparation of example 279, step 2 but using ethyl 1-(4-methoxy-2-methylphenyl)-5-methyl-1*H*-1,2,3-triazole-4-carboxylate as starting material. (1-(4-Methoxy-2-methylphenyl)-5-methyl-1*H*-1,2,3-triazol-4-yl)methanol is obtained as a pale yellow gum. LC-MS (acidic): $t_R$ = 0.64, $[M+H]^+$ = 234.21.

Step 3: Preparation of 1-(4-methoxy-2-methylphenyl)-5-methyl-1H-1,2,3-triazole-4-carbaldehyde

Prepared following the procedure described for Example 222, step 3 but using (1-(4-methoxy-2-methylphenyl)-5-methyl-1*H*-1,2,3-triazol-4-yl)methanol. 1-(4-Methoxy-2-methylphenyl)-5-methyl-1H-1,2,3-triazole-4-carbaldehyde is obtained as a brown gum. LC-MS (acidic): $t_R$ = 0.82, $[M+H]^+$ = 232.21.

Step 4: Preparation of *rac*-(2-cyclopropyl-imidazo[5,1-*b*]thiazol-3-yl)-[1-(4-methoxy-2-methyl-phenyl)-5-methyl-1*H*-[1,2,3]triazol-4-yl]-methanol (Example 326)

Prepared following the procedure described for Example 96 using intermediate 10, 3-bromo-2-cyclopropylimidazo[5,1-*b*]thiazole and 1-(4-methoxy-2-methylphenyl)-5-methyl-1*H*-1,2,3-triazole-4-carbaldehyde. Purification by preparative HPLC (basic conditions) followed by preparative HPLC (acidic conditions) to give *rac*-(2-cyclopropyl-imidazo[5,1-*b*]thiazol-3-yl)-[1-(4-methoxy-2-methyl-phenyl)-5-methyl-1*H*-[1,2,3]triazol-4-yl]-methanol. LC-MS (QC): $t_R$ = 0.631; $[M+H]^+$ = 396.1.

**Example 327: *rac*-(2-Cyclopropyl-imidazo[5,1-*b*]thiazol-3-yl)-[1-(3-fluoro-4-methoxy-phenyl)-1*H*-[1,2,3]triazol-4-yl]-methanol**

Step 1: Preparation of 4-azido-2-fluoro-1-methoxybenzene

Prepared according to the procedure described for Example 45, step 1 using (3-fluoro-4-methoxyphenyl)boronic acid.

Step 2: Prepared according to the procedure described for Example 45, step 2 and using intermediate 1, rac-1-(2-cyclopropylimidazo[5,1-*b*]thiazol-3-yl)prop-2-yn-1-ol and 4-azido-2-fluoro-1-methoxybenzene. Purification by preparative HPLC (basic conditions) to give rac-(2-cyclopropyl-imidazo[5,1-b]thiazol-3-yl)-[1-(3-fluoro-4-methoxy-phenyl)-1*H*-[1,2,3]triazol-4-yl]-methanol. LC-MS (QC): $t_R$ = 0.615; $[M+H]^+$ = 386.1.

**Example 328: 2-Chloro-4-(4-{[2-(*trans*-2-fluoro-cyclopropyl)-imidazo[5,1-*b*]thiazol-3-yl]-hydroxy-methyl)-[1,2,3]triazol-1-yl)-phenol**

Prepared according to the procedure described for Example 148, using intermediate 9, *trans*-1-[(2-fluoro-cyclopropyl)-imidazo[5,1-*b*]thiazol-3-yl]prop-2-yn-1-ol and 4-azido-2-chlorophenol. Purification by preparative HPLC (basic conditions) followed by preparative HPLC (acidic conditions) to give 2-chloro-4-(4-{[2-(*trans*-2-fluoro-cyclopropyl)-imidazo[5,1-b]thiazol-3-yl]-hydroxy-methyl}-[1,2,3]triazol-1-yl)-phenol. LC-MS (QC): $t_R$ = 0.574; $[M+H]^+$ = 406.0.

**Example 329: *rac*-2-Chloro-4-(4-{[2-(1,1-difluoro-ethyl)-imidazo[5,1-*b*]thiazol-3-yl]-hydroxy-methyl)-[1,2,3]triazol-1-yl)-phenol**

Prepared according to the procedure described for Example 148, using intermediate 14, *rac*-1-(2-(1,1-difluoroethyl)imidazo[5,1-*b*]thiazol-3-yl)prop-2-yn-1-ol and 4-azido-2-chlorophenol. Purification by preparative HPLC (basic conditions) to give *rac*-2-chloro-4-(4-{[2-(1,1-difluoro-ethyl)-imidazo[5,1-*b*]thiazol-3-yl]-hydroxy-methyl}-[1,2,3]triazol-1-yl)-phenol. LC-MS (QC): $t_R$ = 0.696; $[M+H]^+$ = 412.0.

**Example 330: *rac*-(2-Cyclopropyl-imidazo[5,1-*b*]thiazol-3-yl)-[1-(4-methoxymethyl-phenyl)-1*H*-[1,2,3]triazol-4-yl]-methanol**

Step 1: Preparation of 1-azido-4-(methoxymethyl)benzene

Prepared according to the procedure described for Example 288, step 1 using 4-(methoxymethyl)aniline.

Step 2: Prepared according to the procedure described for Example 45, step 2 and using intermediate 1, *rac-1*-(2-cyclopropylimidazo[5,1-*b*]thiazol-3-yl)prop-2-yn-1-ol and 1-azido-4-(methoxymethyl)benzene. Purification by preparative HPLC (acidic conditions) to give *rac*-(2-cyclopropyl-imidazo[5,1-*b*]thiazol-3-yl)-[1-(4-methoxymethyl-phenyl)-1H-[1,2,3]triazol-4-yl]-methanol. LC-MS (QC): $t_R$ = 0.588; $[M+H]^+$ = 382.1.

**Example 331: *rac*-4-{4-[(2-Cyclopropyl-imidazo[5,1-*b*]thiazol-3-yl)-hydroxy-methyl]-[1,2,3]triazol-1-yl}-2,5-difluoro-phenol**

Step 1: Preparation of 4-azido-2,5-difluorophenol

Prepared according to the procedure described for Example 45, step 1 using (2,5-difluoro-4-hydroxyphenyl)boronic acid.

Step 2: Prepared according to the procedure described for Example 45, step 2 and using intermediate 1, *rac-1*-(2-cyclopropylimidazo[5,1-*b*]thiazol-3-yl)prop-2-yn-1-ol and 4-azido-2,5-difluorophenol. Purification by preparative HPLC (acidic conditions) to give rac-4-{4-[(2-cyclopropyl-imidazo[5,1-b]thiazol-3-yl)-hydroxy-methyl]-[1,2,3]triazol-1-yl}-2,5-difluoro-phenol. LC-MS (QC): $t_R$ = 0.539; $[M+H]^+$ = 390.0.

**Example 332: rac-4-{4-[(2-Ethyl-imidazo[5,1-*b*]thiazol-3-yl)-hydroxy-methyl]-[1,2,3]triazol-1-yl}-2,5-difluoro-phenol**

Prepared according to the procedure described for Example 331, using intermediate 3, rac-1-(2-ethylimidazo[5,1-*b*]thiazol-3-yl)prop-2-yn-1-ol and 4-azido-2,5-difluorophenol. Purification by preparative HPLC (acidic conditions) to give rac-4-{4-[(2-ethyl-imidazo[5,1-b]thiazol-3-yl)-hydroxy-methyl]-[1,2,3]triazol-1-yl}-2,5-difluoro-phenol. LC-MS (QC): $t_R$ = 0.517; $[M+H]^+$ = 378.0.

**Example 333: rac-[1-(5-Chloro-2-fluoro-4-methoxy-phenyl)-5-methyl-1H-[1,2,3]triazol-4-yl]-(2-cyclopropyl-imidazo[5,1-b]thiazol-3-yl)-methanol**

Step 1: Preparation of ethyl 1-(5-chloro-2-fluoro-4-methoxyphenyl)-5-methyl-1*H*-1,2,3-triazole-4-carboxylate According to the procedure described for the preparation of example 279, step 1 but using 1-azido-5-chloro-2-fluoro-4-methoxybenzene (prepared as described for example 288, step 1 using 5-chloro-2-fluoro-4-methoxyaniline) and ethyl 3-oxobutanoate as starting materials. LC-MS (acidic): $t_R$ = 0.93; $[M+H]^+$ = 314.09. Step 2: Preparation of (1-(5-chloro-2-fluoro-4-methoxyphenyl)-5-methyl-1*H*-1,2,3-triazol-4-yl)methanol According to the procedure described for the preparation of example 279, step 2 but using ethyl 1-(5-chloro-2-fluoro-4-methoxyphenyl)-5-methyl-1*H*-1,2,3-triazole-4-carboxylate as starting material. (1-(5-Chloro-2-fluoro-4-methoxyphenyl)-5-methyl-1*H*-1,2,3-triazol-4-yl)methanol is obtained as a pink solid. LC-MS (acidic): $t_R$ = 0.70, $[M+H]^+$ = 272.13.

Step 3: Preparation of 1-(5-chloro-2-fluoro-4-methoxyphenyl)-5-methyl-1*H*-1,2,3-triazole-4-carbaldehyde Prepared following the procedure described for Example 222, step 3 but using (1-(5-chloro-2-fluoro-4-methoxyphenyl)-5-methyl-1*H*-1,2,3-triazol-4-yl)methanol. 1-(5-Chloro-2-fluoro-4-methoxyphenyl)-5-methyl-1*H*-1,2,3-triazole-4-carbaldehyde is obtained as a beige solid. LC-MS (acidic): $t_R$ = 0.87, $[M+H]^+$ = 270.13.

Step 4: Preparation of *rac*-[1-(5-chloro-2-fluoro-4-methoxy-phenyl)-5-methyl-1*H*-[1,2,3]triazol-4-yl]-(2-cyclopropyl-imidazo[5,1-b]thiazol-3-yl)-methanol (Example 333)

Prepared following the procedure described for Example 96 using intermediate 10, 3-bromo-2-cyclopropylimidazo[5,1-*b*]thiazole and 1-(5-chloro-2-fluoro-4-methoxyphenyl)-5-methyl-1*H*-1,2,3-triazole-4-carbaldehyde. Purification by preparative HPLC (acidic conditions) to give rac-[1-(5-chloro-2-fluoro-4-methoxy-phenyl)-5-methyl-1*H*-[1,2,3]triazol-4-yl]-(2-cyclopropyl-imidazo[5,1-*b*]thiazol-3-yl)-methanol. LC-MS (QC): $t_R$ = 0.673; $[M+H]^+$ = 434.1.

**Example 334: *rac*-(2-Cyclopropyl-imidazo[5,1-*b*]thiazol-3-yl)-[5-iodo-1-(4-methoxy-phenyl)-1*H*-[1,2,3]triazol-4-yl]-methanol**

Step 1: Preparation of *rac*-1-(2-cyc!opropylimidazo[5,1-*b*]thiazol-3-yl)-3-iodoprop-2-yn-1-ol

To a degassed solution of intermediate 1, *rac*-1-(2-cyclopropylimidazo[5,1-*b*]thiazol-3-yl)prop-2-yn-1-ol (130 mg; 0.596 mmol) and *N*-iodomorpholine hydroiodide (230 mg; 0.655 mmol) in THF (3 ml) at RT is added copper (I) iodide (5.7 mg; 0.030 mmol). The reaction mixture is stirred at RT for 30 min and the solution is used as such in the step 2.

Step 2: Preparation of *rac*-(2-cyclopropyl-imidazo[5,1-*b*]thiazol-3-yl)-[5-iodo-1-(4-methoxy-phenyl)-1*H*-[1,2,3]triazol-4-yl]-methanol (Example 334)

Prepared according to the procedure described for Example 45, step 2 and using rac-1-(2-cyclopropylimidazo[5,1-*b*]thiazol-3-yl)-3-iodoprop-2-yn-1-ol (as a reaction mixture) and 1-azido-4-methoxybenzene. Purification by preparative HPLC (basic conditions) to give rac-(2-cyclopropyl-imidazo[5,1-*b*]thiazol-3-yl)-[5-iodo-1-(4-methoxy-phenyl)-1*H*-[1,2,3]triazol-4-yl]-methanol. LC-MS (QC): $t_R$ = 0.650; $[M+H]^+$ = 494.0.

**Example 335: [2-(*trans*-2-Fluoro-cyclopropyl)-imidazo[5,1-*b*]thiazol-3-yl]-[5-fluoro-1-(4-methoxy-phenyl)-1*H*-[1,2,3]triazol-4-yl]-methanol**

Step 1: Preparation of 1-(2-(*trans*-2-fluorocyclopropyl)imidazo[5,1-*b*]thiazol-3-yl)-3-iodoprop-2-yn-1-ol

Prepared according to the procedure described for Example 334, step 1 using intermediate 9, *trans*-1-[(2-fluoro-cyclopropyl)-imidazo[5,1-*b*]thiazol-3-yl]prop-2-yn-1-ol.

Step 2: Preparation of [2-(*trans*-2-fluoro-cyclopropyl)-imidazo[5,1-*b*]thiazol-3-yl]-[5-iodo-1-(4-methoxy-phenyl)-1*H*-[1,2,3]triazol-4-yl]-methanol

Prepared according to the procedure described for Example 334, step 2 using 1-(2-(*trans*-2-fluorocyclopropyl)imidazo[5,1-*b*]thiazol-3-yl)-3-iodoprop-2-yn-1-ol and 1-azido-4-methoxybenzene. Purification by preparative HPLC (acidic conditions) to give [2-(*trans*-2-fluoro-cyclopropyl)-imidazo[5,1-*b*]thiazol-3-yl]-[5-iodo-1-(4-methoxy-phenyl)-1H-[1,2,3]triazol-4-yl]-methanol. LC-MS (acidic): $t_R$ = 0.71; $[M+H]^+$ = 512.00.

Step 3: Preparation of [2-(*trans*-2-fluoro-cyclopropyl)-imidazo[5,1-*b*]thiazol-3-yl]-[5-fluoro-1-(4-methoxy-phenyl)-1*H*-[1,2,3]triazol-4-yl]-methanol (Example 335)

To a solution of [2-(*trans*-2-fluoro-cyclopropyl)-imidazo[5,1-*b*]thiazol-3-yl]-[5-iodo-1-(4-methoxy-phenyl)-1*H*-[1,2,3]triazol-4-yl]-methanol (30 mg; 0.059 mmol) in toluene (3 ml) are added silver (I) fluoride (37.6 mg; 0.293 mmol) and *N*,*N*,*N*,*N*-tetramethylethylenediamine (3.44 mg; 0.029 mmol) at RT. The reaction mixture is refluxed for 20 h, then cooled down to RT, filtered and concentrated under reduced pressure. Purification by preparative HPLC (basic conditions) to give [2-(*trans*-2-fluoro-cyclopropyl)-imidazo[5,1-*b*]thiazol-3-yl]-[5-fluoro-1-(4-methoxy-phenyl)-1*H*-[1,2,3]triazol-4-yl]-methanol. LC-MS (QC): $t_R$ = 0.655; $[M+H]^+$ = 404.1.

**Example 336: *rac*-(2-Cyclopropyl-imidazo[5,1-*b*]thiazol-3-yl)-[5-fluoro-1-(4-methoxy-phenyl)-1*H*-[1,2,3]triazol-4-yl]-methanol**

Prepared according to the procedure described for Example 335, step 3 using and rac-(2-cyclopropyl-imidazo[5,1-*b*]thiazol-3-yl)-[5-iodo-1-(4-methoxy-phenyl)-1*H*-[1,2,3]triazol-4-yl]-methanol. Purification by preparative HPLC (basic conditions) to give *rac*-(2-cyclopropyl-imidazo[5,1-*b*]thiazol-3-yl)-[5-fluoro-1-(4-methoxy-phenyl)-1H-[1,2,3]triazol-4-yl]-methanol. LC-MS (QC): $t_R$ = 0.640; $[M+H]^+$ = 386.1.

The absolute chirality and the binding mode of the compound of Example 2a was determined by an X-ray diffraction analysis of the corresponding compound-enzyme co-crystals using the following experimental procedure:

**1. Protein purification and co-crystallization:**

IDO1 protein is expressed and purified following a procedure described in the literature (Biochem et Biophysica Acta 1814 (2011) 1947-1954). IDO1 protein is concentrated to 29 mg/ml in a buffer containing 10 mM MES (2-(N-morpholino)ethanesulfonic acid) pH 6.50, 100 mM NaCl and 2 mM TCEP (Tris(2-carboxyethyl)phosphine hydrochloride).

The protein solution is incubated with the compound of Example 9a at a final concentration of 2 mM for 3 hours at 277 K. The solution is then centrifuged for 5 minutes at 15,000 rpm at 277 K using an Eppendorf 5424R benchtop centrifuge. The centrifuged solution is mixed with a reservoir solution containing 100 mM arginine hydrochloride, 100 mM threonine, 100 mM histidine monohydrochloride monohydrate, 100 mM 5-hydroxylysine hydrochloride, 100 mM trans-4-hydroxy-L-proline, 100 mM BES (N,N-Bis(2-hydroxyethyl)-2-aminoethanesulfonic acid, N,N-Bis(2-hydroxyethyl)taurine )-triethanolamine pH 7.5, 2% (w/v) 3-(N-Phenylmethyl-N,N-dimethylammonio)propanesulfonate, 10% (w/v) PEG 8000 and 20% (w/v) 1,5-Pentanediol. Co-crystals of IDO1 and the compound of Example 2a are finally obtained by vapour diffusion from sitting drops at 293 K.

**2. X-ray data collection and structure determination:**

The above-mentioned co-crystals are harvested using nylon loops and placed directly in liquid nitrogen. Synchrotron data are collected at beamline X06DA of the Swiss Light Source at the Paul Scherrer Institute, Villigen, Switzerland using a Pilatus 2M-F detector. Diffraction images are processed using the program XDS (Acta Cryst. (2010) D66, 125-132). The preliminary structure is solved using the program Phaser (J. Appl. Cryst. (2007) 40, 658-674). Refinement and rebuilding of the structure are carried out using the programs Refmac5 (Acta Cryst. (2004) D60, 2284-2295) and Coot (Acta Cryst. (2010) D66, 486-501), respectively. R-free is calculated using a randomly selected 5% of total data from the observed reflections. Based on the measured electron density, it is unambiguously established that the compound of Example 2a is the (R)-enantiomer.

**Data collection and refinement statistics**

| | |
|---|---|
| Final resolution (A) | 2.5 |
| Space group | $P2_12_12_1$ |
| Unit cell dimensions (A) | a=85.1, b=91.6, c=129.2 |
| Wavelength (Å) | 1.0000 |
| observed/unique reflections | 234411/35638 |
| Resolution range (Å)[a] | 45.78-2.50 (2.65-2.50) |
| Completeness (%) | 99.8 (99.0) |
| Rmerge (%)[b] | 16.9 (329.9) |
| I/σ(I) | 9.35 (0.55) |
| | |
| Refinement | |
| Rwork (%) | 23.5 |
| Rfree (%) | 29.3 |
| | |
| RMSD | |
| bond length (Å) | 0.011 |
| bond angle (°) | 1.8 |
| Ramachandran outliers | 0 |

[a] values shown in parentheses correspond to the highest resolution shell

[b]

$$R = \frac{\sum_{hkl} \sum_j \left| I_{hkl,j} - \langle I_{hkl} \rangle \right|}{\sum_{hkl} \sum_j I_{hkl,j}}$$

(R)- or (S)-configuration of the compounds according to the present invention is assigned to the compounds of Examples 9a, 16a,18a, 35a, 49a, 87a, 100a, 101a, 137a, 139a, 140a, 142a, 145a and 148a, 192a, 268a, 275, 276, 277, 282 and 283 based on the assumption that the binding mode of the more active enantiomer is the same as the one for the compound of Example 2a.

**BIOLOGICAL TESTS**

**1) Testing compounds for IDO inhibitory activity in an IDO1 enzymatic assay:**

Recombinant full-length human IDO1 with a N-terminal hexahistidine tag expressed in *E. coli* and purified to homogeneity is incubated at a final concentration of 2nM in assay buffer consisting of 37.5mM phosphate buffer at pH6.5 supplemented with 10mM ascorbic acid, 0.45μM methylene blue, 50U/ml catalase, 0.01% BSA, and 0.01% Tween 20 (protocol modified from Seegers et al, JBS 2014). Example compounds are serially diluted in DMSO, further diluted in phosphate buffer, and added to the enzyme at final concentrations ranging from 10μM to 0.5 nM. The final DMSO concentration is 0.6%. Following a pre-incubation of 30 minutes at RT, the reaction is started by the addition of L-tryptophan at a final concentration of 5μM in assay buffer. After 30 minutes of incubation at RT, 3μL of the 20 μl reaction mixture are transferred to a 384 deep well plate containing 25μL of deionized water. 100 μl of 200 nM L-Tryptophan-(indole-d5) in cold 100% methanol are added followed by a 10 minutes centrifugation at 3220 x g at 4°C. An additional 75 μL of deionized water are then added followed by a 10 minutes centrifugation at 3220 x g at 4°C. The product of the reaction N'-Formylkynurenine (NFK) is quantified by LCMS and normalized to the L-Tryptophan-(indole-d5) signal. Samples with 0.6% DMSO (0% effect) and a TDO/IDO inhibitor (100% effect) are used as control samples to set the parameters for the non-linear regression necessary for the determination of the half-maximal inhibitory concentration (IC50) for each compound. For each compound concentration the percentage of activity compared to 0% and 100% effect is calculated as average ± STDEV (each concentration measured in duplicate). IC50 values and curves are generated with XLfit software (IDBS) using Dose-Response One Site model 203 (four parameter logistic curve model). The calculated IC50 values may fluctuate depending on the daily assay performance. Fluctuations of this kind are known to those skilled in the art. When compounds are measured multiple times, mean values are given.

**2) Testing compounds for TDO inhibitory activity in a TDO2 enzymatic assay:**

Recombinant human TDO comprising amino acids 19-407 with a N-terminal hexahistidine tag expressed in *E.coli* and purified to homogeneity is incubated at a final concentration of 15nM in assay buffer consisting of 75mM phosphate buffer at pH7 supplemented with 100μM ascorbic acid, 50U/ml Catalase, 0.01% BSA, and 0.01% Tween 20 (protocol modified from Seegers et al, JBS 2014). Example compounds are serially diluted in DMSO, further diluted in phosphate buffer, and added to the reaction mixture at final concentrations ranging from 10μM to 0.5 nM. The final DMSO concentration is 0.6%. Following a pre-incubation of 30 minutes at RT, the reaction is started by the addition of L-tryptophan at a final concentration of 200μM in assay buffer. After 30 minutes of incubation at RT, 3μL of the reaction mixture are transferred to a 384 deep well plate containing 25μL of deionized water. 100μl of 200nM L-Tryptophan-(indole-d5) in cold 100% methanol are added followed by a 10 minutes centrifugation at 3220 x g at 4°C. An additional 75μL of deionized water are then added followed by a 10 minutes centrifugation at 3220 x g at 4°C. The product of the reaction N'-Formylkynurenine (NFK) is quantified by LCMS and normalized to the L-Tryptophan-(indole-d5) signal. Samples with 0.6% DMSO (0% effect) and a TDO/IDO inhibitor (100% effect) are used as control samples to set the parameters for the non-linear regression necessary for the determination of the half-maximal inhibitory concentration (IC50) for each compound. For each compound concentration the percentage of activity compared to 0% and 100% effect is calculated as average ± STDEV (each concentration measured in duplicate). IC50 values and curves are generated with XLfit software (IDBS) using Dose-Response One Site model 203 (four parameter logistic curve model). The calculated IC50 values may fluctuate depending on the daily assay performance. Fluctuations of this kind are known to those skilled in the art. When compounds are measured multiple times, mean values are given.

3) **Testing compounds for IDO/TDO inhibitory activity and toxicity in cell-based assays**

SW48 cells (ATCC, CCL-231) are used to measure compounds for TDO inhibitory activity and are routinely maintained in DMEM high glucose/GlutaMAX™/pyruvate 90 %(v/v), FCS 10%(v/v), Penicilin/streptomycin 1 %(v/v). SKOV3 cells (NCI, No. 0503405) which upregulate IDO1 after stimulation with IFNy are used to measure compounds for IDO inhibitory activity. SKOV3 cells are routinely maintained in RPMI 90 %(v/v), FCS 10%(v/v), Penicilin/streptomycin 1 %(v/v). SW48 or SKOV3 cells are seeded in 384 well plates at a density of 8000 cells in 45ul per well or 4000 cells in 45ul per well, respectively. Plates are incubated at 37°C / 5% $CO_2$ for 24 hours. On the next day, 10ul compound in serial dilutions (tested concentration range 10uM-40nM) and 200uM L-tryptophan are added (SKVO3 receive in addition

IFNy at a final concentration of 50ng/ml). After 24 hours of incubation at 37°C / 5% $CO_2$, 3ul of the supernatant per well is transferred to 25ul $H_2O$ per well in a 384 deep well plate and 25ul of the supernatant per well is transferred to waste. The SKOV3 and SW48 cell plates with 25ul supernatant per well remaining are used to measure viability (see below). The 384 deep well plate containing 3ul supernatant and 25ul $H_2O$ per well are further processed for LCMS: After the addition of 100ul L-tryptophan-(indole-d5) (Sigma 615862) at 200nM in methanol, the 384 deep well plates are centrifuged for 10 minutes at 3220 x g at 4°C, 75ul $H_2O$ is added per well and plates centrifuged again for 10 minutes at 3220 x g at 4°C. N-formylkynurenine and kynurenine are quantified by LCMS, normalized to the internal standard L-tryptophan-(indole-d5) and the sum is calculated. Samples with 0.2% DMSO (0% effect) and a TDO/IDO inhibitor (100% effect) are used as control samples to set the parameters for the non-linear regression necessary for the determination of the IC50 for each compound. For each compound concentration the percentage of activity compared to 0% and 100% effect is calculated as average ± STDEV (each concentration measured in duplicate). IC50 values and curves are generated with XLfit software (IDBS) using Dose-Response One Site model 203. The calculated IC50 values may fluctuate depending on the daily cellular assay performance. Fluctuations of this kind are known to those skilled in the art. When compounds are measured multiple times, mean values are given.

As inhibition of NFK and KYN production can simply be an effect of cytotoxicity, a viability assay (CellTiter-Glo 2.0Luminescent Cell Viability Assay, Promega Catalog # G9243) is performed in parallel. CellTiter-Glo reagent is added (25ul/well) to cell plates, incubated for 15 minutes at room temperature in the dark and luminescence is measured with the EnVision Multilabel Reader from Perkin Elmer according to manufacturer's instructions. The luminescent signal is proportional to the amount of ATP present. The amount of ATP is directly proportional to the number of viable cells present. Samples with 0.2% DMSO (0% effect) and a toxic compound (100% effect) are used as control samples to set the parameters for the non-linear regression. For each compound concentration the percentage of activity compared to 0% and 100% effect is calculated as average ± STDEV (each concentration measured in duplicate). Tox IC50 values and curves are generated with XLfit software (IDBS) using Dose-Response One Site model 203. The calculated IC50 values may fluctuate depending on the daily cellular assay performance. Fluctuations of this kind are known to those skilled in the art. When compounds are measured multiple times, mean values are given.

The results of biological tests 1 and 2 obtained for the compounds of Examples 1 to 336 are summarized in Table 1 below:

| Example Number | hIDO activity (IC50 in nM) | hTDO activity (IC50 in nM) |
|---|---|---|
| **1** | 90.3 | >10200 |
| **2** | 28.1 | >10200 |
| **2a** | 11.7 | >10200 |
| **3** | 49.2 | >10200 |
| **4** | 34.5 | >10200 |
| **5** | 74.2 | >10200 |
| **6** | 119 | >10200 |
| **7** | 50.9 | >10200 |
| **8** | 35.5 | >10200 |
| **9** | 8.62 | >10200 |
| **9a** | 5.98 | >10200 |
| **10** | 18.4 | >10200 |
| **11** | 39.6 | >10200 |
| **12** | 20.8 | 6740 |
| **13** | 54.4 | 8410 |
| **14** | 79.6 | >10200 |
| **15** | 95.8 | >10200 |
| **16** | 9.03 | 7880 |
| **16a** | 4.55 | 4570 |
| **17** | 154 | >10200 |
| **18** | 9.32 | >10200 |
| **18a** | 13.5 | >10200 |
| **19** | 182 | >10200 |
| **20** | 21.3 | >10200 |
| **21** | 18.1 | >10200 |
| **22** | 39.6 | >10200 |
| **23** | 24 | >10200 |
| **24** | 22.8 | >10200 |
| **25** | 18.3 | >10200 |
| **26** | 5.68 | 4190 |
| **27** | 24.1 | >10200 |
| **28** | 28.8 | >10200 |
| **29** | 163 | >10200 |
| **30** | 63.2 | >10200 |
| **31** | 108 | >10200 |
| **32** | 5.30 | >10200 |
| **33** | 20.0 | >10200 |
| **34** | 21.9 | >10200 |
| **35** | 8.83 | >10200 |
| **35a** | 9.7 | >10200 |
| **36** | 11.1 | 8520 |
| **37** | 175 | >10200 |
| **38** | 72.7 | >10200 |
| **39** | 21.9 | >10200 |
| **40** | 68.3 | >10200 |
| **41** | 305 | >10200 |
| **42** | 20.2 | >10200 |
| **43** | 95.0 | >10200 |
| **44** | 38.8 | >10200 |
| **45** | 46.4 | >10200 |
| **46** | 14.3 | >10200 |
| **47** | 44.4 | >10200 |
| **48** | 60.6 | >10200 |
| **49** | 10.9 | 2110 |
| **50** | 9.63 | 1970 |
| **51** | 37.1 | >10200 |
| **52** | 41.8 | 5055 |
| **53** | 73.6 | >10200 |
| **54** | 12.3 | >10200 |
| **55** | 205 | >10200 |
| **56** | 422 | >10200 |
| **57** | 391 | >10200 |
| **58** | 69.1 | 8050 |
| **59** | 50.4 | 8405 |
| **60** | 294 | >10200 |
| **61** | 493 | >10200 |
| **62** | 795 | >10200 |
| **63** | 27.7 | >10200 |
| **64** | 177 | >10200 |
| **65** | 14.2 | >10200 |
| **66** | 200 | >10200 |
| **67** | 50.2 | 9160 |
| **68** | 8.08 | >10200 |
| **69** | 22.7 | >10200 |
| **70** | 201 | >10200 |
| **71** | 55.9 | >10200 |
| **72** | 35.5 | >10200 |
| **73** | 36.1 | >10200 |
| **74** | 24.3 | >10200 |
| **75** | 40.7 | >10200 |
| **76** | 363 | >10200 |
| **77** | 32.7 | >10200 |
| **78** | 17.4 | >10200 |
| **79** | 7.22 | >10200 |
| **80** | 51.2 | >10200 |
| **81** | 36.1 | >10200 |
| **82** | 44.2 | >10200 |
| **83** | 10.9 | >10200 |
| **84** | 81.7 | >10200 |
| **85** | 11.5 | >10200 |
| **86** | 23.1 | >10200 |
| **87** | 20.7 | >10200 |
| **88** | 12.6 | >10200 |
| **89** | 204 | >10200 |
| **90** | 18.7 | >10200 |
| **91** | 12.9 | >10200 |
| **92** | 32.7 | >10200 |
| **93** | 25.0 | >10200 |
| **94** | 12.8 | >10200 |
| **95** | 18.3 | >10200 |
| **96** | 163.4 | 2527 |
| **97** | 121 | 1940 |
| **98** | 186 | 864 |
| **99** | 31.8 | 3570 |
| **100** | 41.8 | 3350 |
| **100a** | 17.5 | 2450 |
| **101** | 17 | 7145 |

| | | |
|---|---|---|
| **102** | 23.2 | >10200 |
| **103** | 78.1 | >10200 |
| **104** | 30.6 | >10200 |
| **105** | 22.1 | >10200 |
| **106** | 19.6 | >10200 |
| **107** | 16.6 | >10200 |
| **108** | 10.0 | >10200 |
| **109** | 24 | 7110 |
| **110** | 13.1 | 4290 |
| **111** | 28.8 | >10200 |
| **112** | 45.6 | 6220 |
| **113** | 14.3 | 7720 |
| **114** | 108 | >10200 |
| **115** | 12.7 | >10200 |
| **116** | 12.3 | 5210 |
| **117** | 93.3 | >10200 |
| **118** | 15.8 | 9550 |
| **119** | 133 | 9720 |
| **120** | 11.0 | >10200 |
| **121** | 32.0 | 7720 |
| **122** | 25.6 | 2070 |
| **123** | 8.09 | 6770 |
| **124** | 109 | >10200 |
| **125** | 15 | 8710 |
| **126** | 20.1 | >10200 |
| **127** | 8.21 | 7750 |
| **128** | 16.5 | 9780 |
| **129** | 9.29 | 9780 |
| **130** | 13.1 | >10200 |
| **131** | 22.3 | 2150 |
| **132** | 18.2 | 1770 |
| **133** | 24.2 | >10200 |
| **134** | 11.4 | >10200 |
| **135** | 14.0 | >10200 |
| **136** | 10.8 | >10200 |
| **137** | 11.1 | >10200 |
| **137a** | 4.09 | >10200 |
| **138** | 16.5 | >10200 |
| **139** | 14.6 | >10200 |

| | | |
|---|---|---|
| **140** | 8.46 | >10200 |
| **140a** | 7.31 | >10200 |
| **141** | 27.2 | >10200 |
| **142** | 13.3 | >10200 |
| **142a** | 9.51 | >10200 |
| **143** | 18.1 | >10200 |
| **144** | 11.2 | >10200 |
| **145** | 7.77 | >10200 |
| **145a** | 8.77 | >10200 |
| **146** | 44.4 | >10200 |
| **147** | 12.9 | >10200 |
| **148** | 4.31 | 5130 |
| **148a** | 5.25 | 4440 |
| **149** | 3.80 | >10200 |
| **150** | 4.83 | 9290 |
| **151** | 55.8 | >10200 |
| **152** | 184 | >10200 |
| **153** | 2320 | >10200 |
| **154** | 366 | 7270 |
| **155** | 734 | >10200 |
| **156** | 406 | >10200 |
| **157** | 586 | >10200 |
| **158** | 466 | 5440 |
| **159** | 238 | 8310 |
| **160** | 230 | 5320 |
| **161** | 257 | >10200 |
| **162** | 1250 | >10200 |
| **163** | 496 | >10200 |
| **164** | 317 | >10200 |
| **165** | 716 | >10200 |
| **166** | 587 | >10200 |
| **167** | 639 | >10200 |
| **168** | 189 | 1065 |
| **169** | 60 | 2940 |
| **170** | 550 | 3825 |
| **171** | 578 | >10200 |
| **172** | 272 | 4080 |
| **173** | 732 | >10200 |
| **174** | 279 | 7710 |

| | | |
|---|---|---|
| **175** | 122 | >10200 |
| **176** | 260 | >10200 |
| **177** | 138 | 8620 |
| **178** | 515 | >10200 |
| **49a** | 2.70 | 1035 |
| **87a** | 4.55 | >10200 |
| **101a** | 6.31 | 4410 |
| **139a** | 5.44 | >10200 |
| **179** | 5.36 | >10200 |
| **180** | 33.6 | >10200 |
| **181** | 7.86 | 4330 |
| **182** | 26.0 | >10200 |
| **183** | 15.5 | >10200 |
| **184** | 14.5 | >10200 |
| **185** | 14.9 | >10200 |
| **186** | 17.3 | >10200 |
| **187** | 13.7 | >10200 |
| **188** | 37.8 | 8320 |
| **189** | 30.6 | >10200 |
| **190** | 8.80 | >10200 |
| **191** | 109 | >10200 |
| **192** | 13.5 | >10200 |
| **192a** | 3.71 | >10200 |
| **193** | 9.02 | >10200 |
| **194** | 70.4 | >10200 |
| **195** | 145 | >10200 |
| **196** | 17.1 | >10200 |
| **197** | 52.4 | >10200 |
| **198** | 52.4 | >10200 |
| **199** | 73.1 | >10200 |
| **200** | 55.4 | >10200 |
| **201** | 27.8 | >10200 |
| **202** | 15.0 | >10200 |
| **203** | 1350 | >10200 |
| **204** | 292 | >10200 |
| **205** | 626 | >10200 |
| **206** | 7.11 | >10200 |
| **207** | 26.9 | >10200 |
| **208** | 9.58 | 6400 |

| | | |
|---|---|---|
| **209** | 22.1 | >10200 |
| **210** | 23.5 | >10200 |
| **211** | 15.8 | >10200 |
| **212** | 10.5 | 6190 |
| **213** | 199 | 2620 |
| **214** | 127 | 8890 |
| **215** | 27.6 | >10200 |
| **216** | 29.1 | >10200 |
| **217** | 9.60 | >10200 |
| **218** | 24.1 | >10200 |
| **219** | 63.2 | >10200 |
| **220** | 27.2 | >10200 |
| **221** | 90.2 | >10200 |
| **222** | 97.2 | 7880 |
| **223** | 164 | >10200 |
| **224** | 1100 | >10200 |
| **225** | 47.2 | >10200 |
| **226** | 63.6 | >10200 |
| **227** | 77.8 | >10200 |
| **228** | 76.1 | >10200 |
| **229** | 43.7 | 1440 |
| **230** | 28.7 | 2710 |
| **231** | 22.3 | >10200 |
| **232** | 40.1 | >10200 |
| **233** | 20.3 | >10200 |
| **234** | 36.7 | 7940 |
| **235** | 24.3 | >10200 |
| **236** | 105 | >10200 |
| **237** | 155 | >10200 |
| **238** | 137 | >10200 |
| **239** | 53.0 | >10200 |
| **240** | 28.3 | 7480 |
| **241** | 66.0 | >10200 |
| **242** | 139 | >10200 |
| **243** | 101 | >10200 |
| **244** | 47.2 | >10200 |
| **245** | 40.8 | 6810 |
| **246** | 29.8 | 3800 |
| **247** | 31.3 | 8140 |
| **248** | 2.60 | 4710 |
| **249** | 74.8 | >10200 |
| **250** | 23.8 | 2300 |
| **251** | 569 | >10200 |
| **252** | 4.13 | 7010 |
| **253** | 17.2 | >10200 |
| **254** | 38.5 | >10200 |
| **255** | 14.2 | 6940 |
| **256** | 26.3 | >10200 |
| **257** | 17.5 | >10200 |
| **258** | 34.0 | >10200 |
| **259** | 85.6 | >10200 |
| **260** | 43.2 | 6510 |
| **261** | 51.7 | >10200 |
| **262** | 83.2 | >10200 |
| **263** | 20.5 | 830 |
| **264** | 93.9 | 5890 |
| **265** | 442 | >10200 |
| **266** | 179 | >10200 |
| **267** | 122 | >10200 |
| **268** | 210 | >10200 |
| **268a** | 123 | 10000 |
| **269** | 7.26 | >10200 |
| **270** | 71.5 | >10200 |
| **271** | 225 | >10200 |
| **272** | 4.44 | 3840 |
| **273** | 43.6 | >10200 |
| **274** | 36.0 | >10200 |
| **275** | 7.03 | >10200 |
| **276** | 4.63 | >10200 |
| **277** | 7.12 | >10200 |
| **278** | 8.71 | >10200 |
| **279** | 13.6 | >10200 |
| **280** | 53.2 | >10200 |
| **281** | 13.7 | 9020 |
| **282** | 5.58 | >10200 |
| **283** | 7.31 | >10200 |
| **284** | 273 | >10200 |
| **285** | 7.91 | 6470 |
| **286** | 704 | >10200 |
| **287** | 88.8 | 8670 |
| **288** | 15.1 | >10200 |
| **289** | 7.56 | >10200 |
| **290** | 15.3 | >10200 |
| **291** | 41.1 | >10200 |
| **292** | 28.9 | >10200 |
| **293** | 17.6 | >10200 |
| **294** | 35.8 | >10200 |
| **295** | 6.63 | 8840 |
| **296** | 14.1 | >10200 |
| **297** | 16.0 | >10200 |
| **298** | 23.3 | >10200 |
| **299** | 34.8 | >10200 |
| **300** | 6.66 | >10200 |
| **301** | 25.1 | >10200 |
| **302** | 6.88 | >10200 |
| **303** | 23.6 | >10200 |
| **304** | 52.2 | >10200 |
| **305** | 71.3 | >10200 |
| **306** | 17.9 | >10200 |
| **307** | 13.9 | >10200 |
| **308** | 12.7 | >10200 |
| **309** | 17.0 | >10200 |
| **310** | 50.1 | >10200 |
| **311** | 137 | >10200 |
| **312** | 29.4 | >10200 |
| **313** | 13.7 | >10200 |
| **314** | 10.2 | >10200 |
| **315** | 19.5 | >10200 |
| **316** | 13.0 | >10200 |
| **317** | 7.18 | >10200 |
| **318** | 2.99 | >10200 |
| **319** | 3.18 | 4430 |
| **320** | 2.10 | 7780 |
| **321** | 2.13 | 3680 |
| **322** | 16.2 | >10200 |
| **323** | 3.82 | 1210 |
| **324** | 12.1 | 9820 |

| | | | | | | | | |
|---|---|---|---|---|---|---|---|---|
| **325** | 8.31 | >10200 | **329** | 2.49 | 9930 | **333** | 1.46 | >10200 |
| **326** | 16.9 | 9520 | **330** | 31.6 | >10200 | **334** | 12.1 | 7280 |
| **327** | 4.29 | >10200 | **331** | 4.55 | 7180 | **335** | 23.0 | >10200 |
| **328** | 13.1 | >10200 | **332** | 5.37 | 2820 | **336** | 32.7 | >10200 |

## Claims

**1.** A compound according to Formula (I)

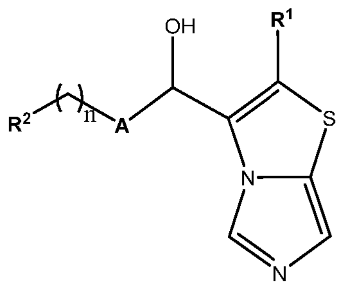

Formula (I)

wherein

A represents phenylene or 5- to 6-membered heteroarylene, wherein said phenylene or 5- to 6-membered heteroarylene independently are unsubstituted, mono- or di-substituted, wherein the substituents are independently selected from $C_{1-4}$-alkyl, halogen or $C_{3-5}$-cycloalkyl;
n represents 1 or 0;
**$R^1$** represents:

- $C_{1-4}$-alkyl;
- -C(**$R^A$**)=C(**$R^B$**)(**$R^C$**), wherein **$R^A$**, **$R^B$** and **$R^C$** are independently selected from hydrogen, $C_{1-4}$-alkyl and $C_{3-5}$-cycloalkyl; or wherein **$R^A$** and **$R^B$** together with the carbon atoms to which they are attached form $C_{4-6}$-cycloalkenyl and **$R^C$** represents hydrogen;
- $C_{1-3}$-fluoroalkyl;
- phenyl, which is unsubstituted or mono, di- or tri-substituted, wherein the substituents are independently selected from $C_{1-3}$-alkyl and halogen;
- 5-membered heteroaryl, which is unsubstituted or mono-substituted with $C_{1-4}$-alkyl; or
- $C_{3-6}$-cycloalkyl, which is unsubstituted or mono, di- or tri-substituted, wherein the substituents are independently selected from $C_{1-3}$-alkyl and halogen;

**$R^2$** represents:

- phenyl or 6-membered heteroaryl, which are independently unsubstituted, or mono-, di- or tri-substituted, wherein the substituents are independently selected from:

  - ➢ $C_{1-4}$-alkyl, cyano, nitro, halogen, hydroxy, hydroxy-$C_{1-4}$-alkyl, $C_{1-3}$-alkoxy-$C_{1-4}$-alkyl, $C_{1-3}$-fluoroalkyl, $C_{1-3}$-fluoroalkoxy, $C_{1-4}$-alkoxy, $C_{3-5}$-cycloalkoxy, hydroxymethyl-cyclopropyl, $C_{1-3}$-alkyl-carbonyl, $C_{1-4}$-alkoxy-carbonyl, amino, morpholin-4-yl or morpholin-4-yl-methyl;
  - ➢ **-N$R^{N1}R^{N2}$**, wherein **$R^{N1}$** represents hydrogen and **$R^{N2}$** represents -(C=O)-**$R^{CO}$**, wherein **$R^{CO}$** represents $C_{1-4}$-alkyl, $C_{1-4}$-alkoxy, amino, $C_{1-4}$-alkylamino, phenyl or morpholin-4-yl;
  - ➢ -(C=O)-N**$R^{N3}R^{N4}$**, wherein **$R^{N3}$** represents hydrogen or $C_{1-3}$-alkyl, and **$R^{N4}$** represents hydrogen, $C_{1-3}$-alkyl, $C_{3-5}$-cycloalkyl, hydroxy-$C_{2-4}$-alkyl, $C_{1-3}$-alkoxy-$C_{2-4}$-alkyl, cyano-$C_{1-3}$-alkyl, $C_{3-4}$-alkenyl, furanyl-$C_{1-3}$-alkyl, 2-di-$C_{1-3}$-alkylamino-$C_{2-3}$-alkyl or allyl; or wherein **$R^{N3}$** and **$R^{N4}$** together with the

nitrogen atom to which they are attached form a morpholine ring;

➢ -(NH)p-$SO_2$-N**$R^{S1}$****$R^{S2}$**, wherein **$R^{S1}$** and **$R^{S2}$** independently represent hydrogen or $C_{1-4}$-alkyl; or wherein **$R^{S1}$** and **$R^{S2}$** together with the nitrogen atom to which they are attached form a 4- to 6-membered heterocycloalkyl selected from azetidinyl, pyrrolidinyl and piperidinyl; and **p** represents 1 or 0; and

➢ **-$OR^6$**, wherein **$R^6$** represents $C_{1-3}$-alkoxy-$C_{2-4}$-alkyl, hydroxy-$C_{2-5}$-alkyl, di-hydroxy-$C_{2-5}$-alkyl, sulfamoyl-$C_{2-4}$-alkyl, pyridinyl-$C_{1-3}$-alkyl, $C_{1-3}$-alkylsulfonyl-$C_{2-4}$-alkyl, $C_{3-5}$-cycloalkyl-$C_{1-3}$-alkyl, oxetan-3-yl-$C_{1-3}$-alkyl, ($C_{1-3}$-alkyl-oxetan-3-yl)-$C_{1-3}$-alkyl, (fluoro-oxetan-3-yl)-$C_{1-3}$-alkyl, tetrahydrofuranyl-$C_{1-3}$-alkyl, hydroxy-$C_{3-5}$-cycloalkyl-$C_{1-3}$-alkyl, tetrahydropyranyl-$C_{1-3}$-alkyl, benzyl, (hydroxy-$C_{2-4}$-alkoxy)-$C_{2-4}$-alkyl, piperidin-1-yl-$C_{1-3}$-alkyl, (1-methyl-piperidin-3-yl)-$C_{1-3}$-alkyl, (1,1-dioxidotetrahydro-thiopyran-4-yl)-$C_{1-3}$-alkyl, $C_{1-3}$-alkoxy-carbonyl-$C_{1-3}$-alkyl, ((hydroxy-$C_{2-3}$-alkoxy)-$C_{2-3}$-alkoxy)-$C_{2-3}$-alkyl, $C_{1-3}$-fluoroalkoxy-$C_{2-3}$-alkyl or $C_{1-3}$-alkyl-carbonyl;

• 5-membered heteroaryl which independently is unsubstituted, or mono-, di- or tri-substituted, wherein the substituents are independently selected from:

➢ $C_{1-4}$-alkyl, cyano, halogen, $C_{1-3}$-fluoroalkyl, $C_{1-3}$-fluoroalkoxy, $C_{1-4}$-alkoxy, $C_{3-5}$-cycloalkoxy, hydroxymethyl-cyclopropyl, morpholin-4-yl, morpholin-4-yl-methyl, $C_{1-3}$-alkyl-carbonyl, $C_{1-4}$-alkoxy-carbonyl;

➢ **-N$R^{N1}$$R^{N2}$**, wherein **$R^{N1}$** represents hydrogen and **$R^{N2}$** represents -(C=O)**-$R^{CO}$**, wherein **$R^{CO}$** represents $C_{1-4}$-alkoxy; or

➢ **-$OR^6$**, wherein **$R^6$** represents $C_{3-5}$-cycloalkyl-$C_{1-3}$-alkyl, benzyl or $C_{1-3}$-alkoxy-carbonyl-$C_{1-3}$-alkyl;

• 9- to 10-membered bicyclic heteroaryl, wherein said 9- to 10-membered bicyclic heteroaryl independently is unsubstituted, or mono-, di- or tri-substituted, wherein the substituents are independently selected from:

➢ $C_{1-4}$-alkyl, cyano, halogen, $C_{1-3}$-fluoroalkyl, $C_{1-3}$-fluoroalkoxy, $C_{1-4}$-alkoxy, hydroxymethyl-cyclopropyl, morpholin-4-yl, morpholin-4-yl-methyl, $C_{1-3}$-alkyl-carbonyl, $C_{1-4}$-alkoxy-carbonyl;

➢ N**$R^{N1}$****$R^{N2}$**, wherein **$R^{N1}$** represents hydrogen and **$R^{N2}$** represents -(C=O)**-$R^{CO}$**, wherein **$R^{CO}$** represents $C_{1-4}$-alkoxy; or

➢ **-$OR^6$**, wherein **$R^6$** represents $C_{3-5}$-cycloalkyl-$C_{1-3}$-alkyl; or

• 2,3-dihydro-benzo[1,4]dioxin-6-yl, 1,3-dihydro-2H-benzoimidazol-2-one-5-yl, 3H-benzooxazol-2-one-6-yl, 3-methyl-1,1-dioxo-2,3-dihydro-1*H*-1l6-benzo[*d*]isothiazol-5-yl, 1-methyl-1H-pyrrolo[2,3-b]pyridin-5-yl or 3,4-dihydro-2H-pyrano[2,3-b]pyridine-6-yl;

or a pharmaceutically acceptable salt thereof.

**2.** A compound according to claim 1, wherein
**A** represents

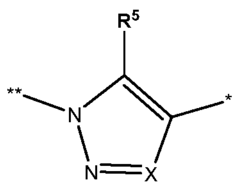
,

wherein one asterisk "*" denotes the point of attachment to the carbon bearing the OH group; two asterisks "**" denote the point of attachment to **$R^2$-**$(CH_2)_n$; **X** represents N or CH; and **$R^5$** represents hydrogen or $C_{1-4}$-alkyl, halogen or $C_{3-5}$-cycloalkyl;
or a pharmaceutically acceptable salt thereof.

**3.** A compound according to any one of claims 1 or 2, wherein
**$R^2$** represents

• phenyl or 6-membered heteroaryl, wherein said phenyl or 6-membered heteroaryl is independently unsubstituted, or mono-, di- or tri-substituted; and wherein the substituents are independently selected from:

➢ $C_{1-4}$-alkyl, cyano, nitro, halogen, hydroxy, hydroxy-$C_{1-4}$-alkyl, $C_{1-3}$-alkoxy-$C_{1-4}$-alkyl, $C_{1-3}$-fluoroalkyl, $C_{1-3}$-fluoroalkoxy, $C_{1-4}$-alkoxy, $C_{3-5}$-cycloalkoxy, hydroxymethyl-cyclopropyl, $C_{1-3}$-alkyl-carbonyl, $C_{1-4}$-alkoxy-carbonyl, amino, morpholin-4-yl or morpholin-4-yl-methyl;
➢ **-NR$^{N1}$R$^{N2}$**, wherein **R$^{N1}$** represents hydrogen and **R$^{N2}$** represents -(C=O)-**R$^{CO}$**, wherein **R$^{CO}$** represents $C_{1-4}$-alkyl, $C_{1-4}$-alkoxy, amino, phenyl or morpholin-4-yl;
➢ -(C=O)-N**R$^{N3}$R$^{N4}$**, wherein **R$^{N3}$** represents hydrogen or $C_{1-3}$-alkyl, and **R$^{N4}$** represents hydrogen, $C_{1-3}$-alkyl, $C_{3-5}$-cycloalkyl, hydroxy-$C_{2-4}$-alkyl, $C_{1-3}$-alkoxy-$C_{2-4}$-alkyl, cyano-$C_{1-3}$-alkyl, $C_{3-4}$-alkenyl or furanyl-$C_{1-3}$-alkyl or allyl; or wherein **R$^{N3}$** and **R$^{N4}$** together with the nitrogen atom to which they are attached form a morpholine ring; and
➢ **-OR$^6$**, wherein **R$^6$** represents $C_{1-3}$-alkoxy-$C_{2-4}$-alkyl, hydroxy-$C_{2-5}$-alkyl, di-hydroxy-$C_{2-5}$-alkyl, sulfamoyl-$C_{2-4}$-alkyl, pyridinyl-$C_{1-3}$-alkyl, $C_{1-3}$-alkylsulfonyl-$C_{2-4}$-alkyl, $C_{3-5}$-cycloalkyl-$C_{1-3}$-alkyl, oxetan-3-yl-$C_{1-3}$-alkyl, ($C_{1-3}$-alkyl-oxetan-3-yl)-$C_{1-3}$-alkyl, (fluoro-oxetan-3-yl)-$C_{1-3}$-alkyl, tetrahydrofuranyl-$C_{1-3}$-alkyl, hydroxy-$C_{3-5}$-cycloalkyl-$C_{1-3}$-alkyl, tetrahydropyranyl-$C_{1-3}$-alkyl, benzyl, (hydroxy-$C_{2-4}$-alkoxy)-$C_{2-4}$-alkyl, piperidin-1-yl-$C_{1-3}$-alkyl, (1-methyl-piperidin-3-yl)-$C_{1-3}$-alkyl, (1,1-dioxidotetrahydro-thiopyran-4-yl)-$C_{1-3}$-alkyl, $C_{1-3}$-alkoxy-carbonyl-$C_{1-3}$-alkyl, ((hydroxy-$C_{2-3}$-alkoxy)-$C_{2-3}$-alkoxy)-$C_{2-3}$-alkyl, $C_{1-3}$-fluoroalkoxy-$C_{2-3}$-alkyl or $C_{1-3}$-alkyl-carbonyl;

• 9- to 10-membered bicyclic heteroaryl, wherein said 9- to 10-membered bicyclic heteroaryl is unsubstituted; or
• 2,3-dihydro-benzo[1,4]dioxin-6-yl, 1,3-dihydro-2H-benzoimidazol-2-one-5-yl, 3H-benzooxazol-2-one-6-yl, 3-methyl-1,1-dioxo-2,3-dihydro-1*H*-1l6-benzo[*d*]isothiazol-5-yl, 1-methyl-1H-pyrrolo[2,3-b]pyridin-5-yl or 3,4-dihydro-2H-pyrano[2,3-b]pyridine-6-yl;

or a pharmaceutically acceptable salt thereof.

**4.** A compound according to to any one of claims 1 or 2, wherein the fragment **R$^2$-**$(CH_2)_n$— is selected from group I, II, III, IV, V, VI, VII, VIII, IX, X, XI or XII:

I. phenyl, 4-hydroxy-phenyl, 4-chloro-phenyl, 2-methyl-phenyl, 3-methyl-phenyl, 4-methyl-phenyl, 2-methoxy-phenyl, 3-methoxy-phenyl, 4-methoxy-phenyl, 4-ethoxy-phenyl, 4-(2-methoxy-ethoxy)-phenyl, 4-benzyloxy-phenyl, 3-cyano-phenyl, 4-cyano-phenyl, 2-trifluoromethyl-phenyl, 3-trifluoromethyl-phenyl, 4-trifluoromethyl-phenyl, 4-isopropoxy-phenyl, 4-(cyclopropyl-methoxy)-phenyl, 4-(1-hydroxymethyl-cyclopropyl)-phenyl, 4-trifluoromethoxy-phenyl, 4-(morpholin-4-yl)-phenyl, 4-(morpholin-4-yl-methyl)-phenyl or 4-(methoxy-carbamoyl)-phenyl;
II. 4-(2-hydroxy-2-methyl-propoxy)-phenyl, 4-(3-hydroxy-propoxy)-phenyl, 3-cyano-4-methoxy-phenyl, 4-(2-hydroxy-ethoxy)-phenyl, 3-chloro-4-hydroxy-phenyl, 3-fluoro-4-hydroxy-phenyl, 3,5-difluoro-4-hydroxy-phenyl, 4-((methoxy-carbonyl)amino)-phenyl, 4-(morpholine-4-carbonyl)-phenyl, 4-((2-cyano-ethyl)-carbamoyl)-phenyl, 4-(morpholine-4-yl-carboxamido)-phenyl, 4-(*tert*-butyl-carboxamido)-phenyl, 2-fluoro-4-(*tert*-butoxyl-carboxamido)-phenyl, 4-(3-ethylureido)-3-methoxy-phenyl, 4-(methoxy-carboxamido)-phenyl, 4-(3-ethyl-ureido)-3-methoxy-phenyl, 4-(3-(3-methoxy-propyl)-ureido)-phenyl, 4-(3-butyl-ureido)-phenyl, 4-isobutyramido-phenyl, 4-acetyl-phenyl, 4-(tetrahydropyran-4-yl-methoxy)-phenyl, 4-((3-fluoro-oxetan-3-yl)-methoxy)-phenyl, 4-((3-methyl-oxetan-3-yl)-methoxy)-phenyl, 4-(oxetan-3-yl-methoxy)-phenyl, 4-(2-(1-hydroxy-cyclopropyl)-ethoxy)-phenyl, 4-(3-hydroxy-2,2-dimethyl-propyl)-phenyl, 4-(3-hydroxy-3-methyl-butyl)-phenyl, 4-(2-(2-hydroxy-ethoxy)-ethoxy)-phenyl, 4-(2-(piperidin-1-yl)-ethoxy)-phenyl, 4-(tetrahydrofuran-2-yl-methoxy)-phenyl, 4-(tetrahydropyran-4-yl-methoxy)-phenyl, 4-(tetrahydropyran-2-yl-methoxy)-phenyl, 4-((2-methoxy-ethyl)-carbamoyl)-phenyl, 4-(cyclopropyl-carbamoyl)-phenyl, 4-(ethyl-carbamoyl)-phenyl, 4-(3-methoxypropyl-carbamoyl)-phenyl, 4-ureido-phenyl, 4-acetamido-phenyl, 4-(methyl-carbamoyl)-phenyl, 4-((1-methyl-piperidin-3-yl)-methoxy)-phenyl 4-((2-hydroxy-ethyl)-carbamoyl)-phenyl, 4-(ethyl(methyl)carbamoyl)-phenyl, 4-((3-hydroxy-propyl)-carbamoyl)-phenyl, 4-carbamoyl-phenyl, 4-((furan-2-yl-methyl)-carbamoyl)-phenyl, 4-(allyl-carbamoyl)-phenyl, 4-(dimethyl-carbamoyl)-phenyl, 4-((2-(dimethylamino)-ethyl)-carbamoyl)-phenyl, 4-((1,1-dioxo-hexahydrothiopyran-4-yl)-methyl)-phenyl;
III. 3-chloro-4-(3-hydroxy-propoxy)-phenyl, 3-chloro-4-(2-methyl-2-hydroxy-propoxy)-phenyl, 3-chloro-4-(3-methyl-3-hydroxy-butoxy)-phenyl, 4-(2-methyl-2-hydroxy-propoxy)-phenyl, 4-(2,3-dihydroxy-propoxy)-phenyl, 3-cyano-5-methoxy-phenyl, 3-hydroxy-4-cyano-phenyl, 3-nitro-4-hydroxy-phenyl, 2-chloro-4-hydroxy-phenyl, 2-fluoro-4-hydroxy-phenyl, 3-hydorxy-4-chloro-phenyl, 2-hydorxy-4-chlorophenyl, 3-hydroxy-phenyl, 2,5-difluoro-4-hydoxy-phenyl, 2,5-difluoro-4-hydoxy-phenyl, 3-trifluoromethoxy-4-hydroxy-phenyl, 3-cyano-4-trifluoromethoxy-phenyl, 2-trifluoromethoxy-4-hydroxy-phenyl, 3-methyl-5-hydroxy-phenyl, 2-methyl-4-hydroxy-phenyl, 3-hydroxy-4-methyl-phenyl, 3-methyl-4-hydroxy-phenyl, 3-ethyl-4-hydroxy-phenyl, 4-(hydroxy-methyl)-phenyl, (4-hydroxy-phenyl)-methyl, (2-hydroxy-phenyl)-methyl, 3-chloro-4-amino-phenyl, 4-(amino-carbonyl)-phenyl,

4-(methyl-aminocarbonyl)-phenyl, (4-(methoxy-carbonyl)-phenyl)-methyl, (3-(methoxy-carbonyl)-phenyl)-methyl, 4-(methyl-carboxamido)-phenyl, 4-(phenyl-carboxamido)-phenyl, 4-(iso-propyl-carboxamido)-phenyl, 3-chloro-4-(iso-propyl-carboxamido)-phenyl, 4-(tert-butoxy-carboxamido)-phenyl, 3-hydroxy-4-methoxy-phenyl, 3-chloro-4-methoxy-phenyl, 3-chloro-4-ethoxy-phenyl, 3-fluoro-4-methoxy-phenyl, 3-bromo-4-methoxy-phenyl, 3-iodo-4-methoxy-phenyl,3-chloro-4-methoxy-5-fluoro-phenyl, 3-chloro-4-methoxy-6-fluoro-phenyl, 3,6-difluoro-4-methoxy-phenyl, 2-fluoro-3-chloro-4-methoxy-phenyl, 2,6-dimethyl-4-methoxy-phenyl, 3-chloro-4-(cyclopropyl-methoxy)-pheny, 3-ethyl-4-hydroxy-phenyl, 4-(methyl-methoxy)-phenyl, 3-hydroxy-5-trifluoromethyl-phenyl, 2-trifluoromethyl-4-hydroxy-phenyl, 3,4-dimethoxy-5-cyano-phenyl, 4-((2-dimethylamino-ethoxy))-phenyl, 4-(2-(2-(2-hydroxyethoxy)-ethoxy)-ethoxy)-phenyl, 3-chloro-4-((3-fluoro-oxetan-3-yl)-methoxy)-phenyl, 4-(pyrrolidine-1-sulfonyl)-phenyl, 4-(pyridine-2-yl-methoxy)-phenyl, 4-(ethoxy-carbonyl-methoxy)-phenyl, 4-((2-hydroxy-ethyl)-carbamoyl)-phenyl, 4-((2-methoxy-ethyl)-carbamoyl)-phenyl, 4-((3-hydroxy-propyl)-carbamoyl)-phenyl, 4-(allyl-carbamoyl)-phenyl, 4-(allyl-carbamoyl)-phenyl, 4-(dimethyl-carbamoyl)-phenyl, 4-(N-methyl-N-ethyl-carbamoyl)-phenyl, 3-fluoro-4-benzyloxy-phenyl, 4-sulfamoyl-phenyl, 4-(methyl-sulfamoyl)-phenyl, 4-(ethyl-sulfamoyl)-phenyl, 4-(dimethyl-sulfamoyl)-phenyl, 4-(diethyl-sulfamoyl)-phenyl, 4-((methyl-sulfamoyl)-amino)-phenyl, 4-(2-methanesulfonyl-ethoxy)-phenyl, 4-(sulfamoyl-propoxy)-phenyl, 4-(2-(2,2,2-trifluoro-ethoxy)-ethoxy)-phenyl;

IV. 6-methoxy-pyridin-3-yl, 5-fluoro-6-methoxy-pyridin-3-yl, 6-methoxy-5-methyl-pyridin-3-yl, 2,6-dimethoxy-pyridin-3-yl, 5,6-dimethoxy-pyridin-3-yl, 5,6-dimethoxy-pyridin-2-yl, 5-chloro-6-ethoxy-pyridin-3-yl, 6-isopropoxy-pyridin-3-yl, 2-methoxy-3-(methoxy-carbonyl)-pyridin-5-yl, 5-chloro-6-isopropoxy-pyridin-3-yl, 6-ethoxy-5-trifluoromethyl-pyridin-3-yl, 6-(methoxy-carbonyl-methoxy)-pyridin-3-yl, 6-methoxy-5-(methoxycarbonyl)-pyridin-3-yl, 2-methoxy-pyrimidin-5-yl, 2-ethoxy-pyrimidin-5-yl, 2,4-dimethoxy-pyrimidin-5-yl, 2-benzyloxy-pyrimidin-5-yl, 2-cyclobutoxy-pyrimidin-5-yl, 3,6-dimethoxy-pyridazin-4-yl or 6-(2,2,2-trifluoro-ethoxy)-pyridin-3-yl;

V. pyridin-3-yl, 4-methoxy-pyridin-3-yl or 3-chloro-4-bromo-pyridin-2-yl;

VI. pyrazol-4-yl, 1-methyl-pyrazol-4-yl, pyrimidin-5-yl, pyridin-2-yl, pyridin-3-yl, pyridin-4-yl or 6-ethoxy-pyridin-3-yl;

VII. indol-4-yl, indol-5-yl, indol-6-yl, indol-7-yl, quinolin-4-yl, quinolin-5-yl, isoquinolin-4-yl, isoquinolin-5-yl or isoquinolin-8-yl;

VIII. 1H-indazol-5-yl, 3-chloro-1H-indazol-6-yl or 1-methyl-1H-indazol-7-yl;

IX. benzyl;

X. 2,3-dihydro-[1,4]benzodioxan-6-yl;

XI. 1,3-dihydro-2H-benzoimidazol-2-one-5-yl or 3,4-dihydro-2H-pyrano[2,3-b]pyridine-6-y; and

XII. 3H-benzooxazol-2-one-6-yl, 3-methyl-1,1-dioxo-2,3-dihydro-1*H*-1l6-benzo[*d*]isothiazol-5-yl, 1-methyl-1H-pyrrolo[2,3-b]pyridin-5-yl, 2,3-dihydro-benzo[1,4]dioxin-6-yl;

or a pharmaceutically acceptable salt thereof.

**5.** A compound according to any one of claims 1 to 3, wherein **n** represents 0; or a pharmaceutically acceptable salt thereof

**6.** A compound according to any one of claims 1 to 5, wherein
**$R^1$** represents:

- $C_{1-4}$-alkyl;
- vinyl, 1-methyl-vinyl or 1-propenyl;
- $C_{1-3}$-fluoroalkyl;
- $C_{3-6}$-cycloalkyl which is unsubstituted, mono- or di-substituted, wherein the substituents are independently selected from $C_{1-3}$-alkyl or fluorine; or
- phenyl, which is unsubstituted or mono, di- or tri-substituted, wherein the substituents are independently selected from $C_{1-3}$-alkyl and halogen;

or a pharmaceutically acceptable salt thereof.

**7.** A compound according to claim 1, wherein

**A** represents

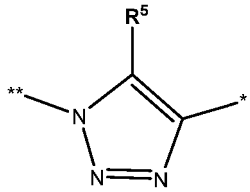

,

wherein one asterisk "*" denotes the point of attachment to the carbon bearing the OH group; two asterisks "**" denote the point of attachment to $\mathbf{R^2}$-$(CH_2)_n$; and $\mathbf{R^5}$ represents hydrogen or $C_{1-3}$-alkyl;
**n** represents 0;
$\mathbf{R^1}$ represents

- $C_{1-3}$-alkyl;
- -C($\mathbf{R^A}$)=C($\mathbf{R^B}$)($\mathbf{R^C}$), wherein $\mathbf{R^A}$, $\mathbf{R^B}$ and $\mathbf{R^C}$ are independently selected from hydrogen and methyl;
- $C_{1-3}$-fluoroalkyl;
- phenyl, which is unsubstituted or mono-substituted with halogene; or
- cyclopropyl, which is unsubstituted or mono-substituted with methyl or halogene;

$\mathbf{R^2}$ represents

- phenyl which is unsubstituted, mono-, di-, or tri-substituted, wherein one substituent independently represents

  ➢ -O$\mathbf{R^6}$, wherein $\mathbf{R^6}$ represents hydrogen, $C_{1-3}$-alkyl, $C_{1-3}$-alkoxy-$C_{2-4}$-alkyl, hydroxy-$C_{2-5}$-alkyl, $C_{3-5}$-cycloalkyl-$C_{1-3}$-alkyl, oxetan-3-yl-$C_{1-3}$-alkyl, (fluoro-oxetan-3-yl)-$C_{1-3}$-alkyl, hydroxy-$C_{3-5}$-cycloalkyl-$C_{1-3}$-alkyl, tetrahydropyranyl-$C_{1-3}$-alkyl, $C_{1-3}$-fluoroalkoxy-$C_{2-3}$-alkyl or benzyl; or
  ➢ -N$\mathbf{R^{N1}}$$\mathbf{R^{N2}}$, wherein $\mathbf{R^{N1}}$ represents hydrogen; $\mathbf{R^{N2}}$ represents hydrogen or -(C=O)-$\mathbf{R^{CO}}$, wherein $\mathbf{R^{CO}}$ represents $C_{1-4}$-alkyl, $C_{1-4}$-alkoxy or phenyl;

  said one substituent being attached to said phenyl in para-position with regard to the point of attachment to the rest of the molecule and wherein any further substituent(s) of said phenyl ring, if present, independently represent halogen, cyano or nitro;

or a pharmaceutically acceptable salt thereof.

**8.** A compound according to any one of claims 1 to 7, wherein the asymmetric carbon atom to which $\mathbf{R^2}$-$(CH_2)$n-**A-** is attached has the absolute configuration depicted in Formula (II)

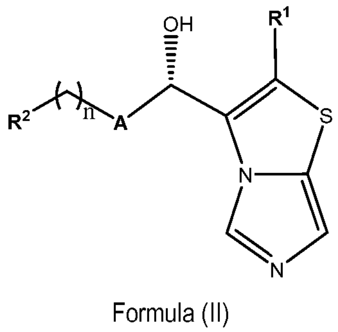

Formula (II)

or a pharmaceutically acceptable salt thereof.

**9.** A compound according to claim 1 selected from a group consisting of

(2-methyl-imidazo[5,1-b]thiazol-3-yl)-(1-phenyl-1H-[1,2,3]triazol-4-yl)-methanol;
(2-ethyl-imidazo[5,1-b]thiazol-3-yl)-(1-phenyl-1H-[1,2,3]triazol-4-yl)-methanol;
(2-cyclopropyl-imidazo[5,1-b]thiazol-3-yl)-(1-phenyl-1H-[1,2,3]triazol-4-yl)-methanol;

(R)-(2-cyclopropyl-imidazo[5,1-b]thiazol-3-yl)-(1-phenyl-1H-[1,2,3]triazol-4-yl)-methanol;
(2-cyclopentyl-imidazo[5,1-b]thiazol-3-yl)-(1-phenyl-1H-[1,2,3]triazol-4-yl)-methanol;
(2-cyclopropyl-imidazo[5,1-b]thiazol-3-yl)-(1-o-tolyl-1H-[1,2,3]triazol-4-yl)-methanol;
(2-cyclopropyl-imidazo[5,1-b]thiazol-3-yl)-(1-m-tolyl-1H-[1,2,3]triazol-4-yl)-methanol;
(2-cyclopropyl-imidazo[5,1-b]thiazol-3-yl)-(1-p-tolyl-1H-[1,2,3]triazol-4-yl)-methanol;
(2-cyclopropyl-imidazo[5,1-b]thiazol-3-yl)-[1-(3-methoxy-phenyl)-1H-[1,2,3]triazol-4-yl]-methanol;
(R)-(2-cyclopropyl-imidazo[5,1-b]thiazol-3-yl)-[1-(4-methoxy-phenyl)-1H-[1,2,3]triazol-4-yl]-methanol;
(2-cyclopropyl-imidazo[5,1-b]thiazol-3-yl)-[1-(4-methoxy-phenyl)-1H-[1,2,3]triazol-4-yl]-methanol;
(2-cyclopropyl-imidazo[5,1-b]thiazol-3-yl)-[1-(2-methoxy-phenyl)-1H-[1,2,3]triazol-4-yl]-methanol;
(1-benzyl-1H-[1,2,3]triazol-4-yl)-(2-cyclopropyl-imidazo[5,1-b]thiazol-3-yl)-methanol;
3-{4-[(2-cyclopropyl-imidazo[5,1-b]thiazol-3-yl)-hydroxy-methyl]-[1,2,3]triazol-1-yl}-benzonitrile;
4-{4-[(2-cyclopropyl-imidazo[5,1-b]thiazol-3-yl)-hydroxy-methyl]-[1,2,3]triazol-1-yl}-benzonitrile;
(2-cyclopropyl-imidazo[5,1-b]thiazol-3-yl)-[1-(2-trifluoromethyl-phenyl)-1H-[1,2,3]triazol-4-yl]-methanol;
(2-cyclopropyl-imidazo[5,1-b]thiazol-3-yl)-[1-(3-trifluoromethyl-phenyl)-1H-[1,2,3]triazol-4-yl]-methanol;
(2-cyclopropyl-imidazo[5,1-b]thiazol-3-yl)-[1-(4-trifluoromethyl-phenyl)-1H-[1,2,3]triazol-4-yl]-methanol;
1-(4-{4-[(2-cyclopropyl-imidazo[5,1-b]thiazol-3-yl)-hydroxy-methyl]-[1,2,3]triazol-1-yl}-phenyl)-ethanone;
(2-cyclopropyl-imidazo[5,1-b]thiazol-3-yl)-[1-(4-cyclopropylmethoxy-phenyl)-1H-[1,2,3]triazol-4-yl]-methanol;
(2-cyclopropyl-imidazo[5,1-b]thiazol-3-yl)-[1-(4-ethoxy-phenyl)-1H-[1,2,3]triazol-4-yl]-methanol;
(4-{4-[(2-cyclopropyl-imidazo[5,1-b]thiazol-3-yl)-hydroxy-methyl]-[1,2,3]triazol-1-yl}-phenyl)-carbamic acid methyl ester;
(2-cyclopropyl-imidazo[5,1-b]thiazol-3-yl)-[1-(4-isopropoxy-phenyl)-1H-[1,2,3]triazol-4-yl]-methanol;
3-{4-[(R)-(2-cyclopropyl-imidazo[5,1-b]thiazol-3-yl)-hydroxy-methyl]-[1,2,3]triazol-1-yl}-benzonitrile;
[1-(4-methoxy-phenyl)-1H-[1,2,3]triazol-4-yl]-[2-(1-methyl-cyclopropyl)-imidazo[5,1-b]thiazol-3-yl]-methanol;
[1-(4-chloro-phenyl)-1H-[1,2,3]triazol-4-yl]-(2-cyclopropyl-imidazo[5,1-b]thiazol-3-yl)-methanol;
(2-cyclopropyl-imidazo[5,1-b]thiazol-3-yl)-{1-[4-(1-hydroxymethyl-cyclopropyl)-phenyl]-1H-[1,2,3]triazol-4-yl}-methanol;
(2-cyclopropyl-imidazo[5,1-b]thiazol-3-yl)-[1-(4-morpholin4-yl-phenyl)-1H-[1,2,3]triazol-4-yl]-methanol;
(2-cyclopropyl-imidazo[5,1-b]thiazol-3-yl)-[1-(4-morpholin-4-ylmethyl-phenyl)-1H-[1,2,3]triazol-4-yl]-methanol;
(2-cyclopropyl-imidazo[5,1-b]thiazol-3-yl)-[1-(4-trifluoromethoxy-phenyl)-1H-[1,2,3]triazol-4-yl]-methanol;
(2-cyclopropyl-imidazo[5,1-b]thiazol-3-yl)-(1-pyrimidin-5-yl-1H-[1,2,3]triazol-4-yl)-methanol;
(2-cyclopropyl-imidazo[5,1-b]thiazol-3-yl)-(1-pyridin-2-yl-1H-[1,2,3]triazol-4-yl)-methanol;
(2-cyclopropyl-imidazo[5,1-b]thiazol-3-yl)-(1-pyridin-3-yl-1H-[1,2,3]triazol-4-yl)-methanol;
(2-cyclopropyl-imidazo[5,1-b]thiazol-3-yl)-(1-pyridin4-yl-1H-[1,2,3]triazol-4-yl)-methanol;
(2-cyclopropyl-imidazo[5,1-b]thiazol-3-yl)-[1-(6-ethoxy-pyridin-3-yl)-1H-[1,2,3]triazol-4-yl]-methanol;
(2-cyclopropyl-imidazo[5,1-b]thiazol-3-yl)-[1-(1-methyl-1H-pyrazol-4-yl)-1H-[1,2,3]triazol-4-yl]-methanol;
(2-cyclopropyl-imidazo[5,1-b]thiazol-3-yl)-[1-(1H-pyrazol-4-yl)-1H-[1,2,3]triazol-4-yl]-methanol;
(2-cyclopropyl-imidazo[5,1-b]thiazol-3-yl)-[1-(1H-indol-6-yl)-1H-[1,2,3]triazol-4-yl]-methanol;
(2-cyclopropyl-imidazo[5,1-b]thiazol-3-yl)-[1-(1H-indol-4-yl)-1H-[1,2,3]triazol-4-yl]-methanol;
(R)-(2-cyclopropyl-imidazo[5,1-b]thiazol-3-yl)-[1-(1H-indol-5-yl)-1H-[1,2,3]triazol-4-yl]-methanol;
(2-cyclopropyl-imidazo[5,1-b]thiazol-3-yl)-[1-(1H-indol-5-yl)-1H-[1,2,3]triazol-4-yl]-methanol;
(2-cyclopropyl-imidazo[5,1-b]thiazol-3-yl)-[1-(1H-indol-7-yl)-1H-[1,2,3]triazol-4-yl]-methanol;
(2-cyclopropyl-imidazo[5,1-b]thiazol-3-yl)-(1-isoquinolin-5-yl-1H-[1,2,3]triazol-4-yl)-methanol;
(2-cyclopropyl-imidazo[5,1-b]thiazol-3-yl)-(1-quinolin-5-yl-1H-[1,2,3]triazol-4-yl)-methanol;
(2-cyclopropyl-imidazo[5,1-b]thiazol-3-yl)-(1-isoquinolin-8-yl-1H-[1,2,3]triazol-4-yl)-methanol;
(2-cyclopropyl-imidazo[5,1-b]thiazol-3-yl)-(1-quinolin-4-yl-1H-[1,2,3]triazol-4-yl)-methanol;
(2-cyclopropyl-imidazo[5,1-b]thiazol-3-yl)-(1-isoquinolin-4-yl-1H-[1,2,3]triazol-4-yl)-methanol; and
(2-cyclopropyl-imidazo[5,1-b]thiazol-3-yl)-[1-(2,3-dihydro-benzo[1,4]dioxin-6-yl)-1H-[1,2,3]triazol-4-yl]-methanol;

or a pharmaceutically acceptable salt thereof.

**10.** A compound according to claim 1 selected from a group consisting of

[1-(4-benzyloxy-phenyl)-1*H*-[1,2,3]triazol-4-yl]-(2-cyclopropyl-imidazo[5,1-*b*]thiazol-3-yl)-methanol;
[1-(6-ethoxy-pyridin-3-yl)-1*H*-[1,2,3]triazol-4-yl]-(2-ethyl-imidazo[5,1-*b*]thiazol-3-yl)-methanol;
(2-ethyl-imidazo[5,1-b]thiazol-3-yl)-[1-(4-morpholin-4-yl-phenyl)-1H-[1,2,3]triazol-4-yl]-methanol;
(2-ethyl-imidazo[5,1-*b*]thiazol-3-yl)-{1-[4-(1-hydroxymethyl-cyclopropyl)-phenyl]-1*H*-[1,2,3]triazol-4-yl}-methanol;

(4-{4-[(2-ethyl-imidazo[5,1-*b*]thiazol-3-yl)-hydroxy-methyl]-[1,2,3]triazol-1-yl}-phenyl)-carbamic acid methyl ester;
(2-ethyl-imidazo[5,1-*b*]thiazol-3-yl)-[1-(1*H*-indol-5-yl)-1*H*-[1,2,3]triazol-4-yl]-methanol;
3-{4-[(2-ethyl-imidazo[5,1-b]thiazol-3-yl)-hydroxy-methyl]-[1,2,3]triazol-1-yl}-benzonitrile;
(2-ethyl-imidazo[5,1-b]thiazol-3-yl)-(1-isoquinolin-4-yl-1H-[1,2,3]triazol-4-yl)-methanol;
(1-benzyl-1*H*-[1,2,3]triazol-4-yl)-(2-ethyl-imidazo[5,1-*b*]thiazol-3-yl)-methanol;
[1-(4-cyclopropylmethoxy-phenyl)-1*H*-[1,2,3]triazol-4-yl]-(2-ethyl-imidazo[5,1-*b*]thiazol-3-yl)-methanol;
[1-(6-ethoxy-pyridin-3-yl)-1*H*-[1,2,3]triazol-4-yl]-(2-methyl-imidazo[5,1-*b*]thiazol-3-yl)-methanol;
(2-methyl-imidazo[5,1-*b*]thiazol-3-yl)-[1-(4-morpholin-4-yl-phenyl)-1*H*-[1,2,3]triazol-4-yl]-methanol;
{1-[4-(1-hydroxymethyl-cyclopropyl)-phenyl]-1*H*-[1,2,3]triazol-4-yl}-(2-methyl-imidazo[5,1-*b*]thiazol-3-yl)-methanol;
(4-{4-[hydroxy-(2-methyl-imidazo[5,1-b]thiazol-3-yl)-methyl]-[1,2,3]triazol-1-yl}-phenyl)-carbamic acid methyl ester;
[1-(1*H*-Indol-5-yl)-1*H*-[1,2,3]triazol-4-yl]-(2-methyl-imidazo[5,1-b]thiazol-3-yl)-methanol;
3-{4-[hydroxy-(2-methyl-imidazo[5,1-*b*]thiazol-3-yl)-methyl]-[1,2,3]triazol-1-yl}-benzonitrile;
(1-isoquinolin-4-yl-1*H*-[1,2,3]triazol-4-yl)-(2-methyl-imidazo[5,1-*b*]thiazol-3-yl)-methanol;
(1-benzyl-1*H*-[1,2,3]triazol-4-yl)-(2-methyl-imidazo[5,1-*b*]thiazol-3-yl)-methanol;
[1-(4-cyclopropylmethoxy-phenyl)-1*H*-[1,2,3]triazol-4-yl]-(2-methyl-imidazo[5,1-*b*]thiazol-3-yl)-methanol;
[2-(3-fluoro-phenyl)-imidazo[5,1-*b*]thiazol-3-yl]-[1-(4-methoxy-phenyl)-1*H*-[1,2,3]triazol-4-yl]-methanol;
(2-cyclopropyl-imidazo[5,1-*b*]thiazol-3-yl)-[1-(2-ethoxy-pyrimidin-5-yl)-1*H*-[1,2,3]triazol-4-yl]-methanol;
(2-cyclopropyl-imidazo[5,1-*b*]thiazol-3-yl)-[1-(5,6-dimethoxy-pyridin-2-yl)-1*H*-[1,2,3]triazol-4-yl]-methanol;
(2-cyclopropyl-imidazo[5,1-*b*]thiazol-3-yl)-[1-(2-methoxy-pyrimidin-5-yl)-1*H*-[1,2,3]triazol-4-yl]-methanol;
4-{4-[(2-cyclopropyl-imidazo[5,1-*b*]thiazol-3-yl)-hydroxy-methyl]-[1,2,3]triazol-1-yl}-phenol;
(2-cyclopropyl-imidazo[5,1-*b*]thiazol-3-yl)-[1-(6-methoxy-pyridin-3-yl)-1*H*-[1,2,3]triazol-4-yl]-methanol;
5-{4-[(2-cyclopropyl-imidazo[5,1-b]thiazol-3-yl)-hydroxy-methyl]-[1,2,3]triazol-1-yl}-2-methoxy-nicotinic acid methyl ester;
(2-cyclopropyl-imidazo[5,1-*b*]thiazol-3-yl)-[1-(6-methoxy-5-methyl-pyridin-3-yl)-1*H*-[1,2,3]triazol-4-yl]-methanol;
(2-cyclopropyl-imidazo[5,1-*b*]thiazol-3-yl)-[1-(3,4-dihydro-2*H*-pyrano[2,3-b]pyridin-6-yl)-1*H*-[1,2,3]triazol-4-yl]-methanol;
[1-(5-chloro-6-isopropoxy-pyridin-3-yl)-1*H*-[1,2,3]triazol-4-yl]-(2-cyclopropyl-imidazo[5,1-*b*]thiazol-3-yl)-methanol;
[1-(5-chloro-6-ethoxy-pyridin-3-yl)-1H-[1,2,3]triazol-4-yl]-(2-cyclopropyl-imidazo[5,1-*b*]thiazol-3-yl)-methanol;
(5-{4-[(2-cyclopropyl-imidazo[5,1-b]thiazol-3-yl)-hydroxy-methyl]-[1,2,3]triazol-1-yl}-pyridin-2-yloxy)-acetic acid methyl ester;
(2-cyclopropyl-imidazo[5,1-*b*]thiazol-3-yl)-[1-(6-ethoxy-5-trifluoromethyl-pyridin-3-yl)-1*H*-[1,2,3]triazol-4-yl]-methanol;
[1-(6-benzyloxy-pyridin-3-yl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-{1-[6-(2,2,2-trifluoro-ethoxy)-pyridin-3-yl]-1*H*-[1,2,3]triazol-4-yl}-methanol;
(2-cyclopropyl-imidazo[5,1-*b*]thiazol-3-yl)-[1-(5-fluoro-6-methoxy-pyridin-3-yl)-1*H*-[1,2,3]triazol-4-yl]-methanol;
(2-cyclopropyl-imidazo[5,1-*b*]thiazol-3-yl)-[1-(5,6-dimethoxy-pyridin-3-yl)-1*H*-[1,2,3]triazol-4-yl]-methanol;
(2-cyclopropyl-imidazo[5,1-*b*]thiazol-3-yl)-[1-(2,6-dimethoxy-pyridin-3-yl)-1*H*-[1,2,3]triazol-4-yl]-methanol;
(2-cyclopropyl-imidazo[5,1-*b*]thiazol-3-yl)-[1-(6-isopropoxy-pyridin-3-yl)-1*H*-[1,2,3]triazol-4-yl]-methanol;
[1-(2-cyclobutoxy-pyrimidin-5-yl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[1-(2,4-dimethoxy-pyrimidin-5-yl)-1*H*-[1,2,3]triazol4-yl]-methanol;
[1-(2-benzyloxy-pyrimidin-5-yl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[1-(3,6-dimethoxy-pyridazin-4-yl)-1*H*-[1,2,3]triazol-4-yl]-methanol;
(2-cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol;
(2-cyclopropyl-imidazo[5,1-*b*]thiazol-3-yl)-{1-[4-(oxetan-3-ylmethoxy)-phenyl]-1*H*-[1,2,3]triazol-4-yl}-methanol;
(2-cyclopropyl-imidazo[5,1-*b*]thiazol-3-yl)-{1-[4-(2-piperidin-1-yl-ethoxy)-phenyl]-1*H*-[1,2,3]triazol-4-yl}-methanol;
(2-cyclopropyl-imidazo[5,1-*b*]thiazol-3-yl)-{1-[4-(2-methoxy-ethoxy)-phenyl]-1*H*-[1,2,3]triazol-4-yl}-methanol;
[2-(1-fluoro-cyclopropyl)-imidazo[5,1-*b*]thiazol-3-yl]-[1-(4-methoxy-phenyl)-1*H*-[1,2,3]triazol-4-yl]-methanol;
*N*-(4-{4-[(2-cyclopropyl-imidazo[5,1-*b*]thiazol-3-yl)-hydroxy-methyl]-[1,2,3]triazol-1-yl}-phenyl)-acetamide;
(2-methyl-imidazo[5,1-*b*]thiazol-3-yl)-(1-phenyl-1*H*-pyrazol-4-yl)-methanol;
[1-(4-methoxy-phenyl)-1*H*-pyrazol-4-yl]-(2-methyl-imidazo[5,1-*b*]thiazol-3-yl)-methanol;

(2-methyl-imidazo[5,1-*b*]thiazol-3-yl)-(5-methyl-1-phenyl-1*H*-pyrazol-4-yl)-methanol;
(2-Cyclopropyl-imidazo[5,1-b]thiazol-3-yl)-[1-(4-methoxy-phenyl)-1*H*-pyrazol-4-yl]-methanol;
(2-cyclopropyl-imidazo[5,1-*b*]thiazol-3-yl)-(1-phenyl-1*H*-pyrazol-4-yl)-methanol;
(*S*)-(2-cyclopropyl-imidazo[5,1-*b*]thiazol-3-yl)-(1-phenyl-1*H*-pyrazol-4-yl)-methanol;
(2-Cyclopropyl-imidazo[5,1-*b*]thiazol-3-yl)-(5-methyl-1-phenyl-1*H*-[1,2,3]triazol-4-yl)-methanol;
4-{4-[(2-cyclopropyl-imidazo[5,1-*b*]thiazol-3-yl)-hydroxy-methyl]-[1,2,3]triazol-1-yl}-*N*-(2-methoxy-ethyl)-benzamide;
(4-{4-[(2-cyclopropyl-imidazo[5,1-*b*]thiazol-3-yl)-hydroxy-methyl]-[1,2,3]triazol-1-yl}-phenyl)-morpholin4-yl-methanone;
morpholine-4-carboxylic acid (4-{4-[(2-cyclopropyl-imidazo[5,1-b]thiazol-3-yl)-hydroxy-methyl]-[1,2,3]triazol-1-yl}-phenyl)-amide;
*N*-cyclopropyl-4-{4-[(2-cyclopropyl-imidazo[5,1-*b*]thiazol-3-yl)-hydroxy-methyl]-[1,2,3]triazol-1-yl}-benzamide;
4-{4-[(2-cyclopropyl-imidazo[5,1-*b*]thiazol-3-yl)-hydroxy-methyl]-[1,2,3]triazol-1-yl}-N-(3-methoxy-propyl)-benzamide;
4-{4-[(2-cyclopropyl-imidazo[5,1-*b*]thiazol-3-yl)-hydroxy-methyl]-[1,2,3]triazol-1-yl}-*N*-ethyl-benzamide;
*N*-(2-cyano-ethyl)-4-{4-[(2-cyclopropyl-imidazo[5,1-*b*]thiazol-3-yl)-hydroxy-methyl]-[1,2,3]triazol-1-yl}-benzamide;
(4-{4-[(2-cyclopropyl-imidazo[5,1-*b*]thiazol-3-yl)-hydroxy-methyl]-[1,2,3]triazol-1-yl}-phenyl)-urea;
*N*-(4-{4-[(2-ethyl-imidazo[5,1-*b*]thiazol-3-yl)-hydroxy-methyl]-[1,2,3]triazol-1-yl}-phenyl)-acetamide;
4-{4-[(2-ethyl-imidazo[5,1-*b*]thiazol-3-yl)-hydroxy-methyl]-[1,2,3]triazol-1-yl}-*N*-methyl-benzamide;
5-{4-[(2-ethyl-imidazo[5,1-*b*]thiazol-3-yl)-hydroxy-methyl]-[1,2,3]triazol-1-yl}-1,3-dihydro-benzoimidazol-2-one;
1-ethyl-3-(4-{4-[(2-ethyl-imidazo[5,1-*b*]thiazol-3-yl)-hydroxy-methyl]-[1,2,3]triazol-1-yl}-2-methoxy-phenyl)-urea;
4-{4-[(2-ethyl-imidazo[5,1-*b*]thiazol-3-yl)-hydroxy-methyl]-[1,2,3]triazol-1-yl}-*N*,*N*-dimethyl-benzamide;
4-{4-[(2-ethyl-imidazo[5,1-*b*]thiazol-3-yl)-hydroxy-methyl]-[1,2,3]triazol-1-yl}-*N*-(3-hydroxy-propyl)-benzamide;
4-{4-[(2-ethyl-imidazo[5,1-*b*]thiazol-3-yl)-hydroxy-methyl]-[1,2,3]triazol-1-yl}-benzamide;
(4-{4-[(2-ethyl-imidazo[5,1-*b*]thiazol-3-yl)-hydroxy-methyl]-[1,2,3]triazol-1-yl}-phenyl)-morpholin-4-yl-methanone;
4-{4-[(2-ethyl-imidazo[5,1-*b*]thiazol-3-yl)-hydroxy-methyl]-[1,2,3]triazol-1-yl}-*N*-(2-hydroxy-ethyl)-benzamide;
*N*-(2-dimethylamino-ethyl)-4-{4-[(2-ethyl-imidazo[5,-*b*]thiazol-3-yl)-hydroxy-methyl]-[1,2,3]triazol-1-yl}-benzamide;
*N*-(4-{4-[(2-ethyl-imidazo[5,1-*b*]thiazol-3-yl)-hydroxy-methyl]-[1,2,3]triazol-1-yl}-phenyl)-2,2-dimethyl-propionamide;
morpholine-4-carboxylic acid (4-{4-[(2-ethyl-imidazo[5,1-*b*]thiazol-3-yl)-hydroxy-methyl]-[1,2,3]triazol-1-yl}-phenyl)-amide;
1-butyl-3-(4-{4-[(2-ethyl-imidazo[5,1-*b*]thiazol-3-yl)-hydroxy-methyl]-[1,2,3]triazol-1-yl}-phenyl)-urea;
4-{4-[(2-ethyl-imidazo[5,1-*b*]thiazol-3-yl)-hydroxy-methyl]-[1,2,3]triazol-1-yl}-*N*-furan-2-ylmethyl-benzamide;
*N*-ethyl-4-{4-[(2-ethyl-imidazo[5,1-*b*]thiazol-3-yl)-hydroxy-methyl]-[1,2,3]triazol-1-yl}-*N*-methyl-benzamide;
*N*-cyclopropyl-4-{4-[(2-ethyl-imidazo[5,1-*b*]thiazol-3-yl)-hydroxy-methyl]-[1,2,3]triazol-1-yl}-benzamide;
4-{4-[(2-ethyl-imidazo[5,1-*b*]thiazol-3-yl)-hydroxy-methyl]-[1,2,3]triazol-1-yl}-*N*-(3-methoxy-propyl)-benzamide;
*N*-(4-{4-[(2-ethyl-imidazo[5,1-*b*]thiazol-3-yl)-hydroxy-methyl]-[1,2,3]triazol-1-yl}-phenyl)-isobutyramide;
*N*-ethyl-4-{4-[(2-ethyl-imidazo[5,1-*b*]thiazol-3-yl)-hydroxy-methyl]-[1,2,3]triazol-1-yl}-benzamide;
*N*-allyl-4-{4-[(2-ethyl-imidazo[5,1-*b*]thiazol-3-yl)-hydroxy-methyl]-[1,2,3]triazol-1-yl}-benzamide;
*N*-(2-cyano-ethyl)-4-{4-[(2-ethyl-imidazo[5,1-*b*]thiazol-3-yl)-hydroxy-methyl]-[1,2,3]triazol-1-yl}-benzamide;
(4-{4-[(2-ethyl-imidazo[5,1-*b*]thiazol-3-yl)-hydroxy-methyl]-[1,2,3]triazol-1-yl}-phenyl)-urea;
(4-{4-[(2-cyclopropyl-imidazo[5,1-*b*]thiazol-3-yl)-hydroxy-methyl]-pyrazol-1-yl}-phenyl)-carbamic acid methyl ester;
(*R*)-(2-cyclopropyl-imidazo[5,1-*b*]thiazol-3-yl)-[1-(4-cyclopropylmethoxy-phenyl)-1*H*-[1,2,3]triazol-4-yl]-methanol;
(2-cyclopropyl-imidazo[5,1-*b*]thiazol-3-yl)-{1-[4-(tetrahydro-furan-2-ylmethoxy)-phenyl]-1*H*-[1,2,3]triazol-4-yl}-methanol;
(1-phenyl-1*H*-[1,2,3]triazol-4-yl)-(2-trifluoromethyl-imidazo[5,1-*b*]thiazol-3-yl)-methanol;
[1-(4-methoxy-phenyl)-1*H*-[1,2,3]triazol-4-yl]-(2-trifluoromethyl-imidazo[5,1-*b*]thiazol-3-yl)-methanol;
5-{4-[(2-cyclopropyl-imidazo[5,1-*b*]thiazol-3-yl)-hydroxy-methyl]-[1,2,3]triazol-1-yl}-2-methoxy-benzonitrile;
2-(4-{4-[(2-cyclopropyl-imidazo[5,1-b]thiazol-3-yl)-hydroxy-methyl]-[1,2,3]triazol-1-yl}-phenoxy)-ethanol;
3-(4-{4-[(2-cyclopropyl-imidazo[5,1-*b*]thiazol-3-yl)-hydroxy-methyl]-[1,2,3]triazol-1-yl}-phenoxy)-propan-1-ol;
3-(4-{4-[(*R*)-(2-cyclopropyl-imidazo[5,1-*b*]thiazol-3-yl)-hydroxy-methyl]-[1,2,3]triazol-1-yl}-phenoxy)-propan-1-ol;

2-[2-(4-{4-[(2-cyclopropyl-imidazo[5,1-*b*]thiazol-3-yl)-hydroxy-methyl]-[1,2,3]triazol-1-yl}-phenoxy)-ethoxy]-ethanol;
1-(4-{4-[(2-cyclopropyl-imidazo[5,1-*b*]thiazol-3-yl)-hydroxy-methyl]-[1,2,3]triazol-1-yl}-phenoxy)-2-methyl-propan-2-ol;
4-(4-{4-[(2-cyclopropyl-imidazo[5,1-b]thiazol-3-yl)-hydroxy-methyl]-[1,2,3]triazol-1-yl}-phenoxy)-2-methyl-butan-2-ol;
3-(4-{4-[(2-cyclopropyl-imidazo[5,1-b]thiazol-3-yl)-hydroxy-methyl]-[1,2,3]triazol-1-yl}-phenoxy)-2,2-dimethyl-propan-1-ol;
1-[2-(4-{4-[(2-cyclopropyl-imidazo[5,1-*b*]thiazol-3-yl)-hydroxy-methyl]-[1,2,3]triazol-1-yl}-phenoxy)-ethyl]-cyclopropanol;
1-[2-(4-{4-[(R)-(2-cyclopropyl-imidazo[5,1-b]thiazol-3-yl)-hydroxy-methyl]-[1,2,3]triazol-1-yl}-phenoxy)-ethyl]-cyclopropanol;
(2-cyclopropyl-imidazo[5,1-*b*]thiazol-3-yl)-{1-[4-(tetrahydro-pyran-2-ylmethoxy)-phenyl]-1*H*-[1,2,3]triazol-4-yl}-methanol;
(2-cyclopropyl-imidazo[5,1-*b*]thiazol-3-yl)-{1-[4-(3-methyl-oxetan-3-ylmethoxy)-phenyl]-1*H*-[1,2,3]triazol-4-yl}-methanol;
(2-cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol;
(*R*)-(2-cyclopropyl-imidazo[5,1-*b*]thiazol-3-yl)-{1-[4-(3-fluoro-oxetan-3-ylmethoxy)-phenyl]-1*H*-[1,2,3]triazol-4-yl}-methanol;
(2-cyclopropyl-imidazo[5,1-*b*]thiazol-3-yl)-{1-[4-(1-methyl-piperidin-3-ylmethoxy)-phenyl]-1*H*-[1,2,3]triazol-4-yl}-methanol;
(2-cyclopropyl-imidazo[5,1-*b*]thiazol-3-yl)-{1-[4-(1,1-dioxo-hexahydro-1l6-thiopyran-4-ylmethoxy)-phenyl]-1*H*-[1,2,3]triazol-4-yl}-methanol;
2-chloro-4-{4-[(2-cyclopropyl-imidazo[5,1-*b*]thiazol-3-yl)-hydroxy-methyl]-[1,2,3]triazol-1-yl}-phenol;
2-chloro-4-{4-[(*R*)-(2-cyclopropyl-imidazo[5,1-*b*]thiazol-3-yl)-hydroxy-methyl]-[1,2,3]triazol-1-yl}-phenol;
4-{4-[(2-cyclopropyl-imidazo[5,1-*b*]thiazol-3-yl)-hydroxy-methyl]-[1,2,3]triazol-1-yl}-2,6-difluoro-phenol;
4-{4-[(2-cyclopropyl-imidazo[5,1-*b*]thiazol-3-yl)-hydroxy-methyl]-[1,2,3]triazol-1-yl}-2-fluoro-phenol;
(4-{4-[(2-cyclopropyl-imidazo[5,1-b]thiazol-3-yl)-hydroxy-methyl]-[1,2,3]triazol-1-yl}-3-fluoro-phenyl)-carbamic acid tert-butyl ester;
biphenyl-4-yl-(2-cyclopropyl-imidazo[5,1-*b*]thiazol-3-yl)-methanol;
biphenyl-3-yl-(2-cyclopropyl-imidazo[5,1-*b*]thiazol-3-yl)-methanol;
(2-methyl-imidazo[5,1-*b*]thiazol-3-yl)-(5-phenyl-thiophen-2-yl)-methanol;
(2-methyl-imidazo[5,1-*b*]thiazol-3-yl)-(2-phenyl-thiazol-5-yl)-methanol;
(2-methyl-imidazo[5,1-*b*]thiazol-3-yl)-(3-phenyl-isoxazol-5-yl)-methanol;
(2-cyclopropyl-imidazo[5,1-b]thiazol-3-yl)-(5-phenyl-[1,3,4]oxadiazol-2-yl)-methanol;
(2-cyclopropyl-imidazo[5,1-*b*]thiazol-3-yl)-(2-phenyl-2*H*-[1,2,3]triazol-4-yl)-methanol;
3-{5-[(2-cyclopropyl-imidazo[5,1-*b*]thiazol-3-yl)-hydroxy-methyl]-thiophen-2-yl}-benzonitrile;
(2-cyclopropyl-imidazo[5,1-b]thiazol-3-yl)-(5-pyridin-3-yl-thiophen-2-yl)-methanol;
(2-cyclopropyl-imidazo[5,1-b]thiazol-3-yl)-[5-(4-methoxy-phenyl)-thiophen-2-yl]-methanol;
(2-cyclopropyl-imidazo[5,1-*b*]thiazol-3-yl)-[5-(4-trifluoromethyl-phenyl)-thiophen-2-yl]-methanol;
(2-cyclopropyl-imidazo[5,1-b]thiazol-3-yl)-(3-phenyl-[1,2,4]oxadiazol-5-yl)-methanol;
(2-cyclopropyl-imidazo[5,1-b]thiazol-3-yl)-(5-phenyl-thiophen-2-yl)-methanol;
(2-cyclopropyl-imidazo[5,1-*b*]thiazol-3-yl)-[5-(3-trifluoromethyl-phenyl)-thiophen-2-yl]-methanol;
(2-cyclopropyl-imidazo[5,1-b]thiazol-3-yl)-[5-(2-methoxy-phenyl)-thiophen-2-yl]-methanol;
(2-cyclopropyl-imidazo[5,1-b]thiazol-3-yl)-[5-(3-methoxy-phenyl)-thiophen-2-yl]-methanol;
(2-cyclopropyl-imidazo[5,1-b]thiazol-3-yl)-(5-pyridin-4-yl-thiophen-2-yl)-methanol;
(2-cyclopropyl-imidazo[5,1-*b*]thiazol-3-yl)-[5-(2-methyl-2*H*-pyrazol-3-yl)-thiophen-2-yl]-methanol;
(2-cyclopropyl-imidazo[5,1-*b*]thiazol-3-yl)-[5-(2-trifluoromethyl-phenyl)-thiophen-2-yl]-methanol;
(2-cyclopropyl-imidazo[5,1-b]thiazol-3-yl)-(5-phenyl-thiazol-2-yl)-methanol;
2-{5-[(2-cyclopropyl-imidazo[5,1-*b*]thiazol-3-yl)-hydroxy-methyl]-thiophen-2-yl}-benzonitrile;
4-{5-[(2-cyclopropyl-imidazo[5,1-*b*]thiazol-3-yl)-hydroxy-methyl]-thiophen-2-yl}-benzonitrile;
(2-cyclopropyl-imidazo[5,1-*b*]thiazol-3-yl)-[5-(3-methyl-3*H*-[1,2,3]triazol-4-yl)-thiophen-2-yl]-methanol;
(2-cyclopropyl-imidazo[5,1-b]thiazol-3-yl)-(5-phenyl-[1,3,4]thiadiazol-2-yl)-methanol;
(2-cyclopropyl-imidazo[5,1-*b*]thiazol-3-yl)-[5-(1*H*-indol-5-yl)-thiophen-2-yl]-methanol;
(2-cyclopropyl-imidazo[5,1-*b*]thiazol-3-yl)-(5-isoquinolin-4-yl-thiophen-2-yl)-methanol;
(2-cyclopropyl-imidazo[5,1-*b*]thiazol-3-yl)-(1-phenyl-1*H*-imidazol-4-yl)-methanol;
[2-(cis-2-fluoro-cyclopropyl)-imidazo[5,1-*b*]thiazol-3-yl]-[1-(4-methoxy-phenyl)-1*H*-[1,2,3]triazol-4-yl]-methanol;
and [2-(trans-2-fluoro-cyclopropyl)-imidazo[5,1-*b*]thiazol-3-yl]-[1-(4-methoxy-phenyl)-1*H*-[1,2,3]triazol-4-yl]-methanol;

or a pharmaceutically acceptable salt thereof.

**11.** A compound according to claim 1 selected from a group consisting of

(4-{4-[(*R*)-(2-ethyl-imidazo[5,1-*b*]thiazol-3-yl)-hydroxy-methyl]-[1,2,3]triazol-1-yl}-phenyl)-carbamic acid methyl ester;
(*R*)-(2-cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol;
1-(4-{4-[(R)-(2-cyclopropyl-imidazo[5,1-b]thiazol-3-yl)-hydroxy-methyl]-[1,2,3]triazol-1-yl}-phenoxy)-2-methyl-propan-2-ol;
(*R*)-(2-cyclopropyl-imidazo[5,1-*b*]thiazol-3-yl)-(5-methyl-1-phenyl-1*H*-[1,2,3]triazol-4-yl)-methanol;
6-{4-[(2-cyclopropyl-imidazo[5,1-*b*]thiazol-3-yl)-hydroxy-methyl]-[1,2,3]triazol-1-yl}-3*H*-benzooxazol-2-one;
(4-{4-[(2-cyclopropyl-imidazo[5,1-b]thiazol-3-yl)-hydroxy-methyl]-[1,2,3]triazol-1-yl}-phenyl)-carbamic acid tert-butyl ester;
6-{4-[(2-ethyl-imidazo[5,1-*b*]thiazol-3-yl)-hydroxy-methyl]-[1,2,3]triazol-1-yl}-3*H*-benzooxazol-2-one;
4-{4-[(2-cyclopropyl-imidazo[5,1-*b*]thiazol-3-yl)-hydroxy-methyl]-[1,2,3]triazol-1-yl}-*N*-methyl-benzamide;
1-(4-{4-[(2-cyclopropyl-imidazo[5,1-b]thiazol-3-yl)-hydroxy-methyl]-[1,2,3]triazol-1-yl}-2-methoxy-phenyl)-3-ethyl-urea;
4-{4-[(2-cyclopropyl-imidazo[5,1-*b*]thiazol-3-yl)-hydroxy-methyl]-[1,2,3]triazol-1-yl}-*N*-(3-hydroxy-propyl)-benzamide;
4-{4-[(2-cyclopropyl-imidazo[5,1-*b*]thiazol-3-yl)-hydroxy-methyl]-[1,2,3]triazol-1-yl}-benzamide;
4-{4-[(2-cyclopropyl-imidazo[5,1-*b*]thiazol-3-yl)-hydroxy-methyl]-[1,2,3]triazol-1-yl}-*N*-(2-hydroxy-ethyl)-benzamide;
*N*-(4-{4-[(2-cyclopropyl-imidazo[5,1-*b*]thiazol-3-yl)-hydroxy-methyl]-[1,2,3]triazol-1-yl}-phenyl)-2,2-dimethyl-propionamide;
1-butyl-3-(4-{4-[(2-cyclopropyl-imidazo[5,1-*b*]thiazol-3-yl)-hydroxy-methyl]-[1,2,3]triazol-1-yl}-phenyl)-urea;
*N*-(4-{4-[(2-cyclopropyl-imidazo[5,1-*b*]thiazol-3-yl)-hydroxy-methyl]-[1,2,3]triazol-1-yl}-phenyl)-benzamide;
4-{4-[(2-cyclopropyl-imidazo[5,1-*b*]thiazol-3-yl)-hydroxy-methyl]-[1,2,3]triazol-1-yl}-*N*-furan-2-ylmethyl-benzamide;
4-{4-[(2-cyclopropyl-imidazo[5,1-*b*]thiazol-3-yl)-hydroxy-methyl]-[1,2,3]triazol-1-yl}-*N*-ethyl-*N*-methyl-benzamide;
*N*-(4-{4-[(2-cyclopropyl-imidazo[5,1-*b*]thiazol-3-yl)-hydroxy-methyl]-[1,2,3]triazol-1-yl}-phenyl)-isobutyramide;
*N*-(4-{4-[(*R*)-(2-cyclopropyl-imidazo[5,1-*b*]thiazol-3-yl)-hydroxy-methyl]-[1,2,3]triazol-1-yl}-phenyl)-isobutyramide;
*N*-allyl-4-{4-[(2-cyclopropyl-imidazo[5,1-*b*]thiazol-3-yl)-hydroxy-methyl]-[1,2,3]triazol-1-yl}-benzamide;
5-{4-[(2-cyclopropyl-imidazo[5,1-*b*]thiazol-3-yl)-hydroxy-methyl]-[1,2,3]triazol-1-yl}-1,3-dihydro-benzoimidazol-2-one;
4-{4-[(2-cyclopropyl-imidazo[5,1-*b*]thiazol-3-yl)-hydroxy-methyl]-[1,2,3]triazol-1-yl}-*N*,*N*-dimethyl-benzamide;
4-{4-[(2-ethyl-imidazo[5,1-*b*]thiazol-3-yl)-hydroxy-methyl]-[1,2,3]triazol-1-yl}-*N*-(2-methoxy-ethyl)-benzamide;
3-{4-[(2-cyclopropyl-imidazo[5,1-*b*]thiazol-3-yl)-hydroxy-methyl]-[1,2,3]triazol-1-yl}-5-methoxy-benzonitrile;
5-{4-[(2-cyclopropyl-imidazo[5,1-*b*]thiazol-3-yl)-hydroxy-methyl]-[1,2,3]triazol-1-yl}-2,3-dimethoxy-benzonitrile;
5-{4-[(2-cyclopropyl-imidazo[5,1-*b*]thiazol-3-yl)-hydroxy-methyl]-[1,2,3]triazol-1-yl}-2-trifluoromethoxy-benzonitrile;
[1-(4-bromo-3-chloro-pyridin-2-yl)-1*H*-[1,2,3]triazol-4-yl]-(2-cyclopropyl-imidazo[5,1-*b*]thiazol-3-yl)-methanol;
2-{2-[2-(4-{4-[(2-cyclopropyl-imidazo[5,1-*b*]thiazol-3-yl)-hydroxy-methyl]-[1,2,3]triazol-1-yl}-phenoxy)-ethoxy]-ethoxy}-ethanol;
(*S*)-3-(4-{4-[(*R*)-(2-cyclopropyl-imidazo[5,1-*b*]thiazol-3-yl)-hydroxy-methyl]-[1,2,3]triazol-1-yl}-phenoxy)-propane-1,2-diol;
(S)-3-(4-{4-[(S)-(2-cyclopropyl-imidazo[5,1-b]thiazol-3-yl)-hydroxy-methyl]-[1,2,3]triazol-1-yl}-phenoxy)-propane-1,2-diol;
(2-cyclopropyl-imidazo[5,1-*b*]thiazol-3-yl)-{1-[4-(pyridin-2-ylmethoxy)-phenyl]-1*H*-[1,2,3]triazol-4-yl}-methanol;
(2-cyclopropyl-imidazo[5,1-*b*]thiazol-3-yl)-{1-[4-(2-dimethylamino-ethoxy)-phenyl]-1*H*-[1,2,3]triazol-4-yl}-methanol;
(2-cyclopropyl-imidazo[5,1-*b*]thiazol-3-yl)-{1-[4-(2-methanesulfonyl-ethoxy)-phenyl]-1*H*-[1,2,3]triazol-4-yl}-methanol;
3-(4-{4-[(2-cyclopropyl-imidazo[5,1-*b*]thiazol-3-yl)-hydroxy-methyl]-[1,2,3]triazol-1-yl}-phenoxy)-propane-1-sulfonic acid amide;
4-{4-[(2-cyclopropyl-imidazo[5,1-*b*]thiazol-3-yl)-hydroxy-methyl]-[1,2,3]triazol-1-yl}-3-methyl-phenol;
4-{4-[(2-cyclopropyl-imidazo[5,1-*b*]thiazol-3-yl)-hydroxy-methyl]-[1,2,3]triazol-1-yl}-3-fluoro-phenol;
4-{4-[(2-cyclopropyl-imidazo[5,1-*b*]thiazol-3-yl)-hydroxy-methyl]-[1,2,3]triazol-1-yl}-2-trifluoromethyl-phenol;
4-{4-[(2-cyclopropyl-imidazo[5,1-*b*]thiazol-3-yl)-hydroxy-methyl]-[1,2,3]triazol-1-yl}-2-trifluoromethoxy-phenol;
4-{4-[(2-cyclopropyl-imidazo[5,1-*b*]thiazol-3-yl)-hydroxy-methyl]-[1,2,3]triazol-1-yl}-2-methyl-phenol;
3-chloro-4-{4-[(2-cyclopropyl-imidazo[5,1-*b*]thiazol-3-yl)-hydroxy-methyl]-[1,2,3]triazol-1-yl}-phenol;
4-{4-[(2-cyclopropyl-imidazo[5,1-*b*]thiazol-3-yl)-hydroxy-methyl]-[1,2,3]triazol-1-yl}-3-trifluoromethoxy-phenol;
4-{4-[(2-cyclopropyl-imidazo[5,1-*b*]thiazol-3-yl)-hydroxy-methyl]-[1,2,3]triazol-1-yl}-3-trifluoromethyl-phenol;
3-{4-[(2-cyclopropyl-imidazo[5,1-*b*]thiazol-3-yl)-hydroxy-methyl]-[1,2,3]triazol-1-yl}-phenol;
5-{4-[(2-cyclopropyl-imidazo[5,1-*b*]thiazol-3-yl)-hydroxy-methyl]-[1,2,3]triazol-1-yl}-2-methoxy-phenol;
2-chloro-5-{4-[(2-cyclopropyl-imidazo[5,1-*b*]thiazol-3-yl)-hydroxy-methyl]-[1,2,3]triazol-1-yl}-phenol;
5-{4-[(2-cyclopropyl-imidazo[5,1-*b*]thiazol-3-yl)-hydroxy-methyl]-[1,2,3]triazol-1-yl}-2-methyl-phenol;
3-{4-[(2-cyclopropyl-imidazo[5,1-*b*]thiazol-3-yl)-hydroxy-methyl]-[1,2,3]triazol-1-yl}-5-trifluoromethyl-phenol;
3-{4-[(2-cyclopropyl-imidazo[5,1-*b*]thiazol-3-yl)-hydroxy-methyl]-[1,2,3]triazol-1-yl}-5-methyl-phenol;
5-chloro-2-{4-[(2-cyclopropyl-imidazo[5,1-*b*]thiazol-3-yl)-hydroxy-methyl]-[1,2,3]triazol-1-yl}-phenol;
(2-cyclopropyl-imidazo[5,1-*b*]thiazol-3-yl)-[1-(2,3-dihydro-benzo[1,4]dioxin-6-yl)-1*H*-pyrazol-4-yl]-methanol;
(2-cyclopropyl-imidazo[5,1-b]thiazol-3-yl)-[1-(6-methoxy-pyridin-3-yl)-1*H*-pyrazol-4-yl]-methanol;
*N*-(4-{4-[(2-cyclopropyl-imidazo[5,1-*b*]thiazol-3-yl)-hydroxy-methyl]-pyrazol-1-yl}-phenyl)-acetamide;
4-{4-[(2-ethyl-imidazo[5,1-*b*]thiazol-3-yl)-hydroxy-methyl]-[1,2,3]triazol-1-yl}-*N*-methyl-benzenesulfonamide;
4-{4-[(2-cyclopropyl-imidazo[5,1-*b*]thiazol-3-yl)-hydroxy-methyl]-[1,2,3]triazol-1-yl}-*N*-methyl-benzenesulfonamide;
(2-ethyl-imidazo[5,1-*b*]thiazol-3-yl)-[1-(3-methyl-1,1-dioxo-2,3-dihydro-1*H*-1l6-benzo[*d*]isothiazol-5-yl)-1H-[1,2,3]triazol-4-yl]-methanol;
(2-cyclopropyl-imidazo[5,1-*b*]thiazol-3-yl)-[1-(3-methyl-1,1-dioxo-2,3-dihydro-1*H*-1l6-benzo[*d*]isothiazol-5-yl)-1*H*-[1,2,3]triazol-4-yl]-methanol;
(2-cyclopropyl-imidazo[5,1-*b*]thiazol-3-yl)-[1-(1-methyl-1*H*-pyrrolo[2,3-*b*]pyridin-5-yl)-1*H*-pyrazol-4-yl]-methanol;
*N*-(4-{4-[(2-ethyl-imidazo[5,1-*b*]thiazol-3-yl)-hydroxy-methyl]-[1,2,3]triazol-1-yl}-phenyl)-benzamide;
(4-{4-[(2-cyclopropyl-imidazo[5,1-b]thiazol-3-yl)-hydroxy-methyl]-[1,2,3]triazol-1-yl}-phenoxy)-acetic acid ethyl ester;
4-{4-[(2-cyclopropyl-imidazo[5,1-b]thiazol-3-yl)-hydroxy-methyl]-[1,2,3]triazol-1-yl}-2-ethyl-phenol;
4-{4-[(2-cyclopropyl-imidazo[5,1-*b*]thiazol-3-yl)-hydroxy-methyl]-[1,2,3]triazol-1-yl}-2-hydroxy-benzonitrile;
(4-{4-[(2-ethyl-imidazo[5,1-*b*]thiazol-3-yl)-hydroxy-methyl]-[1,2,3]triazol-1-yl}-phenyl)-carbamic acid tert-butyl ester;
4-{4-[(2-cyclopropyl-imidazo[5,1-b]thiazol-3-yl)-hydroxy-methyl]-[1,2,3]triazol-1-yl}-benzenesulfonamide;
4-{4-[(2-cyclopropyl-imidazo[5,1-*b*]thiazol-3-yl)-hydroxy-methyl]-[1,2,3]triazol-1-yl}-*N*,*N*-dimethyl-benzenesulfonamide;
(2-cyclopropyl-imidazo[5,1-*b*]thiazol-3-yl)-{1-[4-(pyrrolidine-1-sulfonyl)-phenyl]-1*H*-[1,2,3]triazol-4-yl}-methanol;
4-{4-[(2-cyclopropyl-imidazo[5,1-*b*]thiazol-3-yl)-hydroxy-methyl]-[1,2,3]triazol-1-yl}-*N*,*N*-diethyl-benzenesulfonamide;
4-{4-[(2-cyclopropyl-imidazo[5,1-*b*]thiazol-3-yl)-hydroxy-methyl]-[1,2,3]triazol-1-yl}-*N*-ethyl-benzenesulfonamide;
4-{4-[(2-ethyl-imidazo[5,1-*b*]thiazol-3-yl)-hydroxy-methyl]-[1,2,3]triazol-1-yl}-benzenesulfonamide;
4-{4-[(2-ethyl-imidazo[5,1-*b*]thiazol-3-yl)-hydroxy-methyl]-[1,2,3]triazol-1-yl}-*N*,*N*-dimethyl-benzenesulfonamide;
(2-ethyl-imidazo[5,1-*b*]thiazol-3-yl)-{1-[4-(pyrrolidine-1-sulfonyl)-phenyl]-1*H*-[1,2,3]triazol-4-yl}-methanol;
*N*,*N*-diethyl-4-{4-[(2-ethyl-imidazo[5,1-*b*]thiazol-3-yl)-hydroxy-methyl]-[1,2,3]triazol-1-yl}-benzenesulfonamide;
*N*-ethyl-4-{4-[(2-ethyl-imidazo[5,1-*b*]thiazol-3-yl)-hydroxy-methyl]-[1,2,3]triazol-1-yl}-benzenesulfonamide;
(4-{4-[(2-ethyl-imidazo[5,1-*b*]thiazol-3-yl)-hydroxy-methyl]-[1,2,3]triazol-1-yl}-3-fluoro-phenyl)-carbamic acid tert-butyl ester;
*N*-(4-{4-[(2-cyclopropyl-imidazo[5,1-*b*]thiazol-3-yl)-hydroxy-methyl]-pyrazol-1-yl}-phenyl)-isobutyramide;
5-{4-[(2-cyclopropyl-imidazo[5,1-*b*]thiazol-3-yl)-hydroxy-methyl]-pyrazol-1-yl}-2-methoxy-benzonitrile;
4-{4-[(2-cyclopropyl-imidazo[5,1-*b*]thiazol-3-yl)-hydroxy-methyl]-[1,2,3]triazol-1-yl}-2-nitro-phenol;
[1-(4-methoxy-phenyl)-1*H*-[1,2,3]triazol-4-yl]-(2-phenyl-imidazo[5,1-*b*]thiazol-3-yl)-methanol;

4-{4-[hydroxy-(2-phenyl-imidazo[5,1-*b*]thiazol-3-yl)-methyl]-[1,2,3]triazol-1-yl}-2-nitro-phenol;
(4-{4-[hydroxy-(2-phenyl-imidazo[5,1-b]thiazol-3-yl)-methyl]-[1,2,3]triazol-1-yl}-phenyl)-carbamic acid *tert*-butyl ester;
(2-cyclopropyl-imidazo[5,1-*b*]thiazol-3-yl)-[1-(4-methoxy-phenyl)-5-methyl-1*H*-[1,2,3]triazol-4-yl]-methanol;
(2-cyclopropyl-imidazo[5,1-*b*]thiazol-3-yl)-[1-(1*H*-indazol-5-yl)-1*H*-[1,2,3]triazol-4-yl]-methanol;
(2-cyclopropyl-imidazo[5,1-*b*]thiazol-3-yl)-[1-(1-methyl-1*H*-indazol-7-yl)-1*H*-[1,2,3]triazol-4-yl]-methanol;
[1-(4-methoxy-phenyl)-5-methyl-1*H*-[1,2,3]triazol-4-yl]-(2-methyl-imidazo[5,1-*b*]thiazol-3-yl)-methanol;
4-{4-[(2-cyclopropyl-imidazo[5,1-*b*]thiazol-3-yl)-hydroxy-methyl]-[1,2,3]triazol-1-yl}-((*N*-methylsulfamoyl)amino)-benzene;
[2-(1,1-difluoro-ethyl)-imidazo[5,1-*b*]thiazol-3-yl]-[1-(4-methoxy-phenyl)-1*H*-[1,2,3]triazol-4-yl]-methanol;
[1-(3-chloro-1*H*-indazol-6-yl)-1*H*-[1,2,3]triazol-4-yl]-(2-cyclopropyl-imidazo[5,1-b]thiazol-3-yl)-methanol;
(2-(cyclopent-1-enyl)-imidazo[5,1-*b*]thiazol-3-yl)-[1-(4-methoxy-phenyl)-1*H*-[1,2,3]triazol-4-yl]-methanol;
(2-cyclopropyl-imidazo[5,1-b]thiazol-3-yl)-(1-pyridin-3-yl-1H-pyrazol-4-yl)-methanol;
[1-(4-methoxy-phenyl)-1*H*-[1,2,3]triazol-4-yl]-(2-methyl-imidazo[5,1-*b*]thiazol-3-yl)-methanol;
[1-(4-methoxy-phenyl)-1*H*-[1,2,3]triazol-4-yl]-[2-(2-methyl-propenyl)-imidazo[5,1-*b*]thiazol-3-yl]-methanol;
[1-(4-methoxy-phenyl)-1*H*-[1,2,3]triazol-4-yl]-[((*E*)-2-(prop-1-enyl))-imidazo[5,1-*b*]thiazol-3-yl]-methanol;
2-chloro-4-{4-[hydroxy-(2-phenyl-imidazo[5,1-*b*]thiazol-3-yl)-methyl]-[1,2,3]triazol-1-yl}-phenol;
[1-(3-chloro-4-cyclopropylmethoxy-phenyl)-1*H*-[1,2,3]triazol-4-yl]-(2-phenyl-imidazo[5,1-*b*]thiazol-3-yl)-methanol;
{1-[3-chloro-4-(3-fluoro-oxetan-3-ylmethoxy)-phenyl]-1*H*-[1,2,3]triazol-4-yl}-(2-phenyl-imidazo[5,1-*b*]thiazol-3-yl)-methanol;
3-(2-chloro-4-{4-[hydroxy-(2-phenyl-imidazo[5,1-*b*]thiazol-3-yl)-methyl]-[1,2,3]triazol-1-yl}-phenoxy)-propan-1-ol;
4-(2-chloro-4-{4-[hydroxy-(2-phenyl-imidazo[5,1-b]thiazol-3-yl)-methyl]-[1,2,3]triazol-1-yl}-phenoxy)-2-methyl-butan-2-ol;
4-(2-chloro-4-{4-[(R)-hydroxy-(2-phenyl-imidazo[5,1-b]thiazol-3-yl)-methyl]-[1,2,3]triazol-1-yl}-phenoxy)-2-methyl-butan-2-ol;
(2-isopropenyl-imidazo[5,1-*b*]thiazol-3-yl)-[1-(4-methoxy-phenyl)-1*H*-[1,2,3]triazol-4-yl]-methanol;
[2-((*E*)-2-cyclopropyl-vinyl)-imidazo[5,1-*b*]thiazol-3-yl]-[1-(4-methoxy-phenyl)-1*H*-[1,2,3]triazol-4-yl]-methanol;
[1-(4-methoxy-phenyl)-1*H*-[1,2,3]triazol-4-yl]-[((E)-2-(pent-1-enyl))-imidazo[5,1-*b*]thiazol-3-yl]-methanol;
[1-(4-amino-3-chloro-phenyl)-1*H*-[1,2,3]triazol-4-yl]-(2-ethyl-imidazo[5,1-*b*]thiazol-3-yl)-methanol;
[1-(4-methoxy-phenyl)-1*H*-[1,2,3]triazol-4-yl]-[2-(1-methyl-1*H*-pyrazol-4-yl)-imidazo[5,1-*b*]thiazol-3-yl]-methanol;
[5-*tert*-butyl-1-(4-methoxy-phenyl)-1*H*-[1,2,3]triazol-4-yl]-(2-cyclopropyl-imidazo[5,1-*b*]thiazol-3-yl)-methanol;
3-(2-chloro-4-{4-[(R)-(2-cyclopropyl-imidazo[5,1-b]thiazol-3-yl)-hydroxy-methyl]-[1,2,3]triazol-1-yl}-phenoxy)-propan-1-ol;
(*R*)-{1-[3-chloro-4-(tetrahydro-pyran-4-ylmethoxy)-phenyl]-1*H*-[1,2,3]triazol-4-yl}-(2-cyclopropyl-imidazo[5,1-b]thiazol-3-yl)-methanol;
4-(2-chloro-4-{4-[(R)-(2-cyclopropyl-imidazo[5,1-b]thiazol-3-yl)-hydroxy-methyl]-[1,2,3]triazol-1-yl}-phenoxy)-2-methyl-butan-2-ol;
[1-(4-amino-3-chloro-phenyl)-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[5-ethyl-1-(4-methoxy-phenyl)-1*H*-[1,2,3]triazol-4-yl]-methanol;
(2-cyclopropyl-imidazo[5,1-*b*]thiazol-3-yl)-[5-isopropyl-1-(4-methoxy-phenyl)-1*H*-[1,2,3]triazol-4-yl]-methanol;
(2-cyclopropyl-imidazo[5,1-*b*]thiazol-3-yl)-[5-cyclopropyl-1-(4-methoxy-phenyl)-1*H*-[1,2,3]triazol-4-yl]-methanol;
(*R*)-{1-[3-chloro-4-(3-fluoro-oxetan-3-ylmethoxy)-phenyl]-1*H*-[1,2,3]triazol-4-yl}-(2-cyclopropyl-imidazo[5,1-*b*]thiazol-3-yl)-methanol;
1-(2-chloro-4-{4-[(*R*)-(2-cyclopropyl-imidazo[5,1-*b*]thiazol-3-yl)-hydroxy-methyl]-[1,2,3]triazol-1-yl}-phenoxy)-2-methyl-propan-2-ol;
*N*-(2-chloro-4-{4-[(2-cyclopropyl-imidazo[5,1-*b*]thiazol-3-yl)-hydroxy-methyl]-[1,2,3]triazol-1-yl}-phenyl)-isobutyramide;
(2-ethyl-imidazo[5,1-*b*]thiazol-3-yl)-[1-(4-methoxy-phenyl)-1*H*-[1,2,3]triazol-4-yl]-methanol;
[2-(4-fluoro-phenyl)-imidazo[5,1-*b*]thiazol-3-yl]-[1-(4-methoxy-phenyl)-1*H*-[1,2,3]triazol-4-yl]-methanol;
[2-(2-fluoro-phenyl)-imidazo[5,1-*b*]thiazol-3-yl]-[1-(4-methoxy-phenyl)-1*H*-[1,2,3]triazol-4-yl]-methanol;
[1-(3-chloro-5-fluoro-4-methoxy-phenyl)-1*H*-[1,2,3]triazol-4-yl]-(2-cyclopropyl-imidazo[5,1-*b*]thiazol-3-yl)-methanol;
[1-(3-chloro-4-methoxy-phenyl)-1*H*-[1,2,3]triazol-4-yl]-(2-cyclopropyl-imidazo[5,1-*b*]thiazol-3-yl)-methanol;
[1-(3-bromo-4-methoxy-phenyl)-1*H*-[1,2,3]triazol-4-yl]-(2-cyclopropyl-imidazo[5,1-*b*]thiazol-3-yl)-methanol;

(2-cyclopropyl-imidazo[5,1-*b*]thiazol-3-yl)-[1-(3-iodo-4-methoxy-phenyl)-1*H*-[1,2,3]triazol-4-yl]-methanol;
[1-(3-chloro-2-fluoro-4-methoxy-phenyl)-1*H*-[1,2,3]triazol-4-yl]-(2-cyclopropyl-imidazo[5,1-*b*]thiazol-3-yl)-methanol;
4-(2-chloro-4-{4-[hydroxy-(2-isopropenyl-imidazo[5,1-*b*]thiazol-3-yl)-methyl]-[1,2,3]triazol-1-yl}-phenoxy)-2-methyl-butan-2-ol;
3-{4-[(2-cyclopropyl-imidazo[5,1-*b*]thiazol-3-yl)-hydroxy-methyl]-[1,2,3]triazol-1-ylmethyl}-benzoic acid methyl ester;
[1-(3-chloro-4-methoxy-phenyl)-5-methyl-1H-[1,2,3]triazol-4-yl]-(2-cyclopropyl-imidazo[5,1-*b*]thiazol-3-yl)-methanol;
[1-(3-chloro-4-ethoxy-phenyl)-5-methyl-1*H*-[1,2,3]triazol-4-yl]-(2-cyclopropyl-imidazo[5,1-*b*]thiazol-3-yl)-methanol;
4-{4-[(2-cyclopropyl-imidazo[5,1-*b*]thiazol-3-yl)-hydroxy-methyl]-[1,2,3]triazol-1-ylmethyl}-benzoic acid methyl ester;
3-{4-[(2-cyclopropyl-imidazo[5,1-*b*]thiazol-3-yl)-hydroxy-methyl]-[1,2,3]triazol-1-ylmethyl}-phenol;
{1-[3-chloro-4-(3-fluoro-oxetan-3-ylmethoxy)-phenyl]-1*H*-[1,2,3]triazol-4-yl}-(2-isopropenyl-imidazo[5,1-*b*]thiazol-3-yl)-methanol;
[1-(5-chloro-2-fluoro-4-methoxy-phenyl)-1*H*-[1,2,3]triazol-4-yl]-(2-cyclopropyl-imidazo[5,1-*b*]thiazol-3-yl)-methanol;
3-(2-chloro-4-{4-[hydroxy-(2-isopropenyl-imidazo[5,1-*b*]thiazol-3-yl)-methyl]-[1,2,3]triazol-1-yl}-phenoxy)-propan-1-ol;
4-(2-chloro-4-{4-[(2-ethyl-imidazo[5,1-*b*]thiazol-3-yl)-hydroxy-methyl]-[1,2,3]triazol-1-yl}-phenoxy)-2-methyl-butan-2-ol;
4-[2-chloro-4-(4-{hydroxy-[2-(1-methyl-cyclopropyl)-imidazo[5,1-*b*]thiazol-3-yl]-methyl}-[1,2,3]triazol-1-yl)-phenoxy]-2-methyl-butan-2-ol;
4-{4-[(2-cyclopropyl-imidazo[5,1-*b*]thiazol-3-yl)-hydroxy-methyl]-[1,2,3]triazol-1-ylmethyl}-phenol;
2-{4-[(2-cyclopropyl-imidazo[5,1-*b*]thiazol-3-yl)-hydroxy-methyl]-[1,2,3]triazol-1-ylmethyl}-phenol;
(2-cyclopropyl-imidazo[5,1-*b*]thiazol-3-yl)-[1-(4-methoxy-2-methyl-phenyl)-1*H*-[1,2,3]triazol-4-yl]-methanol;
4-[2-chloro4-(4-{[2-(1,1-difluoro-ethyl)-imidazo[5,1-*b*]thiazol-3-yl]-hydroxy-methyl}-[1,2,3]triazol-1-yl)-phenoxy]-2-methyl-butan-2-ol;
4-[2-chloro-4-(4-{[2-(*trans*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol;
4-[2-chloro-4-(4-{[2-(*cis*-2-fluoro-cyclopropyl)-imidazo[5,1-*b*]thiazol-3-yl]-hydroxy-methyl}-[1,2,3]triazol-1-yl)-phenoxy]-2-methyl-butan-2-ol;
(2-cyclopropyl-imidazo[5,1-*b*]thiazol-3-yl)-[1-(4-hydroxymethyl-phenyl)-1*H*-[1,2,3]triazol-4-yl]-methanol;
(2-cyclopropyl-imidazo[5,1-*b*]thiazol-3-yl)-[1-(4-methoxy-2,6-dimethyl-phenyl)-1*H*-[1,2,3]triazol-4-yl]-methanol;
(2-cyclopropyl-imidazo[5,1-*b*]thiazol-3-yl)-(1-{4-[2-(2,2,2-trifluoro-ethoxy)-ethoxy]-phenyl}-1*H*-[1,2,3]triazol-4-yl)-methanol;
3-[2-chloro-4-(4-{hydroxy-[2-(1-methyl-cyclopropyl)-imidazo[5,1-*b*]thiazol-3-yl]-methyl}-[1,2,3]triazol-1-yl)-phenoxy]-propan-1-ol;
3-[2-chloro4-(4-{[2-(1,1-difluoro-ethyl)-imidazo[5,1-*b*]thiazol-3-yl]-hydroxy-methyl}-[1,2,3]triazol-1-yl)-phenoxy]-propan-1-ol;
{1-[3-chloro-4-(3-fluoro-oxetan-3-ylmethoxy)-phenyl]-1*H*-[1,2,3]triazol-4-yl}-[2-(1-methyl-cyclopropyl)-imidazo[5,1-*b*]thiazol-3-yl]-methanol;
{1-[3-chloro-4-(3-fluoro-oxetan-3-ylmethoxy)-phenyl]-1*H*-[1,2,3]triazol-4-yl}-[2-(1,1-difluoro-ethyl)-imidazo[5,1-*b*]thiazol-3-yl]-methanol;
{1-[3-chloro-4-(3-fluoro-oxetan-3-ylmethoxy)-phenyl]-1*H*-[1,2,3]triazol-4-yl}-(2-ethyl-imidazo[5,1-*b*]thiazol-3-yl)-methanol;
(2-cyclopropyl-imidazo[5,1-*b*]thiazol-3-yl)-[1-(2,5-difluoro-4-methoxy-phenyl)-1*H*-[1,2,3]triazol-4-yl]-methanol;
[1-(5-chloro-2-fluoro-4-methoxy-phenyl)-1*H*-[1,2,3]triazol-4-yl]-(2-ethyl-imidazo[5,1-*b*]thiazol-3-yl)-methanol;
3-(2-chloro-4-{4-[(2-ethyl-imidazo[5,1-*b*]thiazol-3-yl)-hydroxy-methyl]-[1,2,3]triazol-1-yl}-phenoxy)-propan-1-ol;
[1-(2,5-difluoro-4-methoxy-phenyl)-1*H*-[1,2,3]triazol-4-yl]-(2-ethyl-imidazo[5,1-*b*]thiazol-3-yl)-methanol;
[1-(4-benzyloxy-3-fluoro-phenyl)-1*H*-[1,2,3]triazol-4-yl]-[2-(*trans*-2-fluoro-cyclopropyl)-imidazo[5,1-*b*]thiazol-3-yl]-methanol;
2-chloro4-{4-[(2-ethyl-imidazo[5,1-*b*]thiazol-3-yl)-hydroxy-methyl]-[1,2,3]triazol-1-yl}-phenol;
2-chloro-4-(4-{hydroxy-[2-(1-methyl-cyclopropyl)-imidazo[5,1-*b*]thiazol-3-yl]-methyl}-[1,2,3]triazol-1-yl)-phenol;
2-chloro-4-(4-{[2-(*cis*-2-fluoro-cyclopropyl)-imidazo[5,1-*b*]thiazol-3-yl]-hydroxy-methyl}-[1,2,3]triazol-1-yl)-phenol;

(2-cyclopropyl-imidazo[5,1-*b*]thiazol-3-yl)-[1-(4-methoxy-2-methyl-phenyl)-5-methyl-1*H*-[1,2,3]triazol-4-yl]-methanol;
(2-cyclopropyl-imidazo[5,1-*b*]thiazol-3-yl)-[1-(3-fluoro-4-methoxy-phenyl)-1*H*-[1,2,3]triazol-4-yl]-methanol;
2-chloro-4-(4-{[2-(*trans*-2-fluoro-cyclopropyl)-imidazo[5,1-*b*]thiazol-3-yl]-hydroxy-methyl}-[1,2,3]triazol-1-yl)-phenol;
2-chloro4-(4-{[2-(1,1-difluoro-ethyl)-imidazo[5,1-*b*]thiazol-3-yl]-hydroxy-methyl}-[1,2,3]triazol-1-yl)-phenol;
(2-cyclopropyl-imidazo[5,1-*b*]thiazol-3-yl)-[1-(4-methoxymethyl-phenyl)-1*H*-[1,2,3]triazol-4-yl]-methanol;
4-{4-[(2-cyclopropyl-imidazo[5,1-*b*]thiazol-3-yl)-hydroxy-methyl]-[1,2,3]triazol-1-yl}-2,5-difluoro-phenol;
4-{4-[(2-ethyl-imidazo[5,1-*b*]thiazol-3-yl)-hydroxy-methyl]-[1,2,3]triazol-1-yl}-2,5-difluoro-phenol;
[1-(5-chloro-2-fluoro-4-methoxy-phenyl)-5-methyl-1*H*-[1,2,3]triazol-4-yl]-(2-cyclopropyl-imidazo[5,1-*b*]thiazol-3-yl)-methanol;
(2-cyclopropyl-imidazo[5,1-*b*]thiazol-3-yl)-[5-iodo-1-(4-methoxy-phenyl)-1*H*-[1,2,3]triazol-4-yl]-methanol;
[2-(*trans*-2-fluoro-cyclopropyl)-imidazo[5,1-*b*]thiazol-3-yl]-[5-fluoro-1-(4-methoxy-phenyl)-1*H*-[1,2,3]triazol-4-yl]-methanol; and
(2-cyclopropyl-imidazo[5,1-*b*]thiazol-3-yl)-[5-fluoro-1-(4-methoxy-phenyl)-1H-[1,2,3]triazol-4-yl]-methanol;
or a pharmaceutically acceptable salt thereof.

**12.** A pharmaceutical composition comprising a compound according to any one of claims 1 to 11, further comprising at least one pharmaceutically acceptable carrier.

**13.** A compound according to any one of claims 1 to 11, for use in the treatment and/or prevention of cancer, wherein said compound is used in combination with one or more chemotherapeutical agents and/or radiotherapy and/or targeted therapy.

**14.** A compound according to any one of claims 1 to 11 for use as a medicament.

**15.** A compound according to any one of claims 1 to 11, for use in the prevention and/or treatment of cancer.

## Patentansprüche

**1.** Verbindung gemäß Formel (I)

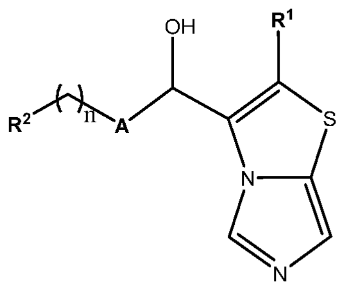

Formel (I)

wobei

**A** Phenylen oder 5- bis 6-gliedriges Heteroarylen repräsentiert, wobei das genannte Phenylen oder 5- bis 6-gliedrige Heteroarylen unabhängig unsubstituiert, mono- oder disubstituiert ist, wobei die Substituenten unabhängig aus $C_{1-4}$-Alkyl, Halogen oder $C_{3-5}$-Cycloalkyl ausgewählt sind;
**n** 1 oder 0 repräsentiert;
**R**$^1$ Folgendes repräsentiert:

- $C_{1-4}$-Alkyl;
- -C(**R**$^A$)=C(**R**$^B$)(**R**$^C$), wobei **R**$^A$, **R**$^B$ und **R**$^C$ unabhängig aus Wasserstoff, $C_{1-4}$-Alkyl und $C_{3-5}$-Cycloalkyl ausgewählt sind; oder wobei **R**$^A$ und **R**$^B$ zusammen mit den Kohlenstoffatomen, an die sie angefügt sind, $C_{4-6}$-Cycloalkenyl bilden, und **R**$^C$ Wasserstoff repräsentiert;

- $C_{1-3}$-Fluoralkyl;
- Phenyl, das unsubstituiert oder mono-, di- oder trisubstituiert ist, wobei die Substituenten unabhängig aus $C_{1-3}$-Alkyl und Halogen ausgewählt sind;
- 5-gliedriges Heteroaryl, das unsubstituiert oder durch $C_{1-4}$-Alkyl monosubstituiert ist; oder
- $C_{3-6}$-Cycloalkyl, das unsubstituiert oder mono-, di- oder trisubstituiert ist, wobei die Substituenten unabhängig aus $C_{1-3}$-Alkyl und Halogen ausgewählt sind;

**R**$^2$ Folgendes repräsentiert:

- Phenyl oder 6-gliedriges Heteroaryl, die unabhängig unsubstituiert oder mono-, di- oder trisubstituiert sind, wobei die Substituenten unabhängig ausgewählt sind aus:

  ➢ $C_{1-4}$-Alkyl, Cyano, Nitro, Halogen, Hydroxy, Hydroxy-$C_{1-4}$-alkyl, $C_{1-3}$-Alkoxy-$C_{1-4}$-alkyl, $C_{1-3}$-Fluoralkyl, $C_{1-3}$-Fluoralkoxy, $C_{1-4}$-Alkoxy, $C_{3-5}$-Cycloalkoxy, Hydroxymethyl-cyclopropyl, $C_{1-3}$-Alkyl-carbonyl, $C_{1-4}$-Alkoxy-carbonyl, Amino, Morpholin-4-yl oder Morpholin-4-yl-methyl;
  ➢ -N**R**$^{N1}$**R**$^{N2}$, wobei **R**$^{N1}$ Wasserstoff repräsentiert und **R**$^{N2}$-(C=O)-**R**$^{CO}$ repräsentiert, wobei **R**$^{CO}$ $C_{1-4}$-Alkyl, $C_{1-4}$-Alkoxy, Amino, $C_{1-4}$-Alkylamino, Phenyl oder Morpholin-4-yl repräsentiert;
  ➢ -(C=O)-N**R**$^{N3}$**R**$^{N4}$, wobei **R**$^{N3}$ Wasserstoff oder $C_{1-3}$-Alkyl repräsentiert, und **R**$^{N4}$ Wasserstoff, $C_{1-3}$-Alkyl, $C_{3-5}$-Cycloalkyl, Hydroxy-$C_{2-4}$-alkyl, $C_{1-3}$-Alkoxy-$C_{2-4}$-alkyl, Cyano-$C_{1-3}$-alkyl, $C_{3-4}$-Alkenyl, Furanyl-$C_{1-3}$-alkyl, 2-Di-$C_{1-3}$-alkylamino-$C_{2-3}$-alkyl oder Allyl repräsentiert; oder wobei **R**$^{N3}$ und **R**$^{N4}$ zusammen mit dem Stickstoffatom, an das sie angefügt sind, einen Morpholinring bilden;
  ➢ -$(NH)_p$-$SO_2$-N**R**$^{S1}$**R**$^{S2}$, wobei **R**$^{S1}$ und **R**$^{S2}$ unabhängig Wasserstoff oder $C_{1-4}$-Alkyl repräsentieren; oder wobei **R**$^{S1}$ und **R**$^{S2}$ zusammen mit dem Stickstoffatom, an das sie angefügt sind, ein 4- bis 6-gliedriges Heterocycloalkyl bilden, ausgewählt aus Azetidinyl, Pyrrolidinyl und Piperidinyl; und **p** 1 oder 0 repräsentiert; und
  ➢ -O**R**$^6$, wobei **R**$^6$ Folgendes repräsentiert: $C_{1-3}$-Alkoxy-$C_{2-4}$-alkyl, Hydroxy-$C_{2-5}$-alkyl, Di-hydroxy-$C_{2-5}$-alkyl, Sulfamoyl-$C_{2-4}$-alkyl, Pyridinyl-$C_{1-3}$-alkyl, $C_{1-3}$-Alkylsulfonyl-$C_{2-4}$-alkyl, $C_{3-5}$-Cycloalkyl-$C_{1-3}$-alkyl, Oxetan-3-yl-$C_{1-3}$-alkyl, ($C_{1-3}$-Alkyl-oxetan-3-yl)-$C_{1-3}$-alkyl, (Fluor-oxetan-3-yl)-$C_{1-3}$-alkyl, Tetrahydrofuranyl-$C_{1-3}$-alkyl, Hydroxy-$C_{3-5}$-cycloalkyl-$C_{1-3}$-alkyl, Tetrahydropyranyl-$C_{1-3}$-alkyl, Benzyl, (Hydroxy-$C_{2-4}$-alkoxy)-$C_{2-4}$-alkyl, Piperidin-1-yl-$C_{1-3}$-alkyl, (1-Methyl-piperidin-3-yl)-$C_{1-3}$-alkyl, (1,1-Di-oxidotetrahydro-thiopyran-4-yl)-$C_{1-3}$-alkyl, $C_{1-3}$-Alkoxy-carbonyl-$C_{1-3}$-alkyl, ((Hydroxy-$C_{2-3}$-alkoxy)-$C_{2-3}$-alkoxy)-$C_{2-3}$-alkyl, $C_{1-3}$-Fluoralkoxy-$C_{2-3}$-alkyl oder $C_{1-3}$-Alkyl-carbonyl;

- 5-gliedriges Heteroaryl, das unabhängig unsubstituiert oder mono-, di- oder trisubstituiert ist, wobei die Substituenten unabhängig ausgewählt sind aus:

  ➢ $C_{1-4}$-Alkyl, Cyano, Halogen, $C_{1-3}$-Fluoralkyl, $C_{1-3}$-Fluoralkoxy, $C_{1-4}$-Alkoxy, $C_{3-5}$-Cycloalkoxy, Hydroxymethyl-cyclopropyl, Morpholin-4-yl, Morpholin-4-yl-methyl, $C_{1-3}$-Alkyl-carbonyl, $C_{1-4}$-Alkoxy-carbonyl;
  ➢ -N**R**$^{N1}$**R**$^{N2}$, wobei **R**$^{N1}$ Wasserstoff repräsentiert und **R**$^{N2}$ -(C=O)-**R**$^{CO}$ repräsentiert, wobei **R**$^{CO}$ $C_{1-4}$-Alkoxy repräsentiert; oder
  ➢ -O**R**$^6$, wobei **R**$^6$ $C_{3-5}$-Cycloalkyl-$C_{1-3}$-alkyl, Benzyl oder $C_{1-3}$-Alkoxy-carbonyl-$C_{1-3}$-alkyl repräsentiert;

- 9- bis 10-gliedriges bicyclisches Heteroaryl, wobei das genannte 9- bis 10-gliedrige bicyclische Heteroaryl unabhängig unsubstituiert oder mono-, di- oder trisubstituiert ist, wobei die Substituenten unabhängig ausgewählt sind aus:

  ➢ $C_{1-4}$-Alkyl, Cyano, Halogen, $C_{1-3}$-Fluoralkyl, $C_{1-3}$-Fluoralkoxy, $C_{1-4}$-Alkoxy, Hydroxymethyl-cyclopropyl, Morpholin-4-yl, Morpholin-4-yl-methyl, $C_{1-3}$-Alkyl-carbonyl, $C_{1-4}$-Alkoxy-carbonyl;
  ➢ N**R**$^{N1}$**R**$^{N2}$, wobei **R**$^{N1}$ Wasserstoff repräsentiert und **R**$^{N2}$ -(C=O)-**R**$^{CO}$ repräsentiert, wobei **R**$^{CO}$ $C_{1-4}$-Alkoxy repräsentiert; oder
  ➢ -O**R**$^6$, wobei **R**$^6$$C_{3-5}$-Cycloalkyl-$C_{1-3}$-alkyl repräsentiert; oder

- 2,3-Dihydro-benzo[1,4]dioxin-6-yl, 1,3-Dihydro-2H-benzoimidazol-2-on-5-yl, 3H-Benzooxazol-2-on-6-yl, 3-Methyl-1,1-dioxo-2,3-dihydro-1*H*-1l6-benzo[*d*]isothiazol-5-yl, 1-Methyl-1H-pyrrolo[2,3-b]pyridin-5-yl oder 3,4-Dihydro-2H-pyrano[2,3-b]pyridin-6-yl;

oder ein pharmazeutisch akzeptables Salz davon.

**2.** Verbindung nach Anspruch 1, wobei **A** Folgendes repräsentiert:

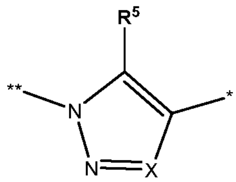

,

wobei ein Sternchen "*" die Anfügungsstelle am Kohlenstoff kennzeichnet, der die OH-Gruppe trägt; zwei Sternchen "**" die Anfügungsstelle an $\mathbf{R}^2$-$(CH_2)_n$ kennzeichnen; **X** N oder CH repräsentiert; und $\mathbf{R}^5$ Wasserstoff oder $C_{1-4}$-Alkyl, Halogen oder $C_{3-5}$-Cycloalkyl repräsentiert;
oder ein pharmazeutisch akzeptables Salz davon.

**3.** Verbindung nach Anspruch 1 oder 2, wobei

$\mathbf{R}^2$ Folgendes repräsentiert:

• Phenyl oder 6-gliedriges Heteroaryl, wobei das genannte Phenyl oder 6-gliedrige Heteroaryl unabhängig unsubstituiert oder mono-, di- oder trisubstituiert ist; und wobei die Substituenten unabhängig ausgewählt sind aus:

➢ $C_{1-4}$-Alkyl, Cyano, Nitro, Halogen, Hydroxy, Hydroxy-$C_{1-4}$-alkyl, $C_{1-3}$-Alkoxy-$C_{1-4}$-alkyl, $C_{1-3}$-Fluoralkyl, $C_{1-3}$-Fluoralkoxy, $C_{1-4}$-Alkoxy, $C_{3-5}$-Cycloalkoxy, Hydroxymethyl-cyclopropyl, $C_{1-3}$-Alkyl-carbonyl, $C_{1-4}$-Alkoxy-carbonyl, Amino, Morpholin-4-yl oder Morpholin-4-yl-methyl;
➢ -N$\mathbf{R}^{N1}\mathbf{R}^{N2}$, wobei $\mathbf{R}^{N1}$ Wasserstoff repräsentiert und $\mathbf{R}^{N2}$ -(C=O)-$\mathbf{R}^{CO}$ repräsentiert, wobei $\mathbf{R}^{CO}$ $C_{1-4}$-Alkyl, $C_{1-4}$-Alkoxy, Amino, Phenyl oder Morpholin-4-yl repräsentiert;
➢ -(C=O)-N$\mathbf{R}^{N3}\mathbf{R}^{N4}$, wobei $\mathbf{R}^{\mathbf{N3}}$ Wasserstoff oder $C_{1-3}$-Alkyl repräsentiert und $\mathbf{R}^{N4}$Wasserstoff, $C_{1-3}$-Alkyl, $C_{3-5}$-Cycloalkyl, Hydroxy-$C_{2-4}$-alkyl, $C_{1-3}$-Alkoxy-$C_{2-4}$-alkyl, Cyano-$C_{1-3}$-alkyl, $C_{3-4}$-Alkenyl oder Furanyl-$C_{1-3}$-alkyl oder Allyl repräsentiert; oder wobei $\mathbf{R}^{N3}$ und $\mathbf{R}^{\mathbf{N4}}$ zusammen mit dem Stickstoffatom, an das sie angefügt sind, einen Morpholinring bilden; und
➢ -O$\mathbf{R}^6$, wobei $\mathbf{R}^6$ Folgendes repräsentiert: $C_{1-3}$-Alkoxy-$C_{2-4}$-alkyl, Hydroxy-$C_{2-5}$-alkyl, Di-hydroxy-$C_{2-5}$-alkyl, Sulfamoyl-$C_{2-4}$-alkyl, Pyridinyl-$C_{1-3}$-alkyl, $C_{1-3}$-Alkylsulfonyl-$C_{2-4}$-alkyl, $C_{3-5}$-Cycloalkyl-$C_{1-3}$-alkyl, Oxetan-3-yl-$C_{1-3}$-alkyl, ($C_{1-3}$-Alkyl-oxetan-3-yl)-$C_{1-3}$-alkyl, (Fluor-oxetan-3-yl)-$C_{1-3}$-alkyl, Tetrahydrofuranyl-$C_{1-3}$-alkyl, Hydroxy-$C_{3-5}$-cycloalkyl-$C_{1-3}$-alkyl, Tetrahydropyranyl-$C_{1-3}$-alkyl, Benzyl, (Hydroxy-$C_{2-4}$-alkoxy)-$C_{2-4}$-alkyl, Piperidin-1-yl-$C_{1-3}$-alkyl, (1-Methyl-piperidin-3-yl)-$C_{1-3}$-alkyl, (1,1-Dioxidotetrahydro-thiopyran-4-yl)-$C_{1-3}$-alkyl, $C_{1-3}$-Alkoxy-carbonyl-$C_{1-3}$-alkyl, ((Hydroxy-$C_{2-3}$-alkoxy)-$C_{2-3}$-alkoxy)-$C_{2-3}$-alkyl, $C_{1-3}$-Fluoralkoxy-$C_{2-3}$-alkyl oder $C_{1-3}$-Alkyl-carbonyl;

• 9- bis 10-gliedriges bicyclisches Heteroaryl, wobei das genannte 9- bis 10-gliedrige bicyclische Heteroaryl unsubstituiert ist; oder
• 2,3-Dihydro-benzo[1,4]dioxin-6-yl, 1,3-Dihydro-2H-benzoimidazol-2-on-5-yl, 3H-Benzooxazol-2-on-6-yl, 3-Methyl-1,1-dioxo-2,3-dihydro-1*H*-1l6-benzo[*d*]isothiazol-5-yl, 1-Methyl-1H-pyrrolo[2,3-b]pyridin-5-yl oder 3,4-Dihydro-2H-pyrano[2,3-b]pyridin-6-yl;

oder ein pharmazeutisch akzeptables Salz davon.

**4.** Verbindung nach Anspruch 1 oder 2, wobei das Fragment $\mathbf{R}^2$-$(CH_2)_n$— ausgewählt ist aus der Gruppe I, II, III, IV, V, VI, VII, VIII, IX, X, XI oder XII:

I. Phenyl, 4-Hydroxy-phenyl, 4-Chlor-phenyl, 2-Methyl-phenyl, 3-Methyl-phenyl, 4-Methyl-phenyl, 2-Methoxy-phenyl, 3-Methoxy-phenyl, 4-Methoxy-phenyl, 4-Ethoxy-phenyl, 4-(2-Methoxy-ethoxy)-phenyl, 4-Benzyloxy-phenyl, 3-Cyano-phenyl, 4-Cyano-phenyl, 2-Trifluormethyl-phenyl, 3-Trifluormethyl-phenyl, 4-Trifluormethyl-phenyl, 4-Isopropoxy-phenyl, 4-(Cyclopropyl-methoxy)-phenyl, 4-(1-Hydroxymethyl-cyclopropyl)-phenyl, 4-Trifluormethoxy-phenyl, 4-(Morpholin-4-yl)-phenyl, 4-(Morpholin-4-yl-methyl)-phenyl oder 4-(Methoxy-carbamoyl)-phenyl;

II. 4-(2-Hydroxy-2-methyl-propoxy)-phenyl, 4-(3-Hydroxy-propoxy)-phenyl, 3-Cyano-4-methoxy-phenyl, 4-(2-Hydroxy-ethoxy)-phenyl, 3-Chlor-4-hydroxy-phenyl, 3-Fluor-4-hydroxy-phenyl, 3,5-Difluor-4-hydroxy-phenyl, 4-((Methoxy-carbonyl)amino)-phenyl, 4-(Morpholin-4-carbonyl)-phenyl, 4-((2-Cyanoethyl)-carbamoyl)-phenyl, 4-(Morpholin-4-yl-carboxamido)-phenyl, 4-(*tert*-Butyl-carboxamido)-phenyl, 2-Fluor-4-(*tert*-butoxyl-carboxamido)-phenyl, 4-(3-Ethylureido)-3-methoxy-phenyl, 4-(Methoxy-carboxamido)-phenyl, 4-(3-Ethyl-ureido)-3-methoxy-phenyl, 4-(3-(3-Methoxy-propyl)-ureido)-phenyl, 4-(3-Butyl-ureido)-phenyl, 4-Isobutyramido-phenyl, 4-Acetyl-phenyl, 4-(Tetrahydropyran-4-yl-methoxy)-phenyl, 4-((3-Fluor-oxetan-3-yl)-methoxy)-phenyl, 4-((3-Methyl-oxetan-3-yl)-methoxy)-phenyl, 4-(Oxetan-3-yl-methoxy)-phenyl, 4-(2-(1-Hydroxy-cyclopropyl)-ethoxy)-phenyl, 4-(3-Hydroxy-2,2-dimethyl-propyl)-phenyl, 4-(3-Hydroxy-3-methyl-butyl)-phenyl, 4-(2-(2-Hydroxy-ethoxy)-ethoxy)-phenyl, 4-(2-(Piperidin-1-yl)-ethoxy)-phenyl, 4-(Tetrahydrofuran-2-yl-methoxy)-phenyl, 4-(Tetrahydropyran-4-yl-methoxy)-phenyl, 4-(Tetrahydropyran-2-yl-methoxy)-phenyl, 4-((2-Methoxyethyl)-carbamoyl)-phenyl, 4-(Cyclopropyl-carbamoyl)-phenyl, 4-(Ethyl-carbamoyl)-phenyl, 4-(3-Methoxypropyl-carbamoyl)-phenyl, 4-Ureido-phenyl, 4-Acetamido-phenyl, 4-(Methyl-carbamoyl)-phenyl, 4-((1-Methyl-piperidin-3-yl)-methoxy)-phenyl, 4-((2-Hydroxy-ethyl)-carbamoyl)-phenyl, 4-(Ethyl(methyl)carbamoyl)-phenyl, 4-((3-Hydroxy-propyl)-carbamoyl)-phenyl, 4-Carbamoyl-phenyl, 4-((Furan-2-yl-methyl)-carbamoyl)-phenyl, 4-(Allyl-carbamoyl)-phenyl, 4-(Dimethyl-carbamoyl)-phenyl, 4-((2-(Dimethylamino)-ethyl)-carbamoyl)-phenyl, 4-((1,1-Dioxo-hexahydrothiopyran-4-yl)-methyl)-phenyl;

III. 3-Chlor-4-(3-hydroxy-propoxy)-phenyl, 3-Chlor-4-(2-methyl-2-hydroxy-propoxy)-phenyl, 3-Chlor-4-(3-methyl-3-hydroxy-butoxy)-phenyl, 4-(2-Methyl-2-hydroxy-propoxy)-phenyl, 4-(2,3-Dihydroxy-propoxy)-phenyl, 3-Cyano-5-methoxy-phenyl, 3-Hydroxy-4-cyano-phenyl, 3-Nitro-4-hydroxy-phenyl, 2-Chlor-4-hydroxy-phenyl, 2-Fluor-4-hydroxy-phenyl, 3-Hydroxy-4-chlor-phenyl, 2-Hydroxy-4-chlor-phenyl, 3-Hydroxy-phenyl, 2,5-Difluor-4-hydroxy-phenyl, 2,5-Difluor-4-hydroxy-phenyl, 3-Trifluormethoxy-4-hydroxy-phenyl, 3-Cyano-4-trifluormethoxy-phenyl, 2-Trifluormethoxy-4-hydroxy-phenyl, 3-Methyl-5-hydroxy-phenyl, 2-Methyl-4-hydroxy-phenyl, 3-Hydroxy-4-methyl-phenyl, 3-Methyl-4-hydroxy-phenyl, 3-Ethyl-4-hydroxy-phenyl, 4-(Hydroxy-methyl)-phenyl, (4-Hydroxy-phenyl)-methyl, (2-Hydroxy-phenyl)-methyl, 3-Chlor-4-amino-phenyl, 4-(Amino-carbonyl)-phenyl, 4-(Methyl-amino-carbonyl)-phenyl, (4-(Methoxy-carbonyl)-phenyl)-methyl, (3-(Methoxy-carbonyl)-phenyl)-methyl, 4-(Methyl-carboxamido)-phenyl, 4-(Phenyl-carboxamido)-phenyl, 4-(Iso-propyl-carboxamido)-phenyl, 3-Chlor-4-(iso-propyl-carboxamido)-phenyl, 4-(tert-Butoxy-carboxamido)-phenyl, 3-Hydroxy-4-methoxy-phenyl, 3-Chlor-4-methoxy-phenyl, 3-Chlor-4-ethoxy-phenyl, 3-Fluor-4-methoxy-phenyl, 3-Brom-4-methoxy-phenyl, 3-Iod-4-methoxy-phenyl, 3-Chlor-4-methoxy-5-fluor-phenyl, 3-Chlor-4-methoxy-6-fluor-phenyl, 3,6-Difluor-4-methoxy-phenyl, 2-Fluor-3-chlor-4-methoxy-phenyl, 2,6-Dimethyl-4-methoxy-phenyl, 3-Chlor-4-(cyclopropyl-methoxy)-phenyl, 3-Ethyl-4-hydroxy-phenyl, 4-(Methyl-methoxy)-phenyl, 3-Hydroxy-5-trifluormethyl-phenyl, 2-Trifluormethyl-4-hydroxy-phenyl, 3,4-Dimethoxy-5-cyano-phenyl, 4-((2-Dimethylamino-ethoxy))-phenyl, 4-(2-(2-(2-Hydroxyethoxy)-ethoxy)-ethoxy)-phenyl, 3-Chlor-4-((3-fluor-oxetan-3-yl)-methoxy)-phenyl, 4-(Pyrrolidin-1-sulfonyl)-phenyl, 4-(Pyridin-2-yl-methoxy)-phenyl, 4-(Ethoxy-carbonyl-methoxy)-phenyl, 4-((2-Hydroxy-ethyl)-carbamoyl)-phenyl, 4-((2-Methoxy-ethyl)-carbamoyl)-phenyl, 4-((3-Hydroxy-propyl)-carbamoyl)-phenyl, 4-(Allyl-carbamoyl)-phenyl, 4-(Allyl-carbamoyl)-phenyl, 4-(Dimethyl-carbamoyl)-phenyl, 4-(N-Methyl-N-ethyl-carbamoyl)-phenyl, 3-Fluor-4-benzyloxy-phenyl, 4-Sulfamoyl-phenyl, 4-(Methyl-sulfamoyl)-phenyl, 4-(Ethyl-sulfamoyl)-phenyl, 4-(Dimethyl-sulfamoyl)-phenyl, 4-(Diethyl-sulfamoyl)-phenyl, 4-((Methyl-sulfamoyl)-amino)-phenyl, 4-(2-Methansulfonyl-ethoxy)-phenyl, 4-(Sulfamoyl-propoxy)-phenyl, 4-(2-(2,2,2-Trifluor-ethoxy)-ethoxy)-phenyl;

IV. 6-Methoxy-pyridin-3-yl, 5-Fluor-6-methoxy-pyridin-3-yl, 6-Methoxy-5-methyl-pyridin-3-yl, 2,6-Dimethoxy-pyridin-3-yl, 5,6-Dimethoxy-pyridin-3-yl, 5,6-Dimethoxy-pyridin-2-yl, 5-Chlor-6-ethoxy-pyridin-3-yl, 6-Isopropoxy-pyridin-3-yl, 2-Methoxy-3-(methoxy-carbonyl)-pyridin-5-yl, 5-Chlor-6-isopropoxy-pyridin-3-yl, 6-Ethoxy-5-trifluormethyl-pyridin-3-yl, 6-(Methoxy-carbonyl-methoxy)-pyridin-3-yl, 6-Methoxy-5-(methoxycarbonyl)-pyridin-3-yl, 2-Methoxy-pyrimidin-5-yl, 2-Ethoxy-pyrimidin-5-yl, 2,4-Dimethoxy-pyrimidin-5-yl, 2-Benzyloxy-pyrimidin-5-yl, 2-Cyclobutoxy-pyrimidin-5-yl, 3,6-Dimethoxy-pyridazin-4-yl oder 6-(2,2,2-Trifluor-ethoxy)-pyridin-3-yl;

V. Pyridin-3-yl, 4-Methoxy-pyridin-3-yl oder 3-Chlor-4-brom-pyridin-2-yl;

VI. Pyrazol-4-yl, 1-Methyl-pyrazol-4-yl, Pyrimidin-5-yl, Pyridin-2-yl, Pyridin-3-yl, Pyridin-4-yl oder 6-Ethoxy-pyridin-3-yl;

VII. Indol-4-yl, Indol-5-yl, Indol-6-yl, Indol-7-yl, Chinolin-4-yl, Chinolin-5-yl, Isochinolin-4-yl, Isochinolin-5-yl oder Isochinolin-8-yl;

VIII. 1H-Indazol-5-yl, 3-Chlor-1H-indazol-6-yl oder 1-Methyl-1H-indazol-7-yl;

IX. Benzyl;

X. 2,3-Dihydro-[1,4]benzodioxan-6-yl;

XI. 1,3-Dihydro-2H-benzoimidazol-2-on-5-yl oder 3,4-Dihydro-2H-pyrano[2,3-b]pyridin-6-yl; und

XII. 3H-Benzooxazol-2-on-6-yl, 3-Methyl-1,1-dioxo-2,3-dihydro-1*H*-1l6-benzo[*d*]isothiazol-5-yl, 1-Methyl-1H-pyrrolo[2,3-b]pyridin-5-yl, 2,3-Dihydro-benzo[1,4]dioxin-6-yl;

oder ein pharmazeutisch akzeptables Salz davon.

5. Verbindung nach einem der Ansprüche 1 bis 3, wobei **n** 0 repräsentiert; oder ein pharmazeutisch akzeptables Salz davon.

6. Verbindung nach einem der Ansprüche 1 bis 5, wobei
**R**$^1$ Folgendes repräsentiert:

- $C_{1-4}$-Alkyl;
- Vinyl, 1-Methyl-vinyl oder 1-Propenyl;
- $C_{1-3}$-Fluoralkyl;
- $C_{3-6}$-Cycloalkyl, das unsubstituiert, mono- oder disubstituiert ist, wobei die Substituenten unabhängig aus $C_{1-3}$-Alkyl oder Fluor ausgewählt sind; oder
- Phenyl, das unsubstituiert oder mono-, di- oder trisubstituiert ist, wobei die Substituenten unabhängig aus $C_{1-3}$-Alkyl und Halogen ausgewählt sind;

oder ein pharmazeutisch akzeptables Salz davon.

7. Verbindung nach Anspruch 1, wobei

**A** Folgendes repräsentiert:

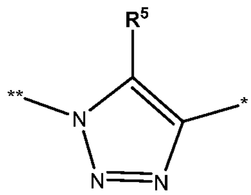

,

wobei ein Sternchen "*" die Anfügungsstelle am Kohlenstoff kennzeichnet, der die OH-Gruppe trägt; zwei Sternchen "**" die Anfügungsstelle an **R**$^2$-$(CH_2)_n$ kennzeichnen; und **R**$^5$ Wasserstoff oder $C_{1-3}$-Alkyl repräsentiert;
**n** 0 repräsentiert;
**R**$^1$ Folgendes repräsentiert:

- $C_{1-3}$-Alkyl;
- -C(**R**$^A$)=C(**R**$^B$)(**R**$^C$), wobei **R**$^A$, **R**$^B$ und **R**$^C$ unabhängig aus Wasserstoff und Methyl ausgewählt sind;
- $C_{1-3}$-Fluoralkyl;
- Phenyl, das unsubstituiert oder durch Halogen monosubstituiert ist; oder
- Cyclopropyl, das unsubstituiert oder durch Methyl oder Halogen monosubstituiert ist;

**R**$^2$ Folgendes repräsentiert:

- Phenyl, das unsubstituiert, mono-, di- oder trisubstituiert ist, wobei ein Substituent unabhängig Folgendes repräsentiert:

  ➢ -O**R**$^6$, wobei **R**$^6$ Wasserstoff, $C_{1-3}$-Alkyl, $C_{1-3}$-Alkoxy-$C_{2-4}$-alkyl, Hydroxy-$C_{2-5}$-alkyl, $C_{3-5}$-Cycloalkyl-$C_{1-3}$-alkyl, Oxetan-3-yl-$C_{1-3}$-alkyl, (Fluor-oxetan-3-yl)-$C_{1-3}$-alkyl, Hydroxy-$C_{3-5}$-cycloalkyl-$C_{1-3}$-alkyl, Tetrahydropyranyl-$C_{1-3}$-alkyl, $C_{1-3}$-Fluoralkoxy-$C_{2-3}$-alkyl oder Benzyl repräsentiert; oder
  ➢ -N**R**$^{N1}$**R**$^{N2}$, wobei **R**$^{N1}$ Wasserstoff repräsentiert; **R**$^{N2}$ Wasserstoff oder -(C=O)-**R**$^{CO}$ repräsentiert, wobei **R**$^{CO}$ $C_{1-4}$-Alkyl, $C_{1-4}$-Alkoxy oder Phenyl repräsentiert;

  wobei der genannte eine Substituent an dem genannten Phenyl in para-Position mit Bezug auf die Anfügungsstelle am Rest des Moleküls angefügt ist, und wobei jeder weitere Substituent des genannten Phenylrings, sofern vorhanden, unabhängig Halogen, Cyano oder Nitro repräsentiert;

oder ein pharmazeutisch akzeptables Salz davon.

**8.** Verbindung nach einem der Ansprüche 1 bis 7, wobei das asymmetrische Kohlenstoffatom, an das **R**$^2$-$(CH_2)_n$-**A**- angefügt ist, die in Formel (II) gezeigte absolute Konfiguration hat

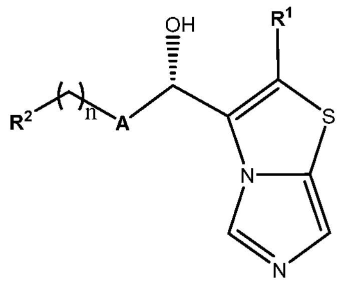

Formel (II)

oder ein pharmazeutisch akzeptables Salz davon.

**9.** Verbindung nach Anspruch 1, ausgewählt aus einer Gruppe bestehend aus:

(2-Methyl-imidazo[5,1-b]thiazol-3-yl)-(1-phenyl-1H-[1,2,3]triazol-4-yl)-methanol;
(2-Ethyl-imidazo[5,1-b]thiazol-3-yl)-(1-phenyl-1H-[1,2,3]triazol-4-yl)-methanol;
(2-Cyclopropyl-imidazo[5,1-b]thiazol-3-yl)-(1-phenyl-1H-[1,2,3]triazol-4-yl)-methanol;
(R)-(2-Cyclopropyl-imidazo[5,1-b]thiazol-3-yl)-(1-phenyl-1H-[1,2,3]triazol-4-yl)-methanol;
(2-Cyclopentyl-imidazo[5,1-b]thiazol-3-yl)-(1-phenyl-1H-[1,2,3]triazol-4-yl)-methanol;
(2-Cyclopropyl-imidazo[5,1-b]thiazol-3-yl)-(1-o-tolyl-1H-[1,2,3]triazol-4-yl)-methanol;
(2-Cyclopropyl-imidazo[5,1-b]thiazol-3-yl)-(1-m-tolyl-1H-[1,2,3]triazol-4-yl)-methanol;
(2-Cyclopropyl-imidazo[5,1-b]thiazol-3-yl)-(1-p-tolyl-1H-[1,2,3]triazol-4-yl)-methanol;
(2-Cyclopropyl-imidazo[5,1-b]thiazol-3-yl)-[1-(3-methoxy-phenyl)-1H-[1,2,3]triazol-4-yl]-methanol;
(R)-(2-Cyclopropyl-imidazo[5,1-b]thiazol-3-yl)-[1-(4-methoxy-phenyl)-1H-[1,2,3]triazol-4-yl]-methanol;
(2-Cyclopropyl-imidazo[5,1-b]thiazol-3-yl)-[1-(4-methoxy-phenyl)-1H-[1,2,3]triazol-4-yl]-methanol;
(2-Cyclopropyl-imidazo[5,1-b]thiazol-3-yl)-[1-(2-methoxy-phenyl)-1H-[1,2,3]triazol-4-yl]-methanol;
(1-Benzyl-1H-[1,2,3]triazol-4-yl)-(2-cyclopropyl-imidazo[5,1-b]thiazol-3-yl)-methanol;
3-{4-[(2-Cyclopropyl-imidazo[5,1-b]thiazol-3-yl)-hydroxy-methyl]-[1,2,3]triazol-1-yl}-benzonitril;
4-{4-[(2-Cyclopropyl-imidazo[5,1-b]thiazol-3-yl)-hydroxy-methyl]-[1,2,3]triazol-1-yl}-benzonitril;
(2-Cyclopropyl-imidazo[5,1-b]thiazol-3-yl)-[1-(2-trifluormethyl-phenyl)-1H-[1,2,3]triazol-4-yl]-methanol;
(2-Cyclopropyl-imidazo[5,1-b]thiazol-3-yl)-[1-(3-trifluormethyl-phenyl)-1H-[1,2,3]triazol-4-yl]-methanol;
(2-Cyclopropyl-imidazo[5,1-b]thiazol-3-yl)-[1-(4-trifluormethyl-phenyl)-1H-[1,2,3]triazol-4-yl]-methanol;
1-(4-{4-[(2-Cyclopropyl-imidazo[5,1-b]thiazol-3-yl)-hydroxy-methyl]-[1,2,3]triazol-1-yl}-phenyl)-ethanon;
(2-Cyclopropyl-imidazo[5,1-b]thiazol-3-yl)-[1-(4-cyclopropylmethoxy-phenyl)-1H-[1,2,3]triazol-4-yl]-methanol;
(2-Cyclopropyl-imidazo[5,1-b]thiazol-3-yl)-[1-(4-ethoxy-phenyl)-1H-[1,2,3]triazol-4-yl]-methanol;
(4-{4-[(2-Cyclopropyl-imidazo[5,1-b]thiazol-3-yl)-hydroxy-methyl]-[1,2,3]triazol-1-yl}-phenyl)-carbaminsäuremethylester;
(2-Cyclopropyl-imidazo[5,1-b]thiazol-3-yl)-[1-(4-isopropoxy-phenyl)-1H-[1,2,3]triazol-4-yl]-methanol;
3-{4-[(R)-(2-Cyclopropyl-imidazo[5,1-b]thiazol-3-yl)-hydroxy-methyl]-[1,2,3]triazol-1-yl}-benzonitril;
[1-(4-Methoxy-phenyl)-1H-[1,2,3]triazol-4-yl]-[2-(1-methyl-cyclopropyl)-imidazo[5,1-b]thiazol-3-yl]-methanol;
[1-(4-Chlor-phenyl)-1H-[1,2,3]triazol-4-yl]-(2-cyclopropyl-imidazo[5,1-b]thiazol-3-yl)-methanol;
(2-Cyclopropyl-imidazo[5,1-b]thiazol-3-yl)-{1-[4-(1-hydroxymethyl-cyclopropyl)-phenyl]-1H-[1,2,3]triazol-4-yl}-methanol;
(2-Cyclopropyl-imidazo[5,1-b]thiazol-3-yl)-[1-(4-morpholin-4-yl-phenyl)-1H-[1,2,3]triazol-4-yl]-methanol;
(2-Cyclopropyl-imidazo[5,1-b]thiazol-3-yl)-[1-(4-morpholin-4-ylmethyl-phenyl)-1H-[1,2,3]triazol-4-yl]-methanol;
(2-Cyclopropyl-imidazo[5,1-b]thiazol-3-yl)-[1-(4-trifluormethoxy-phenyl)-1H-[1,2,3]triazol-4-yl]-methanol;
(2-Cyclopropyl-imidazo[5,1-b]thiazol-3-yl)-(1-pyrimidin-5-yl-1H-[1,2,3]triazol-4-yl)-methanol;
(2-Cyclopropyl-imidazo[5,1-b]thiazol-3-yl)-(1-pyridin-2-yl-1H-[1,2,3]triazol-4-yl)-methanol;
(2-Cyclopropyl-imidazo[5,1-b]thiazol-3-yl)-(1-pyridin-3-yl-1H-[1,2,3]triazol-4-yl)-methanol;
(2-Cyclopropyl-imidazo[5,1-b]thiazol-3-yl)-(1-pyridin-4-yl-1H-[1,2,3]triazol-4-yl)-methanol;
(2-Cyclopropyl-imidazo[5,1-b]thiazol-3-yl)-[1-(6-ethoxy-pyridin-3-yl)-1H-[1,2,3]triazol-4-yl]-methanol;
(2-Cyclopropyl-imidazo[5,1-b]thiazol-3-yl)-[1-(1-methyl-1H-pyrazol-4-yl)-1 H-[1 ,2,3]triazol-4-yl]-methanol;
(2-Cyclopropyl-imidazo[5,1-b]thiazol-3-yl)-[1-(1H-pyrazol-4-yl)-1H-[1,2,3]triazol-4-yl]-methanol;

(2-Cyclopropyl-imidazo[5,1-b]thiazol-3-yl)-[1-(1H-indol-6-yl)-1H-[1,2,3]triazol-4-yl]-methanol;
(2-Cyclopropyl-imidazo[5,1-b]thiazol-3-yl)-[1-(1H-indol-4-yl)-1H-[1,2,3]triazol-4-yl]-methanol;
(R)-(2-Cyclopropyl-imidazo[5,1-b]thiazol-3-yl)-[1-(1H-indol-5-yl)-1H-[1,2,3]triazol-4-yl]-methanol;
(2-Cyclopropyl-imidazo[5,1-b]thiazol-3-yl)-[1-(1H-indol-5-yl)-1H-[1,2,3]triazol-4-yl]-methanol;
(2-Cyclopropyl-imidazo[5,1-b]thiazol-3-yl)-[1-(1H-indol-7-yl)-1H-[1,2,3]triazol-4-yl]-methanol;
(2-Cyclopropyl-imidazo[5,1-b]thiazol-3-yl)-(1-isochinolin-5-yl-1H-[1,2,3]triazol-4-yl)-methanol;
(2-Cyclopropyl-imidazo[5,1-b]thiazol-3-yl)-(1-chinolin-5-yl-1H-[1,2,3]triazol-4-yl)-methanol;
(2-Cyclopropyl-imidazo[5,1-b]thiazol-3-yl)-(1-isochinolin-8-yl-1H-[1,2,3]triazol-4-yl)-methanol;
(2-Cyclopropyl-imidazo[5,1-b]thiazol-3-yl)-(1-chinolin-4-yl-1H-[1,2,3]triazol-4-yl)-methanol;
(2-Cyclopropyl-imidazo[5,1-b]thiazol-3-yl)-(1-isochinolin-4-yl-1H-[1,2,3]triazol-4-yl)-methanol; und
(2-Cyclopropyl-imidazo[5,1-b]thiazol-3-yl)-[1-(2,3-dihydro-benzo[1,4]dioxin-6-yl)-1H-[1,2,3]triazol-4-yl]-methanol;

oder ein pharmazeutisch akzeptables Salz davon.

**10.** Verbindung nach Anspruch 1, ausgewählt aus einer Gruppe bestehend aus:

[1-(4-Benzyloxy-phenyl)-1*H*-[1,2,3]triazol-4-yl]-(2-cyclopropyl-imidazo[5,1-b]thiazol-3-yl)-methanol;
[1-(6-Ethoxy-pyridin-3-yl)-1*H*-[1,2,3]triazol-4-yl]-(2-ethyl-imidazo[5,1-*b*]thiazol-3-yl)-methanol;
(2-Ethyl-imidazo[5,1-*b*]thiazol-3-yl)-[1-(4-morpholin-4-yl-phenyl)-1*H*-[1,2,3]triazol-4-yl]-methanol;
(2-Ethyl-imidazo[5,1-*b*]thiazol-3-yl)-{1-[4-(1-hydroxymethyl-cyclopropyl)-phenyl]-1*H*-[1,2,3]triazol-4-yl}-methanol;
(4-{4-[(2-Ethyl-imidazo[5,1-*b*]thiazol-3-yl)-hydroxy-methyl]-[1,2,3]triazol-1-yl}-phenyl)-carbaminsäuremethylester;
(2-Ethyl-imidazo[5,1-*b*]thiazol-3-yl)-[1-(1H-indol-5-yl)-1*H*-[1,2,3]triazol-4-yl]-methanol;
3-{4-[(2-Ethyl-imidazo[5,1-*b*]thiazol-3-yl)-hydroxy-methyl]-[1,2,3]triazol-1-yl}-benzonitril;
(2-Ethyl-imidazo[5,1-*b*]thiazol-3-yl)-(1-isochinolin-4-yl-1*H*-[1,2,3]triazol-4-yl)-methanol;
(1-Benzyl-1*H*-[1,2,3]triazol-4-yl)-(2-ethyl-imidazo[5,1-*b*]thiazol-3-yl)-methanol;
[1-(4-Cyclopropylmethoxy-phenyl)-1*H*-[1,2,3]triazol-4-yl]-(2-ethyl-imidazo[5,1-*b*]thiazol-3-yl)-methanol;
[1-(6-Ethoxy-pyridin-3-yl)-1*H*-[1,2,3]triazol-4-yl]-(2-methyl-imidazo[5,1-*b*]thiazol-3-yl)-methanol;
(2-Methyl-imidazo[5,1-*b*]thiazol-3-yl)-[1-(4-morpholin-4-yl-phenyl)-1*H*-[1,2,3]triazol-4-yl]-methanol;
{1-[4-(1-Hydroxymethyl-cyclopropyl)-phenyl]-1*H*-[1,2,3]triazol-4-yl}-(2-methyl-imidazo[5,1-*b*]thiazol-3-yl)-methanol;
(4-{4-[Hydroxy-(2-methyl-imidazo[5,1-*b*]thiazol-3-yl)-methyl]-[1,2,3]triazol-1-yl}-phenyl)-carbaminsäuremethylester;
[1-(1*H*-Indol-5-yl)-1*H*-[1,2,3]triazol-4-yl]-(2-methyl-imidazo[5,1-*b*]thiazol-3-yl)-methanol;
3-{4-[Hydroxy-(2-methyl-imidazo[5,1-*b*]thiazol-3-yl)-methyl]-[1,2,3]triazol-1-yl}-benzonitril;
(1-Isochinolin-4-yl-1*H*-[1,2,3]triazol-4-yl)-(2-methyl-imidazo[5,1-*b*]thiazol-3-yl)-methanol;
(1-Benzyl-1*H*-[1,2,3]triazol-4-yl)-(2-methyl-imidazo[5,1-b]thiazol-3-yl)-methanol;
[1-(4-Cyclopropylmethoxy-phenyl)-1*H*-[1,2,3]triazol-4-yl]-(2-methyl-imidazo[5,1-*b*]thiazol-3-yl)-methanol;
[2-(3-Fluor-phenyl)-imidazo[5,1-*b*]thiazol-3-yl]-[1-(4-methoxy-phenyl)-1*H*-[1,2,3]triazol-4-yl]-methanol;
(2-Cyclopropyl-imidazo[5,1-*b*]thiazol-3-yl)-[1-(2-ethoxy-pyrimidin-5-yl)-1*H*-[1,2,3]triazol-4-yl]-methanol;
(2-Cyclopropyl-imidazo[5,1-*b*]thiazol-3-yl)-[1-(5,6-dimethoxy-pyridin-2-yl)-1*H*-[1,2,3]triazol-4-yl]-methanol;
(2-Cyclopropyl-imidazo[5,1-*b*]thiazol-3-yl)-[1-(2-methoxy-pyrimidin-5-yl)-1*H*-[1,2,3]triazol-4-yl]-methanol;
4-{4-[(2-Cyclopropyl-imidazo[5,1-b]thiazol-3-yl)-hydroxy-methyl]-[1,2,3]triazol-1-yl}-phenol;
(2-Cyclopropyl-imidazo[5,1-*b*]thiazol-3-yl)-[1-(6-methoxy-pyridin-3-yl)-1*H*-[1,2,3]triazol-4-yl]-methanol;
5-{4-[(2-Cyclopropyl-imidazo[5,1-b]thiazol-3-yl)-hydroxy-methyl]-[1,2,3]triazol-1-yl}-2-methoxy-nicotinsäuremethylester;
(2-Cyclopropyl-imidazo[5,1-*b*]thiazol-3-yl)-[1-(6-methoxy-5-methyl-pyridin-3-yl)-1*H*-[1,2,3]triazol-4-yl]-methanol;
(2-Cyclopropyl-imidazo[5,1-*b*]thiazol-3-yl)-[1-(3,4-dihydro-2*H*-pyrano[2,3-b]pyridin-6-yl)-1*H*-[1,2,3]triazol-4-yl]-methanol;
[1-(5-Chlor-6-isopropoxy-pyridin-3-yl)-1*H*-[1,2,3]triazol-4-yl]-(2-cyclopropyl-imidazo[5,1-*b*]thiazol-3-yl)-methanol;
[1-(5-Chlor-6-ethoxy-pyridin-3-yl)-1*H*-[1,2,3]triazol-4-yl]-(2-cyclopropyl-imidazo[5,1-b]thiazol-3-yl)-methanol;
(5-{4-[(2-Cyclopropyl-imidazo[5,1-b]thiazol-3-yl)-hydroxy-methyl]-[1,2,3]triazol-1-yl}-pyridin-2-yloxy)-essigsäuremethylester;
(2-Cyclopropyl-imidazo[5,1-*b*]thiazol-3-yl)-[1-(6-ethoxy-5-trifluormethyl-pyridin-3-yl)-1*H*-[1,2,3]triazol-4-yl]-methanol;
[1-(6-Benzyloxy-pyridin-3-yl)-1*H*-[1,2,3]triazol-4-yl]-(2-cyclopropyl-imidazo[5,1-*b*]thiazol-3-yl)-methanol;
(2-Cyclopropyl-imidazo[5,1-b]thiazol-3-yl)-{1-[6-(2,2,2-trifluor-ethoxy)-pyridin-3-yl]-1*H*-[1,2,3]triazol-4-yl}-methanol;
(2-Cyclopropyl-imidazo[5,1-*b*]thiazol-3-yl)-[1-(5-fluor-6-methoxy-pyridin-3-yl)-1*H*-[1,2,3]triazol-4-yl]-methanol;
(2-Cyclopropyl-imidazo[5,1-*b*]thiazol-3-yl)-[1-(5,6-dimethoxy-pyridin-3-yl)-1*H*-[1,2,3]triazol-4-yl]-methanol;
(2-Cyclopropyl-imidazo[5,1-*b*]thiazol-3-yl)-[1-(2,6-dimethoxy-pyridin-3-yl)-1*H*-[1,2,3]triazol-4-yl]-methanol;
(2-Cyclopropyl-imidazo[5,1-*b*]thiazol-3-yl)-[1-(6-isopropoxy-pyridin-3-yl)-1*H*-[1,2,3]triazol-4-yl]-methanol;
[1-(2-Cyclobutoxy-pyrimidin-5-yl)-1*H*-[1,2,3]triazol-4-yl]-(2-cyclopropyl-imidazo[5,1-*b*]thiazol-3-yl)-methanol;
(2-Cyclopropyl-imidazo[5,1-*b*]thiazol-3-yl)-[1-(2,4-dimethoxy-pyrimidin-5-yl)-1*H*-[1,2,3]triazol-4-yl]-methanol;
[1-(2-Benzyloxy-pyrimidin-5-yl)-1*H*-[1,2,3]triazol-4-yl]-(2-cyclopropyl-imidazo[5,1-*b*]thiazol-3-yl)-methanol;
(2-Cyclopropyl-imidazo[5,1-*b*]thiazol-3-yl)-[1-(3,6-dimethoxy-pyridazin-4-yl)-1*H*-[1,2,3]triazol-4-yl]-methanol;
(2-Cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol;
(2-Cyclopropyl-imidazo[5,1-*b*]thiazol-3-yl)-{1-[4-(oxetan-3-ylmethoxy)-phenyl]-1*H*-[1,2,3]triazol-4-yl}-methanol;
(2-Cyclopropyl-imidazo[5,1-*b*]thiazol-3-yl)-{1-[4-(2-piperidin-1-yl-ethoxy)-phenyl]-1*H*-[1,2,3]triazol-4-yl}-methanol;
(2-Cyclopropy-imidazo[5,1-*b*]thiazol-3-yl)-{1-[4-(2-methoxy-ethoxy)-phenyl]-1*H*-[1,2,3]triazol-4-yl}-methanol;
[2-(1-Fluor-cyclopropyl)-imidazo[5,1-*b*]thiazol-3-yl]-[1-(4-methoxy-phenyl)-1*H*-[1,2,3]triazol-4-yl]-methanol;
*N*-(4-{4-[(2-Cyclopropyl-imidazo[5,1-*b*]thiazol-3-yl)-hydroxy-methyl]-[1,2,3]triazol-1-yl}-phenyl)-acetamid;
(2-Methyl-imidazo[5,1-*b*]thiazol-3-yl)-(1-phenyl-1*H*-pyrazol-4-yl)-methanol;
[1-(4-Methoxy-phenyl)-1*H*-pyrazol-4-yl]-(2-methyl-imidazo[5,1-*b*]thiazol-3-yl)-methanol;
(2-Methyl-imidazo[5,1-*b*]thiazol-3-yl)-(5-methyl-1-phenyl-1*H*-pyrazol-4-yl)-methanol;
(2-Cyclopropyl-imidazo[5,1-*b*]thiazol-3-yl)-[1-(4-methoxy-phenyl)-1*H*-pyrazol-4-yl]-methanol;
(2-Cyclopropyl-imidazo[5,1-*b*]thiazol-3-yl)-(1-phenyl-1*H*-pyrazol-4-yl)-methanol;
(S)-(2-Cyclopropyl-imidazo[5,1-*b*]thiazol-3-yl)-(1-phenyl-1*H*-pyrazol-4-yl)-methanol;
(2-Cyclopropyl-imidazo[5,1-*b*]thiazol-3-yl)-(5-methyl-1-phenyl-1H-[1,2,3]triazol-4-yl)-methanol;
4-{4-[(2-Cyclopropyl-imidazo[5,1-*b*]thiazol-3-yl)-hydroxy-methyl]-[1,2,3]triazol-1-yl}-*N*-(2-methoxy-ethyl)-benzamid;
(4-{4-[(2-Cyclopropyl-imidazo[5,1-b]thiazol-3-yl)-hydroxy-methyl]-[1,2,3]triazol-1-yl}-phenyl)-morpholin-4-yl-methanon;
Morpholin-4-carbonsäure (4-{4-[(2-cyclopropyl-imidazo[5,1-*b*]thiazol-3-yl)-hydroxy-methyl]-[1,2,3]triazol-1-yl}-phenyl)-amid;
*N*-Cyclopropyl-4-{4-[(2-cyclopropyl-imidazo[5,1-*b*]thiazol-3-yl)-hydroxy-methyl]-[1,2,3]triazol-1-yl}-benzamid;
4-{4-[(2-Cyclopropyl-imidazo[5,1-*b*]thiazol-3-yl)-hydroxy-methyl]-[1,2,3]triazol-1-yl}-N-(3-methoxy-propyl)-benzamid;
4-{4-[(2-Cyclopropyl-imidazo[5,1-*b*]thiazol-3-yl)-hydroxy-methyl]-[1,2,3]triazol-1-yl}-*N*-ethyl-benzamid;
*N*-(2-Cyano-ethyl)-4-{4-[(2-cyclopropyl-imidazo[5,1-*b*]thiazol-3-yl)-hydroxy-methyl]-[1,2,3]triazol-1-yl}-benzamid;
(4-{4-[(2-Cyclopropyl-imidazo[5,1-b]thiazol-3-yl)-hydroxy-methyl]-[1,2,3]triazol-1-yl}-phenyl)-harnstoff;
*N*-(4-{4-[(2-Ethyl-imidazo[5,1-*b*]thiazol-3-yl)-hydroxy-methyl]-[1,2,3]triazol-1-yl}-phenyl)-acetamid;
4-{4-[(2-Ethyl-imidazo[5,1-*b*]thiazol-3-yl)-hydroxy-methyl]-[1,2,3]triazol-1-yl}-*N*-methyl-benzamid;
5-{4-[(2-Ethyl-imidazo[5,1-*b*]thiazol-3-yl)-hydroxy-methyl]-[1,2,3]triazol-1-yl}-1,3-dihydro-benzoimidazol-2-on;
1-Ethyl-3-(4-{4-[(2-ethyl-imidazo[5,1-*b*]thiazol-3-yl)-hydroxy-methyl]-[1,2,3]triazol-1-yl}-2-methoxy-phenyl)-harnstoff;
4-{4-[(2-Ethyl-imidazo[5,1-*b*]thiazol-3-yl)-hydroxy-methyl]-[1,2,3]triazol-1-yl}-*N*,*N*-dimethyl-benzamid;
4-{4-[(2-Ethyl-imidazo[5,1-*b*]thiazol-3-yl)-hydroxy-methyl]-[1,2,3]triazol-1-yl}-*N*-(3-hydroxy-propyl)-benzamid;
4-{4-[(2-Ethyl-imidazo[5,1-*b*]thiazol-3-yl)-hydroxy-methyl]-[1,2,3]triazol-1-yl}-benzamid;
(4-{4-[(2-Ethyl-imidazo[5,1-*b*]thiazol-3-yl)-hydroxy-methyl]-[1,2,3]triazol-1-yl}-phenyl)-morpholin-4-yl-methanon;
4-{4-[(2-Ethyl-imidazo[5,1-*b*]thiazol-3-yl)-hydroxy-methyl]-[1,2,3]triazol-1-yl}-*N*-(2-hydroxy-ethyl)-benzamid;
*N*-(2-Dimethylamino-ethyl)-4-{4-[(2-ethyl-imidazo[5,1-*b*]thiazol-3-yl)-hydroxy-methyl]-[1,2,3]triazol-1-yl}-benzamid;
*N*-(4-{4-[(2-Ethyl-imidazo[5,1-*b*]thiazol-3-yl)-hydroxy-methyl]-[1,2,3]triazol-1-yl}-phenyl)-2,2-dimethyl-propionamid;
Morpholin-4-carbonsäure (4-{4-[(2-ethyl-imidazo[5,1-b]thiazol-3-yl)-hydroxy-methyl]-[1,2,3]triazol-1-yl}-phenyl)-amid;

1-Butyl-3-(4-{4-[(2-ethyl-imidazo[5,1-*b*]thiazol-3-yl)-hydroxy-methyl]-[1,2,3]triazol-1-yl}-phenyl)-harnstoff;
4-{4-[(2-Ethyl-imidazo[5,1-*b*]thiazol-3-yl)-hydroxy-methyl]-[1,2,3]triazol-1-yl}-*N*-furan-2-ylmethyl-benzamid;
*N*-Ethyl-4-{4-[(2-ethyl-imidazo[5,1-*b*]thiazol-3-yl)-hydroxy-methyl]-[1,2,3]triazol-1-yl}-*N*-methyl-benzamid;
*N*-Cyclopropyl-4-{4-[(2-ethyl-imidazo[5,1-*b*]thiazol-3-yl)-hydroxy-methyl]-[1,2,3]triazol-1-yl}-benzamid;
4-{4-[(2-Ethyl-imidazo[5,1-*b*]thiazol-3-yl)-hydroxy-methyl]-[1,2,3]triazol-1-yl}-*N*-(3-methoxy-propyl)-benzamid;
*N*-(4-{4-[(2-Ethyl-imidazo[5,1-*b*]thiazol-3-yl)-hydroxy-methyl]-[1,2,3]triazol-1-yl}-phenyl)-isobutyramid;
*N*-Ethyl-4-{4-[(2-ethyl-imidazo[5,1-*b*]thiazol-3-yl)-hydroxy-methyl]-[1,2,3]triazol-1-yl}-benzamid;
*N*-Allyl-4-{4-[(2-ethyl-imidazo[5,1-b]thiazol-3-yl)-hydroxy-methyl]-[1,2,3]triazol-1-yl}-benzamid;
*N*-(2-Cyano-ethyl)-4-{4-[(2-ethyl-imidazo[5,1-*b*]thiazol-3-yl)-hydroxy-methyl]-[1,2,3]triazol-1-yl}-benzamid;
(4-{4-[(2-Ethyl-imidazo[5,1-*b*]thiazol-3-yl)-hydroxy-methyl]-[1,2,3]triazol-1-yl}-phenyl)-harnstoff;
(4-{4-[(2-Cyclopropyl-imidazo[5,1-*b*]thiazol-3-yl)-hydroxy-methyl]-pyrazol-1-yl}-phenyl)-carbaminsäuremethylester;
(*R*)-(2-Cyclopropyl-imidazo[5,1-*b*]thiazol-3-yl)-[1-(4-cyclopropylmethoxy-phenyl)-1*H*-[1,2,3]triazol-4-yl]-methanol;
(2-Cyclopropyl-imidazo[5,1-*b*]thiazol-3-yl)-{1-[4-(tetrahydro-furan-2-ylmethoxy)-phenyl]-1*H*-[1,2,3]triazol-4-yl}-methanol;
(1-Phenyl-1*H*-[1,2,3]triazol-4-yl)-(2-trifluormethyl-imidazo[5,1-*b*]thiazol-3-yl)-methanol;
[1-(4-Methoxy-phenyl)-1*H*-[1,2,3]triazol-4-yl]-(2-trifluormethyl-imidazo[5,1-*b*]triazol-3-yl)-methanol;
5-{4-[(2-Cyclopropyl-imidazo[5,1-*b*]thiazol-3-yl)-hydroxy-methyl]-[1,2,3]triazol-1-yl}-2-methoxy-benzonitril;
2-(4-{4-[(2-Cyclopropyl-imidazo[5,1-*b*]thiazol-3-yl)-hydroxy-methyl]-[1,2,3]triazol-1-yl}-phenoxy)-ethanol;
3-(4-{4-[(2-Cyclopropyl-imidazo[5,1-*b*]thiazol-3-yl)-hydroxy-methyl]-[1,2,3]triazol-1-yl}-phenoxy)-propan-1-ol;
3-(4-{4-[(R)-(2-Cyclopropyl-imidazo[5,1-*b*]thiazol-3-yl)-hydroxy-methyl]-[1,2,3]triazol-1-yl}-phenoxy)-propan-1-ol;
2-[2-(4-{4-[(2-Cyclopropyl-imidazo[5,1-*b*]thiazol-3-yl)-hydroxy-methyl]-[1,2,3]triazol-1-yl}-phenoxy)-ethoxy]-ethanol;
1-(4-{4-[(2-Cyclopropyl-imidazo[5,1-*b*]thiazol-3-yl)-hydroxy-methyl]-[1,2,3]triazol-1-yl}-phenoxy)-2-methyl-propan-2-ol;
4-(4-{4-[(2-Cyclopropyl-imidazo[5,1-*b*]thiazol-3-yl)-hydroxy-methyl]-[1,2,3]triazol-1-yl}-phenoxy)-2-methyl-butan-2-ol;
3-(4-{4-[(2-Cyclopropyl-imidazo[5,1-*b*]thiazol-3-yl)-hydroxy-methyl]-[1,2,3]triazol-1-yl}-phenoxy)-2,2-dimethyl-propan-1-ol;
1-[2-(4-{4-[(2-Cyclopropyl-imidazo[5,1-*b*]thiazol-3-yl)-hydroxy-methyl]-[1,2,3]triazol-1-yl}-phenoxy)-ethyl]-cyclopropanol;
1-[2-(4-{4-[(R)-(2-Cyclopropyl-imidazo[5,1-*b*]thiazol-3-yl)-hydroxy-methyl]-[1,2,3]triazol-1-yl}-phenoxy)-ethyl]-cyclopropanol;
(2-Cyclopropyl-imidazo[5,1-*b*]thiazol-3-yl)-{1-[4-(tetrahydro-pyran-2-ylmethoxy)-phenyl]-1*H*-[1,2,3]triazol-4-yl}-methanol;
(2-Cyclopropyl-imidazo[5,1-*b*]thiazol-3-yl)-{1-[4-(3-methyl-oxetan-3-ylmethoxy)-phenyl]-1*H*-[1,2,3]triazol-4-yl}-methanol;
(2-Cyclopropyl-imidazo[5,1-*b*]thiazol-3-yl)-{1-[4-(3-fluor-oxetan-3-ylmethoxy)-phenyl]-1*H*-[1,2,3]triazol-4-yl}-methanol;
(*R*)-(2-Cyclopropyl-imidazo[5,1-*b*]thiazol-3-yl)-{1-[4-(3-fluor-oxetan-3-ylmethoxy)-phenyl]-1*H*-[1,2,3]triazol-4-yl}-methanol;
(2-Cyclopropyl-imidazo[5,1-*b*]thiazol-3-yl)-{1-[4-(1-methyl-piperidin-3-ylmethoxy)-phenyl]-1*H*-[1,2,3]triazol-4-yl}-methanol;
(2-Cyclopropyl-imidazo[5,1-*b*]thiazol-3-yl)-{1-[4-(1,1-dioxo-hexahydro-1l6-thiopyran-4-ylmethoxy)-phenyl]-1*H*-[1,2,3]triazol-4-yl}-methanol;
2-Chlor-4-{4-[(2-cyclopropyl-imidazo[5,1-*b*]thiazol-3-yl)-hydroxy-methyl]-[1,2,3]triazol-1-yl}-phenol;
2-Chlor-4-{4-[(*R*)-(2-cyclopropyl-imidazo[5,1-*b*]thiazol-3-yl)-hydroxy-methyl]-[1,2,3]triazol-1-yl}-phenol;
4-{4-[(2-Cyclopropyl-imidazo[5,1-*b*]thiazol-3-yl)-hydroxy-methyl]-[1,2,3]triazol-1-yl}-2,6-difluor-phenol;
4-{4-[(2-Cyclopropyl-imidazo[5,1-*b*]thiazol-3-yl)-hydroxy-methyl]-[1,2,3]triazol-1-yl}-2-fluor-phenol;
(4-{4-[(2-Cyclopropyl-imidazo[5,1-*b*]thiazol-3-yl)-hydroxy-methyl]-[1,2,3]triazol-1-yl}-3-fluor-phenyl)-carbaminsäure-tert-butylester;
Biphenyl-4-yl-(2-cyclopropyl-imidazo[5,1-*b*]thiazol-3-yl)-methanol;
Biphenyl-3-yl-(2-cyclopropyl-imidazo[5,1-*b*]thiazol-3-yl)-methanol;
(2-Methyl-imidazo[5,1-*b*]thiazol-3-yl)-(5-phenyl-thiophen-2-yl)-methanol;
(2-Methyl-imidazo[5,1-*b*]thiazol-3-yl)-(2-phenyl-thiazol-5-yl)-methanol;
(2-Methyl-imidazo[5,1-*b*]thiazol-3-yl)-(3-phenyl-isoxazol-5-yl)-methanol;

(2-Cyclopropyl-imidazo[5,1-*b*]thiazol-3-yl)-(5-phenyl-[1,3,4]oxadiazol-2-yl)-methanol;
(2-Cyclopropyl-imidazo[5,1-*b*]thiazol-3-yl)-(2-phenyl-2H-[1,2,3]triazol-4-yl)-methanol;
3-{5-[(2-Cyclopropyl-imidazo[5,1-*b*]thiazol-3-yl)-hydroxy-methyl]-thiophen-2-yl}-benzonitril;
(2-Cyclopropyl-imidazo[5,1-*b*]thiazol-3-yl)-(5-pyridin-3-yl-thiophen-2-yl)-methanol;
(2-Cyclopropyl-imidazo[5,1-*b*]thiazol-3-yl)-[5-(4-methoxy-phenyl)-thiophen-2-yl]-methanol;
(2-Cyclopropyl-imidazo[5,1-*b*]thiazol-3-yl)-[5-(4-trifluormethyl-phenyl)-thiophen-2-yl]-methanol;
(2-Cyclopropyl-imidazo[5,1-*b*]thiazol-3-yl)-(3-phenyl-[1,2,4]oxadiazol-5-yl)-methanol;
(2-Cyclopropyl-imidazo[5,1-*b*]thiazol-3-yl)-(5-phenyl-thiophen-2-yl)-methanol;
(2-Cyclopropyl-imidazo[5,1-*b*]thiazol-3-yl)-[5-(3-trifluormethyl-phenyl)-thiophen-2-yl]-methanol;
(2-Cyclopropyl-imidazo[5,1-*b*]thiazol-3-yl)-[5-(2-methoxy-phenyl)-thiophen-2-yl]-methanol;
(2-Cyclopropyl-imidazo[5,1-*b*]thiazol-3-yl)-[5-(3-methoxy-phenyl)-thiophen-2-yl]-methanol;
(2-Cyclopropyl-imidazo[5,1-*b*]thiazol-3-yl)-(5-pyridin-4-yl-thiophen-2-yl)-methanol;
(2-Cyclopropyl-imidazo[5,1-*b*]thiazol-3-yl)-[5-(2-methyl-2*H*-pyrazol-3-yl)-thiophen-2-yl]-methanol;
(2-Cyclopropyl-imidazo[5,1-*b*]thiazol-3-yl)-[5-(2-trifluormethyl-phenyl)-thiophen-2-yl]-methanol;
(2-Cyclopropyl-imidazo[5,1-*b*]thiazol-3-yl)-(5-phenyl-thiazol-2-yl)-methanol;
2-{5-[(2-Cyclopropyl-imidazo[5,1-*b*]thiazol-3-yl)-hydroxy-methyl]-thiophen-2-yl}-benzonitril;
4-{5-[(2-Cyclopropyl-imidazo[5,1-*b*]thiazol-3-yl)-hydroxy-methyl]-thiophen-2-yl}-benzonitril;
(2-Cyclopropyl-imidazo[5,1-*b*]thiazol-3-yl)-[5-(3-methyl-3H-[1,2,3]triazol-4-yl)-thiophen-2-yl]-methanol;
(2-Cyclopropyl-imidazo[5,1-*b*]thiazol-3-yl)-(5-phenyl-[1,3,4]thiadiazol-2-yl)-methanol;
(2-Cyclopropyl-imidazo[5,1-*b*]thiazol-3-yl)-[5-(1H-indol-5-yl)-thiophen-2-yl]-methanol;
(2-Cyclopropyl-imidazo[5,1-*b*]thiazol-3-yl)-(5-isochinolin-4-yl-thiophen-2-yl)-methanol;
(2-Cyclopropyl-imidazo[5,1-*b*]thiazol-3-yl)-(1-phenyl-1H-imidazol-4-yl)-methanol;
[2-(cis-2-Fluor-cyclopropyl)-imidazo[5,1-b]thiazol-3-yl]-[1-(4-methoxy-phenyl)-1H-[1,2,3]triazol-4-yl]-methanol;
und [2-(trans-2-Fluor-cyclopropyl)-imidazo[5,1-*b*]thiazol-3-yl]-[1-(4-methoxy-phenyl)-1*H*-[1,2,3]triazol-4-yl]-methanol;

oder ein pharmazeutisch akzeptables Salz davon.

**11.** Verbindung nach Anspruch 1, ausgewählt aus einer Gruppe bestehend aus:

(4-{4-[(*R*)-(2-Ethyl-imidazo[5,1-*b*]thiazol-3-yl)-hydroxy-methyl]-[1,2,3]triazol-1-yl}-phenyl)-carbaminsäuremethylester;
(*R*)-(2-Cyclopropyl-imidazo[5,1-*b*]thiazol-3-yl)-{1-[4-(tetrahydro-pyran-4-ylmethoxy)-phenyl]-1*H*-[1,2,3]triazol-4-yl}-methanol;
1-(4-{4-[(*R*)-(2-Cyclopropyl-imidazo[5,1-*b*]thiazol-3-yl)-hydroxy-methyl]-[1,2,3]triazol-1-yl}-phenoxy)-2-methyl-propan-2-ol;
(*R*)-(2-Cyclopropyl-imidazo[5,1-*b*]thiazol-3-yl)-(5-methyl-1-phenyl-1*H*-[1,2,3]triazol-4-yl)-methanol;
6-{4-[(2-Cyclopropyl-imidazo[5,1-*b*]thiazol-3-yl)-hydroxy-methyl]-[1,2,3]triazol-1-yl}-3H-benzooxazol-2-on;
(4-{4-[(2-Cyclopropyl-imidazo[5,1-b]thiazol-3-yl)-hydroxy-methyl]-[1,2,3]triazol-1-yl}-phenyl)-carbaminsäure-tert-butylester;
6-{4-[(2-Ethyl-imidazo[5,1-*b*]thiazol-3-yl)-hydroxy-methyl]-[1,2,3]triazol-1-yl}-3*H*-benzooxazol-2-on;
4-{4-[(2-Cyclopropyl-imidazo[5,1-*b*]thiazol-3-yl)-hydroxy-methyl]-[1,2,3]triazol-1-yl}-*N*-methyl-benzamid;
1-(4-{4-[(2-Cyclopropyl-imidazo[5,1-*b*]thiazol-3-yl)-hydroxy-methyl]-[1,2,3]triazol-1-yl}-2-methoxy-phenyl)-3-ethyl-harnstoff;
4-{4-[(2-Cyclopropyl-imidazo[5,1-*b*]thiazol-3-yl)-hydroxy-methyl]-[1,2,3]triazol-1-yl}-*N*-(3-hydroxy-propyl)-benzamid;
4-{4-[(2-Cyclopropyl-imidazo[5,1-*b*]thiazol-3-yl)-hydroxy-methyl]-[1,2,3]triazol-1-yl}-benzamid;
4-{4-[(2-Cyclopropyl-imidazo[5,1-*b*]thiazol-3-yl)-hydroxy-methyl]-[1,2,3]triazol-1-yl}-N-(2-hydroxy-ethyl)-benzamid;
*N*-(4-{4-[(2-Cyclopropyl-imidazo[5,1-*b*]thiazol-3-yl)-hydroxy-methyl]-[1,2,3]triazol-1-yl}-phenyl)-2,2-dimethyl-propionamid;
1-Butyl-3-(4-{4-[(2-cyclopropyl-imidazo[5,1-*b*]thiazol-3-yl)-hydroxy-methyl]-[1,2,3]triazol-1-yl}-phenyl)-harnstoff;
*N*-(4-{4-[(2-Cyclopropyl-imidazo[5,1-*b*]thiazol-3-yl)-hydroxy-methyl]-[1,2,3]triazol-1-yl}-phenyl)-benzamid;
4-{4-[(2-Cyclopropyl-imidazo[5,1-*b*]thiazol-3-yl)-hydroxy-methyl]-[1,2,3]triazol-1-yl}-*N*-furan-2-ylmethyl-benzamid;
4-{4-[(2-Cyclopropyl-imidazo[5,1-*b*]thiazol-3-yl)-hydroxy-methyl]-[1,2,3]triazol-1-yl}-*N*-ethyl-*N*-methyl-benzamid;

*N*-(4-{4-[(2-Cyclopropyl-imidazo[5,1-*b*]thiazol-3-yl)-hydroxy-methyl]-[1,2,3]triazol-1-yl}-phenyl)-isobutyramid;
*N*-(4-{4-[(*R*)-(2-Cyclopropyl-imidazo[5,1-*b*]thiazol-3-yl)-hydroxy-methyl]-[1,2,3]triazol-1-yl}-phenyl)-isobutyramid;
*N*-Allyl-4-{4-[(2-cyclopropyl-imidazo[5,1-*b*]thiazol-3-yl)-hydroxy-methyl]-[1,2,3]triazol-1-yl}-benzamid;
5-{4-[(2-Cyclopropyl-imidazo[5,1-*b*]thiazol-3-yl)-hydroxy-methyl]-[1,2,3]triazol-1-yl}-1,3-dihydro-benzoimidazol-2-on;
4-{4-[(2-Cyclopropyl-imidazo[5,1-*b*]thiazol-3-yl)-hydroxy-methyl]-[1,2,3]triazol-1-yl}-N,N-dimethyl-benzamid;
4-{4-[(2-Ethyl-imidazo[5,1-*b*]thiazol-3-yl)-hydroxy-methyl]-[1,2,3]triazol-1-yl}-*N*-(2-methoxy-ethyl)-benzamid;
3-{4-[(2-Cyclopropyl-imidazo[5,1-*b*]thiazol-3-yl)-hydroxy-methyl]-[1,2,3]triazol-1-yl}-5-methoxy-benzonitril;
5-{4-[(2-Cyclopropyl-imidazo[5,1-*b*]thiazol-3-yl)-hydroxy-methyl]-[1,2,3]triazol-1-yl}-2,3-dimethoxy-benzonitril;
5-{4-[(2-Cyclopropyl-imidazo[5,1-*b*]thiazol-3-yl)-hydroxy-methyl]-[1,2,3]triazol-1-yl}-2-trifluormethoxy-benzonitril;
[1-(4-Brom-3-chlor-pyridin-2-yl)-1*H*-[1,2,3]triazol-4-yl]-(2-cyclopropyl-imidazo[5,1-*b*]thiazol-3-yl)-methanol;
2-{2-[2-(4-{4-[(2-Cyclopropyl-imidazo[5,1-*b*]thiazol-3-yl)-hydroxy-methyl]-[1,2,3]triazol-1-yl}-phenoxy)-ethoxy]-ethoxy}-ethanol;
(*S*)-3-(4-{4-[(*R*)-(2-Cyclopropyl-imidazo[5,1-*b*]thiazol-3-yl)-hydroxy-methyl]-[1,2,3]triazol-1-yl}-phenoxy)-propan-1,2-diol;
(*S*)-3-(4-{4-[(*S*)-(2-Cyclopropyl-imidazo[5,1-*b*]thiazol-3-yl)-hydroxy-methyl]-[1,2,3]triazol-1-yl}-phenoxy)-propan-1,2-diol;
(2-Cyclopropyl-imidazo[5,1-*b*]thiazol-3-yl)-{1-[4-(pyridin-2-ylmethoxy)-phenyl]-1*H*-[1,2,3]triazol-4-yl}-methanol;
(2-Cyclopropyl-imidazo[5,1-b]thiazol-3-y)l-{1-[4-(2-dimethylamino-ethoxy)-phenyl]-1*H*-[1,2,3]triazol-4-yl}-methanol;
(2-Cyclopropyl-imidazo[5,1-*b*]thiazol-3-yl)-{1-[4-(2-methansulfonyl-ethoxy)-phenyl]-1*H*-[1,2,3]triazol-4-yl}-methanol;
3-(4-{4-[(2-Cyclopropyl-imidazo[5,1-*b*]thiazol-3-yl)-hydroxy-methyl]-[1,2,3]triazol-1-yl}-phenoxy)-propan-1-sulfonsäureamid;
4-{4-[(2-Cyclopropyl-imidazo[5,1-*b*]thiazol-3-yl)-hydroxy-methyl]-[1,2,3]triazol-1-yl}-3-methyl-phenol;
4-{4-[(2-Cyclopropyl-imidazo[5,1-*b*]thiazol-3-yl)-hydroxy-methyl]-[1,2,3]triazol-1-yl}-3-fluor-phenol;
4-{4-[(2-Cyclopropyl-imidazo[5,1-*b*]thiazol-3-yl)-hydroxy-methyl]-[1,2,3]triazol-1-yl}-2-trifluormethyl-phenol;
4-{4-[(2-Cyclopropyl-imidazo[5,1-*b*]thiazol-3-yl)-hydroxy-methyl]-[1,2,3]triazol-1-yl}-2-trifluormethoxy-phenol;
4-{4-[(2-Cyclopropyl-imidazo[5,1-*b*]thiazol-3-yl)-hydroxy-methyl]-[1,2,3]triazol-1-yl}-2-methyl-phenol;
3-Chlor-4-{4-[(2-cyclopropyl-imidazo[5,1-*b*]thiazol-3-yl)-hydroxy-methyl]-[1,2,3]triazol-1-yl}-phenol;
4-{4-[(2-Cyclopropyl-imidazo[5,1-*b*]thiazol-3-yl)-hydroxy-methyl]-[1,2,3]triazol-1-yl}-3-trifluormethoxy-phenol;
4-{4-[(2-Cyclopropyl-imidazo[5,1-*b*]thiazol-3-yl)-hydroxy-methyl]-[1,2,3]triazol-1-yl}-3-trifluormethyl-phenol;
3-{4-[(2-Cyclopropyl-imidazo[5,1-*b*]thiazol-3-yl)-hydroxy-methyl]-[1,2,3]triazol-1-yl}-phenol;
5-{4-[(2-Cyclopropyl-imidazo[5,1-*b*]thiazol-3-yl)-hydroxy-methyl]-[1,2,3]triazol-1-yl}-2-methoxy-phenol;
2-Chlor-5-{4-[(2-cyclopropyl-imidazo[5,1-*b*]thiazol-3-yl)-hydroxy-methyl]-[1,2,3]triazol-1-yl}-phenol;
5-{4-[(2-Cyclopropyl-imidazo[5,1-*b*]thiazol-3-yl)-hydroxy-methyl]-[1,2,3]triazol-1-yl}-2-methyl-phenol;
3-{4-[(2-Cyclopropyl-imidazo[5,1-*b*]thiazol-3-yl)-hydroxy-methyl]-[1,2,3]triazol-1-yl}-5-trifluormethyl-phenol;
3-{4-[(2-Cyclopropyl-imidazo[5,1-*b*]thiazol-3-yl)-hydroxy-methyl]-[1,2,3]triazol-1-yl}-5-methyl-phenol;
5-Chlor-2-{4-[(2-cyclopropyl-imidazo[5,1-*b*]thiazol-3-yl)-hydroxy-methyl]-[1,2,3]triazol-1-yl}-phenol;
(2-Cyclopropyl-imidazo[5,1-*b*]thiazol-3-yl)-[1-(2,3-dihydro-benzo[1,4]dioxin-6-yl)-1H-pyrazol-4-yl]-methanol;
(2-Cyclopropyl-imidazo[5,1-*b*]thiazol-3-yl)-[1-(6-methoxy-pyridin-3-yl)-1*H*-pyrazol-4-yl]-methanol;
*N*-(4-{4-[(2-Cyclopropyl-imidazo[5,1-*b*]thiazol-3-yl)-hydroxy-methyl]-pyrazol-1-yl}-phenyl)-acetamid;
4-{4-[(2-Ethyl-imidazo[5,1-*b*]thiazol-3-yl)-hydroxy-methyl]-[1,2,3]triazol-1-yl}-*N*-methyl-benzolsulfonamid;
4-{4-[(2-Cyclopropyl-imidazo[5,1-*b*]thiazol-3-yl)-hydroxy-methyl]-[1,2,3]triazol-1-yl}-*N*-methyl-benzolsulfonamid;
(2-Ethyl-imidazo[5,1-*b*]thiazol-3-yl)-[1-(3-methyl-1,1-dioxo-2,3-dihydro-1*H*-1l6-benzo[*d*]isothiazol-5-yl)-1*H*-[1,2,3]triazol-4-yl]-methanol;
(2-Cyclopropyl-imidazo[5,1-*b*]thiazol-3-yl)-[1-(3-methyl-1,1-dioxo-2,3-dihydro-1*H*-11|6-benzo[*d*]isothiazol-5-yl)-1*H*-[1,2,3]triazol-4-yl]-methanol;
(2-Cyclopropyl-imidazo[5,1-b]thiazol-3-yl)-[1-(1-methyl-1*H*-pyrrolo[2,3-*b*]pyridin-5-yl)-1*H*-pyrazol-4-yl]-methanol;
*N*-(4-{4-[(2-Ethyl-imidazo[5,1-*b*]thiazol-3-yl)-hydroxy-methyl]-[1,2,3]triazol-1-yl}-phenyl)-benzamid;
(4-{4-[(2-Cyclopropyl-imidazo[5,1-b]thiazol-3-yl)-hydroxy-methyl]-[1,2,3]triazol-1-yl}-phenoxy)-essigsäureethylester;
4-{4-[(2-Cyclopropyl-imidazo[5,1-*b*]thiazol-3-yl)-hydroxy-methyl]-[1,2,3]triazol-1-yl}-2-ethyl-phenol;
4-{4-[(2-Cyclopropyl-imidazo[5,1-*b*]thiazol-3-yl)-hydroxy-methyl]-[1,2,3]triazol-1-yl}-2-hydroxy-benzonitril;

(4-{4-[(2-Ethyl-imidazo[5,1-*b*]thiazol-3-yl)-hydroxy-methyl]-[1,2,3]triazol-1-yl}-phenyl)-carbaminsäure-tert-butylester;
4-{4-[(2-Cyclopropyl-imidazo[5,1-*b*]thiazol-3-yl)-hydroxy-methyl]-[1,2,3]triazol-1-yl}-benzolsulfonamid;
4-{4-[(2-Cyclopropyl-imidazo[5,1-*b*]thiazol-3-yl)-hydroxy-methyl]-[1,2,3]triazol-1-yl}-*N*,*N*-dimethyl-benzolsulfonamid;
(2-Cyclopropyl-imidazo[5,1-*b*]thiazol-3-yl)-{1-[4-(pyrrolidin-1-sulfonyl)-phenyl]-1*H*-[1,2,3]triazol-4-yl}-methanol;
4-{4-[(2-Cyclopropyl-imidazo[5,1-*b*]thiazol-3-yl)-hydroxy-methyl]-[1,2,3]triazol-1-yl}-*N*,*N*-diethyl-benzolsulfonamid;
4-{4-[(2-Cyclopropyl-imidazo[5,1-*b*]thiazol-3-yl)-hydroxy-methyl]-[1,2,3]triazol-1-yl}-*N*-ethyl-benzolsulfonamid;
4-{4-[(2-Ethyl-imidazo[5,1-*b*]thiazol-3-yl)-hydroxy-methyl]-[1,2,3]triazol-1-yl}-benzolsulfonamid;
4-{4-[(2-Ethyl-imidazo[5,1-*b*]thiazol-3-yl)-hydroxy-methyl]-[1,2,3]triazol-1-yl}-*N*,*N*-dimethyl-benzolsulfonamid;
(2-Ethyl-imidazo[5,1-*b*]thiazol-3-yl)-{1-[4-(pyrrolidin-1-sulfonyl)-phenyl]-1*H*-[1,2,3]triazol-4-yl}-methanol;
*N*,*N*-Diethyl-4-{4-[(2-ethyl-imidazo[5,1-*b*]thiazol-3-yl)-hydroxy-methyl]-[1,2,3]triazol-1-yl}-benzolsulfonamid;
*N*-Ethyl-4-{4-[(2-ethyl-imidazo[5,1-*b*]thiazol-3-yl)-hydroxy-methyl]-[1,2,3]triazol-1-yl}-benzolsulfonamid;
(4-{4-[(2-Ethyl-imidazo[5,1-*b*]thiazol-3-yl)-hydroxy-methyl]-[1,2,3]triazol-1-yl}-3-fluor-phenyl)-carbaminsäure-tert-butylester;
*N*-(4-{4-[(2-Cyclopropyl-imidazo[5,1-*b*]thiazol-3-yl)-hydroxy-methyl]-pyrazol-1-yl}-phenyl)-isobutyramid;
5-{4-[(2-Cyclopropyl-imidazo[5,1-*b*]thiazol-3-yl)-hydroxy-methyl]-pyrazol-1-yl}-2-methoxy-benzonitril;
4-{4-[(2-Cyclopropyl-imidazo[5,1-*b*]thiazol-3-yl)-hydroxy-methyl]-[1,2,3]triazol-1-yl}-2-nitro-phenol;
[1-(4-Methoxy-phenyl)-1*H*-[1,2,3]triazol-4-yl]-(2-phenyl-imidazo[5,1-*b*]thiazol-3-yl)-methanol;
4-{4-[Hydroxy-(2-phenyl-imidazo[5,1-*b*]thiazol-3-yl)-methyl]-[1,2,3]triazol-1-yl}-2-nitro-phenol;
(4-{4-[Hydroxy-(2-phenyl-imidazo[5,1-*b*]thiazol-3-yl)-methyl]-[1,2,3]triazol-1-yl}-phenyl)-carbaminsäure-*tert*-butylester;
(2-Cyclopropyl-imidazo[5,1-*b*]thiazol-3-yl)-[1-(4-methoxy-phenyl)-5-methyl-1*H*-[1,2,3]triazol-4-yl]-methanol;
(2-Cyclopropyl-imidazo[5,1-*b*]thiazol-3-yl)-[1-(1*H*-indazol-5-yl)-1*H*-[1,2,3]triazol-4-yl]-methanol;
(2-Cyclopropyl-imidazo[5,1-*b*]thiazol-3-yl)-[1-(1-methyl-1*H*-indazol-1-yl)-1*H*-[1,2,3]triazol-4-yl]-methanol;
[1-(4-Methoxy-phenyl)-5-methyl-1*H*-[1,2,3]triazol-4-yl]-(2-methyl-imidazo[5,1-*b*]thiazol-3-yl)-methanol;
4-{4-[(2-Cyclopropyl-imidazo[5,1-*b*]thiazol-3-yl)-hydroxy-methyl]-[1,2,3]triazol-1-yl}-((*N*-methylsulfamoyl)amino)-benzol;
[2-(1,1-Difluor-ethyl)-imidazo[5,1-*b*]thiazol-3-yl]-[1-(4-methoxy-phenyl)-1*H*-[1,2,3]triazol-4-yl]-methanol;
[1-(3-Chlor-1*H*-indazol-6-yl)-1H-[1,2,3]triazol-4-yl]-(2-cyclopropyl-imidazo[5,1-*b*]thiazol-3-yl)-methanol;
(2-(Cyclopent-1-enyl)-imidazo[5,1-*b*]thiazol-3-yl)-[1-(4-methoxy-phenyl)-1*H*-[1,2,3]triazol-4-yl]-methanol;
(2-Cyclopropyl-imidazo[5,1-*b*]thiazol-3-yl)-(1-pyridin-3-yl-1*H*-pyrazol-4-yl)-methanol;
[1-(4-Methoxy-phenyl)-1*H*-[1,2,3]triazol-4-yl]-(2-methyl-imidazo[5,1-*b*]thiazol-3-yl)-methanol;
[1-(4-Methoxy-phenyl)-1*H*-[1,2,3]triazol-4-yl]-[2-(2-methyl-propenyl)-imidazo[5,1-*b*]thiazol-3-yl]-methanol;
[1-(4-Methoxy-phenyl)-1*H*-[1,2,3]triazol-4-yl]-[((*E*)-2-(prop-1-enyl))-imidazo[5,1-*b*]thiazol-3-yl]-methanol;
2-Chlor-4-{4-[hydroxy-(2-phenyl-imidazo[5,1-*b*]thiazol-3-yl)-methyl]-[1,2,3]triazol-1-yl}-phenol;
[1-(3-Chlor-4-cyclopropylmethoxy-phenyl)-1*H*-[1,2,3]triazol-4-yl]-(2-phenyl-imidazo[5,1-*b*]thiazol-3-yl)-methanol;
{1-[3-Chlor-4-(3-fluor-oxetan-3-ylmethoxy)-phenyl]-1*H*-[1,2,3]triazol-4-yl}-(2-phenyl-imidazo[5,1-*b*]thiazol-3-yl)-methanol;
3-(2-Chlor-4-{4-[hydroxy-(2-phenyl-imidazo[5,1-*b*]thiazol-3-yl)-methyl]-[1,2,3]triazol-1-yl}-phenoxy)-propan-1-ol;
4-(2-Chlor-4-{4-[hydroxy-(2-phenyl-imidazo[5,1-*b*]thiazol-3-yl)-methyl]-[1,2,3]triazol-1-yl}-phenoxy)-2-methyl-butan-2-ol;
4-(2-Chlor-4-{4-[(*R*)-hydroxy-(2-phenyl-imidazo[5,1-*b*]thiazol-3-yl)-methyl]-[1,2,3]triazol-1-yl}-phenoxy)-2-methyl-butan-2-ol;
(2-Isopropenyl-imidazo[5,1-*b*]thiazol-3-yl)-[1-(4-methoxy-phenyl)-1*H*-[1,2,3]triazol-4-yl]-methanol;
[2-((*E*)-2-Cyclopropyl-vinyl)-imidazo[5,1-*b*]thiazol-3-yl]-[1-(4-methoxy-phenyl)-1*H*-[1,2,3]triazol-4-yl]-methanol;
[1-(4-Methoxy-phenyl)-1*H*-[1,2,3]triazol-4-yl]-[((*E*)-2-(pent-1-enyl))-imidazo[5,1-*b*]thiazol-3-yl]-methanol;
[1-(4-Amino-3-chlor-phenyl)-1*H*-[1,2,3]triazol-4-yl]-(2-ethyl-imidazo[5,1-*b*]thiazol-3-yl)-methanol;
[1-(4-Methoxy-phenyl)-1*H*-[1,2,3]triazol-4-yl]-[2-(1-methyl-1H-pyrazol-4-yl)-imidazo[5,1-b]thiazol-3-yl]-methanol;
[5-*tert*-Butyl-1-(4-methoxy-phenyl)-1*H*-[1,2,3]triazol-4-yl]-(2-cyclopropyl-imidazo[5,1-*b*]thiazol-3-yl)-methanol;
3-(2-Chlor-4-{4-[(R)-(2-cyclopropyl-imidazo[5,1-b]thiazol-3-yl)-hydroxy-methyl]-[1,2,3]triazol-1-yl}-phenoxy)-propan-1-ol;
(*R*)-{1-[3-Chlor-4-(tetrahydro-pyran-4-ylmethoxy)-phenyl]-1*H*-[1,2,3]triazol-4-yl}-(2-cyclopropyl-imidazo[5,1-*b*]thiazol-3-yl)-methanol;

4-(2-Chlor-4-{4-[(*R*)-(2-cyclopropyl-imidazo[5,1-*b*]thiazol-3-yl)-hydroxy-methyl]-[1,2,3]triazol-1-yl}-phenoxy)-2-methyl-butan-2-ol;
[1-(4-Amino-3-chlor-phenyl)-1*H*-[1,2,3]triazol-4-yl]-(2-cyclopropyl-imidazo[5,1-*b*]thiazol-3-yl)-methanol;
(2-Cyclopropyl-imidazo[5,1-*b*]thiazol-3-yl)-[5-ethyl-1-(4-methoxy-phenyl)-1*H*-[1,2,3]triazol-4-yl]-methanol;
(2-Cyclopropyl-imidazo[5,1-*b*]thiazol-3-yl)-[5-isopropyl-1-(4-methoxy-phenyl)-1*H*-[1,2,3]triazol-4-yl]-methanol;
(2-Cyclopropyl-imidazo[5,1-*b*]thiazol-3-yl)-[5-cyclopropyl-1-(4-methoxy-phenyl)-1*H*-[1,2,3]triazol-4-yl]-methanol;
(*R*)-{1-[3-Chlor-4-(3-fluor-oxetan-3-ylmethoxy)-phenyl]-1*H*-[1,2,3]triazol-4-yl}-(2-cyclopropyl-imidazo[5,1-*b*]thiazol-3-yl)-methanol;
1-(2-Chlor-4-{4-[(*R*)-(2-cyclopropyl-imidazo[5,1-*b*]thiazol-3-yl)-hydroxy-methyl]-[1,2,3]triazol-1-yl}-phenoxy)-2-methyl-propan-2-ol;
*N*-(2-Chlor-4-{4-[(2-cyclopropyl-imidazo[5,1-*b*]thiazol-3-yl)-hydroxy-methyl]-[1,2,3]triazol-1-yl}-phenyl)-isobutyramid;
(2-Ethyl-imidazo[5,1-*b*]thiazol-3-yl)-[1-(4-methoxy-phenyl)-1*H*-[1,2,3]triazol-4-yl]-methanol;
[2-(4-Fluor-phenyl)-imidazo[5,1-*b*]thiazol-3-yl]-[1-(4-methoxy-phenyl)-1*H*-[1,2,3]triazol-4-yl]-methanol;
[2-(2-Fluor-phenyl)-imidazo[5,1-*b*]thiazol-3-yl]-[1-(4-methoxy-phenyl)-1*H*-[1,2,3]triazol-4-yl]-methanol;
[1-(3-Chlor-5-fluor-4-methoxy-phenyl)-1*H*-[1,2,3]triazol-4-yl]-(2-cyclopropyl-imidazo[5,1-*b*]thiazol-3-yl)-methanol;
[1-(3-Chlor-4-methoxy-phenyl)-1*H*-[1,2,3]triazol-4-yl]-(2-cyclopropyl-imidazo[5,1-*b*]thiazol-3-yl)-methanol;
[1-(3-Brom-4-methoxy-phenyl)-1*H*-[1,2,3]triazol-4-yl]-(2-cyclopropyl-imidazo[5,1-*b*]thiazol-3-yl)-methanol;
(2-Cyclopropyl-imidazo[5,1-*b*]thiazol-3-yl)-[1-(3-iod-4-methoxy-phenyl)-1*H*-[1,2,3]triazol-4-yl]-methanol;
[1-(3-Chlor-2-fluor-4-methoxy-phenyl)-1*H*-[1,2,3]triazol-4-yl]-(2-cyclopropyl-imidazo[5,1-*b*]thiazol-3-yl)-methanol;
4-(2-Chlor-4-{4-[hydroxy-(2-isopropenyl-imidazo[5,1-*b*]thiazol-3-yl)-methyl]-[1,2,3]triazol-1-yl}-phenoxy)-2-methyl-butan-2-ol;
3-{4-[(2-Cyclopropyl-imidazo[5,1-*b*]thiazol-3-yl)-hydroxy-methyl]-[1,2,3]triazol-1-ylmethyl}-benzoesäu remethylester;
[1-(3-Chlor-4-methoxy-phenyl)-5-methyl-1*H*-[1,2,3]triazol-4-yl]-(2-cyclopropyl-imidazo[5,1-*b*]thiazol-3-yl)-methanol;
[1-(3-Chlor-4-ethoxy-phenyl)-5-methyl-1*H*-[1,2,3]triazol-4-yl]-(2-cyclopropyl-imidazo[5,1-b]thiazol-3-yl)-methanol;
4-{4-[(2-Cyclopropyl-imidazo[5,1-*b*]thiazol-3-yl)-hydroxy-methyl]-[1,2,3]triazol-1-ylmethyl}-benzoesäuremethylester;
3-{4-[(2-Cyclopropyl-imidazo[5,1-*b*]thiazol-3-yl)-hydroxy-methyl]-[1,2,3]triazol-1-ylmethyl}-phenol;
{1-[3-Chlor-4-(3-fluor-oxetan-3-ylmethoxy)-phenyl]-1*H*-[1,2,3]triazol-4-yl}-(2-isopropenyl-imidazo[5,1-*b*]thiazol-3-yl)-methanol;
[1-(5-Chlor-2-fluor-4-methoxy-phenyl)-1*H*-[1,2,3]triazol-4-yl]-(2-cyclopropyl-imidazo[5,1-*b*]thiazol-3-yl)-methanol;
3-(2-Chlor-4-{4-[hydroxy-(2-isopropenyl-imidazo[5,1-*b*]thiazol-3-yl)-methyl]-[1,2,3]triazol-1-yl}-phenoxy)-propan-1-ol;
4-(2-Chlor-4-{4-[(2-ethyl-imidazo[5,1-*b*]thiazol-3-yl)-hydroxy-methyl]-[1,2,3]triazol-1-yl}-phenoxy)-2-methyl-butan-2-ol;
4-[2-Chlor-4-(4-{hydroxy-[2-(1-methyl-cyclopropyl)-imidazo[5,1-*b*]thiazol-3-yl]-methyl}-[1,2,3]triazol-1-yl)-phenoxy]-2-methyl-butan-2-ol;
4-{4-[(2-Cyclopropyl-imidazo[5,1-b]thiazol-3-yl)-hydroxy-methyl]-[1,2,3]triazol-1-ylmethyl}-phenol;
2-{4-[(2-Cyclopropyl-imidazo[5,1-b]thiazol-3-yl)-hydroxy-methyl]-[1,2,3]triazol-1-ylmethyl}-phenol;
(2-Cyclopropyl-imidazo[5,1-*b*]thiazol-3-yl)-[1-(4-methoxy-2-methyl-phenyl)-1*H*-[1,2,3]triazol-4-yl]-methanol;
4-[2-Chlor-4-(4-{[2-(1,1-difluor-ethyl)-imidazo[5,1-*b*]thiazol-3-yl]-hydroxy-methyl}-[1,2,3]triazol-1-yl)-phenoxy]-2-
4-[2-Chlor-4-(4-{[2-(trans-2-fluor-cyclopropyl)-imidazo[5,1-*b*]thiazol-3-yl]-hydroxy-methyl}-[1,2,3]triazol-1-yl)-phenoxy]-2-methyl-butan-2-ol;
4-[2-Chlor-4-(4-{[2-(*cis*-2-fluor-cyclopropyl)-imidazo[5,1-*b*]thiazol-3-yl]-hydroxy-methyl}-[1,2,3]triazol-1-yl)-phenoxy]-2-methyl-butan-2-ol;
(2-Cyclopropyl-imidazo[5,1-*b*]thiazol-3-yl)-[1-(4-hydroxymethyl-phenyl)-1*H*-[1,2,3]triazol-4-yl]-methanol;
(2-Cyclopropyl-imidazo[5,1-*b*]thiazol-3-yl)-[1-(4-methoxy-2,6-dimethyl-phenyl)-1*H*-[1,2,3]triazol-4-yl]-methanol;
(2-Cyclopropyl-imidazo[5,1-*b*]thiazol-3-yl)-(1-{4-[2-(2,2,2-trifluor-ethoxy)-ethoxy]-phenyl}-1*H*-[1,2,3]triazol-4-yl)-methanol;

3-[2-Chlor-4-(4-{hydroxy-[2-(1-methyl-cyclopropyl)-imidazo[5,1-*b*]thiazol-3-yl]-methyl}-[1,2,3]triazol-1-yl)-phenoxy]-propan-1-ol;
3-[2-Chlor-4-(4-{[2-(1,1-difluor-ethyl)-imidazo[5,1-*b*]thiazol-3-yl]-hydroxy-methyl}-[1,2,3]triazol-1-yl)-phenoxy]-propan-1-ol;
{1-[3-Chlor-4-(3-fluor-oxetan-3-ylmethoxy)-phenyl]-1*H*-[1,2,3]triazol-4-yl}-[2-(1-methyl-cyclopropyl)-imidazo[5,1-*b*]thiazol-3-yl]-methanol;
{1-[3-Chlor-4-(3-fluor-oxetan-3-ylmethoxy)-phenyl]-1H-[1,2,3]triazol-4-yl}-[2-(1,1-difluor-ethyl)-imidazo[5,1-*b*]thiazol-3-yl]-methanol;
{1-[3-Chlor-4-(3-fluor-oxetan-3-ylmethoxy)-phenyl]-1*H*-[1,2,3]triazol-4-yl}-(2-ethyl-imidazo[5,1-*b*]thiazol-3-yl)-methanol;
(2-Cyclopropyl-imidazo[5,1-*b*]thiazol-3-yl)-[1-(2,5-difluor-4-methoxy-phenyl)-1*H*-[1,2,3]triazol-4-yl]-methanol;
[1-(5-Chlor-2-fluor-4-methoxy-phenyl)-1H-[1,2,3]triazol-4-yl]-(2-ethyl-imidazo[5,1-*b*]thiazol-3-yl)-methanol;
3-(2-Chlor-4-{4-[(2-ethyl-imidazo[5,1-*b*]thiazol-3-yl)-hydroxy-methyl]-[1,2,3]triazol-1-yl}-phenoxy)-propan-1-ol;
[1-(2,5-Difluor-4-methoxy-phenyl)-1*H*-[1,2,3]triazol-4-yl]-(2-ethyl-imidazo[5,1-*b*]thiazol-3-yl)-methanol;
[1-(4-Benzyloxy-3-fluor-phenyl)-1*H*-[1,2,3]triazol-4-yl]-[2-(trans-2-fluor-cyclopropyl)-imidazo[5,1-*b*]thiazol-3-yl]-methanol;
2-Chlor-4-{4-[(2-ethyl-imidazo[5,1-*b*]thiazol-3-yl)-hydroxy-methyl]-[1,2,3]triazol-1-yl}-phenol;
2-Chlor-4-(4-{hydroxy-[2-(1-methyl-cyclopropyl)-imidazo[5,1-*b*]thiazol-3-yl]-methyl}-[1,2,3]triazol-1-yl)-phenol;
2-Chlor-4-(4-{[2-(*cis*-2-fluor-cyclopropyl)-imidazo[5,1-*b*]thiazol-3-yl]-hydroxy-methyl}-[1,2,3]triazol-1-yl)-phenol;
(2-Cyclopropyl-imidazo[5,1-*b*]thiazol-3-yl)-[1-(4-methoxy-2-methyl-phenyl)-5-methyl-1*H*-[1,2,3]triazol-4-yl]-methanol;
(2-Cyclopropyl-imidazo[5,1-*b*]thiazol-3-yl)-[1-(3-fluor-4-methoxy-phenyl)-1*H*-[1,2,3]triazol-4-yl]-methanol;
2-Chlor-4-(4-{[2-(trans-2-fluor-cyclopropyl)-imidazo[5,1-*b*]thiazol-3-yl]-hydroxy-methyl}-[1,2,3]triazol-1-yl)-phenol;
2-Chlor-4-(4-{[2-(1,1-difluor-ethyl)-imidazo[5,1-*b*]thiazol-3-yl]-hydroxy-methyl}-[1,2,3]triazol-1-yl)-phenol;
(2-Cyclopropyl-imidazo[5,1-*b*]thiazol-3-yl)-[1-(4-methoxymethyl-phenyl)-1*H*-[1,2,3]triazol-4-yl]-methanol;
4-{4-[(2-Cyclopropyl-imidazo[5,1-b]thiazol-3-yl)-hydroxy-methyl]-[1,2,3]triazol-1-yl}-2,5-difluor-phenol;
4-{4-[(2-Ethyl-imidazo[5,1-*b*]thiazol-3-yl)-hydroxy-methyl]-[1,2,3]triazol-1-yl}-2,5-difluor-phenol;
[1-(5-Chlor-2-fluor-4-methoxy-phenyl)-5-methyl-1*H*-[1,2,3]triazol-4-yl]-(2-cyclopropyl-imidazo[5,1-*b*]thiazol-3-yl)-methanol;
(2-Cyclopropyl-imidazo[5,1-*b*]thiazol-3-yl)-[5-iod-1-(4-methoxy-phenyl)-1*H*-[1,2,3]triazol-4-yl]-methanol;
[2-(*trans*-2-Fluor-cyclopropyl)-imidazo[5,1-*b*]thiazol-3-yl]-[5-fluor-1-(4-methoxy-phenyl)-1*H*-[1,2,3]triazol-4-yl]-methanol; und
(2-Cyclopropyl-imidazo[5,1-*b*]thiazol-3-yl)-[5-fluor-1-(4-methoxy-phenyl)-1*H*-[1,2,3]triazol-4-yl]-methanol;

oder ein pharmazeutisch akzeptables Salz davon.

**12.** Pharmazeutische Zusammensetzung, die eine Verbindung nach einem der Ansprüche 1 bis 11 umfasst und ferner wenigstens einen pharmazeutisch akzeptablen Träger umfasst.

**13.** Verbindung nach einem der Ansprüche 1 bis 11 zur Verwendung bei der Behandlung und/oder Verhütung von Krebs, wobei die genannte Verbindung in Kombination mit einem oder mehreren Chemotherapeutika und/oder Strahlentherapie und/oder gezielter Therapie verwendet wird.

**14.** Verbindung nach einem der Ansprüche 1 bis 11 zur Verwendung als Medikament.

**15.** Verbindung nach einem der Ansprüche 1 bis 11 zur Verwendung bei der Verhütung und/oder Behandlung von Krebs.

**Revendications**

**1.** Composé selon la Formule (I) :

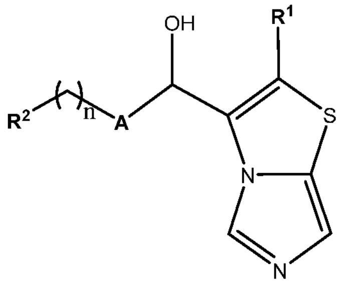

Formule (I)

où

**A** représente un phénylène ou un hétéroarylène à 5 à 6 chaînons, où ledit phénylène ou hétéroarylène à 5 à 6 chaînons est indépendamment non substitué ou mono- ou disubstitué par des substituants indépendamment sélectionnés parmi un $C_{1-4}$-alkyle, un halogène ou un $C_{3-5}$-cycloalkyle ;
**n** prend la valeur 1 ou 0 ;
**R¹** représente :

- un $C_{1-4}$-alkyle ;
- un -C(**R^A**)=C(**R^B**)(**R^C**), où **R^A**, **R^B** et **R^C** sont indépendamment sélectionnés parmi un hydrogène, un $C_{1-4}$-alkyle et un $C_{3-5}$-cycloalkyle ; ou où **R^A** et **R^B** forment, avec les atomes de carbone auxquels ils sont attachés, un $C_{4-6}$-cycloalkényle et **R^C** représente un hydrogène ;
- un $C_{1-3}$-fluoroalkyle ;
- un phényle qui est non substitué ou mono-, di- ou tri-substitué par des substituants indépendamment sélectionnés parmi un $C_{1-3}$-alkyle et un halogène ;
- un hétéroaryle à 5 chaînons qui est non substitué ou monosubstitué par un $C_{1-4}$-alkyle ; ou
- un $C_{3-6}$-cycloalkyle qui est non substitué ou mono-, di- ou trisubstitué par des substituants indépendamment sélectionnés parmi un $C_{1-3}$-alkyle et un halogène ;

**R²** représente :

- un phényle ou un hétéroaryle à 6 chaînons qui sont indépendamment non substitués ou mono-, di- ou trisubstitués par des substituants indépendamment sélectionnés parmi les suivants :

  ➢ $C_{1-4}$-alkyle, cyano, nitro, halogène, hydroxy, hydroxy-$C_{1-4}$-alkyle, $C_{1-3}$-alkoxy-$C_{1-4}$-alkyle, $C_{1-3}$-fluoroalkyle, $C_{1-3}$-fluoroalkoxy, $C_{1-4}$-alkoxy, $C_{3-5}$-cycloalkoxy, hydroxyméthyl-cyclopropyle, $C_{1-3}$-alkyl-carbonyle, $C_{1-4}$-alkoxy-carbonyle, amino, morpholin-4-yle ou morpholin-4-yl-méthyle ;
  ➢ N**R^{N1}R^{N2}**, où **R^{N1}** représente un hydrogène et **R^{N2}**représente un -(C=O)-**R^{CO}**, où **R^{CO}** représente un $C_{1-4}$-alkyle, $C_{1-4}$-alkoxy, amino, $C_{1-4}$-alkylamino, phényle ou morpholin-4-yle ;
  ➢ -(C=O)-N**R^{N3}R^{N4}**, où **R^{N3}** représente un hydrogène ou un $C_{1-3}$-alkyle et **R^{N4}** représente un hydrogène, $C_{1-3}$-alkyle, $C_{3-5}$-cycloalkyle, hydroxy-$C_{2-4}$-alkyle, $C_{1-3}$-alkoxy-$C_{2-4}$-alkyle, cyano-$C_{1-3}$-alkyle, $C_{3-4}$-alkényle, furanyl-$C_{1-3}$-alkyle, 2-di-$C_{1-3}$-alkylamino-$C_{2-3}$-alkyle ou allyle ; ou où **R^{N3}** et **R^{N4}** forment, avec l'atome d'azote auquel ils sont attachés, un cycle morpholine ;
  ➢ -$(NH)_p$-$SO_2$-N**R^{S1}R^{S2}**, où **R^{S1}** et **R^{S2}** représentent indépendamment un hydrogène ou un $C_{1-4}$-alkyle ; ou où **R^{S1}** et **R^{S2}** forment, avec l'atome d'azote auquel ils sont attachés, un hétérocycloalkyle à 4 à 6 chaînons sélectionné parmi un azétidinyle, un pyrrolidinyle et un pipéridinyle ; et **p** prend la valeur 1 ou 0 ; et
  ➢ **-OR⁶**, où **R⁶** représente un $C_{1-3}$-alkoxy-$C_{2-4}$-alkyle, hydroxy-$C_{2-5}$-alkyle, di-hydroxy-$C_{2-5}$-alkyle, sulfamoyl-$C_{2-4}$-alkyle, pyridinyl-$C_{1-3}$-alkyle, $C_{1-3}$-alkylsulfonyl-$C_{2-4}$-alkyle, $C_{3-5}$-cycloalkyl-$C_{1-3}$-alkyle, oxétan-3-yl-$C_{1-3}$-alkyle, ($C_{1-3}$-alkyl-oxétan-3-yl)-$C_{1-3}$-alkyle, (fluoro-oxétan-3-yl)-$C_{1-3}$-alkyle, tétrahydrofuranyl-$C_{1-3}$-alkyle, hydroxy-$C_{3-5}$-cycloalkyl-$C_{1-3}$-alkyle, tétrahydropyranyl-$C_{1-3}$-alkyle, benzyle, (hydroxy-$C_{2-4}$-alkoxy)-$C_{2-4}$-alkyle, pipéridin-1-yl-$C_{1-3}$-alkyle, (1-méthyl-pipéridin-3-yl)-$C_{1-3}$-alkyle, (1,1-dioxydotétrahydro-thiopyran-4-yl)-$C_{1-3}$-alkyle, $C_{1-3}$-alkoxy-carbonyl-$C_{1-3}$-alkyle, ((hydroxy-$C_{2-3}$-alkoxy)-$C_{2-3}$-alkoxy)-$C_{2-3}$-alkyle, $C_{1-3}$-fluoroalkoxy-$C_{23}$-alkyle ou $C_{1-3}$-alkyl-carbonyle ;

• un hétéroaryle à 5 chaînons qui est indépendamment non substitué ou mono-, di- ou trisubstitué par des substituants indépendamment sélectionnés parmi les suivants :

➢ $C_{1-4}$-alkyle, cyano, halogène, $C_{1-3}$-fluoroalkyle, $C_{1-3}$-fluoroalkoxy, $C_{1-4}$-alkoxy, $C_{3-5}$-cycloalkoxy, hydroxyméthyl-cyclopropyle, morpholin-4-yle, morpholin-4-yl-méthyle, $C_{1-3}$-alkyl-carbonyle, $C_{1-4}$-alkoxy-carbonyle ;
➢ -N**$R^{N1}$****$R^{N2}$**, où **$R^{N1}$** représente un hydrogène et **$R^{N2}$** représente un -(C=O)-**$R^{CO}$**, où **$R^{CO}$** représente un $C_{1-4}$-alkoxy ; ou
➢ **-$OR^6$**, où **$R^6$** représente un $C_{3-5}$-cycloalkyl-$C_{1-3}$-alkyle, un benzyle ou un $C_{1-3}$-alkoxy-carbonyl-$C_{1-3}$-alkyle ;

• un hétéroaryle bicyclique à 9 à 10 chaînons, où ledit hétéroaryle bicyclique à 9 à 10 chaînons est indépendamment non substitué ou mono-, di- ou trisubstitué par des substituants indépendamment sélectionnés parmi les suivants :

➢ $C_{1-4}$-alkyle, cyano, halogène, $C_{1-3}$-fluoroalkyle, $C_{1-3}$-fluoroalkoxy, $C_{1-4}$-alkoxy, hydroxyméthyl-cyclopropyle, morpholin-4-yle, morpholin-4-yl-méthyle, $C_{1-3}$-alkyl-carbonyle, $C_{1-4}$-alkoxy-carbonyle ;
➢ -N**$R^{N1}$****$R^{N2}$**, où **$R^{N1}$** représente un hydrogène et **$R^{N2}$** représente un -(C=O)-**$R^{CO}$**, où **$R^{CO}$** représente un $C_{1-4}$-alkoxy ; ou
➢ **-$OR^6$**, où **$R^6$** représente un $C_{3-5}$-cycloalkyl-$C_{1-3}$-alkyle ; ou

• un 2,3-dihydro-benzo[1,4]dioxin-6-yle, 1,3-dihydro-2H-benzoimidazol-2-one-5-yle, 3H-benzooxazol-2-one-6-yle, 3-méthyl-1,1-dioxo-2,3-dihydro-1*H*-1|6-benzo[*d*]isothiazol-5-yle, 1-méthyl-1*H*-pyrrolo[2,3-*b*]pyridin-5-yle ou 3,4-dihydro-2H-pyrano[2,3-b]pyridin-6-yle ;

ou sel pharmaceutiquement acceptable de celui-ci.

2. Composé selon la revendication 1, où
**A** représente un

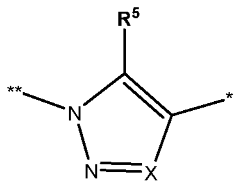

,

où une astérisque « * » indique le point d'attache au carbone qui porte le groupement OH ; deux astérisques « ** » indiquent le point d'attache à **$R^2$**-$(CH_2)_n$ ; X représente un N ou un CH ; et **$R^5$** représente un hydrogène ou un $C_{1-4}$-alkyle, un halogène ou un $C_{3-5}$-cycloalkyle ;
ou sel pharmaceutiquement acceptable de celui-ci.

3. Composé selon l'une quelconque des revendications 1 ou 2, où
**$R^2$** représente

• un phényle ou un hétéroaryle à 6 chaînons, où ledit phényle ou hétéroaryle à 6 chaînons est indépendamment non substitué ou mono-, di- ou trisubstitué par des substituants indépendamment sélectionnés parmi les suivants :

➢ $C_{1-4}$-alkyle, cyano, nitro, halogène, hydroxy, hydroxy-$C_{1-4}$-alkyle, $C_{1-3}$-alkoxy-$C_{1-4}$-alkyle, $C_{1-3}$-fluoroalkyle, $C_{1-3}$-fluoroalkoxy, $C_{1-4}$-alkoxy, $C_{3-5}$-cycloalkoxy, hydroxyméthyl-cyclopropyle, $C_{1-3}$-alkyl-carbonyle, $C_{1-4}$-alkoxy-carbonyle, amino, morpholin-4-yle ou morpholin-4-yl-méthyle ;
➢ -N**$R^{N1}$****$R^{N2}$**, où **$R^{N1}$** représente un hydrogène et **$R^{N2}$** représente un -(C=O)-**$R^{CO}$**, où **$R^{CO}$** représente un $C_{1-4}$-alkyle, $C_{1-4}$-alkoxy, amino, phényle ou morpholin-4-yle ;
➢ -(C=O)-N**$R^{N3}$****$R^{N4}$**, où **$R^{N3}$** représente un hydrogène ou un $C_{1-3}$-alkyle et **$R^{N4}$** représente un hydrogène, $C_{1-3}$-alkyle, $C_{3-5}$-cycloalkyle, hydroxy-$C_{2-4}$-alkyle, $C_{1-3}$-alkoxy-$C_{2-4}$-alkyle, cyano-$C_{1-3}$-alkyle, $C_{3-4}$-alkényle ou furanyl-$C_{1-3}$-alkyle ou allyle ; ou où **$R^{N3}$** et **$R^{N4}$** forment, avec l'atome d'azote auquel ils sont attachés,

un cycle morpholine ; et

➢ **-OR⁶**, où **R⁶** représente un $C_{1-3}$-alkoxy-$C_{2-4}$-alkyle, hydroxy-$C_{2-5}$-alkyle, di-hydroxy-$C_{2-5}$-alkyle, sulfamoyl-$C_{2-4}$-alkyle, pyridinyl-$C_{1-3}$-alkyle, $C_{1-3}$-alkylsulfonyl-$C_{2-4}$-alkyle, $C_{3-5}$-cycloalkyl-$C_{1-3}$-alkyle, oxétan-3-yl-$C_{1-3}$-alkyle, ($C_{1-3}$-alkyl-oxétan-3-yl)-$C_{1-3}$-alkyle, (fluoro-oxétan-3-yl)-$C_{1-3}$-alkyle, tétrahydrofuranyl-$C_{1-3}$-alkyle, hydroxy-$C_{3-5}$-cycloalkyl-$C_{1-3}$-alkyle, tétrahydropyranyl-$C_{1-3}$-alkyle, benzyle, (hydroxy-$C_{2-4}$-alkoxy)-$C_{2-4}$-alkyle, pipéridin-1-yl-$C_{1-3}$-alkyle, (1-méthyl-pipéridin-3-yl)-$C_{1-3}$-alkyle, (1,1-dioxydotétrahydrothiopyran-4-yl)-$C_{1-3}$-alkyle, $C_{1-3}$-alkoxy-carbonyl-$C_{1-3}$-alkyle, ((hydroxy-$C_{2-3}$-alkoxy)-$C_{2-3}$-alkoxy)-$C_{2-3}$-alkyle, $C_{1-3}$-fluoroalkoxy-$C_{2-3}$-alkyle ou $C_{1-3}$-alkyl-carbonyle ;

• un hétéroaryle bicyclique à 9 à 10 chaînons, où ledit hétéroaryle bicyclique à 9 à 10 chaînons est non substitué ; ou

• un 2,3-dihydro-benzo[1,4]dioxin-6-yle, 1,3-dihydro-2H-benzoimidazol-2-one-5-yle, 3H-benzooxazol-2-one-6-yle,3-méthyl-1,1-dioxo-2,3-dihydro-1*H*-116-benzo[dJisothiazol-5-yle,1-méthyl-1*H*-pyrrolo[2,3-*b*]pyridin-5-yle ou 3,4-dihydro-2H-pyrano[2,3-b]pyridin-6-yle ;

ou sel pharmaceutiquement acceptable de celui-ci.

**4.** Composé selon l'une quelconque des revendications 1 ou 2, où le fragment **R²**-$(CH_2)_n$— est sélectionné dans le groupe I, II, III, IV, V, VI, VII, VIII, IX, X, XI ou XII :

I. phényle, 4-hydroxy-phényle, 4-chloro-phényle, 2-méthyl-phényle, 3-méthyl-phényle, 4-méthyl-phényle, 2-méthoxy-phényle, 3-méthoxy-phényle, 4-méthoxy-phényle, 4-éthoxy-phényle, 4-(2-méthoxy-éthoxy)-phényle, 4-benzyloxy-phényle, 3-cyano-phényle, 4-cyano-phényle, 2-trifluorométhyl-phényle, 3-trifluorométhyl-phényle, 4-trifluorométhyl-phényle, 4-isopropoxy-phényle, 4-(cyclopropyl-méthoxy)-phényle, 4-(1-hydroxyméthyl-cyclopropyl)-phényle, 4-trifluorométhoxy-phényle, 4-(morpholin-4-yl)-phényle, 4-(morpholin-4-yl-méthyl)-phényle ou 4-(méthoxy-carbamoyl)-phényle ;

II. 4-(2-hydroxy-2-méthyl-propoxy)-phényle, 4-(3-hydroxy-propoxy)-phényle, 3-cyano-4-méthoxy-phényle, 4-(2-hydroxy-éthoxy)-phényle, 3-chloro-4-hydroxy-phényle, 3-fluoro-4-hydroxy-phényle, 3,5-difluoro-4-hydroxy-phényle, 4-((méthoxy-carbonyl)amino)-phényle, 4-(morpholin-4-carbonyl)-phényle, 4-((2-cyano-éthyl)-carbamoyl)-phényle, 4-(morpholin-4-yl-carboxamido)-phényle, 4-(*tert*-butyl-carboxamido)-phényle, 2-fluoro-4-(*tert*-butoxyl-carboxamido)-phényle, 4-(3-éthyluréido)-3-méthoxy-phényle, 4-(méthoxy-carboxamido)-phényle, 4-(3-éthyl-uréido)-3-méthoxy-phényle, 4-(3-(3-méthoxy-propyl)-uréido)-phényle, 4-(3-butyl-uréido)-phényle, 4-isobutyramido-phényle, 4-acétyl-phényle, 4-(tétrahydropyran-4-yl-méthoxy)-phényle, 4-((3-fluoro-oxétan-3-yl)-méthoxy)-phényle, 4-((3-méthyl-oxétan-3-yl)-méthoxy)-phényle, 4-(oxétan-3-yl-méthoxy)-phényle, 4-(2-(1-hydroxy-cyclopropyl)-éthoxy)-phényle, 4-(3-hydroxy-2,2-diméthyl-propyl)-phényle, 4-(3-hydroxy-3-méthyl-butyl)-phényle, 4-(2-(2-hydroxy-éthoxy)-éthoxy)-phényle, 4-(2-(pipéridin-1-yl)-éthoxy)-phényle, 4-(tétrahydrofuran-2-yl-méthoxy)-phényle, 4-(tétrahydropyran-4-yl-méthoxy)-phényle, 4-(tétrahydropyran-2-yl-méthoxy)-phényle, 4-((2-méthoxy-éthyl)-carbamoyl)-phényle, 4-(cyclopropyl-carbamoyl)-phényle, 4-(éthyl-carbamoyl)-phényle, 4-(3-méthoxypropyl-carbamoyl)-phényle, 4-uréido-phényle, 4-acétamido-phényle, 4-(méthyl-carbamoyl)-phényle, 4-((1-méthyl-pipéridin-3-yl)-méthoxy)-phényle, 4-((2-hydroxy-éthyl)-carbamoyl)-phényle, 4-(éthyl(méthyl)carbamoyl)-phényle, 4-((3-hydroxy-propyl)-carbamoyl)-phényle, 4-carbamoyl-phényle, 4-((furan-2-yl-méthyl)-carbamoyl)-phényle, 4-(allyl-carbamoyl)-phényle, 4-(diméthyl-carbamoyl)-phényle, 4-((2-(diméthylamino)-éthyl)-carbamoyl)-phényle, 4-((1,1-dioxo-hexahydrothiopyran-4-yl)-méthyl)-phényle ;

III. 3-chloro-4-(3-hydroxy-propoxy)-phényle, 3-chloro-4-(2-méthyl-2-hydroxy-propoxy)-phényle, 3-chloro-4-(3-méthyl-3-hydroxy-butoxy)-phényle, 4-(2-méthyl-2-hydroxy-propoxy)-phényle, 4-(2,3-dihydroxy-propoxy)-phényle, 3-cyano-5-méthoxy-phényle, 3-hydroxy-4-cyano-phényle, 3-nitro-4-hydroxy-phényle, 2-chloro-4-hydroxy-phényle, 2-fluoro-4-hydroxy-phényle, 3-hydroxy-4-chloro-phényle, 2-hydroxy-4-chloro-phényle, 3-hydroxy-phényle, 2,5-difluoro-4-hydroxy-phényle, 2,5-difluoro-4-hydroxy-phényle, 3-trifluorométhoxy-4-hydroxy-phényle, 3-cyano-4-trifluorométhoxy-phényle, 2-trifluorométhoxy-4-hydroxy-phényle, 3-méthyl-5-hydroxy-phényle, 2-méthyl-4-hydroxy-phényle, 3-hydroxy-4-méthyl-phényle, 3-méthyl-4-hydroxy-phényle, 3-éthyl-4-hydroxy-phényle, 4-(hydroxyméthyl)-phényle, (4-hydroxy-phényl)-méthyle, (2-hydroxy-phényl)-méthyle, 3-chloro-4-amino-phényle, 4-(amino-carbonyl)-phényle, 4-(méthyl-amino-carbonyl)-phényle, (4-(méthoxy-carbonyl)-phényl)-méthyle, (3-(méthoxy-carbonyl)-phényl)-méthyle, 4-(méthyl-carboxamido)-phényle, 4-(phényl-carboxamido)-phényle, 4-(isopropyl-carboxamido)-phényle, 3-chloro-4-(isopropyl-carboxamido)-phényle, 4-(*tert*-butoxy-carboxamido)-phényle, 3-hydroxy-4-méthoxy-phényle, 3-chloro-4-méthoxy-phényle, 3-chloro-4-éthoxy-phényle, 3-fluoro-4-méthoxy-phényle, 3-bromo-4-méthoxy-phényle, 3-iodo-4-méthoxy-phényle, 3-chloro-4-méthoxy-5-fluoro-phényle, 3-chloro-4-méthoxy-6-fluoro-phényle, 3,6-difluoro-4-méthoxy-phényle, 2-fluoro-3-chloro-4-méthoxy-phényle, 2,6-diméthyl-4-méthoxy-phényle, 3-chloro-4-(cyclopropyl-méthoxy)-phényle, 3-éthyl-4-hydroxy-phényle, 4-(méthyl-méthoxy)-phényle, 3-hydroxy-5-trifluorométhyl-phényle, 2-trifluorométhyl-4-hydroxy-phényle, 3,4-diméthoxy-5-cyano-phényle, 4-((2-diméthylamino-éthoxy))-phényle, 4-(2-(2-(2-hydroxyéthoxy)-éthoxy)-éthoxy)-phényle, 3-chloro-4-((3-fluoro-oxétan-3-yl)-méthoxy)-phényle, 4-(pyrrolidin-1-sulfonyl)-phényle, 4-(pyridin-2-yl-méthoxy)-phényle, 4-(éthoxy-carbonyl-méthoxy)-phényle, 4-((2-hydroxy-éthyl)-carbamoyl)-phényle, 4-((2-méthoxy-éthyl)-carbamoyl)-phényle, 4-((3-hydroxy-propyl)-carbamoyl)-phényle, 4-(allyl-carbamoyl)-phényle, 4-(allyl-carbamoyl)-phényle, 4-(diméthyl-carbamoyl)-phényle, 4-(*N*-méthyl-*N*-éthyl-carbamoyl)-phényle, 3-fluoro-4-benzyloxy-phényle, 4-sulfamoyl-phényle, 4-(méthyl-sulfamoyl)-phényle, 4-(éthyl-sulfamoyl)-phényle, 4-(diméthyl-sulfamoyl)-phényle, 4-(diéthyl-sulfamoyl)-phényle, 4-((méthyl-sulfamoyl)-amino)-phényle, 4-(2-méthanesulfonyl-éthoxy)-phényle, 4-(sulfamoyl-propoxy)-phényle, 4-(2-(2,2,2-trifluoro-éthoxy)-éthoxy)-phényle ;

IV. 6-méthoxy-pyridin-3-yle, 5-fluoro-6-méthoxy-pyridin-3-yle, 6-méthoxy-5-méthyl-pyridin-3-yle, 2,6-diméthoxy-pyridin-3-yle, 5,6-diméthoxy-pyridin-3-yle, 5,6-diméthoxy-pyridin-2-yle, 5-chloro-6-éthoxy-pyridin-3-yle, 6-isopropoxy-pyridin-3-yle, 2-méthoxy-3-(méthoxy-carbonyl)-pyridin-5-yle, 5-chloro-6-isopropoxy-pyridin-3-yle, 6-éthoxy-5-trifluorométhyl-pyridin-3-yle, 6-(méthoxy-carbonyl-méthoxy)-pyridin-3-yle, 6-méthoxy-5-(méthoxycarbonyl)-pyridin-3-yle, 2-méthoxy-pyrimidin-5-yle, 2-éthoxy-pyrimidin-5-yle, 2,4-diméthoxy-pyrimidin-5-yle, 2-benzyloxy-pyrimidin-5-yle, 2-cyclobutoxy-pyrimidin-5-yle, 3,6-diméthoxy-pyridazin-4-yle ou 6-(2,2,2-trifluoro-éthoxy)-pyridin-3-yle ;

V. pyridin-3-yle, 4-méthoxy-pyridin-3-yle ou 3-chloro-4-bromo-pyridin-2-yle ;

VI. pyrazol-4-yle, 1-méthyl-pyrazol-4-yle, pyrimidin-5-yle, pyridin-2-yle, pyridin-3-yle, pyridin-4-yle ou 6-éthoxy-pyridin-3-yle ;

VII. indol-4-yle, indol-5-yle, indol-6-yle, indol-7-yle, quinoléin-4-yle, quinoléin-5-yle, isoquinoléin-4-yle, isoquinoléin-5-yle ou isoquinoléin-8-yle ;

VIII. 1*H*-indazol-5-yle, 3-chloro-1*H*-indazol-6-yle ou 1-méthyl-1*H*-indazol-7-yle ;

IX. benzyle ;

X. 2,3-dihydro-[1,4]benzodioxan-6-yle ;

XI. 1,3-dihydro-2*H*-benzoimidazol-2-on-5-yle ou 3,4-dihydro-2*H*-pyrano[2,3-*b*]pyridin-6-yle ; et

XII. 3H-benzooxazol-2-on-6-yle, 3-méthyl-1,1-dioxo-2,3-dihydro-1*H*-1l6-benzo[*d*]isothiazol-5-yle, 1-méthyl-1*H*-pyrrolo[2,3-*b*]pyridin-5-yle, 2,3-dihydro-benzo[1,4]dioxin-6-yle ;

ou sel pharmaceutiquement acceptable de celui-ci.

**5.** Composé selon l'une quelconque des revendications 1 à 3, où **n** prend la valeur 0 ; ou sel pharmaceutiquement acceptable de celui-ci.

**6.** Composé selon l'une quelconque des revendications 1 à 5, où
**$R^1$** représente :

- un $C_{1-4}$-alkyle ;
- un vinyle, un 1-méthyl-vinyle ou un 1-propényle ;
- un $C_{1-3}$-fluoroalkyle ;
- un $C_{3-6}$-cycloalkyle qui est non substitué ou mono- ou disubstitué par des substituants indépendamment sélectionnés parmi un $C_{1-3}$-alkyle ou un fluor ; ou
- un phényle qui est non substitué ou mono-, di- ou trisubstitué par des substituants indépendamment sélectionnés parmi un $C_{1-3}$-alkyle et un halogène ;

ou sel pharmaceutiquement acceptable de celui-ci.

**7.** Composé selon la revendication 1, où

**A** représente un

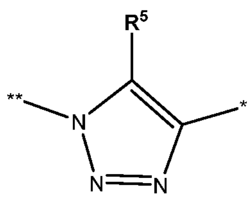
,

où une astérisque « * » indique le point d'attache au carbone qui porte le groupement OH ; deux astérisques « ** » indiquent le point d'attache à **$R^2$**-$(CH_2)_n$ ; et **$R^5$** représente un hydrogène ou un $C_{1-3}$-alkyle ;
**n** prend la valeur 0 ;
**$R^1$** représente

- un $C_{1-3}$-alkyle ;
- un -C(**$R^A$**)=C(**$R^B$**)(**$R^C$**), où **$R^A$**, **$R^B$** et **$R^C$** sont indépendamment sélectionnés parmi un hydrogène et un méthyle ;
- un $C_{1-3}$-fluoroalkyle ;
- un phényle qui est non substitué ou monosubstitué par un halogène ; ou
- un cyclopropyle qui est non substitué ou monosubstitué par un méthyle ou un halogène ;

**$R^2$** représente

- un phényle qui est non substitué ou mono-, di- ou trisubstitué, où un substituant représente indépendamment l'un des suivants :

  ➢ **-$OR^6$**, où **$R^6$** représente un hydrogène, $C_{1-3}$-alkyle, $C_{1-3}$-alkoxy-$C_{2-4}$-alkyle, hydroxy-$C_{2-5}$-alkyle, $C_{3-5}$-cycloalkyl-$C_{1-3}$-alkyle, oxétan-3-yl-$C_{1-3}$-alkyle, (fluoro-oxétan-3-yl)-$C_{1-3}$-alkyle, hydroxy-$C_{3-5}$-cycloalkyl-$C_{1-3}$-alkyle, tétrahydropyranyl-$C_{1-3}$-alkyle, $C_{1-3}$-fluoroalkoxy-$C_{2-3}$-alkyle ou benzyle ; ou
  ➢ -N**$R^{N1}R^{N2}$**, où **$R^{N1}$** représente un hydrogène ; **$R^{N2}$** représente un hydrogène ou un -(C=O)-**$R^{CO}$**, où **$R^{CO}$** représente un $C_{1-4}$-alkyle, un $C_{1-4}$-alkoxy ou un phényle ;

  ledit un substituant étant attaché audit phényle en position *para* par rapport au point d'attache du restant de la molécule, et où tout substituant supplémentaire dudit cycle phényle, si présent, représente indépendamment un halogène, un cyano ou un nitro ;

ou sel pharmaceutiquement acceptable de celui-ci.

**8.** Composé selon l'une quelconque des revendications 1 à 7, où l'atome de carbone asymétrique auquel **$R^2$**-$(CH_2)_n$-**A**- est attaché a la configuration absolue illustrée à la Formule (II) :

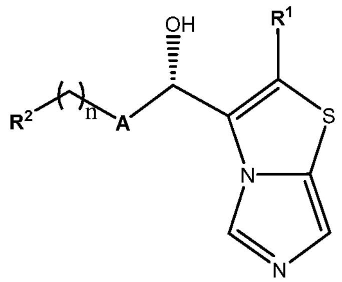

Formule (II)

ou sel pharmaceutiquement acceptable de celui-ci.

**9.** Composé selon la revendication 1 sélectionné dans un groupe consistant en les suivants :

(2-méthyl-imidazo[5,1-*b*]thiazol-3-yl)-(1-phényl-1*H*-[1,2,3]triazol-4-yl)-méthanol ;
(2-éthyl-imidazo[5,1-*b*]thiazol-3-yl)-(1-phényl-1*H*-[1,2,3]triazol-4-yl)-méthanol ;
(2-cyclopropyl-imidazo[5,1-*b*]thiazol-3-yl)-(1-phényl-1*H*-[1,2,3]triazol-4-yl)-méthanol ;
(R)-(2-cyclopropyl-imidazo[5,1-b]thiazol-3-yl)-(1-phényl-1*H*-[1,2,3]triazol-4-yl)-méthanol ;
(2-cyclopentyl-imidazo[5,1-*b*]thiazol-3-yl)-(1-phényl-1*H*-[1,2,3]triazol-4-yl)-méthanol ;
(2-cyclopropyl-imidazo[5,1-b]thiazol-3-yl)-(1-*o*-tolyl-1*H*-[1,2,3]triazol-4-yl)-méthanol ;
(2-cyclopropyl-imidazo[5,1-b]thiazol-3-yl)-(1-*m*-tolyl-1*H*-[1,2,3]triazol-4-yl)-méthanol ;
(2-cyclopropyl-imidazo[5,1-b]thiazol-3-yl)-(1-*p*-tolyl-1*H*-[1,2,3]triazol-4-yl)-méthanol ;
(2-cyclopropyl-imidazo[5,1-*b*]thiazol-3-yl)-[1-(3-méthoxy-phényl)-1*H*-[1,2,3]triazol-4-yl]-méthanol ;
(*R*)-(2-cyclopropyl-imidazo[5,1-*b*]thiazol-3-yl)-[1-(4-méthoxy-phényl)-1*H*-[1,2,3]triazol-4-yl]-méthanol ;

(2-cyclopropyl-imidazo[5,1-*b*]thiazol-3-yl)-[1-(4-méthoxy-phényl)-1*H*-[1,2,3]triazol-4-yl]-méthanol ;
(2-cyclopropyl-imidazo[5,1-*b*]thiazol-3-yl)-[1-(2-méthoxy-phényl)-1*H*-[1,2,3]triazol-4-yl]-méthanol ;
(1-benzyl-1*H*-[1,2,3]triazol-4-yl)-(2-cyclopropyl-imidazo[5,1-*b*]thiazol-3-yl)-méthanol ;
3-{4-[(2-cyclopropyl-imidazo[5,1-b]thiazol-3-yl)-hydroxy-méthyl]-[1,2,3]triazol-1-yl}-benzonitrile ;
4-{4-[(2-cyclopropyl-imidazo[5,1-b]thiazol-3-yl)-hydroxy-méthyl]-[1,2,3]triazol-1-yl}-benzonitrile ;
(2-cyclopropyl-imidazo[5,1-*b*]thiazol-3-yl)-[1-(2-trifluorométhyl-phényl)-1*H*-[1,2,3]triazol-4-yl]-méthanol ;
(2-cyclopropyl-imidazo[5,1-*b*]thiazol-3-yl)-[1-(3-trifluorométhyl-phényl)-1*H*-[1,2,3]triazol-4-yl]-méthanol ;
(2-cyclopropyl-imidazo[5,1-*b*]thiazol-3-yl)-[1-(4-trifluorométhyl-phényl)-1*H*-[1,2,3]triazol-4-yl]-méthanol ;
1-(4-{4-[(2-cyclopropyl-imidazo[5,1-*b*]thiazol-3-yl)-hydroxy-méthyl]-[1,2,3]triazol-1-yl}-phényl)-éthanone ;
(2-cyclopropyl-imidazo[5,1-*b*]thiazol-3-yl)-[1-(4-cyclopropylméthoxy-phényl)-1*H*-[1,2,3]triazol-4-yl]-méthanol ;
(2-cyclopropyl-imidazo[5,1-*b*]thiazol-3-yl)-[1-(4-éthoxy-phényl)-1*H*-[1,2,3]triazol-4-yl]-méthanol ;
ester méthylique de l'acide (4-{4-[(2-cyclopropyl-imidazo[5,1-b]thiazol-3-yl)-hydroxy-méthyl]-[1,2,3]triazol-1-yl}-phényl)-carbamique ;
(2-cyclopropyl-imidazo[5,1-*b*]thiazol-3-yl)-[1-(4-isopropoxy-phényl)-1H-[1,2,3]triazol-4-yl]-méthanol ;
3-{4-[(R)-(2-cyclopropyl-imidazo[5,1-b]thiazol-3-yl)-hydroxy-méthyl]-[1,2,3]triazol-1-yl}-benzonitrile ;
[1-(4-méthoxy-phényl)-1*H*-[1,2,3]triazol-4-yl]-[2-(1-méthyl-cyclopropyl)-imidazo[5,1-*b*]thiazol-3-yl]-méthanol ;
[1-(4-chloro-phényl)-1*H*-[1,2,3]triazol-4-yl]-(2-cyclopropyl-imidazo[5,1-*b*]thiazol-3-yl)-méthanol ;
(2-cyclopropyl-imidazo[5,1-*b*]thiazol-3-yl)-{1-[4-(1-hydroxyméthyl-cyclopropyl)-phényl]-1*H*-[1,2,3]triazol-4-yl}-méthanol ;
(2-cyclopropyl-imidazo[5,1-*b*]thiazol-3-yl)-[1-(4-morpholin-4-yl-phényl)-1*H*-[1,2,3]triazol-4-yl]-méthanol ;
(2-cyclopropyl-imidazo[5,1-*b*]thiazol-3-yl)-[1-(4-morpholin-4-ylméthyl-phényl)-1*H*-[1,2,3]triazol-4-yl]-méthanol ;
(2-cyclopropyl-imidazo[5,1-*b*]thiazol-3-yl)-[1-(4-trifluorométhoxy-phényl)-1*H*-[1,2,3]triazol-4-yl]-méthanol ;
(2-cyclopropyl-imidazo[5,1-*b*]thiazol-3-yl)-(1-pyrimidin-5-yl-1*H*-[1,2,3]triazol-4-yl)-méthanol ;
(2-cyclopropyl-imidazo[5,1-*b*]thiazol-3-yl)-(1-pyridin-2-yl-1*H*-[1,2,3]triazol-4-yl)-méthanol ;
(2-cyclopropyl-imidazo[5,1-*b*]thiazol-3-yl)-(1-pyridin-3-yl-1*H*-[1,2,3]triazol-4-yl)-méthanol ;
(2-cyclopropyl-imidazo[5,1-*b*]thiazol-3-yl)-(1-pyridin-4-yl-1*H*-[1,2,3]triazol-4-yl)-méthanol ;
(2-cyclopropyl-imidazo[5,1-*b*]thiazol-3-yl)-[1-(6-éthoxy-pyridin-3-yl)-1*H*-[1,2,3]triazol-4-yl]-méthanol ;
(2-cyclopropyl-imidazo[5,1-*b*]thiazol-3-yl)-[1-(1-méthyl-1*H*-pyrazol-4-yl)-1*H*-[1,2,3]triazol-4-yl]-méthanol ;
(2-cyclopropyl-imidazo[5,1-*b*]thiazol-3-yl)-[1-(1*H*-pyrazol-4-yl)-1*H*-[1,2,3]triazol-4-yl]-méthanol ;
(2-cyclopropyl-imidazo[5,1-*b*]thiazol-3-yl)-[1-(1*H*-indol-6-yl)-1*H*-[1,2,3]triazol-4-yl]-méthanol ;
(2-cyclopropyl-imidazo[5,1-*b*]thiazol-3-yl)-[1-(1*H*-indol-4-yl)-1*H*-[1,2,3]triazol-4-yl]-méthanol ;
(R)-(2-cyclopropyl-imidazo[5,1-*b*]thiazol-3-yl)-[1-(1*H*-indol-5-yl)-1*H*-[1,2,3]triazol-4-yl]-méthanol ;
(2-cyclopropyl-imidazo[5,1-*b*]thiazol-3-yl)-[1-(1*H*-indol-5-yl)-1*H*-[1,2,3]triazol-4-yl]-méthanol ;
(2-cyclopropyl-imidazo[5,1-*b*]thiazol-3-yl)-[1-(1*H*-indol-7-yl)-1*H*-[1,2,3]triazol-4-yl]-méthanol ;
(2-cyclopropyl-imidazo[5,1-*b*]thiazol-3-yl)-(1-isoquinoléin-5-yl-1*H*-[1,2,3]triazol-4-yl)-méthanol ;
(2-cyclopropyl-imidazo[5,1-*b*]thiazol-3-yl)-(1-quinoléin-5-yl-1*H*-[1,2,3]triazol-4-yl)-méthanol ;
(2-cyclopropyl-imidazo[5,1-*b*]thiazol-3-yl)-(1-isoquinoléin-8-yl-1*H*-[1,2,3]triazol-4-yl)-méthanol ;
(2-cyclopropyl-imidazo[5,1-*b*]thiazol-3-yl)-(1-quinoléin-4-yl-1*H*-[1,2,3]triazol-4-yl)-méthanol ;
(2-cyclopropyl-imidazo[5,1-*b*]thiazol-3-yl)-(1-isoquinoléin-4-yl-1*H*-[1,2,3]triazol-4-yl)-méthanol ; et
(2-cyclopropyl-imidazo[5,1-*b*]thiazol-3-yl)-[1-(2,3-dihydro-benzo[1,4]dioxin-6-yl)-1*H*-[1,2,3]triazol-4-yl]-

méthanol ; ou sel pharmaceutiquement acceptable de celui-ci.

**10.** Composé selon la revendication 1 sélectionné dans un groupe consistant en les suivants :

[1-(4-benzyloxy-phényl)-1*H*-[1,2,3]triazol-4-yl]-(2-cyclopropyl-imidazo[5,1-*b*]thiazol-3-yl)-méthanol ;
[1-(6-éthoxy-pyridin-3-yl)-1*H*-[1,2,3]triazol-4-yl]-(2-éthyl-imidazo[5,1-*b*]thiazol-3-yl)-méthanol ;
(2-éthyl-imidazo[5,1-*b*]thiazol-3-yl)-[1-(4-morpholin-4-yl-phényl)-1*H*-[1,2,3]triazol-4-yl]-méthanol
(2-éthyl-imidazo[5,1-*b*]thiazol-3-yl)-{1-[4-(1-hydroxyméthyl-cyclopropyl)-phényl]-1*H*-11,2,3]triazol-4-yl}-méthanol ;
ester méthylique de l'acide (4-{4-[(2-éthyl-imidazo[5,1-b]thiazol-3-yl)-hydroxy-méthyl]-[1,2,3]triazol-1-yl}-phényl)-carbamique ;
(2-éthyl-imidazo[5,1-*b*]thiazol-3-yl)-[1-(1*H*-indol-5-yl)-1*H*-[1,2,3]triazol-4-yl]-méthanol ;
3-{4-[(2-éthyl-imidazo[5,1-*b*]thiazol-3-yl)-hydroxy-méthyl]-[1,2,3]triazol-1-yl}-benzonitrile ;
(2-éthyl-imidazo[5,1-*b*]thiazol-3-yl)-(1-isoquinoléin-4-yl-1*H*-[1,2,3]triazol-4-yl)-méthanol ;
(1-benzyl-1*H*-[1,2,3]triazol-4-yl)-(2-éthyl-imidazo[5,1-*b*]thiazol-3-yl)-méthanol ;
[1-(4-cyclopropylméthoxy-phényl)-1*H*-[1,2,3]triazol-4-yl]-(2-éthyl-imidazo[5,1-*b*]thiazol-3-yl)-méthanol ;
[1-(6-éthoxy-pyridin-3-yl)-1*H*-[1,2,3]triazol-4-yl]-(2-méthyl-imidazo[5,1-*b*]thiazol-3-yl)-méthanol ;

(2-méthyl-imidazo[5,1-*b*]thiazol-3-yl)-[1-(4-morpholin-4-yl-phényl)-1*H*-[1,2,3]triazol-4-yl]-méthanol ;
{1-[4-(1-hydroxyméthyl-cyclopropyl)-phényl]-1*H*-[1,2,3]triazol-4-yl}-(2-méthyl-imidazo[5,1-*b*]thiazol-3-yl)-méthanol ;
ester méthylique de l'acide (4-{4-[hydroxy-(2-méthyl-imidazo[5,1-*b*]thiazol-3-yl)-méthyl]-[1,2,3]triazol-1-yl}-phényl)-carbamique ;
[1-(1*H*-indol-5-yl)-1*H*-[1,2,3]triazol-4-yl]-(2-méthyl-imidazo[5,1-*b*]thiazol-3-yl)-méthanol ;
3-{4-[hydroxy-(2-méthyl-imidazo[5,1-*b*]thiazol-3-yl)-méthyl]-[1,2,3]triazol-1-yl}-benzonitrile ;
(1-isoquinoléin-4-yl-1*H*-[1,2,3]triazol-4-yl)-(2-méthyl-imidazo[5,1-*b*]thiazol-3-yl)-méthanol ;
(1-benzyl-1*H*-[1,2,3]triazol-4-yl)-(2-méthyl-imidazo[5,1-*b*]thiazol-3-yl)-méthanol ;
[1-(4-cyclopropylméthoxy-phényl)-1*H*-[1,2,3]triazol-4-yl]-(2-méthyl-imidazo[5,1-*b*]thiazol-3-yl)-méthanol ;
[2-(3-fluoro-phényl)-imidazo[5,1-*b*]thiazol-3-yl]-[1-(4-méthoxy-phényl)-1*H*-[1,2,3]triazol-4-yl]-méthanol ;
(2-cyclopropyl-imidazo[5,1-*b*]thiazol-3-yl)-[1-(2-éthoxy-pyrimidin-5-yl)-1*H*-[1,2,3]triazol-4-yl]-méthanol ;
(2-cyclopropyl-imidazo[5,1-*b*]thiazol-3-yl)-[1-(5,6-diméthoxy-pyridin-2-yl)-1*H*-[1,2,3]triazol-4-yl]-méthanol ;
(2-cyclopropyl-imidazo[5,1-b]thiazol-3-yl)-[1-(2-méthoxy-pyrimidin-5-yl)-1H-[1,2,3]triazol-4-yl]-méthanol ;
4-{4-[(2-cyclopropyl-imidazo[5,1-b]thiazol-3-yl)-hydroxy-méthyl]-[1,2,3]triazol-1-yl}-phénol ;
(2-cyclopropyl-imidazo[5,1-*b*]thiazol-3-yl)-[1-(6-méthoxy-pyridin-3-yl)-1*H*-[1,2,3]triazol-4-yl]-méthanol ;
ester méthylique de l'acide 5-{4-[(2-cyclopropyl-imidazo[5,1-*b*]thiazol-3-yl)-hydroxy-méthyl]-[1,2,3]triazol-1-yl}-2-méthoxy-nicotinique ;
(2-cyclopropyl-imidazo[5,1-*b*]thiazol-3-yl)-[1-(6-méthoxy-5-méthyl-pyridin-3-yl)-1*H*-[1,2,3]triazol-4-yl]-méthanol ;
(2-cyclopropyl-imidazo[5,1-*b*]thiazol-3-yl)-[1-(3,4-dihydro-2*H*-pyrano[2,3-*b*]pyridin-6-yl)-1*H*-[1,2,3]triazol-4-yl]-méthanol ;
[1-(5-chloro-6-isopropoxy-pyridin-3-yl)-1*H*-[1,2,3]triazol-4-yl]-(2-cyclopropyl-imidazo[5,1-*b*]thiazol-3-yl)-méthanol ;
[1-(5-chloro-6-éthoxy-pyridin-3-yl)-1H-[1,2,3]triazol-4-yl]-(2-cyclopropyl-imidazo[5,1-b]thiazol-3-yl)-méthanol ;
ester méthylique de l'acide (5-{4-[(2-cyclopropyl-imidazo[5,1-b]thiazol-3-yl)-hydroxy-méthyl]-[1,2,3]triazol-1-yl}-pyridin-2-yloxy)-acétique ;
(2-cyclopropyl-imidazo[5,1-*b*]thiazol-3-yl)-[1-(6-éthoxy-5-trifluorométhyl-pyridin-3-yl)-1*H*-[1,2,3]triazol-4-yl]-méthanol ;
[1-(6-benzyloxy-pyridin-3-yl)-1*H*-[1,2,3]triazol-4-yl]-(2-cyclopropy1-imidazo[5,1-*b*]thiazol-3-yl)-méthanol ;
(2-cyclopropyl-imidazo[5,1-*b*]thiazol-3-yl)-{1-[6-(2,2,2-trifluoro-éthoxy)-pyridin-3-yl]-1*H*-[1,2,3]triazol-4-yl}-méthanol ;
(2-cyclopropyl-imidazo[5,1-*b*]thiazol-3-yl)-[1-(5-fluoro-6-méthoxy-pyridin-3-yl)-1*H*-[1,2,3]triazol-4-yl]-méthanol ;
(2-cyclopropyl-imidazo[5,1-*b*]thiazol-3-yl)-[1-(5,6-diméthoxy-pyridin-3-yl)-1*H*-[1,2,3]triazol-4-yl]-méthanol ;
(2-cyclopropyl-imidazo[5,1-*b*]thiazol-3-yl)-[1-(2,6-diméthoxy-pyridin-3-yl)-1*H*-[1,2,3]triazol-4-yl]-méthanol ;
(2-cyclopropyl-imidazo[5,1-*b*]thiazol-3-yl)-[1-(6-isopropoxy-pyridin-3-yl)-1*H*-[1,2,3]triazol-4-yl]-méthanol ;
[1-(2-cyclobutoxy-pyrimidin-5-yl)-1*H*-[1,2,3]triazol-4-yl]-(2-cyclopropyl-imidazo[5,1-*b*]thiazol-3-yl)-méthanol ;
(2-cyclopropyl-imidazo[5,1-*b*]thiazol-3-yl)-[1-(2,4-diméthoxy-pyrimidin-5-yl)-1*H*-[1,2,3]triazol-4-yl]-méthanol ;
[1-(2-benzyloxy-pyrimidin-5-yl)-1*H*-[1,2,3]triazol-4-yl]-(2-cyclopropyl-imidazo[5,1-*b*]thiazol-3-yl)-méthanol ;
(2-cyclopropyl-imidazo[5,1-*b*]thiazol-3-yl)-[1-(3,6-diméthoxy-pyridazin-4-yl)-1*H*-[1,2,3]triazol-4-yl]-méthanol ;
(2-cyclopropyl-imidazo[5,1-*b*]thiazol-3-yl)-{1-[4-(tétrahydro-pyran-4-ylméthoxy)-phényl]-1*H*-[1,2,3]triazol-4-yl}-méthanol ;
(2-cyclopropyl-imidazo[5,1-*b*]thiazol-3-yl)-{1-[4-(oxétan-3-ylméthoxy)-phényl]-1*H*-[1,2,3]triazol-4-yl}-méthanol ;
(2-cyclopropyl-imidazo[5,1-*b*]thiazol-3-yl)-{1-[4-(2-pipéridin-1-yl-éthoxy)-phényl]-1*H*-[1,2,3]triazol-4-yl}-méthanol ;
(2-cyclopropyl-imidazo[5,1-*b*]thiazol-3-yl)-{1-[4-(2-méthoxy-éthoxy)-phényl]-1*H*-[1,2,3]triazol-4-yl}-méthanol ;
[2-(1-fluoro-cyclopropyl)-imidazo[5,1-*b*]thiazol-3-yl]-[1-(4-méthoxy-phényl)-1*H*-[1,2,3]triazol-4-yl]-méthanol ;
N-(4-{4-[(2-cyclopropyl-imidazo[5,1-*b*]thiazol-3-yl)-hydroxy-méthyl]-[1,2,3]triazol-1-yl}-phényl)-acétamide ;
(2-méthyl-imidazo[5,1-*b*]thiazol-3-yl)-(1-phényl-1*H*-pyrazol-4-yl)-méthanol ; [1-(4-méthoxy-phényl)-1*H*-pyrazol-4-yl]-(2-méthyl-imidazo[5,1-*b*]thiazol-3-yl)-méthanol ;
(2-méthyl-imidazo[5,1-*b*]thiazol-3-yl)-(5-méthyl-1-phényl-1*H*-pyrazol-4-yl)-méthanol ;
(2-cyclopropyl-imidazo[5,1-*b*]thiazol-3-yl)-[1-(4-méthoxy-phényl)-1*H*-pyrazol-4-yl]-méthanol ;
(2-cyclopropyl-imidazo[5,1-*b*]thiazol-3-yl)-(1-phényl-1*H*-pyrazol-4-yl)-méthanol ;
(*S*)-(2-cyclopropyl-imidazo[5,1-*b*]thiazol-3-yl)-(1-phényl-1*H*-pyrazol-4-yl)-méthanol ;
(2-cyclopropyl-imidazo[5,1-*b*]thiazol-3-yl)-(5-méthyl-1-phényl-1*H*-[1,2,3]triazol-4-yl)-méthanol ;
4-{4-[(2-cyclopropyl-imidazo[5,1-*b*]thiazol-3-yl)-hydroxy-méthyl]-[1,2,3]triazol-1-yl}-*N*-(2-méthoxy-éthyl)-benzamide ;
(4-{4-[(2-cyclopropyl-imidazo[5,1-*b*]thiazol-3-yl)-hydroxy-méthyl]-[1,2,3]triazol-1-yl}-phényl)-morpholin-4-yl-méthanone ;
(4-{4-[(2-cyclopropyl-imidazo[5,1-*b*]thiazol-3-yl)-hydroxy-méthyl]-[1,2,3]triazol-1-yl}-phényl)-amide de l'acide morpholin-4-carboxylique ;
*N*-cyclopropyl-4-{4-[(2-cyclopropyl-imidazo[5,1-*b*]thiazol-3-yl)-hydroxy-méthyl]-[1,2,3]triazol-1-yl}-benzamide ;
4-{4-[(2-cyclopropyl-imidazo[5,1-*b*]thiazol-3-yl)-hydroxy-méthyl]-[1,2,3]triazol-1-yl}-N-(3-méthoxy-propyl)-benzamide ;
4-{4-[(2-cyclopropyl-imidazo[5,1-*b*]thiazol-3-yl)-hydroxy-méthyl]-[1,2,3]triazol-1-yl}-*N*-éthyl-benzamide ;
*N*-(2-cyano-éthyl)-4-{4-[(2-cyclopropyl-imidazo[5,1-*b*]thiazol-3-yl)-hydroxy-méthyl]-[1,2,3]triazol-1-yl}-benzamide ;
(4-{4-[(2-cyclopropyl-imidazo[5,1-*b*]thiazol-3-yl)-hydroxy-méthyl]-[1,2,3]triazol-1-yl}-phényl)-urée ;
*N*-(4-{4-[(2-éthyl-imidazo[5,1-*b*]thiazol-3-yl)-hydroxy-méthyl]-[1,2,3]triazol-1-yl}-phényl)-acétamide ;
4-{4-[(2-éthyl-imidazo[5,1-*b*]thiazol-3-yl)-hydroxy-méthyl]-[1,2,3]triazol-1-yl}-*N*-méthyl-benzamide ;
5-{4-[(2-éthyl-imidazo[5,1-*b*]thiazol-3-yl)-hydroxy-méthyl]-[1,2,3]triazol-1-yl}-1,3-dihydro-benzoimidazol-2-one ;
1-éthyl-3-(4-{4-[(2-éthyl-imidazo[5,1-*b*]thiazol-3-yl)-hydroxy-méthyl]-[1,2,3]triazol-1-yl}-2-méthoxy-phényl)-urée ;
4-{4-[(2-éthyl-imidazo[5,1-*b*]thiazol-3-yl)-hydroxy-méthyl]-[1,2,3]triazol-1-yl}-*N*,*N*-diméthyl-benzamide ;
4-{4-[(2-éthyl-imidazo[5,1-*b*]thiazol-3-yl)-hydroxy-méthyl]-[1,2,3]triazol-1-yl}-*N*-(3-hydroxy-propyl)-benzamide ;
4-{4-[(2-éthyl-imidazo[5,1-*b*]thiazol-3-yl)-hydroxy-méthyl]-[1,2,3]triazol-1-yl}-benzamide ;
(4-{4-[(2-éthyl-imidazo[5,1-*b*]thiazol-3-yl)-hydroxy-méthyl]-[1,2,3]triazol-1-yl}-phényl)-morpholin-4-yl-méthanone ;
4-{4-[(2-éthyl-imidazo[5,1-*b*]thiazol-3-yl)-hydroxy-méthyl]-[1,2,3]triazol-1-yl}-*N*-(2-hydroxy-éthyl)-benzamide ;
*N*-(2-diméthylamino-éthyl)-4-{4-[(2-éthyl-imidazo[5,1-*b*]thiazol-3-yl)-hydroxy-méthyl]-[1,2,3]triazol-1-yl}-benzamide ;
*N*-(4-{4-[(2-éthyl-imidazo[5,1-*b*]thiazol-3-yl)-hydroxy-méthyl]-[1,2,3]triazol-1-yl}-phényl)-2,2-diméthyl-propionamide ;
(4-{4-[(2-éthyl-imidazo[5,1-*b*]thiazol-3-yl)-hydroxy-méthyl]-[1,2,3]triazol-1-yl}-phényl)-amide de l'acide morpholin-4-carboxylique ;
1-butyl-3-(4-{4-[(2-éthyl-imidazo[5,1-*b*]thiazol-3-yl)-hydroxy-méthyl]-[1,2,3]triazol-1-yl}-phényl)-urée ;
4-{4-[(2-éthyl-imidazo[5,1-*b*]thiazol-3-yl)-hydroxy-méthyl]-[1,2,3]triazol-1-yl}-*N*-furan-2-ylméthyl-benzamide ;
*N*-éthyl-4-{4-[(2-éthyl-imidazo[5,1-*b*]thiazol-3-yl)-hydroxy-méthyl]-[1,2,3]triazol-1-yl}-*N*-méthyl-benzamide ;
*N*-cyclopropyl-4-{4-[(2-éthyl-imidazo[5,1-*b*]thiazol-3-yl)-hydroxy-méthyl]-[1,2,3]triazol-1-yl}-benzamide ;
4-{4-[(2-éthyl-imidazo[5,1-*b*]thiazol-3-yl)-hydroxy-méthyl]-[1,2,3]triazol-1-yl}-*N*-(3-méthoxy-propyl)-benzamide ;
*N*-(4-{4-[(2-éthyl-imidazo[5,1-*b*]thiazol-3-yl)-hydroxy-méthyl]-[1,2,3]triazol-1-yl}-phényl)-isobutyramide ;
*N*-éthyl-4-{4-[(2-éthyl-imidazo[5,1-*b*]thiazol-3-yl)-hydroxy-méthyl]-[1,2,3]triazol-1-yl}-benzamide ;
*N*-allyl-4-{4-[(2-éthyl-imidazo[5,1-*b*]thiazol-3-yl)-hydroxy-méthyl]-[1,2,3]triazol-1-yl}-benzamide ;
*N*-(2-cyano-éthyl)-4-{4-[(2-éthyl-imidazo[5,1-*b*]thiazol-3-yl)-hydroxy-méthyl]-[1,2,3]triazol-1-yl}-benzamide ;
(4-{4-[(2-éthyl-imidazo[5,1-*b*]thiazol-3-yl)-hydroxy-méthyl]-[1,2,3]triazol-1-yl}-phényl)-urée ;
ester méthylique de l'acide (4-{4-[(2-cyclopropyl-imidazo[5,1-b]thiazol-3-yl)-hydroxy-méthyl]-pyrazol-1-yl}-phényl)-carbamique ;
(*R*)-(2-cyclopropyl-imidazo[5,1-*b*]thiazol-3-yl)-[1-(4-cyclopropylméthoxy-phényl)-1*H*-[1,2,3]triazol-4-yl]-méthanol ;
(2-cyclopropyl-imidazo[5,1-*b*]thiazol-3-yl)-{1-[4-(tétrahydro-furan-2-ylméthoxy)-phényl]-1*H*-[1,2,3]triazol-4-yl}-méthanol ;
(1-phényl-1*H*-[1,2,3]triazol-4-yl)-(2-trifluorométhyl-imidazo[5,1-*b*]thiazol-3-yl)-méthanol ;
[1-(4-méthoxy-phényl)-1*H*-[1,2,3]triazol-4-yl]-(2-trifluorométhyl-imidazo[5,1-*b*]thiazol-3-yl)-méthanol ;
5-{4-[(2-cyclopropyl-imidazo[5,1-*b*]thiazol-3-yl)-hydroxy-méthyl]-[1,2,3]triazol-1-yl}-2-méthoxy-benzonitrile ;
2-(4-{4-[(2-cyclopropyl-imidazo[5,1-*b*]thiazol-3-yl)-hydroxy-méthyl]-[1,2,3]triazol-1-yl}-phénoxy)-éthanol ;
3-(4-{4-[(2-cyclopropyl-imidazo[5,1-*b*]thiazol-3-yl)-hydroxy-méthyl]-[1,2,3]triazol-1-yl}-phénoxy)-propan-1-ol ;
3-(4-{4-[(*R*)-(2-cyclopropyl-imidazo[5,1-*b*]thiazol-3-yl)-hydroxy-méthyl]-[1,2,3]triazol-1-yl}-phénoxy)-propan-1-ol ;
2-[2-(4-{4-[(2-cyclopropyl-imidazo[5,1-*b*]thiazol-3-yl)-hydroxy-méthyl]-[1,2,3]triazol-1-yl}-phénoxy)-éthoxy]-éthanol ;
1-(4-{4-[(2-cyclopropyl-imidazo[5,1-*b*]thiazol-3-yl)-hydroxy-méthyl]-[1,2,3]triazol-1-yl}-phénoxy)-2-méthyl-propan-2-ol ;

4-(4-{4-[(2-cyclopropyl-imidazo[5,1-*b*]thiazol-3-yl)-hydroxy-méthyl]-[1,2,3]triazol-1-yl}-phénoxy)-2-méthyl-butan-2-ol ;
3-(4-{4-[(2-cyclopropyl-imidazo[5,1-*b*]thiazol-3-yl)-hydroxy-méthyl]-[1,2,3]triazol-1-yl}-phénoxy)-2,2-diméthyl-propan-1-ol ;
1-[2-(4-{4-[(2-cyclopropyl-imidazo[5,1-*b*]thiazol-3-yl)-hydroxy-méthyl]-[1,2,3]triazol-1-yl}-phénoxy)-éthyl]-cyclopropanol ;
1-[2-(4-{4-[(*R*)-(2-cyclopropyl-imidazo[5,1-*b*]thiazol-3-yl)-hydroxy-méthyl]-[1,2,3]triazol-1-yl}-phénoxy)-éthyl]-cyclopropanol ;
(2-cyclopropyl-imidazo[5,1-*b*]thiazol-3-yl)-{1-[4-(tétrahydro-pyran-2-ylméthoxy)-phényl]-1*H*-[1,2,3]triazol-4-yl}-méthanol ;
(2-cyclopropyl-imidazo[5,1-*b*]thiazol-3-yl)-{1-[4-(3-méthyl-oxétan-3-ylméthoxy)-phényl]-1*H*-[1,2,3]triazol-4-yl}-méthanol ;
(2-cyclopropyl-imidazo[5,1-*b*]thiazol-3-yl)-{1-[4-(3-fluoro-oxétan-3-ylméthoxy)-phényl]-1*H*-[1,2,3]triazol-4-yl}-méthanol ;
(*R*)-(2-cyclopropyl-imidazo[5,1-*b*]thiazol-3-yl)-{1-[4-(3-fluoro-oxétan-3-ylméthoxy)-phényl]-1*H*-[1,2,3]triazol-4-yl}-méthanol ;
(2-cyclopropyl-imidazo[5,1-*b*]thiazol-3-yl)-{1-[4-(1-méthyl-pipéridin-3-ylméthoxy)-phényl]-1*H*-[1,2,3]triazol-4-yl}-méthanol ;
(2-cyclopropyl-imidazo[5,1-*b*]thiazol-3-yl)-{1-[4-(1,1-dioxo-hexahydro-1l6-thiopyran-4-ylméthoxy)-phényl]-1*H*-[1,2,3]triazol-4-yl}-méthanol ;
2-chloro-4-{4-[(2-cyclopropyl-imidazo[5,1-*b*]thiazol-3-yl)-hydroxy-méthyl]-[1,2,3]triazol-1-yl}-phénol ;
2-chloro-4-{4-[(*R*)-(2-cyclopropyl-imidazo[5,1-*b*]thiazol-3-yl)-hydroxy-méthyl]-[1,2,3]triazol-1-yl}-phénol ;
4-{4-[(2-cyclopropyl-imidazo[5,1-*b*]thiazol-3-yl)-hydroxy-méthyl]-[1,2,3]triazol-1-yl}-2,6-difluoro-phénol ;
4-{4-[(2-cyclopropyl-imidazo[5,1-*b*]thiazol-3-yl)-hydroxy-méthyl]-[1,2,3]triazol-1-yl}-2-fluoro-phénol ;
ester *tert*-butylique de l'acide (4-{4-[(2-cyclopropyl-imidazo[5,1-*b*]thiazol-3-yl)-hydroxy-méthyl]-[1,2,3]triazol-1-yl}-3-fluoro-phényl)-carbamique ;
biphényl-4-yl-(2-cyclopropyl-imidazo[5,1-*b*]thiazol-3-yl)-méthanol ;
biphényl-3-yl-(2-cyclopropyl-imidazo[5,1-*b*]thiazol-3-yl)-méthanol ;
(2-méthyl-imidazo[5,1-*b*]thiazol-3-yl)-(5-phényl-thiophén-2-yl)-méthanol ;
(2-méthyl-imidazo[5,1-*b*]thiazol-3-yl)-(2-phényl-thiazol-5-yl)-méthanol ;
(2-méthyl-imidazo[5,1-*b*]thiazol-3-yl)-(3-phényl-isoxazol-5-yl)-méthanol ;
(2-cyclopropyl-imidazo[5,1-*b*]thiazol-3-yl)-(5-phényl-[1,3,4]oxadiazol-2-yl)-méthanol ;
(2-cyclopropyl-imidazo[5,1-*b*]thiazol-3-yl)-(2-phényl-2*H*-[1,2,3]triazol-4-yl)-méthanol ;
3-{5-[(2-cyclopropyl-imidazo[5,1-*b*]thiazol-3-yl)-hydroxy-méthyl]-thiophén-2-yl}-benzonitrile ;
(2-cyclopropyl-imidazo[5,1-*b*]thiazol-3-yl)-(5-pyridin-3-yl-thiophén-2-yl)-méthanol ;
(2-cyclopropyl-imidazo[5,1-*b*]thiazol-3-yl)-[5-(4-méthoxy-phényl)-thiophén-2-yl]-méthanol ;
(2-cyclopropyl-imidazo[5,1-*b*]thiazol-3-yl)-[5-(4-trifluorométhyl-phényl)-thiophén-2-yl]-méthanol ;
(2-cyclopropyl-imidazo[5,1-*b*]thiazol-3-yl)-(3-phényl-[1,2,4]oxadiazol-5-yl)-méthanol ;
(2-cyclopropyl-imidazo[5,1-*b*]thiazol-3-yl)-(5-phényl-thiophén-2-yl)-méthanol ;
(2-cyclopropyl-imidazo[5,1-*b*]thiazol-3-yl)-[5-(3-trifluorométhyl-phényl)-thiophén-2-yl]-méthanol ;
(2-cyclopropyl-imidazo[5,1-*b*]thiazol-3-yl)-[5-(2-méthoxy-phényl)-thiophén-2-yl]-méthanol ;
(2-cyclopropyl-imidazo[5,1-*b*]thiazol-3-yl)-[5-(3-méthoxy-phényl)-thiophén-2-yl]-méthanol ;
(2-cyclopropyl-imidazo[5,1-*b*]thiazol-3-yl)-(5-pyridin-4-yl-thiophén-2-yl)-méthanol ;
(2-cyclopropyl-imidazo[5,1-*b*]thiazol-3-yl)-[5-(2-méthyl-2H-pyrazol-3-yl)-thiophén-2-yl]-méthanol ;
(2-cyclopropyl-imidazo[5,1-*b*]thiazol-3-yl)-[5-(2-trifluorométhyl-phényl)-thiophén-2-yl]-méthanol ;
(2-cyclopropyl-imidazo[5,1-*b*]thiazol-3-yl)-(5-phényl-thiazol-2-yl)-méthanol ;
2-{5-[(2-cyclopropyl-imidazo[5,1-*b*]thiazol-3-yl)-hydroxy-méthyl]-thiophén-2-yl}-benzonitrile ;
4-{5-[(2-cyclopropyl-imidazo[5,1-*b*]thiazol-3-yl)-hydroxy-méthyl]-thiophén-2-yl}-benzonitrile ;
(2-cyclopropyl-imidazo[5,1-*b*]thiazol-3-yl)-[5-(3-méthyl-3*H*-[1,2,3]triazol-4-yl)-thiophén-2-yl]-méthanol ;
(2-cyclopropyl-imidazo[5,1-*b*]thiazol-3-yl)-(5-phényl-[1,3,4]thiadiazol-2-yl)-méthanol ;
(2-cyclopropyl-imidazo[5,1-*b*]thiazol-3-yl)-[5-(1*H*-indol-5-yl)-thiophén-2-yl]-méthanol ;
(2-cyclopropyl-imidazo[5,1-*b*]thiazol-3-yl)-(5-isoquinoléin-4-yl-thiophén-2-yl)-méthanol ;
(2-cyclopropyl-imidazo[5,1-*b*]thiazol-3-yl)-(1-phényl-1*H*-imidazol-4-yl)-méthanol ;
[2-(*cis*-2-fluoro-cyclopropyl)-imidazo[5,1-*b*]thiazol-3-yl]-[1-(4-méthoxy-phényl)-1*H*-[1,2,3]triazol-4-yl]-méthanol ; et
[2-(*trans*-2-fluoro-cyclopropyl)-imidazo[5,1-*b*]thiazol-3-yl]-[1-(4-méthoxy-phényl)-1*H*-[1,2,3]triazol-4-yl]-méthanol ;

ou sel pharmaceutiquement acceptable de celui-ci.

**11.** Composé selon la revendication 1 sélectionné dans un groupe consistant en les suivants :

ester méthylique de l'acide (4-{4-[(*R*)-(2-éthyl-imidazo[5,1-*b*]thiazol-3-yl)-hydroxy-méthyl]-[1,2,3]triazol-1-yl}-phényl)-carbamique ;
(*R*)-(2-cyclopropyl-imidazo[5,1-*b*]thiazol-3-yl)-{1-[4-(tétrahydro-pyran-4-ylméthoxy)-phényl]-1*H*-[1,2,3]triazol-4-yl}-méthanol ;
1-(4-{4-[(*R*)-(2-cyclopropyl-imidazo[5,1-*b*]thiazol-3-yl)-hydroxy-méthyl]-[1,2,3]triazol-1-yl}-phénoxy)-2-méthyl-propan-2-ol ;
(*R*)-(2-cyclopropyl-imidazo[5,1-*b*]thiazol-3-yl)-(5-méthyl-1-phényl-1*H*-[1,2,3]triazol-4-yl)-méthanol ;
6-{4-[(2-cyclopropyl-imidazo[5,1-*b*]thiazol-3-yl)-hydroxy-méthyl]-[1,2,3]triazol-1-yl}-3*H*-benzooxazol-2-one ;
ester *tert*-butylique de l'acide (4-{4-[(2-cyclopropyl-imidazo[5,1-b]thiazol-3-yl)-hydroxy-méthyl]-[1,2,3]triazol-1-yl}-phényl)-carbamique ;
6-{4-[(2-éthyl-imidazo[5,1-*b*]thiazol-3-yl)-hydroxy-méthyl]-[1,2,3]triazol-1-yl}-3*H*-benzooxazol-2-one ;
4-{4-[(2-cyclopropyl-imidazo[5,1-*b*]thiazol-3-yl)-hydroxy-méthyl]-[1,2,3]triazol-1-yl}-*N*-méthyl-benzamide ;
1-(4-{4-[(2-cyclopropyl-imidazo[5,1-*b*]thiazol-3-yl)-hydroxy-méthyl]-[1,2,3]triazol-1-yl}-2-méthoxy-phényl)-3-éthyl-urée ;
4-{4-[(2-cyclopropyl-imidazo[5,1-*b*]thiazol-3-yl)-hydroxy-méthyl]-[1,2,3]triazol-1-yl}-*N*-(3-hydroxy-propyl)-benzamide ;
4-{4-[(2-cyclopropyl-imidazo[5,1-*b*]thiazol-3-yl)-hydroxy-méthyl]-[1,2,3]triazol-1-yl}-benzamide ;
4-{4-[(2-cyclopropyl-imidazo[5,1-*b*]thiazol-3-yl)-hydroxy-méthyl]-[1,2,3]triazol-1-yl}-*N*-(2-hydroxy-éthyl)-benzamide ;
*N*-(4-{4-[(2-cyclopropyl-imidazo[5,1-*b*]thiazol-3-yl)-hydroxy-méthyl]-[1,2,3]triazol-1-yl}-phényl)-2,2-diméthyl-propionamide ;
1-butyl-3-(4-{4-[(2-cyclopropyl-imidazo[5,1-*b*]thiazol-3-yl)-hydroxy-méthyl]-[1,2,3]triazol-1-yl}-phényl)-urée ;
*N*-(4-{4-[(2-cyclopropyl-imidazo[5,1-*b*]thiazol-3-yl)-hydroxy-méthyl]-[1,2,3]triazol-1-yl}-phényl)-benzamide ;
4-{4-[(2-cyclopropyl-imidazo[5,1-*b*]thiazol-3-yl)-hydroxy-méthyl]-[1,2,3]triazol-1-yl}-*N*-furan-2-ylméthyl-benzamide ;
4-{4-[(2-cyclopropyl-imidazo[5,1-*b*]thiazol-3-yl)-hydroxy-méthyl]-[1,2,3]triazol-1-yl}-*N*-éthyl-*N*-méthyl-benzamide ;
*N*-(4-{4-[(2-cyclopropyl-imidazo[5,1-*b*]thiazol-3-yl)-hydroxy-méthyl]-[1,2,3]triazol-1-yl}-phényl)-isobutyramide ;
*N*-(4-{4-[(*R*)-(2-cyclopropyl-imidazo[5,1-*b*]thiazol-3-yl)-hydroxy-méthyl]-[1,2,3]triazol-1-yl}-phényl)-isobutyramide ;
*N*-allyl-4-{4-[(2-cyclopropyl-imidazo[5,1-*b*]thiazol-3-yl)-hydroxy-méthyl]-[1,2,3]triazol-1-yl}-benzamide ;
5-{4-[(2-cyclopropyl-imidazo[5,1-*b*]thiazol-3-yl)-hydroxy-méthyl]-[1,2,3]triazol-1-yl}-1,3-dihydro-benzoimidazol-2-one ;
4-{4-[(2-cyclopropyl-imidazo[5,1-*b*]thiazol-3-yl)-hydroxy-méthyl]-[1,2,3]triazol-1-yl}-*N*,*N*-diméthyl-benzamide ;
4-{4-[(2-éthyl-imidazo[5,1-*b*]thiazol-3-yl)-hydroxy-méthyl]-[1,2,3]triazol-1-yl}-*N*-(2-méthoxy-éthyl)-benzamide ;
3-{4-[(2-cyclopropyl-imidazo[5,1-*b*]thiazol-3-yl)-hydroxy-méthyl]-[1,2,3]triazol-1-yl}-5-méthoxy-benzonitrile ;
5-{4-[(2-cyclopropyl-imidazo[5,1-*b*]thiazol-3-yl)-hydroxy-méthyl]-[1,2,3]triazol-1-yl}-2,3-diméthoxy-benzonitrile ;
5-{4-[(2-cyclopropyl-imidazo[5,1-*b*]thiazol-3-yl)-hydroxy-méthyl]-[1,2,3]triazol-1-yl}-2-trifluorométhoxy-benzonitrile ;
[1-(4-bromo-3-chloro-pyridin-2-yl)-1*H*-[1,2,3]triazol-4-yl]-(2-cyclopropyl-imidazo[5,1-*b*]thiazol-3-yl)-méthanol ;
2-{2-[2-(4-{4-[(2-cyclopropyl-imidazo[5,1-*b*]thiazol-3-yl)-hydroxy-méthyl]-[1,2,3]triazol-1-yl}-phénoxy)-éthoxy]-éthoxy}-éthanol ;
(*S*)-3-(4-{4-[(*R*)-(2-cyclopropyl-imidazo[5,1-*b*]thiazol-3-yl)-hydroxy-méthyl]-[1,2,3]triazol-1-yl}-phénoxy)-propane-1,2-diol ;
(*S*)-3-(4-{4-[(*S*)-(2-cyclopropyl-imidazo[5,1-*b*]thiazol-3-yl)-hydroxy-méthyl]-[1,2,3]triazol-1-yl}-phénoxy)-propane-1,2-diol ;
(2-cyclopropyl-imidazo[5,1-*b*]thiazol-3-yl)-{1-[4-(pyridin-2-ylméthoxy)-phényl]-1*H*-[1,2,3]triazol-4-yl}-méthanol ;
(2-cyclopropyl-imidazo[5,1-*b*]thiazol-3-yl)-{1-[4-(2-diméthylamino-éthoxy)-phényl]-1*H*-[1,2,3]triazol-4-yl}-méthanol ;
(2-cyclopropyl-imidazo[5,1-*b*]thiazol-3-yl)-{1-[4-(2-méthanesulfonyl-éthoxy)-phényl]-1*H*-[1,2,3]triazol-4-yl}-méthanol ;
amide de l'acide 3-(4-{4-[(2-cyclopropyl-imidazo[5,1-*b*]thiazol-3-yl)-hydroxy-méthyl]-[1,2,3]triazol-1-yl}-phénoxy)-propane-1-sulfonique ;
4-{4-[(2-cyclopropyl-imidazo[5,1-*b*]thiazol-3-yl)-hydroxy-méthyl]-[1,2,3]triazol-1-yl}-3-méthyl-phénol ;
4-{4-[(2-cyclopropyl-imidazo[5,1-*b*]thiazol-3-yl)-hydroxy-méthyl]-[1,2,3]triazol-1-yl}-3-fluoro-phénol ;
4-{4-[(2-cyclopropyl-imidazo[5,1-*b*]thiazol-3-yl)-hydroxy-méthyl]-[1,2,3]triazol-1-yl}-2-trifluorométhyl-phénol ;
4-{4-[(2-cyclopropyl-imidazo[5,1-*b*]thiazol-3-yl)-hydroxy-méthyl]-[1,2,3]triazol-1-yl}-2-trifluorométhoxy-phénol ;
4-{4-[(2-cyclopropyl-imidazo[5,1-*b*]thiazol-3-yl)-hydroxy-méthyl]-[1,2,3]triazol-1-yl}-2-méthyl-phénol ;
3-chloro-4-{4-[(2-cyclopropyl-imidazo[5,1-*b*]thiazol-3-yl)-hydroxy-méthyl]-[1,2,3]triazol-1-yl}-phénol ;
4-{4-[(2-cyclopropyl-imidazo[5,1-*b*]thiazol-3-yl)-hydroxy-méthyl]-[1,2,3]triazol-1-yl}-3-trifluorométhoxy-phénol ;
4-{4-[(2-cyclopropyl-imidazo[5,1-*b*]thiazol-3-yl)-hydroxy-méthyl]-[1,2,3]triazol-1-yl}-3-trifluorométhyl-phénol ;
3-{4-[(2-cyclopropyl-imidazo[5,1-*b*]thiazol-3-yl)-hydroxy-méthyl]-[1,2,3]triazol-1-yl}-phénol ;
5-{4-[(2-cyclopropyl-imidazo[5,1-*b*]thiazol-3-yl)-hydroxy-méthyl]-[1,2,3]triazol-1-yl}-2-méthoxy-phénol ;
2-chloro-5-{4-[(2-cyclopropyl-imidazo[5,1-*b*]thiazol-3-yl)-hydroxy-méthyl]-[1,2,3]triazol-1-yl}-phénol ;
5-{4-[(2-cyclopropyl-imidazo[5,1-*b*]thiazol-3-yl)-hydroxy-méthyl]-[1,2,3]triazol-1-yl}-2-méthyl-phénol ;
3-{4-[(2-cyclopropyl-imidazo[5,1-*b*]thiazol-3-yl)-hydroxy-méthyl]-[1,2,3]triazol-1-yl}-5-trifluorométhyl-phénol ;
3-{4-[(2-cyclopropyl-imidazo[5,1-*b*]thiazol-3-yl)-hydroxy-méthyl]-[1,2,3]triazol-1-yl}-5-méthyl-phénol ;
5-chloro-2-{4-[(2-cyclopropyl-imidazo[5,1-*b*]thiazol-3-yl)-hydroxy-méthyl]-[1,2,3]triazol-1-yl}-phénol ;
(2-cyclopropyl-imidazo[5,1-*b*]thiazol-3-yl)-[1-(2,3-dihydro-benzo[1,4]dioxin-6-yl)-1*H*-pyrazol-4-yl]-méthanol ;
(2-cyclopropyl-imidazo[5,1-*b*]thiazol-3-yl)-[1-(6-méthoxy-pyridin-3-yl)-1*H*-pyrazol-4-yl]-méthanol ;
*N*-(4-{4-[(2-cyclopropyl-imidazo[5,1-*b*]thiazol-3-yl)-hydroxy-méthyl]-pyrazol-1-yl}-phényl)-acétamide ;
4-{4-[(2-éthyl-imidazo[5,1-*b*]thiazol-3-yl)-hydroxy-méthyl]-[1,2,3]triazol-1-yl}-*N*-méthyl-benzènesulfonamide ;
4-{4-[(2-cyclopropyl-imidazo[5,1-*b*]thiazol-3-yl)-hydroxy-méthyl]-[1,2,3]triazol-1-yl}-*N*-méthyl-benzènesulfonamide ;
(2-éthyl-imidazo[5,1-*b*]thiazol-3-yl)-[1-(3-méthyl-1,1-dioxo-2,3-dihydro-1*H*-1l6-benzo[*d*]isothiazol-5-yl)-1*H*-[1,2,3]triazol-4-yl]-méthanol ;
(2-cyclopropyl-imidazo[5,1-*b*]thiazol-3-yl)-[1-(3-méthyl-1,1-dioxo-2,3-dihydro-1*H*-1l6-benzo[*d*]isothiazol-5-yl)-1*H*-[1,2,3]triazol-4-yl]-méthanol ;
(2-cyclopropyl-imidazo[5,1-*b*]thiazol-3-yl)-[1-(1-méthyl-1*H*-pyrrolo[2,3-*b*]pyridin-5-yl)-1*H*-pyrazol-4-yl]-méthanol ;
*N*-(4-{4-[(2-éthyl-imidazo[5,1-*b*]thiazol-3-yl)-hydroxy-méthyl]-[1,2,3]triazol-1-yl}-phényl)-benzamide ;
ester éthylique de l'acide (4-{4-[(2-cyclopropyl-imidazo[5,1-*b*]thiazol-3-yl)-hydroxy-méthyl]-[1,2,3]triazol-1-yl}-phénoxy)-acétique ;
4-{4-[(2-cyclopropyl-imidazo[5,1-*b*]thiazol-3-yl)-hydroxy-méthyl]-[1,2,3]triazol-1-yl}-2-éthyl-phénol ;
4-{4-[(2-cyclopropyl-imidazo[5,1-*b*]thiazol-3-yl)-hydroxy-méthyl]-[1,2,3]triazol-1-yl}-2-hydroxy-benzonitrile ;
ester *tert*-butylique de l'acide (4-{4-[(2-éthyl-imidazo[5,1-*b*]thiazol-3-yl)-hydroxy-méthyl]-[1,2,3]triazol-1-yl}-phényl)-carbamique ;
4-{4-[(2-cyclopropyl-imidazo[5,1-*b*]thiazol-3-yl)-hydroxy-méthyl]-[1,2,3]triazol-1-yl}-benzènesulfonamide ;
4-{4-[(2-cyclopropyl-imidazo[5,1-*b*]thiazol-3-yl)-hydroxy-méthyl]-[1,2,3]triazol-1-yl}-*N*,*N*-diméthyl-benzènesulfonamide ;
(2-cyclopropyl-imidazo[5,1-b]thiazol-3-yl)-{1-[4-(pyrrolidin-1-sulfonyl)-phényl]-1*H*-[1,2,3]triazol-4-yl}-méthanol ;
4-{4-[(2-cyclopropyl-imidazo[5,1-*b*]thiazol-3-yl)-hydroxy-méthyl]-[1,2,3]triazol-1-yl}-*N*,*N*-diéthyl-benzènesulfonamide ;
4-{4-[(2-cyclopropyl-imidazo[5,1-*b*]thiazol-3-yl)-hydroxy-méthyl]-[1,2,3]triazol-1-yl}-*N*-éthyl-benzènesulfonamide ;
4-{4-[(2-éthyl-imidazo[5,1-*b*]thiazol-3-yl)-hydroxy-méthyl]-[1,2,3]triazol-1-yl}-benzènesulfonamide ;
4-{4-[(2-éthyl-imidazo[5,1-*b*]thiazol-3-yl)-hydroxy-méthyl]-[1,2,3]triazol-1-yl}-*N*,*N*-diméthyl-benzènesulfonamide ;
(2-éthyl-imidazo[5,1-*b*]thiazol-3-yl)-{1-[4-(pyrrolidin-1-sulfonyl)-phényl]-1*H*-[1,2,3]triazol-4-yl}-méthanol ;
*N*,*N*-diéthyl-4-{4-[(2-éthyl-imidazo[5,1-*b*]thiazol-3-yl)-hydroxy-méthyl]-[1,2,3]triazol-1-yl}-benzènesulfonamide ;
*N*-éthyl-4-{4-[(2-éthyl-imidazo[5,1-*b*]thiazol-3-yl)-hydroxy-méthyl]-[1,2,3]triazol-1-yl}-benzènesulfonamide ;
ester *tert*-butylique de l'acide (4-{4-[(2-éthyl-imidazo[5,1-*b*]thiazol-3-yl)-hydroxy-méthyl]-[1,2,3]triazol-1-yl}-3-fluoro-phényl)-carbamique ;
*N*-(4-{4-[(2-cyclopropyl-imidazo[5,1-*b*]thiazol-3-yl)-hydroxy-méthyl]-pyrazol-1-yl}-phényl)-isobutyramide ;
5-{4-[(2-cyclopropyl-imidazo[5,1-*b*]thiazol-3-yl)-hydroxy-méthyl]-pyrazol-1-yl}-2-méthoxy-benzonitrile ;
4-{4-[(2-cyclopropyl-imidazo[5,1-*b*]thiazol-3-yl)-hydroxy-méthyl]-[1,2,3]triazol-1-yl}-2-nitro-phénol ;
[1-(4-méthoxy-phényl)-1*H*-[1,2,3]triazol-4-yl]-(2-phényl-imidazo[5,1-*b*]thiazol-3-yl)-méthanol ;
4-{4-[hydroxy-(2-phényl-imidazo[5,1-*b*]thiazol-3-yl)-méthyl]-[1,2,3]triazol-1-yl}-2-nitro-phénol ;

ester *tert*-butylique de l'acide (4-{4-[hydroxy-(2-phényl-imidazo[5,1-*b*]thiazol-3-yl)-méthyl]-[1,2,3]triazol-1-yl}-phényl)-carbamique ;
(2-cyclopropyl-imidazo[5,1-*b*]thiazol-3-yl)-[1-(4-méthoxy-phényl)-5-méthyl-1*H*-[1,2,3]triazol-4-yl]-méthanol ;
(2-cyclopropyl-imidazo[5,1-*b*]thiazol-3-yl)-[1-(1*H*-indazol-5-yl)-1*H*-[1,2,3]triazol-4-yl]-méthanol ;
(2-cyclopropyl-imidazo[5,1-*b*]thiazol-3-yl)-[1-(1-méthyl-1*H*-indazol-7-yl)-1*H*-[1,2,3]triazol-4-yl]-méthanol ;
[1-(4-méthoxy-phényl)-5-méthyl-1*H*-[1,2,3]triazol-4-yl]-(2-méthyl-imidazo[5,1-*b*]thiazol-3-yl)-méthanol ;
4-{4-[(2-cyclopropyl-imidazo[5,1-*b*]thiazol-3-yl)-hydroxy-méthyl]-[1,2,3]triazol-1-yl}-((*N*-méthylsulfamoyl)amino)-benzène ;
[2-(1,1-difluoro-éthyl)-imidazo[5,1-*b*]thiazol-3-yl]-[1-(4-méthoxy-phényl)-1*H*-[1,2,3]triazol-4-yl]-méthanol ;
[1-(3-chloro-1*H*-indazol-6-yl)-1*H*-[1,2,3]triazol-4-yl]-(2-cyclopropyl-imidazo[5,1-*b*]thiazol-3-yl)-méthanol ;
(2-(cyclopent-1-ényl)-imidazo[5,1-*b*]thiazol-3-yl)-[1-(4-méthoxy-phényl)-1*H*-[1,2,3]triazol-4-yl]-méthanol ;
(2-cyclopropyl-imidazo[5,1-*b*]thiazol-3-yl)-(1-pyridin-3-yl-1*H*-pyrazol-4-yl)-méthanol ;
[1-(4-méthoxy-phényl)-1*H*-[1,2,3]triazol-4-yl]-(2-méthyl-imidazo[5,1-*b*]thiazol-3-yl)-méthanol ;
[1-(4-méthoxy-phényl)-1*H*-[1,2,3]triazol-4-yl]-[2-(2-méthyl-propényl)-imidazo[5,1-*b*]thiazol-3-yl]-méthanol ;
[1-(4-méthoxy-phényl)-1*H*-[1,2,3]triazol-4-yl]-[((*E*)-2-(prop-1-ényl))-imidazo[5,1-*b*]thiazol-3-yl]-méthanol ;
2-chloro-4-{4-[hydroxy-(2-phényl-imidazo[5,1-*b*]thiazol-3-yl)-méthyl]-[1,2,3]triazol-1-yl}-phénol ;
[1-(3-chloro-4-cyclopropylméthoxy-phényl)-1*H*-[1,2,3]triazol-4-yl]-(2-phényl-imidazo[5,1-*b*]thiazol-3-yl)-méthanol ;
{1-[3-chloro-4-(3-fluoro-oxétan-3-ylméthoxy)-phényl]-1*H*-[1,2,3]triazol-4-yl}-(2-phényl-imidazo[5,1-*b*]thiazol-3-yl)-méthanol ;
3-(2-chloro-4-{4-[hydroxy-(2-phényl-imidazo[5,1-*b*]thiazol-3-yl)-méthyl]-[1,2,3]triazol-1-yl}-phénoxy)-propan-1-ol ;
4-(2-chloro-4-{4-[hydroxy-(2-phényl-imidazo[5,1-*b*]thiazol-3-yl)-méthyl]-[1,2,3]triazol-1-yl}-phénoxy)-2-méthyl-butan-2-ol ;
4-(2-chloro-4-{4-[(*R*)-hydroxy-(2-phényl-imidazo[5,1-*b*]thiazol-3-yl)-méthyl]-[1,2,3]triazol-1-yl}-phénoxy)-2-méthyl-butan-2-ol ;
(2-isopropényl-imidazo[5,1-*b*]thiazol-3-yl)-[1-(4-méthoxy-phényl)-1*H*-[1,2,3]triazol-4-yl]-méthanol ;
[2-((*E*)-2-cyclopropyl-vinyl)-imidazo[5,1-*b*]thiazol-3-yl]-[1-(4-méthoxy-phényl)-1*H*-[1,2,3]triazol-4-yl]-méthanol ;
[1-(4-méthoxy-phényl)-1*H*-[1,2,3]triazol-4-yl]-[((*E*)-2-(pent-1-ényl))-imidazo[5,1-*b*]thiazol-3-yl]-méthanol ;
[1-(4-amino-3-chloro-phényl)-1*H*-[1,2,3]triazol-4-yl]-(2-éthyl-imidazo[5,1-*b*]thiazol-3-yl)-méthanol ;
[1-(4-méthoxy-phényl)-1*H*-[1,2,3]triazol-4-yl]-[2-(1-méthyl-1*H*-pyrazol-4-yl)-imidazo[5,1-*b*]thiazol-3-yl]-méthanol ;
[5-*tert*-butyl-1-(4-méthoxy-phényl)-1*H*-[1,2,3]triazol-4-yl]-(2-cyclopropyl-imidazo[5,1-*b*]thiazol-3-yl)-méthanol ;
3-(2-chloro-4-{4-[(*R*)-(2-cyclopropyl-imidazo[5,1-*b*]thiazol-3-yl)-hydroxy-méthyl]-[1,2,3]triazol-1-yl}-phénoxy)-propan-1-ol ;
(*R*)-{1-[3-chloro-4-(tétrahydro-pyran-4-ylméthoxy)-phényl]-1*H*-[1,2,3]triazol-4-yl}-(2-cyclopropyl-imidazo[5,1-*b*]thiazol-3-yl)-méthanol ;
4-(2-chloro-4-{4-[(R)-(2-cyclopropyl-imidazo[5,1-b]thiazol-3-yl)-hydroxy-méthyl]-[1,2,3]triazol-1-yl}-phénoxy)-2-méthyl-butan-2-ol ;
[1-(4-amino-3-chloro-phényl)-1*H*-[1,2,3]triazol-4-yl]-(2-cyclopropyl-imidazo[5,1-*b*]thiazol-3-yl)-méthanol ;
(2-cyclopropyl-imidazo[5,1-*b*]thiazol-3-yl)-[5-éthyl-1-(4-méthoxy-phényl)-1*H*-[1,2,3]triazol-4-yl]-méthanol ;
(2-cyclopropyl-imidazo[5,1-*b*]thiazol-3-yl)-[5-isopropyl-1-(4-méthoxy-phényl)-1*H*-[1,2,3]triazol-4-yl]-méthanol ;
(2-cyclopropyl-imidazo[5,1-*b*]thiazol-3-yl)-[5-cyclopropyl-1-(4-méthoxy-phényl)-1*H*-[1,2,3]triazol-4-yl]-méthanol ;
(*R*)-{1-[3-chloro-4-(3-fluoro-oxétan-3-ylméthoxy)-phényl]-1*H*-[1,2,3]triazol-4-yl}-(2-cyclopropyl-imidazo[5,1-*b*]thiazol-3-yl)-méthanol ;
1-(2-chloro-4-{4-[(*R*)-(2-cyclopropyl-imidazo[5,1-*b*]thiazol-3-yl)-hydroxy-méthyl]-[1,2,3]triazol-1-yl}-phénoxy)-2-méthyl-propan-2-ol ;
*N*-(2-chloro-4-{4-[(2-cyclopropyl-imidazo[5,1-*b*]thiazol-3-yl)-hydroxy-méthyl]-[1,2,3]triazol-1-yl}-phényl)-isobutyramide ;
(2-éthyl-imidazo[5,1-*b*]thiazol-3-yl)-[1-(4-méthoxy-phényl)-1*H*-[1,2,3]triazol-4-yl]-méthanol ;
[2-(4-fluoro-phényl)-imidazo[5,1-*b*]thiazol-3-yl]-[1-(4-méthoxy-phényl)-1*H*-[1,2,3]triazol-4-yl]-méthanol ;
[2-(2-fluoro-phényl)-imidazo[5,1-*b*]thiazol-3-yl]-[1-(4-méthoxy-phényl)-1*H*-[1,2,3]triazol-4-yl]-méthanol ;
[1-(3-chloro-5-fluoro-4-méthoxy-phényl)-1*H*-[1,2,3]triazol-4-yl]-(2-cyclopropyl-imidazo[5,1-*b*]thiazol-3-yl)-méthanol ;
[1-(3-chloro-4-méthoxy-phényl)-1*H*-[1,2,3]triazol-4-yl]-(2-cyclopropyl-imidazo[5,1-*b*]thiazol-3-yl)-méthanol ;
[1-(3-bromo-4-méthoxy-phényl)-1*H*-[1,2,3]triazol-4-yl]-(2-cyclopropyl-imidazo[5,1-*b*]thiazol-3-yl)-méthanol ;
(2-cyclopropyl-imidazo[5,1-*b*]thiazol-3-yl)-[1-(3-iodo-4-méthoxy-phényl)-1*H*-[1,2,3]triazol-4-yl]-méthanol ;

[1-(3-chloro-2-fluoro-4-méthoxy-phényl)-1*H*-[1,2,3]triazol-4-yl]-(2-cyclopropyl-imidazo[5,1-*b*]thiazol-3-yl)-méthanol ;
4-(2-chloro-4-{4-[hydroxy-(2-isopropényl-imidazo[5,1-*b*]thiazol-3-yl)-méthyl]-[1,2,3]triazol-1-yl}-phénoxy)-2-méthyl-butan-2-ol ;
ester méthylique de l'acide 3-{4-[(2-cyclopropyl-imidazo[5,1-b]thiazol-3-yl)-hydroxy-méthyl]-[1,2,3]triazol-1-ylméthyl}-benzoïque ;
[1-(3-chloro-4-méthoxy-phényl)-5-méthyl-1*H*-[1,2,3]triazol-4-yl]-(2-cyclopropyl-imidazo[5,1-*b*]thiazol-3-yl)-méthanol ;
[1-(3-chloro-4-éthoxy-phényl)-5-méthyl-1*H*-[1,2,3]triazol-4-yl]-(2-cyclopropyl-imidazo[5,1-*b*]thiazol-3-yl)-méthanol ;
ester méthylique de l'acide 4-{4-[(2-cyclopropyl-imidazo[5,1-*b*]thiazol-3-yl)-hydroxy-méthyl]-[1,2,3]triazol-1-ylméthyl}-benzoïque ;
3-{4-[(2-cyclopropyl-imidazo[5,1-*b*]thiazol-3-yl)-hydroxy-méthyl]-[1,2,3]triazol-1-ylméthyl}-phénol ;
{1-[3-chloro-4-(3-fluoro-oxétan-3-ylméthoxy)-phényl]-1*H*-[1,2,3]triazol-4-yl}-(2-isopropényl-imidazo[5,1-*b*]thiazol-3-yl)-méthanol ;
[1-(5-chloro-2-fluoro-4-méthoxy-phényl)-1*H*-[1,2,3]triazol-4-yl]-(2-cyclopropyl-imidazo[5,1-*b*]thiazol-3-yl)-méthanol ;
3-(2-chloro-4-{4-[hydroxy-(2-isopropényl-imidazo[5,1-*b*]thiazol-3-yl)-méthyl]-[1,2,3]triazol-1-yl}-phénoxy)-propan-1-ol ;
4-(2-chloro-4-{4-[(2-éthyl-imidazo[5,1-*b*]thiazol-3-yl)-hydroxy-méthyl]-[1,2,3]triazol-1-yl}-phénoxy)-2-méthyl-butan-2-ol ;
4-[2-chloro-4-(4-{hydroxy-[2-(1-méthyl-cyclopropyl)-imidazo[5,1-*b*]thiazol-3-yl]-méthyl}-[1,2,3]triazol-1-yl)-phénoxy]-2-méthyl-butan-2-ol ;
4-{4-[(2-cyclopropyl-imidazo[5,1-*b*]thiazol-3-yl)-hydroxy-méthyl]-[1,2,3]triazol-1-ylméthyl}-phénol ;
2-{4-[(2-cyclopropyl-imidazo[5,1-*b*]thiazol-3-yl)-hydroxy-méthyl]-[1,2,3]triazol-1-ylméthyl}-phénol ;
(2-cyclopropyl-imidazo[5,1-*b*]thiazol-3-yl)-[1-(4-méthoxy-2-méthyl-phényl)-1*H*-[1,2,3]triazol-4-yl]-méthanol ;
4-[2-chloro-4-(4-{[2-(1,1-difluoro-éthyl)-imidazo[5,1-*b*]thiazol-3-yl]-hydroxy-méthyl}-[1,2,3]triazol-1-yl)-phénoxy]-2-méthyl-butan-2-ol ;
4-[2-chloro-4-(4-{[2-(*trans*-2-fluoro-cyclopropyl)-imidazo[5,1-*b*]thiazol-3-yl]-hydroxy-méthyl}-[1,2,3]triazol-1-yl)-phénoxy]-2-méthyl-butan-2-ol ;
4-[2-chloro-4-(4-{[2-(*cis*-2-fluoro-cyclopropyl)-imidazo[5,1-*b*]thiazol-3-yl]-hydroxy-méthyl}-[1,2,3]triazol-1-yl)-phénoxy]-2-méthyl-butan-2-ol ;
(2-cyclopropyl-imidazo[5,1-*b*]thiazol-3-yl)-[1-(4-hydroxyméthyl-phényl)-1*H*-[1,2,3]triazol-4-yl]-méthanol ;
(2-cyclopropyl-imidazo[5,1-*b*]thiazol-3-yl)-[1-(4-méthoxy-2,6-diméthyl-phényl)-1*H*-[1,2,3]triazol-4-yl]-méthanol ;
(2-cyclopropyl-imidazo[5,1-*b*]thiazol-3-yl)-(1-{4-[2-(2,2,2-trifluoro-éthoxy)-éthoxy]-phényl}-1*H*-[1,2,3]triazol-4-yl)-méthanol ;
3-[2-chloro-4-(4-{hydroxy-[2-(1-méthyl-cyclopropyl)-imidazo[5,1-*b*]thiazol-3-yl]-méthyl}-[1,2,3]triazol-1-yl)-phénoxy]-propan-1-ol ;
3-[2-chloro-4-(4-{[2-(1,1-difluoro-éthyl)-imidazo[5,1-*b*]thiazol-3-yl]-hydroxy-méthyl}-[1,2,3]triazol-1-yl)-phénoxy]-propan-1-ol ;
{1-[3-chloro-4-(3-fluoro-oxétan-3-ylméthoxy)-phényl]-1*H*-[1,2,3]triazol-4-yl}-[2-(1-méthyl-cyclopropyl)-imidazo[5,1-*b*]thiazol-3-yl]-méthanol ;
{1-[3-chloro-4-(3-fluoro-oxétan-3-ylméthoxy)-phényl]-1*H*-[1,2,3]triazol-4-yl}-[2-(1,1-difluoro-éthyl)-imidazo[5,1-*b*]thiazol-3-yl]-méthanol ;
{1-[3-chloro-4-(3-fluoro-oxétan-3-ylméthoxy)-phényl]-1*H*-[1,2,3]triazol-4-yl}-(2-éthyl-imidazo[5,1-*b*]thiazol-3-yl)-méthanol ;
(2-cyclopropyl-imidazo[5,1-b]thiazol-3-yl)-[1-(2,5-difluoro-4-méthoxy-phényl)-1H-[1,2,3]triazol-4-yl]-méthanol ;
[1-(5-chloro-2-fluoro-4-méthoxy-phényl)-1*H*-[1,2,3]triazol-4-yl]-(2-éthyl-imidazo[5,1-*b*]thiazol-3-yl)-méthanol ;
3-(2-chloro-4-{4-[(2-éthyl-imidazo[5,1-*b*]thiazol-3-yl)-hydroxy-méthyl]-[1,2,3]triazol-1-yl}-phénoxy)-propan-1-ol ;
[1-(2,5-difluoro-4-méthoxy-phényl)-1*H*-[1,2,3]triazol-4-yl]-(2-éthyl-imidazo[5,1-*b*]thiazol-3-yl)-méthanol ;
[1-(4-benzyloxy-3-fluoro-phényl)-1*H*-[1,2,3]triazol-4-yl]-[2-(*trans*-2-fluoro-cyclopropyl)-imidazo[5,1-*b*]thiazol-3-yl]-méthanol ;
2-chloro-4-{4-[(2-éthyl-imidazo[5,1-*b*]thiazol-3-yl)-hydroxy-méthyl]-[1,2,3]triazol-1-yl}-phénol ;
2-chloro-4-(4-{hydroxy-[2-(1-méthyl-cyclopropyl)-imidazo[5,1-*b*]thiazol-3-yl]-méthyl}-[1,2,3]triazol-1-yl)-phénol ;
2-chloro-4-(4-{[2-(*cis*-2-fluoro-cyclopropyl)-imidazo[5,1-*b*]thiazol-3-yl]-hydroxy-méthyl}-[1,2,3]triazol-1-yl)-phénol ;
(2-cyclopropyl-imidazo[5,1-*b*]thiazol-3-yl)-[1-(4-méthoxy-2-méthyl-phényl)-5-méthyl-1*H*-[1,2,3]triazol-4-yl]-méthanol ;
(2-cyclopropyl-imidazo[5,1-*b*]thiazol-3-yl)-[1-(3-fluoro-4-méthoxy-phényl)-1*H*-[1,2,3]triazol-4-yl]-méthanol ;
2-chloro-4-(4-{[2-(*trans*-2-fluoro-cyclopropyl)-imidazo[5,1-*b*]thiazol-3-yl]-hydroxy-méthyl}-[1,2,3]triazol-1-yl)-phénol ;
2-chloro-4-(4-{[2-(1,1-difluoro-éthyl)-imidazo[5,1-*b*]thiazol-3-yl]-hydroxy-méthyl}-[1,2,3]triazol-1-yl)-phénol ;
(2-cyclopropyl-imidazo[5,1-*b*]thiazol-3-yl)-[1-(4-méthoxyméthyl-phényl)-1*H*-[1,2,3]triazol-4-yl]-méthanol ;
4-{4-[(2-cyclopropyl-imidazo[5,1-*b*]thiazol-3-yl)-hydroxy-méthyl]-[1,2,3]triazol-1-yl}-2,5-difluoro-phénol ;
4-{4-[(2-éthyl-imidazo[5,1-*b*]thiazol-3-yl)-hydroxy-méthyl]-[1,2,3]triazol-1-yl}-2,5-difluoro-phénol ;
[1-(5-chloro-2-fluoro-4-méthoxy-phényl)-5-méthyl-1*H*-[1,2,3]triazol-4-yl]-(2-cyclopropyl-imidazo[5,1-*b*]thiazol-3-yl)-méthanol ;
(2-cyclopropyl-imidazo[5,1-*b*]thiazol-3-yl)-[5-iodo-1-(4-méthoxy-phényl)-1*H*-[1,2,3]triazol-4-yl]-méthanol ;
[2-(*trans*-2-fluoro-cyclopropyl)-imidazo[5,1-*b*]thiazol-3-yl]-[5-fluoro-1-(4-méthoxy-phényl)-1*H*-[1,2,3]triazol-4-yl]-méthanol ; et
(2-cyclopropyl-imidazo[5,1-*b*]thiazol-3-yl)-[5-fluoro-1-(4-méthoxy-phényl)-1*H*-[1,2,3]triazol-4-yl]-méthanol ;

ou sel pharmaceutiquement acceptable de celui-ci.

**12.** Composition pharmaceutique comprenant un composé selon l'une quelconque des revendications 1 à 11, comprenant en outre au moins un véhicule pharmaceutiquement acceptable.

**13.** Composé selon l'une quelconque des revendications 1 à 11 pour une utilisation dans le traitement et/ou la prévention d'un cancer, où ledit composé est utilisé en combinaison avec un ou plusieurs agents chimiothérapeutiques et/ou une radiothérapie et/ou une thérapie ciblée.

**14.** Composé selon l'une quelconque des revendications 1 à 11 pour une utilisation en tant que médicament.

**15.** Composé selon l'une quelconque des revendications 1 à 11 pour une utilisation dans la prévention et/ou le traitement d'un cancer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010005958 A
- WO 2011037780 A
- WO 2012142237 A
- WO 2015173764 A
- WO 2016073770 A
- WO 2016161960 A
- WO 2017134555 A
- WO 2018036414 A
- WO 2017007700 A
- WO 2017189386 A
- WO 2017133258 A
- CN 107556244
- WO 2018057973 A
- WO 2018136887 A
- WO 2018171602 A
- WO 2018054365 A
- EP 2018072187 W
- WO 2015033299 A
- WO 2015044900 A
- WO 2015034820 A


### Non-patent literature cited in the description


- **PILOTTE et al.** *Proc Natl Acad Sci.,* 2012, vol. 109 (7), 2497-502
- **D'AMATO et al.** *Cancer Res.,* 2015, vol. 75 (21), 4651-64
- **UYTTENHOVE et al.** *Nat Med.,* 2003, vol. 9 (10), 1269-74
- **THEATE et al.** *Cancer Immunol Res.,* 2015, vol. 3 (2), 161-72
- **MUNN ; MELLOR.** *Trends in Immunol.,* 2016, vol. 37 (3), 193-207
- **LIU et al.** *Blood,* 2010, vol. 115 (17), 3520-30
- **FERNS et al.** *Oncoimmunology,* 2015, vol. 4 (2), e981457
- **ZHAI et al.** *Clin Cancer Res.,* 2015, vol. 21 (24), 5427-33
- **MULLER et al.** *Proc Natl Acad Sci USA.,* 2008, vol. 105 (44), 17073-8
- **HOLMGAARD et al.** *Cell Rep.,* 2015, vol. 13 (2), 412-24
- **BALACHANDRAN et al.** *Nat Med.,* 2011, vol. 17 (9), 1094-100
- **OPITZ et al.** *Nature,* 2011, vol. 478 (7368), 197-203
- **LIN et al.** *J Med Chem.,* 2016, vol. 59 (1), 419-30
- **KOBLISH et al.** *Mol Cancer Ther.,* 2010, vol. 9 (2), 489-98
- **KRAUS et al.** *AACR Annual Meeting,* 16 April 2016
- **WISE et al.** *AACR Annual Meeting,* 16 April 2016
- **LIU et al.** *AACR Annual Meeting,* 16 April 2016
- **YUE et al.** *J Med Chem.,* 2009, vol. 52 (23), 7364-7
- **YANG et al.** *J Med Chem.,* 2013, vol. 56 (21), 8321-31
- **HANIHARA et al.** *J Neurosurg.,* 2016, vol. 124 (6), 1594-601
- **HOU et al.** *Cancer Res.,* 2007, vol. 67 (2), 792-801
- **LI et al.** *Journal Immunother Cancer,* 2014, vol. 2, 21
- **ZHENG.** *ITOC3 conference,* 21 March 2016
- **KHLEIF.** *ITOC3 conference,* 21 March 2016
- **KOBLISH et al.** *AACR Annual Meeting,* 01 April 2017
- **MAUTINO et al.** *AACR Annual Meeting,* 05 April 2014
- **SPAHN et al.** *Journal for ImmunoTherapy of Cancer,* 2015, vol. 3 (2), 303
- **SPRANGER et al.** *J Immunother Cancer,* 2014, vol. 2, 3
- **HOLMGAARD et al.** *J Exp Med.,* 2013, vol. 210 (7), 1389-402
- **WAINWRIGHT et al.** *Clin Cancer Res.,* 2014, vol. 20 (20), 5290-301
- **REARDON et al.** *AACR Annual Meeting,* 01 April 2017
- **WANG et al.** *AACR Annual Meeting,* 16 April 2016
- **HOLMGAARD et al.** *EBioMedicine,* 2016, vol. 6, 50-8
- **NINOMIYA et al.** *Blood,* 2015, vol. 125 (25), 3905-16
- **LEMOS et al.** *Cancer Res.,* 2016, vol. 76 (8), 2076-81
- **THEVANDAVAKKAM et al.** *CNS Neurol Disord. Drug Targets,* 2010, vol. 9 (6), 791-800

- **ISHII et al.** *Arch Biochem Biophys.,* 1992, vol. 294 (2), 616-622
- **HIRAKU et al.** *Carcinogenesis,* 1995, vol. 16 (2), 349-56
- **STONE ; PERKINS.** *Eur J Pharmacol.,* 1981, vol. 72 (4), 411-2
- **SCHWARCZ et al.** *Science,* 1983, vol. 219 (4582), 316-8
- **VECSEI ; BEAL.** *Brain Res Bull.,* 1990, vol. 25 (4), 623-7
- **FOSTER et al.** *Neurosci Lett.,* 1984, vol. 48 (3), 273-8
- **CARPENEDO et al.** *Eur J Neurosci.,* 2001, vol. 13 (11), 2141-7
- **GODA et al.** *Adv. Exp. Med. Biol.,* 1999, vol. 467, 397-402
- **GUILLEMIN et al.** *Redox Rep,* 2000, vol. 5 (2-3), 108-11
- **LIM et al.** *International Congress Series,* 2007, vol. 1304, 213-7
- **SALTER ; POGSON.** *Biochem J.,* 1985, vol. 229 (2), 499-504
- **MILLER et al.** *Neurobiol Dis.,* 2004, vol. 15 (3), 618-29
- **GRAVES et al.** *Amyotroph Lateral Scler Other Motor Neuron Disord.,* 2004, vol. 5 (4), 213-9
- **HENKEL et al.** *Ann Neurol.,* 2004, vol. 55 (2), 221-35
- **MCGEER ; MCGEER.** *Muscle Nerve,* 2002, vol. 26 (4), 459-70
- **CHEN et al.** *Neurotox Res.,* 2010, vol. 18 (2), 132-42
- **FORREST et al.** *J Neurochem,* 2010, vol. 112 (1), 112-22
- **SAPKO et al.** *Exp Neurol.,* 2006, vol. 197 (1), 31-40
- **CAMPESAN et al.** *Curr Biol.,* 2011, vol. 21 (11), 961-6
- **GUILLEMIN et al.** *Redox Rep.,* 2002, vol. 7 (4), 199-206
- **WALKER et al.** *J Leukoc Biol.,* 2006, vol. 79, 596-610
- **GUILLEMIN et al.** *Neuropathol Appl Neurobiol.,* 2005, vol. 31 (4), 395-404
- **RAHMAN et al.** *PLOS One.,* 2009, vol. 4 (7), e6344
- **WU et al.** *PLOS One,* 2013, vol. 8 (4), e59749
- **WIDNER et al.** *J Neural Transm (Vienna),* 2000, vol. 107 (3), 343-53
- **BREDA et al.** *Proc Natl Acad Sci.,* 2016, vol. 113 (19), 5435-40
- **GAO ; HONG.** *Trends Immunol.,* 2008, vol. 29 (8), 357-65
- **BLOCK et al.** *Nat Rev Neurosci.,* 2007, vol. 8 (1), 57-69
- **OKUDA et al.** *J Neurochem.,* 1998, vol. 70 (1), 299-307
- **BRAIDY et al.** *Neurotox Res.,* 2009, vol. 16 (1), 77-86
- **JHAMANDAS et al.** *Brain Res.,* 1990, vol. 529 (1-2), 185-91
- **TRAPP et al.** *Curr Opin Neurol.,* 1999, vol. 12, 295-302
- **OWENS.** *Curr Opin Neurol.,* 2003, vol. 16, 259-265
- **FLANAGAN et al.** *J Neurochem.,* 1995, vol. 64, 1192-6
- **GUILLEMIN et al.** *J Interferon Cytokine Res.,* 2001, vol. 21, 1097-1101
- **AMIRKHANI et al.** *Eur. J. Neurol.,* 2005, vol. 12, 625-31
- **CAMMER et al.** *Brain Res.,* 2001, vol. 896, 157-160
- **BALL et al.** *Gene,* 2007, vol. 396 (1), 203-13
- **YUASA et al.** *J Mol Evol,* 2007, vol. 65 (6), 705-14
- **MERLO ; MANDIK-NAYAK.** *Clinical Medicine Insights: Pathology,* 2016, vol. 9 (S1), 21-28
- **HOLTZE et al.** *J Psychiatry Neurosci.,* 2012, vol. 37 (1), 53-7
- **BARRY et al.** *J Psychopharmacol.,* 2009, vol. 23 (3), 287-94
- **MILLER et al.** *Brain Res.,* 2006, (1073-1074), 25-37
- **MILLER et al.** *Neurochem Int.,* 2008, vol. 52 (6), 1297-303
- **DANTZER et al.** *Nat Rev Neurosci.,* 2008, vol. 9 (1), 46-56
- **SULLIVAN et al.** *Pain,* 1992, vol. 50 (1), 5-13
- **HEYES et al.** *Brain,* 1992, vol. 115 (5), 1249-73
- **KIM et al.** *J Clin Invest.,* 2012, vol. 122 (8), 2940-54
- **YAN et al.** *Journal of Neuroinflammation,* 2015, vol. 12 (110), 1-17
- **HOSHI et al.** *J Immunol.,* 2010, vol. 185 (6), 3305-3312
- **VAN DER SLUIJS et al.** *J Infect Dis.,* 2006, vol. 193 (2), 214-22
- **SUZUKI et al.** *J Infect.,* September 2011, vol. 63 (3), 215-22
- **MARANON et al.** *Parasite Immunol.,* 2013, vol. 35 (5-6), 180-7
- **BARTH ; RAGHURAMAN.** *Crit Rev Microbiol.,* 2014, vol. 40 (4), 360-8
- **SUZUKI et al.** *Clin Vaccine Immunol.,* 2012, vol. 19 (3), 436-442
- **MURRAY.** *Lancet Infect Dis.,* 2003, vol. 3 (10), 644-52
- **KANDANEARATCHI ; BREW.** *FEBS J.,* 2012, vol. 279 (8), 1366-74
- **STONE ; DARLINGTON.** *Trends Pharmacol Sci.,* 2013, vol. 34 (2), 136-43
- **LARREA et al.** *J Virol.,* 2007, vol. 81 (7), 3662-6

- **ASGHAR et al.** *Exp Ther Med.,* 2015, vol. 9 (3), 901-4
- **HARRINGTON et al.** *Infect Immunol.,* 2008, vol. 76 (7), 3045-53
- **LAURANS et al.** *Nature Medicine,* 2018, https://doi.org/10.1038/s41591-018-0060-4
- **NATIVIDAD et al.** *Cell Metabolism,* 2018, vol. 28, 1-13
- **TAKIKAWA et al.** *Adv Exp Med Biol.,* 1999, vol. 467, 241-5
- **TAYLOR et al.** *Exp Eye Res.,* 2002, vol. 75 (2), 165-75
- **MAILANKOT et al.** *Lab Invest.,* 2009, vol. 89 (5), 498-512
- **BURNEY et al.** *Endocrinology,* 2007, vol. 148 (8), 3814-26
- **AGHAJANOVA et al.** *Reprod Sci.,* 2011, vol. 18 (3), 229-251
- **MEI et al.** *Int J Clin Exp Pathol.,* 2013, vol. 6 (3), 431-44
- **MUNN et al.** *Science,* 1998, vol. 281 (5380), 1191-3
- **DÜRR ; KINDLER.** *J Leukoc Biol.,* 2013, vol. 93 (5), 681-700
- **TANKIEWICZ et al.** *Adv Exp Med Biol.,* 2003, vol. 527, 409-14
- **SALA et al.** *J Biol Chem.,* 2004, vol. 279 (49), 51033-41
- **WIRLEITNER et al.** *Eur J Clin Invest.,* 2003, vol. 33 (7), 550-4
- **FORREST et al.** *J. Neurochem.,* vol. 201,119 (1), 136-52
- **CUARTERO et al.** *Curr Pharm Des.,* 2016, vol. 22 (8), 1060-1073
- Handbook of Pharmaceutical Salts. Properties, Selection and Use. Wiley-VCH, 2008
- Pharmaceutical Salts and Co-crystals. RSC Publishing, 2012
- Pharmaceutical Manufacturing. **REMINGTON.** The Science and Practice of Pharmacy. Lippincott Williams & Wilkins, 2005
- **FEIG C et al.** *PNAS,* 2013
- **GOLDSTEIN et al.** *Clin. Cancer Res.,* 1995, vol. 1, 1311-1318
- **KOHL et al.** *Nat. Med.,* 1995, vol. 1 (8), 792-797
- **SEKULIC et al.** *Cancer Res.,* 2000, vol. 60, 3504-3513
- **SAUSVILLE.** *Curr. Med. Chem. Anti-Canc. Agents,* 2003, vol. 3, 47-56
- **VLAHOS et al.** *J Biol. Chem.,* 1994, vol. 269, 5241-5248
- **T.W. GREENE ; P.G.M. WUTS.** Protective Groups in Organic Synthesis. Wiley-Interscience, 1999
- *Biochem et Biophysica Acta,* 2011, vol. 1814, 1947-1954
- *J. Appl. Cryst.,* 2007, vol. 40, 658-674
- **SEEGERS et al.** *JBS,* 2014